(19) Europäisches Patentamt
European Patent Office
Office européen des brevets

(11) **EP 4 778 929 A1**

(12) **EUROPEAN PATENT APPLICATION**
published in accordance with Art. 153(4) EPC

(43) Date of publication:
**22.07.2026 Bulletin 2026/30**

(21) Application number: **24876585.1**

(22) Date of filing: **10.10.2024**

(51) International Patent Classification (IPC):
***C07D 491/22*** *(2006.01)* ***A61K 31/4745*** *(2006.01)*
***A61P 35/00*** *(2006.01)* ***A61P 31/00*** *(2006.01)*

(52) Cooperative Patent Classification (CPC):
**A61K 31/4353; A61K 31/4745; A61K 31/5375; A61K 31/542; A61P 31/00; A61P 35/00; C07D 491/22; C07D 498/22; C07D 513/22**

(86) International application number:
**PCT/CN2024/123915**

(87) International publication number:
**WO 2025/077773 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.10.2023 CN 202311305399**

(71) Applicant: **Shanghai Innovkong Pharmaceutical Technology Co., Ltd**
**Shanghai 201203 (CN)**

(72) Inventors:
- **DANG, Qun**
  **Shanghai 201203 (CN)**
- **FU, Xiaodan**
  **Shanghai 201203 (CN)**
- **LI, Yaozong**
  **Shanghai 201203 (CN)**
- **HU, Dongmei**
  **Shanghai 201203 (CN)**
- **WANG, Qilin**
  **Shanghai 201203 (CN)**
- **LIU, Hui**
  **Shanghai 201203 (CN)**
- **QIAN, Jianghui**
  **Shanghai 201203 (CN)**
- **LI, Shuai**
  **Shanghai 201203 (CN)**
- **LIU, Weimin**
  **Shanghai 201203 (CN)**

(74) Representative: **Sun, Yanan**
**Hassler International Germany GmbH**
**Postfach 940247**
**60460 Frankfurt am Main (DE)**

# (54) NOVEL TYPE I TOPOISOMERASE INHIBITOR, AND PREPARATION METHOD THEREFOR AND USE THEREOF

(57) The application relates to a new type I topoisomerase inhibitor and a preparation method and use thereof. Specifically, the application relates to compounds shown in formulas (I) and (I)B, and stereoisomers, geometric isomers, tautomers, pharmaceutically acceptable salts, prodrugs, solvates, esters, polymorphs, N-oxides, metabolites or isotopically labeled analogues thereof. The application also relates to a method and application of the compound for treating diseases mediated by topoisomerase I (Topo I).



Processed by Luminess, 75001 PARIS (FR)

**(Cont. next page)**

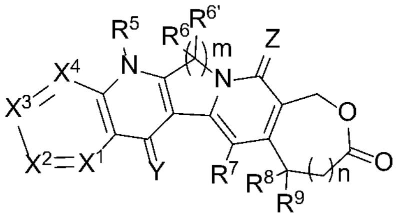

(I)

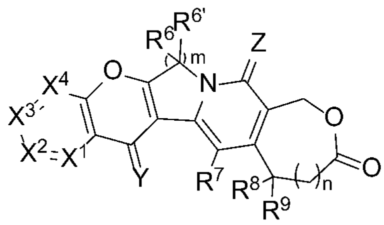

(I)B

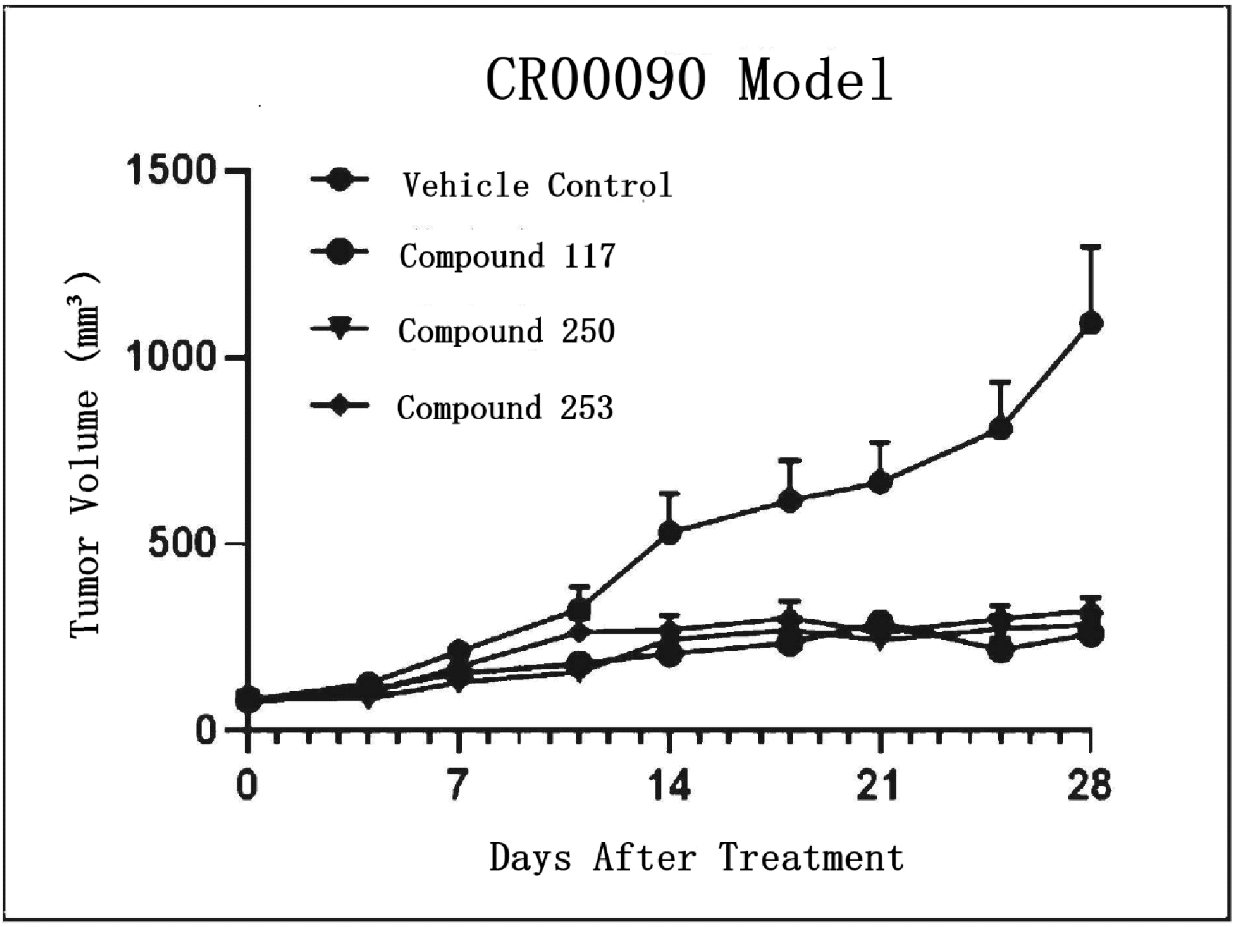
CR00090 Model
Vehicle Control
Compound 117
Compound 250
Compound 253
Tumor Volume (mm³)
1500
1000
500
0
0
7
14
21
28
Days After Treatment

FIG. 2

## Description

### Field

The present invention relates to the field of medicinal chemistry, and in particular to topoisomerase I (Topo I) inhibitory compounds, processes for preparing the same, and uses thereof..

### Background

Camptothecin (CPT) is a pyrroloquinoline cytotoxic alkaloid, which is mainly derived from Camptotheca acuminata Decne of the Nyssaceae family, a plant endemic to China. CPT was first isolated from the stems of Camptotheca acuminata Decne introduced from China by Wall et al. in 1966. Subsequently, the structure of CPT was identified, which is formed by the annulation of five rings, namely Ring A, Ring B, Ring C, Ring D and Ring E. Among them, Ring A and Ring B are quinoline ring, Ring C is a pyrrole ring, Ring D is a pyridone ring, and Ring E is an $\alpha$-hydroxyl lactone ring with a chiral carbon of S-configuration. The structure and carbon atom numbering of CPT are shown as follows:

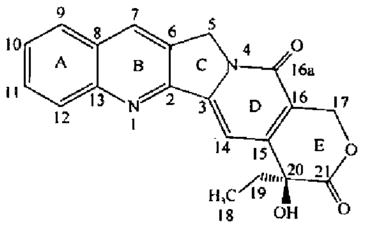

Due to the novel structure of camptothecin and its significant anti-tumor efficacy demonstrated in in vitro experiments and in vivo animal experiments, an upsurge of research on camptothecin was set off in the academic and pharmaceutical field for the first time. However, researchers found that camptothecin has the defects of poor water solubility, inactivation caused by the instability of its lactone ring and severe side effects, which led to a slowdown in the research on camptothecin. Until 1985, Hsiang et al. from Johns Hopkins University reported the unique mechanism of action of camptothecin, i.e., the selective inhibition of DNA topoisomerase I, which made camptothecin attract widespread attention again. To overcome the defects of camptothecin, the development of camptothecin derivatives with high efficiency and low toxicity has become a new research hotspot.

DNA topoisomerases are a class of essential enzymes widely existing in organisms, which affect the topological structure of DNA by regulating supercoiling, catenation, decatenation and nucleic acid dissociation. DNA topoisomerases are mainly divided into topoisomerase I (Topo I) and topoisomerase II (Topo II). Compared with Topo II inhibitors, Topo I inhibitors have high curative effect and a broad anti-tumor spectrum, and thus have become an important target enzyme for the design of novel anti-tumor drugs. Meanwhile, the content of Topo I in a variety of tumor cells such as colon cancer cells, cervical cancer cells and ovarian cancer cells is significantly higher than that in normal tissues, and the activity of Topo I is greatly enhanced in tumor cells at the S phase. Therefore, drugs for inhibiting Topo I can selectively inhibit the DNA replication of tumor cells in the proliferative phase, and have good selectivity. Experiments have confirmed that Topo I is the main target of CPT and its analogs.

Since the discovery of the Topo I inhibitory effect of camptothecin, continuous efforts have been devoted to the search for camptothecin derivatives with high efficiency and low toxicity. Camptothecins are currently the most prominent class of alkaloid anti-tumor drugs with the broadest development and application prospects, which have attracted research efforts from numerous research groups. In recent years, researchers from the United States, Japan, France, Germany, South Korea and other countries have taken a leading position in the research and development of camptothecin derivatives. Hundreds of camptothecin derivatives have been synthesized successively and subjected to activity screening. The camptothecin-based anti-tumor compounds that have been approved for clinical application or are under clinical research are shown as follows:

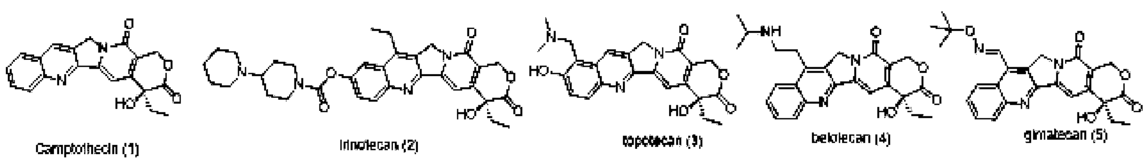

Camptothecin (1) Irinotecan (2) topotecan (3) belotecan (4) gimatecan (5)

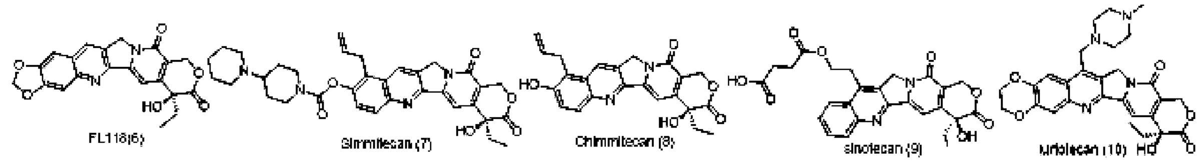

FL118(6) Simmitecan (7) Chimmitecan (8) sinotecan (9) lurtotecan (10)

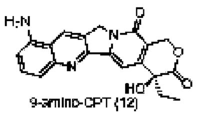
9-amino-CPT (12)

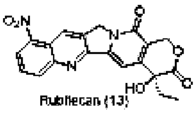
Rubitecan (13)

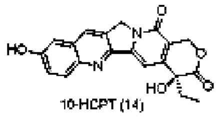
10-HCPT (14)

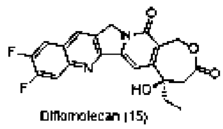
Diflomotecan (15)

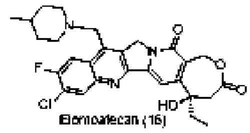
Elomotecan (16)

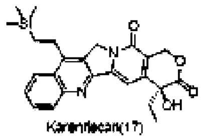
Karenitecan(17)

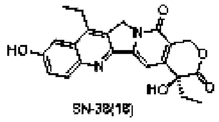
SN-38(18)

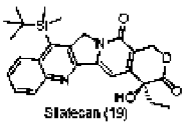
Silatecan (19)

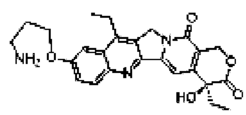
Delimotecan(20)

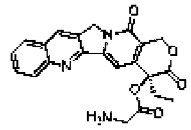
NSC606985(21)

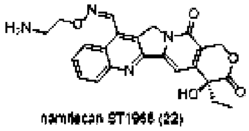
namitecan ST1968 (22)

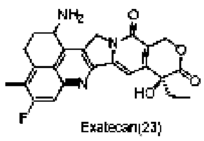
Exatecan(23)

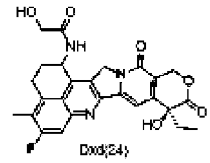
Dxd(24)

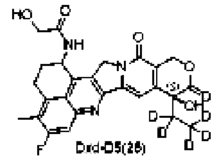
Dxd-D5(25)

After decades of development, camptothecin-based anti-tumor drugs with better anti-tumor activity and lower toxic and side effects, such as topotecan, irinotecan (CPT-11), lurtotecan and hydroxycamptothecin (HCPT), which have been obtained through the structural modification of CPT at the 7-, 9-, 10- and 11-positions of the ring. Three camptothecin-based compounds have been approved for marketing for tumor therapy: irinotecan, which was first approved by the FDA in 1994 and is a first-line therapeutic drug for colorectal cancer; topotecan, which was first approved by the FDA in 1996 and is used for the treatment of ovarian cancer; and belotecan, which was approved in South Korea in 2005 and is used for the treatment of small cell lung cancer. Since the approval of the aforesaid drugs, certain achievements have also been made in the research on expanding their indications and dosage forms.

However, the research and development of camptothecin-based compounds mostly have several major problems: 1. Poor druggability, the special structure of camptothecin results in extremely poor liposolubility and water solubility, thus water-solubility modification is imperative; 2. Camptothecin-based compounds have certain toxicity, and an over-optimized water-solubility modification will also lead to an instantaneous rise in blood drug concentration, thereby inducing toxic and side effects; 3. The prodrug modification of camptothecin needs to take into account the release efficiency and stability, which are always a pair of contradictory factors in the rational prodrug design. The development of both prodrugs and conjugate drugs (XDC) relies on various enzymes and targets, which leads to a large individual difference, resulting in inconsistent responses of patients to prodrugs and a high tendency to induce toxicity simultaneously.

Camptothecin-based drugs or their derivatives have hematological toxicity caused by myelosuppression, such as neutropenia, leukopenia, thrombocytopenia and anemia, as well as gastrointestinal side effects, such as nausea, vomiting and diarrhea. Clinical studies have found that the measures for improving the safety and effectiveness of camptothecin-based compounds include ameliorating their pharmacokinetic properties, regulating their activity, reducing the dosage, or preparing antibody-drug conjugates by conjugating their conjugates with antibodies.

For decades, the modification of camptothecin-based compounds has basically been focused on side chain modification while keeping the five-ring parent nucleus structure unchanged, and such modification strategies may not fundamentally overcome the existing problems of camptothecin-based drugs. Therefore, the research and development of camptothecin-based compounds and their conjugates with novel structures, improved effectiveness and enhanced safety still have high clinical demand and application value. At present, there is an urgent need to adopt new strategies to develop novel compounds with market potential, better druggability, pharmacodynamic effects and pharmacokinetic outcomes, so as to solve the defects faced by camptothecin-based compounds.

## Content

The objective of the invention is to provide a camptothecin analog with a novel parent nucleus structure,

preparation methods, and uses in the treatment of diseases mediated by topoisomerase I (Topo I).

In the first part of the invention, there is provided a compound of formula (I) or (I)B, and stereoisomers, geometric isomers, tautomers, pharmaceutically acceptable salts, prodrugs, solvates, esters, polymorphs, N-oxides, metabolites or isotopically labeled analogs.

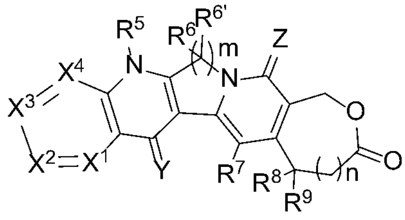

**(I)**

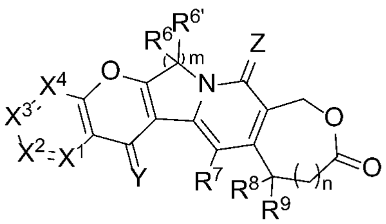

(I)B

wherein:

Y is selected from S, O, -$CR^{10}$a$R^{10}$b or -$NR^{10}$a, wherein $R^{10}$a and $R^{10b}$ are independently selected from hydrogen, hydroxyl, amino, alkyl, alkenyl, alkynyl, cycloalkyl, heterocycloalkyl, aryl and heteroaryl;
Z is selected from S, O, -$CR^{11}$a$R^{11}$b or -$NR^{11}$a, wherein $R^{11}$a and $R^{11b}$ are independently selected from hydrogen, hydroxyl, amino, alkyl, alkenyl, alkynyl, cycloalkyl, heterocycloalkyl, aryl and heteroaryl;
n is 0, 1, 2 or 3; m is 0, 1, 2 or 3;
$X^1$ is selected from N or C($R^1$), wherein $R^1$ is selected from hydrogen, deuterium, halogen, hydroxyl, mercapto, amino, cyano, nitro, optionally substituted alkyl, optionally substituted alkoxy, optionally substituted cycloalkyl, optionally substituted heterocycloalkyl, -$OR^{1a}$, -$SR^{1a}$, -$NR^{1a}R^{1b}$, -$COR^{1a}$, -$SOR^{1a}$, -$SO_2R^{1a}$, -$COOR^{1a}$, -$CONR^{1a}R^{1b}$, - $NR^{1a}$-$COR^{1b}$, -$OCOR^{1a}$, -$SONR^{1a}R^{1b}$, -$SO_2NR^{1a}R^{1b}$, -$NR^{1a}SOR^{1b}$, -$NR^{1a}SO_2R^{1b}$ and -Si($R^{1a}$)($R^{1b}$)($R^{1c}$), wherein $R^{1a}$, $R^{1b}$ and $R^{1c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;
$X^2$ is selected from N or C($R^2$), wherein $R^2$ is selected from hydrogen, deuterium, halogen, hydroxyl, mercapto, amino, cyano, nitro, optionally substituted alkyl, optionally substituted alkoxy, optionally substituted cycloalkyl, optionally substituted heterocycloalkyl, -$OR^{2a}$, -$SR^{2a}$, -$NR^{2a}R^{2b}$, -$COR^{2a}$, -$SOR^{2a}$, -$SO_2R^{2a}$, -$COOR^{2a}$, -$CONR^{2a}R^{2b}$, - $NR^{2a}$-$COR^{2b}$, -$OCOR^{2a}$, -$SONR^{2a}R^{2b}$, -$SO_2NR^{2a}R^{2b}$, -$NR^{2a}SOR^{2b}$, -$NR^{2a}SO_2R^{2b}$ and -Si($R^{2a}$)($R^{2b}$)($R^{2c}$), wherein $R^{2a}$, $R^{2b}$ and $R^{2c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;
or $R^1$ and $R^2$, together with the atoms to which they are attached, form an optionally substituted cyclic structure;
$X^3$ is selected from N or C($R^3$), wherein $R^3$ is selected from hydrogen, deuterium, halogen, hydroxyl, mercapto, amino, cyano, nitro, optionally substituted alkyl, optionally substituted alkoxy, optionally substituted cycloalkyl, optionally substituted heterocycloalkyl, -$OR^{3a}$, -$SR^{3a}$, -$NR^{3a}R^{3b}$, -$COR^{3a}$, -$SOR^{3a}$, -$SO_2R^{3a}$, -$COOR^{3a}$, -$CONR^{3a}R^{3b}$, - $NR^{3a}$-$COR^{3b}$, -$OCOR^{3a}$, -$SONR^{3a}R^{3b}$, -$SO_2NR^{3a}R^{3b}$, -$NR^{3a}SOR^{3b}$, -$NR^{3a}SO_2R^{3b}$ and -Si($R^{3a}$)($R^{3b}$)($R^{3c}$), wherein $R^{3a}$, $R^{3b}$ and $R^{3c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;
or $R^2$ and $R^3$, together with the atoms to which they are attached, form an optionally substituted cyclic structure;
$X^4$ is selected from N or C($R^4$), wherein $R^4$ is selected from hydrogen, deuterium, halogen, hydroxyl, mercapto, amino, cyano, nitro, optionally substituted alkyl, optionally substituted alkoxy, optionally substituted cycloalkyl, optionally substituted heterocycloalkyl, -$OR^{4a}$, -$SR^{4a}$, -$NR^{4a}R^{4b}$, -$COR^{4a}$, -$SOR^{4a}$, -$SO_2R^{4a}$, -$COOR^{4a}$, -$CONR^{4a}R^{4b}$, - $NR^{4a}$-$COR^{4b}$, -$OCOR^{4a}$, -$SONR^{4a}R^{4b}$, -$SO_2NR^{4a}R^{4b}$, -$NR^{4a}SOR^{4b}$, -$NR^{4a}SO_2R^{4b}$ and -Si($R^{4a}$)($R^{4b}$)($R^{4c}$), wherein $R^{4a}$, $R^{4b}$ and $R^{4c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;
or $R^3$ and $R^4$, together with the atoms to which they are attached, form an optionally substituted cyclic structure;
$R^5$ is selected from hydrogen, deuterium, hydroxyl, amino, optionally substituted alkyl, optionally substituted alkoxy, optionally substituted cycloalkyl, optionally substituted heterocycloalkyl, optionally substituted aryl, optionally substituted heteroaryl, -$OR^{5a}$, -$SR^{5a}$, -$NR^{5a}R^{5b}$, -$COR^{5a}$, -$SOR^{5a}$, -$SO_2R^{5a}$, -$COOR^{5a}$, -$CONR^{5a}R^{5b}$, - $NR^{5a}COR^{5b}$, -$OCOR^{5a}$, -$SONR^{5a}R^{5b}$, -$SO_2NR^{5a}R^{5b}$, -$NR^{5a}SOR^{5b}$, -$NR^{5a}SO_2R^{5b}$ and -Si($R^{5a}$)($R^{5b}$)($R^{5c}$), wherein $R^{5a}$, $R^{5b}$ and $R^{5c}$ are independently selected from hydrogen, deuterium, -Boc, -Cbz, optionally substituted alkyl, optionally

substituted alkenyl, optionally substituted alkynyl, optionally substituted cycloalkyl, optionally substituted heterocycloalkyl, optionally substituted aryl and optionally substituted heteroaryl;

$R^6$ and $R^6$ are independently selected from hydrogen, deuterium, hydroxyl, amino, optionally substituted alkyl, optionally substituted alkoxy, optionally substituted cycloalkyl, optionally substituted heterocycloalkyl, $-OR^{6a}$, $-SR^{6a}$, $-NR^{6a}R^{6b}$, $-COR^{6a}$, $-SOR^{6a}$, $-SO_2R^{6a}$, $-COOR^{6a}$, $-CONR^{6a}R^{6b}$, $-NR^{6b}COR^{6a}$, $-OCOR^{6a}$, $-SONR^{6a}R^{6b}$, $-SO_2NR^{6a}R^{6b}$, $-NR^{6a}SOR^{6b}$, $-NR^{6a}SO_2R^{6b}$ and $-Si(R^{6a})(R^{6b})(R^{6c})$, wherein $R^{6a}$, $R^{6b}$ and $R^{6c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

$R^7$ is selected from hydrogen, deuterium, halogen, hydroxyl, mercapto, amino, cyano, nitro, optionally substituted alkyl, optionally substituted alkoxy, optionally substituted cycloalkyl, optionally substituted heterocycloalkyl, $-OR^{7a}$, $-SR^{7a}$, $-NR^{7a}R^{7b}$, $-COR^{7a}$, $-SOR^{7a}$, $-SO_2R^{7a}$, $-COOR^{7a}$, $-CONR^{7a}R^{7b}$, $-NR^{7a}COR^{7b}$, $-OCOR^{7a}$, $-SONR^{7a}R^{7b}$, $-SO_2NR^{7a}R^{7b}$, $-NR^{7a}SOR^{7b}$, $-NR^{7a}SO_2R^{7b}$ and $-Si(R^{7a})(R^{7b})(R^{7c})$, wherein $R^{7a}$, $R^{7b}$ and $R^{7c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

$R^8$ is selected from hydrogen, deuterium, halogen, $-OR^{8a}$, $-OCOR^{8a}$, $-SR^{8a}$, $-OR^{8a}-SR^{8b}$ or $-NR^{8a}R^{8b}$, wherein $R^{8a}$ and $R^{8b}$ are independently selected from hydrogen, deuterium, optionally substituted alkyl and optionally substituted cycloalkyl;

$R^9$ is selected from $-CH_2R^{9a}$, $-CF_2R^{9a}$, $-CHFR^{9a}$, $-CD_2R^{9a}$, $-CDHR^{9a}$, $-CDFR^{9a}$, $-OR^{9a}$, $-SR^{9a}$ and $-NR^{9a}R^{9b}$, wherein $R^{9a}$ and $R^{9b}$ are independently selected from hydrogen, deuterium, optionally substituted alkyl, optionally substituted alkenyl, optionally substituted alkynyl and optionally substituted cycloalkyl.

The present invention also provides a method for treating diseases mediated by topoisomerase I (Topo I), the method comprising administering to a patient in need thereof a compound of formula (I), and stereoisomers, geometric isomers, tautomers, pharmaceutically acceptable salts, prodrugs, solvates, esters, polymorphs, N-oxides, metabolites or isotopically labeled analogs thereof.

The present invention also provides a pharmaceutical composition comprising a compound of formula (I), and stereoisomers, geometric isomers, tautomers, pharmaceutically acceptable salts, prodrugs, solvates, esters, polymorphs, N-oxides, metabolites or isotopically labeled analogs thereof, and a pharmaceutically acceptable excipient.

The present invention also provides an application of a compound of formula (I), and stereoisomers, geometric isomers, tautomers, pharmaceutically acceptable salts, prodrugs, solvates, esters, polymorphs, N-oxides, metabolites or isotopically labeled analogs thereof, in the preparation of a medicament for the treatment of diseases mediated by topoisomerase I (Topo I).

## Brief Description of the Drawings

**Figure 1** The pharmacodynamic effects of Example 2, Example 250 and Example 253 in a subcutaneous xenograft model of human colon cancer HCT-116 cell line in female BALB/c nude mice.

**Figure 2** The pharmacodynamic effects of Example 117, Example 250 and Example 253 in the CR00090 human colon cancer model.

## Detailed Description of the Embodiments

### Definitions

Unless otherwise specified, the term alkyl refers to a monovalent saturated aliphatic hydrocarbon group, including straight-chain or branched-chain groups containing 1 to 20 carbon atoms, preferably 1 to 10 carbon atoms (i.e., C1-10 alkyl), more preferably 1 to 8 carbon atoms (C1-8 alkyl), and still more preferably 1 to 6 carbon atoms (i.e., C1-6 alkyl). For example, C1-6 alkyl means the group is an alkyl group with the number of carbon atoms in the carbon chain being 1 to 6 (specifically 1, 2, 3, 4, 5 or 6). Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl and the like.

Unless otherwise specified, the term cycloalkyl refers to a cyclic saturated aliphatic hydrocarbon group with a specific number of carbon atoms, preferably containing 3 to 12 carbon atoms (i.e., C3-12 cycloalkyl), more preferably 3 to

10 carbon atoms (C3-10 cycloalkyl), still more preferably 3 to 8 carbon atoms (C3-8 cycloalkyl), 3 to 6 carbon atoms (C3-6 cycloalkyl), 4 to 6 carbon atoms (C4-6 cycloalkyl) or 5 to 6 carbon atoms (C5-6 cycloalkyl). Examples include, but are not limited to, cyclopropyl, cyclobutyl, cyclopentyl, cyclohexyl, spiro[3.3]heptan-2-yl, spiro[3.3]heptan-1-yl, bicyclo[3.1.0]hexan-3-yl, spiro[2.3]hexan-5-yl, bicyclo[1.1.1]pentan-2-yl, spiro[2.5]octan-6-yl and the like.

Unless otherwise specified, the term alkoxy refers to -O-alkyl, wherein the alkyl is as defined above, i.e., containing 1 to 20 carbon atoms, preferably 1 to 10 carbon atoms, more preferably 1 to 8 carbon atoms, and still more preferably 1 to 6 carbon atoms (specifically 1, 2, 3, 4, 5 or 6). Representative examples include, but are not limited to, methoxy, ethoxy, propoxy, isopropoxy, butoxy, 1-methylpropoxy, 2-methylpropoxy, tert-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy and the like.

Unless otherwise specified, the term halogen or halogenated refers to F, Cl, Br or I. The term haloalkyl refers to an alkyl group as defined above in which one, two, more or all hydrogen atoms are substituted with halogen atoms. Representative examples of haloalkyl include CCl3, CF3, CHCl2, CH2Cl, CH2Br, CH2I, CH2CF3, CF2CF3 and the like.

Unless otherwise specified, the term heterocycloalkyl refers to a saturated or partially unsaturated monocyclic, bicyclic or polycyclic cyclic hydrocarbon substituent with a non-aromatic structure, containing 3 to 20 ring atoms, wherein 1, 2, 3 or more ring atoms are selected from N, O or S, and the remaining ring atoms are C. Preferably, it contains 3 to 12 ring atoms, more preferably 3 to 10 ring atoms, 3 to 8 ring atoms, 3 to 6 ring atoms, 4 to 6 ring atoms or 5 to 6 ring atoms. The number of heteroatoms is preferably 1 to 4, more preferably 1 to 3 (i.e., 1, 2 or 3). Examples of monocyclic heterocycloalkyl include pyrrolidinyl, imidazolidinyl, tetrahydrofuranyl, dihydropyrrolyl, piperidinyl, piperazinyl, pyranyl and the like. Polycyclic heterocycloalkyl includes spiro, fused and bridged heterocycloalkyl groups, such as 8-azabicyclo[3.2.1]octan-8-yl and 5-azaspiro[2.3]hexan-5-yl.

Unless otherwise specified, the term carbocyclyl or carbocycle refers to a non-aromatic cyclic hydrocarbon group having 3 to 14 ring carbon atoms ("C3-14 carbocyclyl") and no heteroatoms in the non-aromatic ring system. In some embodiments, the carbocyclyl group has 3 to 12 ring carbon atoms ("C3-12 carbocyclyl"), 4 to 12 ring carbon atoms ("C4-12 carbocyclyl"), or 3 to 10 ring carbon atoms ("C3-10 carbocyclyl"). In some embodiments, the carbocyclyl group has 3 to 8 ring carbon atoms ("C3-8 carbocyclyl"). In some embodiments, the carbocyclyl group has 3 to 7 ring carbon atoms ("C3-7 carbocyclyl"). In some embodiments, the carbocyclyl group has 4 to 6 ring carbon atoms ("C4-6 carbocyclyl"). In some embodiments, the carbocyclyl group has 5 to 10 ring carbon atoms ("C5-10 carbocyclyl") or 5 to 7 ring carbon atoms ("C5-7 carbocyclyl"). Exemplary C3-6 carbocyclyl groups include, but are not limited to, cyclopropyl (C3), cyclopropenyl (C3), cyclobutyl (C4), cyclobutenyl (C4), cyclopentyl (C5), cyclopentenyl (C5), cyclohexyl (C6), cyclohexenyl (C6), cyclohexadienyl (C6) and the like. Exemplary C3-8 carbocyclyl groups include, but are not limited to, the aforementioned C3-6 carbocyclyl groups as well as cycloheptyl (C7), cycloheptenyl (C7), cycloheptadienyl (C7), cycloheptatrienyl (C7), cyclooctyl (C8), cyclooctenyl (C8), bicyclo[2.2.1]heptyl (C7), bicyclo[2.2.2]octyl (C8) and the like. Exemplary C3-10 carbocyclyl groups include, but are not limited to, the aforementioned C3-8 carbocyclyl groups as well as cyclononyl (C9), cyclononenyl (C9), cyclodecyl (C10), cyclodecenyl (C10), octahydro-1H-indenyl (C9), decahydronaphthyl (C10), spiro[4.5]decyl (C10) and the like. As illustrated by the above examples, in certain embodiments, the carbocyclyl group is monocyclic ("monocyclic carbocyclyl") or a fused (fused carbocyclyl), bridged (bridged carbocyclyl) or spiro-fused (spiro carbocyclyl) ring system, such as a bicyclic system ("bicyclic carbocyclyl"), and may be saturated or partially unsaturated. Carbocyclyl also includes ring systems in which the carbocyclyl ring as defined above is fused to one or more aryl or heteroaryl groups, where the point of attachment is on the carbocyclyl ring, and in such cases, the number of carbons continues to indicate the number of carbons in the carbocyclic system. In certain embodiments, each instance of a carbocyclyl group is independently optionally substituted, e.g., unsubstituted ("unsubstituted carbocyclyl") or substituted with one or more substituents ("substituted carbocyclyl"). In certain embodiments, the carbocyclyl group is unsubstituted C3-10 carbocyclyl. In certain embodiments, the carbocyclyl group is substituted C3-10 carbocyclyl.

Unless otherwise specified, the term aryl refers to a monocyclic, bicyclic and tricyclic aromatic carbocyclic system containing 6 to 16 carbon atoms, 6 to 14 carbon atoms, 6 to 12 carbon atoms or 6 to 10 carbon atoms, preferably 6 to 10 carbon atoms. The term aryl may be used interchangeably with the term aromatic ring. Examples of aryl groups include, but are not limited to, phenyl, naphthyl, anthryl, phenanthryl, pyrenyl and the like.

Unless otherwise specified, the term heteroaryl refers to an aromatic monocyclic or polycyclic ring system having a 5 to 12 membered structure, preferably a 5 to 10 membered structure or a 5 to 8 membered structure, and still more preferably a 5 to 6 membered structure, wherein 1, 2, 3 or more ring atoms are heteroatoms and the remaining atoms are carbon, the heteroatoms are independently selected from O, N or S, and the number of heteroatoms is preferably 1, 2 or 3. Examples of heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl and the like.

Unless otherwise specified, the terms pharmaceutically acceptable salt, medicinal salt or acceptable salt refer to salts that are suitable for contact with the tissues of mammals, particularly humans, within the scope of sound medical judgment, without excessive toxicity, irritation, allergic reactions and the like, and are commensurate with a reasonable benefit/risk ratio. Pharmaceutically acceptable salts of amines, carboxylic acids and other types of compounds are well known in this field. The salts may be prepared in situ during the final isolation and purification of the compounds of the present invention, or separately by reacting the free base or free acid with a suitable reagent, as outlined below. For example, free base functionalities may be reacted with a suitable acid.

Unless otherwise specified, the term solvate means a physical association of a compound of the present invention with one or more solvent molecules (whether organic or inorganic). The physical association includes hydrogen bonding. In certain cases, the solvate can be isolated, for example, when one or more solvent molecules are incorporated into the crystal lattice of a crystalline solid. The solvent molecules in the solvate may be present in a regular arrangement and/or an unordered arrangement. A solvate may contain stoichiometric or non-stoichiometric amounts of solvent molecules. Solvate encompasses solution-phase and isolable solvates. Exemplary solvates include, but are not limited to, hydrates, ethanolates, methanolates, isopropanolates and the like. Methods of solvation are well known in the art.

Unless otherwise specified, the term isotopically labeled analog refers to a molecule in which the compound is labeled with an isotope, thereby providing an isotopically labeled analog that may have improved pharmacological activity. Isotopes commonly used for isotopic labeling are: hydrogen isotopes, 2H and 3H; carbon isotopes: 11C, 13C and 14C; chlorine isotopes: 35Cl and 37Cl; fluorine isotope: 18F; iodine isotopes: 123I and 125I; nitrogen isotopes: 13N and 15N; oxygen isotopes: 150, 170 and 180; and sulfur isotope: 35S. These isotopically labeled compounds can be used to study the distribution of medicinal molecules in tissues. In particular, deuterium (3H) and carbon (13C) are more widely used due to their ease of labeling and detection. Substitution with certain heavy isotopes, such as deuterium (2H), can enhance metabolic stability and prolong half-life, thereby achieving the purpose of reducing dosage and providing therapeutic advantages. Isotopically labeled compounds are generally synthesized starting from labeled starting materials using known synthetic techniques in the same manner as the synthesis of non-isotopically labeled compounds.

Unless otherwise specified, the term prodrug refers to a drug that is converted to the parent drug in vivo. Prodrugs are often useful because, in some cases, they may be easier to administer than the parent drug. For example, they may be bioavailable by oral administration, whereas the parent drug may not be. Prodrugs also have improved solubility in pharmaceutical compositions compared to the parent drug. An example of a prodrug, without limitation, may be any compound of formula (I) administered as an ester ("prodrug") to facilitate transport across cell membranes where water solubility is detrimental to mobility, but beneficial once inside the cell, which is then metabolically hydrolyzed to the carboxylic acid, i.e., the active entity. Another example of a prodrug may be a short peptide (polypeptide) conjugated to an acid group, wherein the peptide is metabolized to reveal the active moiety.

Unless otherwise specified, the term stereoisomer refers to compounds having the same chemical constitution but differing in the spatial arrangement of atoms or groups. Stereoisomers include enantiomers, diastereomers, conformers (rotamers), geometric (cis/trans) isomers, atropisomers and the like. Any resulting mixture of stereoisomers may be separated into pure or substantially pure geometric isomers, enantiomers or diastereomers based on differences in the physicochemical properties of the components, for example, by chromatography and/or fractional crystallization.

Unless otherwise specified, the term tautomer refers to structural isomers with different energies that can be interconverted through a low energy barrier. If tautomerism is possible (e.g., in solution), a chemical equilibrium of tautomers can be achieved. For example, proton tautomers (also called prototropic tautomers) include interconversions via proton migration, such as keto-enol isomerization and imine-enamine isomerization. Valence tautomers include interconversions via the reorganization of some bonding electrons.

Unless otherwise specified, the term N-oxide refers to an oxide (e.g., mono- or di-oxide) of at least one nitrogen atom in the structure of the compound of the present application. The mono-oxide of nitrogen may exist as a single positional isomer or a mixture of positional isomers.

Unless otherwise specified, the term ester refers to an ester derived from each of the general formula compounds in the present application, including physiologically hydrolysable esters (esters that can be hydrolyzed under physiological conditions to release the compound of the present invention in the form of a free acid or alcohol). The compounds of the present invention may themselves be esters.

Also included within the scope of the present invention are metabolites of the compounds of the present invention, i.e., substances formed in vivo upon administration of the compounds of the present invention. Such products may result from, for example, oxidation, reduction, hydrolysis, amidation, deamidation, esterification, deesterification, enzymatic hydrolysis and the like of the administered compound. Accordingly, the present invention includes metabolites of the compounds of the present invention, including compounds prepared by a method of contacting a compound of the present invention with a mammal for a time sufficient to produce a metabolite thereof.

Unless otherwise indicated, the structural formulas depicted in the present invention include all isomeric forms (e.g., enantiomeric, diastereomeric, and geometric (or conformational) isomers): for example, the R and S configurations at asymmetric centers, the (Z) and (E) isomers of double bonds, and the conformational isomers of (Z) and (E). Thus,

individual stereochemical isomers of the compounds of the present invention, or mixtures of their enantiomers, diastereomers, or geometric (or conformational) isomers, are within the scope of the present invention.

Unless otherwise specified, the term optionally substituted means that the hydrogen atom at the substitutable position of the group is unsubstituted or substituted with one or more substituents, the substituents are preferably selected from the group consisting of: halogen, hydroxyl, mercapto, cyano, nitro, amino, azido, carbonyl, carboxyl, C2-6 alkenyl, C2-6 alkynyl, C1-6 alkyl, C1-6 alkoxy, C3-10 cycloalkyl, C3-10 cycloalkylsulfonyl, 3- to 10-membered heterocycloalkyl, C6-14 aryl or 5- to 10-membered heteroaryl, wherein the C2-6 alkenyl, C2-6 alkynyl, C1-6 alkyl, C1-6 alkoxy, C3-10 cycloalkyl, C3-10 cycloalkylsulfonyl, 3- to 10-membered heterocycloalkyl, C6-14 aryl or 5- to 10-membered heteroaryl may be optionally substituted with one or more groups selected from halogen, hydroxyl, amino, cyano, C1-6 alkyl or C1-6 alkoxy. The term carbonyl means that two H atoms at the same substitution position are replaced by the same O atom to form a double bond.

The present invention provides a compound of formula (I) or formula (I)B, and stereoisomers, geometric isomers, tautomers, pharmaceutically acceptable salts, prodrugs, solvates, esters, polymorphs, N-oxides, metabolites or isotopically labeled analogs thereof.

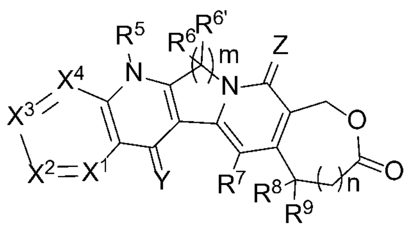

**(I)**

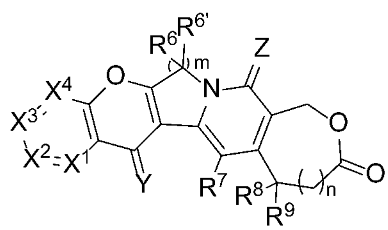

(I)B

wherein:

Y is selected from S, O, $-CR^{10a}R^{10b}$, or $-NR^{10a}$, wherein $R^{10a}$ and $R^{10b}$ are each independently selected from hydrogen, hydroxy, amino, alkyl, alkenyl, alkynyl, cycloalkyl, heterocyclyl, aryl, and heteroaryl;

Z is selected from S, O, $-CR^{11a}R^{11b}$, or $-NR^{11a}$, wherein $R^{11a}$ and $R^{11b}$ are each independently selected from hydrogen, hydroxy, amino, alkyl, alkenyl, alkynyl, cycloalkyl, heterocyclyl, aryl, and heteroaryl;

n is 0, 1, 2, or 3; m is 0, 1, 2, or 3;

$X^1$ is selected from N or $C(R^1)$, wherein $R^1$ is selected from hydrogen, deuterium, halogen, hydroxy, mercapto, amino, cyano, nitro, optionally substituted alkyl, optionally substituted alkoxy, optionally substituted cycloalkyl, optionally substituted heterocyclyl, $-OR^{1a}$, $-SR^{1a}$, $-NR^{1a}R^{1b}$, $-COR^{1a}$, $-SOR^{1a}$, $-SO_2R^{1a}$, $-COOR^{1a}$, $-CONR^{1a}R^{1b}$, $-NR^{1a}COR^{1b}$, $-OCOR^{1a}$, $-SONR^{1a}R^{1b}$, $-SO_2NR^{1a}R^{1b}$, $-NR^{1a}SOR^{1b}$, $-NR^{1a}SO_2R^{16}$, and $-Si(R^{1a})(R^{1b})(R^{1c})$, wherein $R^{1a}$, $R^{1b}$, and $R^{1c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

$X^2$ is selected from N or $C(R^2)$, wherein $R^2$ is selected from hydrogen, deuterium, halogen, hydroxy, mercapto, amino, cyano, nitro, optionally substituted alkyl, optionally substituted alkoxy, optionally substituted cycloalkyl, optionally substituted heterocyclyl, $-OR^{2a}$, $-SR^{2a}$, $-NR^{2a}R^{2b}$, $-COR^{2a}$, $-SOR^{2a}$, $-SO_2R^{2a}$, $-COOR^{2a}$, $-CONR^{2a}R^{2b}$, $-NR^{2a}COR^{2b}$, $-OCOR^{2a}$, $-SONR^{2a}R^{2b}$, $-SO_2NR^{2a}R^{2b}$, $-NR^{2a}SOR^{2b}$, $-NR^{2a}SO_2R^{2b}$, and $-Si(R^{2a})(R^{2b})(R^{2c})$, wherein $R^{2a}$, $R^{2b}$, and $R^{2c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

or $R^1$ and $R^2$, together with the atoms to which they are attached, form an optionally substituted cyclic structure;

$X^3$ is selected from N or $C(R^3)$, wherein $R^3$ is selected from hydrogen, deuterium, halogen, hydroxy, mercapto, amino, cyano, nitro, optionally substituted alkyl, optionally substituted alkoxy, optionally substituted cycloalkyl, optionally substituted heterocyclyl, $-OR^{3a}$, $-SR^{3b}$, $-NR^{3a}R^{3b}$, $-COR^{3a}$, $-SOR^{3a}$, $-SO_2R^{3a}$, $-COOR^{3a}$, $-CONR^{3a}R^{3b}$, $-NR^{3a}$-$COR^{3b}$, $-OCOR^{3a}$, $-SONR^{3a}R^{3b}$, $-SO_2NR^{3a}R^{3b}$, $-NR^{3a}SOR^{3b}$, $-NR^{3a}SO_2R^{3b}$, and $-Si(R^{3a})(R^{3b})(R^{3c})$, wherein $R^{3a}$, $R^{3b}$, and $R^{3c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

or $R^2$ and $R^3$, together with the atoms to which they are attached, form an optionally substituted cyclic structure;

$X^4$ is selected from N or $C(R^4)$, wherein $R^4$ is selected from hydrogen, deuterium, halogen, hydroxy, mercapto, amino, cyano, nitro, optionally substituted alkyl, optionally substituted alkoxy, optionally substituted cycloalkyl, optionally substituted heterocyclyl, $-OR^{4a}$, $-SR^{4a}$, $-NR^{4a}R^{4b}$, $-COR^{4a}$, $-SOR^{4a}$, $-SO_2R^{4a}$, $-COOR^{4a}$, $-CONR^{4a}R^{4b}$, $-NR^{4a}$-

$COR^{4b}$, $-OCOR^{4a}$, $-SONR^{4a}R^{4b}$, $-SO_2NR^{4a}R^{46}$, $-NR^{4a}SOR^{4b}$, $-NR^{4a}SO_2R^{4b}$, and $-Si(R^{4a})(R^{4b})(R^{4c})$, wherein $R^{4a}$, $R^{4b}$, and $R^{4c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

or $R^3$ and $R^4$, together with the atoms to which they are attached, form an optionally substituted cyclic structure;

$R^5$ is selected from hydrogen, deuterium, hydroxy, amino, optionally substituted alkyl, optionally substituted alkoxy, optionally substituted cycloalkyl, optionally substituted heterocyclyl, optionally substituted aryl, optionally substituted heteroaryl, $-OR^{5a}$, $-SR^{5a}$, $-NR^{5a}R^{5b}$, $-COR^{5a}$, $-SOR^{5a}$, $-SO_2R^{5a}$, $-COOR^{5a}$, $-CONR^{5a}R^{5b}$, $-NR^{5a}COR^{5b}$, $- OCOR^{5a}$, $-SONR^{5a}R^{5b}$, $-SO_2NR^{5a}R^{5b}$, $-NR^{5a}SOR^{5b}$, $-NR^{5a}SO_2R^{5b}$, and $-Si(R^{5a})(R^{5b})(R^{5c})$, wherein $R^{5a}$, $R^{5b}$, and $R^{5c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

$R^6$ and $R^{6'}$ are each independently selected from hydrogen, deuterium, hydroxy, amino, optionally substituted alkyl, optionally substituted alkoxy, optionally substituted cycloalkyl, optionally substituted heterocyclyl, $-OR^{6a}$, $- SR^{6a}$, $-NR^{6a}R^{6b}$, $-COR^{6a}$, $-SOR^{6a}$, $-SO_2R^{6a}$, $-COOR^{6a}$, $-CONR^{6a}R^{6b}$, $-NR^{6b}COR^{6a}$, $-OCOR^{6a}$, $-SONR^{6a}R^{6b}$, $- SO_2NR^{6a}R^{6b}$, $-NR^{6a}SOR^{6b}$, $-NR^{6a}SO_2R^{6b}$, and $-Si(R^{6a})(R^{6b})(R^{6c})$, wherein $R^{6a}$, $R^{6b}$, and $R^{6c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

$R^7$ is selected from hydrogen, deuterium, halogen, hydroxy, mercapto, amino, cyano, nitro, optionally substituted alkyl, optionally substituted alkoxy, optionally substituted cycloalkyl, optionally substituted heterocyclyl, $-OR^{7a}$, $-SR^{7a}$, $-NR^{7a}R^{7b}$, $-COR^{7a}$, $-SOR^{7a}$, $-SO_2R^{7a}$, $-COOR^{7a}$, $-CONR^{7a}R^{7b}$, $-NR^{7a}COR^{7b}$, $-OCOR^{7a}$, $-SONR^{7a}R^{7b}$, $- SO_2NR^{7a}R^{7b}$, $-NR^{7a}SOR^{7b}$, $-NR^{7a}SO_2R^{7b}$, and $-Si(R^{7a})(R^{7b})(R^{7c})$, wherein $R^{7a}$, $R^{7b}$, and $R^{7c}$ are each independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl;

$R^8$ is selected from hydrogen, deuterium, halogen, $-OR^{8a}$, $-OCOR^{8a}$, $-SR^{8a}$, $-OR^{8a}-SR^{8b}$, or $-NR^{8a}R^{8b}$, wherein $R^{8a}$ and $R^{8b}$ are each independently selected from hydrogen, deuterium, optionally substituted alkyl, and optionally substituted cycloalkyl;

$R^9$ is selected from $-CH_2R^{9a}$, $-CF_2R^{9a}$, $-CHFR^{9a}$, $-CD_2R^{9a}$, $-CDHR^{9a}$, $-CDFR^{9a}$, $-OR^{9a}$, $-SR^{9a}$, and $-NR^{9a}R^{9b}$, wherein $R^{9a}$ and $R^{9b}$ are each independently selected from hydrogen, deuterium, optionally substituted alkyl, optionally substituted alkenyl, optionally substituted alkynyl, and optionally substituted cycloalkyl.

**$X^{1*}$-$X^4$**

In some embodiments, $X^1$ is $C(R^1)$, $X^2$ is $C(R^2)$, $X^3$ is $C(R^3)$, and $X^4$ is $C(R^4)$.

In some embodiments, $X^1$ is N, $X^2$ is $C(R^2)$, $X^3$ is $C(R^3)$, and $X^4$ is $C(R^4)$.

In some embodiments, $X^1$ is $C(R^1)$, $X^2$ is N, $X^3$ is $C(R^3)$, and $X^4$ is $C(R^4)$.

In some embodiments, $X^1$ is $C(R^1)$, $X^2$ is $C(R^2)$, $X^3$ is N, and $X^4$ is $C(R^4)$.

In some embodiments, $X^1$ is $C(R^1)$, $X^2$ is $C(R^2)$, $X^3$ is $C(R^3)$, and $X^4$ is N.

**Y and Z**

In some embodiments, $R^{10a}$ and $R^{10b}$ are each independently selected from hydrogen, hydroxy, amino, C1-6 alkyl, $C_{2-6}$ alkenyl, $C_{2-6}$ alkynyl, $C_{3-6}$ cycloalkyl, 4-6-membered heterocyclyl, phenyl, and 5-6-membered heteroaryl.

In some embodiments, $R^{11a}$ and $R^{11b}$ are each independently selected from hydrogen, hydroxy, amino, $C_{1-6}$ alkyl, $C_{2-6}$ alkenyl, $C_{2-6}$ alkynyl, $C_{3-6}$ cycloalkyl, 4-6-membered heterocyclyl, phenyl, and 5-6-membered heteroaryl.

In some embodiments, Y is O.

In some embodiments, Z is S or O.

**n**

In some embodiments, n is 0 or 1.

**m**

In some embodiments, m is 1 or 2.

**$R^1$**

In some embodiments, $R^1$ is selected from hydrogen, deuterium, halogen, hydroxy, mercapto, amino, cyano, nitro, optionally substituted $C_{1-6}$ alkyl, optionally substituted $C_{1-6}$ alkoxy, optionally substituted $C_{3-12}$ cycloalkyl, optionally substituted 3-12 membered heterocyclyl, $-OR^{1a}$, $-SR^{1a}$, $-NR^{1a}R^{1b}$, $-COR^{1a}$, $-SOR^{1a}$, $-SO_2R^{1a}$, $-COOR^{1a}$, $-CONR^{1a}R^{1b}$, $-NR^{1a}COR^{1b}$, $-OCOR^{1a}$, $-SONR^{1a}R^{1b}$, $-SO_2NR^{1a}R^{1b}$, $-NR^{1a}SOR^{1b}$, $-NR^{1a}SO_2R^{1b}$, and $-Si(R^{1a})(R^{1b})(R^{1c})$, wherein $R^{1a}$, $R^{1b}$, and $R^{1c}$ are each independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, $R^1$ is selected from unsubstituted alkyl, alkoxy, cycloalkyl, or heterocyclyl; and alkyl, alkoxy, cycloalkyl, or heterocyclyl substituted with one or more substituents independently selected from:

deuterium, -Boc, -Cbz, halogen, hydroxy, mercapto, amino, cyano, nitro, $-(CH_2)_oOR^{1d}$, $-(CH_2)_oSR^{1d}$, $-(CH_2)_oNR^{1d}R^{1e}$, $-(CH_2)_oCOR^{1d}$, $-(CH_2)_oSOR^{1d}$, $-(CH_2)_oSO_2R^{1d}$, $-(CH_2)_oCOOR^{1d}$, $-(CH_2)_oCONR^{1d}R^{1e}$, $-(CH_2)_oNR^{1d}COR^{1e}$, $-(CH_2)_oOCOR^{1d}$, $-(CH_2)_oSONR^{1d}R^{1e}$, $-(CH_2)_oSO_2NR^{1d}R^{1e}$, $-(CH_2)_oNR^{1d}SOR^{1e}$, $-(CH_2)_oNR^{1d}SO_2R^{1e}$, =O, $=N-OR^{1d}$, $=N-NR^{1d}R^{1e}$, $-(CH_2)_oSi(R^{1d})(R^{1e})(R^{1f})$, optionally substituted alkyl, optionally substituted cycloalkyl, optionally substituted heterocyclyl, optionally substituted aryl, and optionally substituted heteroaryl,
wherein o is independently 0, 1, 2, or 3, and $R^{1d}$, $R^{1e}$, and $R^{1f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl.

In some embodiments, $R^1$ is selected from alkyl, alkoxy, cycloalkyl, heterocyclyl, -alkylene-cycloalkyl, or -alkylene-heterocyclyl substituted with one or more substituents independently selected from:

deuterium; -Boc; -Cbz; halogen; hydroxy; mercapto; amino; cyano; nitro;
$-(CH_2)_oOR^{1d}$, $-(CH_2)_oSR^{1d}$, $-(CH_2)_oNR^{1d}R^{1e}$, $-(CH_2)_oCOR^{1d}$, $-(CH_2)_oSOR^{1d}$, $-(CH_2)_oSO_2R^{1d}$, $-(CH_2)_oCOOR^{1d}$, $-(CH_2)_oCONR^{1d}R^{1e}$, $-(CH_2)_oNR^{1d}COR^{1e}$, $-(CH_2)_oOCOR^{1d}$, $-(CH_2)_oSONR^{1d}R^{1e}$, $-(CH_2)_oSO_2NR^{1d}R^{1e}$, $-(CH_2)_oNR^{1d}SOR^{1e}$, $-(CH_2)_oNR^{1d}SO_2R^{1e}$, =O, $=N-OR^{1d}$, $=N-NR^{1d}R^{1e}$, $-(CH_2)_oSi(R^{1d})(R^{1e})(R^{1f})$,
wherein o is independently 0, 1, 2, or 3, and $R^{1d}$, $R^{1e}$, and $R^{1f}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from deuterium, halogen, hydroxy, mercapto, amino, cyano, nitro, carboxy, phenyl, alkyl, alkoxy, cycloalkyl, or heterocyclyl; alkyl, cycloalkyl, heterocyclyl, aryl, or heteroaryl optionally substituted with one or more substituents independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with one or more of deuterium, halogen, hydroxy, mercapto, amino, cyano, nitro, and/or carboxy.

In some embodiments, $R^{1a}$, $R^{1b}$, and $R^{1c}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{1a}$, $R^{1b}$, and $R^{1c}$ are independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, $R^1$ is selected from hydrogen or halogen.

In some embodiments, $R^1$ is selected from hydrogen or fluoro.

**$R^2$**

In some embodiments, $R^2$ is selected from hydrogen, deuterium, halogen, hydroxy, mercapto, amino, cyano, nitro, optionally substituted $C_{1-6}$ alkyl, optionally substituted $C_{1-6}$ alkoxy, optionally substituted $C_{3-12}$ cycloalkyl, optionally substituted 3-12 membered heterocyclyl, $-OR^{2a}$, $-SR^{2a}$, $-NR^{2a}R^{2b}$, $-COR^{2a}$, $-SOR^{2a}$, $-SO_2R^{2a}$, $-COOR^{2a}$, $-CONR^{2a}R^{2b}$,

$-NR^{2a}COR^{2b}$, $-OCOR^{2a}$, $-SONR^{2a}R^{2b}$, $-SO_2NR^{2a}R^{2b}$, $-NR^{2a}SOR^{2b}$, $-NR^{2a}SO_2R^{2b}$, and $-Si(R^{2a})(R^{2b})(R^{2c})$, wherein $R^{2a}$, $R^{2b}$, and $R^{2c}$ are independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, $R^2$ is selected from unsubstituted alkyl, alkoxy, cycloalkyl, or heterocyclyl; and alkyl, alkoxy, cycloalkyl, or heterocyclyl substituted with one or more substituents independently selected from: deuterium, -Boc, -Cbz, halogen, hydroxy, mercapto, amino, cyano, nitro, $-(CH_2)_oOR^{2d}$, $-(CH_2)_oSR^{2d}$, $-(CH_2)_oNR^{2d}R^{2e}$, $-(CH_2)_oCOR^{2d}$, $-(CH_2)_oSOR^{2d}$, $-(CH_2)_oSO_2R^{2d}$, $-(CH_2)_oCOOR^{2d}$, $-(CH_2)_oCONR^{2d}R^{2e}$, $-(CH_2)_oNR^{2d}COR^{2e}$, $-(CH_2)_oOCOR^{2d}$, $-(CH_2)_oSONR^{2d}R^{2e}$, $-(CH_2)_oSO_2NR^{2d}R^{2e}$, $-(CH_2)_oNR^{2d}SOR^{2e}$, $-(CH_2)_oNR^{2d}SO_2R^{2e}$, =O, $=N-OR^{2d}$, $=N-NR^{2d}R^{2e}$, $-(CH_2)_o Si(R^{2d})(R^{2e})(R^{2f})$, optionally substituted alkyl, optionally substituted cycloalkyl, optionally substituted heterocyclyl, optionally substituted aryl, and optionally substituted heteroaryl, wherein o is independently 0, 1, 2, or 3, and $R^{2d}$, $R^{2e}$, and $R^{2f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl.

In some embodiments, $R^2$ is selected from alkyl, alkoxy, cycloalkyl, heterocyclyl, -alkylene-cycloalkyl, or -alkylene-heterocyclyl substituted with one or more substituents independently selected from: deuterium; -Boc; -Cbz; halogen; hydroxy; mercapto; amino; cyano; nitro; $-(CH_2)_oOR^{2d}$, $-(CH_2)_oSR^{2d}$, $-(CH_2)_oNR^{2d}R^{2e}$, $-(CH_2)_oCOR^{2d}$, $-(CH_2)_oSOR^{2d}$, $-(CH_2)_oSO_2R^{2d}$, $-(CH_2)_oCOOR^{2d}$, $-(CH_2)_oCONR^{2d}R^{2e}$, $-(CH_2)_oNR^{2d}COR^{2e}$, $-(CH_2)_oOCOR^{2d}$, $-(CH_2)_oSONR^{2d}R^{2e}$, $-(CH_2)_oSO_2NR^{2d}R^{2e}$, $-(CH_2)_oNR^{2d}SOR^{2e}$, $-(CH_2)_oNR^{2d}SO_2R^{2e}$, =O, $=N-OR^{2d}$, $=N-NR^{2d}R^{2e}$, $-(CH_2)_oSi(R^{2d})(R^{2e})(R^{2f})$, wherein o is independently 0, 1, 2, or 3, and $R^{2d}$, $R^{2e}$, and $R^{2f}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from deuterium, halogen, hydroxy, mercapto, amino, cyano, nitro, carboxy, phenyl, alkyl, alkoxy, cycloalkyl, or heterocyclyl; alkyl, cycloalkyl, heterocyclyl, aryl, or heteroaryl optionally substituted with one or more substituents; independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with one or more of deuterium, halogen, hydroxy, mercapto, amino, cyano, nitro, and/or carboxy.

In some embodiments, $R^{2a}$, $R^{2b}$, and $R^{2c}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{2a}$, $R^{2b}$, and $R^{2c}$ are independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, $R^2$ is selected from hydrogen, amino, halogen, hydroxy, alkoxy, alkyl, or substituted alkyl.

In some embodiments, $R^2$ is selected from hydrogen, amino, fluoro, chloro, hydroxy, methoxy, methyl, ethyl, or hydroxymethyl.

## $R^3$

In some embodiments, $R^3$ is selected from hydrogen, deuterium, halogen, hydroxy, mercapto, amino, cyano, nitro, optionally substituted $C_{1-6}$ alkyl, optionally substituted $C_{1-6}$ alkoxy, optionally substituted $C_{3-12}$ cycloalkyl, optionally substituted 3-12 membered heterocyclyl, $-OR^{3a}$, $-SR^{3a}$, $-NR^{3a}R^{3b}$, $-COR^{3a}$, $-SOR^{3a}$, $-SO_2R^{3a}$, $-COOR^{3a}$, $-CONR^{3a}R^{3b}$, $-NR^{3a}COR^{3b}$, $-OCOR^{3a}$, $-SONR^{3a}R^{3b}$, $-SO_2NR^{3a}R^{3b}$, $-NR^{3a}SOR^{3b}$, $-NR^{3a}SO_2R^{3b}$, and $-Si(R^{3a})(R^{3b})(R^{3c})$, wherein $R^{3a}$, $R^{3b}$, and $R^{3c}$ are independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, $R^3$ is selected from unsubstituted alkyl, alkoxy, cycloalkyl, or heterocyclyl; and alkyl, alkoxy, cycloalkyl, or heterocyclyl substituted with one or more substituents independently selected from: deuterium, -Boc, -Cbz, halogen, hydroxy, mercapto, amino, cyano, nitro, $-(CH_2)_oOR^{3d}$, $-(CH_2)_oSR^{3d}$, $-(CH_2)_oNR^{3d}R^{3e}$, $-(CH_2)_oCOR^{3d}$, $-(CH_2)_oSOR^{3d}$, $-(CH_2)_oSO_2R^{3d}$, $-(CH_2)_oCOOR^{3d}$, $-(CH_2)_oCONR^{3d}R^{3e}$, $-(CH_2)_oNR^{3d}COR^{3e}$, $-(CH_2)_oOCOR^{3d}$, $-(CH_2)_oSONR^{3d}R^{3e}$, $-(CH_2)_oSO_2NR^{3d}R^{3e}$, $-(CH_2)_oNR^{3d}SOR^{3e}$, $-(CH_2)_oNR^{3d}SO_2R^{3e}$, =O, $=N-OR^{3d}$, $=N-NR^{3d}R^{3e}$, $-(CH_2)_o$-

$Si(R^{3d})(R^{3e})(R^{3f})$, optionally substituted alkyl, optionally substituted cycloalkyl, optionally substituted heterocyclyl, optionally substituted aryl, and optionally substituted heteroaryl, wherein o is independently 0, 1, 2, or 3, and $R^{3d}$, $R^{3e}$, and $R^{3f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl.

In some embodiments, $R^3$ is selected from alkyl, alkoxy, cycloalkyl, heterocyclyl, -alkylene-cycloalkyl, or -alkylene-heterocyclyl substituted with one or more substituents independently selected from:

deuterium; -Boc; -Cbz; halogen; hydroxy; mercapto; amino; cyano; nitro;$-(CH_2)_oOR^{3d}$, $-(CH_2)_oSR^{3d}$, $-(CH_2)_oNR^{3d}R^{3e}$, $-(CH_2)_oCOR^{3d}$, $-(CH_2)_oSOR^{3d}$, $-(CH_2)_oSO_2R^{3d}$, $-(CH_2)_oCOOR^{3d}$, $-(CH_2)_oCONR^{3d}R^{3e}$, $-(CH_2)_oNR^{3d}COR^{3e}$, $-(CH_2)_oOCOR^{3d}$, $-(CH_2)_oSONR^{3d}R^{3e}$, $-(CH_2)_oSO_2NR^{3d}R^{3e}$, $-(CH_2)_oNR^{3d}SOR^{3e}$, $-(CH_2)_oNR^{3d}SO_2R^{3e}$, =O, $=N-OR^{3d}$, $=N-NR^{3d}R^{3e}$, $-(CH_2)_oS^i(R^{3d})(R^{3e})(R^{3f})$,wherein o is independently 0, 1, 2, or 3, and $R^{3d}$, $R^{3e}$, and $R^{3f}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from deuterium, halogen, hydroxy, mercapto, amino, cyano, nitro, carboxy, phenyl, alkyl, alkoxy, cycloalkyl, or heterocyclyl;

alkyl, cycloalkyl, heterocyclyl, aryl, or heteroaryl optionally substituted with one or more substituents independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with one or more of deuterium, halogen, hydroxy, mercapto, amino, cyano, nitro, and/or carboxy.

In some embodiments, $R^{3a}$, $R^{3b}$, and $R^{3c}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{3a}$, $R^{3b}$, and $R^{3c}$ are independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, $R^3$ is selected from: hydrogen; halogen; amino; alkyl; alkoxy; cycloalkyl; $-NR^{3a}COR^{3b}$, wherein $R^{3a}$ is selected from hydrogen, alkyl, or cycloalkyl and $R^{3b}$ is selected from alkyl or alkyl substituted with hydroxy or amino; alkyl substituted with halogen, hydroxy, cycloalkyl, and/or heterocyclyl; alkyl substituted with $-(CH_2)_oNR^{3d}R^{3e}$, wherein o is independently 0, 1, 2, or 3, and $R^{3d}$ and $R^{3e}$ are independently selected from hydrogen, - Boc, alkyl, cycloalkyl, halocycloalkyl, and cycloalkyl-substituted alkyl; alkyl substituted with $-(CH_2)_oNR^{3d}COR^{3e}$, wherein o is 0 and $R^{3d}$ and $R^{3e}$ are independently selected from hydrogen, alkyl, cycloalkyl, and hydroxyalkyl; and alkyl substituted with $-(CH_2)_oOCOR^{3d}$, wherein o is 0 and $R^{3d}$ is selected from alkyl or amino-substituted alkyl.

In some embodiments, $R^3$ is selected from: hydrogen; fluoro; chloro; hydroxy; mercapto; amino; methyl; methoxy; cyclopropyl; $-NHCOCH_2NH_2$; $-CH_2N(cyclopropyl)COCH_2OH$; trifluoromethyl; hydroxymethyl; hydroxyethyl (e.g., 1-hydroxyethyl and 2-hydroxyethyl); hydroxypropyl (e.g., 1-hydroxypropyl, 1-hydroxypropan-2-yl, 2-hydroxypropyl, 2-hydroxyisopropyl, and 3-hydroxypropyl); 2,2,2-trifluoro-1-hydroxyethyl; methyl or ethyl substituted with $-NR^{3d}R^{3e}$, wherein $R^{3d}$ and $R^{3e}$ are independently selected from hydrogen, -Boc, methyl, ethyl, n-propyl, isopropyl, cyclopropyl, n-butyl, isobutyl, tert-butyl, cyclobutyl, cyclopentyl, difluorocyclobutyl, and cyclopropylmethyl; methyl substituted with hydroxy and cyclopropyl (e.g., 1-hydroxy-1-cyclopropylmethyl); methyl substituted with pyrrolidinyl; and $-(CH_2)_2O-CO(CH_2)_5NH_2$.

### $R^4$

In some embodiments, $R^4$ is selected from hydrogen, deuterium, halogen, hydroxy, mercapto, amino, cyano, nitro, optionally substituted $C_{1-6}$ alkyl, optionally substituted $C_{1-6}$ alkoxy, optionally substituted $C_{3-12}$ cycloalkyl, optionally substituted 3-12 membered heterocyclyl, $-OR^{4a}$, $-SR^{4a}$, $-NR^{4a}R^{4b}$, $-COR^{4a}$, $-SOR^{4a}$, $-SO_2R^{4a}$, $-COOR^{4a}$, $-CONR^{4a}R^{4b}$, $-NR^{4a}COR^{4b}$, $-OCOR^{4a}$, $-SONR^{4a}R^{4b}$, $-SO_2NR^{4a}R^{4b}$, $-NR^{4a}SOR^{4b}$, $-NR^{4a}SO_2R^{4b}$, and $-Si(R^{4a})(R^{4b})(R^{4c})$, wherein $R^{4a}$, $R^{4b}$, and $R^{4c}$ are independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, $R^4$ is selected from unsubstituted alkyl, alkoxy, cycloalkyl, or heterocyclyl; and alkyl, alkoxy, cycloalkyl, or heterocyclyl substituted with one or more substituents independently selected from: deuterium, -Boc, -Cbz, halogen, hydroxy, mercapto, amino, cyano, nitro, $-(CH_2)_oOR^{4d}$, $-(CH_2)_oSR^{4d}$, $-(CH_2)_oNR^{4d}R^{4e}$, $-(CH_2)_oCOR^{4d}$, $-(CH_2)_oSOR^{4d}$, $-(CH_2)_oSO_2R^{4d}$, $-(CH_2)_oCOOR^{4d}$, $-(CH_2)_oCONR^{4d}R^{4e}$, $-(CH_2)_oNR^{4d}COR^{4e}$, $-(CH_2)_oOCOR^{4d}$, $-(CH_2)_o$-$SONR^{4d}R^{4e}$, $-(CH_2)_oSO_2NR^{4d}R^{4e}$, $-(CH_2)_oNR^{4d}SOR^{4e}$, $-(CH_2)_oNR^{4d}SO_2R^{4e}$, =O, $=N-OR^{4d}$, $=N-NR^{4d}R^{4e}$, $-(CH_2)_o$-$Si(R^{4d})(R^{4e})(R^{4f})$, optionally substituted alkyl, optionally substituted cycloalkyl, optionally substituted heterocyclyl, optionally substituted aryl, and optionally substituted heteroaryl, wherein o is independently 0, 1, 2, or 3, and $R^{4d}$, $R^{4e}$, and $R^{4f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl.

In some embodiments, $R^4$ is selected from alkyl, alkoxy, cycloalkyl, heterocyclyl, -alkylene-cycloalkyl, or -alkylene-heterocyclyl substituted with one or more substituents independently selected from: deuterium; -Boc; -Cbz; halogen; hydroxy; mercapto; amino; cyano; nitro; $-(CH_2)_oOR^{4d}$, $-(CH_2)_oSR^{4d}$, $-(CH_2)_oNR^{4d}R^{4e}$, $-(CH_2)_oCOR^{4d}$, $-(CH_2)_o$-$SOR^{4d}$, $-(CH_2)_oSO_2R^{4d}$, $-(CH_2)_oCOOR^{4d}$, $-(CH_2)_oCONR^{4d}R^{4e}$, $-(CH_2)_oNR^{4d}COR^{4e}$, $-(CH_2)_oOCOR^{4d}$, $-(CH_2)_o$-$SONR^{4d}R^{4e}$, $-(CH_2)_oSO_2NR^{4d}R^{4e}$, $-(CH_2)_oNR^{4d}SOR^{4e}$, $-(CH_2)_oNR^{4d}SO_2R^{4e}$, =O, $=N-OR^{4d}$, $=N-NR^{4d}R^{4e}$, $-(CH_2)_o$-$Si(R^{4d})(R^{4e})(R^{4f})$, wherein o is independently 0, 1, 2, or 3, and $R^{4d}$, $R^{4e}$, and $R^{4f}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from deuterium, halogen, hydroxy, mercapto, amino, cyano, nitro, carboxy, phenyl, alkyl, alkoxy, cycloalkyl, or heterocyclyl;

alkyl, cycloalkyl, heterocyclyl, aryl, or heteroaryl optionally substituted with one or more substituents independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with one or more of deuterium, halogen, hydroxy, mercapto, amino, cyano, nitro, and/or carboxy.

In some embodiments, $R^{4a}$, $R^{4b}$, and $R^{4c}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{4a}$, $R^{4b}$, and $R^{4c}$ are independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, $R^4$ is selected from: hydrogen; halogen; hydroxy; mercapto; $-NR^{4a}R^{4b}$; alkyl; alkoxy; alkyl substituted with halogen, cycloalkyl, heterocyclyl, $-OR^{4d}$, $-NR^{4d}R^{4e}$, =O, $=N-OR^{4d}$, and/or $=N-NR^{4d}R^{4e}$; and alkoxy substituted with heterocyclyl, $-OR^{4d}$, $-COOR^{4d}$, and/or $-NR^{4d}R^{4e}$, wherein o is independently 0, 1, 2, or 3, and $R^{4a}$, $R^{4b}$, $R^{4d}$, and $R^{4e}$ are independently selected from hydrogen, -Boc, alkyl, cycloalkyl, cycloalkylalkyl, halocycloalkyl, hydroxycycloalkyl.

In some embodiments, $R^4$ is selected from: hydrogen; fluoro; hydroxy; $-NR^{4a}R^{4b}$, wherein $R^{4a}$ and $R^{4b}$ are independently selected from hydrogen and alkyl (e.g., methyl); mercapto; methyl; ethyl; propyl; methoxy; hydroxymethyl; hydroxyethyl (e.g., 1-hydroxyethyl and 2-hydroxyethyl); 2-hydroxyisopropyl; hydroxy-n-propyl; hydroxy-n-butyl; hydroxy-n-pentyl; methyl or ethyl substituted with tetrahydropyranyl and/or $-NR^{4d}R^{4e}$, wherein $R^{4d}$ and $R^{4e}$ are independently selected from hydrogen, -Boc, methyl, ethyl, n-propyl, isopropyl, cyclopropylmethyl, n-butyl, isobutyl, tert-butyl, cyclopropyl, cyclobutyl, cyclopentyl, difluorocyclobutyl, and hydroxycyclopentyl; methyl substituted with fluoro, hydroxy, amino, ethoxy, cyclopropyl, =O, =N-OH, $=N-OCH_3$, and/or $=N-N(CH_3)_2$, such as 1-hydroxy-1-cyclopropylmethyl; ethyl substituted with fluoro, hydroxy, amino, ethoxy, cyclopropyl, =O, =N-OH, $=N-OCH_3$, and/or $=N-N(CH_3)_2$ (e.g., 1-ethyl or 2-ethyl), such as 1-cyclopropyl-2-hydroxyethyl and 1-(methoxyimino)ethyl; propyl substituted with fluoro, hydroxy, -NH(Boc), $-NHCH_3$, $-N(CH_3)_2$, =O, $=N-OCH_3$, and/or substituents; butyl substituted with ethoxy; methoxy substituted with methoxy and/or carboxy; ethoxy substituted with hydroxy, amino, $-N(CH_3)_2$, and/or morpholinyl.

## $R^5$

In some embodiments, $R^5$ is selected from hydrogen, deuterium, hydroxy, amino, optionally substituted $C_{1-6}$ alkyl, optionally substituted $C_{1-6}$ alkoxy, optionally substituted $C_{3-12}$ cycloalkyl, optionally substituted 3-12 membered heterocyclyl, optionally substituted 5-10 membered aryl, optionally substituted 5-10 membered heteroaryl, $-OR^{5a}$, $-SR^{5a}$, $-NR^{5a}R^{5b}$, $-COR^{5a}$, $-SOR^{5a}$, $-SO_2R^{5a}$, $-COOR^{5a}$, $-CONR^{5a}R^{5b}$, $-NR^{5a}COR^{5b}$, $-OCOR^{5a}$, $-SONR^{5a}R^{5b}$, $-SO_2NR^{5a}R^{5b}$,

$-NR^{5a}SOR^{5b}$, $-NR^{5a}SO_2R^{5b}$, and $-Si(R^{5a})(R^{5b})(R^{5c})$, wherein $R^{5a}$, $R^{5b}$, and $R^{5c}$ are independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, $R^5$ is selected from unsubstituted alkyl, alkoxy, cycloalkyl, heterocyclyl, aryl, or heteroaryl; and alkyl, alkoxy, cycloalkyl, heterocyclyl, aryl, or heteroaryl substituted with one or more substituents independently selected from: deuterium, -Boc, -Cbz, halogen, hydroxy, mercapto, amino, cyano, nitro, $-(CH_2)_oOR^{5d}$, $-(CH_2)_oSR^{5d}$, $-(CH_2)_oNR^{5d}R^{5e}$, $-(CH_2)_oCOR^{5d}$, $-(CH_2)_oSOR^{5d}$, $-(CH_2)_oSO_2R^{5d}$, $-(CH_2)_oCOOR^{5d}$, $-(CH_2)_oCONR^{5d}R^{5e}$, $-(CH_2)_oNR^{5d}COR^{5e}$, $-(CH_2)_oOCOR^{5d}$, $-(CH_2)_oSONR^{5d}R^{5e}$, $-(CH_2)_oSO_2NR^{5d}R^{5e}$, $-(CH_2)_oNR^{5d}SOR^{5e}$, $-(CH_2)_oNR^{5d}SO_2R^{5e}$, =O, $=N-OR^{5d}$, $=N-NR^{5d}R^{5e}$, $-(CH_2)_oSi(R^{5d})(R^{5e})(R^{5f})$, optionally substituted alkyl, optionally substituted cycloalkyl, optionally substituted heterocyclyl, optionally substituted aryl, and optionally substituted heteroaryl, wherein o is independently 0, 1, 2, or 3, and $R^{5d}$, $R^{5e}$, and $R^{5f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocyclyl, optionally substituted aryl, and optionally substituted heteroaryl.

In some embodiments, $R^5$ is selected from alkyl, alkoxy, cycloalkyl, heterocyclyl, aryl, heteroaryl, -alkylene-cycloalkyl, -alkylene-heterocyclyl, -alkylene-aryl, or -alkylene-heteroaryl substituted with one or more substituents independently selected from: deuterium; -Boc; -Cbz; halogen; hydroxy; mercapto; amino; cyano; nitro; $-(CH_2)_oOR^{5d}$, $-(CH_2)_oSR^{5d}$, $-(CH_2)_oNR^{5d}R^{5e}$, $-(CH_2)_oCOR^{5d}$, $-(CH_2)_oSOR^{5d}$, $-(CH_2)_oSO_2R^{5d}$, $-(CH_2)_oCOOR^{5d}$, $-(CH_2)_oCONR^{5d}R^{5e}$, $-(CH_2)_oNR^{5d}COR^{5e}$, $-(CH_2)_oOCOR^{5d}$, $-(CH_2)_oSONR^{5d}R^{5e}$, $-(CH_2)_oSO_2NR^{5d}R^{5e}$, $-(CH_2)_oNR^{5d}SOR^{5e}$, $-(CH_2)_oNR^{5d}SO_2R^{5e}$, =O, $=N-OR^{5d}$, $=N-NR^{5d}R^{5e}$, $-(CH_2)_oSi(R^{5d})(R^{5e})(R^{5f})$, wherein o is independently 0, 1, 2, or 3, and $R^{5d}$, $R^{5e}$, and $R^{5f}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from deuterium, halogen, hydroxy, mercapto, amino, cyano, nitro, carboxy, phenyl, alkyl, alkoxy, cycloalkyl, or heterocyclyl;

alkyl, cycloalkyl, heterocyclyl, aryl, or heteroaryl optionally substituted with one or more substituents independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with one or more of deuterium, halogen, hydroxy, mercapto, amino, cyano, nitro, and/or carboxy.

In some embodiments, $R^{5a}$, $R^{5b}$, and $R^{5c}$ are independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{5a}$, $R^{5b}$, and $R^{5c}$ are independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, $R^5$ is selected from: hydrogen; $-COR^{5d}$, wherein $R^{5a}$ is heterocyclyl; alkyl; cycloalkyl; heterocyclyl; aryl; heteroaryl; alkyl substituted with $-OR^{5d}$, $-NR^{5d}R^{5e}$, $-COR^{5d}$, $-COOR^{5d}$, $-NR^{5d}COR^{5e}$, $-NR^{5d}SO_2R^{5e}$, $=N-OR^{5d}$, heterocyclyl, heteroaryl, and/or heterocyclyl substituted with halogen, acyl, carbonyl, alkyl, cycloalkyl, and/or halocycloalkyl; -alkylene-cycloalkyl unsubstituted or substituted with $-(CH_2)_oOR^{5d}$ and/or $-(CH_2)_oNR^{5d}R^{5e}$; cycloalkyl substituted with halogen, alkyl, heterocyclyl, hydroxyalkyl, $-(CH_2)_oOR^{5d}$, $-(CH_2)_oNR^{5d}R^{5e}$, or $-(CH_2)_oNR^{5d}COR^{5e}$; heterocyclyl substituted with hydroxy, -Boc, -Cbz, $-(CH_2)_oCOR^{5d}$, alkyl, hydroxyalkyl, deuteroalkyl, haloalkyl, cycloalkylalkyl, heterocyclyl, cycloalkyl, and/or cycloalkyl substituted with halogen, hydroxy, alkyl, hydroxyalkyl, and/or haloalkyl; aryl substituted with halogen, $-OR^{5d}$, $-NR^{5d}R^{5e}$, alkyl, haloalkyl, and/or hydroxyalkyl; heteroaryl substituted with alkyl, hydroxyalkyl, cycloalkyl, $-(CH_2)_oNR^{5d}R^{5e}$, $-(CH_2)_oCOOR^{5d}$, and/or $-(CH_2)_oSO_2R^{5d}$; wherein o is independently 0, 1, 2, or 3, and $R^{5d}$ and $R^{5e}$ are independently selected from hydrogen, -Boc, alkyl, cycloalkyl, heterocyclyl, and alkyl or cycloalkyl substituted with deuterium, halogen, hydroxy, alkyl, cycloalkyl, and/or phenyl.

In some embodiments, $R^5$ is selected from:

hydrogen;
$-COR^{5a}$, wherein $R^{5a}$ is morpholinyl;
methyl, ethyl, propyl (e.g., n-propyl, isopropyl), butyl;
hydroxyethyl (e.g., 2-hydroxyethyl), hydroxypropyl (e.g., 2-hydroxyisopropyl, hydroxy-n-propyl), hydroxybutyl (e.g., hydroxy-n-butyl), hydroxypentyl (e.g., hydroxy-n-pentyl);
cyclopropylmethyl, cyclopentylmethyl;

methyl or ethyl substituted with N-methylpyrrolidinyl, N-propylpyrrolidinyl (e.g., N-isopropylpyrrolidinyl), N-cyclobutylpyrrolidinyl, N-difluorocyclobutylpyrrolidinyl, N-acetylpyrrolidinyl, difluoropyrrolidinyl, N-methyldifluoropyrrolidinyl, dimethylpyrrolidinyl, fluoropyrrolidinyl, dimethylpiperidinyl, difluoropiperidinyl, difluoroazetidinyl, 3,3-difluoro-8-azabicyclo[3.2.1]octan-8-yl, tetrahydrofuranyl, morpholinyl, pyrrolidinyl, 5-azaspiro[2.3]hex-5-yl, and/or pyridinyl;

cyclopropylmethyl substituted with $-NR^{5d}R^{5e}$, wherein $R^{5d}$ and $R^{5e}$ are independently selected from hydrogen, methyl, cyclopentyl, difluorocyclobutyl, or cyclopropylmethyl;

cyclopropylbutyl substituted with hydroxy;

methyl, ethyl, propyl, butyl, pentyl, or hexyl substituted with $-OR^{5d}$, $-NR^{5d}R^{5e}$, $-COR^{5d}$, $-COOR^{5d}$, $-NR^{5d}COR^{5e}$, $-NR^{5d}SO_2R^{5e}$, and/or $=N-OR^{5d}$, wherein $R^{5d}$ and $R^{5e}$ are independently selected from hydrogen, -Boc, methyl, ethyl, propyl (e.g., n-propyl and isopropyl), butyl (e.g., isobutyl and tert-butyl), dideuteriomethyl, hydroxymethyl, hydroxyethyl, cyclopropyl, cyclobutyl, cyclopentyl, spiro[2.3]hex-5-yl, bicyclo[1.1.1]pent-2-yl, difluorocyclobutyl, hydroxycyclobutyl, dimethylcyclopentyl, oxetanyl, morpholinyl, tetrahydrofuranyl, cyclopropylmethyl, cyclobutylmethyl, benzyl;

cyclopropyl, cyclobutyl, cyclopentyl, cyclohexyl, spiro[3.3]hept-2-yl, spiro[2.5]oct-6-yl, bicyclo[1.1.1]pent-2-yl;

cyclobutyl, cyclopentyl, or cyclohexyl substituted with fluoro, hydroxy, methyl, hydroxymethyl, hydroxyethyl, $-NHCH_3$, $-N(CH_3)_2$, $-N(CH_3)(Boc)$, $-N(CH_3)(COCH_3)$, $-N(CH_3)(cyclopropyl)$, or $-N(CH_3)(cyclobutyl)$ (e.g., 2,2-difluorocyclobutyl, dimethylcyclobutyl, difluorocyclopentyl, dimethylcyclopentyl, difluorocyclohexyl, dimethylcyclohexyl);

piperidinyl, pyrrolidinyl, oxetanyl, tetrahydrofuranyl, tetrahydropyranyl;

piperidinyl substituted with -Boc, methyl, propyl (e.g., isopropyl), cyclobutyl, cyclopentyl, and/or difluorocyclobutyl;

pyrrolidinyl substituted with one or more substituents selected from hydroxy, -Boc, $-COCH_3$, $-COCH_2OH$, methyl, ethyl, propyl (e.g., n-propyl, isopropyl), butyl (e.g., n-butyl, isobutyl, tert-butyl), pentyl (e.g., n-pentyl, tert-butylmethyl, pentan-3-yl), hydroxyethyl, hydroxypropyl, hydroxybutyl, dideuteriomethyl, trifluoromethyl, trifluoroethyl, cyclopropyl, cyclobutyl, cyclopentyl, cyclohexyl, difluorocyclobutyl, difluorocyclohexyl, dimethylcyclobutyl, hydroxycyclobutyl, cyclobutyl substituted with hydroxymethyl and/or chloromethyl, cyclopropylmethyl, cyclobutylmethyl, azetidinyl, spiro[3.3]hept-2-yl, bicyclo[3.1.0]hex-3-yl, spiro[2.3]hex-5-yl;

5-azaspiro[2.4]hept-7-yl, 5-azaspiro[2.4]hept-7-yl substituted with methyl and/or -Cbz;

phenyl;

phenyl substituted with fluoro, amino, methyl, propyl (e.g., isopropyl), methoxy, trifluoromethyl, and/or hydroxymethyl;

benzo[d][1,3]dioxol-5-yl, benzo[d][1,3]dioxol-5-yl substituted with fluoro;

pyridinyl, pyrazolyl;

pyrazolyl substituted with amino, $-NHCH_3$, methyl, hydroxymethyl, hydroxyethyl, cyclopropyl, $-COOCH_3$, $-SO_2CH_3$, and/or $-CH_2NH(cyclopropyl)$; 4,5,6,7-tetrahydropyrazino[1,5-a]pyrazol-3-yl, 5-methyl-4,5,6,7-tetrahydropyrazino[1,5-a]pyrazol-3-yl.

**$R^6$ and $R^{6'}$**

In some embodiments, $R^6$ and $R^{6'}$ are independently selected from hydrogen, deuterium, hydroxy, amino, optionally substituted $C_{1-6}$ alkyl, optionally substituted $C_{1-6}$ alkoxy, optionally substituted $C_{3-12}$ cycloalkyl, optionally substituted 3-12 membered heterocyclyl, $-OR^{6a}$, $-SR^{6a}$, $-NR^{6a}R^{6b}$, $-COR^{6a}$, $-SOR^{6a}$, $-SO_2R^{6a}$, $-COOR^{6a}$, $-CONR^{6a}R^{6b}$, $-NR^{6b}COR^{6a}$, $-OCOR^{6a}$, $-SONR^{6a}R^{6b}$, $-SO_2NR^{6a}R^{6b}$, $-NR^{6a}SOR^{6b}$, $-NR^{6a}SO_2R^{6b}$, and $-Si(R^{6a})(R^{6b})(R^{6c})$, wherein $R^{6a}$, $R^{6b}$, and $R^{6c}$ are independently selected from hydrogen, deuterium, -Boc, -Cbz, optionally substituted $C_{1-6}$ alkyl, optionally substituted $C_{2-6}$ alkenyl, optionally substituted $C_{2-6}$ alkynyl, optionally substituted $C_{3-12}$ cycloalkyl, optionally substituted 3-12 membered heterocyclyl, optionally substituted 5-10 membered aryl, and optionally substituted 5-10 membered heteroaryl.

In some embodiments, R6 and R6' are each independently selected from alkyl, alkoxy, cycloalkyl, heterocyclyl, -alkylene-cycloalkyl, or -alkylene-heterocyclyl, each substituted with one or more substituents each independently selected from:

deuterium; -Boc; -Cbz; halogen; hydroxy; mercapto; amino; cyano; nitro;

$-(CH_2)_oOR^{6d}$, $-(CH_2)_oSR^{6d}$, $-(CH_2)_oNR^{6d}R^{6e}$, $-(CH_2)_oCOR^{6d}$, $-(CH_2)_oSOR^{6d}$, $-(CH_2)_oSO_2R^{6d}$, $-(CH_2)_oCOOR^{6d}$, $-(CH_2)_oCONR^{6d}R^{6e}$, $-(CH_2)_oNR^{6d}COR^{6e}$, $-(CH_2)_oOCOR^{6d}$, $-(CH_2)_oSONR^{6d}R^{6e}$, $-(CH_2)_oSO_2NR^{6d}R^{6e}$, $-(CH_2)_oNR^{6d}SOR^{6e}$, $-(CH_2)_oNR^{6d}SO_2R^{6e}$, =O, $=N-OR^{6d}$, $=N-NR^{6d}R^{6e}$, $-(CH_2)_oSi(R^{6d})(R^{6e})(R^{6f})$, wherein each o is independently 0, 1, 2, or 3, and $R^{6d}$, $R^{6e}$, and $R^{6f}$ are each independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents each independently selected from deuterium, halogen, hydroxy, mercapto, amino, cyano, nitro, carboxy, phenyl, alkyl, alkoxy, cycloalkyl, or heterocyclyl;

alkyl, cycloalkyl, heterocyclyl, aryl, or heteroaryl optionally substituted with one or more substituents;

each independently selected from deuterium; -Boc; -Cbz; halogen; hydroxy; mercapto; amino; cyano; nitro; carboxy; acyl; carbonyl; alkyl; alkoxy; cycloalkyl; heterocyclyl; and alkyl, alkoxy, cycloalkyl, and heterocyclyl substituted with

one or more of deuterium, halogen, hydroxy, mercapto, amino, cyano, nitro, and/or carboxy.

In some embodiments, $R^{6a}$, $R^{6b}$, and $R^{6c}$ are each independently selected from hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents each independently selected from: deuterium, halogen, hydroxy, mercapto, amino, cyano, nitro, carboxy, phenyl, optionally substituted alkyl, optionally substituted alkoxy, optionally substituted cycloalkyl, and optionally substituted heterocyclyl.

In some embodiments, $R^{6a}$, $R^{6b}$, and $R^{6c}$ are each independently selected from: hydrogen; deuterium; -Boc; -Cbz; unsubstituted alkyl, alkenyl, alkynyl, cycloalkyl, heterocyclyl, aryl, or heteroaryl; and alkyl, alkenyl, alkynyl, cycloalkyl, heterocyclyl, aryl, or heteroaryl substituted with one or more substituents each independently selected from: deuterium; halogen; hydroxy; mercapto; amino; cyano; nitro; carboxy; phenyl; and alkyl, alkoxy, cycloalkyl, or heterocyclyl optionally substituted with one or more substituents selected from deuterium, halogen, hydroxy, mercapto, amino, cyano, nitro, carboxy, alkyl, alkoxy, cycloalkyl, or heterocyclyl.

In some embodiments, R6 and R6' are each independently selected from hydrogen, alkyl, $-(CH_2)_pOR^{6a}$, $-(CH_2)_pSR^{6a}$, $-(CH_2)_pNR^{6a}R^{6b}$, $-(CH_2)_pCOR^{6a}$, wherein p is 0, 1, 2, or 3.

In some embodiments, R6 and R6' are each independently selected from hydrogen and methyl.

### Cyclic Structures Formed by $R^1$ and $R^2$, $R^2$ and $R^3$, $R^3$ and $R^4$

In some embodiments, $R^1$ and $R^2$, $R^2$ and $R^3$, $R^3$ and $R^4$, together with the atoms to which they are attached, form an optionally substituted 5-12 membered cyclic structure.

In some embodiments, the optionally substituted 5-12 membered cyclic structure may be an optionally substituted 5, 6, or 7 membered monocyclic structure, or an optionally substituted 6-12 membered polycyclic (e.g., bicyclic) structure (including spiro, fused, and bridged cyclic structures).

In some embodiments, the cyclic structure is optionally substituted with one or more substituents each independently selected from: deuterium, -Boc, -Cbz, halogen, hydroxy, mercapto, amino, cyano, nitro, optionally substituted alkyl, optionally substituted alkenyl, optionally substituted alkynyl, optionally substituted alkoxy, optionally substituted cycloalkyl, optionally substituted heterocyclyl, optionally substituted aryl, optionally substituted heteroaryl, $-(CH_2)qOR^d$, $-(CH_2)qSR^d$, $-(CH_2)qNR^dR^e$, $-(CH_2)qCOR^d$, $-(CH_2)qSOR^d$, $-(CH_2)qSO_2R^d$, $-(CH_2)qCOOR^d$, $-(CH_2)qCONR^dR^e$, $-(CH_2)qNR^dCOR^e$, $-(CH_2)qOCOR^d$, $-(CH_2)qSONR^dR^e$, $-(CH_2)qSO_2NR^dR^e$, $-(CH_2)qNR^dSOR^e$, $-(CH_2)qNR^dSO_2R^e$, =O, $=N-OR^d$, $=N-NR^dR^e$, $-(CH_2)qSi(R^d)(R^e)(R^f)$, wherein q is 0, 1, 2, or 3, and $R^d$, $R^e$, and $R^f$ may each be the same as any one of $R^{1d}$-$R^{4d}$, $R^{1e}$-$R^{4e}$, and $R^{1f}$-$R^{4f}$, respectively.

The substituents of the cyclic structure may be determined according to the specific $R^1$-$R^4$, as described above for the definitions of $R^1$-$R^4$ (and specific substitutions thereof). For example, in such substituents, the definitions of optionally substituted groups and $R^d$, $R^e$, and $R^f$, or specifically $R^{1d}$-$R^{4d}$, $R^{1e}$-$R^{4e}$, and $R^{1f}$-$R^{4f}$, are as described above for the definitions of $R^1$-$R^4$ (and specific substitutions thereof). Those skilled in the art can determine the ring type, number and type of ring atoms, and substituents of the formed cyclic structure based on the definitions of $R^1$-$R^4$.

In some embodiments, $R^2$-$R^3$, together with the atoms to which they are attached, form an optionally substituted cyclic structure.

In some embodiments, $R^2$ is selected from optionally substituted alkoxy, $R^3$ is selected from optionally substituted alkoxy, and $R^2$ and $R^3$, together with the atoms to which they are attached, form a substituted or unsubstituted 5-12 membered cyclic structure (preferably a 5 or 6 membered cyclic structure).

In some embodiments, $R^2$ is selected from optionally substituted alkoxy, $R^3$ is selected from hydroxy, and $R^2$ and $R^3$, together with the atoms to which they are attached, form a substituted or unsubstituted 5-12 membered cyclic structure (preferably a 5 or 6 membered cyclic structure).

In some embodiments, $R^2$ is selected from alkoxy, $R^3$ is selected from hydroxy, and $R^2$ and $R^3$, together with the atoms to which they are attached, form an unsubstituted or alkyl-substituted 5-12 membered cyclic structure (preferably a 5 or 6 membered cyclic structure). Preferably, the ring atoms of the cyclic structure may contain two oxygen atoms (i.e., the oxygen atoms in the alkoxy and hydroxy groups).

In some embodiments, $R^2$ is selected from methoxy, $R^3$ is selected from hydroxy, and $R^2$ and $R^3$, together with the atoms to which they are attached, form a 5 membered cyclic structure, wherein the ring atoms of the cyclic structure contain two oxygen atoms (i.e., the oxygen atoms in the methoxy and hydroxy groups).

In some embodiments, $R^3$ and $R^4$, together with the atoms to which they are attached, form an optionally substituted cyclic structure.

In some embodiments, $R^3$ is selected from optionally substituted alkyl (e.g., alkyl substituted with halogen, hydroxy, $-NR^{3d}R^{3e}$, cycloalkyl, and/or heterocyclyl), $R^4$ is selected from optionally substituted alkyl (e.g., alkyl substituted with halogen, cycloalkyl, heterocyclyl, $-OR^{4d}$, $-NR^{4d}R^{4e}$, =O, $=N-OR^{4d}$, and/or $=N-NR^{4d}R^{4e}$), and $R^3$ and $R^4$, together with the atoms to which they are attached, form a substituted or unsubstituted 5-12 membered cyclic structure (preferably a 5 or

6 membered cyclic structure). In some embodiments, the cyclic structure may be substituted with one or more substituents selected from: -$R^{3d}$, -$R^{3e}$, -$R^{4d}$, -$R^{4e}$, halogen, hydroxy, -$NR^{3d}R^{3e}$, -$OR^{4d}$, - $NR^{4d}R^{4e}$, =O, =N-$OR^{4d}$, =N-$NR^{4d}R^{4e}$, cycloalkyl, heterocyclyl, haloalkyl, hydroxyalkyl, alkyl substituted with - $NR^{3d}R^{3e}$, alkyl substituted with -$OR^{4d}$, alkyl substituted with -$NR^{4d}R^{4e}$, alkyl substituted with =O, alkyl substituted with =N-$OR^{4d}$, alkyl substituted with =N-$NR^{4d}R^{4e}$, cycloalkylalkyl, heterocyclylalkyl, and the like.

In some embodiments, $R^3$ is selected from hydroxyalkyl, $R^4$ is selected from alkyl (or $R^3$ is selected from alkyl, $R^4$ is selected from hydroxyalkyl), and $R^3$ and $R^4$, together with the atoms to which they are attached, form a 5-12 membered cyclic structure (preferably a 5 or 6 membered cyclic structure) substituted with hydroxy, alkyl, and/or hydroxyalkyl. Preferably, the ring atoms of the cyclic structure do not contain an oxygen atom (i.e., the oxygen atom in the hydroxy group).

In some embodiments, $R^3$ is selected from hydroxyethyl (e.g., 2-hydroxyethyl), $R^4$ is selected from methyl, and $R^3$ and $R^4$, together with the atoms to which they are attached, form a hydroxy-substituted 5-membered cyclic structure, wherein all ring atoms of the cyclic structure are carbon atoms.

**$R^7$**

In some embodiments, $R^7$ is hydrogen.

**$R^8$**

In some embodiments, $R^{8a}$ and $R^{8b}$ are each independently selected from hydrogen, deuterium, unsubstituted alkyl or cycloalkyl, and alkyl or cycloalkyl substituted with one or more substituents each independently selected from: deuterium, halogen, hydroxy, mercapto, amino, cyano, nitro, carboxy, alkyl (e.g., methyl, ethyl, n-propyl, cyclopropyl, isopropyl, n-butyl, t-butyl, isobutyl, sec-butyl), alkenyl (e.g., vinyl, prop-1-enyl, prop-2-enyl), alkynyl (e.g., ethynyl, propynyl), and cycloalkyl (e.g., cyclopropyl, cyclobutyl, cyclopentyl, cyclohexyl).

In some embodiments, $R^{8a}$ and $R^{8b}$ are each independently selected from hydrogen, alkyl, and alkyl substituted with hydroxy, amino, and/or carboxy.

In some embodiments, $R^8$ is selected from -OH, -$OCOCH_3$, -$OCOCH_2CH_3$, -$OCOCH_2CH_2COOH$, -$OCOCH_2CH_2CH_2COOH$, -$OCOCH_2OH$, -$OCH_2OH$, -$OCH_2SCH_3$, -$OCOCH_2CH_2OH$, -$OCOCH_2NH_2$, -$OCOCH_2CH_2NH_2$.

In some embodiments, $R^8$ is selected from -OH, -$OCOCH_2OH$, -$OCOCH_2CH_2COOH$, -$OCOCH_2CH_2CH_2COOH$, -$OCH_2OH$, -$OCOCH_2NH_2$.

In some embodiments, $R^8$ is -OH.

**$R^9$**

In some embodiments, $R^{9a}$ and $R^{9b}$ are each independently selected from hydrogen; deuterium; unsubstituted alkyl, alkenyl, alkynyl, or cycloalkyl; and alkyl, alkenyl, alkynyl, or cycloalkyl substituted with one or more substituents each independently selected from: deuterium, halogen, hydroxy, mercapto, amino, cyano, nitro, carboxy, alkyl (e.g., methyl, ethyl, n-propyl, cyclopropyl, isopropyl, n-butyl, t-butyl, isobutyl, sec-butyl), alkenyl (e.g., vinyl, prop-1-enyl, prop-2-enyl), alkynyl (e.g., ethynyl, propynyl), and cycloalkyl (e.g., cyclopropyl, cyclobutyl, cyclopentyl, cyclohexyl).

In some embodiments, $R^{9a}$ and $R^{9b}$ are each independently selected from alkyl.

In some embodiments, $R^9$ is alkyl.

In some embodiments, $R^9$ is ethyl.

In a preferred embodiment, the compound of Formula (I) is further a compound of Formula (II).

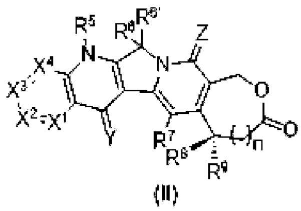

(II)

wherein each substituent in Formula (II) is as defined in Formula (I).

In a preferred embodiment, the compound of Formula (I) is further a compound of Formula (III)a or Formula (III)b:

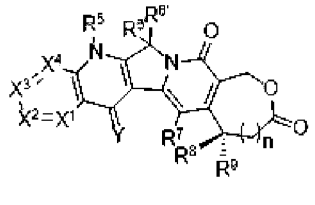

(III)a

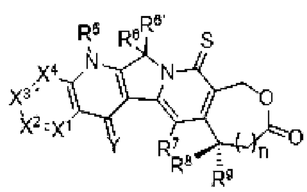

(III)b

wherein each substituent in Formula (III)a or Formula (III)b is as defined in Formula (I).

In a preferred embodiment, the compound of Formula (I) is further a compound of Formula (IV)a, Formula (IV)b, Formula (IV)c, or Formula (IV)d:

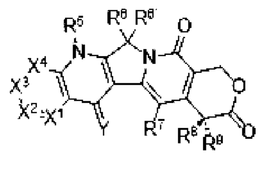

(IV)a

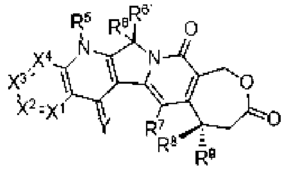

(IV)b

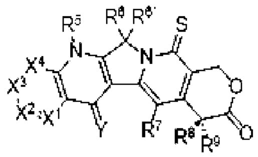

(IV)c

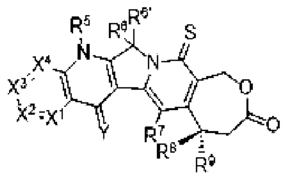

(IV)d

wherein each substituent in Formula (IV)a, Formula (IV)b, Formula (IV)c, or Formula (IV)d is as defined in Formula (I).

In a preferred embodiment, the compound of Formula (I) is further a compound of Formula (V)a, Formula (V)b, Formula (V)c, or Formula (V)d:

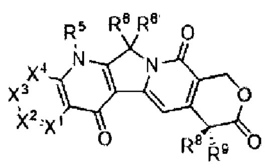

(V)a

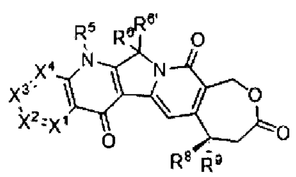

(V)b

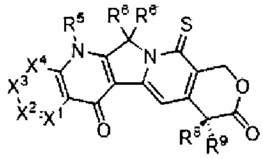

(V)c

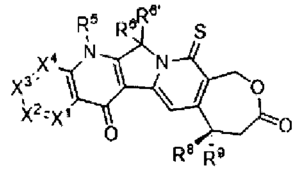

(V)d

wherein each substituent in Formula (V)a, Formula (V)b, Formula (V)c, or Formula (V)d is as defined in Formula (I).

In a preferred embodiment, the compound of Formula (I) is further a compound of Formula (VI)a, Formula (VI)b, Formula (VI)c, or Formula (VI)d:

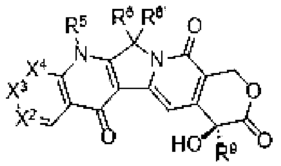

(VI)a

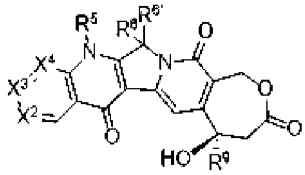

(VI)b

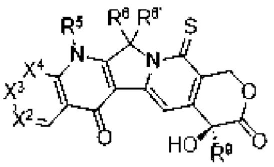

(VI)c

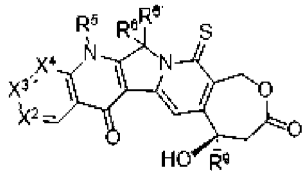

(VI)d

wherein each substituent in Formula (VI)a, Formula (VI)b, Formula (VI)c, or Formula (VI)d is as defined in Formula (I).

In a preferred embodiment, the compound of Formula (I) is further a compound of Formula (VII)a, Formula (VII)b, Formula (VII)c, Formula (VII)d, Formula (VII)a1, Formula (VII)b1, Formula (VII)c1, or Formula (VII)d1:

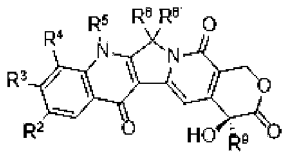

(VII)a

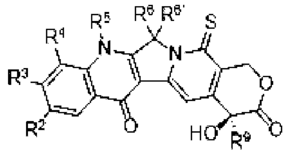

(VII)b

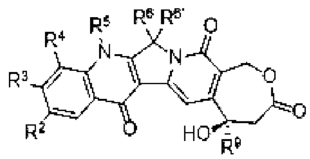

(VII)c

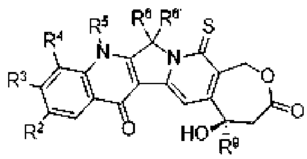

(VII)d

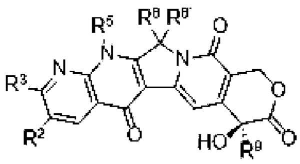

(VII)a1

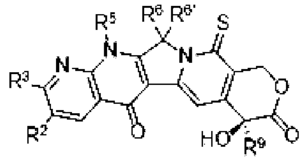

(VII)b1

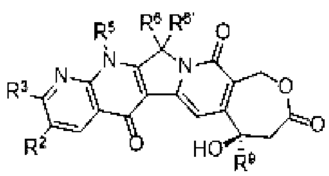

(VII)c1

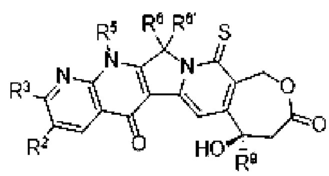

(VII)d1

Wherein the definitions of each substituent in formula (VII)a, formula (VII)b, formula (VII)c, formula (VII)d, formula (VII)a1, formula (VII)b1, formula (VII)c1 or formula (VII)d1 are as described in formula (I).

The compounds of the present invention, and their stereoisomers, geometric isomers, tautomers, pharmaceutically acceptable salts, prodrugs, solvates, esters, polymorphs, N-oxides, metabolites or isotope-labeled analogs are selected from the following compounds:

| Examples | Structure | Examples | Structure |
|---|---|---|---|
| 1 | 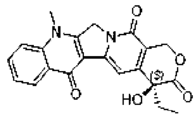 | 2 | 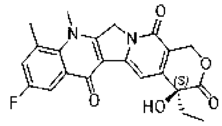 |
| 3 | 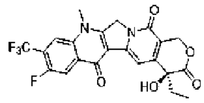 | 4 | 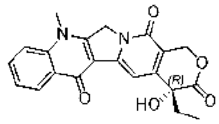 |
| 5 | 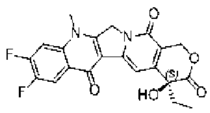 | 6 | 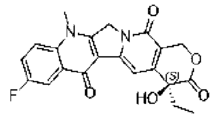 |
| 7 | 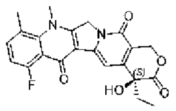 | 8 | 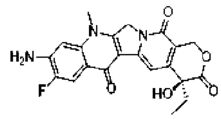 |
| 9 | 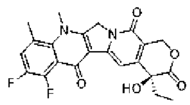 | 10 | 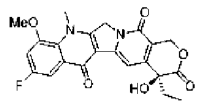 |
| 11 | 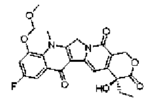 | 12 | 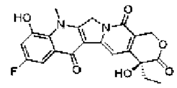 |
| 13 | 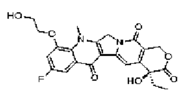 | 14 | 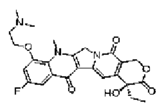 |
| 15 | 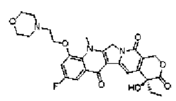 | 16 | 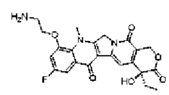 |
| 17 | 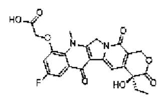 | 18 | 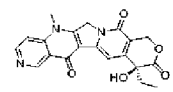 |
| 19 | 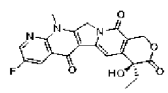 | 20 | 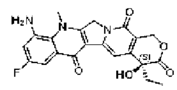 |
| 21 | 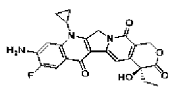 | 22 | 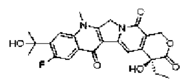 |
| 23 | 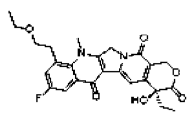 | 24 | 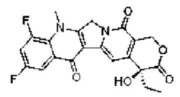 |
| 25 | 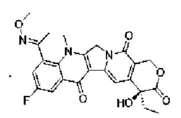 | 26 | 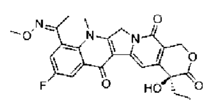 |

(continued)

| Examples | Structure | Examples | Structure |
|---|---|---|---|
| 27 | 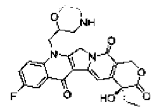 | 28 | 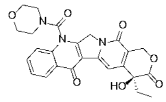 |
| 29 | 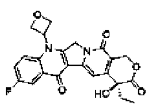 | 30 | 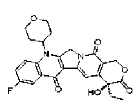 |
| 31 | 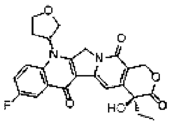 | 32 | 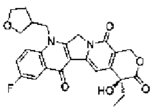 |
| 33 | 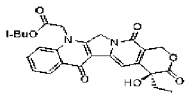 | 34 | 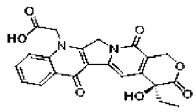 |
| 35 | 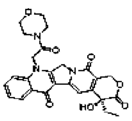 | 36 | 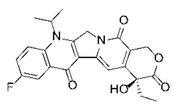 |
| 37 | 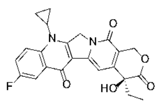 | 38 | 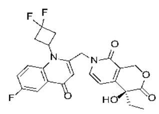 |
| 39 | 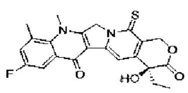 | 40 | 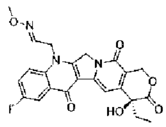 |
| 41 | 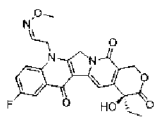 | 42 | 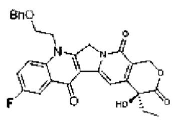 |
| 43 | 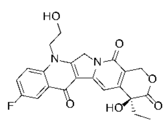 | 44 | 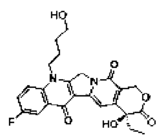 |
| 45 | 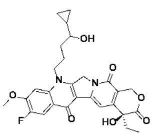 | 46 | 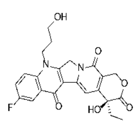 |
| 47 | 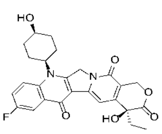 | 48 | 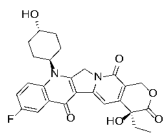 |
| 49 | 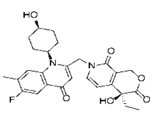 | 50 | 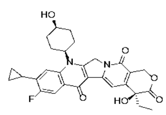 |

(continued)

| Examples | Structure | Examples | Structure |
|---|---|---|---|
| 51 | 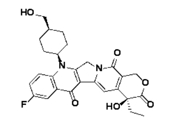 | 52 | 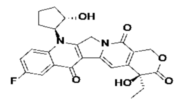 |
| 53 | 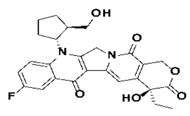 | 54 | 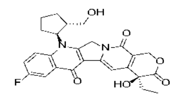 |
| 55 | 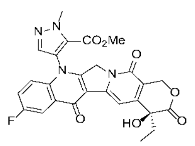 | 56 | 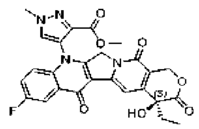 |
| 57 | 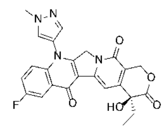 | 58 | 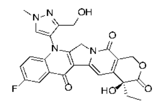 |
| 59 | 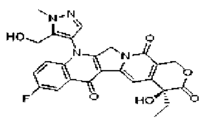 | 60 | 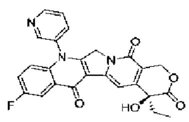 |
| 61 | 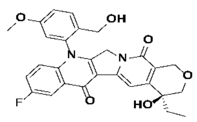 | 62 | 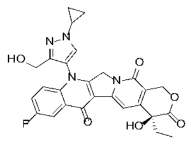 |
| 63 | 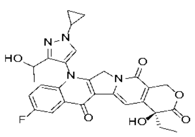 | 64 | 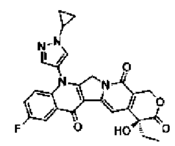 |
| 65 | 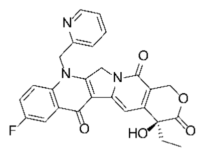 | 66 | 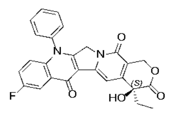 |
| 67 | 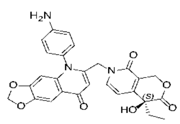 | 68 | 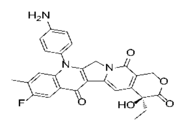 |
| 69 | 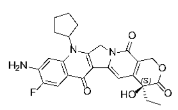 | 70 | 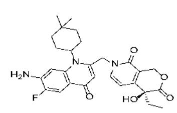 |
| 71 | 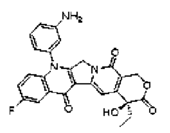 | 72 | 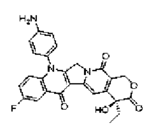 |

(continued)

| Examples | Structure | Examples | Structure |
|---|---|---|---|
| 73 | 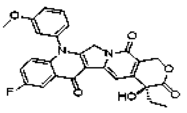 | 74 | 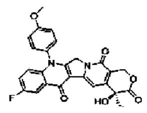 |
| 75 | 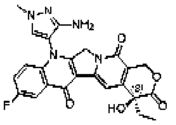 | 76 | 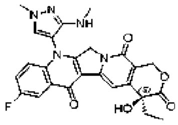 |
| 77 | 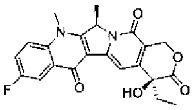 | 78 | 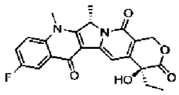 |
| 79 | 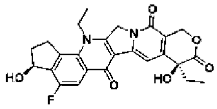 | 80 | 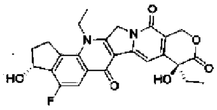 |
| 81 | 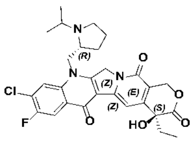 | 82 | 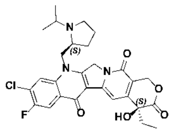 |
| 83 | 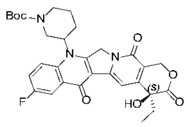 | 84 | 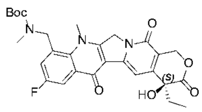 |
| 85 | 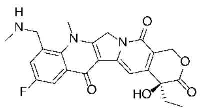 | 86 | 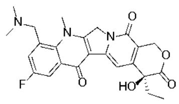 |
| 87 | 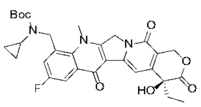 | 88 | 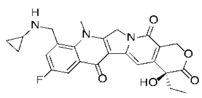 |
| 89 | 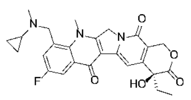 | 90 | 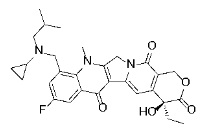 |
| 91 | 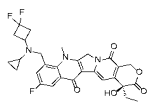 | 92 | 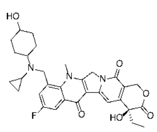 |
| 93 | 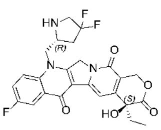 | 94 | 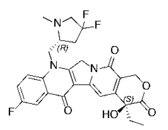 |
| 95 | 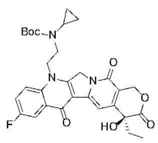 | 96 | 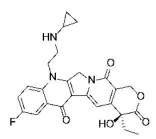 |

(continued)

| Examples | Structure | Examples | Structure |
|---|---|---|---|
| 97 | 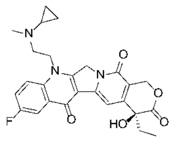 | 98 | 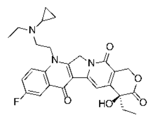 |
| 99 | 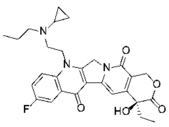 | 100 | 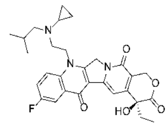 |
| 101 | 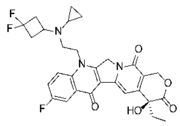 | 102 | 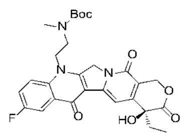 |
| 103 | 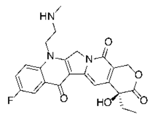 | 104 | 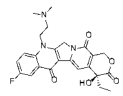 |
| 105 | 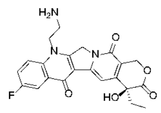 | 106 | 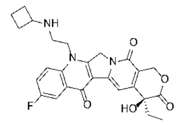 |
| 107 | 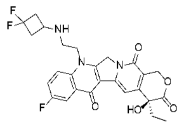 | 108 | 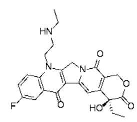 |
| 109 | 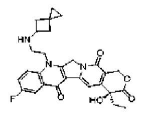 | 110 | 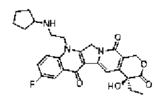 |
| 111 | 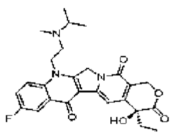 | 112 | 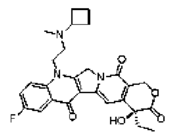 |
| 113 | 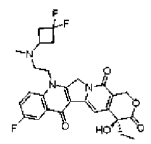 | 114 | 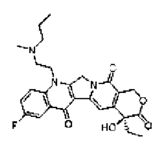 |
| 115 | 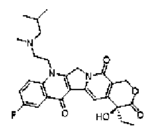 | 116 | 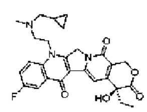 |
| 117 | 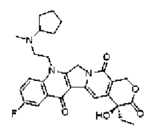 | 118 | 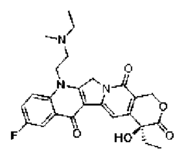 |

(continued)

| Examples | Structure | Examples | Structure |
|---|---|---|---|
| 119 | 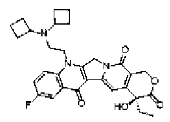 | 120 | 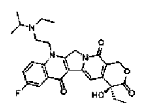 |
| 121 | 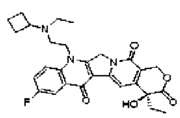 | 122 | 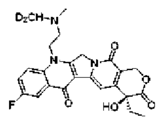 |
| 123 | 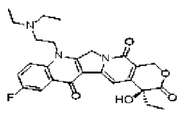 | 124 | 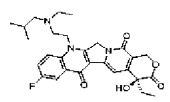 |
| 125 | 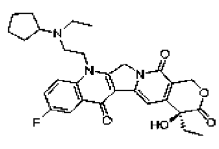 | 126 | 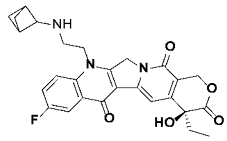 |
| 127 | 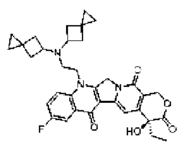 | 128 | 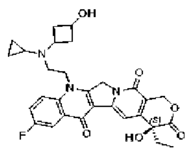 |
| 129 | 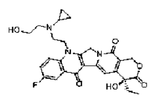 | 130 | 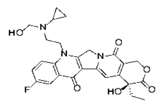 |
| 131 | 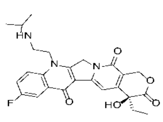 | 132 | 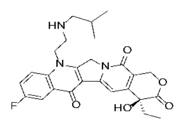 |
| 133 | 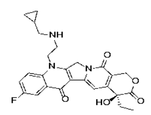 | 134 | 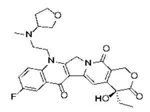 |
| 135 | 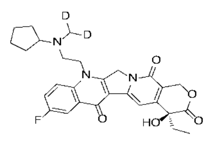 | 136 | 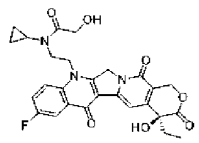 |
| 137 | 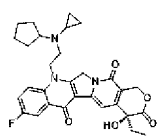 | 138 | 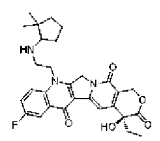 |
| 139 | 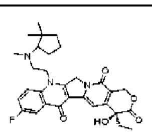 | 140 | 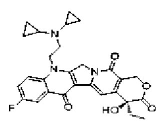 |

(continued)

| Examples | Structure | Examples | Structure |
|---|---|---|---|
| 141 | 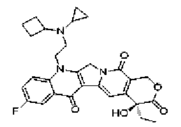 | 142 | 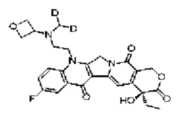 |
| 143 | 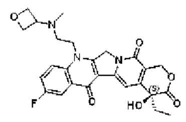 | 144 | 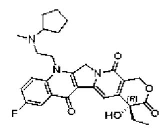 |
| 145 | 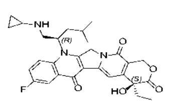 | 146 | 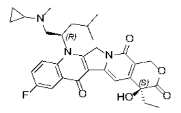 |
| 147 | 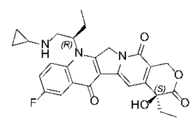 | 148 | 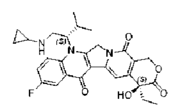 |
| 149 | 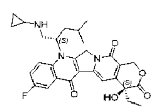 | 150 | 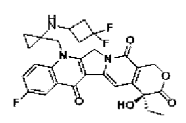 |
| 151 | 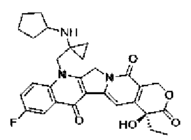 | 152 | 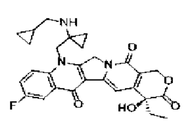 |
| 153 | 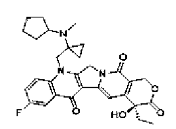 | 154 | 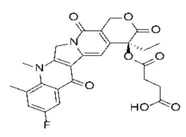 |
| 155 | 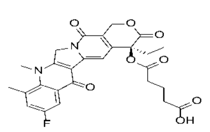 | 156 | 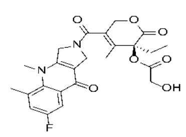 |
| 157 | 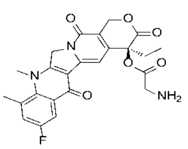 | 158 | 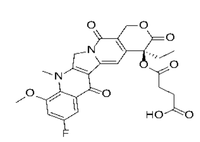 |
| 159 | 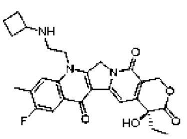 | 160 | 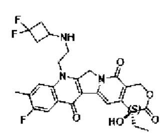 |
| 161 | 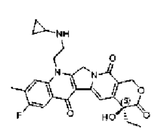 | 162 | 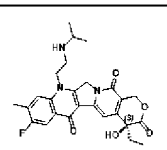 |

(continued)

| Examples | Structure | Examples | Structure |
|---|---|---|---|
| 163 | 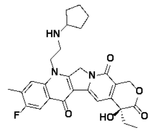 | 164 | 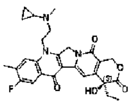 |
| 165 | 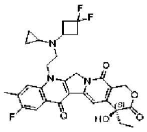 | 166 | 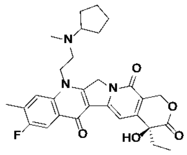 |
| 167 | 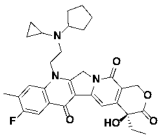 | 168 | 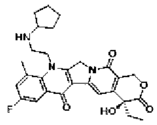 |
| 169 | 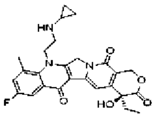 | 170 | 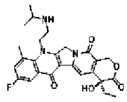 |
| 171 | 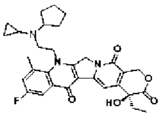 | 172 | 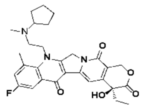 |
| 173 | 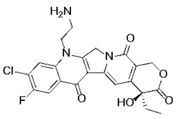 | 174 | 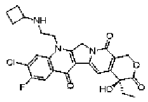 |
| 175 | 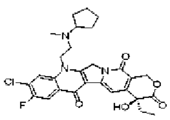 | 176 | 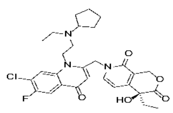 |
| 177 | 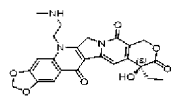 | 178 | 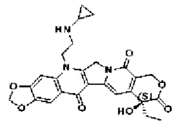 |
| 179 | 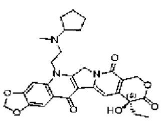 | 180 | 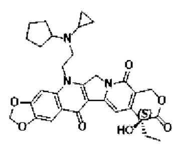 |
| 181 | 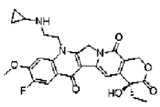 | 182 | 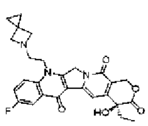 |
| 183 | 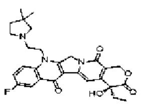 | 184 | 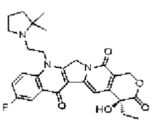 |

(continued)

| Examples | Structure | Examples | Structure |
|---|---|---|---|
| 185 | 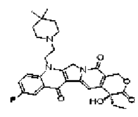 | 186 | 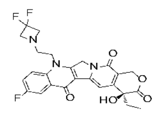 |
| 187 | 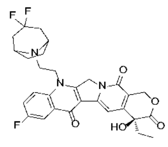 | 188 | 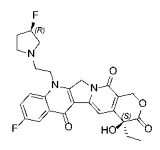 |
| 189 | 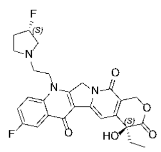 | 190 | 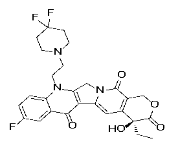 |
| 191 | 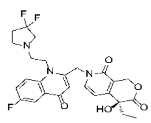 | 192 | 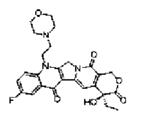 |
| 193 | 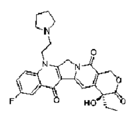 | 194 | 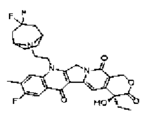 |
| 195 | 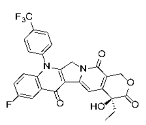 | 196 | 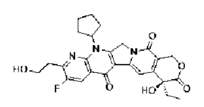 |
| 197 | 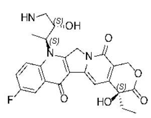 | 198 | 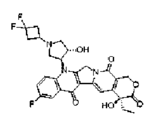 |
| 199 | 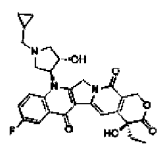 | 200 | 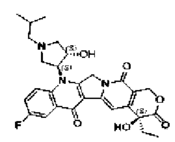 |
| 201 | 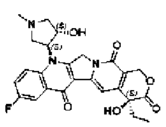 | 202 | 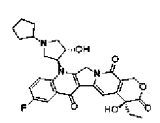 |
| 203 | 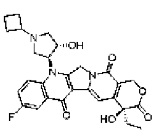 | 204 | 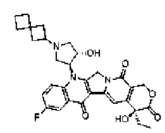 |
| 205 | 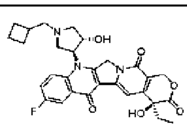 | 206 | 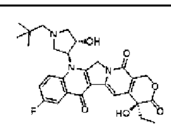 |
| 207 | 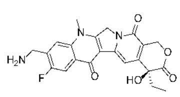 | 208 | 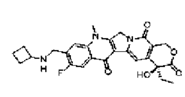 |

(continued)

| Examples | Structure | Examples | Structure |
|---|---|---|---|
| 209 | 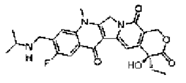 | 210 | 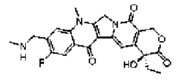 |
| 211 | 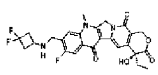 | 212 | 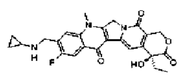 |
| 213 | 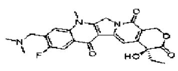 | 214 | 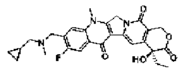 |
| 215 | 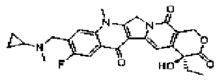 | 216 | 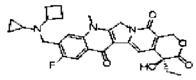 |
| 217 | 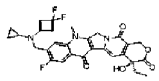 | 218 | 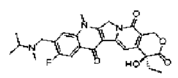 |
| 219 | 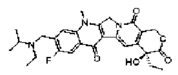 | 220 | 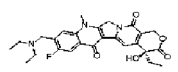 |
| 221 | 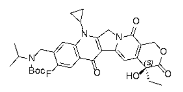 | 222 | 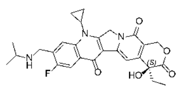 |
| 223 | 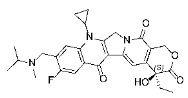 | 224 | 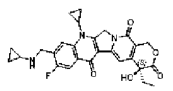 |
| 225 | 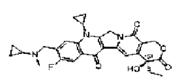 | 226 | 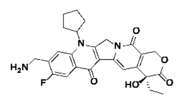 |
| 227 | 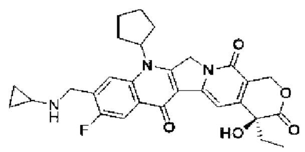 | 228 | 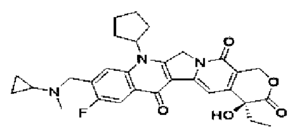 |
| 229 | 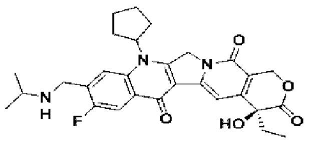 | 230 | 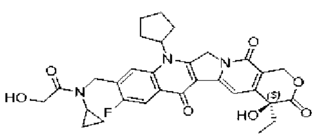 |
| 231 | 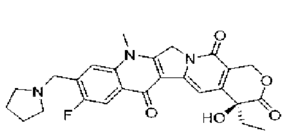 | 232 | 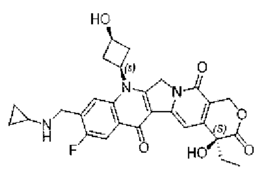 |
| 233 | 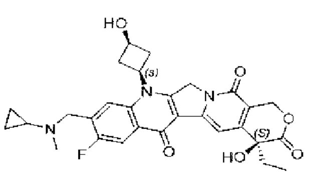 | 234 | 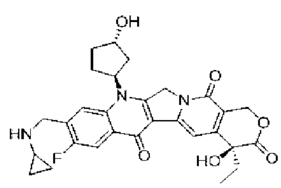 |

(continued)

| Examples | Structure | Examples | Structure |
|---|---|---|---|
| 235 | 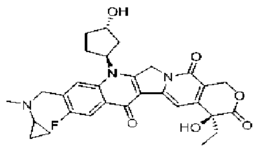 | 236 | 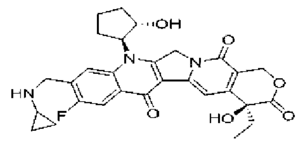 |
| 237 | 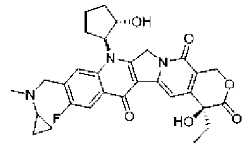 | 238 | 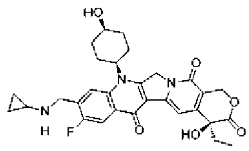 |
| 239 | 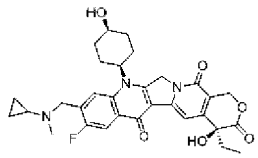 | 240 | 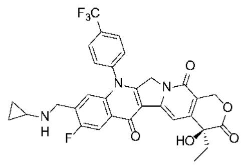 |
| 241 | 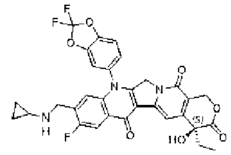 | 242 | 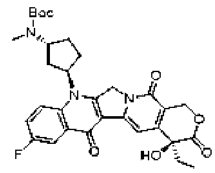 |
| 243 | 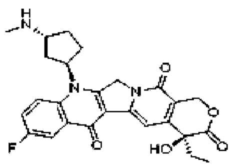 | 244 | 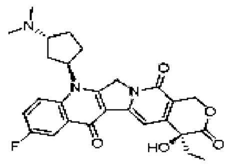 |
| 245 | 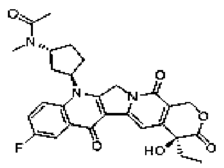 | 246 | 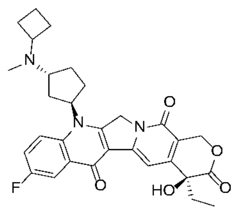 |
| 247 | 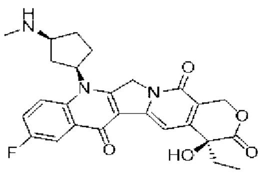 | 248 | 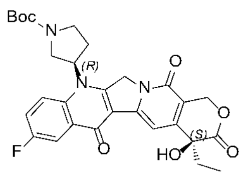 |
| 249 | 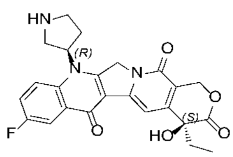 | 250 | 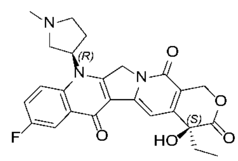 |
| 251 | 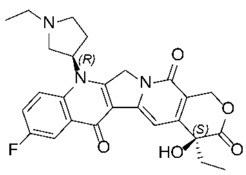 | 252 | 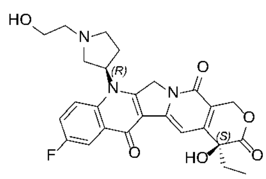 |

(continued)

| Examples | Structure | Examples | Structure |
|---|---|---|---|
| 253 | 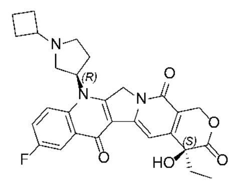 | 254 | 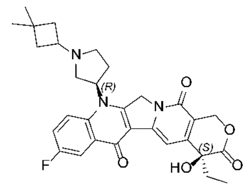 |
| 255 | 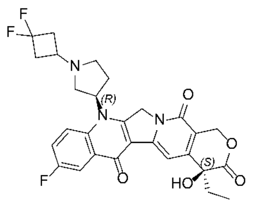 | 256 | 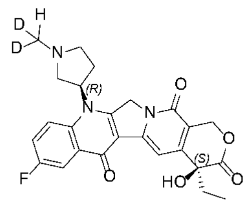 |
| 257 | 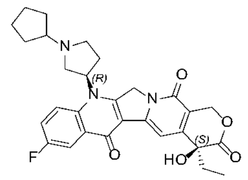 | 258 | 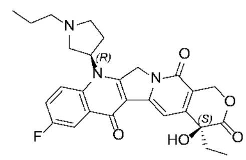 |
| 259 | 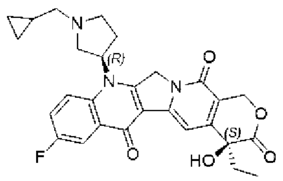 | 260 | 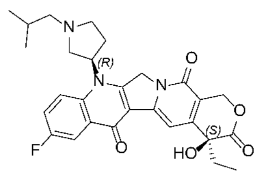 |
| 261 | 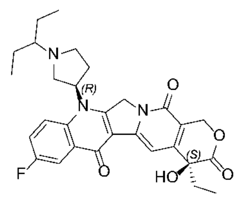 | 262 | 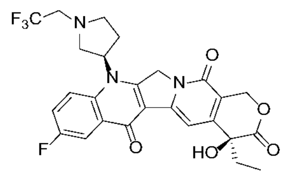 |
| 263 | 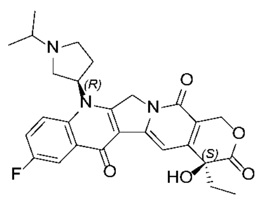 | 264 | 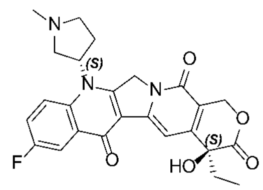 |
| 265 | 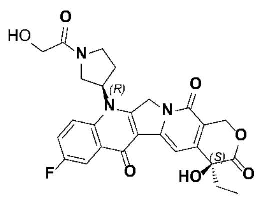 | 266 | 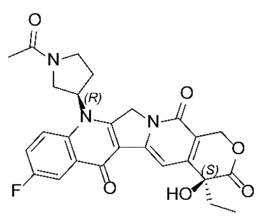 |
| 267 | 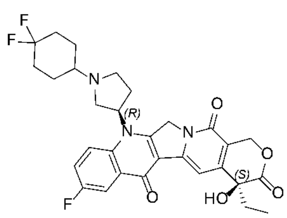 | 268 | 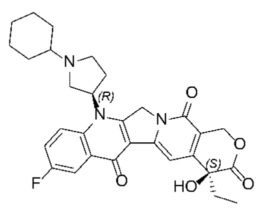 |

(continued)

| Examples | Structure | Examples | Structure |
|---|---|---|---|
| 269 | 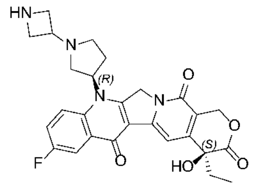 | 270 | 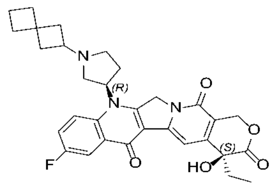 |
| 271 | 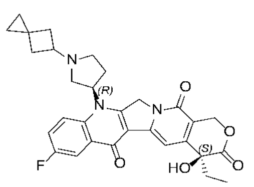 | 272 | 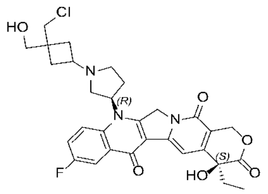 |
| 273 | 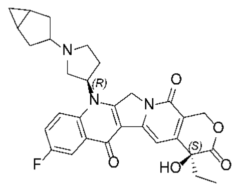 | 274 | 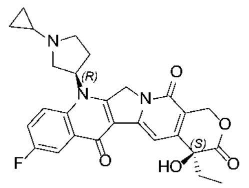 |
| 275 | 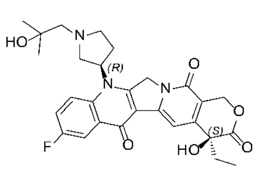 | 276 | 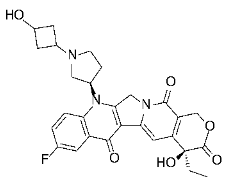 |
| 277 | 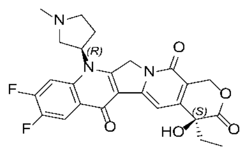 | 278 | 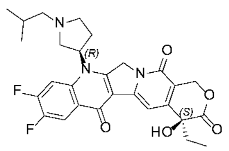 |
| 279 | 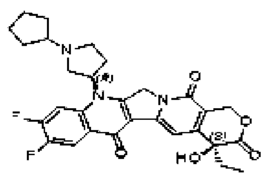 | 280 | 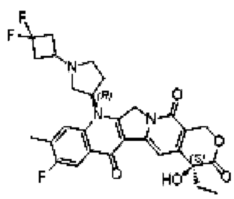 |
| 281 | 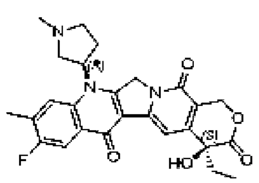 | 282 | 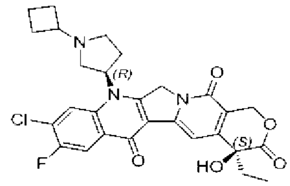 |
| 283 | 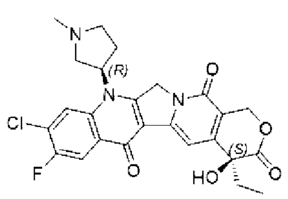 | 284 | 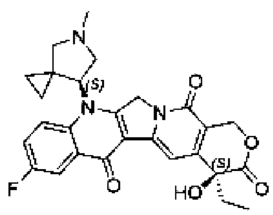 |

(continued)

| Examples | Structure | Examples | Structure |
|---|---|---|---|
| 285 | 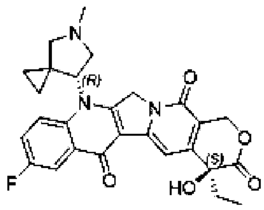 | 286 | 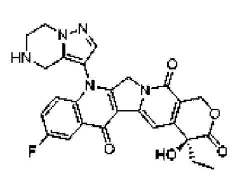 |
| 287 | 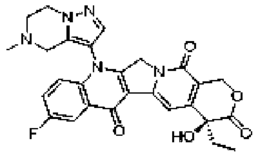 | 288 | 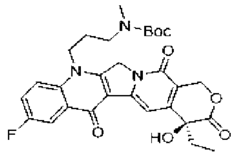 |
| 289 | 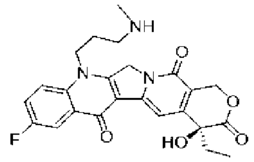 | 290 | 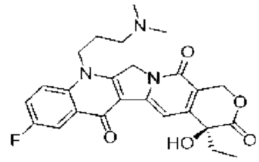 |
| 291 | 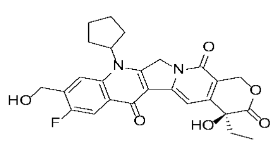 | 292 | 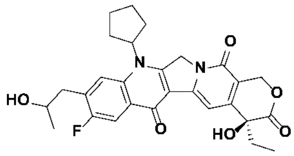 |
| 293 | 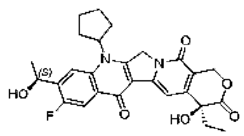 | 294 | 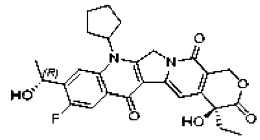 |
| 295 | 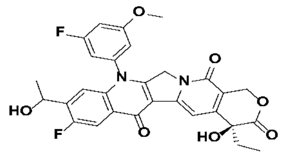 | 296 | 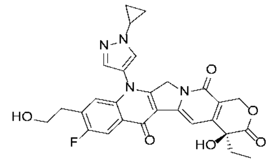 |
| 297 | 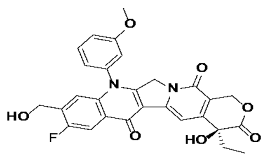 | 298 | 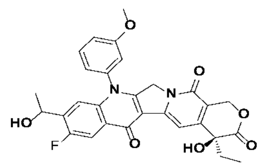 |
| 299 | 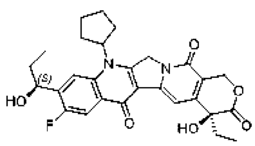 | 300 | 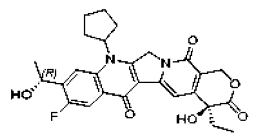 |
| 301 | 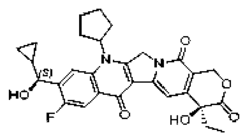 | 302 | 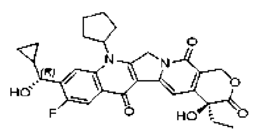 |

(continued)

| Examples | Structure | Examples | Structure |
|---|---|---|---|
| 303 | 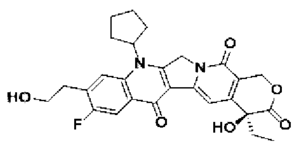 | 304 | 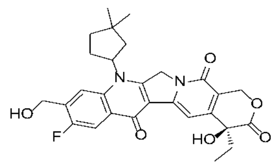 |
| 305 | 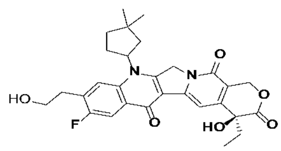 | 306 | 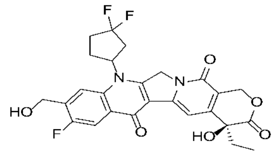 |
| 307 | 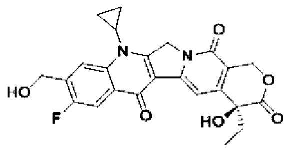 | 308 | 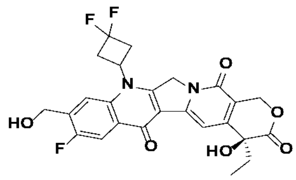 |
| 309 | 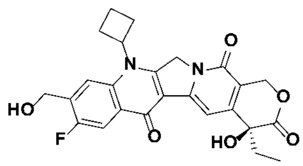 | 310 | 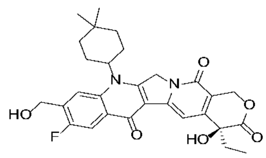 |
| 311 | 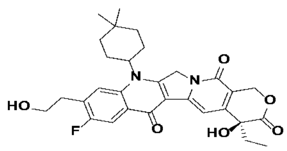 | 312 | 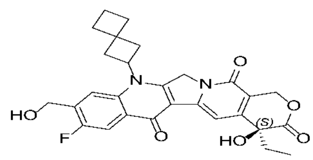 |
| 313 | 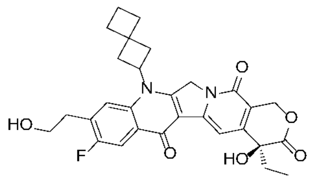 | 314 | 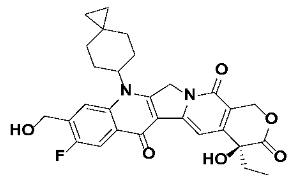 |
| 315 | 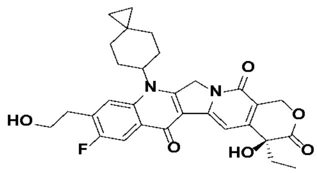 | 316 | 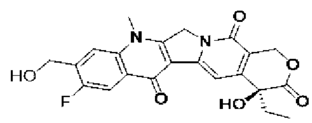 |
| 317 | 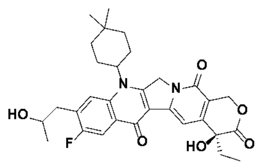 | 318 | 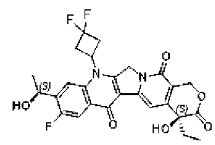 |
| 319 | 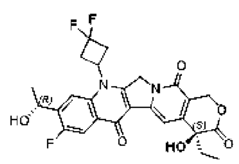 | 320 | 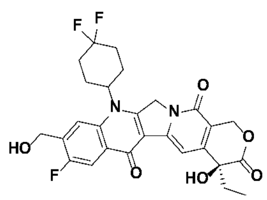 |

(continued)

| Examples | Structure | Examples | Structure |
|---|---|---|---|
| 321 | 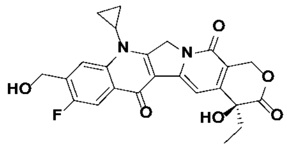 | 322 | 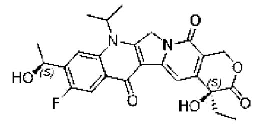 |
| 323 | 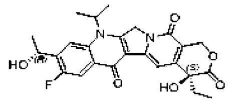 | 324 | 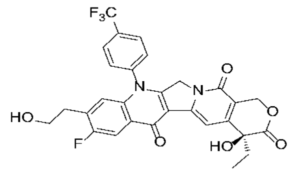 |
| 325 | 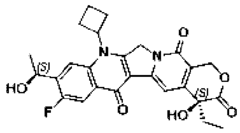 | 326 | 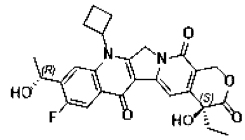 |
| 327 | 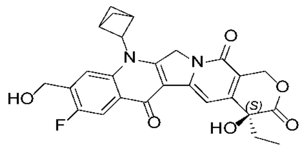 | 328 | 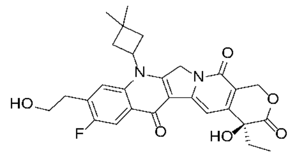 |
| 329 | 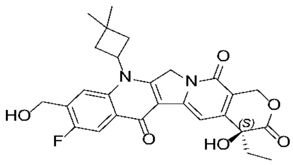 | 330 | 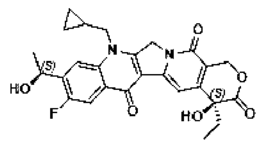 |
| 331 | 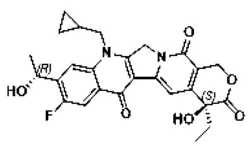 | 332 | 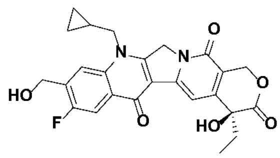 |
| 333 | 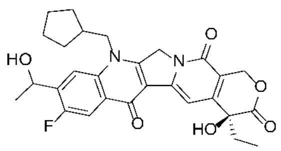 | 334 | 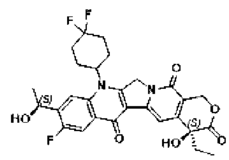 |
| 335 | 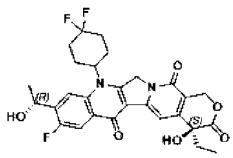 | 336 | 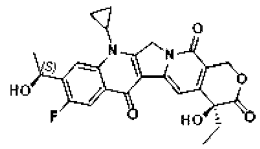 |
| 337 | 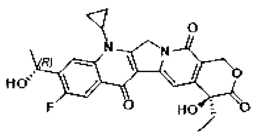 | 338 | 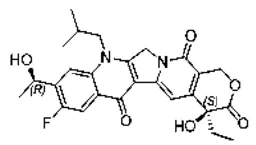 |

(continued)

| Examples | Structure | Examples | Structure |
|---|---|---|---|
| 339 | 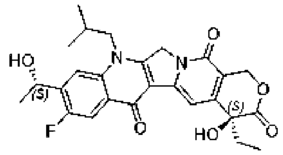 | 340 | 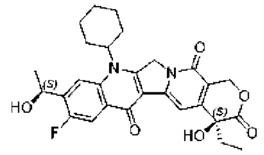 |
| 341 | 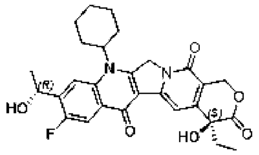 | 342 | 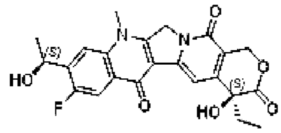 |
| 343 | 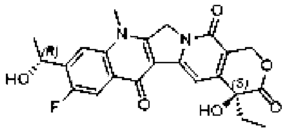 | 344 | 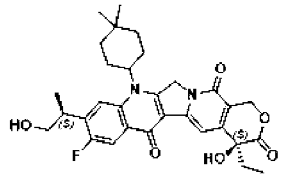 |
| 345 | 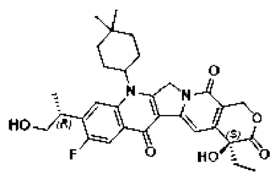 | 346 | 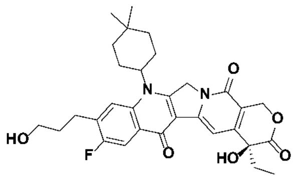 |
| 347 | 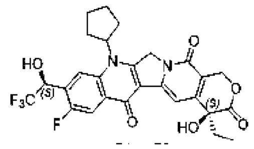 | 348 | 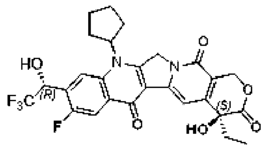 |
| 349 | 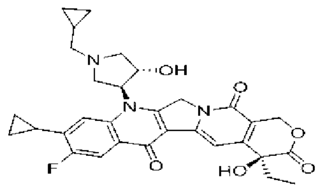 | 350 | 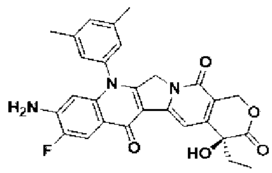 |
| 351 | 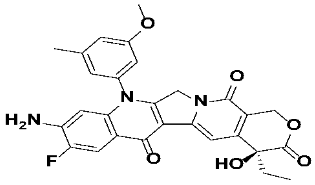 | 352 | 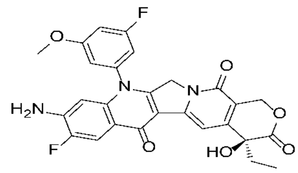 |
| 353 | 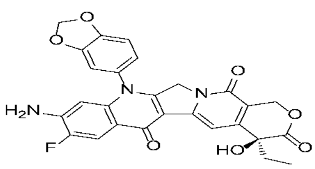 | 354 | 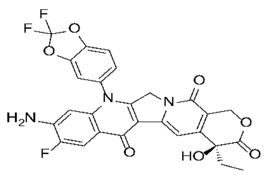 |
| 355 | 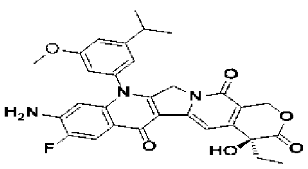 | 356 | 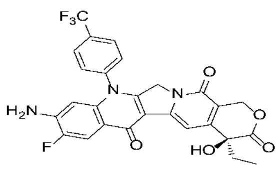 |

(continued)

| Examples | Structure | Examples | Structure |
|---|---|---|---|
| 357 | 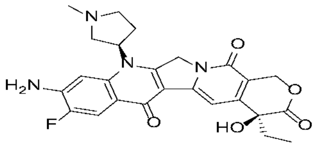 | 358 | 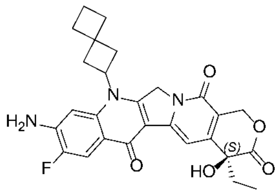 |
| 359 | 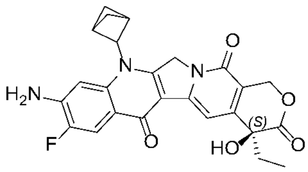 | 360 | 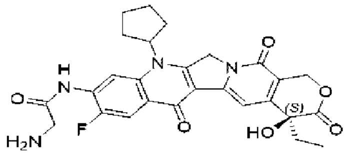 |
| 361 | 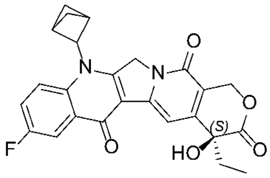 | 362 | 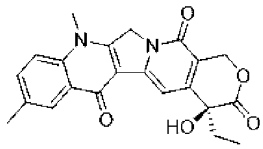 |
| 363 | 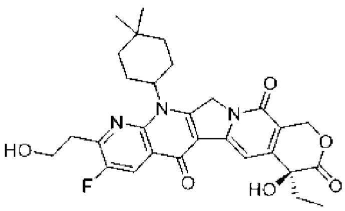 | 364 | 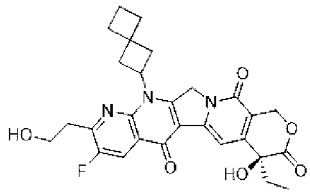 |
| 365 | 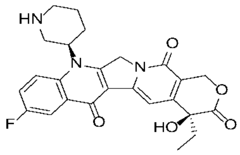 | 366 | 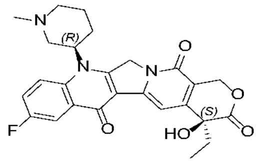 |
| 367 | 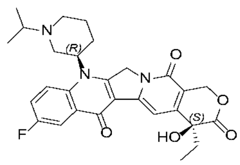 | 368 | 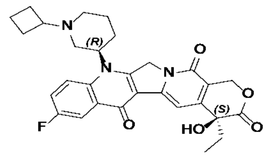 |
| 369 | 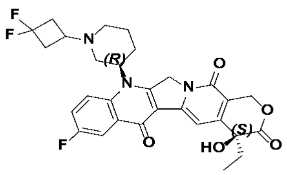 | 370 | 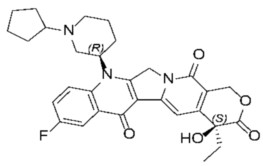 |
| 371 | 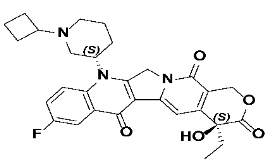 | 372 | 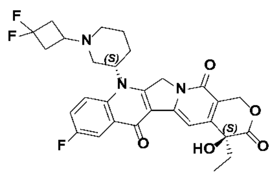 |

(continued)

| Examples | Structure | Examples | Structure |
|---|---|---|---|
| 373 | 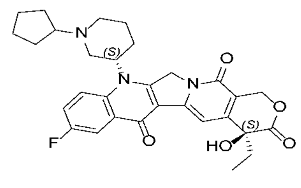 | 374 | 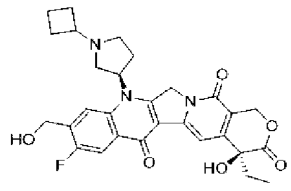 |
| 375 | 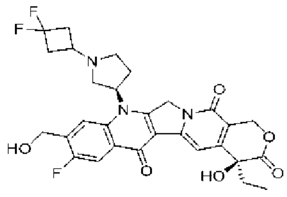 | 376 | 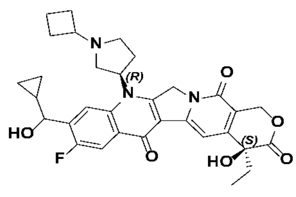 |
| 377 | 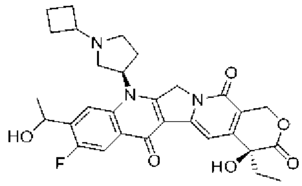 | 378 | 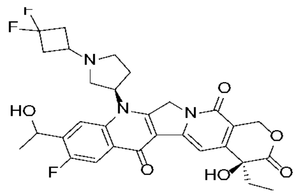 |
| 379 | 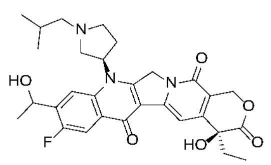 | 380 | 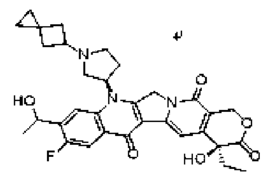 |
| 381 | 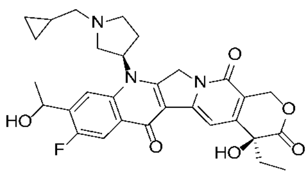 | 382 | 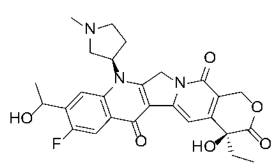 |
| 383 | 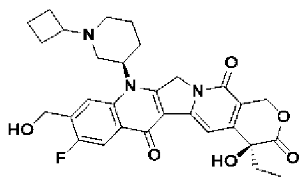 | 384 | 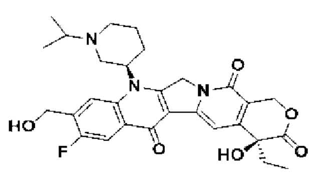 |
| 385 | 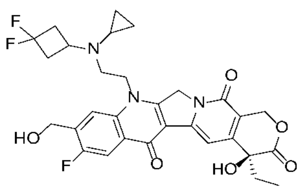 | 386 | 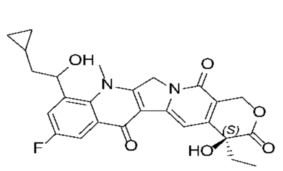 |
| 387 | 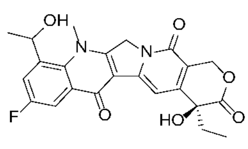 | 388 | 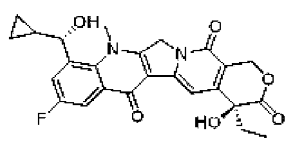 |
| 389 | 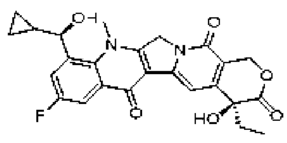 | 390 | 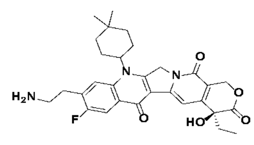 |

(continued)

| Examples | Structure | Examples | Structure |
|---|---|---|---|
| 391 | 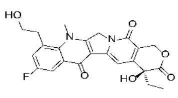 | 392 | 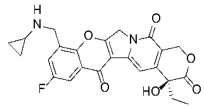 |
| 393 | 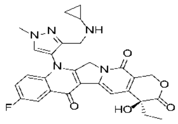 | 394 | 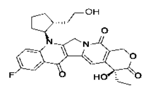 |
| 395 | 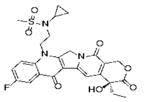 | 396 | 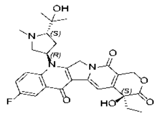 |
| 397 | 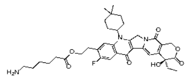 | 398 | 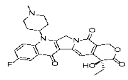 |
| 399 | 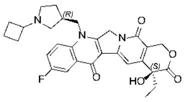 | 400 | 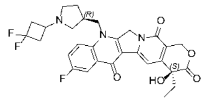 |
| 401 | 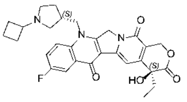 | 402 | 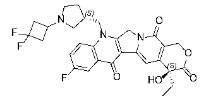 |
| 403 | 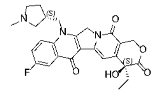 | 404 | 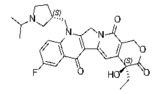 |
| 405 | 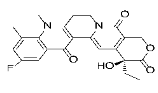 | 406 | 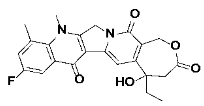 |
| 407 | 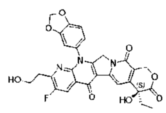 | 408 | 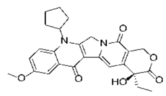 |
| 409 | 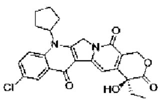 | 410 | 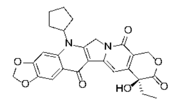 |
| 411 | 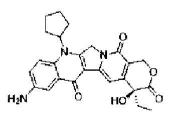 | 412 | 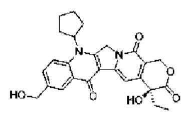 |
| 413 | 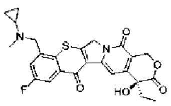 | | |

In one aspect, the present invention provides also includes providing a method for preparing the compound represented by the general formula (I) and its stereoisomers, geometric isomers, tautomers, pharmaceutically acceptable salts, prodrugs, solvates, esters, polymorphs, N-oxides, metabolites or isotope-labeled analogs.

For example, the compound can be prepared by the method shown in Scheme 1. First, connect $LG^a$ (leaving group a) of the right-hand side chain of the structure of formula $(I)_{INT-A}$ with the NH of the structure of formula $(I)_{INT-B}$ to

obtain formula $(I)_{INT}$. Then, intramolecularly connect $LG^b$ (leaving group b) of the right-hand side chain of the structure of formula $(I)_{INT-A}$ with the double bond adjacent to the NH of the structure of formula $(I)_B$, thereby synthesizing the target compound (I).

Scheme 1 is as follows:

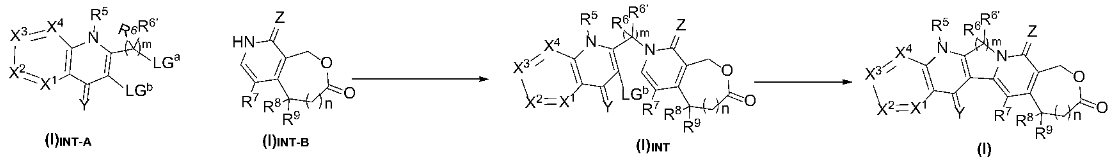

For example, the compound can also be prepared by the method shown in Scheme 2. First, connect $LG^b$ (leaving group b) of the right-hand side chain of the structure of formula $(I)_{INT-A}$ with the double bond adjacent to the NH of the structure of formula $(I)_{INT-B}$. Then, connect $LG^a$ (leaving group a) of the right-hand side chain of the structure of formula $(I)_{INT-A}$ with the NH of the structure of formula $(I)_{INT-B}$ to synthesize the target compound (I). Scheme 2 is as follows:

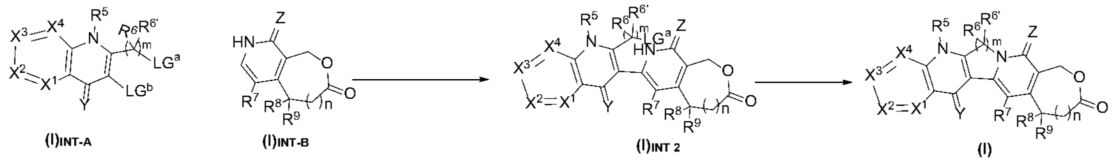

Wherein, in the above - mentioned preparation methods, the definitions of each substituent in the shown compounds are as described previously.

The compound represented by formula $(I)_B$ and its stereoisomers, geometric isomers, tautomers, pharmaceutically acceptable salts, prodrugs, solvates, esters, polymorphs, N-oxides, metabolites or isotope-labeled analogs can be prepared according to similar methods.

## Pharmaceutical Composition

The present invention also provides a pharmaceutical composition, which comprises the compound of the present invention, or its stereoisomers, geometric isomers, tautomers, pharmaceutically acceptable salts, prodrugs, solvates, esters, polymorphs, N-oxides, metabolites or isotope-labeled analogs, as well as pharmaceutically acceptable excipients.

In some embodiments, the pharmaceutical composition of the present invention may further comprise additional medicaments for preventing and/or treating cancer or tumors (such as small molecules, polypeptides, nucleic acids, antibodies, antibody - drug conjugates). When the compound of formula (I) of the present invention and its stereoisomers, geometric isomers, tautomers, pharmaceutically acceptable salts, prodrugs, solvates, esters, polymorphs, N-oxides, metabolites or isotope-labeled analogs are administered in combination with additional medicaments for preventing and/or treating cancer or tumors, the compound of the present invention or its pharmaceutically acceptable salts, etc. can provide enhanced anti-cancer effects.

In some embodiments, representative examples of medicaments for treating cancer or tumors include, but are not limited to: cell signal transduction inhibitors, chlorambucil, melphalan, cyclophosphamide, ifosfamide, busulfan, carmustine, lomustine, streptozocin, cisplatin, carboplatin, oxaliplatin, dacarbazine, temozolomide, procarbazine, methotrexate, fluorouracil, cytarabine, gemcitabine, azvudine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunorubicin, mitoxantrone, bleomycin, mitomycin C, ixabepilone, tamoxifen, flutamide, gonadorelin and analogs, megestrol acetate, prednisone, dexamethasone, methylprednisolone, thalidomide, interferon - $\alpha$, calcium folinate, sirolimus, sirolimus esters, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib, motesanib, neratinib, nilotinib, niraparib, oprozomib, olaparib, pazopanib, pictilisib, ponatinib, quizartinib, regorafenib, rigosertib, rucaparib, ruxolitinib, ceritinib, saridegib, sorafenib, sunitinib, teratinib, tivantinib, tivozanib, tofacitinib, trametinib, vandetanib, veliparib, vemurafenib, vismodegib, volasertib, alemtuzumab, bevacizumab, brentuximab vedotin, catumaxomab, cetuximab, denosumab, gemtuzumab, ipilimumab, nimotuzumab, ofatumumab, panitumumab, rituximab, tositumomab, trastuzumab, PI3K inhibitors, CSF1R inhibitors, A2A and/or A2B receptor antagonists, IDO inhibitors, anti - PD - 1 antibodies, anti - PD - L1 antibodies, LAG3 antibodies, TIM - 3 antibodies, TIGIT antibodies, CD47 antibodies,

CLAUDIN 18.2 antibodies, anti - CTLA - 4 antibodies, or any combination thereof.

**Pharmaceutical Use**

The compounds of the present invention can exhibit a strong effect in inhibiting abnormal cell proliferation.

In one aspect, the present invention provides also includes providing the use of the compound of the present invention or its stereoisomers, geometric isomers, tautomers, pharmaceutically acceptable salts, prodrugs, solvates, esters, polymorphs, N-oxides, metabolites or isotope-labeled analogs, or the pharmaceutical composition of the present invention in the preparation of a medicament for preventing and/or treating diseases related to abnormal cell proliferation.

In one aspect, the present invention provides also includes providing a method for preventing and/or treating diseases related to abnormal cell proliferation, which comprises administering to a patient a therapeutically effective amount of the compound of the present invention or its stereoisomers, geometric isomers, tautomers, pharmaceutically acceptable salts, prodrugs, solvates, esters, polymorphs, N-oxides, metabolites or isotope-labeled analogs, or the pharmaceutical composition of the present invention.

In some embodiments, the disease related to abnormal cell proliferation is a disease mediated by topoisomerase I (Topo I).

In some embodiments, the disease mediated by topoisomerase I (Topo I) is a cancer or tumor - related disease. Representative examples of cancers and tumors include, but are not limited to, skin cancer, bladder cancer, ovarian cancer, breast cancer, gastric cancer, pancreatic cancer, prostate cancer, lung cancer, bone cancer, brain cancer, neuroblastoma, rectal cancer, colon cancer, familial adenomatous polyposis cancer, hereditary non - polyposis colorectal cancer, esophageal cancer, lip cancer, laryngeal cancer, hypopharyngeal cancer, tongue cancer, salivary gland cancer, adenocarcinoma, medullary thyroid cancer, papillary thyroid cancer, kidney cancer, renal parenchymal cancer, cervical cancer, corpus uteri cancer, endometrial cancer, choriocarcinoma, testicular cancer, urinary cancer, melanoma, brain tumors (such as glioblastoma, astrocytoma, meningioma, medulloblastoma and peripheral neuroectodermal tumors), Hodgkin lymphoma, non-Hodgkin lymphoma, Burkitt lymphoma, leukemia, acute lymphoblastic leukemia (ALL), chronic lymphocytic leukemia (CLL), acute myeloid leukemia (AML), chronic myeloid leukemia (CML), adult T-cell leukemia lymphoma, diffuse large B-cell lymphoma (DLBCL), hepatocellular carcinoma, gallbladder cancer, bronchial cancer, small-cell lung cancer, non - small-cell lung cancer, multiple myeloma, basal cell carcinoma, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewings sarcoma and plasmacytoma.

**Combination Therapy**

When the compound of the present invention or its stereoisomers, geometric isomers, tautomers, pharmaceutically acceptable salts, prodrugs, solvates, esters, polymorphs, N-oxides, metabolites or isotope-labeled analogs, or the pharmaceutical composition of the present invention is administered in combination with additional anti-cancer agents or immune checkpoint inhibitors for treating cancer or tumors, the compound of the present invention or its pharmaceutically acceptable salts, etc. can provide enhanced anti-cancer effects.

In some embodiments, representative examples of medicaments for treating cancer or tumors include, but are not limited to: cell signal transduction inhibitors, chlorambucil, melphalan, cyclophosphamide, ifosfamide, busulfan, carmustine, lomustine, streptozocin, cisplatin, carboplatin, oxaliplatin, dacarbazine, temozolomide, procarbazine, methotrexate, fluorouracil, cytarabine, gemcitabine, azvudine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunorubicin, mitoxantrone, bleomycin, mitomycin C, ixabepilone, tamoxifen, flutamide, gonadorelin and analogs, megestrol acetate, prednisone, dexamethasone, methylprednisolone, thalidomide, interferon - $\alpha$, calcium folinate, sirolimus, sirolimus esters, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib, motesanib, neratinib, nilotinib, niraparib, oprozomib, olaparib, pazopanib, pictilisib, ponatinib, quizartinib, regorafenib, rigosertib, rucaparib, ruxolitinib, ceritinib, saridegib, sorafenib, sunitinib, teratinib, tivantinib, tivozanib, tofacitinib, trametinib, vandetanib, veliparib, vemurafenib, vismodegib, volasertib, alemtuzumab, bevacizumab, brentuximab vedotin, catumaxomab, cetuximab, denosumab, gemtuzumab, ipilimumab, nimotuzumab, ofatumumab, panitumumab, rituximab, tositumomab, trastuzumab, PI3K inhibitors, CSF1R inhibitors, A2A and/or A2B receptor antagonists, IDO inhibitors, anti - PD - 1 antibodies, anti - PD - L1 antibodies, LAG3 antibodies, TIM - 3 antibodies, TIGIT antibodies, CD47 antibodies, CLAUDIN 18.2 antibodies, anti - CTLA - 4 antibodies, or any combination thereof.

## Beneficial Effects of the Present Invention

The present invention designs a class of compounds with novel structures, providing a new direction for the development of Topo I inhibitor drugs. In vitro enzyme activity inhibition studies show that these compounds have a strong inhibitory effect on Topo I enzyme. In the subcutaneous xenograft experiment of human colon cancer HCT - 116 cells in female BALB/c nude mice, excellent tumor - suppressing effects were demonstrated. The pharmacokinetic experiment in mice shows that it has excellent metabolic properties. Therefore, it can be used as a promising compound for preventing and/or treating diseases mediated by topoisomerase I (Topo I). In addition, the present invention studies a specific synthesis method, which has a simple process and is convenient to operate, facilitating large - scale industrial production and application.

## Examples

The present invention will be further elaborated with specific examples. It should be understood that these examples are only used to illustrate the invention and are not intended to limit the scope of the invention. The experimental methods without specified conditions in the following examples are generally carried out under conventional conditions or the conditions recommended by the manufacturer. Unless otherwise defined, all professional and scientific terms used herein have the same meanings as those commonly understood by persons skilled in the art. In addition, any methods and materials similar or eq.alent to those described can be applied to the method of the present invention. The preferred implementation methods and materials described herein are for demonstration purposes only.

The structures of the compounds of the invention were determined by nuclear magnetic resonance (NMR) and/or liquid chromatography-mass spectrometry (LC-MS) and/or high-performance liquid chromatography (HPLC). The instrument used for NMR measurement was Bruker AVANCE NEO 400 MHz; the instrument used for LC-MS was Waters Acquity UPLC H-Class Plus and/or SQD2; the instrument used for HPLC was WATERS ACQUITY UPLC and/or Agilent 1260. The starting materials in the examples of the present invention are known and commercially available, or can be synthesized by methods known in the art.

### Examples: Preparation Example 1-Synthesis of Preparation Example 47

### *Step 1: Preparation of methyl 1-ethyl-3-hydroxy-4-methoxy-1,3-dihydrofuro[3,4-c]pyridine-1-carboxylate:*

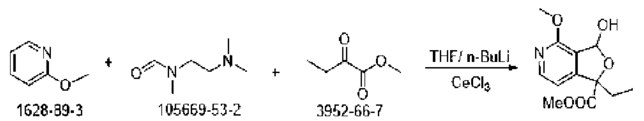


1628-89-3 + 105669-53-2 + 3952-66-7

To a solution of anhydrous THF (15 L, 25 V) and 2-bromomesitylene (1.2 kg, 6.6 mol) in a 50 L dry reactor under a nitrogen atmosphere at -78 °C, 2 mol/L n-BuLi (8.25 L, 16.5 mol) was slowly added. After the addition, the reaction mixture was stirred at -78 °C for 1 hour. Then 2-methoxypyridine (0.6 kg, 5.5 mol) was slowly added, and the mixture was stirred at -78 °C for 2 hours after the addition, followed by warming to 10-15 °C and stirring for another 2 hours. The mixture was then cooled again to -78 °C, and while maintaining the temperature at approximately -78 °C, N-[2-(dimethylamino)ethyl]-N-methylformamide (0.93 kg, 7.15 mol) was slowly added. After the addition, the mixture was stirred at -78 °C for 1 hour, then warmed to -25 to -20 °C, and 2 mol/L n-butyllithium (4.12 L, 8.25 mol) was slowly added while maintaining the temperature at -25 to -20 °C. After the addition, the mixture was stirred at -25 to -20 °C for 2-3 hours.

A mixture of cerium(III) chloride (3.4 kg, 13.75 mol) and tetrahydrofuran (THF, 6 L, 10 V) was slowly added to the reaction system while maintaining the temperature at -25 to -20 °C. After the addition, the mixture was cooled to - 78 °C, and methyl 2-oxobutanoate (1.02 kg, 8.8 mol) was slowly added. After the addition, the mixture was gradually warmed to -25 to -20 °C and stirred for 2-3 hours, with the reaction monitored by thin-layer chromatography (TLC) until completion.

After the reaction was completed, the reaction was quenched by adding glacial acetic acid, and then saturated sodium bicarbonate solution was added to adjust the pH of the system to 8-9, with the reaction temperature kept below 20 °C. After quenching, the mixture was extracted with DCM (DCM, 6 L × 3). The combined organic phases were washed with water and concentrated to obtain the title compound as a yellow solid (1 kg, crude product), which was used directly in the next reaction without further purification. LCMS (ESI): $[M + H]^+ = 254.1$.

*Step 2: Preparation of 4-ethyl-4-hydroxy-8-methoxy-1,4-dihydro-3H-pyrano[3,4-c]pyridin-3-one:*

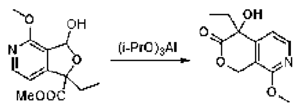


To a 50 L dry reactor were added the intermediate obtained from Step 1 (506g, 2mol), aluminum isopropoxide (1.23kg, 6mol) and isopropanol (12.5L, 25V). Under nitrogen protection, the reaction mixture was heated to reflux and stirred overnight until the reaction was completed. After cooling to room temperature, the reaction was quenched with saturated potassium sodium tartrate solution, then extracted with DCM (DCM, 6 L×3). The combined organic phases were washed with water and concentrated to obtain the title compound as a crude product (380 g), which was directly used in the next step. LCMS (ESI): $[M + H]^+ = 224.1$.

*Step 3: Preparation of 4-ethyl-4-hydroxy-1,7-dihydro-3H-pyrano[3,4-c]pyridine-3,8(4H)-dione:*

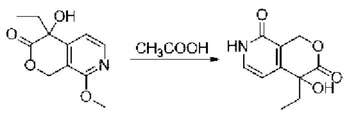


To a 50 L dry reactor were added the intermediate obtained from Step 2 (1.3 kg, 5.82 mol) and acetic acid (6.5 L, 5 V). The mixture was heated to 110 °C for reflux and maintained at this temperature overnight. The reaction mixture was concentrated under reduced pressure, then 2 L of water was added, and the mixture was stirred at room temperature for 0.5-1 hour, filtered, and rinsed with an appropriate amount of water. The obtained filter cake was slurried and purified once with ethanol (2 L), and the wet solid was collected by suction filtration, followed by blast drying at 45-50 °C to obtain the title compound (218 g, HPLC purity: 95%, yield: 18% (three steps)). LCMS (ESI): $[M + H]^+ = 210.1$.

*Step 4: Preparation of (S)-4-ethyl-4-hydroxy-1,7-dihydro-3H-pyrano[3,4-c]pyridine-3,8(4H)-dione (Intermediate 1) and (R)-4-ethyl-4-hydroxy-1,7-dihydro-3H-pyrano[3,4-c]pyridine-3,8(4H)-dione (Intermediate 2):*

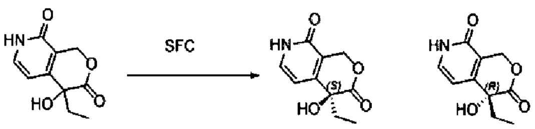


Intermediate 1 Intermediate 2

Racemic 4-ethyl-4-hydroxy-1,7-dihydro-3H-pyrano[3,4-c]pyridine-3,8(4H)-dione (155 g, 741 mmol) was separated by SFC to afford Intermediate 1 (70 g, yield: 45%) and Intermediate 2 (66 g, yield: 38%).

**Intermediate 1:** HPLC purity: 95%, ee: 98%; $^1$H-NMR (400 MHz, $CDCl_3$) δ 11.83 (s, 1H), 7.43 (d, J = 6.8 Hz, 1H), 6.35 (d, J = 6.8 Hz, 1H), 6.25 (s, 1H), 5.22 (s, 2H), 1.77-1.72 (m, 2H), 0.80 (t, J = 7.2 Hz, 3H). **Intermediate 2:** HPLC purity: 95%, ee: 98%; $^1$H-NMR (400 MHz, $CDCl_3$) δ 11.83 (s, 1H), 7.43 (d, J = 6.8 Hz, 1H), 6.35 (d, J = 6.8 Hz, 1H), 6.25 (s, 1H), 5.22 (s, 2H), 1.77-1.72 (m, 2H), 0.80 (t, J = 7.2 Hz, 3H).

**Preparation Example 2: Preparation of (S)-4-ethyl-4-hydroxy-1,7-dihydro-3H-pyrano[3,4-c]pyridine-3,8(4H)-dione (Intermediate 1)**

*Step 1: Preparation of (E)-3-((but-2-en-1-yloxy)methyl)-4-iodo-2-methoxypyridine:*

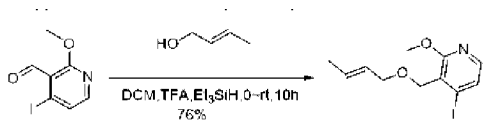


Under a nitrogen atmosphere, to a mixture of 4-iodo-2-methoxypyridine-3-carbaldehyde (15.0 g, 58.0mmol, 1.0 eq.), (E)-but-2-en-1-ol (15.0 mL, 176mmol, 3.0 eq.) and triethylsilane ($Et_3SiH$, 14 mL, 3.0 eq.) was added trifluoroacetic acid (TFA, 35 mL). The reaction mixture was stirred at room temperature overnight. The mixture was poured into saturated sodium bicarbonate solution (200 mL), extracted with petroleum ether (PE, 250mL×3). The combined organic layers were washed with saturated brine (300mL×3), dried over anhydrous sodium sulfate, filtered, concentrated, and the residue was mixed with silica gel. Purification by silica gel column chromatography (eluent gradient: PE/EA= 40:1 to 15:1) afforded the target compound as pale yellow oil(14.5 g, yield: 76%). $^1H$-NMR (400 MHz, $CDCl_3$) δ 7.69 (d, J = 5.2 Hz, 1H), 7.33 (d, J = 5.2 Hz, 1H), 5.79-5.68 (m, 1H), 5.67-5.63 (m, 1H), 4.58 (s, 2H), 4.01 (d, J = 6.0 Hz, 2H), 3.94 (s, 3H), 1.72 (d, J = 6.4 Hz, 3H).

***Step 2: Preparation of 4-ethyl-8-methoxy-1H-pyrano[3,4-c]pyridine:***

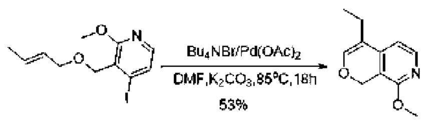


To a 1 L dry reactor were added the intermediate obtained from step 1 (14.6 g, 40.0 mmol, 1.0 eq.), tetrabutylammonium bromide ($Bu_4NBr$, 7.4 g, 23.0 mmol, 0.58 eq.), palladium(II) acetate ($Pd(OAc)_2$, 206 mg, 0.92 mmol, 0.02 eq.), potassium carbonate ($K_2CO_3$, 12.6 g, 92.0 mmol, 2.3 eq.) and N,N-dimethylformamide (DMF, 300 mL). The mixture was heated to 85 °C and stirred for 18 hours. After the reaction was completed, the mixture was cooled to room temperature (24-30 °C), diluted with ethyl acetate (EA, 500 mL), washed with saturated brine (300 mL × 3), dried over anhydrous $Na_2SO_4$, filtered and concentrated to obtain the title compound as a pale yellow oil (4.62 g, yield: 53%). $^1H$-NMR (400 MHz, $CDCl_3$) δ 8.03 (d, J = 5.2 Hz, 1H), 6.64 (d, J = 5.2 Hz, 1H), 6.54 (s, 1H), 5.04 (s, 2H), 3.94 (s, 3H), 2.31-2.28 (m, 2H), 1.12 (t, J = 7.2 Hz, 3H).

*Step 3: Preparation of (S)-4-ethyl-4-hydroxy-8-methoxy-1,4-dihydro-3H-pyrano[3,4-c]pyridin-3-one:*

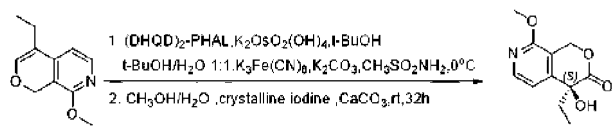


To a solution of $(DHQD)_2PHAL$ (748 mg, 0.96 mmol, 0.1 eq.) in tert-butanol (t-BuOH, 48 mL) were successively added water (48 mL), potassium ferricyanide ($K_3[Fe(CN)_6]$, 9.482 g, 28.8 mmol, 3.0 eq.), potassium carbonate ($K_2CO_3$, 3.053 g, 22.1 mmol, 2.3 eq.), potassium osmate dihydrate ($K_2OsO_2(OH)_4$, 7 mg, 0.02 mmol, 0.002 eq.) and methanesulfonamide (913 mg, 9.6 mmol, 1.0 eq.). The mixture was stirred at 25 °C for 0.5 hours, then cooled to 0 °C to form a red slurry. the intermediate obtained from step 2 (1.84 g, 9.6 mmol, 1.0 eq.) was added, and the thick slurry was vigorously stirred at 0 °C for 48 hours. HPLC monitoring confirmed the reaction was completed. Iodine ($I_2$, 12.18 g, 48.0 mmol, 5.0 eq.) and calcium carbonate ($CaCO_3$, 4.8 g, 48.0 mmol, 5.0 eq.) were added to the reaction solution, and the mixture was stirred for 32 hours. LCMS monitoring confirmed the reaction was complete. The mixture was cooled to 0 °C, and saturated sodium thiosulfate solution (150 mL) was added to quench the reaction. The mixture was filtered, and the filter cake was washed with ethyl acetate/MeOH (EA/MeOH, volume ratio 10:1, 150mL×3). The filtrate was concentrated to obtain the title compound (1.39 g, yield: 65%). $^1H$-NMR (400 MHz, $CDCl_3$) δ 8.20 (d, J = 4.4 Hz, 1H), 7.16 (d, J = 5.6 Hz, 1H), 5.57 (d, J = 16.0 Hz, 1H), 5.26 (d, J = 16.0 Hz, 1H), 3.99 (s, 3H), 3.63 (s, 1H), 3.11 (s, 1H), 1.82-1.77 (m, 2H), 0.95 (t, J = 7.2 Hz, 3H).

*Step 4: Preparation of (S)-4-ethyl-4-hydroxy-1,7-dihydro-3H-pyrano[3,4-c]pyridine-3,8(4H)-dione (Intermediate* 1):

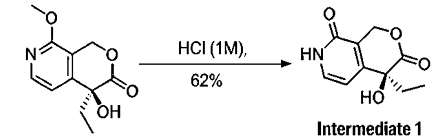


Intermediate 1

To a round-bottom flask (RBF) were added the intermediate obtained from step 3 (470 mg, 2.1 mmol, 1.0 eq.) and 1.0 mol/L hydrochloric acid (HCl, 20 mL). The mixture was heated to reflux overnight. The reaction solution was concentrated, and the residue was purified by silica gel column chromatography (eluent gradient: DCM/MeOH = 30:1 to 10:1) to obtain the title compound as a white solid (270 mg, yield: 62%). $^1$H-NMR (400 MHz, $CDCl_3$) δ 11.83 (s, 1H), 7.43 (d, J = 6.8 Hz, 1H), 6.35 (d, J = 6.8 Hz, 1H), 6.25 (s, 1H), 5.22 (s, 2H), 1.77-1.72 (m, 2H), 0.80 (t, J = 7.2 Hz, 3H).

**Preparation Example 3: Preparation of methyl 6-fluoro-8-methoxy-1-methyl-4-oxo-1,4-dihydroquinoline-2-carboxylate (Intermediate 3)**

*Step 1: Preparation of dimethyl 2-((4-fluoro-2-methoxyphenyl)amino)maleate:*

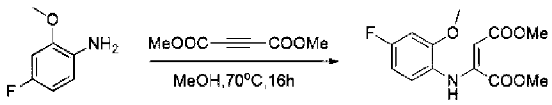


To a solution of 4-fluoro-2-methoxyaniline (20.0 g, 142 mmol, 1.0 eq.) in MeOH (200 mL) was added dimethyl but-2-ynedioate (20.1 g, 142 mmol, 1.0 eq.). The resulting mixture was stirred at 70 °C for 16 hours. The reaction progress was monitored by LC-MS, and upon completion, the reaction mixture was cooled to 0 °C. The formed precipitate was collected by filtration, washed with cold MeOH, and dried under vacuum to afford the title compound as a pale yellow solid (29.5 g, yield: 73.5%). LC-MS (ESI): $[M+H]^+$ = 284.2.

***Step 2: Preparation of 2-((4-fluoro -2- methoxyphenyl) (methyl) amino) dimethyl maleate:***

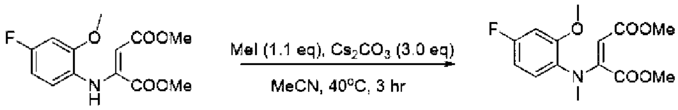


The intermediate obtained from step 1 (18.7 g, 66.0 mmol, 1.0 eq.) was dissolved in anhydrous ACN (200 mL), and $Cs_2CO_3$ (64.6 g, 198 mmol, 3.0 eq.) was then added to the solution. Under a nitrogen atmosphere, methyl iodide (12.2 g, 85.9 mmol, 1.3 eq.) was introduced into the reaction system, and the mixture was maintained at 25 °C for 2 hours to proceed with the reaction. The reaction progress was monitored by liquid chromatography-mass spectrometry (LCMS) until the complete consumption of the starting material was verified. The reaction mixture was filtered, and the filtrate was collected and concentrated under reduced pressure to remove the solvent. The crude residue was purified by silica gel column chromatography (100-200 mesh silica gel) using a gradient elution system of petroleum ether/ethyl acetate (PE/EA) with a volume ratio ranging from 10:1 to 5:1 (v/v).The title compound was obtained as a yellow solid (9.88 g, yield: 50.3%). LCMS (ESI): $[M+H]^+$ =298.1.

*Step 3: Preparation of methyl 6-fluoro-8-methoxy-1-methyl-4-oxo-1,4-dihydroquinoline-2-carboxylate (Intermediate 3)*:

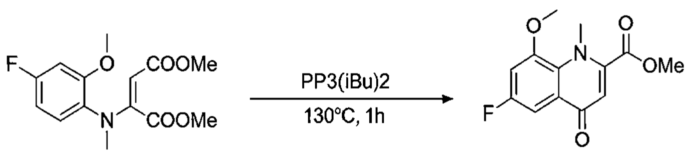


Intermediate 3

A mixture of the intermediate obtained from step 2 (9.0 g, 30.3 mmol, 1.0 eq.) and polyphosphoric acid (72 g, 878 mmol, 29.0 eq.) was heated to 130 °C and stirred for 1 hour. The reaction progress was monitored by LCMS until completion was confirmed. After the reaction mixture was cooled to room temperature, it was poured into ice-cold saturated sodium bicarbonate solution (500 mL). The resulting solid was collected by filtration and further purified via silica gel column chromatography (100-200 mesh silica gel, eluent gradient: PE/EA = 10:1 to 3:1, v/v) to afford the title compound as a yellow solid (4.0 g, yield: 49.8%). LCMS (ESI): $[M + H]^+$ = 266.2.

Preparation Example 4: Preparation of (Z)-1-(4-bromo-2,5-difluorophenyl)-3-(dimethylamino)but-2-en-1-one (Intermediate 4):

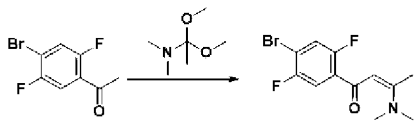

Intermediate 4

1-(4-Bromo-2,5-difluorophenyl)ethanone (25.0 g, 106.37 mmol, 1.0 eq.) was added to anhydrous toluene (200 mL), followed by the addition of 1,1-dimethoxy-N,N-dimethylethylamine, (28.3 g, 212.74 mmol, 2.0 eq.). The reaction mixture was stirred at 90 °C for 12 hours, and the reaction progress was monitored by LCMS. After completion, the reaction solution was concentrated under reduced pressure. The residue was dissolved in EA (300 mL), washed with saturated brine (200 mL×2), dried over anhydrous $MgSO_4$, filtered and concentrated under reduced pressure. The crude product was purified by flash silica gel column chromatography (eluent gradient: PE/EA = 10:1 to 5:1) to afford the title compound as a brown solid (30.2 g, yield: 92.73%). LCMS (ESI): $[M+H]^+$ = 304.2.

Preparation Example 5: Preparation of 7-bromo-1-cyclopropyl-6-fluoro-2-methylquinolin-4(1H)-one (Intermediate 5)

*Step 1: Preparation of (Z)-1-(4-bromo-2,5-difluorophenyl)-3-(cyclopropylamino)but-2-en-1-one:*

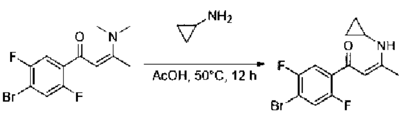


Intermediate 5(15.0 g, 49.32 mmol, 1.0 eq.) was dissolved in acetic acid (200 mL), and cyclopropylamine (5.63 g, 98.64 mmol, 2.0 eq.) was added dropwise to the solution. The reaction mixture was maintained at 50 °C for 12 hours, with the reaction progress monitored by LCMS until complete conversion was achieved. The reaction mixture was then poured into saturated aqueous solution of $NH_4Cl$ (300 mL), and the resulting mixture was extracted with EA (3 × 200 mL). The combined organic layers were dried over anhydrous $MgSO_4$, filtered, and concentrated under reduced pressure. The obtained crude product was purified by flash silica gel column chromatography (eluent gradient: PE/EA = 50:1 to 3:1), yielding the title compound as a yellow solid (11.2 g, yield: 71.83%). LCMS (ESI): $[M+H]^+$ = 316.0.

*Step 2: Preparation of 7-bromo-1-cyclopropyl-6-fluoro-2-methylquinolin-4(1H)-one:*

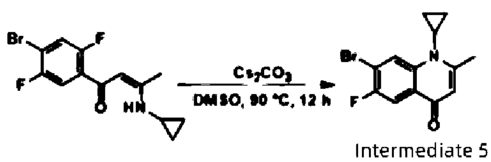


Intermediate 5

The intermediate obtained from step 1 (11.0 g, 34.79 mmol, 1.0 eq.) was dissolved in DMSO (, 100 mL). $Cs_2CO_3$ (22.6 g, 69.58 mmol, 2.0 eq.) was added to the solution at room temperature. The mixture was stirred at 90 °C for 12 h, and reaction progress was monitored by LCMS until complete conversion. The reaction mixture was poured into water (500

mL), and then was extracted with EA (3×300 mL). The combined EA phases were dried over anhydrous $MgSO_4$, filtered, and concentrated under reduced pressure. The crude product was purified by flash silica gel column chromatography (eluent gradient: PE/EA = 10:1 to 0:1) to afford the title compound as a yellow solid (8.2 g, yield: 79.59%). LCMS (ESI): $[M+H]^+$= 296.0.

Preparation Example 6: Preparation of 7-bromo-1-cyclopentyl-6-fluoro-2-methylquinolin-4(1H)-one (Intermediate 6)

*Step 1: Preparation of (Z)-1-(4-bromo-2,5-difluorophenyl)-3-cyclopentylamino)but-2-en-1-one:*

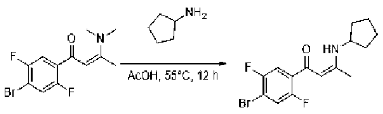


Intermediate 4 (15.0 g, 49.32 mmol, 1.0 eq.) was added to acetic acid (150 mL), followed by the addition of cyclopentylamine (8.4 g, 98.64 mmol, 2.0 eq.). The mixture was stirred at 55 °C for 12 h, and reaction progress was monitored by LCMS until complete conversion. The reaction mixture was then poured into water (600 mL), and then was extracted with EA (3×200 mL). The combined EA layers were dried over anhydrous $MgSO_4$, filtered, and concentrated under reduced pressure. The crude product was purified by silica gel column chromatography (100-200 mesh silica gel, eluent: PE/EA = 5:1) to afford the title compound as a yellow oil (12.1 g, yield: 71.28%). LCMS (ESI): $[M+H]^+$ = 345.9.

***Step 2: Preparation of 7-bromo-1-cyclopentyl-6-fluoro-2-methylquinolin-4(1H)-one:***

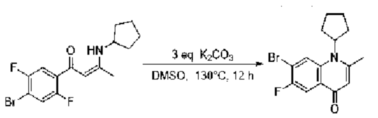


The intermediate obtained from step 1 (12.1 g, 35.15 mmol, 1.0 eq.) was dissolved in DMSO (120 mL), and $K_2CO_3$ (14.58 g, 105.46 mmol, 3.0 eq.) was added. The reaction mixture was maintained at 130 °C for 12 hours, and reaction progress was monitored by LCMS until complete conversion. The mixture was then added to saturated aqueous solution of $NH_4Cl$ (800 mL), and extracted with EA (3×300 mL). The combined EA phases were dried over anhydrous $MgSO_4$, filtered, and the solvent was evaporated under reduced pressure. The crude product was purified by silica gel column chromatography (100-200 mesh silica gel, eluent: DCM/MeOH = 20:1) to obtain the title compound as a dark red solid (9.6 g, yield: 84.24%). LCMS (ESI): $[M+H]^+$ = 323.0.

Preparation Examples 7-25

Intermediates 7-25 were prepared from Intermediate 4 and various commercially available substituted amines by following steps 1-2 of the procedures described in preparation of Intermediate 5 and Intermediate 6.

| Number and Structure | Raw Material | $^1$HNMR | LC-MS(ESI) $(M+H)^+$ |
|---|---|---|---|
| Intermediate 7 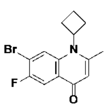 | 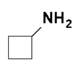 | N/A | 310.0, $t_R$=1.050min |

(continued)

| Number and Structure | Raw Material | $^1$HNMR | LC-MS(ESI) $(M+H)^+$ |
|---|---|---|---|
| Intermediate 8 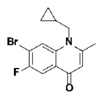 | 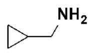 | N/A | 310.2, $t_R$= 1.613min |
| Intermediate 9 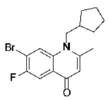 | 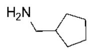 | N/A | 338.2, $t_R$ =1.73 min |
| Intermediate 10 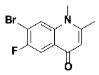 | 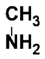 | (400 MHz, $CDCl_3$) δ 8.09 (dd, *J* = 14, 5.2 Hz, 1H), 7.73 (d, *J* = 7.2 Hz, 1H), 3.73 (s, 3H), 2.49 (s, 3H). | 270.0, $t_R$=1.634min |
| Intermediate 11 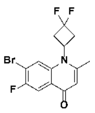 | 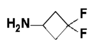 | N/A | 346.10, $t_R$=1.543min |
| Intermediate 12 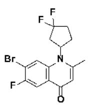 | 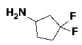 | N/A | 360.2, $t_R$=1.303 min |
| Intermediate 13 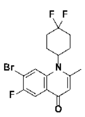 | 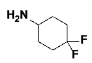 | N/A | 374.0, $t_R$=1.097 min |
| Intermediate 14 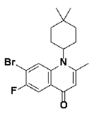 | 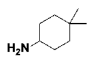 | (400 MHz, DMSO-$d_6$) δ 8.21 (d, *J* = 5.6 Hz, 1H), 7.91 (d, *J* = 8.8 Hz, 1H), 6.10 (s, 1H), 4.55-4.40 (m, 1H), 2.54 (s, 3H), 2.45-2.35 (m, 2H), 1.80-1.70 (m, 2H), 1.65-1.48 (m, 4H), 1.09 (s, 3H), 0.98 (s, 3H). | 366.2, $t_R$=1.457min |
| Intermediate 15 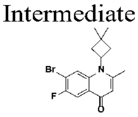 | 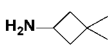 | N/A | 340.0, $t_R$ =1.717 min |
| Intermediate 16 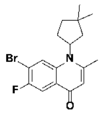 | 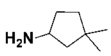 | N/A | 352.10, $t_R$=1.601min |

(continued)

| Number and Structure | Raw Material | $^1$HNMR | LC-MS(ESI) $(M+H)^+$ |
|---|---|---|---|
| Intermediate 17 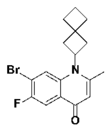 | 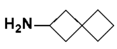 | (400 MHz, DMSO-$d_6$) δ 7.94 (d, J = 5.7 Hz, 1H), 7.84 (d, J = 8.8 Hz, 1H), 6.02 (s, 1H), 5.03 - 4.92 (m, 1H), 2.87 - 2.79 (m, 2H), 2.38 (d, J = 5.6 Hz, 3H), 2.30 - 2.21 (m, 2H), 2.19 - 2.12 (m, 2H), 1.98 - 1.90 (m, 2H), 1.87 - 1.79 (m, 2H). | 350.10, $t_R$=1.243min |
| Intermediate 18 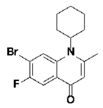 | 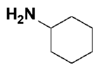 | (400 MHz, DMSO-$d_6$) δ 8.23 (d, J = 5.6 Hz, 1H), 7.91 (d, J = 8.8 Hz, 1H), 6.11 (s, 1H), 4.57 - 4.43 (m, 1H), 2.55 (s, 3H), 2.39 - 2.21 (m, 2H), 1.96 - 1.82 (m, 4H), 1.75 - 1.65 (m, 1H), 1.58 - 1.44 (m, 2H), 1.37 - 1.27 (m, 1H). | 338.10, $t_R$=1.338min |
| Intermediate 19 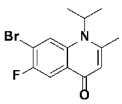 | 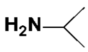 | (400 MHz, DMSO-$d_6$) δ 8.15 (d, J = 5.5 Hz, 1H), 7.93 (d, J = 8.8 Hz, 1H), 6.09 (s, 1H), 5.13 - 5.00 (m, 1H), 2.53 (s, 3H), 1.62 (d, J = 7.1 Hz, 6H). | 300.0, $t_R$=1.431min |
| Intermediate 20 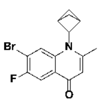 | 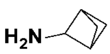 | N/A | 322.0, $t_R$=1.818min |
| Intermediate 21 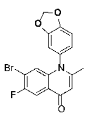 | 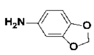 | (400 MHz, DMSO-$d_6$) δ 8.01 (d, J = 8.7 Hz, 1H), 7.79 (d, J = 2.0 Hz, 1H), 7.75 (d, J = 8.5 Hz, 1H), 7.45 (dd, J = 8.5, 2.0 Hz, 1H), 7.18 (d, J = 5.6 Hz, 1H), 6.33 (s, 1H), 3.41 (s, 2H), 2.11 (s, 3H). | 376.13, $t_R$ =1.546 min |
| Intermediate 22 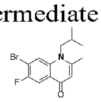 | 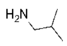 | N/A | 361.3, $t_R$ =2.009 min |
| Intermediate 23 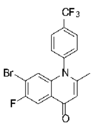 | 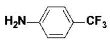 | (400 MHz, DMSO-$d_6$) δ 8.09 (d, J = 8.3 Hz, 2H), 7.96 (d, J = 8.7 Hz, 1H), 7.82 (d, J = 8.2 Hz, 2H), 6.87 (d, J = 5.5 Hz, 1H), 6.29 (s, 1H), 2.01 (s, 3H). | 400.03, $t_R$ =1.729 min |
| Intermediate 25 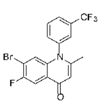 | 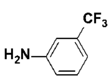 | N/A | 400.05, $t_R$ =1.703 min |

Preparation Example 26: Preparation of (Z)-1-(2,5-difluoro-3-methylphenyl)-3-(dimethylamino)but-2-en-1-one (Intermediate 26)

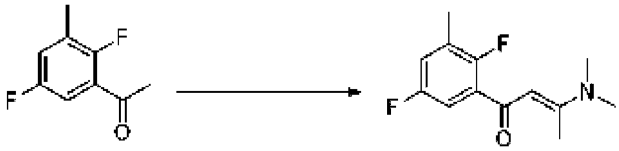

Intermediate 26

2,5-Difluoro-3-methylacetophenone (13 g, 76.4 mmol, 1.0 eq.) was dissolved in anhydrous dioxane (200 mL), and 1,1-dimethoxy-N,N-dimethylethanamine (15.25 g, 114.6 mmol, 1.5 eq.) was added. The reaction mixture was stirred at 100°C overnight, with the reaction completion monitored by LCMS. The reaction mixture was directly concentrated under reduced pressure. The residue was triturated with petroleum ether (PE), filtered, and the collected solid was dried to afford the title compound as a pale yellow solid (13.9 g, yield: 76%). LCMS (ESI): $[M+H]^+$= 239.1.

Preparation Example 27: Preparation of (Z)-1-(2,5-difluorophenyl)-3-(dimethylamino)but-2-en-1-one (Intermediate 27)

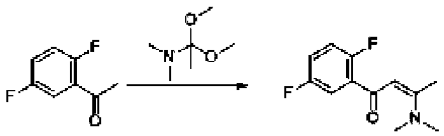

Intermediate 27

2,5-Difluoroacetophenone (60.0 g, 384.30 mmol, 1.0 eq.) was dissolved in anhydrous dioxane (600 mL), and 1,1-dimethoxy-N,N-dimethylethylamine (76.78 g, 576.44 mmol, 1.5 eq.) was added. The reaction mixture was stirred at 100 °C overnight, with the reaction completion monitored by LCMS. After the reaction was complete, the reaction mixture was directly concentrated under reduced pressure. The residue was triturated with PE, filtered, and the collected solid was dried to afford the title compound as a red-brown solid (75.2 g, yield: 86.88%). LCMS (ESI): $[M+H]^+$= 226.1.

Preparation Example 28: Preparation of (Z)-1-(4-chloro-2,5-difluorophenyl)-3-(dimethylamino)but-2-en-1-one (Intermediate 28)

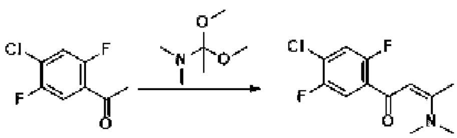

Intermediate 28

At room temperature, 1-(4-chloro-2,5-difluorophenyl)ethanone (65.0 g, 341.08 mmol, 1.0 eq.) was added to anhydrous toluene (500 mL), followed by the addition of 1,1-dimethoxy-N,N-dimethylethanamine (45.3 g, 341.08 mmol, 1.0 eq.). The reaction was conducted at 90 °C for 12 hours, and the completion of the reaction was monitored by LCMS. The reaction solution was concentrated under reduced pressure, and the crude product was triturated with PE(300 mL) at room temperature for 3 hours, then filtered. The filter cake was dried under vacuum at 30 °C for 12 hours, affording the title compound as a brown solid (57 g, yield: 64.35%). LCMS (ESI): $[M+H]^+$ = 260.1.

Preparation Example 29: Preparation of (Z)-1-(4-methyl-2,5-difluorophenyl)-3-(dimethylamino)but-2-en-1-one (Intermediate 29)

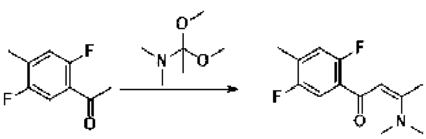

Intermediate 29

1-(4-Methyl-2,5-difluorophenyl)ethanone (25.0 g, 146.92 mmol, 1.0 eq.) was added to anhydrous toluene (200 mL), followed by the addition of 1,1-dimethoxy-N,N-dimethylmethanamine (19.57 g, 146.92 mmol, 1.0 eq.). The mixture was stirred at 90 °C for 12 h, and reaction progress was monitored by LCMS until complete conversion. The reaction solution was concentrated under reduced pressure, and the crude product was slurried with PE (300 mL). The mixture was filtered, and the filter cake was dried under reduced pressure to afford the title compound as a yellow solid (21 g, yield: 59.74%). LCMS (ESI): $[M+H]^+$= 240.1.

Preparation Example 30: Preparation of (Z)-3-(dimethylamino)-1-(2,4,5-trifluorophenyl)but-2-en-1-one (Intermediate 30)

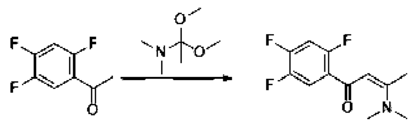

Intermediate 30

1-(2,4,5-Trifluorophenyl)ethanone (7.0 g, 40.2 mmol, 1.0 eq.) was dissolved in anhydrous toluene (100 mL), and1,1-dimethoxy-N,N-dimethylethanamine (6.43 g, 48.24 mmol, 1.2 eq.) was added. The reaction mixture was stirred at 110 °C overnight, and reaction progress was monitored by LCMS. After the reaction was complete, the mixture was directly concentrated under reduced pressure. The residue was triturated with PE, filtered, and the collected solid was dried to afford the title compound as a reddish-brown solid (6.5 g, yield: 66.48%). LCMS (ESI): $[M+H]^+$ = 244.1.

Preparation Examples 31-38

Intermediates 31-37 were prepared from Intermediate 27 and various commercially available substituted amines by following step 2 of the procedure described in Preparation Example 6 (for Intermediate 6). Intermediate 38 was prepared from Intermediate 29 and various commercially available substituted amines by following Step 2 of the procedure described in Preparation Example 6 (for Intermediate 6)

| Number and Structure | Raw Material | $^1$H NMR | LC-MS(ESI) $(M+H)^+$ |
|---|---|---|---|
| Intermediate 31 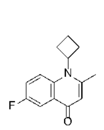 | 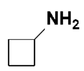 | N/A | 268.0, $t_R$=1.33min |
| Intermediate 32 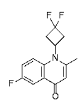 | 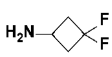 | N/A | 232.1, $t_R$= 1.413min |
| Intermediate 33 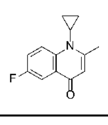 | 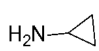 | (400 MHz, DMSO-$d_6$) δ 7.84 (dd, J = 9.5, 4.4 Hz, 1H), 7.76 (dd, J = 9.1, 3.2 Hz, 1H), 7.55 - 7.49 (m, 1H), 6.01 (s, 1H), 5.23 - 5.11 (m, 1H), 2.70 - 2.60 (m, 2H), 2.43 (s, 3H), 1.88 - 1.73 (m, 2H). | 218.1, $t_R$ =1.17 min |
| Intermediate 34 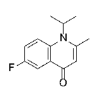 | 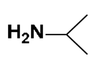 | N/A | 220.1, $t_R$ =1.33 min |

(continued)

| Number and Structure | Raw Material | $^1$H NMR | LC-MS(ESI) $(M+H)^+$ |
|---|---|---|---|
| Intermediate 35 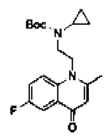 | 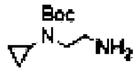 | (400 MHz, DMSO-$d_6$) δ 7.92 (s, 1H), 7.81 (dd, *J* = 9.0, 3.2 Hz, 1H), 7.71 - 7.62 (m, 1H), 6.12 (s, 1H), 4.40 (t, *J* = 6.4 Hz, 2H), 3.49 (t, *J* = 5.9 Hz, 2H), 2.48 - 2.38 (m, 1H), 1.38 (s, 9H), 0.68 - 0.48 (m, 2H), 0.51 - 0.29 (m, 2H). | 361.3, $t_R$ =2.009 min |
| Intermediate 36 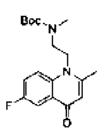 | 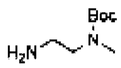 | (400 MHz, DMSO-$d_6$) δ 7.99 - 7.77 (m, 2H), 7.68 - 7.56 (m, 1H), 6.10 (s, 1H), 4.46 - 4.35 (m, 2H), 3.61 - 3.43 (m, 2H), 2.80 (d, *J* = 24.7 Hz, 2H), 2.55 (s, 3H), 1.24 (d, *J* = 104.1 Hz, 9H). | 335.1, $t_R$ =0.972 min |
| Intermediate 37 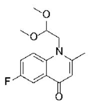 | 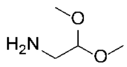 | (400 MHz, DMSO-$d_6$) δ 7.88 (dd, J = 9.5, 4.3 Hz, 1H), 7.76 (dd, J = 9.0, 3.2 Hz, 1H), 7.66 - 7.53 (m, 1H), 6.08 (s, 1H), 4.67 (t, J = 5.3 Hz, 1H), 4.39 (d, J = 5.3 Hz, 2H), 3.29 (s, 6H), 2.48 (s, 3H). | 266.3, $t_R$ =1.247 min |
| Intermediate 38 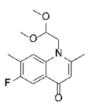 | 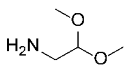 | N/A | 280.3, $t_R$ =1.430 min |

Preparation Example 39: Preparation of (E)-3-(dimethylamino)-1-(5-fluoro-4-methoxy-2-methylphenyl)but-2-en-1-one (Intermediate 39):

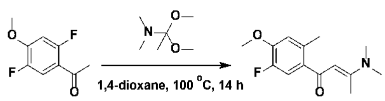


2,5-Difluoro-4-methoxyphenylacetone (15.0 g, 64.1 mmol, 1.0 eq.) was dissolved in anhydrous 1,4-dioxane (300 mL), and 1,1-dimethoxy-N,N-dimethylethanamine (12.8 g, 96.2 mmol, 1.5 eq.) was added. The reaction mixture was stirred at 100 °C for 14 hours, with the reaction progress monitored by LCMS. The solvent was removed under reduced pressure, and the residue was purified by silica gel column chromatography (200-300 mesh silica gel, eluent: PE/THF = 4:1) to afford the title compound as a brown solid (16.2 g, 64.5 mmol, yield: 78.8%). LCMS (ESI): $[M+H]^+$=256.2.

Preparation Example 40: Preparation of (R)-2-((7-chloro-6-fluoro-2-methyl-4-oxoquinolin-1(4H)-yl)methyl)pyrrolidine-1-carboxylic acid tert-butyl ester

*Step 1: Preparation of (R,E)-2-(((4-(4-chloro-2,5-difluorophenyl)-4-oxobut-2-en-2-yl)amino)methyl)pyrrolidine-1-carboxylic acid tert-butyl ester:*

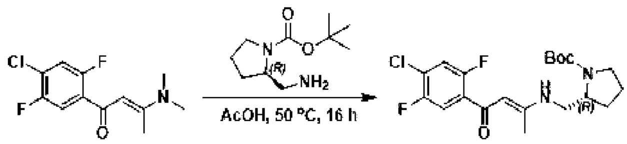


Intermediate 28

Intermediate 28 (10.0 g, 38.51 mmol, 1.0 eq) was added to acetic acid (100 mL), followed by the addition of (R)-2-(aminomethyl)pyrrolidine-1-carboxylic acid tert-butyl ester (7.7 g, 38.51 mmol, 1.0 eq.). The reaction mixture was maintained at 50 °C for 16 hours, with the reaction completion monitored by LCMS. The mixture was poured into ice water (500 mL), and the resulting mixture was extracted with ethyl acetate (EA, 3 × 500 mL). The combined organic phases were dried over anhydrous magnesium sulfate ($MgSO_4$), filtered, and concentrated under reduced pressure. The crude product was purified by flash silica gel column chromatography (eluent gradient: PE/EA = 10:1 to 5:1) to afford the title compound as a yellow solid (9.3 g, yield: 58%). LCMS (ESI): $[M+H]^+$= 415.2, $t_R$ = 1.57 min.

*Step 2: Preparation of (R)-2-((7-chloro-6-fluoro-2-methyl-4-oxoquinolin-1(4H)-yl)methyl)pyrrolidine-1-carboxylic acid tert-butyl ester:*

The intermediate obtained from step 1 (10.4 g, 25.07 mmol, 1.0 eq.] was dissolved in dimethyl sulfoxide (DMSO, 100 mL), and $Cs_2CO_3$ (24.5 g, 75.2 mmol, 3.0 eq.) was added. The reaction mixture was maintained at 80 °C for 2 hours, with the reaction completion monitored by LCMS. After the reaction mixture was cooled to room temperature, it was added to saturated aqueous solutionof $NH_4Cl$ (400 mL), and the resulting mixture was extracted with EA (3×400 mL). The combined organic phases were dried over anhydrous magnesium sulfate ($MgSO_4$), filtered, and the solvent was evaporated under reduced pressure. The crude product was purified by flash silica gel column chromatography (eluent: PE/EA = 5:1) to afford the title compound as a yellow solid (6.2 g, yield: 63%). LCMS (ESI): $[M+H]^+$= 395.2, $t_R$=1.27 min.

Preparation Example 41: Preparation of 7-bromo-6-fluoro-2-methyl-1-{spiro[2.5]octan-6-yl}-1,4-dihydroquinolin-4-one (Intermediate 41):

*Step 1: Preparation of (Z)-1-(4-bromo-2,5-difluorophenyl)-3-({spiro[2.5]octan-6-yl}amino)but-2-en-1-one:*

Intermediate 4 (8.5 g, 28.0 mmol, 1.0 eq.) was dissolved in a mixed solvent of MeOH (100 mL) and EA (10 mL), followed by the addition of triethylamine (TEA, 8.5 g, 83.9 mmol, 3.0 eq.). After the addition was completed, the reaction mixture was maintained at 80 °C for 2 hours, with the reaction completion monitored by LCMS, the mixture was cooled to room temperature and concentrated under reduced pressure to remove most of the MeOH. Ice water (500 mL) was added to the residue, and the resulting mixture was extracted with EA (3×300 mL). The combined organic layers were washed with saturated brine (2×100mL), dried over anhydrous $MgSO_4$, filtered, and concentrated under reduced pressure. The crude product was purified by silica gel column chromatography (eluent: PE/EA = 10:1) to afford the title compound as a yellow solid (6.3 g, yield: 58.7%). LCMS (ESI): $[M+H]^+$ = 384.2, $t_R$= 1.610 min.

*Step 2: Preparation of 7-bromo-6-fluoro-2-methyl-1-{spiro[2.5]octan-6-yl}-1,4-dihydroquinolin-4-one:*

The intermediate obtained from step 1 (6.3 g, 16.4 mmol, 1.0 eq.) was dissolved in DMSO (63 mL), and cesium carbonate ($Cs_2CO_3$, 10.7 g, 32.8 mmol, 2.0 eq.) was added. The reaction mixture was stirred at 80 °C for 16 hours, with the reaction completion monitored by LCMS. The mixture was cooled to room temperature and quenched with ice water (200 mL). The resulting mixture was extracted with EA (3×100 mL), and the combined EA phases were washed with saturated brine (3×50mL). The organic phase was dried over anhydrous $MgSO_4$, filtered, and concentrated under reduced pressure. The crude product was purified by silica gel column chromatography (eluent: PE/THF = 1:1.5) to afford the title compound as a yellow solid (4.2 g, yield: 70.3%). LCMS (ESI): $[M+H]^+$= 364.2, $t_R$= 1.410 min.

Preparation Example 42: Preparation of 7-bromo-1-(3,5-dimethylphenyl)-6-fluoro-2-methylquinolin-4(1H)-one (Intermediate 42)

*Step 1: Preparation of (E)-1-(4-bromo-2,5-difluorophenyl)-3-((3,5-dimethylphenyl)amino)but-2-en-1-one:*

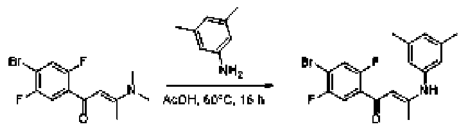

Intermediate 4

Intermediate 4 [(Z)-1-(4-bromo-2,5-difluorophenyl)-3-(dimethylamino)but-2-en-1-one, 1.5 g, 4.95 mmol, 1.0 eq.] was dissolved in ice-cold acetic acid (15 mL), followed by the addition of 3,5-dimethylaniline (1.2 g, 9.9 mmol, 2.0 eq.). After the addition was completed, the reaction mixture was heated to 60 °C and stirred for 16 hours. LCMS analysis confirmed the completion of the reaction. The reaction mixture was poured into ice-cold saturated sodium bicarbonate solution (100 mL), and the pH of the mixture was adjusted to slightly basic. The resulting mixture was extracted with ethyl acetate (EA, 3 × 50 mL). The organic layers were combined and washed with saturated brine (200 mL), then dried over anhydrous sodium sulfate ($Na_2SO_4$), filtered, and concentrated under reduced pressure.

The title compound was obtained as a yellow solid (1.5 g, yield: 79.7%). LCMS (ESI): $[M + H]^+$ = 380.0, $t_r$ = 2.444 min.

*Step 2: Preparation of 7-bromo-1-(3,5-dimethylphenyl)-6-fluoro-2-methylquinolin-4(1H)-one:*

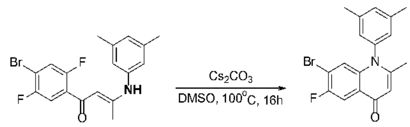


The intermediate obtained from step 1 (2 g, 5.26 mmol, 1.0 eq.] was dissolved in DMSO (20 mL), and $Cs_2CO_3$ (5.14 g, 15.78 mmol, 3.0 eq.) was added. After the addition was completed, the reaction mixture was heated to 100 °C and stirred for 16 hours. LCMS analysis confirmed the completion of the reaction. The reaction mixture was cooled to room temperature and filtered, and the filtrate was poured into ice-cold saturated brine (60 mL). The resulting mixture was extracted with ethyl acetate (EA, 3 × 30 mL), and the organic phases were combined, dried over anhydrous $Na_2SO_4$, filtered, and the filtrate was concentrated under reduced pressure to afford a crude product. The crude product was purified by silica gel column chromatography (eluent gradient: DCM/MeOH = 1:0 to 100:1) to yield the title compound as a brown solid (1 g, yield: 52.78%). LCMS (ESI): $[M+H]^+$ = 360.0, $t_R$ = 2.019 min.

Preparation Example 43: Preparation of 7-bromo-6-fluoro-1-(3-methoxy-5-methylphenyl)-2-methylquinolin-4(1H)-one (Intermediate 43):

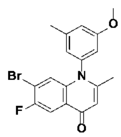

Intermediate 43 was synthesized following a method similar to that described in Preparation Example 42. The title compound was obtained as a yellow solid. LCMS (ESI): $[M+H]^+$= 396.1, $t_R$= 1.370 min; $^1H$ NMR (400 MHz, DMSO-$d_6$) δ 7.90 (d, J = 8.8 Hz, 1H), 7.04 (s, 1H), 6.92 (d, J = 5.8 Hz, 2H), 6.87 (s, 1H), 6.22 (s, 1H), 3.79 (s, 3H), 2.37 (s, 3H), 2.05 (s, 3H).

Preparation Example 44: Preparation of 7-bromo-6-fluoro-1-(4-trifluoromethylphenyl)-2-methylquinolin-4(1H)-one (Intermediate 44):

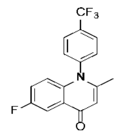

Intermediate 44 was synthesized using Intermediate 27 as the starting material, following a method similar to that described in Preparation Example 42. The title compound was obtained as a yellow solid. LCMS (ESI): $[M+H]^+$=322.1, $t_R$ = 1.370 min; $^1$H NMR (400 MHz, DMSO-$d_6$) δ 7.90 (d, J = 8.8 Hz, 1H), 7.04 (s, 1H), 6.92 (d, J = 5.8 Hz, 2H), 6.87 (s, 1H), 6.22 (s, 1H), 3.79 (s, 3H), 2.37 (s, 3H), 2.05 (s, 3H).

Preparation Example 45: Preparation of (3S,4S)-3-(7-bromo-6-fluoro-2-methyl-4-oxoquinolin-1(4H)-yl)-4-hydroxypyrrolidine-1-carboxylic acid tert-butyl ester

*Step* 1: *Preparation of (3S,4S)-3-(((E)-4-(4-bromo-2,5-difluorophenyl)-4-oxobut-2-en-2-yl)amino)-4-hydroxypyrrolidine-1-carboxylic acid tert-butyl ester:*

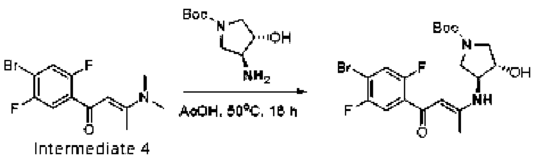


Intermediate 4 (10 g, 32.88 mmol, 1.0 eq.] and (3S,4S)-3-amino-4-hydroxypyrrolidine-1-carboxylic acid tert-butyl ester (6.65 g, 32.88 mmol, 1.0 eq.) were sequentially dissolved in acetic acid (100 mL). Under nitrogen protection, the reaction mixture was heated to 50 °C and stirred for 16 hours, with the reaction completion monitored by LCMS. The reaction mixture was poured into ice-cold saturated sodium bicarbonate solution (150 mL) and extracted with EA (3×150 mL). The EA layers were combined, dried over anhydrous $Na_2SO_4$, filtered, and the filtrate was concentrated under reduced pressure to afford a crude product. The crude product was purified by silica gel column chromatography (eluent gradient: PE/EA = 1:0 to 3:1) to yield the title compound as a brown solid (11 g, yield: 75.52%). LCMS (ESI): $[M+H]^+$ = 461.3, $t_R$= 1.914 min; $^1$H-NMR (400 MHz, DMSO-$d_6$) δ 11.39 (dd, J = 8.9, 3.6 Hz, 1H), 7.71 (dd, J = 9.8, 5.4 Hz, 1H), 7.54 (dd, J = 9.1, 6.2 Hz, 1H), 5.58 (d, J = 4.0 Hz, 1H), 5.52 (d, J = 1.9 Hz, 1H), 4.14 - 3.77 (m, 2H), 3.70 - 3.56 (m, 1H), 3.52 - 3.39 (m, 1H), 3.23 - 3.03 (m, 2H), 2.10 (s, 3H), 1.36 (s, 9H).

*Step 2: Preparation of (3S,4S)-3-(7-bromo-6-fluoro-2-methyl-4-oxoquinolin-1(4H)-yl)-4-hydroxypyrrolidine-1-carboxylic acid tert-butyl ester:*

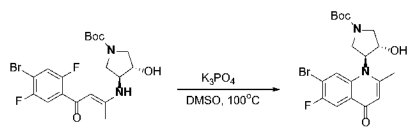


The intermediate obtained from step 1 (9.2 g, 19.94 mmol, 1.0 eq.] and $K_3PO_4$ (8.27 g, 59.83 mmol, 3.0 eq.) were dissolved in DMSO (92 mL). Under nitrogen protection, the reaction mixture was heated to 100 °C and stirred for 16 hours, with the reaction completion monitored by LCMS. The mixture was filtered, and the filter cake was washed with ethyl acetate (EA). The resulting filtrate was poured into water (60 mL) and extracted with EA (3×80mL). The organic layers were combined, washed with saturated brine (3 × 80 mL), dried over anhydrous $Na_2SO_4$, filtered, and the filtrate was concentrated under reduced pressure to afford a crude product. The crude product was purified by silica gel column chromatography (eluent gradient: DCM/MeOH = 1:0 to 20:1) to afford the title compound as a brown-yellow solid (4.1 g, yield: 46.59%). LCMS (ESI): $[M+H]^+$ = 441.1, $t_R$= 1.783 min; $^1$H-NMR (400 MHz, DMSO-$d_6$) δ 7.89 (d, J = 8.7 Hz, 1H), 7.60 (s, 1H), 6.07 (s, 1H), 5.82 (d, J = 5.3 Hz, 1H), 5.24 - 5.14 (m, 1H), 4.84 - 4.75 (m, 1H), 4.04 - 3.95 (m, 1H), 3.78 (s, 1H), 3.66 - 3.58 (m, 1H), 3.03 (s, 1H), 2.50 (s, 3H), 1.38 (s, 9H).

Preparation Example 46: Preparation of 8-bromo-6-fluoro-1-methyl-4-oxo-1,4-dihydroquinoline-2-carboxylic acid ethyl ester (Intermediate 46):

*Step 1: Preparation of 1-(5-fluoro-2-((4-methoxybenzyl)(methyl)amino)phenyl)ethanone:*

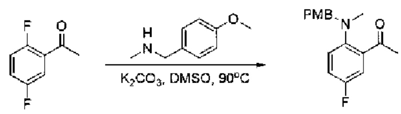


2,5-Difluoroacetophenone (500 mg, 3.21 mmol, 1.0 eq.) was dissolved in DMSO (5 mL), followed by the sequential addition of 1-(4-methoxyphenyl)-N-methylmethanamine (485 mg, 3.21 mmol, 1.0 eq.) and $K_2CO_3$ (1.33 g, 9.63 mmol, 3.0 eq.). After the addition was completed, the mixture was stirred at 90°C for 16 h, and reaction progress was monitored by LCMS until complete conversion. The reaction mixture was filtered, and the filtrate was poured into water (30 mL). The resulting mixture was extracted with EA (2×20 mL), and the EA layers were combined, washed with saturated brine (30 mL), dried over anhydrous $Na_2SO_4$, filtered, and the filtrate was concentrated under reduced pressure to afford a crude product. The crude product was purified by silica gel column chromatography (eluent gradient: PE/EA = 1:0 to 100:1) to afford the title compound as a brown oily substance (412 mg, yield: 44.8%). LCMS (ESI): $[M+H]^+$= 288.1.

*Step 2: Preparation of 1-(5-fluoro-2-(methylamino)phenyl)ethenone:*

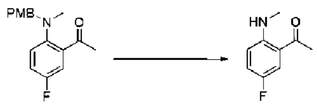

Under ice bath conditions, 1-(5-fluoro-2-((4-methoxybenzyl)(methyl)amino) phenyl)ethanone (412 mg, 1.44 mmol, 1.0 eq.) and triethylsilane (TES, 167 mg, 1.44 mmol, 1.0 eq.) were sequentially added to TFA (657 mg, 5.76 mmol, 4.0 eq.). After the addition was completed, the mixture was stirred at 70°C for 12h, and reaction progress was monitored by LCMS until complete conversion. The mixture was concentrated to afford a crude product, which was dissolved in EA (30 mL) and washed with saturated sodium bicarbonate solution (20 mL). The organic phase was dried over anhydrous $Na_2SO_4$, filtered, and the filtrate was concentrated under reduced pressure to obtain a crude product. The crude product was purified by silica gel column chromatography (eluent gradient: PE/EA = 1:0 to 100:1) to afford the title compound as a yellow oily substance (170 mg, yield: 70.7%). LCMS (ESI): $[M+H]^+$=168.2.

***Step 3: Preparation of 1-(3-bromo-5-fluoro-2-(methylamino)phenyl)ethanone:***

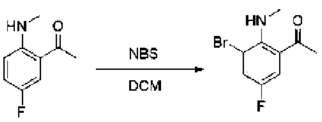


The intermediate obtained from step 2 (170 mg, 1.02 mmol, 1.0 eq.) was dissolved in acetonitrile (5 mL), and NBS (199 mg, 1.12 mmol, 1.1 eq.) was added. The reaction was maintained at room temperature for 40 minutes, with the completion monitored by LCMS. The mixture was concentrated under reduced pressure to afford a crude product, which was purified by silica gel column chromatography (eluent gradient: PE/EA = 1:0 to 100:1) to afford the title compound as a yellow solid (130 mg, yield: 52.1%). LCMS (ESI): $[M+H]^+$ = 246.0; $^1H$ NMR (400 MHz, $CDCl_3$) $\delta$ 7.50 (dd, J = 7.4, 2.9 Hz, 1H), 7.43 (dd, J = 8.8, 2.9 Hz, 1H), 3.04 (s, 3H), 2.67 (s, 3H).

***Step 4: Preparation of 8-bromo-6-fluoro-1-methyl-4-oxo-1,4-dihydroquinoline-2-carboxylic acid ethyl ester:***

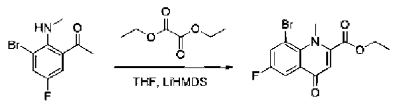


The intermediate obtained from step 3 (50 mg, 0.204 mmol, 1.0 eq.) and diethyl oxalate (74.5 mg, 0.510 mmol, 2.5 eq.) were sequentially dissolved in anhydrous THF (3 mL). Under nitrogen protection and at -65 °C, LiHMDS in tetrahydrofuran (1 mol/L, 1.79 mL, 1.79 mmol, 3.5 eq.) was slowly added. After the addition was completed, the dry ice bath was not removed; the reaction mixture was allowed to slowly warm to room temperature and stirred overnight, with the reaction progress monitored by LCMS. The reaction mixture was poured into a ice-cold saturated solution of $NH_4Cl$ (30 mL) and extracted with EA (3 × 15 mL). The organic layers were combined, dried over anhydrous $Na_2SO_4$, filtered, and the filtrate was concentrated to obtain the title compound (crude product, directly used in the next step). LCMS (ESI): $[M+H]^+$= 299.9.

**Preparation Example 47: Preparation of (R)-3-(6-fluoro-2-methyl-4-oxoquinolin-1(4H)-yl)piperidine-1-carboxylic acid tert-butyl ester (Intermediate 47):**

***Step 1: Preparation of (R,Z)-3-((4-(2,5-difluorophenyl)-4-oxobut-2-en-2-yl)amino)piperidine-1-carboxylic acid tert-butyl ester:***

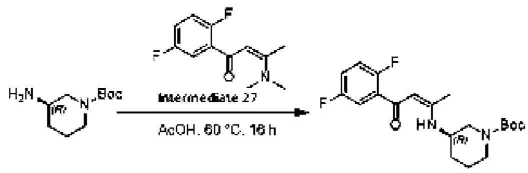


(R)-1-tert-Butoxycarbonyl-3-aminopiperidine (40.0 g, 199.72 mmol, 1.0 eq.) was added to acetic acid (100 mL), followed by the addition of Intermediate 27 (53.98 g, 239.66 mmol, 1.2 eq.). The reaction mixture was maintained at 60 °C for 16 hours, with the completion monitored by LCMS. After the reaction was complete, the mixture was added to water (500 mL) and extracted with EA (3×200 mL). The combined organic phase was dried over anhydrous $MgSO_4$, filtered, and concentrated to obtain a crude product was purified by silica gel column chromatography (200-300 mesh silica gel, eluent: PE/EA = 5:1) to afford the target compound as a reddish-brown oily substance (62.5 g, yield: 82.26%). LCMS (ESI): $[M+H]^+$= 381.3, $t_R$= 2.167 min.

***Step 2: Preparation of (R)-3-(6-fluoro-2-methyl-4-oxoquinolin-1(4H)-yl)piperidine-1-carboxylic acid tert-butyl ester:***

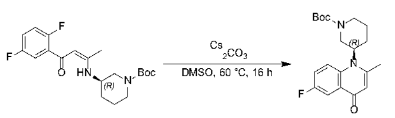


The intermediate obtained from step 1(50 g, 131.43 mmol, 1.0 eq.] was dissolved in DMSO (500 mL), and $Cs_2CO_3$ (128.47 g, 394.29 mmol, 3.0 eq.) was added. The reaction mixture was maintained at 60 °C for 16 hours, with the completion monitored by LCMS. After the reaction was complete, the mixture was added to saturated solution of $NH_4Cl$ (2000 mL) and extracted with EA (3×500 mL). The combined organic phase was dried over anhydrous $MgSO_4$, filtered, and concentrated under reduced pressure. The crude product was purified by silica gel column chromatography (200-300 mesh silica gel, eluent: DCM/MeOH = 30:1) to afford the target compound as a reddish-brown solid (33.7 g, yield: 71.14%). LCMS (ESI): $[M+H]^+$ = 361.3, $t_R$= 1.767 min.

Example 1: Preparation of (S)-4-ethyl-4-hydroxy-11-methyl-1,12-dihydro-14H-pyrano[3,4:6,7]indolizino[2,1 - b]quinoline-3,6,14(4H,11H)-trione:

*Step 1: Preparation of 3-bromo-2-methylquinolin-4(1H)-one:*

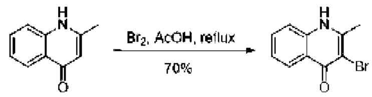


2-Methylquinolin-4(1H)-one (14.8 g, 93.0 mmol, 1.0 eq.) was dissolved in acetic acid (AcOH, 220 mL), and the mixture was heated to reflux. Bromine ($Br_2$, 14.9 g, 93.0 mmol, 1.0 eq.) was added dropwise. After the addition was completed, the reaction mixture was continuously stirred under reflux for 3 hours. When the reaction was complete, the mixture was poured into ice water (500 mL), resulting in the formation of a large amount of white solid. The solid was filtered, and the filter cake was washed with water (500 mL). The product was dried under vacuum to obtain the target compound as a pale yellow solid (15.2 g, yield: 70%). $^1$H-NMR (400 MHz, DMSO-$d_6$) δ 12.14 (s, 1H), 8.09 (d, J = 7.6 Hz, 1H), 7.68 (t, J = 8.0 Hz, 1H), 7.62 (d, J = 8.4 Hz, 1H), 7.36 (t, J = 8.0 Hz, 1H), 2.56 (s, 3H).

***Step 2: Preparation of 3-bromo-1,2-dimethylquinolin-4(1H)-one:***

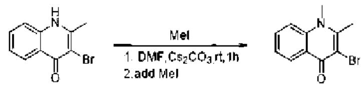


Under a nitrogen atmosphere and in an ice-water bath, cesium carbonate ($Cs_2CO_3$, 31.3 g, 96.2 mmol, 1.5 eq.) was added to an anhydrous DMF (150 mL) solution of 3-bromo-2-methylquinolin-4(1H)-one (15.2 g, 64.1 mmol, 1.0 eq.). The mixture was stirred for 1.5 hours, then methyl iodide (MeI, 45.5 g, 321.0 mmol, 5.0 eq.) was added. The reaction mixture was continuously stirred while warming from 0 °C to room temperature (27-34 °C) overnight, with the reaction progress monitored by TLC until completion. The reaction was quenched by adding ice water (400 mL), and the mixture was extracted with EA (3×300 mL). The organic layers were combined, washed with saturated brine (3×500 mL), dried over anhydrous $Na_2SO_4$, and concentrated to obtain a yellow oily residue. The residue was purified by silica gel column chromatography (eluent gradient: DCM/MeOH = 30:1 to 10:1) to afford the target compound as a pale yellow solid (5.78 g, yield: 36%). $^1$H-NMR (400 MHz, $CDCl_3$) δ 8.50 (dd, J = 8.0, 2.8 Hz, 1H), 7.66 (t, J = 8.8 Hz, 1H), 7.51 (d, J = 8.4 Hz, 1H), 7.40 (t, J = 8.0 Hz, 1H), 3.87 (s, 3H), 2.84 (s, 3H).

***Step 3: Preparation of 3-bromo-2-(bromomethyl)-1-methylquinolin-4(1H)-one:***

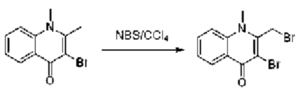


Under a nitrogen atmosphere, NBS (4.79 g, 26.9 mmol, 1.1 eq.) and benzoyl peroxide (BPO, 590 mg, 2.4 mmol, 0.1 eq.) were added to a $CCl_4$ (120 mL) solution of 3-bromo-1,2-dimethylquinolin-4(1H)-one (6.13 g, 24.4 mmol, 1.0 eq.). The reaction mixture was refluxed for 2 hours, turning reddish-brown and turbid. TLC monitoring confirmed the reaction was complete. The mixture was filtered to remove solids, then diluted with EA (150 mL). The resulting solution was washed with saturated brine (3×300 mL), dried over anhydrous $Na_2SO_4$, filtered, and concentrated under reduced pressure. The crude product was purified by silica gel column chromatography (eluent gradient: DCM/MeOH = 40:1 to 15:1) to afford the title compound as a yellowish solid (2.4 g, yield: 37%). $^1$H NMR (400 MHz, $CDCl_3$) δ 8.26 (d, J = 1.6 Hz, 1H), 8.24 (d, J = 2.8 Hz, 1H), 7.85 (d, J = 8.0 Hz, 1H), 7.52 (t, J = 7.2 Hz, 1H), 5.13 (s, 2H), 3.99 (s, 3H).

***Step 4: Preparation of (S)-7-((3-bromo-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

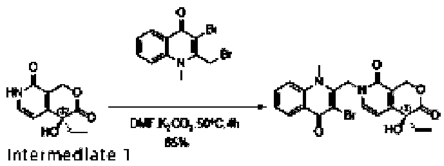


Under a nitrogen atmosphere and in an ice-water bath, $K_2CO_3$ (594 mg, 4.3 mmol, 2.5 eq.) was added to an DMF (20 mL) solution of Intermediate 1 (360 mg, 1.7 mmol, 1.0 eq.) and 3-bromo-2-(bromomethyl)-1-methylquinolin-4(1H)-one (569 mg, 1.7 mmol, 1.0 eq.). The reaction mixture was warmed to room temperature (26-33 °C) and stirred overnight. After the reaction was complete, the mixture was cooled to 0 °C and quenched with water (40 mL), then extracted with EA (3× 30mL). The organic layers were combined, washed with saturated sodium chloride solution (brine, 3×50 mL), dried over anhydrous $Na_2SO_4$, filtered, and concentrated under reduced pressure. The residue was mixed with silica gel and purified by column chromatography using a gradient eluent of DCM:MeOH=40:1 to 15:1, yielding the title compound as a light yellow solid (670 mg, yield: 85%). $^1$H NMR (400 MHz, DMSO-$d_6$) δ 8.26 (dd, J = 8.0, 1.2 Hz, 1H), 7.86 (d, J = 8.8 Hz, 1H), 7.84-7.82 (m, 1H), 7.53-7.50 (m, 2H), 6.44 (d, J = 7.2 Hz, 1H). 6.32 (s, 1H), 5.70- 5.57 (m, 2H), 5.31 (s, 2H), 3.80 (s, 3H), 3.17 (d, J = 5.2 Hz, 1H), 1.79-1.75 (m, 2H), 0.80 (t, J = 7.2 Hz, 3H).

**Example 2: Preparation of (S)-4-ethyl-8-fluoro-4-hydroxy-10,11-dimethyl-1,12-dihydro-14H-pyrano[3,4:6,7]indolizino[2,1 -b]quinoline-3,6,14(4H,11H)-trione:**

***Step 1: Preparation of 2-((4-fluoro-2-methylphenyl)amino)maleic acid dimethyl ester:***

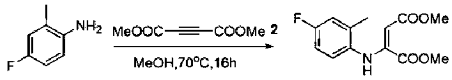


4-Fluoro-2-methylaniline (10 g, 0.08 mol, 1.0 eq.) and dimethyl butynedioate (11.4 g, 0.08 mol, 1.0 eq.) were dissolved in MeOH (MeOH, 100 mL). The reaction mixture was stirred at 70 °C for 16 hours, with the completion monitored by LCMS. After the reaction was complete, the solvent was removed under reduced pressure. The crude product was purified by flash silica gel column chromatography (eluent gradient: PE/EA = 100:0 to 97:3, v/v) to yield the title compound as a light yellow oil (18.0 g, yield: 84.2%). LCMS (ESI): $[M+H]^+$ = 268.2.

***Step 2: Preparation of 2-((4-fluoro-2-methylphenyl)(methyl)amino)maleic acid dimethyl ester:***

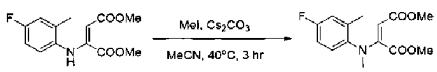


The intermediate obtained from step 1 (20 g, 0.08 mol, 1.0 eq.) was dissolved in acetonitrile (200 mL). $Cs_2CO_3$ (73.2 g, 0.23 mol, 3.0 eq.) was added, and under a nitrogen atmosphere, methyl iodide (MeI, 11.7 g, 0.08 mol, 1.1 eq.) was added dropwise. The reaction mixture was maintained at 40 °C for 3 hours. The reaction progress was monitored by LCMS until completion. Then the mixture was filtered, and the filtrate was collected. The solvent was evaporated to obtain a crude product, which was purified by silica gel column chromatography (100-200 mesh silica gel, eluent gradient: PE/EA= 90:10 to 80:20, v/v) to afford the target compound as a yellow solid (13 g, yield: 61.7%). LCMS (ESI): $[M+H]^+$ = 282.2.

***Step 3: Preparation of methyl 6-fluoro-1,8-dimethyl-4-oxo-1,4-dihydroquinoline-2-carboxylate:***

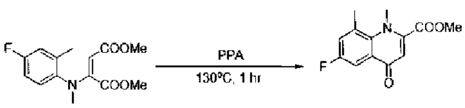


A mixture of the intermediate obtained from step 2 (13.0 g, 46.22 mmol, 1.0 eq.) and polyphosphoric acid (PPA,

100 g, 100 mmol, 27.4 eq.) was heated to 130 °C and stirred for 1 hour. The reaction progress was monitored by LCMS until completion. After the reaction mixture was cooled to room temperature, it was poured into ice-cold saturated solution of $NaHCO_3$ (300 mL). The resulting solid was collected by filtration and purified by silica gel column chromatography (eluent gradient: PE/EA = 100:0 to 50:50, v/v) to obtain the target compound as a white solid (1.6 g, yield: 13.9%). LCMS (ESI): $[M+H]^+$= 250; $^1H$ NMR (400 MHz, $CDCl_3$) δ 7.83 (dd, *J*= 8.3, 3.1 Hz, 1H), 7.26 (s, 1H), 6.79 (s, 1H), 3.98 (s, 3H), 3.73 (s, 3H), 2.67 (s, 3H).

***Step 4: Preparation of 6-fluoro-2-(hydroxymethyl)-1,8-dimethylquinolin-4(1H)-one:***

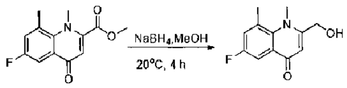


The intermediate obtained from step 3 (701 mg, 2.98 mmol, 1.0 eq.) was added to MeOH (15 mL). Under an ice-water bath (0 °C), $NaBH_4$ (564 mg, 14.9 mmol, 5.0 eq.) was added portionwise. The reaction mixture was warmed to 20 °C and stirred for 4 hours, with the completion monitored by LCMS. Ice water (5 mL) was added to quench the reaction, and the mixture was extracted with EA (3×10 mL). The combined organic layers were washed with saturated brine (3×10 mL), dried over anhydrous $Na_2SO_4$, filtered, and concentrated to afford the title compound as a yellow solid (640 mg, yield: 96.98%). LCMS (ESI): m/z = 222.0 $[M+H]^+$; $^1H$ NMR (400 MHz, DMSO-*$d_6$*) δ 7.62 (dd, J = 8.8, 3.2 Hz, 1H), 7.46 (dd, J = 9.2, 3.2 Hz, 1H), 6.23 (s, 1H), 5.74 (s, 1H), 4.57 (s, 2H), 3.75 (s, 3H), 2.69 (s, 3H).

***Step 5: Preparation of 6-fluoro-2-(hydroxymethyl)-3-iodo-1,8-dimethylquinolin-4(1H)-one:***

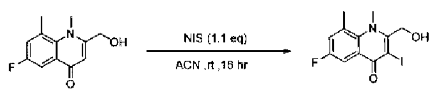


The intermediate obtained from step 4 (9.2 g, 41.6 mmol, 1.0 eq.) was dissolved in acetonitrile (100 mL), and NIS (10.3 g, 45.8 mmol, 1.1 eq.) was added. The reaction mixture was stirred at room temperature for 16 hours, with the completion confirmed by LCMS. The solvent was removed to obtain a crude product, which was purified by silica gel column chromatography (eluent: PE/EA = 1:1, v/v) to afford the title compound as a yellow solid (9.2 g, yield: 63.7%). LCMS (ESI): $[M+H]^+$=348.2.

***Step 6: Preparation of 2-(chloromethyl)-6-fluoro-3-iodo-1,8-dimethylquinolin-4(1H)-one:***

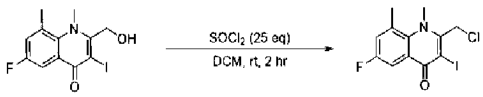


The intermediate obtained from step 5 (9.2 g, 26.5 mmol, 1.0 eq.) was dissolved in DCM (800 mL). Under an icebath condition, thionyl chloride (78.8 g, 663.0 mmol, 25.0 eq.) was added dropwise. The reaction mixture was warmed to room temperature and stirred for 2 hours, with the completion confirmed by LCMS. The reaction mixture was concentrated, and saturated sodium bicarbonate solution was added to the residue to adjust the pH to slightly basic. The mixture was extracted with EA, and the organic layer was concentrated to obtain the title compound as a yellow solid (7.6 g, crude product). LCMS (ESI): $[M+H]^+$ =366.1.

*Step 7: Preparation of (S)-4-ethyl-7-((6-fluoro-3-iodo-1,8-dimethyl-4-oxo-1,4-dihydroquinolin-2-yl)methyl)-4-hydroxy-1,7-dihydro- 3H-pyrano[3,4-c]pyridine-3,8(4H)-dione:*

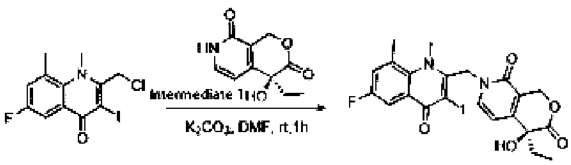


The intermediate obtained from step 6 (7.6 g, 20.8 mmol, 1.0 eq.) was dissolved in anhydrous DMF (80.0 mL). Intermediate 1 (4.4 g, 20.8 mmol, 1.0 eq.) and $K_2CO_3$ (8.6 g, 62.4 mmol, 3.0 eq.) were added sequentially. The reaction mixture was stirred at room temperature for 1 hour, with the completion confirmed by LCMS. The mixture was poured into water and stirred for 5 minutes, then extracted with EA. The aqueous phase was acidified with dilute HCl to precipitate a solid, which was filtered and collected. The crude product was purified by silica gel column chromatography (eluent: PE/EA = 1:9, v/v) to obtain the title compound as a yellow solid (7 g, yield: 62.6%). LCMS (ESI): $[M+H]^+$=539.2.

***Step 8: Preparation of (S)-4-ethyl-8-fluoro-4-hydroxy-10,11-dimethyl-1,12-dihydro-14H-pyrano[3,4:6,7]indolizino[2,1 -b]quinoline-3,6,14(4H,11H)-trione:***

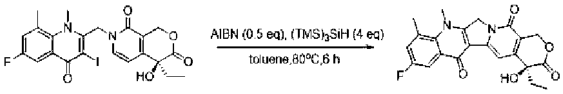


To a dry three-necked flask, the intermediate obtained from step 7 (3.0 g, 5.6 mmol, 1.0 eq.) and AIBN (457.0 mg, 2.8 mmol, 0.5 eq.) were added. The flask was evacuated using an oil pump and then protected with nitrogen. Anhydrous toluene (600 mL) was added, and tris(trimethylsilyl)silane ($(TMS)_3SiH$, 5.5 g, 22.3 mmol, 4.0 eq.) diluted with a small amount of anhydrous toluene was slowly added to the reaction mixture. The reaction mixture was stirred at 80 °C for 6 hours, with the completion confirmed by LCMS. After cooling to room temperature, the mixture was filtered to collect the solid, which was then washed with a mixed solvent of DCM:MeOH = 10:1 (v/v). The solid was collected by filtration again, dissolved in DMSO (4 mL), and precipitated by adding water (1 mL). The resulting solid was filtered and collected to obtain the title compound as a yellow solid (350 mg, yield: 15.3%). LCMS (ESI): $[M+H]^+$=411.1; $^1H$ NMR (400 MHz, DMSO-$d_6$) $\delta$ 7.84 (dd, J = 8.4, 3.3 Hz, 1H), 7.59 (dd, J = 9.0, 3.3 Hz, 1H), 7.23 (s, 1H), 6.36 (s, 1H), 5.35 (t, J = 2.9 Hz, 4H), 4.09 (s, 3H), 2.87 (s, 3H), 1.83 (dd, J = 7.5, 2.7 Hz, 2H), 0.88 (t, J = 7.3 Hz, 3H).

**Example 3: Preparation of (S)-4-ethyl-8-fluoro-4-hydroxy-11-methyl-9-(trifluoromethyl)-1,12-dihydro-14H-pyrano[3,4:6,7]indazolo[2,1 -b]quinoline-3,6,14(4H,11H)-trione:**

***Step 1: Preparation of 6-fluoro-7-iodo-1,2-dimethylquinolin-4(1H)-one:***

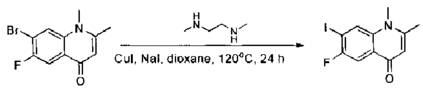


Intermediate 10 (1.4 g, 5.18 mmol, 1.2 eq.) was dissolved in 1,4-dioxane (20 mL). CuI (98.65 mg, 0.52 mmol, 0.1 eq.), NaI (1.553 g, 10.36 mmol, 2.0 eq.) and N,N-dimethylethylenediamine (DME-DMA, 91.32 mg, 1.04 mmol, 0.2 eq.) were added sequentially. The reaction mixture was heated to reflux at 120 °C for 24 hours, with the progress monitored by LCMS. After completion, the reaction solution was spin-dried under reduced pressure. The crude product was purified by flash silica gel column chromatography using a gradient eluent of DCM:MeOH= 100:1 to 10:1, yielding the title compound as a brown solid (1.2 g, yield: 72.97%). LCMS (ESI): $[M+H]^+$ = 317.9; $^1H$-NMR (400 MHz, DMSO-$d_6$) $\delta$ 8.28 (d, J = 4.8 Hz, 1H), 7.80-7.72 (m, 1H), 6.08 (s, 1H), 3.72 (s, 3H), 2.46 (s, 3H).

***Step 2: Preparation of 6-fluoro-1,2-dimethyl-7-(trifluoromethyl)quinolin-4(1H)-one:***

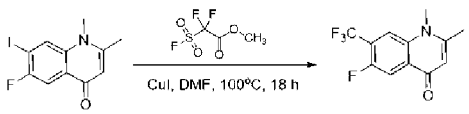


6-Fluoro-7-iodo-1,2-dimethylquinolin-4(1H)-one (1.0 g, 3.15 mmol, 1.0 eq.) was dissolved in anhydrous DMF (10 mL). Under nitrogen protection, CuI (0.9 g, 4.73 mmol, 1.5 eq.) and methyl fluorosulfonyldifluoroacetate (3.03 g, 15.77 mmol, 5.0 eq.) were added sequentially. The reaction progress was monitored by LCMS and TLC until completion. The reaction solution was diluted with DCM (20 mL), filtered to remove insoluble solids, and the filtrate was spin-dried under reduced pressure. The crude product was purified by reversed-phase column chromatography (mobile phase: 0.1% trifluoroacetic acid (TFA)/water/acetonitrile). The target fractions were collected and spin-dried to obtain the title compound as a brown solid (110 mg, yield: 13.46%). LCMS (ESI): $[M+H]^+$ = 259.8; $^1H$ NMR (400 MHz, $CDCl_3$) δ 8.21 (d, J = 10.4 Hz, 1H), 7.76 (d, J = 5.2 Hz, 1H), 6.26 (s, 1H), 3.79 (s, 3H), 2.52 (s, 3H).

***Step 3: Preparation of 3-bromo-6-fluoro-1,2-dimethyl-7-(trifluoromethyl)quinolin-4(1H)-one:***

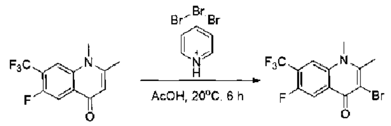


At 20 °C and under nitrogen protection, $PyBr_3$ (185.08 mg, 0.58 mmol, 1.0 eq.) was added to a solution of the intermediate obtained from step 2 (150 mg, 0.58 mmol, 1.0 eq.) in glacial acetic acid (AcOH, 5.0 mL). The reaction mixture was stirred continuously at 20 °C for another 2 hours, with the completion monitored by TLC. The reaction was quenched by adding ice water (30 mL), and the mixture was extracted with EA (3×30 mL). The combined organic layers were washed with saturated brine (3×30mL), dried over anhydrous $Na_2SO_4$, filtered, and concentrated to obtain a crude product, which was purified by flash silica gel column chromatography using a gradient eluent of DCM:MeOH = 40:1 to 20:1, yielding the title compound as a light yellow solid (118 mg, yield: 60.48%). LCMS (ESI): $[M+H]^+$=338.0; $^1H$ NMR (400 MHz, $CDCl_3$) δ 8.22 (d, J = 10.4 Hz, 1H), 7.79 (d, J = 5.2 Hz, 1H), 3.93 (s, 3H), 2.88 (s, 3H).

***Step 4: Preparation of 3-bromo-2-(bromomethyl)-6-fluoro-1-methyl-7-(trifluoromethyl)quinolin-4(1H)-one:***

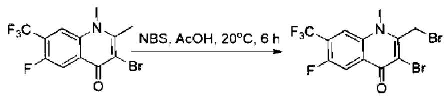


At 20 °C and under nitrogen protection, NBS (52.64 mg, 0.3 mmol, 1.0 eq.) was added to a solution of the intermediate obtained from step 3 (100 mg, 0.3 mmol, 1.0 eq.) in glacial acetic acid (5.0 mL). The mixture was stirred at 20 °C for 6 hours, with the completion monitored by TLC. The reaction was quenched by adding ice water (20 mL), and extracted with EA (3×20 mL). The combined EA layers were washed with saturated brine (3×20 mL), dried over anhydrous $Na_2SO_4$, filtered, and concentrated to obtain a yellow solid. The crude product was purified by silica gel column chromatography (100-200 mesh silica gel) using a gradient eluent of DCM:MeOH = 40:1 to 20:1, affording the title compound as a light yellow solid (105 mg, yield: 83.93%). LCMS (ESI): m/z = 415.8/417.8/419.8 $[M+H]^+$; $^1H$ NMR (400 MHz, $CDCl_3$) δ 8.26 (d, J = 10.0 Hz, 1H), 7.86 (d, J = 5.2 Hz, 1H), 4.90 (s, 2H), 4.02 (s, 3H).

***Step 5: Preparation of (S)-7-((3-bromo-6-fluoro-1-methyl-4-oxo-7-(trifluoromethyl)-1,4-dihydroquinolin-2-yl) methyl)-4-ethyl-4-hydroxy-1,7-dihydro-3H-pyrano[3,4-c]pyridine-3,8(4H)-dione:***

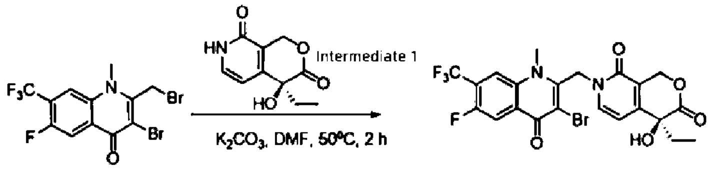


Under nitrogen protection at 25 °C, potassium carbonate (112.69 mg, 0.82 mmol, 4.0 eq.) was added to a solution of the intermediate obtained from step 4 (85 mg, 0.2 mmol, 1.0 eq.) and Intermediate 1 (42.64 mg, 0.2 mmol, 1.0 eq.) in anhydrous DMF (3 mL). The reaction mixture was stirred at 50 °C for 2 hours, with the progress monitored by LCMS. After completion, the reaction mixture was filtered to remove insoluble potassium carbonate, and DMF was removed under reduced pressure. The resulting residue was purified by flash silica gel column chromatography using a gradient eluent of DCM:MeOH=100:1 to 10:1. The target fractions were collected and rotary evaporated under reduced pressure to afford the title compound as a white solid (82 mg, yield: 75%). LCMS (ESI): $[M+H]^+$ = 545.0/547.0; $^1$H NMR (400 MHz, $CDCl_3$) δ 8.28 (d, J = 10.0 Hz, 1H), 7.82 (d, J = 5.2 Hz, 1H), 7.33 (d, J = 7.2 Hz, 1H), 6.61-6.57 (m, 1H), 5.98-5.95 (m, 1H), 5.80-5.75 (m, 1H), 5.65-5.59 (m, 1H), 5.25-5.15 (m, 1H), 3.86 (s, 3H), 3.59 (s, 1H), 1.85-1.75 (m, 2H), 0.98 (t, J = 7.46 Hz, 3H).

***Step 6: Preparation of (S)-4-ethyl-8-fluoro-4-hydroxy-11-methyl-9-(trifluoromethyl)-1,12-dihydro-14H-pyrano [3',4':6,7]indolizino[2,1-b]quinoline-3,6,14(4H,11H)-trione:***

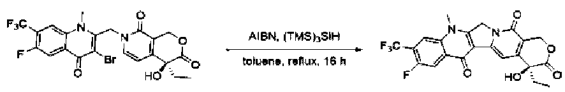


The intermediate obtained from step 5 (100 mg, 0.18 mmol, 1.0 eq.), AIBN (, 6.02 mg, 0.04 mmol, 0.2 eq.) and $(TMS)_3SiH$ (182.42 mg, 0.73 mmol, 4.0 eq.) were suspended in anhydrous toluene (10 mL),which was heated to reflux at 130 °C for 16 hours, with the completion confirmed by LCMS. MeOH (3 mL) was added to quench the reaction, and toluene was removed. The resulting residue was triturated with MeOH (10 mL), filtered to collect a solid (30 mg), and further purified by reversed-phase HPLC (C18 column, mobile phase: 0.1% formic acid in water/acetonitrile). The target fractions were collected and concentrated to afford the title compound as a white solid (4.14 mg). LCMS(ESI):$[M+H]^+$=465.0; $^1$H NMR (400 MHz, $(CD_3)_2SO$) δ 8.33 (d, J = 5.4 Hz, 1H), 8.23 (d, J = 10.4 Hz, 1H), 7.28 (s, 1H), 6.38 (s, 1H), 5.45 (s, 2H), 5.35 (d, J = 3.6 Hz, 2H), 4.02 (s, 3H), 1.85-1.96 (m, 2H), 1.44 (s, 1H), 0.86 (t, J = 7.2 Hz, 3H).

**Example 4: Preparation of (R)-4-ethyl-4-hydroxy-11-methyl-1,12-dihydro-14H-pyrano[3',4':6,7]indolizino[2,1-b] quinoline-3,6,14(4H,11H)-trione:**

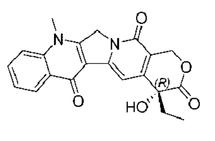

Using the intermediate 3-bromo-2-(bromomethyl)-1-methylquinolin-4(1H)-one from step 3 of Example 1 and intermediate 2 as starting materials, Example 4 was prepared following the methods of steps 4 and 5 of Example 1, resulting in a light yellow solid.LCMS (ESI): $[M+H]^+$ = 379.0; $^1$H-NMR (400 MHz, $(CD_3)_2SO$) δ 8.35 (dd, J = 8.0, 1.2 Hz, 1H), 7.95 (d, J = 8.4 Hz, 1H), 7.90-7.85 (m, 1H), 7.56 (t, J = 7.2 Hz, 1H), 7.29 (s, 1H), 6.35 (s, 1H), 5.40 (s, 2H), 5.35 (d, J = 5.6 Hz, 2H), 3.92 (s, 3H), 1.85-1.79 (m, 1H), 0.87 (t, J = 7.2 Hz, 3H).

**Example 5: Preparation of (S)-4-ethyl-8,9-difluoro-4-hydroxy-11-methyl-1,12-dihydro-14H-pyrano[3',4':6,7]indolizino[2,1-b]quinoline-3,6,14(4H,11H)-trione:**

***Step 1: Preparation of 6,7-difluoro-2-(hydroxymethyl)-1-methylquinolin-4(1H)-one:***

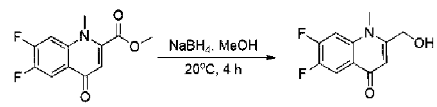


Methyl 6,7-difluoro-1-methyl-4-oxo-1,4-dihydroquinoline-2-carboxylate (500 mg, 1.97 mmol, 1.0 eq.) was added to MeOH (8 mL). Under an ice bath (0 °C), $NaBH_4$ (374 mg, 9.87 mmol, 5.0 eq.) was added portionwise. The reaction mixture was warmed to 20 °C and stirred for 4 hours, with the progress monitored by LCMS. Ice water (5 mL) was added to quench the reaction, and the mixture was extracted with EA (3 × 10 mL). The combined organic layers were washed with saturated sodium chloride solution (brine, 3 × 10 mL), dried over anhydrous sodium sulfate ($Na_2SO_4$), filtered, and concentrated under reduced pressure to afford the title compound as a yellow solid (460 mg, yield not calculated). LCMS (ESI): $[M+H]^+$ = 225.8; $^1$H NMR (400 MHz, $(CD_3)_2SO$) δ 8.02-7.94 (m, 2H), 6.25 (s, 1H), 5.79 (s, 1H), 4.58 (s, 2H), 3.69 (s, 3H).

***Step 2: Preparation of 3-bromo-6,7-difluoro-2-(hydroxymethyl)-1-methylquinolin-4(1H)-one:***

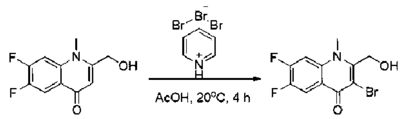


6,7-Difluoro-2-(hydroxymethyl)-1-methylquinolin-4(1H)-one (460 mg, 2.04 mmol, 1.0 eq.) was dissolved in AcOH (8 mL).under nitrogen protection, pyridinium tribromide ($PyBr_3$, 649.6 mg, 2.04 mmol, 1.0 eq.) was added slowly. The reaction mixture was stirred continuously at 20 °C for 2 hours, with the completion monitored by TLC. The reaction was quenched by adding ice water (20 mL), and the resulting mixture was adjusted to neutral pH with saturated sodium bicarbonate solution. The mixture was extracted with ethyl acetate (EA, 3 × 15 mL), and the combined organic layers were washed with saturated sodium chloride solution (brine, 3 × 15 mL), dried over anhydrous sodium sulfate ($Na_2SO_4$), filtered, and concentrated under reduced pressure. The crude product was purified by silica gel column chromatography using a gradient eluent of DCM (DCM):MeOH (MeOH) = 50:1 to 25:1, affording the title compound as a light yellow solid (yield to be determined). LCMS (ESI): $[M + H]^+$ = 303.8/305.8; $^1$H-NMR (400 MHz, $(CD_3)_2SO$) δ (to be supplemented based on actual detection) 8.10-8.05 (m, 2H), 5.82 (s, 1H), 4.60 (s, 2H), 3.72 (s, 3H).

***Step 3: Preparation of 3-bromo-2-(chloromethyl)-6,7-difluoro-1-methylquinolin-4(1H)-one:***

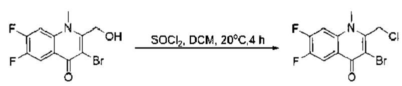


The intermediate obtained from step 2 (237 mg, 0.78 mmol, 1.0 eq.) was dissolved in DCM (8 mL). $SOCl_2$ (464 mg, 3.90 mmol, 5.0 eq.) was added dropwise at 0 °C. The reaction mixture was warmed to rt and stirred for 4 hours, with the progress monitored by LCMS. After completion, the reaction solution was concentrated under reduced pressure to afford the title compound as a white solid (260 mg). $^1$H NMR (400 MHz, $(CD_3)_2SO$) δ 8.17-8.11 (m, 1H), 8.09-8.06 (m, 1H), 5.24 (s, 2H), 3.96 (s, 3H).

***Step 4: Preparation of (S)-7-((3-bromo-6,7-difluoro-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

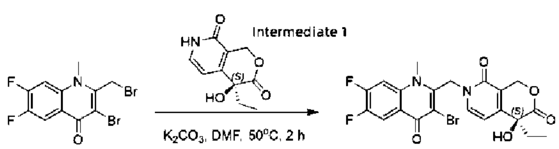

Under nitrogen protection and in an ice-water bath, $K_2CO_3$ (448 mg, 3.24 mmol, 4.0 eq.) was added to an anhydrous DMF (3 mL) solution of the intermediate obtained from step 2 (260 mg, 0.81 mmol, 1.0 eq.) and Intermediate 1 (169 mg, 0.81 mmol, 1.0 eq.). The reaction mixture was stirred at 50 °C for 2 hours, with the completion monitored by LCMS. After the reaction, the mixture was cooled to 0 °C and quenched with water (10 mL), then extracted with EA (3×15 mL). The combined organic layers were washed with saturated brine (3×10 mL), dried over anhydrous $Na_2SO_4$, filtered, and concentrated. The residue was purified by silica gel column chromatography (100-200 mesh silica gel) using a gradient eluent of DCM:MeOH=20:1 to 10:1, affording the title compound as a brown solid (190 mg, yield: 46.9%). LCMS (ESI): $[M+H]^+$=495.0. $^1H$ NMR (400 MHz, $(CD_3)_2SO$) δ 8.14-8.09 (m, 2H), 7.55 (d, J = 7.2 Hz, 1H), 6.45 (d, J = 7.2 Hz, 1H), 6.33 (s, 1H), 5.67-5.55 (m, 2H), 5.30 (s, 2H), 3.80 (s, 3H), 1.81-1.74 (m, 2H), 0.80 (t, J = 7.2 Hz, 3H).

***Step 5: Preparation of (S)-4-ethyl-8,9-difluoro-4-hydroxy-11-methyl-1,12-dihydro-14H-pyran fused [3',4':6,7] indazino [2,1-b] quinoline-3,6,14(4H,11H)-trione:***

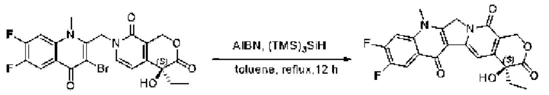


To a solution of the intermediate obtained from step 4 (150 mg, 0.3 mmol, 1.0 eq.) in anhydrous toluene (20 mL) were added AIBN (25 mg, 0.15 mmol, 0.5 eq.)and (TMS)3SiH (301 mg, 1.21 mmol, 4.0 eq.). the reaction mixture was heated to reflux at 130°C for 12 hours. LCMS indicated the reaction was completed. Quenched the reaction with MeOH (3 mL) and removed toluene under reduced pressure. Purified by reverse-phase HPLC (C18, 0.1% TFA in water, acetonitrile) to obtain the title compound (10.44 mg, yield: 8.4%) as a white solid. LCMS: m/z (ESI) $(M+H)(^+)$ = 415.2; (1)H NMR (400 MHz, $(CD_3)_2SO$) δ 8.19-8.15 (m, 2H), 7.26 (s, 1H), 6.36 (s, 1H), 5.41 - 5.39 (m, 2H), 5.35 -5.34 (m, 2H), 3.90 (s, 3H), 1.83-1.81 (m, 2H), 0.86 (t, *J* = 7.2 Hz, 3H).

**Example 6: Preparation of (S)-4-ethyl-8-fluoro-4-hydroxy-11-methyl-1,12-dihydro-14H-pyrano[3',4':6,7]indazino[2,1-b]quinoline-3,6,14(4H,11H)-trione:**

***Step 1: Preparation of 6-fluoro-2-(hydroxymethyl)-1-methylquinolin-4(1H)-one:***

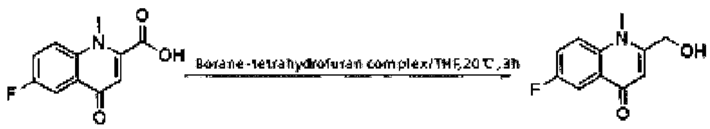


6-Fluoro-1-methyl-4-oxo-1,4-dihydroquinoline-2-carboxylic acid (800 mg, 3.4 mmol, 1.0 eq.) was dissolved in anhydrous THF (20 mL). To the ice-cooled stirred solution was added 1 M borane-THF solution (3.74 mL, 3.74 mmol, 1.1 eq.). The mixture was stirred at 0 °C for 2 h, then warmed to 20 °C and stirred for an additional 3 h. The reaction was quenched with ice water, the solvent was removed, and the residue was purified by column chromatography (EA:PE = 1:1) to afford the title compound (462 mg, 65% yield) as a white solid. LCMS (ESI) [M+1]=208.3; $^1H$ NMR (400 MHz, $CD_3OD$) δ 7.98 - 7.93 (m, 2H), 7.62 (ddd, J = 9.4, 7.7, 3.2 Hz, 1H), 6.53 (s, 1H), 4.76 (s, 2H), 3.92 (s, 3H).

***Step 2: Preparation of 3-bromo-6-fluoro-2-(hydroxymethyl)-1-methylquinolin-4(1H)-one:***

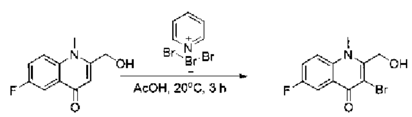


Under nitrogen protection, pyridinium tribromide (870.55 mg, 2.72 mmol, 1.2 eq.) was added to a solution of the intermediate obtained from step 1 (470 mg, 2.27 mmol, 1.0 eq.) in AcOH (10 mL) at 20 °C. The mixture was stirred at 20 °C for 3 h, which was monitored by LCMS until completion. The reaction was quenched with ice water (10 mL) and extracted

with EA (3×30 mL). The combined organic layers were washed with saturated brine (3×20mL), dried over anhydrous $Na_2SO_4$, and purified by flash silica gel column chromatography (DCM/MeOH=100:1 to 15:1) to afford the title compound (210 mg, 32.34% yield) as a pale yellow solid. LCMS (ESI) [M+1]=288.0; $^1$H NMR (400 MHz, $(CD_3)_2SO$) δ 8.01-7.97 (m, 1H), 7.88-7.85 (m, 1H), 7.75-7.69 (m, 1H), 5.97 (t, J = 5.6 Hz, 1H), 5.03 (d, J = 5.6 Hz, 2H), 3.99 (s, 3H).

***Step 3: Preparation of 3-bromo-2-(chloromethyl)-6-fluoro-1-methylquinolin-4(1H)-one:***

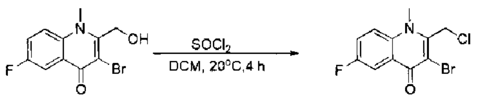


At 0°C under nitrogen protection, the intermediate obtained from step 2 (210 mg, 0.73 mmol, 1 eq.) was dissolved in DCM (8 mL), and $SOCl_2$ (436.63 mg, 3.67 mmol, 5 eq.) was added dropwise slowly at 0°C. The reaction was then stirred at 20°C for 4 hours. LCMS monitoring indicated the reaction was completed. The reaction mixture was concentrated to yield the title compound (280 mg, crude product) as a pale yellow solid. LCMS (ESI) [M+H]=306.0; $^1$H NMR (400 MHz, $(CD_3)_2SO$) δ 8.06-8.03 (m, 1H), 7.89-7.86 (m, 1H), 7.78-7.73 (m, 1H), 5.26 (s, 2H), 4.00 (s, 3H).

***Step 4: Preparation of (S)-7-((3-bromo-6-fluoro-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

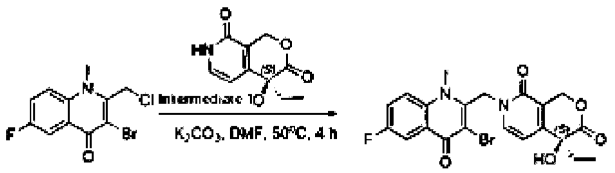


Under nitrogen at 20 °C, $K_2CO_3$ (508.29 mg, 3.68 mmol, 4.0 eq.) was added to a DMF (8 mL) solution of the intermediate obtained from step 3 (280 mg, 0.92 mmol, 1.0 eq.) and Intermediate 1 (173.11 mg, 0.83 mmol, 0.9 eq.). The mixture was stirred at 50 °C for 4 h (LCMS monitored). After completion, the mixture was filtered, DMF was evaporated, and the residue was purified by silica gel column chromatography (DCM:MeOH=100:1 to 10:1) to give the title compound as a light yellow solid (300 mg, 68.37% yield). LCMS (ESI): $[M+H]^+$=479.0; $^1$H NMR (400 MHz, $(CD_3)_2SO$) δ 8.02-7.98 (m, 1H), 7.91-7.88 (m, 1H), 7.77-7.72 (m, 1H), 7.54-7.52 (m, 1H), 6.44 (d, J = 7.2 Hz, 1H), 6.32 (s, 1H), 5.69-5.56 (m, 2H), 5.30 (s, 2H), 3.84 (s, 3H), 1.82-1.71 (m, 2H), 0.79 (t, J = 7.2 Hz, 3H).

***Step 5: Preparation of (S)-4-ethyl-8-fluoro-4-hydroxy-11-methyl-1,12-dihydro-14H-pyran[3',4':6,7]indazino[2,1-b]quinoline-3,6,14(4H,11H)-trione:***

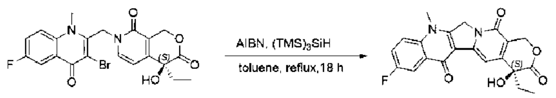


The compound obtained in step 4 (130 mg, 0.27 mmol, 1.0 eq.), AIBN (22.36 mg, 0.14 mmol, 0.5 eq.), and $(TMS)_3SiH$ (338.65 mg, 1.36 mmol, 5.0 eq.) were suspended in anhydrous toluene (15 mL). The reaction mixture was heated under reflux at 130 °C for 18 hours, and LCMS analysis indicated the reaction completion. MeOH (5 mL) was added to quench the reaction, and toluene was removed under reduced pressure. The residue was triturated with MeOH (5 mL) and filtered to obtain a solid (150 mg). The target compound was purified by preparative reversed-phase HPLC (C18 column, mobile phase: 0.1% formic acid in water/acetonitrile), yielding the title compound as a pale yellow solid (11 mg, yield: 10%). LCMS (ESI): $[M+H]^+$= 397.2; $^1$H NMR (400 MHz, $(CD_3)_2SO$) δ 8.06-8.02 (m, 1H), 7.99-7.96 (m, 1H), 7.80-7.75 (m, 1H), 7.27 (s, 1H), 6.35 (s, 1H), 5.40 (d, J = 4.8 Hz, 2H), 5.34 (d, J = 5.2 Hz, 2H), 3.93 (s, 3H), 1.85-1.79 (m, 2H), 0.87 (t, J = 7.2 Hz, 3H).

Example 7: Preparation of (S)-4-ethyl-7-fluoro-4-hydroxy-10,11-dimethyl-1,12-dihydro-14H-pyrano[3,4:6,7]indolizino [2,1 -b]quinoline-3,6,14(4H,11H)-trione:

*Step 1: Preparation of 2-((5-fluoro-2-methylphenyl)amino)maleic acid dimethyl ester:*

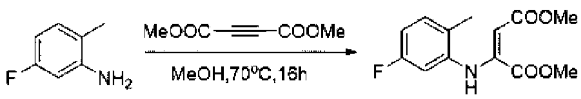


5-Fluoro-2-methylaniline (30.0 g, 0.24 mol, 1.0 eq.) and dimethyl butynedioate (34.1 g, 0.24 mol, 1.0 eq.) were dissolved in MeOH (300 mL). The mixture was stirred at 70 °C for 16 hours, with reaction progress monitored by LCMS until completion. The solvent was removed, and the residue was purified by flash silica gel column chromatography (100-200 mesh silica gel) using a gradient eluent of PE:EA = 100:0 to 95:5 (v/v). The title compound was obtained as a yellow solid (55.0 g, yield: 87.6%). LCMS (ESI): $[M+H]^+$ = 268.2.

***Step 2: Preparation of 2-((5-fluoro-2-methylphenyl)(methyl)amino)maleic acid dimethyl ester:***

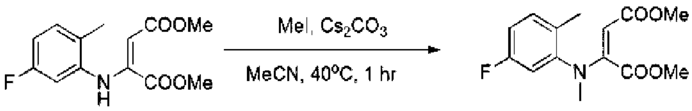


The intermediate obtained from step 1 (20.0 g, 0.07 mol, 1.0 eq.) was dissolved in acetonitrile (200 mL). $Cs_2CO_3$ (73.2 g, 0.22 mol, 3.0 eq.) was added, followed by the dropwise addition of methyl iodide ($CH_3I$, 10.6 g, 0.07 mol, 1.0 eq.) under nitrogen protection. The reaction mixture was stirred at 25 °C for 2 hours, with completion monitored by LCMS. The mixture was filtered, and the filtrate was collected. The solvent was evaporated, and the residue was purified by silica gel column chromatography (100-200 mesh silica gel) using a gradient eluent of PE:EA= 90:10 to 80:20 (v/v). The title compound was obtained as a yellow oil (11.2 g, yield: 53.2%). LCMS (ESI): $[M+H]^+$ = 282.2.

***Step 3: Preparation of methyl 5-fluoro-1,8-dimethyl-4-oxo-1,4-dihydroquinoline-2-carboxylate:***

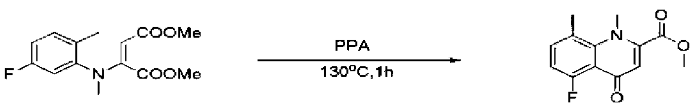


A mixture of the intermediate obtained from step 2 (3.0 g, 0.01 mol, 1.0 eq.) and polyphosphoric acid (8.0 g, 0.1 mol, 10.0 eq.) was heated to 130 °C and stirred for 1 hour. Reaction completion was monitored by LCMS, after which the mixture was cooled to room temperature. It was poured into 300 mL of ice-cold saturated $NaHCO_3$ solution, and the resulting solid was collected by filtration. The solid was purified by silica gel column chromatography (100-200 mesh silica gel) using a gradient eluent of PE:EA= 100:0 to 50:50 (v/v), affording the title compound as a pale yellow solid (987 mg, yield: 37.13%). LCMS (ESI): $[M+H]^+$= 250.1; $^1H$ NMR (400 MHz, $CDCl_3$) δ 7.41 (dd, J = 8.3, 5.7 Hz, 1H), 6.93 (dd, J = 10.8, 8.3 Hz, 1H), 6.80 (s, 1H), 3.98 (s, 3H), 3.67 (s, 3H), 2.58 (s, 3H).

***Step 4: Preparation of 5-fluoro-2-(hydroxymethyl)-1,8-dimethylquinolin-4(1H)-one:***

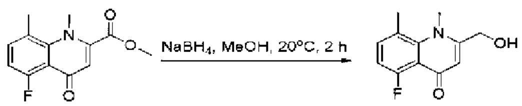

Under nitrogen protection and an ice bath (0 °C), $NaBH_4$ (210.37 mg, 5.56 mmol, 3.0 eq.) was added to a MeOH (8 mL) solution of the intermediate obtained from step 3 (462 mg, 1.85 mmol, 1.0 eq.). The mixture was stirred at 20 °C overnight, and LCMS analysis confirmed complete consumption of the starting material. The reaction mixture was concentrated under reduced pressure, and the residue was purified by silica gel column chromatography (100-200 mesh silica gel) using DCM:MeOH = 10:1 (v/v) as the eluent. Collected fractions were concentrated to afford the title compound as a white solid (300 mg, yield: 73.3%). LCMS(ESI): $[M+H]^+$=222.2; $^1H$ NMR (400 MHz, DMSO-$d_6$) δ 7.53-7.41 (m, 1H), 7.05-6.90 (m, 1H), 6.13 (s, 1H), 5.66 (s, 1H), 4.53 (d, J = 4.8 Hz, 2H), 3.65 (s, 3H), 2.55 (s, 3H).

***Step 5: Preparation of 3-bromo-5-fluoro-2-(hydroxymethyl)-1,8-dimethylquinolin-4(1H)-one:***

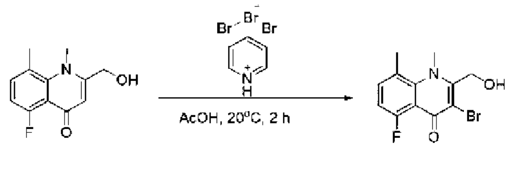


Under nitrogen protection, tripyridyl tribromide (475.04 mg, 1.49 mmol, 1.0 eq.) was added to a AcOH (5mL) solution of the intermediate obtained from step 4 (328.6 mg, 1.49 mmol, 1.0 eq.). The reaction mixture was stirred at 20 °C for 2 hours, then concentrated under reduced pressure. The residue was purified by flash silica gel column chromatography using a gradient eluent of DCM:MeOH = 50:1 to 10:1 (v/v). Collected fractions were concentrated under reduced pressure to afford the title compound as a white solid (371 mg, yield: 82.96%). LCMS (ESI): $[M+H]^+$=300.0. $^1H$-NMR (400 MHz, DMSO-$d_6$) δ 7.64-7.47 (m, 1H), 7.15-6.99 (m, 1H), 5.84 (t, J = 5.2 Hz, 1H), 4.89 (d, J = 5.2 Hz, 2H), 3.82 (s, 3H), 2.55 (s, 3H).

***Step 6: Preparation of 3-bromo-2-(bromomethyl)-5-fluoro-1,8-dimethylquinolin-4(1H)-one:***

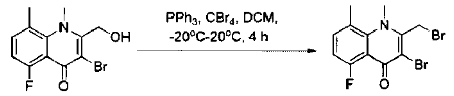


Under nitrogen at -20 °C, a solution of the intermediate obtained from step 5 (371 mg, 1.24 mmol, 1.0 eq.) and $CBr_4$ (819.9 mg, 2.47 mmol, 2.0 eq.) in DCM (10 mL)was treated dropwise with $PPh_3$ (680.89 mg, 2.6 mmol, 2.1 eq.) in DCM. The mixture was stirred at -20 °C for 10 min, then at 20 °C for 4 h (LCMS monitored). After concentration, the crude product was purified by flash silica gel column chromatography (DCM:MeOH = 20:1) to afford the title compound as a white solid (380 mg, 85% yield). LCMS (ESI): $[M+H]^+$ = 361.8; $^1H$ NMR (400 MHz, $CDCl_3$) δ 7.65-7.57 (m, 1H), 7.19-7.11 (m, 1H), 5.34 (s, 2H), 4.01 (s, 3H), 2.74 (s, 3H); $^{19}F$-NMR (376 MHz, DMSO-$d_6$) δ - 115.80 (s).

***Step 7: Preparation of (S)-7-((3-bromo-5-fluoro-1,8-dimethyl-4-oxo-1,4-dihydroquinolin-2-yl)methyl)-4-ethyl-4-hydroxy-1,7-dihydro-3H-pyrano[3,4-c]pyridine-3,8(4H)-dione:***

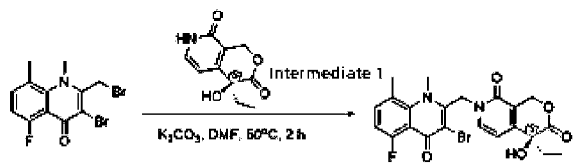


Under nitrogen, $K_2CO_3$ (699.97 mg, 5.06 mmol, 2.08 eq.) and Intermediate 1 (238.38 mg, 1.14 mmol, 0.47 eq.) were added to a solution of the intermediate obtained from step 6 (875.6 mg, 2.43 mmol, 1.0 eq.) in DMF (15 mL). The mixture was stirred at 50 °C for 2 h (LCMS monitored to complete). After concentration and filtration, the organic phase was dried and re-concentrated. The residue was purified by silica gel column chromatography (100-200 mesh) with a DCM:MeOH gradient (100: 1 to 20:1) to afford the title compound as a pale yellow solid (427.9 mg, 36% yield).LCMS (ESI): $[M+H]^+$ = 490.6; $^1H$ NMR (400 MHz, $CDCl_3$) δ 7.44-7.36 (m, 2H), 7.01-6.93 (m, 1H), 6.57 (d, J = 7.2 Hz, 1H), 5.78 (d, J

= 16.0 Hz, 1H), 5.66-5.54 (m, 2H), 5.21 (d, J = 16.4 Hz, 1H), 3.58 (s, 3H), 2.40 (s, 3H), 1.90-1.74 (m, 2H), 0.98 (t, J = 7.6 Hz, 3H).

*Step 8: Preparation of (S)-4-ethyl-7-fluoro-4-hydroxy-10,11-dimethyl-1,12-dihydro-14H-pyrano[3,4:6,7]indolizino[2,1 -b]quinoline-3,6,14(4H,11H)-trione:*

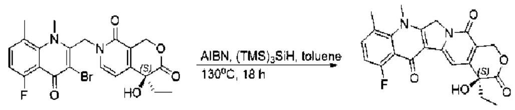


Under nitrogen, the intermediate obtained from step 6 (300 mg, 0.61 mmol, 1.0 eq.) and AIBN (40.11 mg, 0.24 mmol, 0.4 eq.) were degassed (evacuation-$N_2$, 3 cycles). Anhydrous toluene (10 mL) and $(TMS)_3SiH$ (607.34 mg, 2.44 mmol, 4.0 eq.) were added, and the mixture was heated at 130 °C for 18 h (LCMS monitored). The reaction was quenched with MeOH (5 mL), and toluene was removed in vacuo. The crude product was purified by preparative HPLC (C18, $H_2O$/ACN with 0.1% TFA) to afford the title compound as a white solid (13 mg, 5% yield). LCMS (ESI): $[M+H]^+$=410.8; $^1H$ NMR (400 MHz, DMSO-$d_6$) δ 7.65-7.57 (m, 1H), 7.24 (s, 1H), 7.19-7.11 (m, 1H), 6.33 (s, 1H), 5.42-5.26 (m, 4H), 4.01 (s, 3H), 2.74 (s, 3H), 1.89-1.74 (m, 2H), 0.86 (t, J = 7.2 Hz, 3H); $^{19}F$-NMR (376 MHz, DMSO-$d_6$) δ -115.80 (s).

Example 8: Preparation of (S)-9-amino-4-ethyl-8-fluoro-4-hydroxy-11-methyl-1,12-dihydro-14H-pyranob[3,4:6,7]indolezine -and[2,1-b]quinoline-3,6,14 (4H,11H)-trione:

*Step 1: Preparation of (6-fluoro-1,2-dimethyl-4-carbonyl-1,4-dihydroquinoline-7-yl)tert-butyl carbamate:*

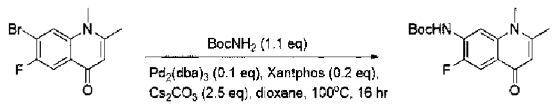


Intermediate 10 (935 mg, 3.48 mmol, 1.0 eq.), tert-butyl carbamate (448 mg, 3.83 mmol, 1.1 eq.), $Cs_2CO_3$ (2.83 g, 8.70 mmol, 2.5 eq.), $Pd_2(dba)_3$ (402 mg, 0.696 mmol, 0.2 eq.) and Xantphos (204 mg, 0.348 mmol, 0.1 eq.) were sequentially dissolved in 1,4-dioxane (10 mL). Under nitrogen protection, the mixture was heated to 100 °C and stirred for 16 hours. Reaction completion was monitored by LCMS, after which the mixture was filtered and the filtrate was concentrated under reduced pressure to afford a crude product, which was purified by silica gel column chromatography using a gradient eluent of DCM:MeOH= 100:0 to 10:1, yielding the title compound as a light brown solid (400 mg, yield: 37.7%). LCMS (ESI): $[M+H]^+$=307.2.

***Step 2: Preparation of (3-bromo-6-fluoro-1,2-dimethyl-4-oxo-1,4-dihydroquinolin-7-yl) carbamate tert-butyl ester:***

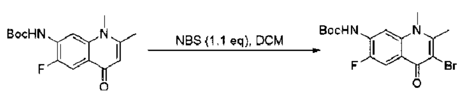


The intermediate obtained from step 1 (400 mg, 1.31 mmol, 1.0 eq.) was dissolved in DCM (5 mL). NBS (256 mg, 1.44 mmol, 1.1 eq.) was added, and the reaction mixture was stirred at room temperature for 16 hours. LCMS analysis confirmed reaction completion. The mixture was concentrated under reduced pressure to obtain a crude product, which was purified by silica gel column chromatography using a gradient eluent of PE:EA=20:1 to 5:1. The title compound was obtained as a brown solid (413 mg, yield: 82.1%). LCMS (ESI): $[M+H]^+$=385.2.

***Step 3: Preparation of (3-bromo-2-(bromomethyl)-6-fluoro-1-methyl-4-oxo-1,4-dihydroquinolin-7-yl)carbamic acid tert-butyl ester:***

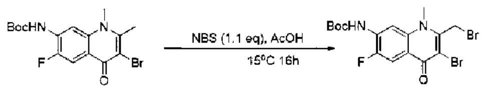


The intermediate obtained from step 2 (285 mg, 0.740 mmol, 1.0 eq.) was dissolved in AcOH (3 mL). NBS (145 mg, 0.814 mmol, 1.1 eq.) was added, and the reaction mixture was stirred at room temperature for 16 hours. Reaction completion was confirmed by LCMS. The mixture was poured into 30 mL of ice-cold saturated sodium bicarbonate solution and extracted with EA (20 mL × 3). The combined organic layers were dried over anhydrous $Na_2SO_4$, filtered, and concentrated to afford a crude product. The crude product was purified by silica gel column chromatography using a gradient eluent of PE:EA = 5:1 to 1:1, yielding the title compound as a brown solid (130 mg, yield: 37.8%). LCMS (ESI): $[M+H]^+$=463.1.

***Step 4: Preparation of (S)-(3-bromo-2-((4-ethyl-4-hydroxy-3,8-dioxo-4,8-dihydro-1H-pyrano[3,4-c]pyridin-7(3H)-yl)methyl)-6-fluoro-1-methyl-4-oxo-1,4-dihydroquinolin-7-yl)carbamate tert-butyl ester:***

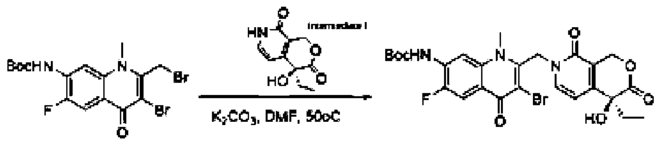


The intermediate obtained from step 3 (130 mg, 0.280 mmol, 1.0 eq.) and Intermediate 1 (80.0 mg, 0.310 mmol, 1.1 eq.) were dissolved in DMF (2 mL). $K_2CO_3$ (65.0 mg, 0.560 mmol, 2.0 eq.) was added, and the mixture was heated to 50 °C and stirred for 2 hours. Reaction completion was confirmed by LCMS. The mixture was filtered, and the filtrate was poured into 20 mL of ice-cold saturated ammonium chloride solution, followed by extraction with EA (10 mL × 3). The combined organic layers were dried over anhydrous $Na_2SO_4$, filtered, and the filtrate was concentrated to afford a crude product. The crude product was purified by silica gel column chromatography using a gradient eluent of DCM:MeOH=100:0 to 5:1, yielding the title compound as a light brown solid (85 mg, yield: 51.2%). LCMS(ESI):$[M+H]^+$=592.2.

***Step 5: Preparation of (S)-(4-ethyl-8-fluoro-4-hydroxy-11-methyl-3,6,14-trioxo-3,4,6,11,12,14-hexahydro-1H-pyrano[3',4':6,7]indazino[2,1-b]quinolin-9-yl) carbamate:***

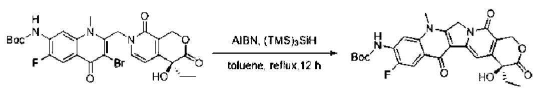


Under nitrogen protection, in a 25 mL three-necked flask, AIBN (, 9.7 mg, 0.06 mmol, 0.5 eq.) and $(TMS)_3SiH$ (117.53 mg, 0.47 mmol, 4.0 eq.) were added to a toluene (5 mL) solution of the intermediate obtained in Step 4 (70 mg, 0.12 mmol, 1.0 eq.). The reaction was conducted at 120 °C for 12 hours. LCMS monitoring showed that approximately 60% of the starting material remained, indicating the reaction proceeded successfully. The reaction mixture was quenched with MeOH (2.0 mL) and concentrated under reduced pressure to afford a crude product, which was subjected to preparative HPLC purification. The collected fractions were lyophilized to give the title compound (6 mg, yield: 9.78%).

***Step 6: Preparation of (S)-9-amino-4-ethyl-8-fluoro-4-hydroxy-11-methyl-1,12-dihydro-14H-pyranof3',4':6,7]indolizinof2,1-b]quinoline-3,6,14(4H,11H)-trione:***

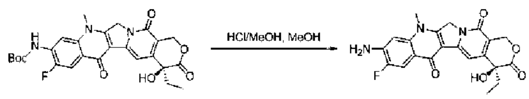


Under nitrogen, the intermediate obtained from step 5 (6.0 mg, 0.01 mmol, 1.0 eq.) was stirred in 4 M HCl in MeOH at 25 °C for 1 h. LCMS monitoring showed the product as the major component. After completion, the mixture was concentrated, then treated with ACN (2 mL) and water (6.0 mL), mixed well, and lyophilized to afford the title compound (2.14 mg, 47.78% yield). LCMS (ESI): $[M+H]^+$ = 412.2.

Example 9: Preparation of (S)-4-Ethyl-7,8-difluoro-4-hydroxy-10,11-dimethyl-1,12-dihydro-14H-pyrano[3,4:6,7]indazolo[2,1 -b]quinoline-3,6,14(4H,11H)-trione:

*Step 1: Preparation of 2-((4, 5-difluoro-2-methylphenyl)amino)maleic acid dimethyl ester:*

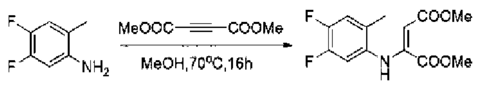


4,5-Difluoro-2-methylaniline (3 g, 21.0 mmol, 1.0 eq.) and dimethyl acetylenedicarboxylate (3 g, 21.1 mmol, 1.01 eq.) were dissolved in MeOH (30 mL). The mixture was stirred at 70 °C for 16 hours, with reaction completion monitored by LCMS. The solvent was evaporated under reduced pressure, and the residue was purified by flash silica gel column chromatography (100-200 mesh silica gel) using a gradient eluent of PE: EA= 100:0 to 95:5. The title compound was obtained as a yellow solid (4 g, yield: 66.9%). LCMS (ESI): $[M+H]^+$ = 286.08.

***Step 2: Preparation of 2-((4,5-difluoro-2-methylphenyl)(methyl)amino)maleic acid dimethyl ester:***

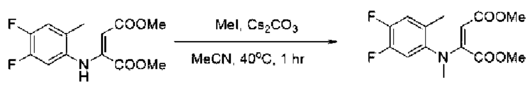


The intermediate obtained from step 1 (3 g, 12.6 mmol, 1.0 eq.) was dissolved in acetonitrile (40 mL). $Cs_2CO_3$ (12.4 g, 37.9 mmol, 3.0 eq.) was added, followed by the addition of methyl iodide (2.69 g, 19.0 mmol, 1.5 eq.) under a nitrogen atmosphere. The reaction mixture was stirred at 40 °C for 1 hour, with completion monitored by LCMS. The mixture was filtered, and the filtrate was collected. The solvent was evaporated, and the residue was purified by silica gel column chromatography (100-200 mesh silica gel) using a gradient eluent of PE:EA= 90:10 to 80:20. The title compound was obtained as a yellow oil (2 g, yield: 52.9%). LCMS(ESI):$[M+H]^+$=300.2.

***Step 3: Preparation of 5,6-difluoro-1,8-dimethyl-4-oxo-1,4-dihydroquinoline-2-carboxylate:***

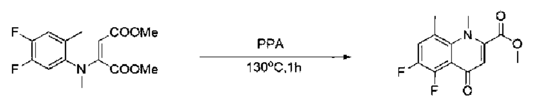


The intermediate obtained from step 2 (1.0 g, 3.34 mmol, 1.0 eq.) was mixed with polyphosphoric acid (PPA, 8.0 g, 0.1 mol, 10.0 eq.). The mixture was heated to 130 °C and stirred for 1 hour, with reaction completion monitored by LCMS. After cooling to room temperature, the mixture was poured into 300 mL of ice-cold saturated sodium bicarbonate solution and extracted with EA. The combined organic phases were dried over anhydrous $Na_2SO_4$ and concentrated. The residue was purified by silica gel column chromatography (100-200 mesh silica gel) using a gradient eluent of PE:EA = 100:0 to 50:50, affording the title compound as a pale yellow solid (100 mg, yield: 11.8%). LCMS(ESI):$[M+H]^+$=268.2; $^1$H NMR (400 MHz, MeOH-$d_4$) $\delta$ 7.63-7.56 (m, 1H), 6.65 (s, 1H), 3.99 (s, 3H), 3.76 (s, 3H), 2.64 (s, 3H).

***Step 4: Preparation of 5,6-difluoro-2-(hydroxymethyl)-1,8-dimethylquinolin-4(1H)-one:***

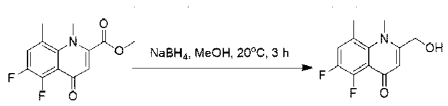


The intermediate obtained from step 3 (120 mg, 0.45 mmol, 1.0 eq.) was added to MeOH (20 mL). $NaBH_4$ (51 mg, 1.35 mmol, 3.0 eq.) was added at 0 °C, and the mixture was stirred at 20 °C for 3 hours, with completion monitored by LCMS. The reaction was quenched with 2 mL of ice water, and the mixture was extracted with EA (10 mL×3). The combined EA layers were washed with saturated brine (10mL×3), dried over anhydrous $Na_2SO_4$, and concentrated. The title compound was obtained as a yellow solid (100 mg, yield: 92.5%). LCMS(ESI): $[M+H]^+$=240.0; $^1$H NMR (400 MHz, $(CD_3)_2SO$) δ 7.67-7.62 (m, 1H), 6.13 (s, 1H), 4.53 (s, 2H), 3.65 (s, 3H), 2.57 (s, 3H).

***Step 5: Preparation of 3-bromo-5,6-difluoro-2-(hydroxymethyl)-1,8-dimethylquinolin-4(1H)-one:***

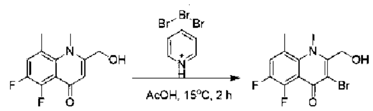


The intermediate obtained from step 4 (280 mg, 1.17 mmol, 1.0 eq.) was dissolved in AcOH (8 mL). Pyridinium tribromide (374 mg, 1.17 mmol, 1.0 eq.) was added, and the reaction mixture was stirred at 15 °C for 2 hours. Reaction completion was monitored by LCMS. The solvent was concentrated, and the residue was purified by flash silica gel column chromatography using a gradient eluent of DCM:MeOH=20:1 to 10:1. The title compound was obtained as a light yellow solid (239 mg, yield: 64.21%). LCMS (ESI):$[M+H]^+$=319.9; $^1$H-NMR (400 MHz, $(CD_3)_2SO$) δ 7.75 (dd, J = 11.6, 8.4 Hz, 1H), 5.86 (t, J = 5.2 Hz, 1H), 4.89 (d, J = 5.6 Hz, 2H), 3.82 (s, 3H), 2.57 (s, 3H).

***Step 6: Preparation of 3-bromo-2-(bromoethyl)-5,6-difluoro-1,8-dimethylquinolin-4(1H)-one:***

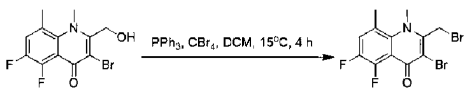


The intermediate obtained from step 5 (239 mg, 0.75 mmol, 1.0 eq.) and $CBr_4$ (498.32 mg, 1.5 mmol, 2.0 eq.) were dissolved in DCM. At -20 °C, $PPh_3$ (414 mg, 1.58 mmol, 2.1 eq.) was added, and the reaction mixture was stirred at 15 °C for 4 hours. Reaction completion was monitored by LCMS. The solid was filtered off, and the filtrate was concentrated under reduced pressure. The residue was purified by flash silica gel column chromatography using a gradient eluent of DCM:MeOH=20:1 to 10:1. The title compound was obtained as a white solid (233 mg, yield: 81.4%). LCMS(ESI): $[M+H]^+$=382.0; $^1$H NMR (400 MHz, $(CD_3)_2SO$) δ 7.77 (dd, J = 11.2, 8.4 Hz, 1H), 4.92 (s, 2H), 3.83 (s, 3H), 2.57 (s, 3H).

***Step 7: Preparation of (S)-7-((3-bromo-5,6-difluoro-1,8-dimethyl-4-oxo-1,4-dihydroquinolin-2-yl)methyl)-4-ethyl-4-hydroxy-1,7-dihydro-3H-pyrano[3,4-c]pyridine-3,8(4H)-dione:***

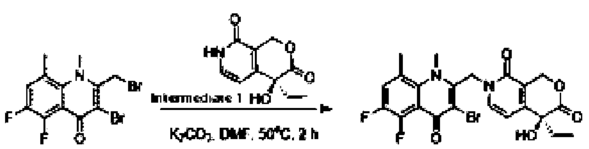


Under $N_2$ atmosphere and ice bath, $K_2CO_3$ (316 mg, 2.29 mmol, 4.0 eq.) was added to a DMF (5 mL) solution of

the intermediate obtained from step 6 (218 mg, 0.57 mmol, 1.0 eq.) and Intermediate 1 (108 mg, 0.51 mmol, 0.9 eq.). The mixture was warmed to 50 °C and stirred for 2 h. After LCMS-confirmed completion, the mixture was cooled to 0 °C, quenched with water (10 mL), and extracted with EA (3 × 15 mL). The combined organics were washed with saturated brine (3 × 10 mL), dried over anhydrous $Na_2SO_4$, filtered, and concentrated. The residue was mixed with silica gel and purified by flash column chromatography (DCM:MeOH gradient, 20:1 to 10:1) to afford the title compound as a light yellow solid (230 mg, 79.23% yield). LCMS (ESI): $[M+H]^+$=509.0; $^1$H NMR (400 MHz, $CDCl_3$) δ 7.42 (d, J = 7.2 Hz, 1H), 7.31 (dd, J = 10.4, 8.0 Hz, 1H), 6.58 (d, J = 7.2 Hz, 1H), 5.77 (d, J = 16.0 Hz, 1H), 5.62-5.54 (m, 2H), 5.20 (d, J = 16.4 Hz, 1H), 3.59 (s, 4H), 2.41 (s, 3H), 1.85-1.78 (m, 2H), 0.98 (t, J = 7.6 Hz, 3H).

***Step 8: Preparation of (S)-4-ethyl-7,8-difluoro-4-hydroxy-10,11-dimethyl-1,12-dihydro-14H-pyran[3',4':6,7]indolizino[2,1-b]quinoline-3,6,14(4H,11H)-trione:***

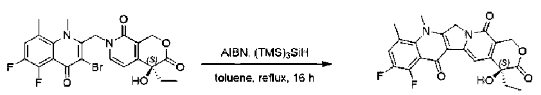


The intermediate obtained from step 7 (100 mg, 0.2 mmol, 1.0 eq.), AIBN (16.12 mg, 0.1 mmol, 0.5 eq.) and $(TMS)_3SiH$ (195.3 mg, 0.79 mmol, 4.0 eq.) were dissolved in anhydrous toluene (5 mL). The reaction solution was heated to 130 °C and refluxed for 16 hours, with reaction completion confirmed by LCMS. MeOH (3 mL) was added to quench the reaction, and toluene was removed by vacuum distillation. The crude product was purified by reversed-phase preparative HPLC (C18 column, mobile phase: 0.1% trifluoroacetic acid (TFA) in water/acetonitrile), yielding the title compound as a brown solid (10.8 mg). LCMS (ESI): $[M+H]^+$=429.2; $^1$H NMR (400 MHz, $(CD_3)_2SO$) δ 7.81 (dd, J = 11.2, 8.4 Hz, 1H), 7.22 (s, 1H), 6.34 (s, 1H), 5.39-5.29 (m, 4H), 4.00 (s, 3H), 2.76 (s, 3H), 1.85-1.78 (m, 2H), 0.86 (t, J = 7.2 Hz, 3H).

**Example 10: Preparation of (S)-4-ethyl-8-fluoro-4-hydroxy-10-methoxy-11-methyl-1,12-dihydro-14H-pyrano [3,4:6,7]indolizino[2,1 -b]quinoline-3,6,14 (4H,11H)-trione:**

***Step 1: Preparation of 6-fluoro-2-(hydroxymethyl)-8-methoxy-1-methylquinolin-4(1H)-one:***

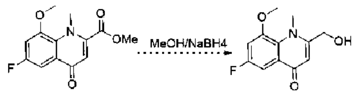


Intermediate 3 (120 mg, 0.45 mmol, 1.0 eq.) was added to MeOH (20 mL). $NaBH_4$ (51 mg, 1.35 mmol, 3.0 eq.) was added at 0 °C, and the reaction mixture was stirred at 20 °C for 3 hours, with completion monitored by LCMS. The reaction was quenched by pouring the mixture into 2 mL of ice-cold saturated $NH_4Cl$ solution, then extracted with EA (10 mL×3). The combined organic layers were washed with saturated brine (10 mL×3), dried over anhydrous $Na_2SO_4$, and concentrated. The title compound was obtained as a yellow solid (100 mg, yield: 92.5%). LCMS (ESI): $[M+H]^+$=238.1.

***Step 2: Preparation of 6-fluoro-2-(hydroxymethyl)-3-iodo-8-methoxy-1-methylquinolin-4(1H)-one:***

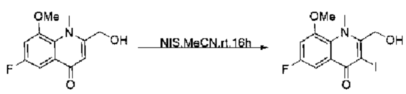


The intermediate obtained from step 1 (2.01 g, 8.47 mmol, 1.0 eq.) was dissolved in acetonitrile (30 mL). NIS (2.10 g, 9.32 mmol, 1.1 eq.) was added, and the mixture was stirred at room temperature for 16 hours, with completion monitored by LCMS. The mixture was filtered, and the filter cake was washed with EA(15 mL) to obtain the title compound as an off-white solid (2.29 g, yield: 74.5%). LCMS(ESI): $[M+H]^+$=364.0.

***Step 3: Preparation of 2-(chloromethyl)-6-fluoro-3-iodo-8-methoxy-1-methylquinolin-4(1H)-one:***

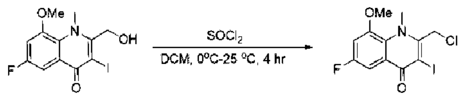


The intermediate obtained from step 2 (3.50 g, 9.64 mmol, 1.0 eq.) was dissolved in DCM (800 mL). Under an ice bath, $SOCl_2$ (64.6 g, 101 mmol, 10.5 eq.) was added dropwise. After the addition was complete, the mixture was stirred at room temperature for 1 hour, with completion monitored by LCMS. The mixture was evaporated to dryness. Ice was added, and the pH was adjusted to approximately 8 with saturated $NaHCO_3$ solution. The mixture was extracted with EA(50mL×3), and the combined organic layers were dried over anhydrous $Na_2SO_4$, filtered, and the filtrate was concentrated to obtain the title compound as a gray solid (3.4 g, yield: 92.4%). LCMS (ESI):$[M+H]^+$=382.1.

***Step 4: Preparation of (S)-4-ethyl-7-((6-fluoro-3-iodo-8-methoxy-1-methyl-4-oxo-1,4-dihydroquinolin-2-yl) methyl)-4-hydroxy-1,7-dihydro-3H-pyrano[3,4-c]pyridine-3,8(4H)-dione:***

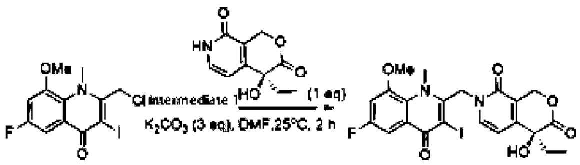


The intermediate obtained from step 3 (3.40 g, 8.91 mmol, 1.0 eq.) and Intermediate 1 (1.86 g, 8.91 mmol, 1.0 eq.) were dissolved in DMF (, 35 mL). $K_2CO_3$ (3.70 g, 26.8 mmol, 3.0 eq.) was added, and the mixture was stirred at 25 °C for 2 hours, with completion monitored by LCMS. The mixture was filtered, and the filtrate was poured into 50 mL of ice water. The pH was adjusted to approximately 5 with 1 mol/L dilute hydrochloric acid (HCl), and the mixture was extracted with EA (50mL×3). The combined EA layers were dried over anhydrous $Na_2SO_4$, filtered, and the filtrate was concentrated to afford a crude product. The crude product was purified by silica gel column chromatography (100-200 mesh silica gel) using a gradient eluent of PE:EA= 100:0 to 30:70 (v/v). The title compound was obtained as a pale white solid (3.57 g, yield: 72.3%). LCMS(ESI): $[M+H]^+$=555.2.

***Step 5: Preparation of (S)-4-ethyl-8-fluoro-4-hydroxy-10-methoxy-11-methyl-1,12-dihydro-14H-pyrano[3,4:6,7] indolizino[2,1 -b]quinoline-3,6,14(4H,11H)-trione:***

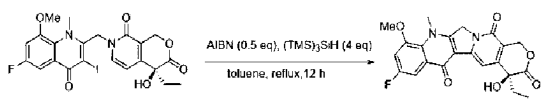


The intermediate obtained from step 4 (500mg, 0.900mmol, 1.0eq.) and AIBN(74.0mg, 0.450mmol, 0.5eq.) were dissolved in anhydrous toluene(60mL) with the $N_2$ atmosphere, and $(TMS)_3SiH$ (890mg, 3.60mmol, 4.0eq.) was added sequentially. The mixture was stirred at 100 °C for 12 hours, then cooled to room temperature and filtered. The filter cake was triturated with MTBE (20 mL) to obtain a crude product (375 mg). Crude product (190 mg ) was purified by silica gel column chromatography (100-200 mesh silica gel) using a gradient eluent of DCM:MeOH=100:0 to 95:5 (v/v), affording a further crude product (100 mg). Subsequent purification by preparative HPLC yielded the title compound as a white solid (20 mg, yield: 5.20%). LCMS (ESI): $[M+H]^+$=427.3; $^1H$ NMR (400 MHz, DMSO-$d_6$) δ 7.46 (dd, J = 8.6, 3.0 Hz, 1H), 7.33 (dd, J = 10.2, 3.0 Hz, 1H), 7.15 (s, 1H), 6.38-6.11 (s, 1H), 5.25 (m, 4H), 4.01 (s, 3H), 3.91 (s, 3H), 1.73 (m, 2H), 0.78 (t, J = 7.3 Hz, 3H).

**Example 11: Preparation of (S)-4-ethyl-8-fluoro-4-hydroxy-10-(methoxymethoxy)-11-methyl-1,12-dihydro-14H-pyrano[3,4:6,7]indolizino[2,1 -b]quinoline-3,6,14 (4H,11H)-trione:**

***Step 1: Preparation of methyl 6-fluoro-8-hydroxy-1-methyl-4-oxo-1,4-dihydroquinoline-2-carboxylate:***

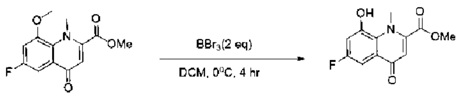


Intermediate 3 (1 g, 3.77 mmol, 1.0 eq.) was dissolved in DCM (10.0 mL). The solution was cooled to -78 °C, and under a nitrogen atmosphere, $BBr_3$ (1.89 g, 7.54 mmol, 2.0 eq.) was added slowly. The temperature was raised to 0 °C, and the mixture was stirred at 0 °C for 4 hours. LCMS analysis confirmed reaction completion. The reaction was quenched by pouring the mixture into iced MeOH, then concentrated. The residue was purified by silica gel column chromatography (100-200 mesh silica gel) using a gradient eluent of DCM:MeOH = 100:0 to 95:5 (v/v). The title compound was obtained as a yellow solid (700 mg, yield: 74.0%). LCMS(ESI): $[M+H]^+$=252.1.

***Step 2: Preparation of methyl 6-fluoro-8-(methoxymethoxy)-1-methyl-4-oxo-1,4-dihydroquinoline-2-carboxylate:***

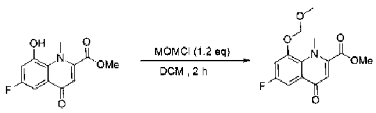


The intermediate obtained from step 1 (2 g, 7.96 mmol, 1.0 eq.) was dissolved in DCM (20.0 mL) and cooled in an ice bath. Chloromethyl methyl ether (769 mg, 9.55 mmol, 1.2 eq.) was added slowly, and the mixture was stirred at room temperature for 2 hours. LCMS showed the reaction was complete. The reaction mixture was poured into water, extracted with DCM, the organic phase was dried over anhydrous $Na_2SO_4$, and purified by silica gel column chromatography (PE:EA=2:1, v/v) to obtain the title compound (2 g, yield: 85.1%). LCMS (ESI): $[M+H]^+$=296.1.

***Step 3: Preparation of 6-fluoro-2-(hydroxymethyl)-8-(methoxymethoxy)-1-methylquinolin-4(1H)-one:***

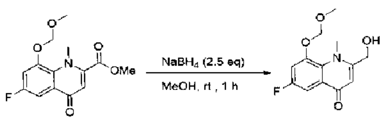


The intermediate obtained from step 2 (2 g, 6.77 mmol, 1.0 eq.) was dissolved in MeOH (20.0 mL) and cooled in an ice bath. $NaBH_4$ (641 mg, 16.9 mmol, 2.5 eq.) was added slowly. After stirring for an appropriate period, the reaction solution was poured into ice-cold saturated $NH_4Cl$ aqueous solution. The mixture was extracted with EA (20 mL×3), and the combined organic phases were dried over anhydrous $Na_2SO_4$, then concentrated under reduced pressure. The title compound was obtained as a white solid (1.6 g, crude product). LCMS (ESI): $[M+H]^+$=268.5.

***Step 4: Preparation of 6-fluoro-2-(hydroxymethyl)-3-iodo-8-(methoxymethoxy)-1-methylquinolin-4(1H)-one:***

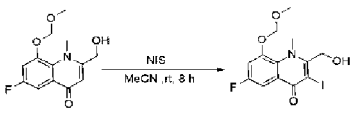


The intermediate obtained from step 3 (1.7 g, 6.36 mmol, 1.0 eq.) was dissolved in MeCN (20.0 mL). Under ice bath conditions, NIS (1.6 g, 7.0 mmol, 1.1 eq.) was added, and the reaction mixture was stirred at room temperature for 8 hours, and then filtered, and the mother liquor was dried over anhydrous $Na_2SO_4$, concentrated, and purified by silica gel column chromatography (PE:EA= 1:1, v/v). The title compound was obtained as a yellow solid (1.8 g, yield: 72.0%). LCMS

(ESI): $[M+H]^+$=393.9.

***Step 5: Preparation of 2-(chloromethyl)-6-fluoro-3-iodo-8-(methoxymethoxy)-1-methylquinolin-4(1H)-one:***

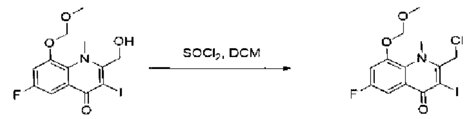


The intermediate obtained from step 4 (800 mg, 2.03 mmol, 1.0 eq.) was dissolved in DCM (200 mL). Under ice bath conditions, $SOCl_2$ (2.41 mL, 20.3 mmol, 10.0 eq.) was added, and the reaction mixture was stirred at room temperature for 1 hour. LCMS analysis confirmed reaction completion. The reaction solution was concentrated to dryness, and ice-cold $NaHCO_3$ solution was added slowly until the mixture was weakly alkaline. The mixture was extracted with EA, washed with saturated brine, and the organic phase was concentrated to dryness. The residue was purified by silica gel column chromatography (PE:EA = 3:1, v/v) to obtain the title compound as a yellow solid (400 mg, yield: 47.9%). LCMS (ESI): $[M+H]^+$ = 411.9.

***Step 6: Preparation of (S)-7-((3-iodo-6-fluoro-8-(methoxymethoxy)-1-methyl-4-oxo-1,4-dihydroquinolin-2-yl) methyl)-4-ethyl-4-hydroxy-1,7-dihydro-3H-pyrano[3,4-c]pyridine-3,8(4H)-dione:***

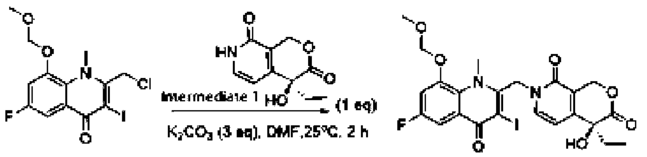


The intermediate obtained from step 5 (400 mg, 0.97 mmol, 1.0 eq.) was dissolved in DMF (5.0 mL). $K_2CO_3$ (403 mg, 2.92 mmol, 3.0 eq.) was added, followed by Intermediate 1 (203 mg, 0.97 mmol, 1.0 eq.). The reaction mixture was stirred at room temperature for 2 hours, and LCMS analysis confirmed reaction completion. Water was added to the reaction solution, and the mixture was adjusted to weakly acidic with dilute HCl. It was then extracted with EA (15 mL$\times$3), and the combined organic phases were concentrated. The residue was purified by silica gel column chromatography (PE:EA = 1:4, v/v) to obtain the title compound as a yellow solid (400 mg, yield: 70.6%). LCMS (ESI): $[M+H]^+$=585.0.

***Step 7: Preparation of (S)-4-ethyl-8-fluoro-4-hydroxy-10-(methoxymethoxy)-11-methyl-1,12-dihydro-14H-pyrano[3,4:6,7]indolizino[2,1 -b]quinoline-3,6,14(4H,11H)-trione:***

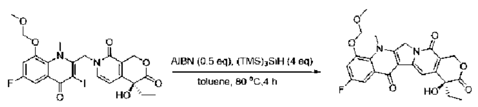


The intermediate obtained from Step 6 (400 mg, 0.68 mmol, 1.0 eq.) was dissolved in toluene (200 mL). AIBN (56.2 mg, 0.34 mmol, 0.5 eq.) was added, and under nitrogen protection, $(TMS)_3SiH$ (272 mg, 2.74 mmol, 4.0 eq.) diluted with toluene was slowly added to the reaction solution. The mixture was reacted at 80 °C for 4 hours, and LCMS detection confirmed reaction completion. The reaction solution was concentrated to dryness, and the residue was purified by silica gel column chromatography (DCM:MeOH = 40:1, v/v) to obtain the title compound as a yellow solid (130 mg, yield: 41.89%). LCMS (ESI): $[M+H]^+$= 457.3; $^1H$ NMR (400 MHz, DMSO-$d_6$) $\delta$ 7.65 (dd, J = 8.5, 3.0 Hz, 1H), 7.44 (dd, J = 10.0, 3.0 Hz, 1H), 7.27 (s, 1H), 6.36 (s, 1H), 5.47 (s, 2H), 5.40 (s, 2H), 5.35 (d, J = 5.5 Hz, 2H), 4.16 (s, 3H), 3.52 (s, 3H), 1.87-1.80 (m, 2H), 0.87 (t, J = 7.3 Hz, 3H).

**Example 12: Preparation of (S)-4-ethyl-7-fluoro-4,10-dihydroxy-1,12-dihydro-14H-pyrano[3',4':6,7]indolizino[2,1-b]quinoline-3,6,14(4H,11H)-trione:**

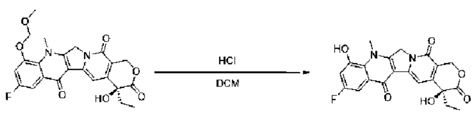


The compound obtained from Example 11 (110 mg, 0.26 mmol, 1.0 eq.) was dissolved in DCM (5.0 mL). 1 mol/L HCl-DCM solution (0.5 mL, 0.26 mmol, 1.0 eq.) was added, and the reaction mixture was stirred at room temperature for 0.5 hours. LCMS detection confirmed reaction completion. The reaction solution was concentrated under reduced pressure, and the residue was separated and purified by preparative methods to obtain the title compound as a white solid (11.0 mg, yield: 10.4%). LCMS (ESI): $[M+H]^+$= 413.3; $^1H$ NMR (400 MHz, DMSO-$d_6$) δ 11.42 (s, 1H), 7.46 (dd, J = 8.8, 3.1 Hz, 1H), 7.26 (s, 1H), 7.06 (dd, J = 9.5, 3.1 Hz, 1H), 6.34 (s, 1H), 5.40-5.29 (m, 4H), 4.18 (s, 3H), 1.83 (dd, J = 7.3, 3.8 Hz, 2H), 0.87 (t, J = 7.3 Hz, 3H).

**Example 13: Preparation of (S)-4-ethyl-7-fluoro-4-hydroxy-10-(2-hydroxyethoxy)-1,12-dihydro-14H-pyrano[3',4':6,7]indolizino[2,1-b]quinoline-3,6,14(4H,11H)-trione:**

***Step 1: Preparation of methyl 6-fluoro-1-methyl-4-oxo-8-(2-((tetrahydro-2H-pyran-2-yl)oxy)ethoxy)-1,4-dihydroquinoline-2-carboxylate:***

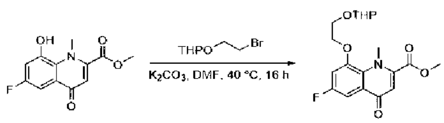


Under nitrogen protection, potassium carbonate ($K_2CO_3$, 2.2 g, 15.92 mmol, 2.0 eq.) and 2-(2-bromoethoxy) tetrahydropyran (1.83 g, 8.76 mmol, 1.1 eq.) were added to an DMF(20mL) solution of methyl 6-fluoro-8-hydroxy-1-methyl-4-oxo-1,4-dihydroquinoline-2-carboxylate (2 g, 7.96 mmol, 1.0 eq.). The reaction mixture was stirred at 40 °C for 16 hours, and LCMS monitoring showed that the raw materials were completely reacted, with the reaction solution mainly containing the product. The reaction solution was cooled to room temperature, and DCM (300 mL) and water (300 mL) were added for extraction. The organic phase was washed with saturated brine (300mL×3), then concentrated to dryness under reduced pressure. The residue was separated and purified by silica gel column chromatography (100-200 mesh silica gel, eluent: DCM:MeOH=10:1), and the collected fractions were concentrated to dryness to obtain the title compound as a brownish-yellow oil (2.18 g, yield: 72.19%). LCMS (ESI): $[M+H]^+$= 380.4; $^1H$ NMR (400 MHz, $CDCl_3$) δ 7.63 (dd, J = 8.4, 2.8 Hz, 1H), 6.95 (dd, J = 9.6, 2.8 Hz, 1H), 6.65 (s, 1H), 4.70 (t, J = 3.2 Hz, 1H), 4.27 (t, J = 4.8 Hz, 2H), 4.20-4.11 (m, 1H), 3.98 (s, 3H), 3.89 (s, 3H), 3.87-3.81 (m, 2H), 3.60-3.51 (m, 1H), 1.84-1.70 (m, 2H), 1.63-1.51 (m, 4H).

***Step 2: Preparation of 6-fluoro-2-(hydroxymethyl)-1-methyl-8-(2-((tetrahydro-2H-pyran-2-yl)oxy)ethoxy)quinolin-4(1H)-one:***

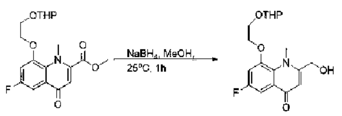


The intermediate obtained from step 1 (2.18 g, 5.75 mmol, 1.0 eq.) was dissolved in MeOH (30 mL) and cooled in an ice bath. $NaBH_4$ (543 mg, 14.38 mmol, 2.5 eq.) was added slowly, and the reaction mixture was stirred at room temperature for 3 hours after the addition was complete. LCMS monitoring confirmed the completion of the reaction. The reaction was quenched by pouring into ice-cold saturated ammonium chloride aqueous solution (20 mL), then extracted with EA (50 mL×3). The combined organic phases were washed with saturated brine (50 mL×2), dried over anhydrous $Na_2SO_4$, concentrated, and purified by silica gel column chromatography (100-200 mesh silica gel, eluent: DCM:MeOH =

20:1) to obtain the title compound as a pale yellow solid (1.72 g, yield: 84.2%). LCMS (ESI): $[M+H]^+$=352.4; $^1H$ NMR (400 MHz, $CDCl_3$) δ 7.58 (dd, J = 8.4, 2.9 Hz, 1H), 6.92 (dd, J = 9.5, 2.9 Hz, 1H), 6.59 (s, 1H), 4.69 (t, J = 3.2 Hz, 1H), 4.62 (d, J = 5.2 Hz, 2H), 4.25 (t, J = 4.8 Hz, 2H), 4.18-4.10 (m, 1H), 3.88 (s, 3H), 3.86-3.80 (m, 2H), 3.59-3.50 (m, 1H), 2.35 (t, J = 5.2 Hz, 1H, -OH), 1.83-1.69 (m, 2H), 1.62-1.50 (m, 4H).

***Step 3: Preparation of 6-fluoro-2-(hydroxymethyl)-3-iodo-1-methyl-8-(2-((tetrahydro-2H-pyran-2-yl)oxy)ethoxy)quinolin-4(1H)-one:***

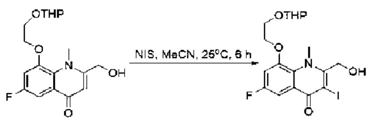


Under nitrogen protection, NIS (1.54 g, 6.84 mmol, 2.0 eq.) was added to MeCN (20 mL) solution of the intermediate obtained from step 2 (1.2 g, 3.42 mmol, 1.0 eq.). The reaction mixture was stirred at 25 °C for 6 hours, then concentrated and purified by reversed-phase C18 column chromatography (mobile phase: $H_2O$-MeCN) to obtain the title compound as a white solid (1.6 g, yield: 98.02%). LCMS (ESI):$[M+H]^+$=478.2; $^1H$ NMR (400 MHz, DMSO-$d_6$) δ 7.38 (s, 1H), 7.36 (s, 1H), 5.86 (t, J = 5.2 Hz, 1H), 5.02 (d, J = 5.2 Hz, 2H), 4.70 (d, J = 3.6 Hz, 1H), 4.40-4.29 (m, 2H), 4.04 (s, 3H), 3.85-3.71 (m, 2H), 3.51-3.42 (m, 1H), 1.76-1.59 (m, 2H), 1.54-1.39 (m, 4H).

***Step 4: Preparation of 2-(chloromethyl)-6-fluoro-3-iodo-1-methyl-8-(2-((tetrahydro-2H-pyran-2-yl)oxy)ethoxy)quinolin-4(1H)-one:***

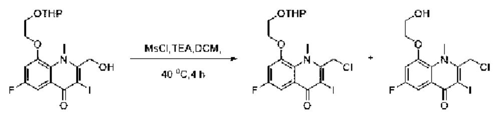


Under nitrogen protection at 0 °C (ice bath), triethylamine (1.85 g, 18.24 mmol, 6.0 eq.) and MsCl (2.09 g, 18.24 mmol, 6.0 eq.) were added to a solution of the Step 3 intermediate (1.45 g, 3.04 mmol, 1.0 eq.) in DCM (20 mL). The mixture was stirred at 40 °C for 4 h. LCMS monitoring indicated complete consumption of starting materials, with the target product and THP-deprotected byproduct formed in approximately 1:1 ratio. The reaction was quenched with water (40 mL) and extracted. The organic layer was washed with saturated brine (20 mL × 3), dried over anhydrous $Na_2SO_4$, and concentrated under reduced pressure to afford a crude mixture of the title compound and THP-deprotected byproduct (1.34 g). LCMS (ESI): title compound $[M+H]^+$=496.0; THP-deprotected byproduct $[M+H]^+$=412.0. The crude mixture was used directly in the next step without further purification; isolation and purification were carried out in subsequent steps.

***Step 5: Preparation of (S)-7-((3-iodo-6-fluoro-1-methyl-4-oxo-8-(2-((tetrahydro-2H-pyran-2-yl)oxy)ethoxy)-1,4-dihydroquinolin-2-yl)methyl)-4-ethyl-4-hydroxy-1,7-dihydro-3H-pyrano[3,4-c]pyridine-3,8(4H)-dione:***

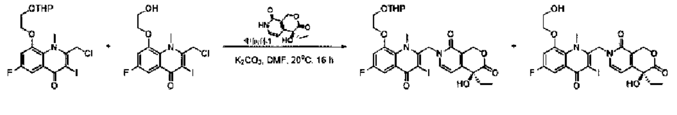


Under nitrogen protection, $K_2CO_3$ (1.45 g, 10.48 mmol, 4.0 eq.) was added to a solution of the step 4 crude mixture and Intermediate 1 (0.6 g, 2.88 mmol, 1.1 eq.) in DMF (20 mL). The mixture was stirred at 20 °C for 16 h. LCMS showed complete consumption of starting materials, with the title compound and THP-deprotected byproduct as main products. The mixture was concentrated under reduced pressure, filtered, and the filtrate was concentrated to dryness. The residue was purified by silica gel flash column chromatography (DCM:MeOH = 200:1 to 30:1) to afford two products.

**Title Product:** Yellow solid (520 mg, yield: 29.69%). LCMS (ESI): $[M + H]^+$ = 669.2, $t_R$ = 1.207 min. $^1H$ NMR (400 MHz, DMSO-$d_6$) δ 7.55 (dd, J = 7.2, 3.2 Hz, 1H), 7.46-7.33 (m, 2H), 7.21-7.08 (m, 1H), 6.45 (d, J = 7.2 Hz, 1H), 6.40-6.30 (m, 1H), 5.78-5.49 (m, 2H), 5.29 (d, J = 5.6 Hz, 2H), 4.30 (dd, J = 8.0, 5.2 Hz, 1H), 4.17 (t, J = 4.8 Hz, 1H), 4.07-3.86 (m, 1H), 3.80

(d, J = 2.0 Hz, 3H), 3.77-3.60 (m, 2H), 2.31 (d, J = 12.4 Hz, 1H), 1.87-1.72 (m, 2H), 1.57 (dd, J = 22.0, 10.0 Hz, 2H), 1.48-1.13 (m, 4H), 0.81 (t, J = 7.2 Hz, 3H).

**THP-Deprotected By-Product:** Yellow solid (450 mg, yield: 29.39%). LCMS (ESI): $[M + H]^+$ = 585.2, $t_R$= 0.941 min. $^1$H-NMR (400 MHz, DMSO-$d_6$) δ 7.60-7.42 (m, 1H), 7.41-7.33 (m, 1H), 7.29-7.10 (m, 1H), 6.50-6.30 (m, 1H), 5.38-5.25 (m, 2H), 5.20 (d, J = 5.6 Hz, 2H), 4.85 (t, J = 5.2 Hz, 1H, -OH), 4.20 (t, J = 4.8 Hz, 2H), 4.05-3.88 (m, 1H), 3.82 (s, 3H), 3.70-3.58 (m, 2H), 1.85-1.70 (m, 2H), 0.82 (t, J = 7.2 Hz, 3H).

***Step 6: Preparation of (S)-4-ethyl-7-fluoro-4-hydroxy-10-(2-hydroxyethoxy)-1,12-dihydro-14H-pyrano[3',4':6,7] indolizino[2,1-b]quinoline-3,6,14(4H,11H)-trione:***

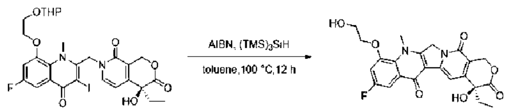


Under nitrogen protection, the intermediate obtained from step 5 (100 mg, 0.15 mmol, 1.0 eq.) and AIBN (12.28 mg, 0.07 mmol, 0.5 eq.) were dissolved in anhydrous toluene (10 mL), followed by $(TMS)_3SiH$ (186 mg, 0.75 mmol, 5.0 eq.). The mixture was degassed three times via oil pump, heated to 100 °C, and stirred for 12 h. LCMS indicated completion, with ~60% target product and ~20% THP-deprotected uncyclized byproduct. The reaction was quenched with MeOH (5 mL), and toluene was removed in vacuo. The residue was dissolved in DMSO-MeOH and purified by reversed-phase HPLC (C18, 0.1% TFA in water-acetonitrile) to afford the title compound as a pale yellow solid (4 mg, yield not determined).LCMS (ESI): $[M+H]^+$ = 457.0; $^1$H-NMR (400 MHz, DMSO-$d_6$) δ 7.57 (dd, J = 8.8, 2.8 Hz, 1H), 7.44 (dd, J = 10.0, 2.8 Hz, 1H), 7.26 (s, 1H), 6.36 (s, 1H), 5.38 (s, 2H), 5.34 (d, J = 4.4 Hz, 2H), 5.08 (s, 1H, -OH), 4.26-4.21 (m, 2H), 4.17 (s, 3H), 3.85 (s, 2H), 1.89-1.74 (m, 2H), 0.86 (t, J = 7.2 Hz, 3H).

Example 14: Preparation of (S)-10-(2-(dimethylamino)ethoxy)-4-ethyl-7-fluoro-4-hydroxy-1,12-dihydro-14H-pyrano [3,4:6,7]indolizino[2,1 -b]quinoline-3,6,14(4H,11H)-trione:

*Step 1: Preparation of (S)-2-((2-(((4-ethyl-4-hydroxy-3,8-dioxo-4,8-dihydro-1H-pyrano[3,4-c]pyridin-7(3H)-yl) methyl)-6-fluoro-3-iodo-1-methyl-4-oxo-1,4-dihydroquinolin-8-yl)oxy)ethyl methanesulfonate:*

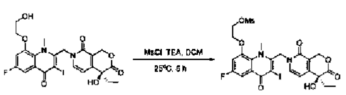


(S)-7-((6-fluoro-8-(2-hydroxyethoxy)-3-iodo-1-methyl-4-oxo-1,4-dihydroquinolin-2-yl)methyl)-4-ethyl-4-hydroxy-1,7-dihydro-3H-pyrano[3,4-c]pyridine-3,8(4H)-dione (340 mg, 0.58 mmol, 1.0 eq.) was dissolved in DCM (10 mL). Triethylamine (147.2 mg, 1.45 mmol, 2.5 eq.) and MsCl (99.66 mg, 0.87 mmol, 1.5 eq.) were added sequentially, and the reaction mixture was stirred at 25 °C for 6 hours. LCMS monitoring confirmed complete reaction. The reaction solution was directly concentrated to dryness obtain the title compound (160 mg, yield: 42%), which was used directly in the next step without purification. LCMS(ESI): $[M+H]^+$=663.0.

*Step 2: Preparation of (S)-7-((8-(2-(dimethylamino)ethoxy)-6-fluoro-3-iodo-1-methyl-4-oxo-1,4-dihydroquinolin-2-yl) methyl)-4-ethyl-4-hydroxy-1,7-dihydro-3H-pyrano[3,4-c]pyridine-3,8(4H)-dione:*

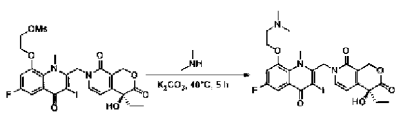


Under nitrogen protection, the intermediate obtained from step 1 (160 mg, 0.24 mmol, 1.0 eq.) was dissolved in THF (8 mL). $K_2CO_3$ (100 mg, 0.72 mmol, 3.0 eq.) and dimethylamine (22 mg, 0.48 mmol, 2.0 eq.) were added, and the mixture was stirred at 40 °C for 5 h. After filtering off $K_2CO_3$, the filtrate was concentrated. The residue was treated with

water (20 mL) and extracted with DCM (15 mL × 3). Combined organic phases were concentrated, and the residue was purified by silica gel flash column chromatography (eluent: DCM:MeOH = 15:1, v/v) to afford the title compound as a yellow solid (70 mg, 48% yield). LCMS(ESI): $[M+H]^+$=632.1. $^1$H NMR (400 MHz, DMSO-$d_6$) δ 7.56 (d, J = 7.2 Hz, 1H), 7.40-7.34 (m, 2H), 6.46 (d, J = 7.2 Hz, 1H), 6.35 (s, 1H), 5.62 (q, J = 16.4 Hz, 2H), 5.28 (s, 2H), 4.19 (t, J = 5.6 Hz, 2H), 3.74 (s, 3H), 2.61 (t, J = 5.2 Hz, 2H), 2.13 (s, 6H), 1.83-1.75 (m, 2H), 0.82 (t, J = 7.2 Hz, 3H).

***Step 3: Preparation of (S)-10-(2-(dimethylamino)ethoxy)-4-ethyl-7-fluoro-4-hydroxy-1,12-dihydro-14H-pyrano [3,4:6,7]indolizino[2,1 -b]quinoline-3,6,14 (4H,11H)-trione:***

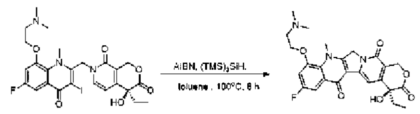


The intermediate obtained from step 2 (50 mg, 0.08 mmol, 1.0 eq.) was dissolved in toluene (8 mL). AIBN (6.71 mg, 0.04 mmol, 0.5 eq.) and $(TMS)_3SiH$ (81.34 mg, 0.33 mmol, 4.0 eq.) were added sequentially. Under nitrogen protection, the reaction system was heated to 100 °C and stirred for 8 hours. LCMS monitoring showed 13% product formation. MeOH (5 mL) was added to quench the reaction, and the mixture was concentrated to dryness under reduced pressure. The residue was purified by preparative HPLC (mobile phase: 0.1% TFA in ACN-$H_2O$) to obtain the title compound as a yellow solid (2 mg, yield: 5.17%). LCMS(ESI): $[M+H]^+$=484.2.

**Example 15: Preparation of (S)-4-ethyl-7-fluoro-4-hydroxy-10-(2-morpholinoethoxy)-1,12-dihydro-14H-pyrano [3,4:6,7]indolizino[2,1 -b]quinoline-3,6,14(4H,11H)-trione:**

***Step 1: Preparation of (S)-7-((8-(2-morpholinoethoxy)-6-fluoro-3-iodo-1-methyl-4-oxo-1,4-dihydroquinolin-2-yl) methyl)-4-ethyl-4-hydroxy-1,7-dihydro-3H-pyrano [3,4-c]pyridine-3,8(4H)-dione:***

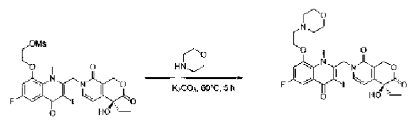


(S)-2-((2-(((4-ethyl-4-hydroxy-3,8-dioxo-4,8-dihydro-1H-pyrano[3,4-c]pyridin-7(3H)-yl)methyl)-6-fluoro-3-iodo-1-methyl-4-oxo-1,4-dihydroquinolin-8-yl)oxy)ethyl methanesulfonate (100 mg, 0.15 mmol, 1.0 eq.) was dissolved in DCM (8 mL). $K_2CO_3$ (41.73 mg, 0.3 mmol, 2.0 eq.) and morpholine (26.3 mg, 0.3 mmol, 2.0 eq.) were added, and the reaction mixture was stirred at 60 °C for 5 hours. LCMS monitoring showed 65% product formation. $K_2CO_3$ was removed by filtration, and the filtrate was concentrated under reduced pressure. The residue was purified by silica gel flash column chromatography (eluent: DCM:MeOH = 12:1, v/v) to obtain the title compound as a gray solid (80 mg, yield: 81%). LCMS (ESI): $[M+H]^+$ = 654.3.

***Step 2: Preparation of (S)-4-ethyl-7-fluoro-4-hydroxy-10-(2-morpholinoethoxy)-1,12-dihydro-14H-pyrano [3,4:6,7]indolizino[2,1 -b]quinoline-3,6,14(4H,11H)-trione:***

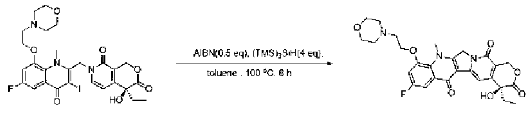


The compound obtained from Step 1 (80 mg, 0.12 mmol, 1.0 eq.) was dissolved in toluene (10 mL). AIBN (10.06 mg, 0.06 mmol, 0.5 eq.) and $(TMS)_3SiH$ (121.78 mg, 0.48 mmol, 4.0 eq.) were added sequentially. Under nitrogen protection, the reaction system was degassed with an oil pump, then heated to 100 °C and stirred for 8 hours. LCMS monitoring showed 10% product formation. MeOH (5 mL) was added to quench the reaction, and the mixture was concentrated to dryness under reduced pressure quickly. The residue was dissolved in DMF, and purified by preparative

HPLC (mobile phase: 0.1% TFA in acetonitrile-water) to obtain the title compound as a yellow solid (8 mg, yield: 12.69%). LCMS (ESI): $[M + H]^+$ = 526.2; $^1$H-NMR (400 MHz, DMSO-$d_6$) δ 7.64 (dd, J = 8.4, 2.8 Hz, 1H), 7.53 (dd, J = 10.0, 2.8 Hz, 1H), 7.26 (s, 1H), 6.36 (s, 1H), 5.42 (s, 2H), 5.35 (d, J = 4.0 Hz, 2H), 4.57 (s, 2H), 4.15 (s, 3H), 4.06-3.45 (m, 10H), 1.82 (dt, J = 10.4, 6.8 Hz, 2H), 0.86 (t, J = 7.2 Hz, 3H).

Example 16: Preparation of (S)-10-(2-aminoethoxy)-4-ethyl-7-fluoro-4-hydroxy-1,12-dihydro-14H-pyrano[3,4:6,7]indolizino[2,1 -b]quinoline-3,6,14(4H,11H)-trione:

*Step 1: Preparation of methyl 8-(2-((tert-butoxycarbonyl)amino)ethoxy)-6-fluoro-1-methyl-4-oxo-1,4-dihydroquinoline-2-carboxylate:*

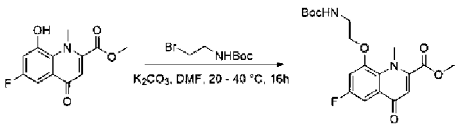


$K_2CO_3$ (4.4 g, 31.85 mmol, 4.0 eq.) and N-Boc-2-bromoethylamine (4.28 g, 19.11 mmol, 2.4 eq.) were added to a solution of methyl 6-fluoro-8-hydroxy-1-methyl-4-oxo-1,4-dihydroquinoline-2-carboxylate (2 g, 7.96 mmol, 1.0 eq.) in DMF (30 mL). The reaction was stirred until LCMS indicated completion. Water (800 mL) was added, and the mixture was extracted with EA (500mL×3). Combined organic phases were washed with saturated brine (400mL×3), concentrated to dryness under reduced pressure, and the residue was purified by silica gel column chromatography (eluent: DCM:MeOH=10:1) to afford the title compound as a brown solid (1.58 g, 50.33% yield). LCMS (ESI): $[M + H]^+$ = 395.2; $^1$H-NMR (400 MHz, $CDCl_3$) δ 7.56 (dd, J = 8.4, 2.8 Hz, 1H), 6.86 (dd, J = 9.6, 2.8 Hz, 1H), 6.62 (s, 1H), 5.31 (s, 1H), 4.15 (t, J = 5.2 Hz, 2H), 3.98 (s, 3H), 3.83 (s, 3H), 3.73-3.59 (m, 2H), 1.47 (s, 9H).

***Step 2: Preparation of tert-butyl (2-((6-fluoro-2-(hydroxymethyl)-1-methyl-4-oxo-1,4-dihydroquinolin-8-yl)oxy) ethyl)carbamate:***

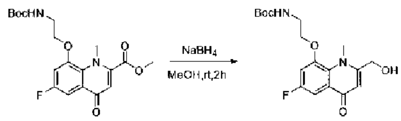


The compound obtained from step 1 (1.63 g, 4.13 mmol, 1.0 eq.) was dissolved in MeOH (20 mL) and cooled to 0 °C. $NaBH_4$ (312.7 mg, 8.27 mmol, 2.0 eq.) was added to the reaction solution, and the mixture was stirred at 25 °C for 2 hours. The reaction solution was concentrated to dryness, and the residue was separated and purified by silica gel column chromatography (eluent: DCM:MeOH = 10:1) to obtain the title compound as a pale yellow solid (1.2 g, yield: 79%). LCMS (ESI): $[M+H]^+$ = 367.4; $^1$H NMR (400 MHz, DMSO-$d_6$) δ 7.38 (dd, J = 8.8, 2.8 Hz, 1H), 7.29 (dd, J = 10.4, 2.8 Hz, 1H), 7.15 (t, J = 5.2 Hz, 1H), 6.24 (s, 1H), 5.72 (t, J = 5.6 Hz, 1H), 4.57 (d, J = 5.6 Hz, 2H), 4.08 (t, J = 5.2 Hz, 2H), 3.81 (s, 3H), 3.48-3.42 (m, 2H), 1.41 (s, 9H).

***Step 3: Preparation of tert-butyl (2-((6-fluoro-2-(hydroxymethyl)-3-iodo-1-methyl-4-oxo-1,4-dihydroquinolin-8-yl)oxy)ethyl)carbamate:***

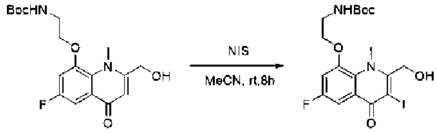


The intermediate obtained from Step 2 (1.2 g, 3.28 mmol, 1.0 eq.) was dissolved in acetonitrile (20 mL). NIS (884.25 mg, 3.93 mmol, 1.2 eq.) was added, and the reaction mixture was stirred at room temperature for 8 hours. LCMS monitoring confirmed complete reaction. The solvent was removed under reduced pressure, water (5 mL) was added to

the residue, and the mixture was extracted with EA (10 mL × 3). The combined organic phases were washed with aqueous sodium sulfite solution to remove excess iodine, dried over anhydrous $Na_2SO_4$, concentrated, and purified by silica gel flash column chromatography (eluent: PE:EA = 1:1) to obtain the title compound as a yellow solid (1.2 g, yield: 68.13%). LCMS (ESI): $[M+H]^+$=493.2; $^1$H NMR (400 MHz, DMSO-$d_6$) δ 7.40-7.12 (m, 2H), 7.13 (t, J = 5.6 Hz, 1H), 5.00 (s, 2H), 4.16 (t, J = 5.2 Hz, 2H), 4.02 (s, 3H), 3.44 (dd, J = 10.4, 5.2 Hz), 1.38 (s, 9H,).

***Step 4: Preparation of tert-butyl (2-((2-(chloromethyl)-6-fluoro-3-iodo-1-methyl-4-oxo-1,4-dihydroquinolin-8-yl) oxy)ethyl)carbamate:***

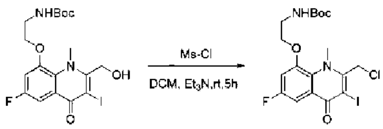


The compound obtained from step 3 (1.1 g, 2.23 mmol, 1.0 eq.) was dissolved in DCM (20 mL). TEA (902.61 mg, 8.92 mmol, 4.0 eq.) was added, and MsCl (638.62 mg, 5.57 mmol, 2.5 eq.) was slowly added dropwise under ice bath (0 °C). The reaction mixture was stirred at room temperature for 5 hours, and LCMS monitoring confirmed complete reaction. Water (5 mL) was added, and the mixture was extracted with EA (10 mL×3). The combined organic phases were dried over anhydrous $Na_2SO_4$, concentrated and purified by silica gel flash column chromatography (eluent: PE:EA = 2:1) to obtain the title compound as a pale yellow solid (850 mg, yield: 74.63%). LCMS (ESI): $[M+H]^+$ = 511.1. LCMS (ESI) [M+1] (+) =511.0; (1)H NMR (400 MHz, DMSO-*d*(*6*)) δ 7.40 - 7.35 (m, 2H), 7.13 (t, *J* = 5.6 Hz, 1H), 5.18 (s, 2H), 4.18 (t, *J* = 5.2 Hz, 2H), 4.06 (s, 3H), 3.45 (dd, *J* = 10.4, 5.2 Hz, 2H), 1.38 (s, 9H).

***Step 5: Preparation of (S)-tert-butyl (2-((2-(((4-ethyl-4-hydroxy-3,8-dioxo-4,8-dihydro-1H-pyrano[3,4-c]pyridin-7(3H)-yl)methyl)-6-fluoro-3-iodo-1-methyl-4-oxo-1,4-dihydroquinolin-8-yl)oxy)ethyl)carbamate:***

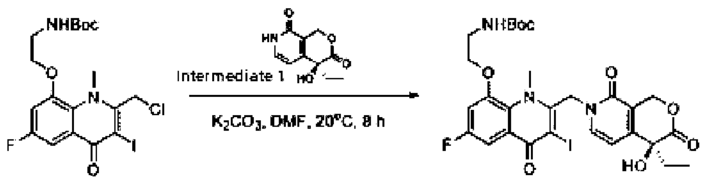


The compound obtained from step 4 (850 mg, 1.66 mmol, 1.0 eq.) was dissolved in DMF (15 mL). $K_2CO_3$ (345.03 mg, 2.5 mmol, 1.5 eq.) was added, followed by Intermediate 1 (382.99 mg, 1.83 mmol, 1.1 eq.). The reaction mixture was stirred at room temperature for 8 hours, and LCMS monitoring confirmed complete reaction. The residue was purified by silica gel flash column chromatography (eluent: PE:EA = 1:1) to obtain the title compound as a yellow solid (800 mg, 1.17 mmol, yield: 70.33%). LCMS (ESI): $[M+H]^+$=684.0; $^1$H-NMR (400 MHz, DMSO-$d_6$) δ 7.53 (d, J = 7.2 Hz, 1H), 7.44-7.35 (m, 2H), 7.10 (d, J = 5.2 Hz, 1H, -NH-), 6.47 (d, J = 7.2 Hz, 1H), 6.37 (s, 1H), 5.59 (d, J = 4.4 Hz, 2H), 5.32 (s, 2H), 4.14 (s, 2H), 3.78 (s, 3H, $-NCH_3$), 1.80 (dt, J = 20.0, 6.8 Hz, 2H), 1.36 (s, 9H, $-C(CH_3)_3$), 0.83 (t, J = 7.2 Hz, 3H).

***Step 6: Preparation of (S)-tert-butyl (2-((4-ethyl-7-fluoro-4-hydroxy-11-methyl-3,6,14-trioxo-3,4,6,11,12,14-hexahydro-1H-pyrano[3',4':6,7]indolizino[2,1-b]quinolin-10-yl)oxy)ethyl)carbamate:***

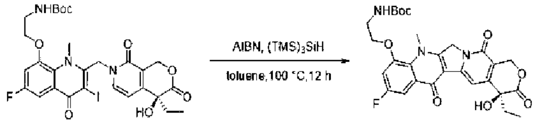


The compound obtained from step 5 (800 mg, 1.17 mmol, 1.0 eq.) and AIBN (50.46 mg, 0.31 mmol, 0.3 eq.) were dissolved in 50 mL of ultra-dry toluene, degassed and purged with nitrogen via an oil pump, then a solution of $(TMS)_3SiH$ (1018.71 mg, 4.1 mmol, 4.0 eq.) in 2 mL ultra-dry toluene was added. The reaction mixture was heated to 100 °C and stirred

for 12 hours. LCMS monitoring showed that the raw materials were almost completely consumed, generating the target product and Boc-deprotected by-products. Toluene was removed under reduced pressure, and the residue was triturated with acetonitrile at 60 °C. After cooling to room temperature, the mixture was filtered to collect the filter cake, obtaining the title compound (crude product) as a yellow solid (200 mg, yield not calculated). LCMS (ESI): $[M+H]^+$ = 456.2/556.2.

***Step 7: Preparation of (S)-10-(2-aminoethoxy)-4-ethyl-7-fluoro-4-hydroxy-1,12-dihydro-14H-pyrano[3',4':6,7]indolizino[2,1-b]quinoline-3,6,14(4H,11H)-trione:***

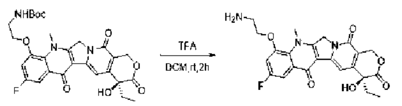


The intermediate obtained from Step 6 (crude product, 500 mg, 0.9 mmol, 1.0 eq.) was dissolved in DCM (10 mL). TFA (205.24 mg, 1.8 mmol, 2.0 eq.) was added, and the reaction mixture was stirred at room temperature for 2 hours. LCMS monitoring confirmed complete consumption of raw materials. The reaction solution was concentrated to dryness, and purified by preparative HPLC (mobile phase: acetonitrile-0.5% TFA aqueous solution) to obtain the title compound as a yellow solid (70 mg, yield: 17.08%). LCMS (ESI): $[M+H]^+$ = 456.2; $^1$H-NMR (400 MHz, DMSO-$d_6$) δ 8.08 (s, 3H, -$NH_3^+$), 7.65-7.62 (m, 1H), 7.52-7.49 (m, 1H), 7.26 (s, 1H), 6.35 (s, 1H), 5.40 (d, J = 7.6 Hz, 2H), 5.33 (t, J = 10.0 Hz, 2H), 4.42 (t, J = 4.8 Hz, 2H), 4.15 (s, 3H, -$NCH_3$), 3.40 (s, 2H), 1.85-1.78 (m, 2H), 0.86 (t, J = 7.2 Hz, 3H).

**Example 17: Preparation of (S)-2-((4-ethyl-7-fluoro-4-hydroxy-11-methyl-3,6,14-trioxo-3,4,6,11,12,14-hexahydro-1H-pyrano[3',4':6,7]indolizino[2,1-b]quinolin-10-yl)oxy)acetic acid**

***Step 1: Preparation of methyl 8-((tert-butoxycarbonyl)methoxy)-6-fluoro-1-methyl-4-oxo-1,4-dihydroquinoline-2-carboxylate:***

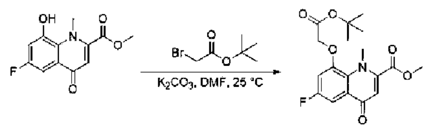


At 0 °C, $K_2CO_3$ (, 5.5 g, 39.81 mmol, 2.0 eq.) was added to DMF (50 mL) solution of methyl 6-fluoro-8-hydroxy-1-methyl-4-oxo-1,4-dihydroquinoline-2-carboxylate (5.0 g, 19.9 mmol, 1.0 eq.), and the mixture was stirred at room temperature for 1 hour. tert-Butyl bromoacetate (5.82 g, 29.86 mmol, 1.5 eq.) was added slowly, and the reaction was continued at room temperature for 2 hours. LCMS monitoring confirmed complete reaction. The residual potassium carbonate was filtered off, and the remaining organic solvent was removed. The residue was purified by silica gel column chromatography (100-200 mesh silica gel, eluent gradient: PE/EA = 100:1 to 15:1) to obtain the title compound as a pale yellow solid (5.77 g, yield: 79.36%). LCMS (ESI): $[M+H]^+$ = 366.4; $^1$H-NMR (400 MHz, DMSO-$d_6$) δ 7.40 (s, 1H), 7.37 (s, 1H), 6.45 (s, 1H), 4.94 (s, 2H), 3.94 (s, 3H), 3.86 (s, 3H), 1.45 (s, 9H).

***Step 2: Preparation of tert-butyl 2-((6-fluoro-2-(hydroxymethyl)-1-methyl-4-oxo-1,4-dihydroquinolin-8-yl)oxy)acetate:***

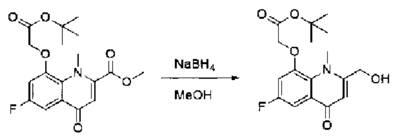


Under ice-water bath and nitrogen protection, $NaBH_4$ (1.19 g, 31.59 mmol, 2.0 eq.) was slowly added to a MeOH (50 mL) solution of The intermediate obtained from Step 1 (5.77 g, 15.79 mmol, 1.0 eq.). The reaction mixture was stirred at room temperature for 1 hour, and LCMS monitoring confirmed complete reaction. At 0 °C, 5 mL of saturated aqueous

ammonium chloride solution was added dropwise to quench the reaction, and the solution was concentrated to dryness. The residue was purified by silica gel flash column chromatography (eluent gradient: PE/EA = 100:1 to 15:1) to obtain the title compound as a pale yellow solid (2.4 g, yield: 45.06%). LCMS (ESI): $[M + H]^+$ = 338.4; $^1$H-NMR (400 MHz, DMSO-$d_6$) δ 7.38 (dd, J = 8.8, 2.8 Hz, 1H), 7.29 (dd, J = 10.4, 2.8 Hz, 1H), 6.25 (s, 1H), 5.73 (t, J = 5.6 Hz, 1H, -OH), 4.92 (s, 2H), 4.58 (d, J = 5.6 Hz, 2H, $-CH_2OH$), 3.82 (s, 3H, $-NCH_3$), 1.44 (s, 9H, $-C(CH_3)_3$).

***Step 3: Preparation of tert-butyl 2-((6-fluoro-2-(hydroxymethyl)-3-iodo-1-methyl-4-oxo-1,4-dihydroquinolin-8-yl)oxy)acetate:***

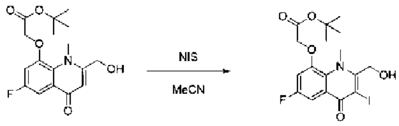


Under nitrogen protection, NIS (1.12 g, 4.98 mmol, 1.2 eq.) was added to an MeCN (15 mL) solution of the intermediate obtained from step 2 (1.4 g, 4.15 mmol, 1.0 eq.). The mixture was stirred at 40 °C for 2 hours, and LCMS monitoring confirmed complete reaction. The solution was concentrated under reduced pressure, and purified by silica gel flash column chromatography (eluent gradient: PE/EA = 5:1 to 2:1) to obtain the title compound as a yellowish solid (1.5 g, yield: 78.03%). LCMS (ESI): $[M+H]^+$ = 464.0; $^1$H NMR (400 MHz, DMSO-$d_6$) δ 7.53-7.37 (m, 1H), 7.37-7.27 (m, 1H), 5.91-5.82 (m, 1H), 5.03 (d, J = 5.2 Hz, 2H), 4.94 (s, 2H), 4.08 (s, 3H), 1.46 (s, 9H).

***Step 4: Preparation of tert-butyl 2-((2-(chloromethyl)-6-fluoro-3-iodo-1-methyl-4-oxo-1,4-dihydroquinolin-8-yl) oxy)acetate:***

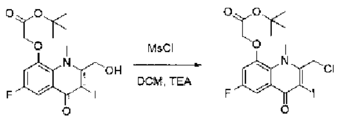


Under ice-water bath and nitrogen protection, TEA (982.98 mg, 9.71 mmol, 3.0 eq.) was added to a DCM (15 mL) solution of the compound obtained from step 3 (1.5 g, 3.24 mmol, 1.0 eq.). The temperature was maintained at 0 °C, and MsCl (1.85 g, 16.19 mmol, 5.0 eq.) was added dropwise. The reaction mixture was stirred at 40 °C for 2 hours, and LCMS monitoring confirmed complete reaction. The organic solvent was removed by concentration, and the residue was separated and purified by silica gel flash column chromatography (eluent gradient: PE/EA = 100:1 to 10:1) to obtain the title compound as a pale yellow solid (1.2 g, yield: 76.89%). LCMS (ESI): $[M + H]^+$ = 482.0; $^1$H NMR (400 MHz, DMSO-$d_6$) δ 7.43-7.36 (m, 2H), 5.22 (s, 2H), 4.96 (s, 2H), 4.12 (s, 3H), 1.46 (s, 9H).

***Step 5: Preparation of (S)-tert-butyl 2-((2-(((4-ethyl-4-hydroxy-3,8-dioxo-4,8-dihydro-1H-pyrano[3,4-c]pyridin-7(3H)-yl)methyl)-6-fluoro-3-iodo-1-methyl-4-oxo-1,4-dihydroquinolin-8-yl)oxy)acetate:***

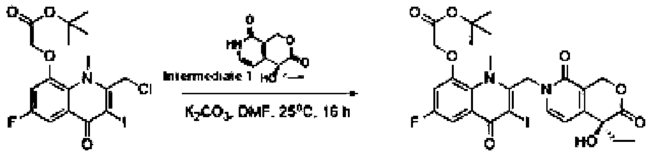


Under $N_2$ protection at 20 °C, $K_2CO_3$ (1.03 g, 7.47 mmol, 3.0 eq.) was added to a DMF (15 mL) solution of the compound obtained from step 4 ( 1.2 g, 2.49 mmol, 1.0 eq.) and Intermediate 1 (469.06 mg, 2.24 mmol, 0.9 eq.). The mixture was stirred at 25 °C for 16 h, with LCMS confirming complete conversion. After filtering off $K_2CO_3$, DMF was evaporated to dryness. The residue was purified by silica gel column chromatography (100-200 mesh, eluent gradient: DCM:MeOH=100:1 to 10:1), and collected fractions were concentrated to dryness to afford the title compound as a pale yellow solid (1.0 g, 61.34% yield). LCMS (ESI): $[M+H]^+$ = 655.0; $^1$H NMR (400 MHz, DMSO-$d_6$) δ 7.62-7.50 (m, 1H),

7.48-7.40 (m, 1H), 7.39-7.28 (m, 1H), 6.45 (d, J = 7.2 Hz, 1H), 6.35 (s, 1H), 5.76-5.51 (m, 2H), 5.28 (s, 2H), 4.88 (s, 2H), 3.84 (s, 3H, $-NCH_3$), 1.78-1.73 (m, 2H), 1.40 (s, 9H, $-C(CH_3)_3$), 0.92-0.71 (m, 3H).

***Step 6: Preparation of (S)-tert-butyl 2-((4-ethyl-7-fluoro-4-hydroxy-11-methyl-3,6,14-trioxo-3,4,6,11,12,14-hexahydro-1H-pyrano[3',4':6,7]indolizino[2,1-b]quinolin-10-yl)oxy)acetate:***

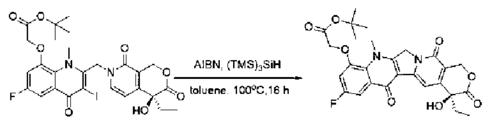


The intermediate obtained from Step 5 (800 mg, 1.22 mmol, 1.0 eq.) and AIBN (100.37 mg, 0.61 mmol, 0.5 eq.) were dissolved in toluene (5 mL) under nitrogen protection, followed by a toluene solution (5 mL) of $(TMS)_3SiH$ (1.52 g, 6.11 mmol, 5.0 eq.). The mixture was refluxed at 100 °C for 16 hours, and LCMS monitoring confirmed complete reaction. After cooling to room temperature, the solid was collected by filtration. The solid was triturated with MTBE (5 mL) and filtered again to obtain the title compound (crude product) as a yellowish solid (128 mg, yield: 20%). LCMS (ESI): $[M+H]^+$ = 527.0.

***Step 7: Preparation of (S)-2-((4-ethyl-7-fluoro-4-hydroxy-11-methyl-3,6,14-trioxo-3,4,6,11,12,14-hexahydro-1H-pyrano[3',4':6,7]indolizino[2,1-b]quinolin-10-yl)oxy)acetic acid:***

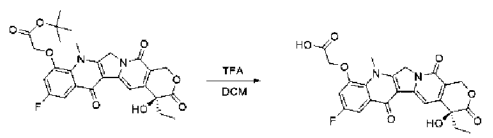


Under nitrogen protection at 0 °C, the intermediate obtained from Step 6 (crude product, 550 mg, 1.04 mmol, 1.0 eq.) was dissolved in a mixed solution of DCM and TFA (v/v = 4:1, 4 mL:1 mL). The reaction mixture was warmed to room temperature and stirred for 1 hour, with LCMS monitoring confirming complete reaction. The crude product (580 mg) was obtained by vacuum distillation and purified by reversed-phase preparative HPLC (C18 column, mobile phase: 0.1% formic acid in water-acetonitrile) to obtain the title compound as a yellowish solid (41 mg, yield: 8.4%).LCMS (ESI) $[M+H]^+$ =471.3. $^1H$ NMR (400 MHz, DMSO-$d_6$) δ 13.32 (s, 1H), 7.65- 7.59 (m, 1H), 7.51-7.45 (m, 1H), 7.26 (s, 1H), 6.35 (s, 1H), 5.40 (d, *J* = 9.2 Hz, 2H), 5.37-5.29 (m, 2H), 5.01 (s, 2H), 4.20 (s, 3H), 1.90-1.71 (m, 2H), 0.86 (t, *J* = 7.2 Hz, 3H).

**Example 18: Preparation of (S)-12-ethyl-12-hydroxy-5-methyl-9,12-dihydro-8H-pyrano[3',4':6,7]indolizino[2,1-b][1,6]diazepine-8,11,14(5H,6H)-trione:**

***Step 1: Preparation of 4-(methylamino)nicotinonitrile:***

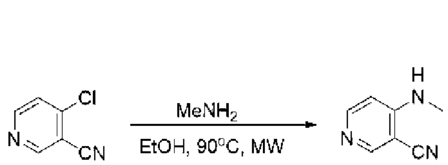


4-Chloronicotinonitrile (1.00 g, 7.25 mmol) was placed in a microwave tube, followed by 2 mol/L methylamine in ethanol (10 mL). The tube was sealed and irradiated under microwave at 90 °C for 1 h. LCMS confirmed complete conversion. The crude product, obtained via reduced-pressure concentration, was triturated with MTBE, filtered, and the filter cake was vacuum-dried to afford the title compound as a white solid (870 mg, 90.2% yield). LCMS (ESI): $[M+H]^+$=134.3.

***Step 2: Preparation of 1-(4-(methylamino)pyridin-3-yl)propan-2-one:***

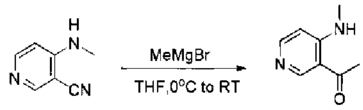


Under $N_2$ protection and ice bath, 4-(methylamino)nicotinonitrile (2.6 g, 19.5 mmol, 1.0 eq.) was dissolved in anhydrous THF (30 mL). 3 mol/L methylmagnesium bromide in THF (13 mL, 39.0 mmol, 2.0 eq.) was added slowly. The ice bath was removed, and the reaction was stirred at rt for 5 h. LCMS confirmed complete conversion. The reaction was quenched with ice-cold saturated aq. $NH_4Cl$ (50 mL), extracted with EA (20 mL×5). Combined organic phases were dried over anhydrous $Na_2SO_4$, filtered, and the filtrate was concentrated under reduced pressure. The crude product was purified by silica gel column chromatography (eluent gradient: DCM:MeOH = 1:0 to 20:1) to afford the title compound as a pale brown solid (1.30 g, 44.4% yield). LCMS (ESI): $[M+H]^+$ = 151.4.

***Step 3: Preparation of ethyl 1-methyl-4-oxo-1,4-dihydro-1,6-naphthyridine-2-carboxylate:***

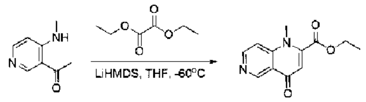


The compound obtained from step 2 (1.3 g, 8.67 mmol, 1.0 eq.) and diethyl oxalate (3.16 g, 21.7 mmol, 2.5 eq.) were dissolved in anhydrous THF (15 mL). Under $N_2$ protection at -65 °C, 1 mol/L LiHMDS in THF (30.3 mL, 30.3 mmol, 3.5 eq.) was added slowly. The dry ice bath was maintained, and the mixture was allowed to warm slowly to rt, then stirred overnight. The reaction was quenched with ice-cold saturated aq. $NH_4Cl$ (50 mL) and extracted with EA (20 mL × 3). Combined organic phases were dried over anhydrous $Na_2SO_4$, concentrated under reduced pressure, and the crude product was purified by silica gel column chromatography (eluent gradient: DCM:MeOH = 1:0 to 30:1) to afford the title compound (420 mg, 20.9% yield). LCMS (ESI): $[M+H]^+$ = 233.3.

***Step 4: Preparation of 2-(hydroxymethyl)-1-methyl-1,6-naphthyridin-4(1H)-one:***

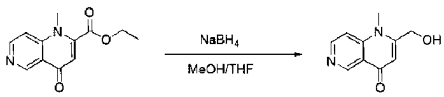


The compound obtained from step 3 (540 mg, 2.33 mmol, 1.0 eq.) was dissolved in a mixed solvent of THF (6 mL) and MeOH (6 mL). Under ice bath conditions, $NaBH_4$ (220 mg, 5.81 mmol, 2.5 eq.) was added slowly, and the reaction was continued for 15 minutes under ice bath. TLC detection confirmed complete reaction. Saturated sodium chloride solution (1 mL) was added to quench the reaction, and the crude product was obtained by concentration under reduced pressure. The crude product was separated and purified by silica gel column chromatography (eluent gradient: DCM:MeOH = 1:0 to 10:1) to obtain the title compound as a white solid (190 mg, yield: 42.8%). LCMS (ESI): $[M + H]^+$ = 189.2. (1)H NMR (400 MHz, DMSO-d(6)) δ 9.23 (s, 1H), 8.69 (d, *J* = 6.2 Hz, 1H), 7.71 (d, *J* = 6.2 Hz, 1H), 6.37 (s, 1H), 5.83 (t, *J* = 5.7 Hz, 1H), 4.61 (d, *J* = 5.6 Hz, 2H), 3.70 (s, 3H).

***Step 5: Preparation of 2-(hydroxymethyl)-3-iodo-1-methyl-1,6-naphthyridin-4(1H)-one:***

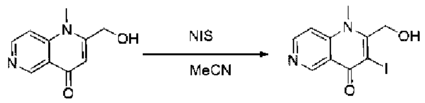


The compound obtained from step 4 (100 mg, 0.530 mmol, 1.0 eq.) was dissolved in acetonitrile (MeCN, 20 mL). N-Iodosuccinimide (NIS, 142 mg, 0.630 mmol, 1.2 eq.) was added, and the reaction was stirred continuously at room temperature for 4 days. TLC detection confirmed complete reaction. Under ice bath, saturated sodium bicarbonate

solution (6 mL) was added to quench the reaction, and the reaction solution was concentrated under reduced pressure to obtain a crude product. The crude product was separated and purified by silica gel column chromatography (eluent gradient: DCM:MeOH = 1:0 to 10:1) to obtain the title compound as a white solid (75.0 mg, yield: 44.8%). LCMS (ESI): $[M + H]^+ = 316.9$; $^1H$ NMR (400 MHz, DMSO-$d_6$) δ 9.29 (s, 1H), 8.76 (d, J = 6.2 Hz, 1H), 7.79 (d, J = 6.2 Hz, 1H), 6.01 (t, J = 5.4 Hz, 1H), 5.12 (d, J = 5.4 Hz, 2H), 3.97 (s, 3H).

***Step 6: Preparation of 2-(chloromethyl)-3-iodo-1-methyl-1,6-naphthyridin-4(1H)-one:***

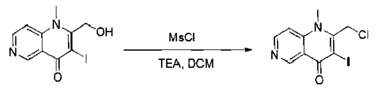


The compound obtained from step 5 (62 mg, 0.200 mmol, 1.0 eq.) was dissolved in anhydrous DCM (5 mL). Under ice bath conditions, TEA (59.5 mg, 0.590 mmol, 3.0 eq.) and MsCl (44.9 mg, 0.390 mmol, 2.0 eq.) were added sequentially, and the reaction was stirred continuously for 1 hour under ice bath conditions. TLC detection confirmed complete reaction. The reaction solution was poured into ice water (20 mL), extracted with DCM (10 mL × 2). The combined organic phases were dried over anhydrous $Na_2SO_4$, filtered, and the filtrate was concentrated to obtain the title compound as a light brown oil (72.0 mg, crude product). LCMS (ESI): $[M+H]^+ = 335.0$.

***Step 7: Preparation of (S)-4-ethyl-4-hydroxy-7-((3-iodo-1-methyl-4-oxo-1,4-dihydro-1,6-naphthyridin-2-yl) methyl)-1,7-dihydro-3H-pyrano[3,4-c]pyridine-3,8(4H)-dione:***

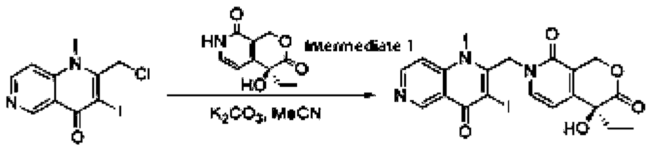


The product from step 6 (73 mg, 0.220 mmol, 1.0 eq.) and Intermediate 1 (36.5 mg, 0.170 mmol, 0.8 eq.) were sequentially dissolved in MeCN (10 mL). $K_2CO_3$ (90.5 mg, 0.650 mmol, 3.0 eq.) was added, and the mixture was heated to 45 °C under $N_2$ protection and stirred overnight. LCMS confirmed complete conversion. Insoluble solids were filtered off, and the filtrate was concentrated under reduced pressure. The crude product was purified by silica gel column chromatography (eluent gradient: DCM:MeOH = 1:0 to 10:1) to afford the title compound as a light brown solid (60.0 mg, 54.2% yield). LCMS (ESI): $[M + H]^+ = 508.0$; $^1H$-NMR (400 MHz, DMSO-$d_6$) δ 9.35 (s, 1H), 8.80 (d, J = 6.3 Hz, 1H), 7.86 (d, J = 6.2 Hz, 1H), 7.54 (d, J = 7.2 Hz, 1H), 6.44 (d, J = 7.2 Hz, 1H), 5.66 (q, J = 32.2, 16.0 Hz, 2H), 5.31 (s, 2H), 3.79 (s, 3H), 1.87-1.66 (m, 2H), 0.81 (t, J = 7.3 Hz, 3H).

***Step 8: Preparation of (S)-12-ethyl-12-hydroxy-5-methyl-9,12-dihydro-8H-pyrano[3',4':6,7]indolizino[2,1-b][1,6] diazepine-8,11,14(5H,6H)-trione:***

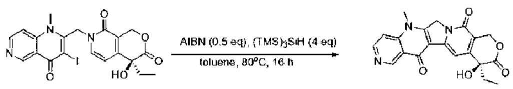


The intermediate from step 7 (60.0 mg, 0.12 mmol, 1.0 eq.) and AIBN (9.71 mg, 0.06 mmol, 0.5 eq.) were added to a dry three-necked flask. The flask was degassed via oil pump vacuum and then argon-protected. Anhydrous toluene (3 mL) was added, followed by $(TMS)_3SiH$ (118 mg, 0.470 mmol, 4.0 eq.). The mixture was heated to 80 °C and stirred for 16 h. After r concentration, the crude product was triturated with MTBE to give a crude solid (40 mg), which was further purified by reversed-phase preparative HPLC to afford the title compound as a light brown solid (1.1 mg, 2.45% yield). LCMS (ESI): $[M+H]^+ = 411.1$; $^1H$ NMR (400 MHz, DMSO-$d_6$) δ 9.44 (s, 1H), 8.86 (d, J = 6.1 Hz, 1H), 7.91 (d, J = 6.1 Hz, 1H), 7.30 (s, 1H), 6.40 (s, 1H), 5.47-5.31 (m, 4H), 3.91 (s, 3H, $-NCH_3$), 1.88-1.76 (m, 2H), 0.88 (t, J = 7.2 Hz, 3H).

**Example 19: Preparation of (S)-7-ethyl-3-fluoro-7-hydroxy-14-methyl-10,14-dihydro-11H-pyrano[3',4':6,7]indolizino[2,1-b][1,8]diazepine-5,8,11(7H,13H)-trione:**

***Step 1: Preparation of ethyl 6-fluoro-1-methyl-4-oxo-1,4-dihydro-1,8-naphthyridine-2-carboxylate:***

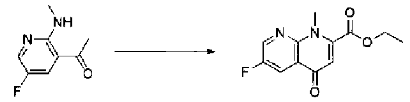

1-(5-Fluoro-2-(methylamino)pyridin-3-yl)ethanone (1.77 g, 10.53 mmol, 1.0 eq.) was dissolved in anhydrous THF (20 mL). Under $N_2$ at -78 °C, 1 mol/L LiHMDS in THF (26.31 mL, 2.5 eq.) was added dropwise, stirred 1 h, then diethyl oxalate (3.85 mL, 2.5 eq.) was added at -78 °C and stirred 1 h. After removing the low-temperature bath, the mixture was warmed to rt, heated to 75 °C and stirred overnight. LCMS confirmed full conversion. Quenched with ice-cold saturated aq. $NH_4Cl$, extracted with EA, organic phases dried over $Na_2SO_4$ and concentrated. Crude was purified by silica gel column chromatography (100-200 mesh, eluent gradient: PE:EA = 10:1 to 5:1) to afford the title compound as a yellow solid (1.1 g, 41.75% yield). LCMS (ESI): $[M+H]^+$=251.1.

***Step 2: Preparation of 6-fluoro-2-(hydroxymethyl)-1-methyl-1,8-naphthyridin-4(1H)-one:***

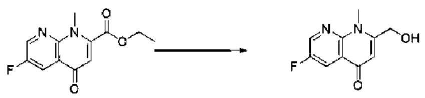

The intermediate from step 1 (1.1 g, 4.4 mmol, 1.0 eq.) was added to MeOH (10 mL). $NaBH_4$ (, 332.6 mg, 8.79 mmol, 2.0 eq.) was added at 20 °C, and the reaction was stirred for 16 hours. LCMS monitoring confirmed complete reaction. The reaction solution was diluted with water, and 2 mol/L hydrochloric acid was added dropwise to adjust the pH to 5-6, resulting in precipitation of a white solid. The solid was collected by filtration, washed twice with water, and drained to obtain the title compound as a white solid (700 mg, yield: 76.49%). LCMS (ESI): $[M+H]^+$ = 209.1.

***Step 3: Preparation of 6-fluoro-2-(hydroxymethyl)-3-iodo-1-methyl-1,8-naphthyridin-4(1H)-one:***

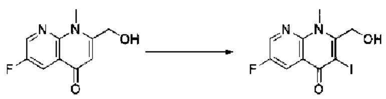

The intermediate from step 2 (140 mg, 0.67 mmol, 1.0 eq.) was dissolved in AcOH (2 mL). NIS (226.94 mg, 1.01 mmol, 1.5 eq.) was added, and the mixture was stirred at 25 °C for 16 h. LCMS confirmed full conversion, poured into saturated aq. $NH_4Cl$, extracted with EA, organic phases dried over anhydrous $Na_2SO_4$ and concentrated. The crude was purified by silica gel column chromatography (100-200 mesh, eluent: PE:EA = 1:1) to afford the title compound as a yellow solid (180 mg, 80.12% yield). LCMS (ESI): $[M+H]^+$ = 335.0.

***Step 4: Preparation of 2-(chloromethyl)-6-fluoro-3-iodo-1-methyl-1,8-naphthyridin-4(1H)-one:***

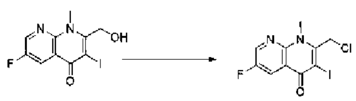

The intermediate from step 3 (140 mg, 0.42 mmol, 1.0 eq.) was dissolved in ultra-dry THF (4 mL). $SOCl_2$ (249.27 mg, 2.1 mmol, 5.0 eq.) was added at rt, and the mixture was stirred for 5 h. LCMS confirmed full conversion. The reaction

was poured into saturated aq. $NH_4Cl$, extracted with EA, organic phases dried over anhydrous $Na_2SO_4$ and concentrated. Crude product was purified by silica gel column chromatography (100-200 mesh, eluent: PE:EA = 3:1) to afford the title compound as a yellow solid (130 mg, 87.9% yield). LCMS (ESI): $[M + H]^+$ = 353.0.

***Step 5: Preparation of (S)-4-ethyl-7-((6-fluoro-3-iodo-1-methyl-4-oxo-1,4-dihydro-1,8-naphthyridin-2-yl) methyl)-4-hydroxy-1,7-dihydro-3H-pyrano[3,4-c]pyridine-3,8(4H)-dione:***

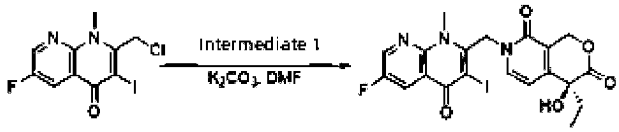


The intermediate from step 4 (140 mg, 0.42 mmol, 1.0 eq.) was dissolved in ultra-dry THF (4 mL). $SOCl_2$ (249.27 mg, 2.1 mmol, 5.0 eq.) was added at rt, and the mixture was stirred for 5 h. LCMS confirmed full conversion. The reaction was poured into saturated aq. $NH_4Cl$, extracted with EA, organic phases dried over anhydrous $Na_2SO_4$ and concentrated. Crude product was purified by silica gel column chromatography (100-200 mesh, eluent: PE:EA = 3:1) to afford the title compound as a yellow solid (130 mg, 87.9% yield). LCMS (ESI): $[M + H]^+$ = 353.0.

***Step 6: Preparation of (S)-7-ethyl-3-fluoro-7-hydroxy-14-methyl-10,14-dihydro-11H-pyrano[3',4':6,7]indolizino [2,1-b][1,8]diazepine-5,8,11(7H,13H)-trione:***

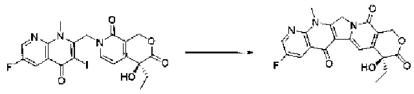

The intermediate obtained from Step 5 (150 mg, 0.29 mmol, 1.0 eq.) was dissolved in toluene (5 mL). Under an nitrogen atmosphere, AIBN (, 23.45 mg, 0.14 mmol, 0.5 eq.) and $(TMS)_3SiH$ (284.04 mg, 1.14 mmol, 4.0 eq.) were added. The mixture was heated to 90 °C and reacted for 16 hours. LCMS detection confirmed complete reaction. The reaction solution was concentrated under reduced pressure to obtain a crude product, which was purified by reversed-phase preparative HPLC to obtain the title compound as a yellow solid (13.0 mg, yield: 11.28%). $^1H$ NMR (400 MHz, DMSO-$d_6$) $\delta$ 9.01 (d, J = 3.0 Hz, 1H), 8.48-8.45 (m, 1H), 7.26 (s, 1H), 5.48 (s, 2H), 5.36-5.31 (m, 2H), 4.01 (s, 3H $_3$), 1.85-1.79 (m, 2H), 0.86 (t, J = 7.3 Hz, 3H); LCMS (ESI): $[M + H]^+$ = 398.2.

**Example 20: Preparation of (S)-10-amino-4-ethyl-8-fluoro-4-hydroxy-11-methyl-1,12-dihydro-14H-pyrano [3',4':6,7]indolizino[2,1-b]quinoline-3,6,14(4H,11H)-trione:**

***Step 1: Preparation of ethyl 6-fluoro-1-methyl-4-oxo-8-((trifluoromethyl)sulfonyl)oxy-1,4-dihydroquinoline-2-carboxylate:***

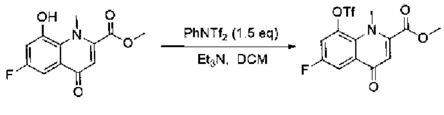


The intermediate obtained from step 1 of Example 11 (10 g, 39.8 mmol, 1.0 eq.) was dissolved in DCM (200 mL). Under ice bath and $N_2$ protection, TEA (12.9 g, 120 mmol, 3.0 eq.) and N-phenylbis(trifluoromethanesulfonimide) (21.3 g, 59.7 mmol, 1.5 eq.) were added sequentially. The ice bath was removed, and the mixture was stirred at rt for 16 h. LCMS confirmed full conversion. Concentrated under reduced pressure, the crude was purified by silica gel column chromatography (eluent gradient: PE:EA = 1:0 to 5:1) to afford the title compound as a white solid (10 g, 65.6% yield). LCMS (ESI): [M + H]$^+$ = 384.1.

***Step 2: Preparation of ethyl 8-((diphenylmethylene)amino)-6-fluoro-1-methyl-4-oxo-1,4-dihydroquinoline-2-carboxylate:***

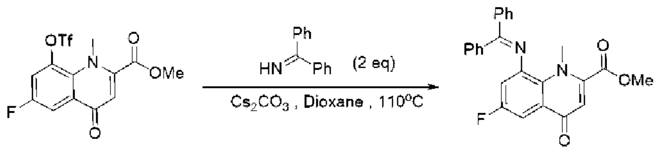


The intermediate obtained from step 1 (12 g, 31.3 mmol, 1.0 eq.) was dissolved in 1,4-dioxane (120 mL). Diphenylketimine (11.4 g, 62.6 mmol, 2.0 eq.), Xantphos (3.62 g, 6.26 mmol, 0.2 eq.), $Cs_2CO_3$ (30.6 g, 93.9 mmol, 3.0 eq.) and $Pd_2(dba)_3$ (2.87 g, 3.13 mmol, 0.1 eq.) were added sequentially. Under $N_2$ protection, the mixture was heated to 110 °C for 1.5 h. LCMS confirmed full conversion. Filtered through diatomaceous earth, the organic phase was concentrated under reduced pressure. The crude was purified by silica gel column chromatography (eluent: PE:EA = 3:1) to afford the title compound as a yellow solid (6 g, 46.2% yield). LCMS (ESI): $[M + H]^+$ = 415.5.

***Step 3: Preparation of 8-((diphenylmethylene)amino)-6-fluoro-2-(hydroxymethyl)-1-methylquinolin-4(1H)-one:***

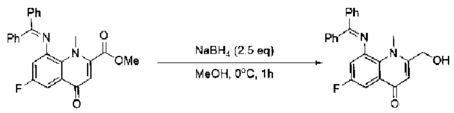


The intermediate obtained from step 2 (6 g, 14.5 mmol, 1.0 eq.) was dissolved in MeOH (60.0 mL). Under ice bath, $NaBH_4$ (1.38 g, 36.2 mmol, 2.5 eq.) was added, and the mixture was stirred at 0 °C for 1 h. LCMS confirmed full conversion. Poured into ice water, extracted with EA, combined organic phases concentrated to give the title compound as a yellow solid (5.1 g, crude). LCMS (ESI): $[M + H]^+$ = 387.2.

***Step 4: Preparation of 8-((diphenylmethylene)amino)-6-fluoro-2-(hydroxymethyl)-3-iodo-1-methylquinolin-4(1H)-one:***

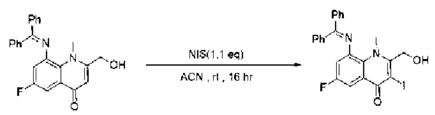


The intermediate obtained from step 3 (5.0 g, 12.9 mmol, 1.0 eq.) was dissolved in acetonitrile (60.0 mL). NIS (3.2 g, 14.2 mmol, 1.1 eq.) was then added, and the reaction mixture was stirred at room temperature for 16 hours. LCMS monitoring confirmed complete reaction. The reaction solution was evaporated to dryness under reduced pressure, and the residue was purified by silica gel column chromatography (eluent: PE:EA = 1:1) to afford the target compound as a yellow solid (6 g, yield: 90.5%). LCMS (ESI): $[M + H]^+$ = 513.4.

***Step 5: Preparation of 2-(Chloromethyl)-8-((diphenylmethylene)amino)-6-fluoro-3-iodo-1-methylquinolin-4(1H)-one:***

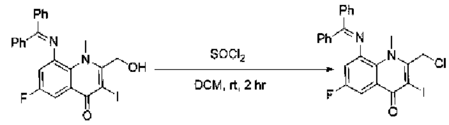


The intermediate obtained from step 4 (6 g, 11.7 mmol, 1.0 eq.) was dissolved in DCM (500 mL). Under ice bath

conditions, $SOCl_2$ (, 19.5 g, 164 mmol, excess) was added. The mixture was stirred at room temperature for 2 hours, and LCMS monitoring indicated complete reaction. The mixture was concentrated, then saturated sodium bicarbonate solution was added to the residue. The mixture was extracted with DCM, and the combined organic phases were dried over anhydrous sodium sulfate. After filtration and concentration, the title compound was obtained as a yellow solid (5.5 g, crude product, yield not calculated). LCMS (ESI): $[M + H]^+$ = 531.1.

***Step 6: Preparation of (S)-7-((8-((diphenylmethylene)amino)-6-fluoro-3-iodo-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

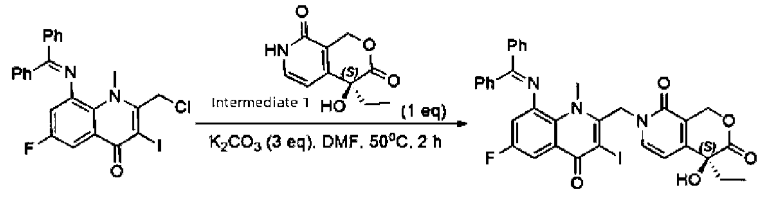


The intermediate obtained from step 5 (5.5 g, 10.4 mmol, 1.0 eq.) was dissolved in DMF (80.0 mL). Then Intermediate 1 (2.02 g, 10.4 mmol, 1.0 eq.) and $K_2CO_3$ (4.3 g, 31.1 mmol, 3.0 eq.) were added sequentially, and the mixture was reacted at 50 °C for 2 hours. LCMS monitoring showed the reaction was complete. The reaction mixture was poured into water and extracted with EA. The aqueous layer was separated, adjusted to acidic pH with dilute hydrochloric acid, and extracted again with EA. The combined organic phases were concentrated under reduced pressure, and the residue was purified by silica gel column chromatography (eluent: PE:EA = 1:1) to obtain the title compound as a yellow solid (4.5 g, yield: 61.7%). LCMS (ESI): $[M + H]^+$ = 704.1.

***Step 7: Preparation of (S)-10-((diphenylmethylene)amino)-4-ethyl-8-fluoro-4-hydroxy-11-methyl-1,12-dihydro-14H-pyrano[3',4':6,7]indolizino[2,1-b]quinoline-3,6,14(4H,11H)-trione:***

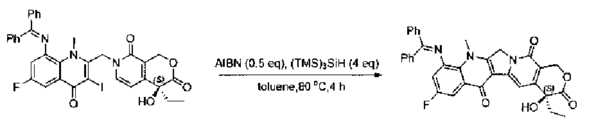


The intermediate obtained from step 6 (4.5 g, 6.4 mmol, 1.0 eq.) and AIBN (, 525 mg, 3.2 mmol, 0.5 eq.) were added to a dry three-necked flask. The flask was evacuated with an oil pump for degassing, then maintained under nitrogen protection. Anhydrous toluene (250 mL) was added, and $(TMS)_3SiH$ (6.36 g, 25.6 mmol, 4.0 eq.) was diluted with a small amount of toluene before being added to the reaction mixture. The reaction was allowed to proceed at 80 °C for 4 hours, and LCMS monitoring confirmed complete reaction. The reaction mixture was concentrated, and purified by silica gel column chromatography (eluent: DCM:MeOH = 32:1) to obtain the title product as a yellow solid (2 g, yield: 54.3%). LCMS (ESI): $[M + H]^+$ = 576.2.

***Step 8: Preparation of (S)-10-amino-4-ethyl-8-fluoro-4-hydroxy-11-methyl-1,12-dihydro-14H-pyrano[3',4':6,7]indolizino[2,1-b]quinoline-3,6,14(4H,11H)-trione:***

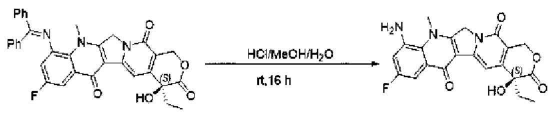


The intermediate obtained from Step 7 (150 mg, 0.26 mmol, 1.0 eq.) was dissolved in a mixed solvent of MeOH and water (7.0 mL, volume ratio 5:2). Concentrated hydrochloric acid (36%, 5.0 mL) was added, and the reaction solution was stirred at room temperature for 16 hours. LCMS analysis indicated the reaction was complete. The reaction mixture was evaporated to dryness, and the residue was triturated with ethanol to give the title compound (55 mg, crude). The

crude product was purified by preparative HPLC (mobile phase: acetonitrile-0.5% aqueous formic acid) to yield the title compound as a white solid (35 mg, yield: 32.7%). LCMS (ESI): $[M + H]^+ = 412.3$.

Example 21: Preparation of (S)-9-Amino-4-ethyl-8-fluoro-4-hydroxy-11-cyclopropyl-1,12-dihydro-14H-pyrano[3,4:6,7] indazolo[2,1 -b]quinoline-3,6,14(4H,11H)-trione

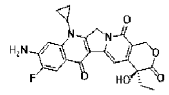

Starting from Intermediate 5 obtained from Example 5), the relevant synthesis was carried out according to steps 1-6 of Example 8, affording the title compound as a white solid. LCMS (ESI): $[M + H]^+ = 438.2$; $^1$H NMR (400 MHz, DMSO-*$d_6$*) δ 7.69 (d, J = 11.5 Hz, 1H), 7.38 (d, J = 7.7 Hz, 1H), 7.21 (s, 1H), 6.11-6.54 (m, 1H), 5.34-5.24 (m, 4H), 3.46-3.41 (m, 1H), 1.80-1.72 (m, 2H), 1.28-1.34 (m, 2H), 1.26-1.20 (m, 2H), 0.81 (t, J = 7.1 Hz, 3H).

Example 22: Preparation of (S)-4-ethyl-8-fluoro-4-hydroxy-9-(2-hydroxypropane-2-yl)-11-methyl-1,12-dihydro-14H-pyrano[3,4:6,7]indolizino [2,1-b]quinoline-3,6,14(4H,11H)-trione:

*Step 1: Preparation of 6-fluoro-7-(2-hydroxypropane-2-yl)-1,2-dimethylquinolin-4(1H)-one:*

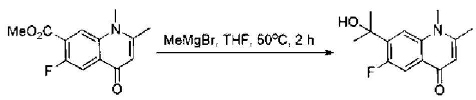


Methyl 6-fluoro-1,2-dimethyl-4-oxo-1,4-dihydroquinoline-7-carbamate (2 g, 8.02 mmol, 1.0 eq.) was dissolved in tetrahydrofuran (THF, 100 mL) under nitrogen protection at 0 °C. Methylmagnesium bromide (48 mL, 1 mol/L in THF, 48.15 mmol, 6.0 eq.) was slowly added dropwise. The reaction mixture was allowed to warm to room temperature naturally, then heated to 50 °C and stirred for 2 hours. LCMS monitoring showed complete reaction. The crude product was purified by flash column chromatography (eluent gradient: DCM:MeOH = 100:1 to 10:1, v/v) to obtain the title compound as a white solid (1.1 g, yield: 55.02%). LCMS (ESI): m/z = 250.0 $[M + H]^+$; $^1$H-NMR (400 MHz, $(CD_3)_2SO$) δ 7.96 (d, J = 6.4 Hz, 1H), 7.71 (d, J = 12.0 Hz, 1H), 6.04 (s, 1H), 3.74 (s, 3H), 2.47 (s, 3H), 1.54 (s, 6H).

***Step 2: Preparation of 3-bromo-6-fluoro-7-(2-hydroxypropane-2-yl)-1,2-dimethylquinolin-4(1H)-one:***

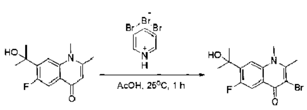


The intermediate obtained from step 1 (1 g, 4.01 mmol, 1.0 eq.) was dissolved in AcOH (10 mL). Pyridinium tribromide (1.28 g, 4.01 mmol, 1.0 eq.) was added, and the reaction was stirred at 25 °C for 1 hour. LCMS monitoring showed complete reaction. EA (10 mL) and water (10 mL) were added to the solution, and a solid precipitated. The solid was collected by filtration, and the organic phase was washed with saturated brine, then concentrated to obtain the title compound as a white solid (1.1 g, yield: 83.55%). LCMS (ESI): m/z = 330.0 $[M + H]^+$; $^1$H-NMR (400 MHz, $(CD_3)_2SO$) δ 8.03 (s, 1H), 7.77 (d, J = 11.2 Hz, 1H), 3.89 (s, 3H), 2.82 (s, 3H), 1.55 (s, 6H).

***Step 3: Preparation of 3-bromo-2-(bromomethyl)-6-fluoro-7-(2-hydroxypropane-2-yl)-1-methylquinolin-4(1H)-one:***

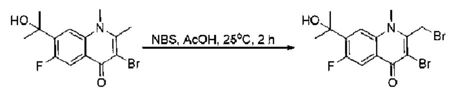


The intermediate obtained from Step 2 (1.1 g, 3.35 mmol, 1.0 eq.) was dissolved in AcOH (10 mL). N-Bromosuccinimide (NBS, 715.48 mg, 4.02 mmol, 1.2 eq.) was added, and the reaction was stirred at 25 °C for 2 hours. LCMS monitoring showed complete reaction. The mixture was extracted with EA, and the organic phase was washed with saturated brine, concentrated under reduced pressure. The crude product was purified by flash column chromatography (eluent gradient: DCM:MeOH = 100:1 to 20:1, v/v) to obtain the title compound as a yellow solid (1.1 g, yield: 80.66%). LCMS (ESI): m/z = 407.8 $[M + H]^+$; $^1$H-NMR (400 MHz, $(CD_3)_2SO$) δ 8.08 (d, J = 6.4 Hz, 1H), 7.79 (d, J = 11.6 Hz, 1H), 5.10 (s, 2H), 3.99 (s, 3H), 1.55 (s, 6H).

***Step 4: Preparation of (S)-7-((3-bromo-6-fluoro-7-(2-hydroxypropane-2-yl)-1-methyl-4-oxo-1,4-dihydroquinolin-2-yl)methyl)-4-ethyl-4-hydroxy-1,7-dihydro-3H-pyrano [3,4-c]pyridine-3,8(4H)-dione:***

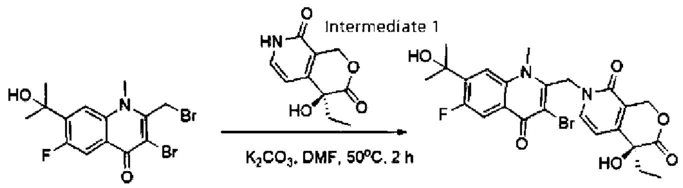


The intermediate obtained from Step 3 (500 mg, 1.23 mmol, 1.0 eq.) and Intermediate 1 (282.65 mg, 1.35 mmol, 1.1 eq.) were dissolved in DMF (10 mL). $K_2CO_3$ (339.52 mg, 2.46 mmol, 2.0 eq.) was added, and the reaction was carried out at 50 °C for 2 hours under nitrogen protection. LCMS monitoring confirmed the formation of the target product. The reaction mixture was filtered, and the filtrate was concentrated under reduced pressure. The crude product was purified by flash column chromatography (100-200 mesh silica gel, eluent gradient: DCM:MeOH = 100:1 to 20:1, v/v) to obtain the title compound as a white solid (500 mg, yield: 75.93%). LCMS (ESI): m/z = 535.20 $[M + H]^+$; $^1$H NMR (400 MHz, $CDCl_3$) δ 8.04 (d, J = 11.2 Hz, 1H), 7.95 (d, J = 6.4 Hz, 1H), 7.30 (d, J = 7.2 Hz, 1H), 6.56 (d, J = 7.2 Hz, 1H), 5.95 (d, J = 15.6 Hz, 1H), 5.74 (d, J = 15.6 Hz, 1H), 5.61 (d, J = 16.4 Hz, 1H), 5.21 (d, J = 16.4 Hz, 1H), 3.79 (s, 3H), 1.84-1.75 (m, 2H), 1.69 (s, 6H), 0.97 (t, J = 7.2 Hz, 3H).

***Step 5: Preparation of (S)-4-ethyl-8-fluoro-4-hydroxy-9-(2-hydroxypropane-2-yl)-11-methyl-1,12-dihydro-14H-pyrano[3,4:6,7]indolizino[2,1 -b]quinoline-3,6,14 (4H,11H)-trione:***

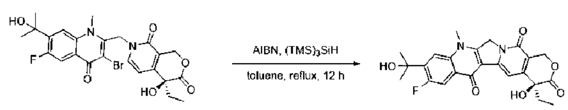


The compound obtained from step 4 (300 mg, 0.56 mmol, 1.0 eq.) was dissolved in toluene (20 mL). AIBN (18.4 mg, 0.11 mmol, 0.2 eq.) and $(TMS)_3SiH$ (418.03 mg, 1.68 mmol, 3.0 eq.) were added, and the reaction was carried out at 130 °C for 12 hours under nitrogen protection. LCMS monitoring showed the formation of the target product and the complete disappearance of the starting material. MeOH (5 mL) was added to the reaction mixture, and the solvent was removed by vacuum evaporation. The residue was purified by preparative HPLC (mobile phase: acetonitrile-0.5% aqueous formic acid) to obtain the title compound (37.6 mg, yield: 15%). LCMS (ESI): m/z = 455.20 $[M + H]^+$; $^1$H NMR (400 MHz, $(CD_3)_2SO$) δ 8.09 (d, J = 6.4 Hz, 1H), 7.91 (d, J = 11.6 Hz, 1H), 7.27 (s, 1H), 6.34 (s, 1H), 5.70 (s, 1H), 5.40 (d, J = 6.8 Hz, 2H), 5.34 (d, J = 5.2 Hz, 2H), 3.92 (s, 3H), 1.83-1.80 (m, 2H), 1.58 (s, 6H), 0.87 (t, J = 7.2 Hz, 3H).

Example 23: Preparation of (S)-10-(2-ethoxyethyl)-4-ethyl-8-fluoro-4-hydroxy-11-methyl-1,12-dihydro-14H-pyrano [3,4:6,7]indolizino[2,1 -b]quinoline-3,6,14 (4H,11H)-trione:

*Step 1: Preparation of methyl (E)-8-(2-ethoxyvinyl)-6-fluoro-1-methyl-4-oxo-1,4-dihydroquinoline-2-carboxylate:*

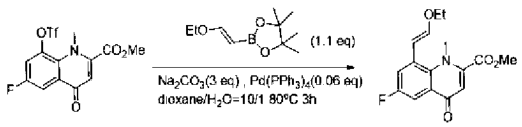


The intermediate from step 1 of Example 20 (5 g, 13.1 mmol, 1.0 eq.) was dissolved in a mixed solvent of 1,4-dioxane (100mL) and water (10mL). Then (E)-2-ethoxyvinylboronic acid pinacol ester (2.84 g, 14.4 mmol, 1.1 eq.), sodium carbonate (4.15 g, 39.1 mmol, 3.0 eq.) and $Pd(PPh_3)_4$ (905 mg, 0.78 mmol, 0.06 eq.) were added sequentially. After the addition, the reaction solution was heated to 80 °C and stirred for 3 hours under nitrogen protection. LCMS monitoring showed the reaction was complete. The reaction mixture was filtered, and the filtrate was concentrated to obtain a crude product, which was separated and purified by silica gel column chromatography (eluent gradient: PE:EA = 1:0 to 1:4, v/v) to obtain the title compound as a pale yellow solid (2.5 g, yield: 62.7%). LCMS (ESI): $[M + H]^+$ = 306.3.

***Step 2: Preparation of methyl 8-(2-ethoxyethyl)-6-fluoro-1-methyl-4-oxo-1,4-dihydroquinoline-2-carboxylate:***

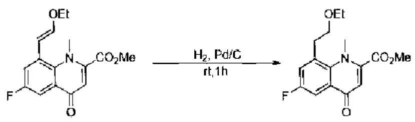


The intermediate from step 1 (100 mg, 0.33 mmol, 1.0 eq.) was dissolved in MeOH (10 mL). Palladium on carbon (Pd/C, 10 mg) was added, and the system was purged with hydrogen. The reaction was conducted at room temperature for 2 hours. LCMS analysis confirmed the reaction was complete. The mixture was filtered, and the filtrate was concentrated under reduced pressure to obtain the title compound as a solid (80.0 mg, yield: 78.8%). LCMS (ESI): $[M + H]^+$ = 308.6.

***Step 3: Preparation of 8-(2-ethoxyethyl)-6-fluoro-2-(hydroxymethyl)-1-methylquinolin-4(1H)-one:***

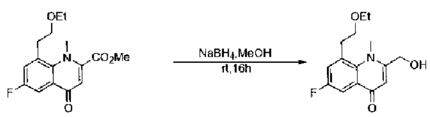


The intermediate from step 2 (84.0 mg, 0.280 mmol, 1.0 eq.) was dissolved in MeOH (5 mL). The solution was cooled in an ice bath, and $NaBH_4$ (26.2 mg, 0.690 mmol, 2.5 eq.) was added. The reaction mixture was stirred at room temperature overnight, and LCMS monitoring confirmed complete reaction. The reaction was quenched with water, concentrated under reduced pressure, and the crude product was purified by silica gel column chromatography (eluent: DCM:MeOH = 4:1, v/v) to obtain the title compound as a white solid (40.0 mg, yield: 51.2%). LCMS (ESI): $[M + H]^+$ = 280.1.

***Step 4: Preparation of 8-(2-ethoxyethyl)-6-fluoro-2-(hydroxymethyl)-3-iodo-1-methylquinolin-4(1H)-one:***

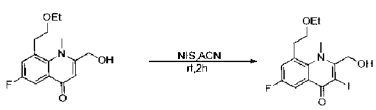


The intermediate from step 3 (160 mg, 0.573 mmol, 1.0 eq.) was dissolved in MeCN (20 mL). NIS (, 155 mg, 0.690 mmol, 1.2 eq.) was added, and the mixture was stirred at room temperature for 2 hours. LCMS monitoring confirmed complete reaction. The reaction mixture was concentrated, and the crude product was purified by silica gel column chromatography (eluent: EA:PE = 10:1, v/v) to obtain the title compound as a yellow solid (200 mg, yield: 86.6%). LCMS (ESI): $[M+H]^+$=406.2.

***Step 5: Preparation of 2-(chloromethyl)-8-(2-ethoxyethyl)-6-fluoro-3-iodo-1-methylquinolin-4(1H)-one :***

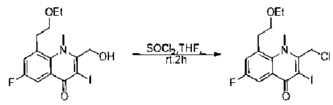


The intermediate from step 4 (200 mg, 0.490 mmol, 1.0 eq.) was dissolved in THF (50 mL). Thionyl chloride ($SOCl_2$, 2 mL) was added, and the mixture was stirred at room temperature for 2 hours. LCMS confirmed the reaction was complete. The mixture was concentrated under reduced pressure, and the residue was dissolved in EA (20 mL). Under ice bath conditions, the pH was adjusted to ~8 with saturated $NaHCO_3$ solution. The mixture was then extracted with EA (20 mL × 2), and the combined organic layers were dried over anhydrous $Na_2SO_4$, filtered, and concentrated to afford the title compound as a yellow solid (200 mg, yield: 95.0%). LCMS (ESI): $[M+H]^+$=424.1.

***Step 6: Preparation of (S)-7-((8-(2-ethoxyethyl)-6-fluoro-3-iodo-1-methyl-4-oxo-1,4-dihydroquinolin-2-yl) methyl)-4-ethyl-4-hydroxy-1,7-dihydro-3H-pyrano[3,4-c]pyridine-3,8(4H)-dione :***

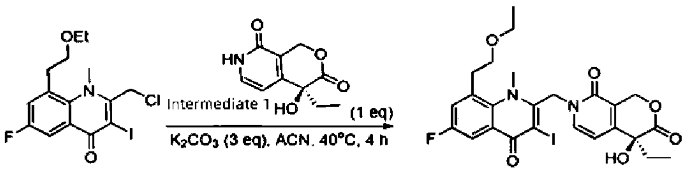


The intermediate obtained from Step 5 (200 mg, 0.473 mmol, 1.0 eq.), Intermediate 1 (98.8 mg, 0.426 mmol, 0.9 eq.) and $K_2CO_3$ (196 mg, 1.42 mmol, 3.0 eq.) were added to MeCN (10 mL). The mixture was heated to 40 °C and stirred for 4 hours. LCMS detection confirmed the reaction was complete. The pH was adjusted to 5 with 1 mol/L dilute HCl, and the mixture was extracted with EA (20 mL × 2). The combined organic phases were dried over anhydrous $Na_2SO_4$, filtered, and the filtrate was concentrated to obtain a crude product. The crude product was separated and purified by silica gel column chromatography (eluent: EA:PE = 1:1, v/v) to obtain the title compound as a pale brown solid (70.0 mg, yield: 24.8%). LCMS (ESI): $[M + H]^+$ = 597.2.

***Step 7: Preparation of (S)-10-(2-ethoxyethyl)-4-ethyl-8-fluoro-4-hydroxy-11-methyl-1,12-dihydro-14H-pyrano [3,4:6,7]indolizino[2,1 -b]quinoline-3,6,14(4H,11H)-trione:***

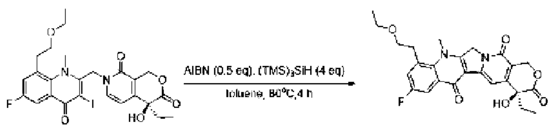


The intermediate obtained from Step 6 (50.0 mg, 0.084 mmol, 1.0 eq.) and AIBN (6.57 mg, 0.042 mmol, 0.5 eq.) were added to a three-necked flask. The flask was evacuated three times with an oil pump, then toluene (10 mL) was added. $(TMS)_3SiH$ (83 mg, 0.335 mmol, 4.0 eq.) was dissolved in toluene (2 mL) and added to the reaction solution. After the addition, nitrogen was purged three times, and the reaction solution was heated to 80 °C and stirred for 4 hours. LCMS detected that the reaction was complete. The reaction mixture was concentrated, and the residue was purified by preparative HPLC to obtain the title compound as a white solid (3.5 mg, yield: 9.35%).

Example 24: Preparation of (S)-4-ethyl-8,10-difluoro-4-hydroxy-11-methyl-1,12-dihydro-14H-pyrano[3,4:6,7]indolizino [2,1 -b]quinoline-3,6,14(4H,11H)-trione:

*Step 1: Preparation of dimethyl 2-((2,4-difluorophenyl)amino)maleate :*

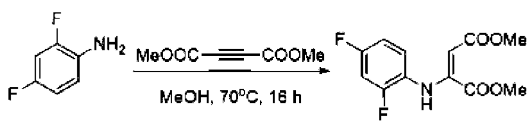


2,4-Difluoroaniline (18 g, 139.4 mmol, 1.0 eq.) and dimethyl acetylenedicarboxylate (21.3 g, 159.3 mmol, 1.1 eq.) were dissolved in MeOH (180 mL), and the mixture was stirred at 70 °C for 16 hours. LCMS monitoring confirmed the reaction was complete. The solvent was removed by rotary evaporation, and the residue was triturated with MeOH (50 mL) to obtain the title compound as a yellowish-green solid (29 g, yield: 77%). LCMS (ESI): $[M + H]^+$ = 254.2.

***Step 2: Preparation of dimethyl 2-((2,4-difluorophenyl)(methyl)amino)maleate:***

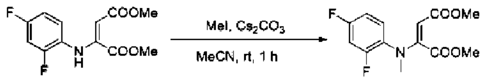


The intermediate obtained from step 1(20 g, 73.7 mmol, 1.0 eq.) was dissolved in MeCN (200 mL). $Cs_2CO_3$(71 g, 219 mmol, 3.0 eq.) was added, and iodomethane ($CH_3I$, 11.4 g, 80 mmol, 1.1 eq.) was added under nitrogen protection. The reaction was conducted at 25 °C for 1 hour. LCMS monitoring confirmed the reaction was complete. Water (200 mL) was added to quench the reaction, and the mixture was extracted with EA (80 mL × 3). The combined organic phases were dried, filtered, and concentrated by rotary evaporation. The crude product was separated and purified by silica gel column chromatography (100-200 mesh silica gel, eluent: PE:EA = 5:1, v/v) to obtain the title compound as a white solid (13 g, yield: 61.9%). LCMS (ESI): $[M + H]^+$ = 286.1.

***Step 3: Preparation of methyl 6,8-difluoro-1-methyl-4-oxo-1,4-dihydroquinoline-2-carboxylate:***

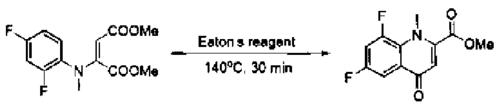


The intermediate obtained from step 2 (12.7 g, 44.5 mmol, 1.0 eq.) was added in three batches to preheated Eatons reagent (96 g, 1157 mmol, 26.09 eq.) at 140 °C, with 10 minutes interval between each batch. LCMS monitoring confirmed the reaction was complete. When the reaction mixture was cooled to room temperature, it was poured into ice-cold saturated $NaHCO_3$ solution (300 mL), stirred, and the precipitated product was collected by filtration to obtain the title compound as a brown solid (3.9 g, yield: 35%). LCMS (ESI): $[M + H]^+$ = 254.1.

*Step 4: Preparation of 6,8-difluoro-2-(hydroxymethyl)-1-methylquinolin-4(1H)-one:*

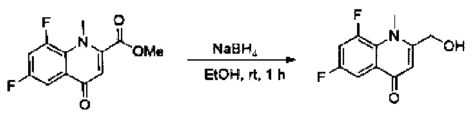


The intermediate obtained from step 3 (3.7 g, 14.6 mmol, 1.0 eq.) was dissolved in EtOH (100 mL). Under an ice bath, $NaBH_4$ (1.4 g, 36.5 mmol, 2.5 eq.) was added, and the mixture was stirred at room temperature for 1 hour. The reaction was quenched with ice water, and the mixture was extracted with EA (30 mL × 4). The combined organic phases were concentrated to obtain a crude product, which was triturated with MeOH at room temperature to obtain the title compound as a white solid (1.1 g, yield: 33.3%). LCMS (ESI): $[M + H]^+$ = 226.1; $^1H$ NMR (400 MHz, DMSO-$d_6$) δ 7.87-7.53 (m, 2H), 6.29 (s, 1H), 5.81 (t, J = 5.8 Hz, 1H), 4.59 (d, J = 5.9 Hz, 2H), 3.84 (s, 3H).

***Step 5: Preparation of 6,8-difluoro-2-(hydroxymethyl)-3-iodo-1-methylquinolin-4(1H)-one:***

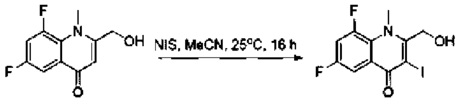


NIS (959 mg, 4.26 mmol, 1.2 eq.) was added to a MeCN (70 mL) solution of the intermediate obtained from Step 4 ( 800 mg, 3.55 mmol, 1.0 eq.). The mixture was stirred at 25 °C for 16 hours, and LCMS monitoring confirmed the reaction was complete. The reaction solution was concentrated, and the crude product was separated and purified by silica gel column chromatography (100-200 mesh silica gel, eluent: PE:EA = 3:1, v/v) to obtain the title compound as a yellow solid (1.2 g, yield: 96.3%). LCMS (ESI): $[M + H]^+ = 352.1$; $^1$H-NMR (400 MHz, $CDCl_3$) δ 7.91-7.81 (m, 1H), 7.23-7.18 (m, 1H), 5.16 (d, J = 7.0 Hz, 2H), 4.15 (s, 3H).

***Step 6: Preparation of 2-(chloromethyl)-6,8-difluoro-3-iodo-1-methylquinolin-4(1H)-one:***

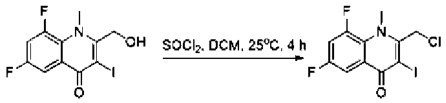


Under an ice water bath, $SOCl_2$ (1.22 g, 10.3 mmol, 3.0 eq.) was added to an anhydrous DCM (10 mL) solution of the intermediate obtained from step 5 (1.2 g, 3.42 mmol, 1.0 eq.). The mixture was stirred at 25 °C for 4 hours, and TLC monitoring confirmed the reaction was complete. The mixture was concentrated, and the crude was purified by flash silica gel column chromatography (eluent: DCM:MeOH = 50:1, v/v) to obtain the title compound as a yellow solid (1.0 g, yield: 79.1%). $^1$H-NMR (400 MHz, $(CD_3)_2SO$) δ 7.90-7.83 (m, 1H), 7.71-7.69 (m, 1H), 5.25 (s, 2H), 4.09 (d, J = 7.6 Hz, 3H).

***Step 7: Preparation of (S)-7-((6,8-difluoro-3-iodo-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

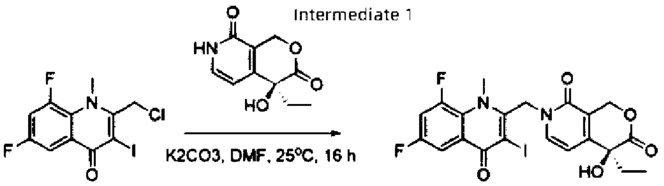


$K_2CO_3$ (1.12 g, 8.12 mmol, 3.0 eq.) was added to a DMF (2 mL) solution of the intermediate obtained from Step 6 (2 1.0 g, 2.71 mmol, 1.0 eq.) and Intermediate 1 (63.2 mg, 0.3 mmol, 0.11 eq.). The mixture was stirred at 25 °C for 16 hours, and LCMS monitoring confirmed the reaction was complete. The reaction was diluted with EA (150 mL), washed with water (30 mL × 3), dried over anhydrous sodium sulfate, and concentrated under reduced pressure. The crude product was separated and purified by flash silica gel column chromatography (eluent gradient: DCM:MeOH = 40:1 to 15:1, v/v) to obtain the title compound as a yellow solid (900 mg, yield: 61.2%). LCMS (ESI): $[M + H]^+ = 543.0$; $^1$H-NMR (400 MHz, $CDCl_3$) δ 7.90-7.83 (m, 1H), 7.75-7.73 (m, 1H), 7.57 (d, J = 7.2 Hz, 1H), 6.46 (d, J = 7.2 Hz, 1H), 6.38 (s, 1H), 5.69-5.57 (m, 2H), 5.33 (s, 2H), 3.84 (d, J = 7.6 Hz, 3H), 1.84-1.76 (m, 2H), 0.83 (t, J = 7.2 Hz, 3H).

***Step 8: Preparation of (S)-4-ethyl-8,10-difluoro-4-hydroxy-11-methyl-1,12-dihydro-14H-pyrano[3,4:6,7]indolizino[2,1 -b]quinoline-3,6,14(4H,11H)-trione:***

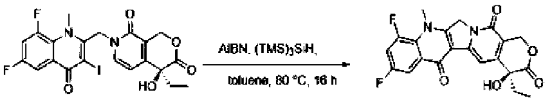


The intermediate obtained from Step 7 (300 mg, 0.55 mmol, 1.0 eq.), AIBN (45.2 mg, 0.28 mmol, 0.5 eq.) and $(TMS)_3SiH$ (412 mg, 1.66 mmol, 3.0 eq.) were suspended in anhydrous toluene (15 mL). The reaction solution was heated at 80 °C for 16 hours, and LCMS showed the reaction was complete. MeOH (3 mL) was added to quench the reaction, and toluene was removed by vacuum distillation. The residue was triturated with MeOH (10 mL), filtered to collect the solid, and purified by reverse-phase preparative HPLC (C18 column, mobile phase: 0.1% aqueous TFA-acetonitrile) to obtain the title compound as a yellow solid (4.05 mg, yield: 1.8%). LCMS (ESI): $[M + H]^+ = 415.0$; $^1$H-NMR (400 MHz, $(CD_3)_2SO$) $\delta$ 7.95-7.87 (m, 2H), 7.27 (s, 1H), 6.37 (s, 1H), 5.42 (s, 2H), 5.35 (d, J = 3.6 Hz, 2H), 4.07 (d, J = 7.6 Hz, 3H), 1.83-1.79 (m, 2H), 0.86 (t, J = 7.2 Hz, 3H).

Example 25: Preparation of (S,E)-4-ethyl-8-fluoro-4-hydroxy-10-(1-(methoxyimino)ethyl)-11-methyl-1,12-dihydro-14H-pyrano[3,4:6,7]indazolo [2,1-b]quinoline-3,6,14(4H,11H)-trione (Isomer 1):

Example 26: Preparation of (S,Z)-4-ethyl-8-fluoro-4-hydroxy-10-(1-(methoxyimino)ethyl)-11-methyl-1,12-dihydro-14H-pyrano[3,4:6,7]indolizino [2,1-b]quinoline-3,6,14(4H,11H)-trione (Isomer 2):

*Step 1: Preparation of methyl 8-(1-ethoxyvinyl)-6-fluoro-1-methyl-4-oxo-1,4-dihydroquinoline-2-carboxylate:*

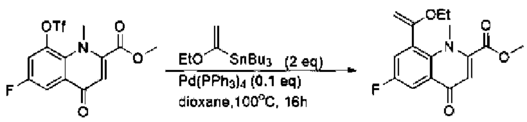


The intermediate from step 1 of Example 20 (3 g, 7.86 mmol, 1.0 eq.) was dissolved in a mixed solvent of 1,4-dioxane (60 mL) and water (6 mL). Then (1-ethoxyvinyl)boronic acid pinacol ester (1.86 g, 8.65 mmol, 1.1 eq.), $Na_2CO_3$ (2.48 g, 23.58 mmol, 3.0 eq.) and $Pd(PPh_3)_4$ (546 mg, 0.47 mmol, 0.06 eq.) were added sequentially. The reaction mixture was heated to 85 °C under nitrogen protection and stirred for 4 hours. LCMS monitoring showed the reaction was complete. The mixture was filtered, the filtrate was concentrated under reduced pressure, and the crude product was purified by silica gel column chromatography (eluent gradient: PE:EA = 2:1 to 1:2, v/v) to obtain the title compound as a pale yellow oil (1.6 g, yield: 64.5%). LCMS (ESI): $[M + H]^+ = 306.2$.

***Step 2: Preparation of methyl 8-acetyl-6-fluoro-1-methyl-4-oxo-1,4-dihydroquinoline-2-carboxylate:***

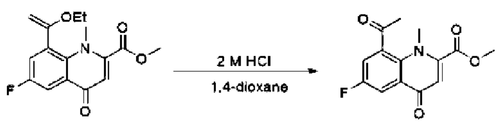


The intermediate from step 1 (1.5 g, 4.91 mmol, 1.0 eq.) was dissolved in THF (30 mL). Aqueous hydrochloric acid (2 mol/L, 10 mL) was added, and the mixture was stirred at 50 °C for 2 hours. LCMS monitoring showed the reaction was completed. The reaction solution was adjusted to pH 7 with saturated $NaHCO_3$ solution, extracted with EA (30 mL×3), and the combined organic phases were dried over anhydrous $Na_2SO_4$, concentrated under reduced pressure. The crude product was purified by silica gel column chromatography (eluent: DCM:MeOH = 20:1, v/v) to obtain the title compound as a yellow solid (1.1 g, yield: 81.2%). LCMS (ESI): $[M + H]^+ = 278.1$.

***Step 3: Preparation of 8-acetyl-6-fluoro-1-methyl-4-oxo-1,4-dihydroquinoline-2-carboxylic acid:***

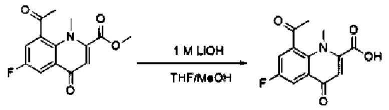


The intermediate from step 2 (1.40 g, 5.05 mmol, 1.0 eq.) was dissolved in a mixed solvent of MeOH (MeOH, 15 mL) and tetrahydrofuran (THF, 15 mL). Under ice bath conditions, 1 mol/L aqueous lithium hydroxide (LiOH) solution (15.2 mL, 15.2 mmol, 3.0 eq.) was added slowly. After the addition, the reaction mixture was stirred at room temperature for 2 hours, and the reaction progress was monitored by thin-layer chromatography (TLC). The pH of the reaction solution was adjusted to ~3 with 1 mol/L dilute hydrochloric acid (HCl). The mixture was concentrated, and the resulting solid was collected by filtration, washed with water, and dried to obtain the title compound as a pale yellow solid (1.5 g, crude product). LCMS (ESI): $[M + H]^+ = 264.3$.

***Step 4: Preparation of (Z)-6-fluoro-8-(1-(methoxyimino)ethyl)-1-methyl-4-oxo-1,4-dihydroquinoline-2-carboxylic acid:***

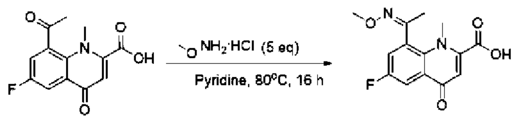


The intermediate from step 3 (1.5 g, 5.70 mmol, 1.0 eq.) was dissolved in pyridine (10 mL). Methoxyamine hydrochloride (714 mg, 8.55 mmol, 1.5 eq.) was added to the solution, and the mixture was heated to 80 °C and stirred for 16 hours. LCMS monitoring confirmed the reaction was completed. The reaction solution was concentrated to remove pyridine and obtain a crude product. The crude product was purified by silica gel column chromatography (eluent gradient: DCM:MeOH = 1:0 to 20:1, v/v) to afford the title compound as a yellow solid (950 mg, yield: 64.4% over two steps). LCMS (ESI): $[M + H]^+ = 293.1$.

***Step 5: Preparation of (Z)-methyl 6-fluoro-8-(1-(methoxyimino)ethyl)-1-methyl-4-oxo-1,4-dihydroquinoline-2-carboxylate:***

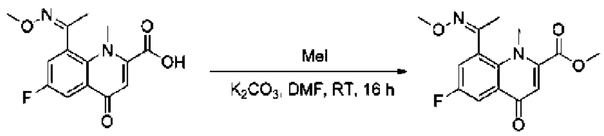


The intermediate obtained from Step 4 (910 mg, 3.12 mmol, 1.0 eq.) was dissolved in N,N-dimethylformamide (DMF, 10 mL). $K_2CO_3$ (1.29 g, 9.35 mmol, 3.0 eq.) and $CH_3I$ (886 mg, 6.24 mmol, 2.0 eq.) were added sequentially to the solution. The reaction mixture was stirred at room temperature for 16 hours. After the reaction, the mixture was filtered to remove insoluble solids, and the filtrate was poured into ice-cold saturated $NH_4Cl$ solution (30 mL). The resulting mixture was extracted with ethyl acetate (EA, 15 mL × 3). The combined organic layers were dried over anhydrous sodium sulfate ($Na_2SO_4$), filtered, and concentrated to obtain a crude product. The crude product was purified by silica gel column chromatography (eluent gradient: petroleum ether (PE):ethyl acetate (EtOAc) = 1:0 to 3:1, v/v) to afford the title compound as a light yellow solid (440 mg, yield: 46.1%). LCMS (ESI): $[M + H]^+ = 307.3$.

***Step 6: Preparation of (Z)-6-fluoro-2-(hydroxymethyl)-8-(1-(methoxyimino)ethyl)-1-methylquinolin-4(1H)-one:***

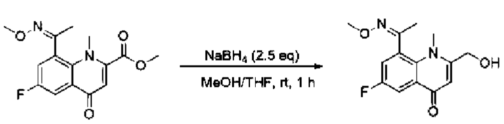

The intermediate obtained from Step 5 (440 mg, 1.44 mmol, 1.0 eq.) was dissolved in a mixed solvent of MeOH (14 mL) and THF (, 14 mL). The solution was cooled to 0 °C in an ice bath, and $NaBH_4$ (136 mg, 3.60 mmol, 2.5 eq.) was added slowly with stirring. After the addition was complete, the reaction mixture was allowed to warm to room temperature naturally and stirred continuously for 2 hours. Thin-layer chromatography (TLC) monitoring confirmed the reaction was complete. The reaction was quenched by adding saturated sodium chloride solution (0.5 mL). The mixture was concentrated to obtain a crude product, which was purified by silica gel column chromatography (eluent gradient: DCM:MeOH = 1:0 to 10:1, v/v) to afford the title compound as an off-white solid (215 mg, yield: 53.7%). □ LCMS (ESI): $[M + H]^+$ = 279.3 $^1H$ NMR (400 MHz, DMSO-$d_6$) $\delta$ 7.86-7.79 (m, 1H), 7.65-7.50 (m, 1H), 6.28 (d, J = 6.8 Hz, 1H), 5.80-5.68 (m, 1H), 4.56 (d, J = 5.6 Hz, 2H), 3.79 (d, J = 83.2 Hz, 3H), 3.49 (d, J = 10.4 Hz, 3H), 2.31 (d, J = 28.6 Hz, 3H, $-CH_3$).

***Step 7: Preparation of (Z)-6-fluoro-2-(hydroxymethyl)-3-iodo-8-(1-(methoxyimino)ethyl)-1-methylquinolin-4(1H)-one:***

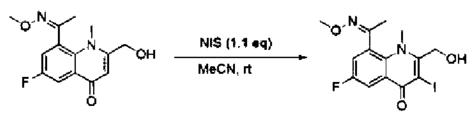


The intermediate obtained from Step 6 (210 mg, 0.750 mmol, 1.0 eq.) was dissolved in MeCN (8 mL). Then NIS (204 mg, 0.91 mmol, 1.2 eq.) was slowly added, and the reaction was continued to stir at room temperature for 16 hours. LCMS detection confirmed the reaction was complete. The reaction solution was concentrated to obtain a crude product, which was separated and purified by silica gel column chromatography (eluent gradient: DCM:MeOH = 1:0 to 10:1) to obtain the title compound as a white solid (260 mg, yield: 85.2%). LCMS (ESI): $[M + H]^+$ = 404.9.

***Step 8: Preparation of (Z)-2-(chloromethyl)-6-fluoro-3-iodo-8-(1-(methoxyimino)ethyl)-1-methylquinolin-4(1H)-one:***

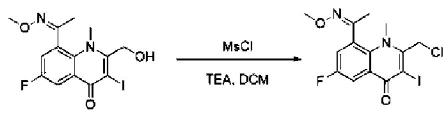


The intermediate obtained from Step 7 (220 mg, 0.54 mmol, 1.0 eq.) was dissolved in anhydrous DCM (3 mL). Under ice bath conditions, TEA (165 mg, 1.63 mmol, 3.0 eq.) and MsCl (124.7 mg, 1.09 mmol, 2.0 eq.) were added, and stirring was continued for 1 hour. TLC detected that the starting materials were completely consumed. The reaction solution was poured into ice water (20 mL), extracted with DCM (10 mL×2), and the combined organic phases were dried over anhydrous sodium sulfate ($Na_2SO_4$), filtered. The filtrate was concentrated under reduced pressure to obtain the title compound as a pale brown solid (252 mg, crude product). LCMS (ESI): $[M + H]^+$ = 422.9.

***Step 9: Preparation of (S,Z)-4-ethyl-7-((6-fluoro-3-iodo-8-(1-(methoxyimino)ethyl)-1-methyl-4-oxo-1,4-dihydroquinolin-2-yl)methyl)-4-hydroxy-1,7-dihydro-3H-pyrano[3,4-c]pyridine-3,8(4H)-dione:***

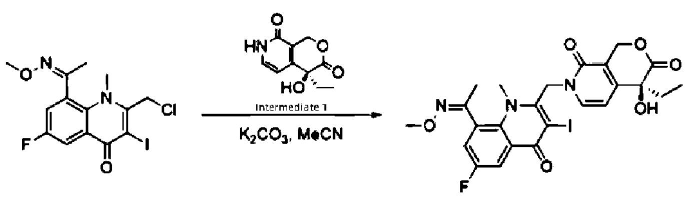


The intermediate obtained from Step 8 (252 mg, 0.6 mmol, 1.0 eq.) and Intermediate 1 (99.8 mg, 0.48 mmol, 0.8 eq.) were dissolved in MeCN (10 mL). $K_2CO_3$ (247 mg, 1.79 mmol, 3.0 eq.) was added, and the reaction was heated to 35 °C and stirred overnight. LCMS detection confirmed the reaction was complete. The mixture was filtered, and the filtrate

was concentrated to obtain a crude product. The crude product was separated and purified by silica gel column chromatography (eluent gradient: DCM:MeOH = 1:0 to 50:1) to obtain the title compound as a white solid (205 mg, two-step yield: 42.4%).

LCMS(ESI)[M+1]$^+$=596.2; $^1$H NMR (400 MHz, DMSO-$d_6$) δ 7.89-7.83 (m, 1H), 7.70-7.51 (m, 2H), 6.53-6.46 (m, 1H), 6.37-6.31 (m, 1H), 5.86-5.59 (m, 2H), 5.32-5.22 (m, 2H), 3.72-3.42 (m, 6H), 3.40 (s, 1.5H), 2.72 (s, 1.5H), 1.82-1.70 (m, 2H), 0.82 (t, J = 7.4 Hz, 3H).

***Step 10: Preparation of (S,E)-4-ethyl-8-fluoro-4-hydroxy-10-(1-(methoxyimino)ethyl)-11-methyl-1,12-dihydro-14H-pyrano[3',4':6,7]indolizino[2,1-b]quinoline-3,6,14(4H,11H)-trione (isomer 1 or isomer 2) and (S,Z)-4-ethyl-8-fluoro-4-hydroxy-10-(1-(methoxyimino)ethyl)-11-methyl-1,12-dihydro-14H-pyrano[3',4':6,7]indolizino [2,1-b]quinoline-3,6,14(4H,11H)-trione (isomer 2 or isomer 1):***

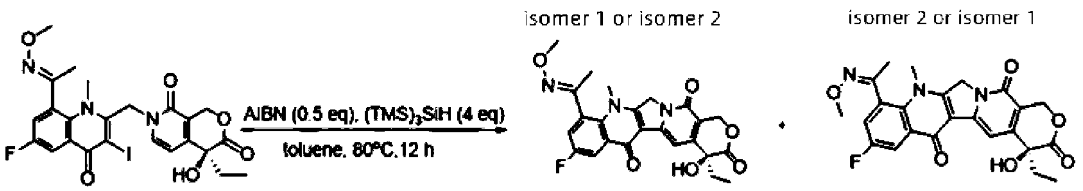


The intermediate obtained from Step 9 (170 mg, 0.29 mmol, 1.0 eq.) was dissolved in toluene (30 mL). AIBN (23.44 mg, 0.14 mmol, 0.5 eq.) and (TMS)$_3$SiH (284.01 mg, 1.14 mmol, 4.0 eq.) were added sequentially. The reaction system was protected with nitrogen, evacuated with an oil pump, then heated to 80 °C and stirred for 12 hours. LCMS detection confirmed the reaction was complete. Toluene was removed by rotary evaporation, and the residue was dissolved in DMF. Purification by preparative HPLC (mobile phase: acetonitrile-0.1% aqueous TFA) afforded two isomers as white solids, with no specific configuration distinguished.

**Product (isomer 1 or isomer 2):** Yield: 1.48% (2.09 mg). LCMS (ESI): m/z = 468.2 [M - H]$^-$; Retention time ($t_r$) = 0.960 min. $^1$H-NMR (400 MHz, $(CD_3)_2SO$) δ 8.08-8.04 (m, 1H), 7.68 (dd, J = 8.4, 3.2 Hz, 1H), 7.27 (s, 1H), 6.38 (s, 1H), 5.44 (dd, J = 7.2, 3.6 Hz, 2H), 5.35 (d, J = 4.9 Hz, 2H), 3.77-3.72 (m, 6H), 2.40 (s, 3H), 1.87-1.78 (m, 2H), 0.87 (t, J = 5.6 Hz, 3H). **Product (isomer 2 or isomer 1):** Yield: 5.18% (7 mg). LCMS (ESI): m/z = 468.2 [M - H]$^-$; Retention time ($t_r$) = 0.991 min. $^1$H-NMR (400 MHz, $(CD_3)_2SO$) δ 8.08 (dd, J = 8.4, 3.2 Hz, 1H), 7.78 (dd, J = 8.4, 3.2 Hz, 1H), 7.27 (s, 1H), 6.38 (s, 1H), 5.44 (s, 2H), 5.35 (d, J = 4.4 Hz, 2H), 3.94 (s, 3H), 3.76 (s, 3H), 2.35 (s, 3H), 1.87-1.78 (m, 2H), 0.87 (t, J = 7.2 Hz, 3H).

**Example 27: Preparation of (4S)-4-ethyl-8-fluoro-4-hydroxy-11-(morpholin-2-ylmethyl)-1,12-dihydro-14H-pyrano[3',4':6,7]indazolo[2,1-b]quinoline-3,6,14 (4H,11H)-trione:**

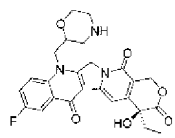

Commercially available 2-amino-5-fluorophenylacetone (1.0 eq.) and tert-butyl 2-formylmorpholine-4-carboxylate (1.1 eq.) were used as the starting materials. The synthesis was carried out following the method described in Example 2, including condensation, cyclization, deprotection, and coupling reactions with Intermediate 1, followed by cyclization to obtain the title compound. It is a pale yellow solid. LCMS (ESI): m/z = 482.2 [M + H]$^+$.

**Example 28: Preparation of (S)-4-ethyl-4-hydroxy-11-(morpholine-4-carbonyl)-1,12-dihydro-14H-pyrano [3',4':6,7]indazolo[2,1-b]quinoline-3,6,14(4H,11H)-trione:**

***Step 1: Preparation of 2-methyl-1-(morpholine-4-carbonyl)-2,3-dihydroquinolin-4(1H)-one:***

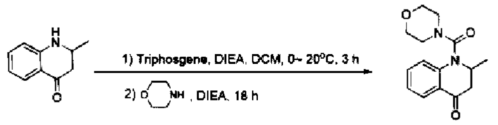


2-Methyl-2,3-dihydroquinolin-4(1H)-one (1.2 g, 7.44 mmol, 1.0 eq.) was suspended in DCM (20 mL). At 0 °C, DIEA (2.12 g, 16.38 mmol, 2.2 eq.) and triphosgene (2.43 g, 8.19 mmol, 1.1 eq.) were added sequentially, stirred for 3 h (TLC, complete). Warmed to 20 °C, DIEA (2.12 g, 2.2 eq.) and morpholine (1.3 g, 14.89 mmol, 2.0 eq.) were added, stirred for 18 h (LCMS, complete). Quenched with ice water (30 mL), extracted with DCM (30 mL × 3). Organic layer washed with saturated brine (30 mL × 3), dried over $Na_2SO_4$, concentrated to a yellow oil. Crude purified by silica gel column chromatography (100-200 mesh, eluent gradient: DCM:MeOH = 30:1 to 10:1) to afford the title compound as a pale yellow solid (2 g, 98% yield).LCMS (ESI): m/z = 275.1 $[M + H]^+$; $^1$H-NMR (400 MHz, $CDCl_3$) δ 7.99-7.96 (m, 1H), 7.49-7.45 (m, 1H), 7.06-7.01 (m, 2H), 4.54-4.44 (m, 1H), 3.75-3.61 (m, 4H), 3.60-3.51 (m, 2H), 3.40-3.30 (m, 2H), 2.95-2.93 (m, 1H), 2.65-2.59 (m, 1H), 1.34 (d, J = 6.8 Hz, 3H).

***Step 2: Preparation of 3-bromo-2-methyl-1-(morpholine-4-carbonyl)quinolin-4(1H)-one:***

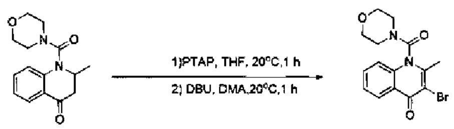


Under nitrogen protection at 20 °C, phenyltrimethylammonium tribromide (2.46 g, 6.55 mmol, 1.5 eq.) was added to a tetrahydrofuran (THF, 15 mL) solution of the intermediate obtained from step 1 (1.2 g, 4.37 mmol, 1.0 eq.). The reaction was stirred at 20 °C for 1 hour, then filtered, and the solvent was removed by rotary evaporation. The residue was dissolved in DMA (15 mL), and DBU (1.99 g, 13.11 mmol, 3.0 eq.) was added, followed by stirring at room temperature for 1 hour. LCMS monitoring confirmed the reaction was complete. Water (30 mL) was added to the reaction mixture, and the mixture was extracted with ethyl acetate (EA, 30 mL × 3). The organic layer was washed with saturated brine (30 mL × 3), dried over anhydrous sodium sulfate ($Na_2SO_4$), and concentrated under reduced pressure to obtain a yellow oil. The crude product was separated and purified by silica gel column chromatography (100-200 mesh silica gel, eluent gradient: DCM:MeOH = 30:1 to 10:1) to obtain the title compound as a yellow solid (1.12 g, yield: 73.0%). LCMS(ESI)$[M+H]^+$=352.2; $^1$H NMR (400 MHz, $CDCl_3$) δ 8.46-8.43 (m, 1H), 7.68-7.64 (m, 1H), 7.44-7.40 (m, 1H), 7.23 (d, *J* = 8.8 Hz, 1H), 3.98-3.92 (m, 1H), 3.90-3.81 (m, 3H), 3.61-3.57 (m, 1H), 3.54 -3.46 (m, 1H), 3.14 -3.08 (m,1H), 3.03-2.98 (m, 1H), 2.66 (s, 3H).

***Step 3: Preparation of 3-bromo-2-(bromomethyl)-1-(morpholine-4-carbonyl)quinolin-4(1H)-one:***

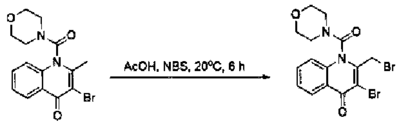


Under nitrogen protection at 0 °C, glacial acetic acid (AcOH, 10 mL) was added to the intermediate obtained from step 2 (600 mg, 1.71 mmol, 1.0 eq.). Then NBS (304.07 mg, 1.71 mmol, 1.0 eq.) was added, and the reaction was stirred at 20 °C for 6 hours. The reaction solution was diluted with EA (20 mL), washed with water (20 mL × 3), dried over anhydrous $Na_2SO_4$, and concentrated under reduced pressure. The crude product was separated and purified by silica gel column chromatography (100-200 mesh silica gel, eluent gradient: PE:EA = 40:1 to 15:1) to obtain the title compound as a white solid (312 mg, yield: 42.24%). LCMS (ESI): m/z = 430.9 $[M+H]^+$; $^1$H NMR (400 MHz, $CDCl_3$) δ 8.47-8.43 (m, 1H), 7.75-7.69 (m, 1H), 7.49-7.47 (m, 1H), 7.24 (d, J = 8.8 Hz, 1H), 4.95 (d, J = 11.6 Hz, 1H), 4.79 (d, J = 11.6 Hz, 1H), 4.01-3.87 (m, 4H), 3.76-3.71 (m, 1H), 3.55-3.50 (m, 1H), 3.27-3.21 (m, 1H), 3.02-2.96 (m, 1H).

***Step 4: Preparation of (S)-7-((3-bromo-1-(morpholine-4-carbonyl)-4-oxo-1,4-dihydroquinolin-2-yl)methyl)-4-ethyl-4-hydroxy-1, 7-dihydro-3H-pyrano[3,4-c]pyridine-3,8(4H)-dione:***

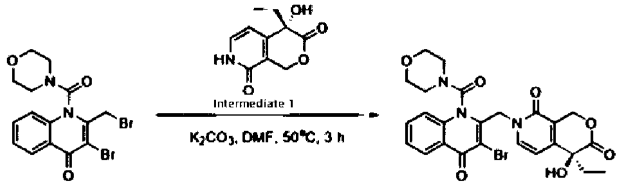


Under nitrogen protection and ice water bath conditions, potassium carbonate ($K_2CO_3$, 272.51 mg, 1.97 mmol, 4.0 eq.) was added to an DMF (5 mL) solution of the intermediate obtained from Step 3 (212 mg, 0.49 mmol, 1.0 eq.) and Intermediate 1 (103.12 mg, 0.49 mmol, 1.0 eq.). The reaction solution was heated to 50 °C and stirred for 3 hours. After the reaction was complete, the reaction mixture was cooled to 0 °C, quenched with water (10 mL), and extracted with EA (15 mL × 3). The combined organic layers were washed with saturated brine (10 mL × 3), dried over anhydrous $Na_2SO_4$, filtered, and concentrated under reduced pressure. The crude product was separated and purified by silica gel column chromatography (100-200 mesh silica gel, eluent gradient: DCM:MeOH = 40:1 to 15:1) to obtain the title compound as a pale yellow solid (140 mg, yield: 51.17%). LCMS (ESI): $[M + H]^+$ = 568.2. $^1$H NMR (400 MHz, $(CD_3)_2SO$) δ 8.25 (d, *J* = 8.0 Hz, 1H), 7.87-7.80 (m, 1H), 7.62-7.47 (m, 3H), 6.45-6.42 (m, 1H), 6.35 (d, *J* = 4.0 Hz, 1H), 5.76-5.54 (m, 1H), 5.30-5.15 (m, 3H), 3.90-3.79 (m, 1H), 3.77-3.63 (m, 3H), 3.44-3.41 (m, 2H), 3.28-3.19 (m, 1H), 3.13-3.04 (m, 1H), 1.87-1.67 (m, 2H), 0.80 (td, *J* = 7.2, 2.4 Hz, 3H).

***Step 5: Preparation of (S)-4-ethyl-4-hydroxy-11-(morpholine-4-carbonyl)-1,12-dihydro-14H-pyrano[3',4':6,7]indolizino[2,1-b]quinoline-3,6,14(4H,11H)-trione:***

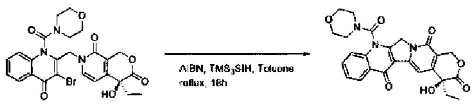


The intermediate obtained from step 4 (140 mg, 0.25 mmol, 1.0 eq.), azobisisobutyronitrile (AIBN, 8.23 mg, 0.05 mmol, 0.2 eq.) and $(TMS)_3SiH$ (249.38 mg, 1 mmol, 4.0 eq.) were suspended in anhydrous toluene (5 mL). The reaction solution was heated to 130 °C and refluxed for 18 hours, and LCMS monitoring confirmed the reaction was complete. MeOH (3 mL) was added to quench the reaction, and toluene was removed by vacuum distillation. The residue was triturated with MeOH (10 mL) and filtered to obtain a solid (180 mg). Purification by reverse-phase preparative HPLC (C18 column, mobile phase: 0.1% aqueous TFA-acetonitrile) afforded the title compound as a yellowish solid (6 mg, yield not calculated). LCMS (ESI): m/z = 478.2 $[M - H]^-$; $^1$H-NMR (400 MHz, $(CD_3)_2SO$) δ 8.46-8.29 (m, 1H), 7.93-7.83 (m, 1H), 7.70 (m, 1H), 7.68-7.58 (m, 1H), 7.34-7.30 (m, 1H), 6.45-6.28 (m, 1H), 5.48-4.96 (m, 3H), 4.05-3.73 (m, 4H), 3.70-3.42 (m, 3H), 3.10 (s, 1H), 1.81 (dd, J = 19.2, 12.0 Hz, 2H), 0.98-0.76 (m, 3H).

**Example 29: Preparation of (S)-4-ethyl-8-fluoro-4-hydroxy-11-(oxetidin-3-yl)-1,12-dihydro-14H-pyrano[3',4':6,7] indolizino[2,1-b]quinoline-3,6,14(4H,11H)-trione:**

***Step 1: Preparation of 1-(5-fluoro-2-(oxetidin-3-ylamino)phenyl)ethan-1-one:***

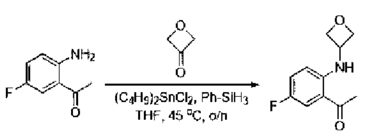


1-(2-Amino-5-fluorophenyl)ethan-1-one (5.0 g, 32.65 mmol, 1.0 eq.) was dissolved in anhydrous tetrahydrofuran (THF, 50 mL). 3-Oxetanone (2.35 g, 32.65 mmol, 1.0 eq.) and dibutyltin dichloride (1.98 g, 6.53 mmol, 0.2 eq.) were added,

and the mixture was stirred at room temperature for 1 hour. Phenylsilane (3.53 g, 32.65 mmol, 1.0 eq.) was added to the reaction system, and the reaction was conducted at 45 °C for 18 hours. LCMS monitoring confirmed the reaction was complete. The reaction solution was concentrated, and purified by flash silica gel column chromatography (200-300 mesh silica gel, eluent gradient: PE:EA = 10:1 to 2:1) to obtain the title compound as a yellow solid (5.2 g, yield: 76%). LCMS (ESI): $[M + H]^+$ = 210.1.

***Step 2: Preparation of ethyl 6-fluoro-1-(oxetidin-3-yl)-4-oxo-1,4-dihydroquinoline-2-carboxylate:***

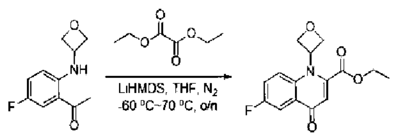


The intermediate obtained from step 1 (5.1 g, 24.38 mmol, 1.0 eq.) was dissolved in anhydrous tetrahydrofuran (THF, 20 mL). A 1 mol/L lithium hexamethyldisilazide (LiHMDS) in THF solution (61 mL) was added at -60 °C, and the reaction was stirred at -60 °C for 30 minutes. Diethyl oxalate (8.91 g, 60.94 mmol, 2.5 eq.) was added, and the mixture was stirred overnight at 70 °C. LCMS monitoring confirmed the reaction was complete. The reaction mixture was concentrated by rotary evaporation, washed with water, extracted with ethyl acetate (EA), and concentrated again to obtain the title compound as a yellow solid (6 g, yield: 84%). LCMS (ESI): $[M + H]^+$ = 292.1.

***Step 3: Preparation of 6-fluoro-2-(hydroxymethyl)-1-(oxetidin-3-yl)quinolin-4(1H)-one:***

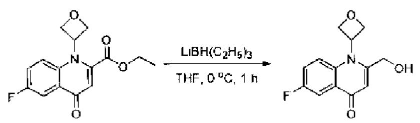


The intermediate obtained from step 2 (5.5 g, 18.88 mmol, 1.0 eq.) was dissolved in tetrahydrofuran (THF, 30 mL). Lithium triethylborohydride ($LiEt_3BH$, 37.76 mL, 37.76 mmol, 2.1 eq.) was added at 0 °C, and the reaction mixture was stirred at 0 °C for 1 hour. LCMS detection confirmed the reaction was complete. The reaction mixture was concentrated, washed with a small amount of water, and filtered to obtain the title compound as a white solid (4.7 g, yield: 85%). LCMS (ESI): $[M + H]^+$ = 250.1.

***Step 4: Preparation of 6-fluoro-2-(hydroxymethyl)-3-iodo-1-(oxetidin-3-yl)quinolin-4(1H)-one:***

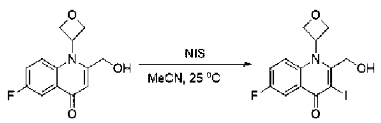


The intermediate obtained from step 3 (4.7 g, 18.86 mmol, 1.0 eq.) was dissolved in acetonitrile (MeCN, 40 mL). N-Iodosuccinimide (NIS, 4.67 g, 20.74 mmol, 1.1 eq.) was added, and the reaction was stirred at room temperature for 18 hours. LCMS detection confirmed the reaction was complete. The reaction mixture was concentrated by rotary evaporation, washed with water, and the solid was collected by filtration. The crude product was separated and purified by flash silica gel column chromatography (200-300 mesh silica gel, eluent gradient: DCM:MeOH = 30:1 to 10:1) to obtain the title compound as a brown solid (1.3 g, yield: 19%). LCMS (ESI): $[M + H]^+$ = 376.0.

**Step 5: Preparation of 2-(chloromethyl)-3-iodo-6-fluoro-1-(oxetidin-3-yl)quinolin-4(1H)-one**

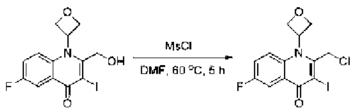


The intermediate obtained from step 4 (100 mg, 0.27 mmol, 1.0 eq.) was dissolved in anhydrous N,N-dimethylformamide (DMF, 2 mL). Methanesulfonyl chloride (MsCl, 152 mg, 1.33 mmol, 5.0 eq.) was added, and the reaction mixture was heated to 60 °C and stirred for 5 hours. LCMS detection confirmed the reaction was complete. The crude product was separated and purified by flash silica gel column chromatography (200-300 mesh silica gel, eluent gradient: DCM:MeOH = 50:1 to 20:1) to obtain the title compound as a white solid (31 mg, yield: 30%). LCMS (ESI): $[M + H]^+$ = 394.0.

***Step 6: Preparation of (S)-4-ethyl-7-((6-fluoro-3-iodo-1-(oxetidin-3-yl)-4-oxo-1,4-dihydroquinolin-2-yl)methyl)-4-hydroxy-1,7-dihydro-3H-pyrano[3,4-c]pyridine-3,8(4H)-dione:***

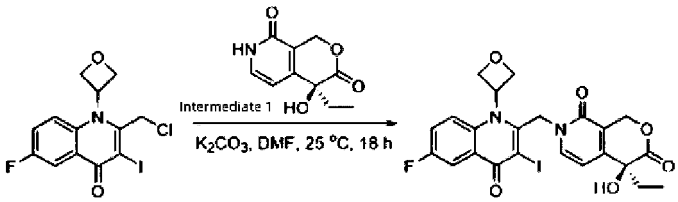


The intermediate obtained from step 5 (30 mg, 0.076 mmol, 1.0 eq.) was dissolved in anhydrous N,N-dimethylformamide (DMF, 5 mL). Potassium carbonate ($K_2CO_3$, 22.12 mg, 0.16 mmol, 2.1 eq.) and Intermediate 1 (15.9 mg, 0.076 mmol, 1.0 eq.) were added, and the reaction was stirred at room temperature for 18 hours. LCMS monitoring confirmed the reaction was complete. Potassium carbonate was removed by filtration, DMF was evaporated, and separated and purified by silica gel column chromatography (100-200 mesh silica gel, eluent gradient: DCM:MeOH = 100:1 to 10:1). The collected fractions were concentrated by rotary evaporation to obtain the title compound as a yellowish solid (19 mg, yield: 45%). LCMS (ESI): m/z = 567.10$[M+H]^+$.

***Step 7: Preparation of (S)-4-ethyl-8-fluoro-4-hydroxy-11-(oxetidin-3-yl)-1,12-dihydro-14H-pyrano[3',4':6,7]indolizino[2,1-b]quinoline-3,6,14(4H,11H)-trione:***

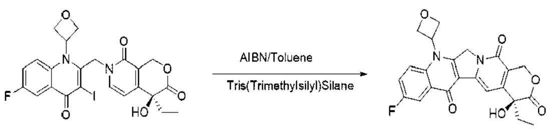


The intermediate obtained from step 6 (19 mg, 0.034 mmol, 1.0 eq.) and azobisisobutyronitrile (AIBN, 5.5 mg, 0.034 mmol, 1.0 eq.) were added into a dry three-necked flask. The flask was evacuated with an oil pump, protected with argon, and anhydrous toluene (20 mL) was added. Then tris(trimethylsilyl)silane ($(TMS)_3SiH$, 33 mg, 0.136 mmol, 4.0 eq.) was added to the reaction mixture. After the addition, the reaction was stirred at 90 °C for 18 hours. The reaction mixture was concentrated under reduced pressure to obtain a crude product, which was first purified by silica gel column chromatography (eluent gradient: DCM:MeOH = 1:0 to 15:1) to collect crude fractions. Further purification by reverse-phase preparative HPLC (C18 column, mobile phase: 0.1% aqueous TFA-acetonitrile) afforded the title compound as a pale yellow solid (1.5 mg, yield: 10%). LCMS (ESI): $[M + H]^+$ = 439.1.

**Example 30: Synthesis of (S)-4-ethyl-8-fluoro-4-hydroxy-11-(tetrahydro-2H-pyran-4-yl)-1,12-dihydro-14H-pyrano[3',4':6,7]indolizino[2,1-b]quinoline-3,6,14(4H,11H)-trione:**

***Step 1: Preparation of 1-(5-fluoro-2-((tetrahydro-2H-pyran-4-yl)amino)phenyl)ethan-1-one:***

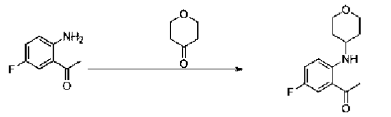

2-Amino-5-fluoroacetophenone (5.0 g, 32.7 mmol, 1.0 eq.) was dissolved in anhydrous tetrahydrofuran (THF, 50 mL). Tetrahydro-4H-pyran-4-one (3.2 g, 32.7 mmol, 1.0 eq.) and dibutyltin dichloride (2.0 g, 6.5 mmol, 0.2 eq.) were added, and the mixture was stirred at room temperature for 1 hour. Phenylsilane (3.5 g, 32.7 mmol, 1.0 eq.) was added to the reaction system, and the reaction was conducted at 70 °C for 16 hours. LCMS monitoring confirmed the reaction was complete. The reaction solution was concentrated, and purified by silica gel column chromatography (200-300 mesh silica gel, eluent gradient: PE:EA = 10:1 to 2:1) to obtain the title compound as a yellow solid (6.27 g, yield: 81%). LCMS (ESI): $[M + H]^+$ = 238.1.

***Step 2: Preparation of ethyl 6-fluoro-4-oxo-1-(tetrahydro-2H-pyran-4-yl)-1,4-dihydroquinoline-2-carboxylate:***

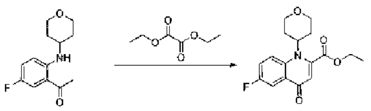

The intermediate obtained from step 1 (5 g, 21.1 mmol, 1.0 eq.) was dissolved in anhydrous tetrahydrofuran (THF, 50 mL). A 1 mol/L lithium hexamethyldisilazide (LiHMDS) in THF solution (40 mL) was added at -70 °C, and the reaction mixture was stirred at -70 °C for 30 minutes. Diethyl oxalate (7.7 g, 52.7 mmol, 2.5 eq.) was added to the reaction system, and the mixture was stirred overnight at room temperature. LCMS monitoring confirmed the reaction was complete. The reaction mixture was concentrated, and separated and purified by silica gel column chromatography (200-300 mesh silica gel, eluent gradient: DCM:MeOH = 40:1 to 20:1) to obtain the title compound as a red solid (4.3 g, yield: 64%). LCMS (ESI): $[M + H]^+$ = 320.1.

***Step 3: Preparation of 6-fluoro-2-(hydroxymethyl)-1-(tetrahydro-2H-pyran-4-yl)quinolin-4(1H)-one:***

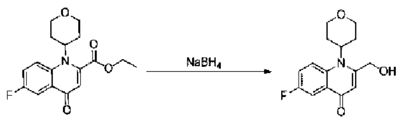


The intermediate obtained from step 2 (6 g, 18.8 mmol, 1.0 eq.) was dissolved in ethanol (EtOH, 110 mL). Sodium borohydride ($NaBH_4$, 1.4 g, 37.6 mmol, 2.0 eq.) was added, and the reaction mixture was stirred at room temperature for 2 minutes. The reaction was quenched with water, extracted with DCM, and the organic phase was separated. The crude product was purified by silica gel column chromatography (200-300 mesh silica gel, eluent gradient: DCM:MeOH = 30:1 to 10:1) to obtain the title compound as a white solid (2.5 g, yield: 48%). LCMS (ESI): $[M + H]^+$ = 278.1.

***Step 4: Preparation of 6-fluoro-2-(hydroxymethyl)-3-iodo-1-(tetrahydro-2H-pyran-4-yl)quinolin-4(1H)-one:***

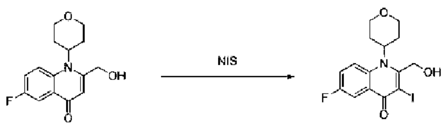


The intermediate obtained from step 3 (2.5 g, 9.0 mmol, 1.0 eq.) was dissolved in acetic acid (AcOH, 30 mL). NIS (4.06 g, 18.0 mmol, 2.0 eq.) was added, and the reaction mixture was stirred at room temperature for 1 hour. LCMS detection confirmed the reaction was complete. The reaction was quenched with 2 mL of saturated aqueous sodium bicarbonate solution, and the solid was collected by filtration. The solid was dissolved in 10 mL of acetonitrile, concentrated

to dryness, and the title compound was obtained as a white solid (500 mg, yield: 13.75%). LCMS (ESI): [M + H]$^+$ = 404.1.

***Step 5: Preparation of 2-(chloromethyl)-6-fluoro-3-iodo-1-(tetrahydro-2H-pyran-4-yl)quinolin-4(1H)-one:***

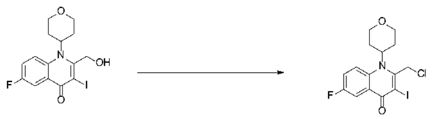

The intermediate obtained from step 4 (300 mg, 0.74 mmol, 1.0 eq.) was dissolved in anhydrous tetrahydrofuran (THF, 3 mL). Thionyl chloride ($SOCl_2$, 878 mg, 7.4 mmol, 10.0 eq.) was added, and the reaction mixture was stirred at room temperature. LCMS detection confirmed the reaction was complete. Thionyl chloride was removed by concentration under reduced pressure to obtain a gray solid crude product, which was washed with 2 mL of DCM (DCM) to obtain the title compound as a white solid (278 mg, yield: 89%). LCMS (ESI): [M + H]$^+$ = 422.1.

***Step 6: Preparation of (S)-4-ethyl-7-((6-fluoro-3-iodo-4-oxo-1-(tetrahydro-2H-pyran-4-yl)-1,4-dihydroquinolin-2-yl)methyl)-4-hydroxy-1,7-dihydro-3H-pyrano [3,4-c]pyridine-3,8(4H)-dione:***

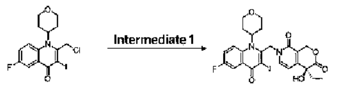


The intermediate obtained from step 5 (506 mg, 1.2 mmol, 1.0 eq.) was dissolved in anhydrous N,N-dimethylformamide (DMF, 15 mL). Potassium carbonate ($K_2CO_3$, 331 mg, 2.4 mmol, 2.0 eq.) and Intermediate 1 (257 mg, 1.23 mmol, 1.1 eq.) were added, and the reaction was stirred at room temperature for 4 hours. Ten milliliters of water was added to the reaction mixture, and the precipitated gray solid was collected by filtration. The crude product was purified by preparative thin-layer chromatography (eluent: DCM:MeOH = 20:1) to obtain the title compound as a white solid (245 mg, yield: 34.2%). LCMS (ESI): [M + H]$^+$ = 595.1.

***Step 7: Preparation of (S)-4-ethyl-8-fluoro-4-hydroxy-11-(tetrahydro-2H-pyran-4-yl)-1,12-dihydro-14H-pyrano [3',4':6,7]indolizino[2,1-b]quinoline-3,6,14(4H, 11H)-trione:***

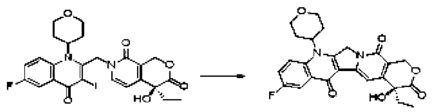

The intermediate obtained from step 6 (101 mg, 0.17 mmol, 1.0 eq.), AIBN (14.15 mg, 0.09 mmol, 0.5 eq.) and anhydrous toluene (5 mL) were added to a 40 mL vial. Under stirring, nitrogen gas was bubbled into the solution via a syringe needle inserted to the bottom of the solution for 5 minutes, and the vial was quickly capped. The system was purged with nitrogen three times. A solution of $(TMS)_3SiH$ (171.39 mg, 0.69 mmol, 4.0 eq.) in toluene (3 mL) was prepared and injected into the reaction vial with a syringe. The reaction mixture was stirred at 80 °C overnight, then purified by preparative HPLC. The collected fractions were transferred to a 250 mL flask and lyophilized to obtain the title compound as a white solid (13 mg, yield: 16.4%). LCMS (ESI): m/z = 466.15[M+H]$^+$.

**Example 31: Preparation of (4S)-4-ethyl-8-fluoro-4-hydroxy-11-(tetrahydrofuran-3-yl)-1,12-dihydro-14H-pyrano [3',4':6,7]indolizino[2,1-b] quinoline-3,6,14(4H,11H)-trione:**

***Step 1: Preparation of 1-(5-fluoro-2-((tetrahydrofuran-3-yl)amino)phenyl)ethan-1-one:***

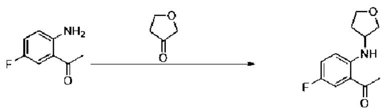

2-Amino-5-fluoroacetophenone (5.0 g, 32.7 mmol, 1.0 eq.) was dissolved in anhydrous tetrahydrofuran (THF, 50 mL). Dihydrofuran-3(2H)-one (2.8 g, 32.7 mmol, 1.0 eq.) and dibutyltin dichloride (2.0 g, 6.5 mmol, 0.2 eq.) were added, and the mixture was stirred at room temperature for 1 hour. Phenylsilane (3.5 g, 32.7 mmol, 1.0 eq.) was added to the reaction system, and the reaction was conducted at 70 °C for 16 hours. LCMS monitoring confirmed the reaction was complete. The solution was concentrated, and the crude product was separated and purified by silica gel column chromatography (200-300 mesh silica gel, eluent gradient: PE:EA = 10:1 to 2:1) to obtain the title compound as a yellow solid (7.1 g, yield: 97%). LCMS (ESI): $[M + H]^+$ = 224.1.

***Step 2: Preparation of ethyl 6-fluoro-4-oxo-1-(tetrahydrofuran-3-yl)-1,4-dihydroquinoline-2-carboxylate:***

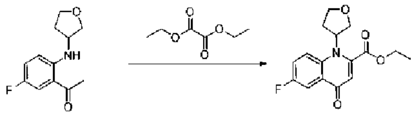

The intermediate obtained from step 1 (5 g, 22.4 mmol, 1.0 eq.) was dissolved in anhydrous tetrahydrofuran (THF, 50 mL). A 1 mol/L lithium hexamethyldisilazide (LiHMDS) in THF solution (40 mL) was added at -70 °C, and the reaction mixture was stirred at -70 °C for 30 minutes. Diethyl oxalate (8.2 g, 56 mmol, 2.5 eq.) was added to the reaction system, and the mixture was stirred overnight at room temperature. LCMS monitoring confirmed the reaction was complete. The reaction mixture was concentrated, and purified by silica gel column chromatography (200-300 mesh silica gel, eluent gradient: DCM:MeOH = 40:1 to 20:1) to obtain the title compound as a yellow solid (5.4 g, yield: 80%). LCMS (ESI): $[M + H]^+$ = 306.1.

***Step 3: Preparation of 6-fluoro-2-(hydroxymethyl)-1-(tetrahydrofuran-3-yl)quinolin-4(1H)-one:***

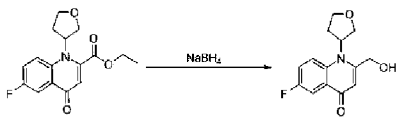


The intermediate obtained from step 2 (6.1 g, 19.9 mmol, 1.0 eq.) was dissolved in ethanol (EtOH, 110 mL). Sodium borohydride ($NaBH_4$, 1.5 g, 39.9 mmol, 2.0 eq.) was added, and the reaction mixture was stirred at room temperature for 20 minutes. The reaction was quenched with water, extracted with DCM (DCM), and the organic phase was separated. The crude product was purified by silica gel column chromatography (200-300 mesh silica gel, eluent gradient: DCM:MeOH = 30:1 to 10:1) to obtain the title compound as a white solid (3.8 g, yield: 72%). LCMS (ESI): $[M + H]^+$ = 264.0.

***Step 4: Preparation of 6-fluoro-2-(hydroxymethyl)-3-iodo-1-(tetrahydrofuran-3-yl)quinolin-4(1H)-one:***

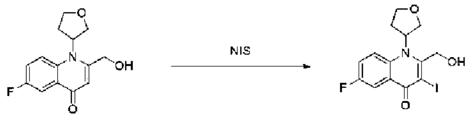


The intermediate obtained from step 3 (3.8 g, 14.4 mmol, 1.0 eq.) was dissolved in acetic acid (AcOH, 30 mL). NIS (6.5 g, 28.9 mmol, 2.0 eq.) was added, and the reaction mixture was stirred at room temperature for 1 hour. LCMS detection confirmed the reaction was complete. The reaction was quenched with saturated aqueous sodium bicarbonate, and the solid was collected by filtration. The solid was dried with acetonitrile to remove residual water, affording the title compound

as a white solid (2 g, yield: 35.6%). LCMS (ESI): $[M + H]^+$ = 390.0.

***Step 5: Preparation of 2-(chloromethyl)-6-fluoro-3-iodo-1-(tetrahydrofuran-3-yl)quinolin-4(1H)-one:***

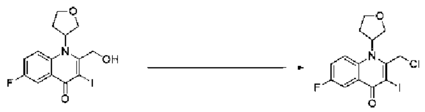

The intermediate obtained from step 4 (300 mg, 0.77 mmol, 1.0 eq.) was dissolved in anhydrous tetrahydrofuran (THF, 3 mL). Thionyl chloride ($SOCl_2$, 92 mg, 0.77 mmol, 1.0 eq.) was added, and the reaction mixture was stirred at room temperature for 1 hour. LCMS detection confirmed the reaction was complete. The solid was collected by filtration, washed with DCM, and dried to obtain the title compound as a white solid (267 mg, yield: 85%). LCMS (ESI): $[M + H]^+$ = 408.0.

***Step 6: Preparation of (4S)-4-ethyl-7-((6-fluoro-3-iodo-4-oxo-1-(tetrahydrofuran-3-yl)-1,4-dihydroquinolin-2-yl) methyl)-4-hydroxy-1,7-dihydro-3H-pyrano[3,4-c]pyridine-3,8(4H)-dione:***

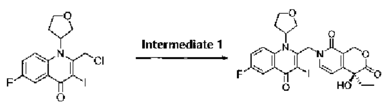


The intermediate obtained from step 5 (520 mg, 1.27 mmol, 1.0 eq.) was dissolved in anhydrous DMF (5 mL). Potassium carbonate ($K_2CO_3$, 352 mg, 2.5 mmol, 2.0 eq.) and Intermediate 1 (267 mg, 1.27 mmol, 1.0 eq.) were added, and the reaction was stirred at room temperature for 4 hours. Water was added to the reaction mixture, and the precipitated solid was collected by filtration. The crude product was purified by preparative thin-layer chromatography (eluent: DCM:MeOH = 20:1) to obtain the title compound as a white solid (115 mg, yield: 15.5%). LCMS (ESI): $[M + H]^+$ = 581.1.

***Step 7: Preparation of (4S)-4-ethyl-8-fluoro-4-hydroxy-11-(tetrahydrofuran-3-yl)-1,12-dihydro-14H-pyrano [3',4':6,7]indolizino[2,1-b]quinoline-3,6,14(4H,11H)-trione:***

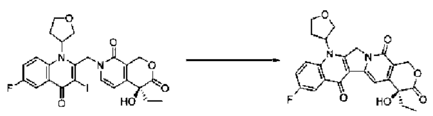

The intermediate obtained from Step 6 (100 mg, 0.17 mmol, 1.0 eq.), AIBN (14.15 mg, 0.09 mmol, 0.5 eq.) and anhydrous toluene (5 mL) were added to a 40 mL vial. Under stirring, nitrogen gas was bubbled into the solution via a syringe needle inserted to the bottom of the solution for 5 minutes to remove air, and the vial was quickly capped. The system was purged with nitrogen three times. A solution of $(TMS)_3SiH$ (171.39 mg, 0.69 mmol, 4.0 eq.) in toluene (3 mL) was prepared and injected into the reaction vial with a syringe. The reaction mixture was stirred at 80 °C overnight, and samples were taken with a syringe for monitoring without opening the cap. After the reaction, the reaction solution was a bright yellow solution containing a small amount of precipitate. Toluene was removed from the reaction solution under reduced pressure by rotary evaporation at 25 °C. The resulting solid was dissolved in DMSO (3 mL) to form a clear and translucent solution, which was purified by preparative HPLC. The collected fractions were transferred to a 250 mL flask and lyophilized to obtain the title compound as a white solid (6.1 mg, yield: 6.49%). LCMS (ESI): m/z = 453.1 $[M + H]^+$; $^1H$-NMR (400 MHz, DMSO-$d_6$) $\delta$ 8.15-8.11 (m, 1H), 7.99 (d, J = 11.8 Hz, 1H), 7.78-7.71 (s, 1H), 7.25 (s, 1H), 6.31 (s, 1H), 5.50-5.31 (m, 4H), 4.38-4.34 (m, 1H), 4.26-4.15 (m, 1H), 4.04-3.92 (m, 1H), 3.69-3.54 (m, 1H), 2.56-2.50 (m, 2H), 2.35-2.25 (m, 1H), 1.79 (q, J = 7.4 Hz, 2H), 0.82 (t, J = 7.3 Hz, 3H).

**Example 32: Synthesis of (4S)-4-ethyl-8-fluoro-4-hydroxy-11-((tetrahydrofuran-3-yl)methyl)-1,12-dihydro-14H-pyrano[3',4':6,7]indolizino[2,1-b]quinoline-3,6,14(4H,11H,12H)-trione:**

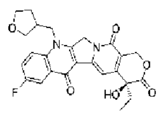

Taking 2-amino-5-fluoroacetophenone and tetrahydrofuran-3-carbaldehyde as the starting materials, the synthesis was carried out by following steps 1-7 of Example 31. LCMS monitoring confirmed the reaction was complete. The reaction solution was concentrated by rotary evaporation, and the resulting residue was dissolved in DMSO (3 mL). One drop of 1 mol/L HCl solution was added, and the mixture was purified by preparative HPLC (C18 column, mobile phase: 0.1% aqueous HCl-acetonitrile). The collected fractions were lyophilized to obtain the title compound as a white solid (5.3 mg, yield: 4.6%). LCMS (ESI): m/z = 467.2 $[M - H]^-$; $^1H$-NMR (400 MHz, DMSO-$d_6$) δ 8.09 (dd, J = 9.3, 4.1 Hz, 1H), 7.96 (dd, J = 8.8, 3.0 Hz, 1H), 7.73-7.77 (m, 1H), 7.25 (s, 1H), 5.40-5.24 (m, 4H), 4.40-4.26 (m, 2H), 3.90-3.89 (m, 1H), 3.67-3.55 (m, 3H), 2.85-2.84 (m, 1H), 2.03-1.97 (m, 1H), 1.79-1.75 (m, 3H), 0.81 (t, J = 7.3 Hz, 3H).

**Example 33: Preparation of (S)-2-(4-ethyl-4-hydroxy-3,6,14-trioxo-4,6,12,14-tetrahydro-1H-pyrano[3',4':6,7]indolizino[2,1-b]quinolin-11(3H)-yl)tert-butyl acetate:**

***Step 1: Preparation of 2-(2-methyl-3-bromo-4-oxoquinolin-1(4H)-yl)tert-butyl acetate:***

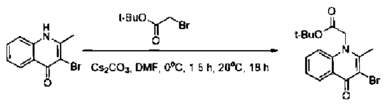


Under an ice-water bath and nitrogen atmosphere, cesium carbonate ($Cs_2CO_3$, 22.57 g, 69.27 mmol, 3.0 eq.) was added to an anhydrous N,N-dimethylformamide (DMF, 100 mL) solution of 3-bromo-2-methylquinolin-4(1H)-one (10 g, 23.09 mmol, 1.0 eq.). The mixture was stirred at 0 °C for 1.5 hours, then tert-butyl bromoacetate (12.51 g, 69.27 mmol, 3.0 eq.) was added. The reaction system was allowed to warm up to room temperature (20 °C) naturally and stirred for 18 hours. TLC monitoring confirmed the reaction was complete. Ice water (150 mL) was added to quench the reaction, and the mixture was extracted with ethyl acetate (EA, 150 mL × 3). The combined organic layers were washed with saturated brine (150 mL × 3), dried over anhydrous sodium sulfate ($Na_2SO_4$), concentrated and purified by flash silica gel column chromatography (eluent gradient: PE/EA = 100/1 to 4/1) to obtain the title compound as a pale yellow solid (6.91 g, yield: 84%). LCMS (ESI): m/z = 352.0 $[M - H]^-$; $^1H$-NMR (400 MHz, $(CD_3)_2SO$) δ 8.23 (dd, J = 8.0, 1.6 Hz, 1H), 7.78-7.74 (m, 1H), 7.64 (d, J = 8.4 Hz, 1H), 7.46-7.42 (m, 1H), 5.24 (s, 2H), 2.73 (s, 3H), 1.44 (s, 9H).

***Step 2: Preparation of 2-(3-bromo-2-(bromomethyl)-4-oxoquinolin-1(4H)-yl)tert-butyl acetate:***

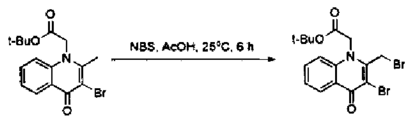


Under nitrogen atmosphere at 0 °C, N-bromosuccinimide (NBS, 27.79 mg, 0.16 mmol, 1.1 eq.) was added to an acetic acid (AcOH, 5 mL) solution of 2-(2-methyl-3-bromo-4-oxoquinolin-1(4H)-yl)tert-butyl acetate (500 mg, 1.42 mmol, 1.0 eq.). The reaction mixture was stirred at room temperature for 6 hours, then diluted with ethyl acetate (EA, 20 mL). The mixture was washed with water (20 mL × 3), dried over anhydrous sodium sulfate, and concentrated, and purified by flash silica gel column chromatography (eluent gradient: PE/EA = 40/1 to 15/1) to obtain the title compound as a pale yellow solid (600 mg, yield: 87.07%). LCMS (ESI): $[M+H]^+$= 430.0. $^1H$ NMR (400 MHz, $(CD_3)_2SO$) δ 8.24 (dd, *J* = 8.0, 1.6 Hz, 1H), 7.83-7.79 (m, 1H), 7.65 (d, *J* = 8.8 Hz, 1H), 7.49 (t, *J* = 7.2 Hz, 1H), 5.40-5.30 (m, 2H), 5.20-5.10 (m, 2H), 1.40 (s, 9H).

***Step 3: Preparation of (S)-2-(3-bromo-2-((4-ethyl-4-hydroxy-3,8-dioxo-4,8-dihydro-1H-pyrano[3,4-c]pyridin-7(3H)-yl)methyl)-4-oxoquinolin-1(4H)-yl)tert-butyl acetate:***

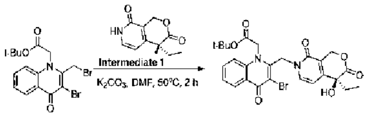


Under nitrogen protection, $K_2CO_3$ (705.28 mg, 5.1 mmol, 4.0 eq.) was added to an anhydrous DMF (10 mL) solution of 2-(3-bromo-2-(bromomethyl)-4-oxoquinolin-1(4H)-yl)tert-butyl acetate (550 mg, 1.28 mmol, 1.0 eq.) and Intermediate 1 (266.89 mg, 1.28 mmol, 1.0 eq.). The reaction mixture was heated to 50 °C and stirred for 2 hours, and LCMS and TLC monitoring showed the product as the main component. The reaction solution was poured into a separating funnel, and ethyl acetate (EA, 50 mL) and water (50 mL) were added for phase separation. The aqueous phase was extracted with EA (50 mL), and the combined organic phases were washed with saturated aqueous sodium chloride (50 mL), dried over anhydrous sodium sulfate ($Na_2SO_4$), and concentrated, and purified by flash silica gel column chromatography (eluent gradient: DCM/MeOH = 30/1 to 15/1), and concentrated to obtain the title compound as a yellowish solid (500 mg, yield: 70.06%). LCMS (ESI): m/z = 558.6 $[M + H]^+$; $^1H$ NMR (400 MHz, $(CD_3)_2SO$) $\delta$ 8.27 (dd, J = 8.0, 1.6 Hz, 1H), 7.87-7.78 (m, 1H), 7.62-7.46 (m, 3H), 6.49 (d, J = 7.2 Hz, 1H), 6.31 (s, 1H), 5.50-5.20 (m, 5H), 1.81-1.67 (m, 2H), 1.40-1.18 (m, 9H), 0.80 (t, J = 7.2 Hz, 3H).

***Step 4: Preparation of (S)-2-(4-ethyl-4-hydroxy-3,6,14-trioxo-4,6,12,14-tetrahydro-1H-pyrano[3',4':6,7]indolizino[2,1-b]quinolin-11(3H)-yl)tert-butyl acetate:***

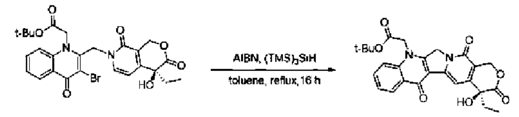


The intermediate obtained from Step 3 (200 mg, 0.36 mmol, 1.0 eq.), AIBN (2.94 mg, 0.02 mmol, 0.05 eq.) and $(TMS)_3SiH$ (355.6 mg, 1.43 mmol, 4.0 eq.) were suspended in anhydrous toluene (10 mL). The reaction solution was heated to 130 °C and refluxed for 16 hours, and LCMS showed the reaction was complete. MeOH (3 mL) was added to quench the reaction, and the solvent was removed by vacuum distillation. The residue was triturated with MeOH (10 mL), filtered to obtain a solid (40 mg), which was purified by reverse-phase preparative HPLC (C18 column, mobile phase: 0.1% aqueous formic acid-acetonitrile) to obtain the title compound as a white solid (13 mg). LCMS (ESI) $[M+H]^+$ = 479.2; $^1H$ NMR (400 MHz, $(CD_3)_2SO$) $\delta$ 8.43-8.31 (m, 1H), 7.86 (t, *J* = 7.2 Hz, 1H), 7.74 (d, *J* = 8.4 Hz, 1H), 7.56 (t, *J* = 7.6 Hz, 1H), 7.31 (s, 1H), 6.37 (s, 1H), 5.43-5.26 (m, 5H), 1.91-1.69 (m, 2H), 1.44 (s, 9H), 0.87 (t, *J* = 7.2 Hz, 3H).

**Example 34: Preparation of (S)-2-(4-ethyl-4-hydroxy-3,6,14-trioxo-4,6,12,14-tetrahydro-1H-pyrano[3',4':6,7]indolizino[2,1-b]quinolin-11(3H)-yl)acetic acid:**

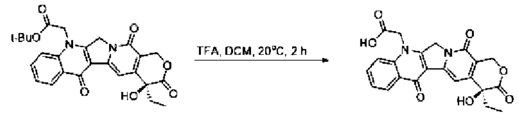


TFA (1.0 mL) was added to a DCM (3.0 mL) solution of the compound from Example 33 (11 mg, 0.03 mmol, 1.0 eq.). The reaction mixture was stirred at 20 °C for 2 hours, and LCMS monitoring confirmed the reaction was complete. The solvent was removed under reduced pressure, and the crude product was purified by reverse-phase preparative HPLC (C18 column, mobile phase: 0.1% aqueous formic acid-acetonitrile) to obtain the title compound as a white solid (2.33 mg). LCMS (ESI): m/z = 422.9 $[M - H]^-$; $^1H$-NMR (400 MHz, $(CD_3)_2SO$) $\delta$ 13.65 (br s, 1H), 8.36 (dd, J = 8.0, 1.2 Hz, 1H), 7.88-7.82 (m, 1H), 7.81-7.76 (m, 1H), 7.55 (t, J = 7.6 Hz, 1H), 7.31 (s, 1H), 6.35 (s, 1H), 5.40-5.27 (m, 4H), 5.29 (s, 2H), 1.92-1.71 (m, 2H), 0.87 (t, J = 7.2 Hz, 3H).

**Example 35: Preparation of (S)-4-ethyl-4-hydroxy-11-(2-morpholinocarbonylmethylene)-1,12-dihydro-14H-pyrano[3',4':6,7]indolizino [2,1-b]quinoline-3,6,14(4H,11H)-trione:**

***Step 1: Preparation of (S)-2-(3-bromo-2-((4-ethyl-4-hydroxy-3,8-dioxo-4,8-dihydro-1H-pyrano[3,4-c]pyridin-7(3H)-yl)methyl)-4-oxoquinolin-1(4H)-yl) acetic acid:***

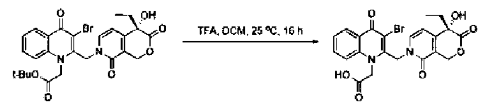


Trifluoroacetic acid (3 mL) was added to a DCM (3 mL) solution of the intermediate obtained from Step 4 of Example 33 (330 mg, 0.59 mmol, 1.0 eq.). The mixture was stirred at 25 °C for 16 hours, and LCMS monitoring confirmed the reaction was complete with the product as the main component. The reaction solution was concentrated under reduced pressure to obtain the title compound (approximately 350 mg, crude product), which was used directly in the next step without further purification. LCMS (ESI): m/z = 503.0/505.0 $[M + H]^+$ (dual peaks due to bromine isotopes).

***Step 2: Preparation of (S)-7-((3-bromo-1-(2-morpholinocarbonylmethylene)-4-oxo-1,4-dihydroquinolin-2-yl) methyl)-4-ethyl-4-hydroxy-1,7-dihydro-3H-pyrano [3,4-c]pyridine-3,8(4H)-dione:***

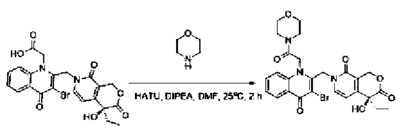


Under nitrogen protection, HATU (135.99 mg, 0.36 mmol, 1.2 eq.) was added to an anhydrous DMF (5 mL) solution of the crude intermediate obtained from Step 1 (150 mg). The mixture was stirred at 25 °C for 10 minutes, then DIPEA (154.07 mg, 1.19 mmol, 4.0 eq.) and morpholine (38.95 mg, 0.45 mmol, 1.5 eq.) were added sequentially. Stirring was continued at 25 °C for 2 hours. The reaction solution was concentrated by rotary evaporation, and the crude product was purified by reverse-phase column chromatography (C18 column, mobile phase: 0.1% aqueous formic acid-acetonitrile) followed by lyophilization to obtain the title compound as a yellowish solid (82 mg, yield: 46.97%). LCMS: (ESI) $[M+H]^+$=572.0; $^1H$ NMR (400 MHz, $CDCl_3$) δ 8.53-8.44 (d, *J* = 8 Hz, 1H), 7.66-7.63 (m, 1H), 7.53 (d, *J*= 7.2 Hz, 1H), 7.42 (t, *J* = 7.6 Hz, 1H), 7.08 (d, *J* = 8.8 Hz, 1H), 6.60 (d, *J* = 7.2 Hz, 1H), 5.78 (m , 2H), 5.56 (d, *J* = 16.0 Hz, 1H), 5.30 (s, 1H), 5.18 (d, *J* = 16.4 Hz, 2H), 3.85-3.82 (m, 2H), 3.70-3.65 (m, 2H), 3.56 (s, 3H), 3.49-3.45 (m, 1H), 1.88-1.69 (m, 2H), 0.97 (t, *J* = 7.2 Hz, 3H)。

***Step 3: Preparation of (S)-4-ethyl-4-hydroxy-11-(2-morpholinocarbonylmethylene)-1,12-dihydro-14H-pyrano [3',4':6,7]indolizino [2,1-b]quinoline-3,6,14(4H,11H)-trione:***

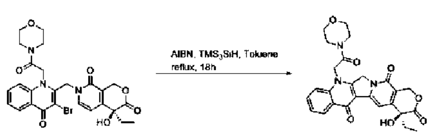


Under nitrogen protection, AIBN (1.18 mg, 0.01 mmol, 0.05 eq.) and $(TMS)_3SiH$ (142.49 mg, 0.57 mmol, 4.0 eq.) were added to an anhydrous toluene (3 mL) solution of the intermediate obtained from step 2. The reaction mixture was heated at 130 °C for 18 hours, and LCMS monitoring confirmed the reaction was complete. The reaction solution was concentrated under reduced pressure, and the residue was triturated with petroleum ether (10 mL). The resulting solid was filtered to obtain a crude product (34 mg), which was purified by reverse-phase preparative HPLC (C18 column, mobile phase: 0.1% aqueous formic acid-acetonitrile) to obtain the title compound as a white solid (3.26 mg, yield: 4.65%). LCMS (ESI): m/z = 492.2 $[M - H]^-$; $^1H$-NMR (400 MHz, $(CD_3)_2SO$) δ 8.36 (dd, J = 8.0, 1.6 Hz, 1H), 7.88-7.79 (m, 1H), 7.73 (d, J = 8.8 Hz, 1H), 7.59-7.49 (m, 1H), 7.31 (s, 1H), 6.37 (s, 1H), 5.52 (br s, 2H), 5.40-5.33 (m, 2H), 5.27-5.20 (m, 2H), 3.78 (d, J = 4.8

Hz, 2H), 3.67-3.63 (m, 2H), 3.65-3.59 (m, 2H), 3.48-3.44 (m, 2H), 1.88-1.77 (m, 2H), 0.92-0.81 (m, 3H).

**Example 36: Preparation of (S)-4-ethyl-8-fluoro-4-hydroxy-11-isopropyl-1,12-dihydro-14H-pyrano[3',4':6,7]indolizino[2,1-b]quinoline-3,6,14(4H,11H)-trione:**

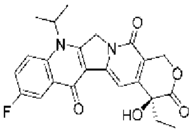

Using commercially available 6-fluoro-1-isopropyl-4-oxo-1,4-dihydroquinoline-2-carboxylic acid as the starting material, the synthesis was carried out by following steps 1-5 of Example 6. LCMS monitoring confirmed the reaction was complete during the whole process. After purification, the title compound was obtained. LCMS (ESI): m/z = 425.2[M-H]$^-$.

**Example 37: Preparation of (S)-4-ethyl-8-fluoro-4-hydroxy-11-cyclopropyl-1,12-dihydro-14H-pyrano[3',4':6,7] indolizino[2,1-b]quinoline-3,6,14(4H,11H)-trione:**

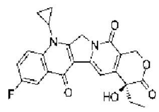

Using commercially available 6-fluoro-1-cyclopropyl-4-oxo-1,4-dihydroquinoline-2-carboxylic acid as the starting material, the title compound was synthesized by following the relevant operations of steps 1-5 of Example 6. LCMS monitoring confirmed the reaction was complete during the whole process. After purification, the target product was obtained. LCMS (ESI): m/z = 423.2 [M-H]$^-$; $^1$H NMR (400 MHz, $CDCl_3$) δ 8.07-8.04 (m, 1H), 7.57 (d, J = 8.5 Hz, 1H), 7.42-7.37 (m, 1H), 7.32 (s, 1H), 5.59 (d, J = 15.7 Hz, 1H), 5.43 (d, J = 20.6 Hz, 1H), 5.19-5.11 (m, 2H), 4.11 (s, 1H), 3.49-3.45 (m, 1H), 1.90-1.78 (m, 2H), 1.52-1.25 (m, 3H), 0.98 (t, J = 7.0 Hz, 3H), 0.89-0.83 (m, 1H).

**Example 38: Preparation of (S)-4-ethyl-8-fluoro-4-hydroxy-11-(3,3-difluorocyclobutyl)-1,12-dihydro-14H-pyrano[3',4':6,7]indolizino[2,1-b]quinoline -3,6,14(4H,11H)-trione:**

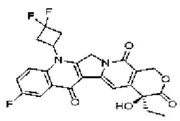

Using commercially available p-fluoroaniline as the starting material, the intermediate 2-((4-fluorophenyl)amino) maleate was synthesized by referring to the synthesis method of step 1 of Example 7. Subsequently, alkylation reaction was carried out with 3,3-difluorocyclobutylamine, and then the title compound was synthesized by following the relevant operations of steps 2-8 of Example 7. LCMS monitoring confirmed the reaction was complete during the whole process. After purification, the target product was obtained. LCMS (ESI): m/z = 373.2[M-H]$^-$.

**Example 39: Preparation of (S)-4-ethyl-8-fluoro-4-hydroxy-10,11-dimethyl-14-thioxo-12,14-dihydro-1H-pyrano [3',4':6,7]indolizino[2,1-b]quinoline-3,6(4H, 11H)-dione:**

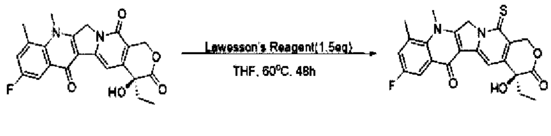

The compound from Example 2 (50.0 mg, 0.12 mmol, 1.0 eq.) was placed in a microwave tube, and anhydrous tetrahydrofuran (THF, 10 mL) was added to dissolve it. Lawson's reagent (73.9 mg, 0.18 mmol, 1.5 eq.) was added, and nitrogen gas was introduced into the microwave tube to replace the air inside. The tube was sealed, and the reaction solution was heated to 55 °C and stirred for 48 hours. LCMS detection showed the reaction conversion rate was about 50%. The reaction solution was concentrated to obtain a crude product, which was purified by silica gel column chromatography (eluent gradient: DCM:MeOH = 1:0 to 30:1) to obtain a partially purified crude product (20 mg). Further purification by reverse-phase preparative HPLC afforded the title compound as a yellow solid (2.0 mg, yield: 3.91%). LCMS (ESI): m/z = 427.0 $[M + H]^+$; $^1$H-NMR (400 MHz, DMSO-$d_6$) δ 8.64 (dd, J = 10.2, 3.2 Hz, 1H), 8.51 (s, 1H), 7.75 - 7.71 (m, 1H), 6.35 (s, 1H), 5.43 - 5.35 (m, 4H), 4.20 (s, 3H), 2.90 (s, 3H), 1.89 - 1.78 (m, 2H), 0.87 (t, J = 7.3 Hz, 3H).

**Example 40: Preparation of ((S,E)-2-(4-ethyl-8-fluoro-4-hydroxy-3,6,14-trioxo-3,4-dihydro-1H-pyrano[3',4':6,7] indolizino[2,1-b]quinolin-11(6H,12H,14H)-yl)acetaldehyde O-methyl oxime (isomer 1 or isomer 2)) and**

**Example 41: Preparation of ((S,Z)-2-(4-ethyl-8-fluoro-4-hydroxy-3,6,14-trioxo-3,4-dihydro-1H-pyrano[3',4':6,7] indolizino[2,1-b]quinolin-11(6H,12H,14H)-yl)acetaldehyde O-methyl oxime (isomer 2 or isomer 1)):**

***Step 1: Preparation of (2-bromoethyl) 6-fluoro-4-oxo-1,4-dihydroquinoline-2-carboxylate:***

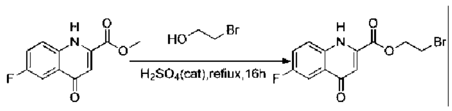


Methyl 6-fluoro-4-oxo-1,4-dihydroquinoline-2-carboxylate (45 g, 203.45 mmol, 1.0 eq) was charged into a reaction vessel, followed by the addition of 2-bromoethanol (200 mL). Concentrated sulfuric acid (9.98 g, 101.73 mmol, 0.5 eq) was added dropwise slowly, and the mixture was refluxed for 16 h. The reaction completion was monitored by LCMS. The mixture was concentrated under reduced pressure, and the residue was purified by silica gel column chromatography (DCM:MeOH = 100:1/20:1) to afford the title compound as a white solid (32 g, yield: 50.07%). LCMS (ESI) $[M+H]^+$ = 316.0.

***Step 2: Preparation of 6-fluoro-1-(2-hydroxyethyl)-4-oxo-1,4-dihydroquinoline-2-carboxylic acid:***

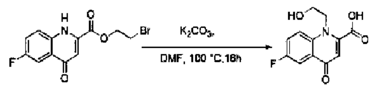


$K_2CO_3$ (21.12 g, 152.82 mmol, 1.5 eq.) was added to a DMF (120 mL) solution of the compound from step 1 (32 g, 101.88 mmol, 1.0 eq.). The mixture was stirred at 100 °C for 16 hours. After completion was confirmed by LCMS, potassium carbonate was filtered off, and DMF in the filtrate was removed, and purified by silica gel column chromatography (eluent gradient: DCM/MeOH = 100:1 to 20:1) to obtain the title compound as a pale yellow solid (21 g, yield: 82.06%). LCMS (ESI): $[M+H]^+$ =252.2; $^1$H-NMR (400 MHz, $(CD_3)_2SO$) δ 7.93-7.84 (m, 1H), 7.82-7.74 (m, 1H), 7.60-7.49 (m, 1H), 6.10 (s, 1H), 5.85 (s, 1H), 4.43 (t, J = 5.6 Hz, 2H), 3.74 (t, J = 5.6 Hz, 2H).

***Step 3: Preparation of 1-(2-(benzyloxy)ethyl)-6-fluoro-4-oxo-1,4-dihydroquinoline-2-carboxylic acid:***

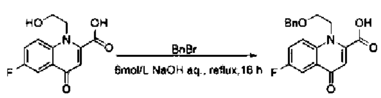


The compound from step 2 (21 g, 83.6 mmol, 1.0 eq.) was dissolved in 6 mol/L aqueous sodium hydroxide solution. Benzyl bromide (12.87 g, 75.24 mmol, 0.9 eq.) was added, and the reaction mixture was refluxed overnight. LCMS monitoring confirmed the reaction was complete. The mixture was acidified at 0 °C, and the resulting solid was

collected by filtration to obtain the title compound as a crude product (15 g, yield: 52.57%), which was used directly in the next step without further purification. LCMS (ESI): $[M + H]^+$ = 342.2.

***Step 4: Preparation of methyl 1-(2-(benzyloxy)ethyl)-6-fluoro-4-oxo-1,4-dihydroquinoline-2-carboxylate:***

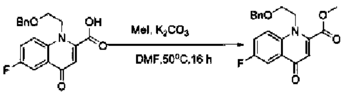


Under nitrogen protection, the compound from step 3 (15 g, 43.95 mmol, 1.0 eq.) was dissolved in anhydrous DMF (150 mL). $K_2CO_3$ (18.22 g, 131.84 mmol, 3.0 eq.) and iodomethane (62.38 g, 439.46 mmol, 10.0 eq.) were slowly added to the reaction mixture, and the reaction was carried out at 50 °C for 16 hours. LCMS monitoring confirmed the reaction was complete. The reaction solution was concentrated, and purified by silica gel column chromatography (eluent gradient: DCM:MeOH = 100:1 to 20:1) to obtain the title compound as a pale yellow solid (10 g, yield: 64.03%). LCMS (ESI): $[M+H]^+$ = 356.2.

***Step 5: Preparation of 1-(2-(benzyloxy)ethyl)-6-fluoro-2-(hydroxymethyl)quinolin-4(1H)-one:***

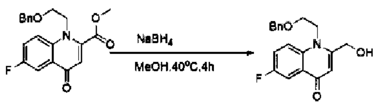


Under an ice-water bath, $NaBH_4$ (2.13 g, 56.28 mmol, 2.0 eq.) was slowly added to a MeOH (100 mL) solution of the compound from step 4 (10 g, 28.14 mmol, 1.0 eq.). The mixture was stirred at 40 °C for 4 hours, and LCMS monitoring confirmed the reaction was complete. The reaction was quenched by slow addition of ice-water (15 mL) at 0 °C, then concentrated to remove the solven, and purified by silica gel column chromatography (eluent gradient: DCM:MeOH = 100:1 to 20:1) to obtain the title compound as a pale white solid (5.4 g, yield: 58.62%). LCMS (ESI): $[M + H]^+$ = 328.2; $^1$H-NMR (400 MHz, $(CD_3)_2SO$) δ 7.95-7.85 (m, 1H), 7.84-7.77 (m, 1H), 7.62-7.52 (m, 1H), 7.30-7.17 (m, 3H), 7.15-7.07 (m, 2H), 6.29 (s, 1H), 5.78 (s, 1H), 4.69 (s, 2H), 4.56 (t, J = 5.2 Hz, 2H), 4.42 (s, 2H), 3.75 (t, J = 5.2 Hz, 2H).

***Step 6: Preparation of 1-(2-(benzyloxy)ethyl)-6-fluoro-2-(hydroxymethyl)-3-iodoquinolin-4(1H)-one:***

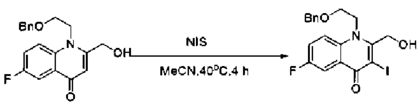


The compound from step 5 (5.4 g, 16.5 mmol, 1.0 eq.) was dissolved in acetonitrile (50 mL). NIS (4.45 g, 19.8 mmol, 1.2 eq.) was added, and the reaction mixture was stirred at 40 °C for 4 hours. LCMS monitoring confirmed the reaction was complete. The solvent was removed, and purified by silica gel column chromatography (eluent gradient: DCM/MeOH = 100:1 to 20:1) to obtain the title compound as a pale white solid (5.9 g, yield: 78.89%). LCMS (ESI): $[M + H]^+$ = 454.0; $^1$H-NMR (400 MHz, $(CD_3)_2SO$) δ 8.07-7.96 (m, 1H), 7.92-7.81 (m, 1H), 7.73-7.58 (m, 1H), 7.35-7.18 (m, 3H), 7.14-7.06 (m, 2H), 5.97 (s, 1H), 5.21 (s, 2H), 4.89 (t, J = 4.9 Hz, 2H), 4.42 (s, 2H), 3.79 (t, J = 5.2 Hz, 2H).

***Step 7: Preparation of 1-(2-(benzyloxy)ethyl)-2-(chloromethyl)-6-fluoro-3-iodoquinolin-4(1H)-one:***

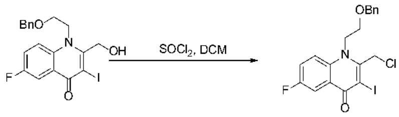

The compound from step 6 (5.4 g, 11.91 mmol, 1.0 eq.) was dissolved in DCM (50 mL). Oxalyl chloride (7.09 g, 59.57 mmol, 5.0 eq.) was slowly added, and the reaction mixture was stirred at 25 °C for 4 hours. LCMS monitoring confirmed the reaction was complete. The solvent was removed, dried, and purified by silica gel column chromatography to obtain the title compound as a light yellow solid (5.0 g, yield: 88.97%). LCMS (ESI): $[M + H]^+ = 472.3$; $^1$H-NMR (400 MHz, $(CD_3)_2SO$) δ 8.12-8.00 (m, 1H), 7.89-7.80 (m, 1H), 7.74-7.62 (m, 1H), 7.27-7.16 (m, 3H), 7.15-7.01 (m, 2H), 5.36 (s, 2H), 4.82 (s, 2H), 4.43 (s, 2H), 3.83 (t, J = 4.8 Hz, 2H).

***Step 8: Preparation of (S)-7-((1-(2-(benzyloxy)ethyl)-6-fluoro-3-iodo-4-oxo-1,4-dihydroquinolin-2-yl)methyl)-4-ethyl-4-hydroxy-1H-pyrano[3,4-c]pyridine-3,8(4H, 7H)-dione:***

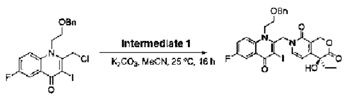


To a solution of the compound from step 2 (5.0 g, 10.6 mmol, 1.0 eq.) and Intermediate 1 (2.2 g, 10.6 mmol, 1.0 eq.) in acetonitrile (60 mL) was added $K_2CO_3$ (4.39 g, 31.8 mmol, 3.0 eq.). The mixture was stirred at room temperature overnight. LCMS monitoring confirmed the reaction was complete, then the mixture was filtered, and the filtrate was concentrated, and purified by silica gel column chromatography (eluent gradient: DCM/MeOH = 100:1 to 20:1) to afford the title compound as a light yellow solid (4.5 g, yield: 65.88%). LCMS (ESI): $[M + H]^+ = 645.1$; $^1$H-NMR (400 MHz, $(CD_3)_2SO$) δ 8.09-8.00 (m, 1H), 7.90-7.86 (m, 1H), 7.73-7.61 (m, 1H), 7.46-7.35 (m, 1H), 7.29-7.17 (m, 3H), 7.15-7.06 (m, 2H), 6.40 (d, J = 7.2 Hz, 1H), 6.32 (s, 1H), 5.74 (d, J = 9.2 Hz, 2H), 5.32 (s, 2H), 4.66 (s, 2H), 4.40 (s, 2H), 3.81-3.64 (m, 2H), 1.85-1.67 (m, 2H), 0.79 (t, J = 7.2 Hz, 3H).

***Step 9: Preparation of (S)-4-ethyl-7-((6-fluoro-1-(2-hydroxyethyl)-3-iodo-4-oxo-1,4-dihydroquinolin-2-yl) methyl)-4-hydroxy-1H-pyrano[3,4-c]pyridine-3,8(4H,7H)-dione:***

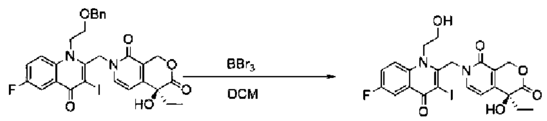


At -5 °C, the intermediate obtained from step 8 (1.0 g, 1.55 mmol, 1.0 eq.) was dissolved in DCM (20 mL). A 1 mol/L DCM solution of boron tribromide (3.1 mL, 3.1 mmol, 2.0 eq.) was slowly added dropwise over approximately 30 minutes. The reaction mixture was stirred at -5 °C for 2 hours, and LCMS monitoring confirmed the reaction was complete. While maintaining the temperature at -5 °C, an aqueous sodium bicarbonate solution (15 mL) was slowly added to quench the excess boron tribromide. The mixture was extracted with DCM, the combined organic layers were dried over anhydrous sodium sulfate, concentrated, and purified by silica gel column chromatography (eluent gradient: DCM/MeOH = 100:1 to 10:1) to obtain the title compound as a pale yellow solid (310 mg, yield: 36.08%). LCMS (ESI): m/z = 554.7 $[M-H]^-$; $^1$H-NMR (400 MHz, $(CD_3)_2SO$) δ 8.08-7.99 (m, 1H), 7.93-7.86 (m, 1H), 7.77-7.67 (m, 1H), 7.45-7.34 (m, 1H), 6.40 (d, J = 7.2 Hz, 1H), 6.32 (s, 1H), 5.72 (s, 2H), 5.33 (s, 2H), 5.16 (s, 1H), 4.50 (s, 2H), 3.72 (d, J = 4.4 Hz, 2H), 1.86-1.68 (m, 2H), 0.80 (t, J = 7.2 Hz, 3H).

***Step 10: Preparation of (S)-2-(2-((4-ethyl-4-hydroxy-3,8-dioxo-3,4-dihydro-1H-pyrano[3,4-c]pyridin-7(8H)-yl) methyl)-6-fluoro-3-iodo-4-oxoquinolin-1(4H)-yl) acetaldehyde:***

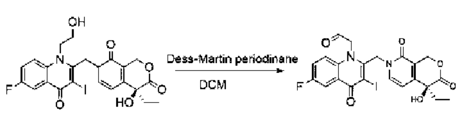


The intermediate obtained from step 9 (200 mg, 0.36 mmol, 1.0 eq.) was dissolved in anhydrous DCM (20 mL). Dess-Martin periodinane (306.07 mg, 0.72 mmol, 2.0 eq.) was slowly added, and the reaction mixture was stirred at r.t. for

16 hours. LCMS monitoring confirmed the reaction was complete. The mixture was filtered, and the filtrate was concentrated to obtain the title compound as a crude product (220 mg), used directly in the next step without further purification. LCMS (ESI): $[M+H]^+$ = 585.2.

***Step 11: Preparation of ((S,E)-2-(2-((4-ethyl-4-hydroxy-3,8-dioxo-3,4-dihydro-1H-pyrano[3,4-c]pyridin-7(8H)-yl) methyl)-6-fluoro-3-iodo-4-oxoquinolin-1(4H)-yl)ethyl)acetaldehyde O-methyloxime:***

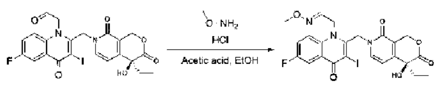


The intermediate obtained from step 10 (220 mg, 0.4 mmol, 1.0 eq.) was dissolved in ethanol (5 mL). Glacial acetic acid (143.52 mg, 2.39 mmol, 6.0 eq.) was added dropwise, followed by methoxyamine hydrochloride (332.69 mg, 3.98 mmol, 10.0 eq.). The reaction mixture was stirred at 25 °C overnight. LCMS monitoring confirmed the reaction was complete. The reaction mixture was concentrated u, and purified by silica gel column chromatography (eluent gradient: DCM:MeOH = 100:1 to 15:1) to give the title compound as a light off-white solid (165 mg, yield: 71%). LCMS (ESI): $[M + H]^+$ = 582.0; $^1$H-NMR (400 MHz, $CDCl_3$) $\delta$ 8.20-7.97 (m, 1H), 7.51-7.43 (m, 2H), 7.37-7.31 (m, 1H), 7.01-6.95 (m, 1H), 6.68-6.59 (m, 1H), 6.20-5.86 (m, 2H), 5.66-5.57 (m, 1H), 5.36-5.21 (m, 1H), 5.18-4.98 (m, 2H), 4.00 (s, 1H), 3.63 (s, 1H), 3.55 (s, 1H), 1.91-1.77 (m, 2H), 1.07-0.97 (m, 3H).

***Step 12: Preparation of ((S,E)-2-(4-ethyl-8-fluoro-4-hydroxy-3,6,14-trioxo-3,4-dihydro-1H-pyrano[3,4:6,7]indolizino[2,1 -b]quinolin-11(6H,12H,14H)-yl)acetaldehyde O-methyloxime (Isomer 1 or Isomer 2) and ((S,Z)-2-(4-ethyl-8-fluoro-4-hydroxy-3,6,14-trioxo-3,4-dihydro-1H-pyrano[3,4:6,7]indolizino[2,1 - b]quinolin-11(6H,12H,14H)-yl)acetaldehyde O-methyloxime (Isomer 2 or Isomer 1):***

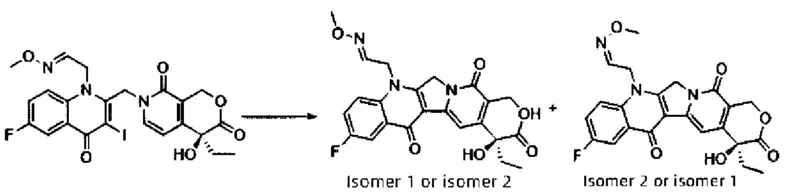

Isomer 1 or isomer 2 Isomer 2 or isomer 1

The product obtained from step 11 (165 mg, 0.28 mmol, 1.0 eq.) and AIBN (23.3 mg, 0.14 mmol, 0.5 eq.) were placed in a dry three-necked flask. Under nitrogen protection, the flask was evacuated using an oil pump, and anhydrous toluene (15 mL) was added. A toluene solution (5 mL) of $(TMS)_3SiH$ (352.89 mg, 1.42 mmol, 5.0 eq.) was added to the mixture. The reaction was heated to 100 °C and stirred for 16 hours. LCMS monitoring indicated the reaction was complete. The mixture was cooled to room temperature, and the solid was collected by filtration. The filter cake was triturated with methyl tert-butyl ether (MTBE, 8 mL) and filtered to obtain the crude title compound (120 mg). Purification by reverse-phase preparative HPLC (C18 column, mobile phase: 0.1% aqueous trifluoroacetic acid (TFA) solution-acetonitrile) afforded two isomers as light yellow solid: the title compound (Isomer 1 or Isomer 2: 2.07 mg, yield not calculated) and the title compound (Isomer 2 or Isomer 1: 4.84 mg, yield not calculated); the specific configuration was not determined. **Isomer 1 or Isomer 2:** LCMS (ESI): $[M + H]^+$ = 453.8; $^1$H-NMR (400 MHz, $(CD_3)_2SO$) $\delta$ 8.04-7.98 (m, 1H), 7.85-7.67 (m, 2H), 7.29 (s, 1H), 7.13-7.05 (m, 1H), 6.37 (s, 1H), 5.45-5.19 (m, 6H), 3.98-3.71 (m, 3H), 1.86-1.78 (m, 2H), 0.90-0.84 (m, 3H).

**Isomer 2 or Isomer 1:** LCMS (ESI): $[M + H]^+$ = 453.8; $^1$H-NMR (400 MHz, $(CD_3)_2SO$) $\delta$ 8.07-7.96 (m, 2H), 7.85-7.75 (m, 1H), 7.72-7.66 (m, 1H), 7.29 (s, 1H), 6.37 (s, 1H), 5.46-5.38 (m, 2H), 5.39-5.31 (m, 4H).

**Example 42: Preparation of (S)-11-(2-(benzyloxy)ethyl)-4-ethyl-8-fluoro-4-hydroxy-1H-pyrano[3,4:6,7]indolizino[2,1 -b]quinoline-3,6,14(4H,11H,12H)-trione:**

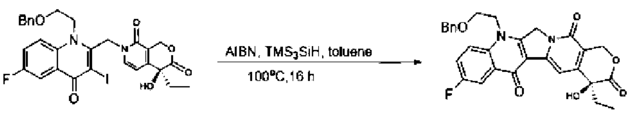


The intermediate obtained from step 8 of Example 40 (500 mg, 0.78 mmol, 1.0 eq.) and azobisisobutyronitrile (AIBN, 63.7 mg, 0.39 mmol, 0.5 eq.) were placed in a dry three-necked flask. Under nitrogen protection, the flask was evacuated using an oil pump, and anhydrous toluene (30 mL) was added via syringe. A toluene solution of tris(trimethylsilyl)silane ($(TMS)_3SiH$, 964.65 mg, 3.88 mmol, 5.0 eq.) was added to the mixture, and the reaction was heated to 100 °C and stirred for 16 hours. LCMS monitoring indicated the reaction was complete. The reaction mixture was allowed to cool to room temperature, and the solid was collected by filtration. The filter cake was slurried with methyl tert-butyl ether (MTBE, 10 mL), stirred, and filtered to obtain the crude product (400 mg). Purification by reverse-phase preparative HPLC (C18 column, mobile phase: 0.1% aqueous trifluoroacetic acid (TFA) solution-acetonitrile) afforded the title compound as a pale yellow solid (45 mg, yield not calculated). LCMS (ESI): $[M + H]^+ = 516.9$; $^1$H-NMR (400 MHz, $(CD_3)_2SO$) $\delta$ 8.13-8.05 (m, 1H), 8.03-7.95 (m, 1H), 7.78-7.66 (m, 1H), 7.29 (s, 1H), 7.18-7.07 (m, 3H), 7.05-6.97 (m, 2H), 6.35 (s, 1H), 5.43 (s, 2H), 5.40-5.28 (m, 2H), 4.70 (s, 2H), 4.41 (s, 2H), 3.86 (t, J = 4.8 Hz, 2H), 1.95-1.71 (m, 2H), 0.87 (t, J = 7.2 Hz, 3H).

**Example 43: Preparation of (S)-4-ethyl-8-fluoro-4-hydroxy-11-(2-hydroxyethyl)-1H-pyrano[3,4:6,7]indolizino [2,1 -b]quinoline-3,6,14(4H,11H,12H)-trione:**

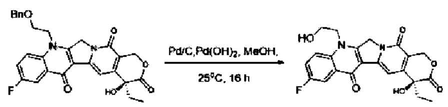


Under a nitrogen atmosphere, the compound from Example 42 (35 mg, 0.07 mmol, 1.0 eq.) was dissolved in MeOH (5 mL). 10% Pd/C (7.45 mg, 0.01 mmol, 0.1 eq.) and 10% $Pd(OH)_2$ (9.69 mg, 0.01 mmol, 0.1 eq.) were added sequentially. The reaction mixture was purged with hydrogen three times, and then stirred under a hydrogen atmosphere at room temperature overnight. LCMS monitoring confirmed the reaction was complete. The mixture was filtered to obtain a crude product (25 mg), which was purified by reverse-phase preparative HPLC (C18 column, mobile phase: 0.1% aqueous formic acid (FA) solution-acetonitrile) to yield the title compound as a pale yellow solid (16.97 mg, yield not calculated). LCMS (ESI): $[M + H]^+ = 427.2$; $^1$H-NMR (400 MHz, $(CD_3)_2SO$) $\delta$ 8.14-8.07 (m, 1H), 8.04-7.96 (m, 1H), 7.80-7.71 (m, 1H), 7.29 (s, 1H), 6.36 (s, 1H), 5.46 (s, 2H), 5.40-5.27 (m, 2H), 5.18 (t, J = 5.6 Hz, 1H), 4.54 (s, 2H), 3.90-3.80 (m, 2H), 1.89-1.72 (m, 2H), 0.86 (t, J = 7.2 Hz, 3H).

**Example 44: Preparation of (S)-4-ethyl-8-fluoro-4-hydroxy-11-(4-hydroxybutyl)-1,12-dihydro-14H-pyrano [3,4:6,7]indolizino[2,1 -b]quinoline-3,6,14(4H,11H)-trione:**

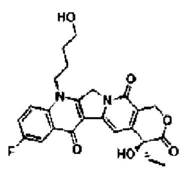

Using commercially available methyl 6-fluoro-4-oxo-1,4-dihydroquinoline-2-carboxylate and 4-bromo-1-butanol as the starting materials, the title compound was synthesized by following the procedures of steps 1-8 of Example 41, combined with the relevant operations of Examples 42 and 43. LCMS (ESI): $[M + H]^+ = 455.2$ $^1$H NMR (400 MHz, DMSO-*d$_6$*): $\delta$ 8.08 (dd, J = 8.9, 3.7 Hz, 1H), 7.98 (dd, J = 8.7, 2.6 Hz, 1H), 7.78-7.72 (m, 1H), 7.28 (s, 1H), 6.35 (s, 1H), 5.43 (s, 2H), 5.39-5.30 (m, 2H), 4.48-4.33 (m, 2H), 3.48 (t, J = 5.9 Hz, 2H), 1.93-1.76 (m, 4H), 1.68-1.55 (m, 2H), 0.87 (t, J = 7.0 Hz, 3H).

**Example 45: Preparation of (4S)-11-(4-cyclopropyl-4-hydroxybutyl)-4-ethyl-8-fluoro-4-hydroxy-9-methoxy-1,12-dihydro-14H-pyrano[3,4:6,7]indazolo[2,1 -b]quinoline-3,6,14(4H,11H)-trione:**

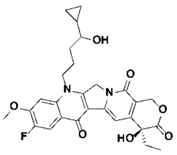

*Step 1: Preparation of (E)-1-(2,5-difluoro-4-methoxyphenyl)-3-((4-hydroxybutyl)amino)but-2-en-1-one:*

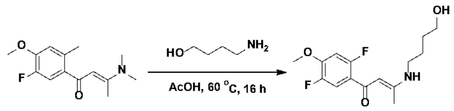


The intermediate obtained from Preparation Example 39 (16.2 g, 64.5 mmol, 1.0 eq.) was dissolved in acetic acid (AcOH, 160 mL). 4-Aminobutan-1-ol (6.3 g, 71.0 mmol, 1.1 eq.) was added to the solution, and the reaction mixture was heated to 60 °C and stirred for 16 hours. The reaction progress was monitored by LCMS until the starting material was completely consumed. The reaction mixture was poured into ethyl acetate (EA, 800 mL). The resulting organic phase was washed with water (100 mL × 3), dried over anhydrous sodium sulfate, and concentrated, purified by silica gel column chromatography (200-300 mesh silica gel, eluent: petroleum ether (PE):tetrahydrofuran (THF) = 1:2, v/v) to afford the title compound as yellow solid(8.55 g, 28.6 mmol, yield: 45.0%). LCMS (ESI): $[M + H]^+$ = 300.2

*Step 2: Preparation of 6-fluoro-1-(4-hydroxybutyl)-7-methoxy-2-methylquinolin-4(1H)-one:*

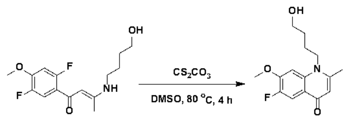


The intermediate obtained from Step 1 (8.55 g, 28.6 mmol, 1.0 eq.) was dissolved in dimethyl sulfoxide (DMSO, 200 mL). Cesium carbonate ($Cs_2CO_3$, 28 g, 85.8 mmol, 3.0 eq.) was added to the solution, and the reaction mixture was heated to 80 °C and stirred for 4 hours. After the reaction was completed, the mixture was cooled to room temperature and poured into water (100 mL). The resulting mixture was filtered to remove insoluble solids. The aqueous filtrate was extracted with ethyl acetate (EA, 200 mL × 3). The combined organic phases were dried over anhydrous sodium sulfate, filtered, and concentrated, and purified by silica gel column chromatography (200-300 mesh silica gel, eluent: PE:THF) = 1:3, v/v) to afford the title compound as a yellow solid (5.43 g, 19.4 mmol, yield: 69.2%). LCMS (ESI): $[M + H]^+$ = 280.2

*Step 3: Preparation of 4-(6-fluoro -7- methoxy -2- methyl -4- carbonyl quinoline -1(4H)- yl) butyraldehyde:*

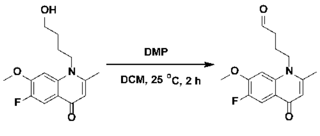


The intermediate obtained from step 2 (5.43 g, 19.4 mmol, 1 eq.) was dissolved in DCM (100 mL), and Dessmartin oxidizer (12.3 g, 29.1 mmol, 1.5 eq.alent) was added at room temperature. The reaction was completed at 25 °C for 2 hours by LCMS. Add the reaction mixture into saturated sodium thiosulfate aqueous solution (100 mL), then extract with DCM (3*200 mL), wash the organic phase with saturated sodium bicarbonate aqueous solution (100 mL), dry, spin-dry the organic phase, and separate and purify with silica gel column (silica gel with 200-300mesh, PE: THF =3:1) to obtain the title compound (4.2 g). LCMS (ESI) $[M+H]^+$ = 278.2.

***Step 4: Preparation of 1-(4-cyclopropyl -4- hydroxybutyl) -6-fluoro -7- methoxy -2- methylquinoline -4(1H)-one:***

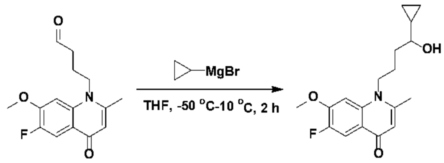


The intermediate obtained from Step 3 (3.1 g, 11.2 mmol, 1.0 eq.) was dissolved in tetrahydrofuran (THF, 60 mL). A solution of cyclopropylmagnesium bromide (1 mol/L in THF, 33.6 mL, 33.6 mmol, 3.0 eq.) was added to the above solution. The reaction mixture was stirred at -50 °C to 10 °C for 2 hours, and the reaction progress was monitored by LCMS until the starting material was completely consumed. After the reaction was completed, saturated ammonium chloride aqueous solution (50 mL) was added to quench the reaction. The mixture was extracted with ethyl acetate (EA, 100 mL × 3). The combined organic phases were washed with saturated sodium chloride aqueous solution (50 mL), dried over anhydrous sodium sulfate, filtered, and concentrated, and purified by silica gel column chromatography (200-300 mesh silica gel, eluent: PE: THF = 3:1, v/v) to afford the title compound as a pale yellow solid (1.8 g). LCMS (ESI): $[M+H]^+$ = 320.2.

Using the intermediate obtained in step 4, the title compound was synthesized as a yellow solid according to steps 3-6 of Example 3. LCMS(ESI)$[M+H]^+$=525.4; $^1$H-NMR (400 MHz, DMSO-$d_6$+$D_2O$) δ 7.97 (d, J = 11.2 Hz, 1H), 7.45 (s, 1H), 7.26 (s, 1H), 5.42-5.24 (m, 2H), 4.48-4.30 (m, 2H), 4.14-3.96 (m, 4H), 1.86-1.52 (m, 5H), 0.94-0.70 (m, 5H), 0.44-0.08 (m, 5H).

**Example 46: Preparation of (S)-4-ethyl-8-fluoro-4-hydroxy-11-(3-hydroxypropyl)-1,12-dihydro-14H-pyrano [3,4:6,7]indolizino[2,1 -b]quinoline-3,6,14(4H,11H)-trione:**

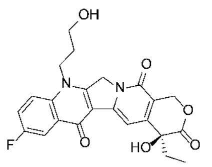

Starting from Intermediate 27 and **3-amino-1-propanol,** the title compound was synthesized following the synthetic procedure of Example 45. Purification was performed by Prep-HPLC (C18, 0.1% aqueous HCl, acetonitrile) to afford the title compound as a yellow solid (4.26 mg, 0.01 mmol, yield: 6.82%). LCMS (ESI) $[M+H]^+$ = 441.3; $^1$H NMR (400 MHz, DMSO-$d_6$+$D_2O$) δ 8.06 (dd, J = 9.5, 4.3 Hz, 1H), 7.99 (dd, J = 8.8, 3.0 Hz, 1H), 7.78-7.73 (m, 1H), 7.28 (s, 1H), 5.44 (s, 2H), 5.33 (dd, J = 24.9, 15.8 Hz, 2H), 4.44 (t, J = 7.0 Hz, 2H), 3.49 (d, J = 5.2 Hz, 2H), 2.03-1.92 (m, 2H), 1.87-1.74 (m, 2H), 0.85 (t, J = 7.3 Hz, 3H).

**Example 47: Preparation of (S)-4-ethyl-8-fluoro-4-hydroxy-11-((1r,4S)-4-hydroxycyclohexyl)-1H-pyrano[3,4:6,7] indolizino[2,1 -b]quinoline-3,6,14(4H, 11H,12H)-trione:**

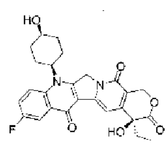

Using Intermediate 27 from Preparation Example 27 and the commercial reagent trans-4-aminocyclohexanol as the starting materials, the synthesis was carried out following steps 1-2 of Preparation Example 6 to afford 6-fluoro-1-((1S,4S)-4-hydroxycyclohexyl)-2-methylquinolin-4(1H)-one as an intermediate. Subsequent to this, two bromination, alkylation and cyclization reactions were performed in accordance with steps 2-5 of Example 8 to yield the title compound, which was obtained as a yellow solid. $^1$H-NMR (400 MHz, DMSO-$d_6$) δ 8.35-8.24 (m, 1H), 8.02-7.95 (m, 1H), 7.66-7.61 (m,

1H), 7.30 (s, 1H), 5.58 (s, 2H), 5.35 (m, 2H), 4.31-4.24 (m, 1H), 3.75 (t, J = 10.6 Hz, 1H), 2.61-2.52 (m, 2H), 2.08-1.97 (m, 4H), 1.87-1.75 (m, 2H), 1.60-1.52 (m, 2H), 0.86 (t, 3H).

**Example 48: Preparation of (S)-4-ethyl-8-fluoro-4-hydroxy-11-((1r,4R)-4-hydroxycyclohexyl)-1H-pyrano[3,4:6,7]indolizino[2,1 -b]quinoline-3,6,14(4H, 11H,12H)-rione**

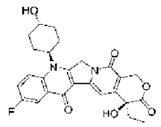

Using intermediate 27 and commercial reagent cis-4-aminocyclohexanol as starting materials, the intermediate 6-fluoro-1-((1r,4r)-4-hydroxycyclohexyl)-2-methylquinolin-4(1H)-one was synthesized according to related steps 1-2 in Preparation Example 6. LCMS (ESI) $[M+H]^+$=481.4; 1H NMR (400 MHz, DMSO-d6) δ 8.34-8.31 (m, 1H), 8.04-8.01 (m, 1H), 7.81-7.76 (m, 1H), 7.30 (s, 1H), 6.34 (s, 1H), 5.59-5.51 (m, 2H), 5.40-5.30 (m, 2H), 4.44-4.37 (m, 1H), 3.98 (s, 1H), 2.79-2.67 (m, 2H), 1.87-1.72 (m, 7H), 1.29-1.20 (m, 1H), 0.86 (t, J = 7.3 Hz, 3H).

**Example 49: Preparation of (S)-4-ethyl-8-fluoro-4-hydroxy-11-((1s,4R)-4-hydroxycyclohexyl)-9-methyl-1,12-dihydro-14H-pyrano[3,4:6,7]indolizino[2,1 -b]quinoline-3,6,14(4H,11H)-trione:**

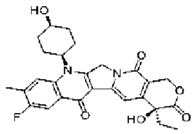

Similarly, using Intermediate 29 and the commercially available reagent cis-4-aminocyclohexanol as the starting materials, the synthesis was carried out following steps 1-2 of Preparation Example 6 to afford the intermediate 6-fluoro-1-((1r,4r)-4-hydroxycyclohexyl)-2,7-dimethylquinolin-4(1H)-one. Subsequently, the intermediate underwent two rounds of bromination, alkylation and cyclization by referring to Steps 2-5 of Example 8. Purification was performed via preparative high-performance liquid to obtain the title compound as pale yellow solid (7.0 mg, yield: 4.04%). LCMS (ESI): $[M+H]^+$ = 495.3, $^1$H NMR (400 MHz, DMSO-$d_6$) δ: 8.91 (s, 1H), 8.28 (d, J = 5.6 Hz, 1H), 8.03 (s, 1H), 7.95 (d, J = 10.0 Hz, 1H), 7.29 (s, 1H), 5.51 (s, 2H), 5.35 (dd, J = 22.4, 16.0 Hz, 2H), 4.00 (s, 1H), 2.82-2.74 (m, 1H), 2.45 (s, 3H), 1.88-1.70 (m, 8H), 1.53 (d, J = 7.2 Hz, 1H), 1.35 (d, J = 7.2 Hz, 1H), 0.86 (t, J = 7.2 Hz, 3H).

**Example 50: Preparation of (S)-9-cyclopropyl-4-ethyl-8-fluoro-4-hydroxy-11-((1s,4R)-4-hydroxycyclohexyl)-1,12-dihydro-14H-pyrano[3,4:6,7]indolizino[2,1 -b]quinoline-3,6,14(4H,11H)-trione:**

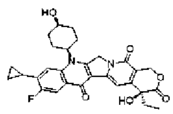

Similarly, using intermediate 4 and the commercially available reagent cis-4-aminocyclohexanol as the starting materials, the synthesis was performed following steps 1-2 of Preparation Example 6 to afford the intermediate 7-bromo-6-fluoro-1-((1r,4r)-4-hydroxycyclohexyl)-2-methylquinolin-4(1H)-one. Subsequently, the bromo group in the intermediate was converted to a cyclopropyl group via a coupling reaction, yielding the intermediate 7-cyclopropyl-6-fluoro-1-((1r,4r)-4-hydroxycyclohexyl)-2-methylquinolin-4(1H)-one. Thereafter, the title compound was obtained as a yellow solid by conducting two rounds of bromination, alkylation and cyclization in accordance with steps 2-5 of Example 8. LCMS (ESI): $[M+H]^+$ = 521.3. $^1$H NMR (400 MHz, DMSO-$d_6$+$D_2O$) δ: 7.91-7.87 (m, 1H), 7.89-7.80 (m, 1H), 7.26 (s, 1H), 5.48-5.39 (m, 2H), 5.32-5.24 (m, 2H), 4.34-4.25 (m, 1H), 2.68-2.64 (m, 1H), 1.86-1.79 (m, 5H), 1.69-1.63 (m, 1H), 1.51 (d, J = 6.9 Hz, 2H), 1.38-1.33 (m, 3H), 1.15-1.13 (m, 2H), 1.01-0.96 (m, 2H), 0.82 (t, J = 7.3 Hz, 3H).

**Example 51: Preparation of (4S)-4-ethyl-8-fluoro-4-hydroxy-11-((1s,4R)-4-(((tetrahydro-2H-pyran-2-yl)oxy) methyl)cyclohexyl)-1,12-dihydro-14H-pyrano [3,4:6,7] indolizino[2,1-b]quinoline-3,6,14(4H,11H)-trione:**

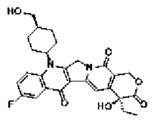

Similarly, using Intermediate 27 and the commercially available reagent cis-4-aminocyclohexylmethanol as the starting materials, the synthesis was carried out following Steps 1-2 of Preparation Example 6 to afford the intermediate 6-fluoro-1-((1s,4s)-4-(hydroxymethyl)cyclohexyl)-2-methylquinolin-4(1H)-one. Subsequently, the intermediate underwent two rounds of bromination, alkylation and cyclization by referring to steps 2-5 of Example 8. Purification was performed via preparative high-performance liquid chromatography (Prep-HPLC) to obtain the title compound(4.2mg, yield: 28%).LCMS (ESI): $[M+H]^+$ = 495.3, $^1$H NMR (400 MHz, DMSO-$d_6$+$D_2O$) δ: 8.40-8.33 (m, 1H), 8.00-7.98 (m, 1H), 7.76-7.64 (m, 1H), 7.33 (s, 1H), 5.59-5.49 (m, 1H), 5.33-5.28 (m, 2H), 4.29-4.17 (m, 1H), 3.33-3.29 (m, 2H), 2.20-2.04 (m, 3H), 1.98-1.75 (m, 5H), 1.68-1.67 (m, 1H), 1.61-1.52 (m, 1H), 1.30-1.20 (m, 2H), 0.85 (t, J = 7.1 Hz, 3H).

**Example 52: Preparation of (S)-4-ethyl-8-fluoro-4-hydroxy-11-((1S,2S)-2-hydroxycyclopentyl)-1,12-dihydro-14H-pyrano[3,4:6,7]indolizino[2,1 -b] quinoline-3,6,14(4H,11H)-trione:**

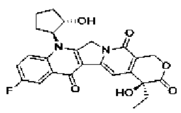

Similarly, using Intermediate 27 and reagent (1S,2S)-2-aminocyclopentan-1-ol as the starting materials, the synthesis was conducted following steps 1-2 of Preparation Example 6 to afford the intermediate 6-fluoro-1-((1S,2S)-2-hydroxycyclopentyl)-2-methylquinolin-4(1H)-one. Subsequently, the intermediate was subjected to two rounds of bromination, alkylation and cyclization according to steps 2-5 of Example 8. Purification was performed via preparative high-performance liquid chromatography (Prep-HPLC) to obtain the title compound as a yellow solid (1.95 mg, yield: 8.36%). LCMS (ESI): $[M+H]^+$ = 467.3. $^1$H NMR (400 MHz, DMSO-$d_6$) δ: 8.03-7.97 (m, 1H), 7.82-7.75 (m, 1H), 7.71-7.64 (m, 1H), 7.26 (s, 1H), 6.31 (s, 1H), 5.62 (d, J = 20.1 Hz, 1H), 5.38-5.19 (m, 4H), 4.82-4.60 (m, 2H), 2.39-2.29 (m, 1H), 2.25-2.11 (m, 2H), 2.06-1.86 (m, 2H), 1.82-7.75 (m, 2H), 1.73-1.62 (m, 1H), 0.82 (t, J = 7.3 Hz, 3H).

**Example 53: Preparation of (S)-4-ethyl-8-fluoro-4-hydroxy-11-((1R,2R)-2-(hydroxymethyl)cyclopentyl)-1,12-dihydro-14H-pyrano[3,4:6,7]indolizino[2,1 -b]quinoline-3,6,14(4H,11H)-trione:**

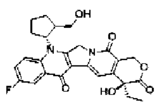

Similarly, using Intermediate 27 and the commercially available reagent ((1R,2R)-2-aminocyclopentyl)methanol as the starting materials, the synthesis was carried out following steps 1-2 of Preparation Example 6 to afford the intermediate 6-fluoro-1-((1R,2R)-2-(hydroxymethyl)cyclopentyl)-2-methylquinolin-4(1H)-one. Subsequently, the intermediate underwent two rounds of bromination, alkylation and cyclization by referring to steps 2-5 of Example 8. Purification was performed via preparative high-performance liquid chromatography (Prep-HPLC) to obtain the title compound as a yellow solid (1.78 mg, yield: 8%). LCMS (ESI): $[M+H]^+$ = 481.3, $^1$H NMR (400 MHz, DMSO-$d_6$) δ: 8.05-8.02 (m, 1H), 7.90-7.88 (m, 1H), 7.75-7.70 (m, 1H), 7.30 (s, 1H), 6.35 (s, 1H), 5.62-5.57 (m, 1H), 5.44-5.28 (m, 3H), 4.78-4.72 (m, 1H), 3.53-3.42 (m, 2H), 2.83-2.81 (m, 1H), 2.36-2.30 (m, 2H), 2.14-1.98 (m, 3H), 1.83-1.76 (m, 3H), 1.63-1.56 (m, 1H), 0.85 (t, J = 7.1 Hz, 3H).

**Example 54: Preparation of (S)-4-ethyl-8-fluoro-4-hydroxy-11-((1S,2S)-2-(hydroxymethyl)cyclopentyl)-1,12-dihydro-14H-pyrano[3,4:6,7]indolizino[2,1 -b]quinoline-3,6,14(4H,11H)-trione**

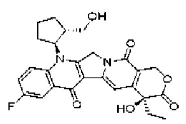

Similarly, using Intermediate 27 nd the commercially available reagent ((1S,2S)-2-aminocyclopentyl)methanol as the starting materials, the synthesis was carried out following steps 1-2 of Preparation Example 6 to afford the intermediate 6-fluoro-1-((1S,2S)-2-(hydroxymethyl)cyclopentyl)-2-methylquinolin-4(1H)-one. Subsequently, the intermediate underwent two rounds of bromination, alkylation and cyclization by referring to Steps 2-5 of Example 8. Purification was performed via preparative high-performance liquid chromatography (Prep-HPLC) to obtain the title compound as a yellow solid. LCMS (ESI): $[M+H]^+$ = 481.3.

Example 55: Preparation of Methyl (S)-4-(8-fluoro-4-hydroxy-4-methyl-3,6,14-trioxo-3,4-dihydro-1H-pyrano[3,4:6,7]indolizino[2,1 -b]quinolin-11(6H,12H,14H) -yl)-1-methyl-1H-pyrazole-5-carboxylate:

*Step 1: Preparation of Methyl (Z)-4-((4-(2,5-difluorophenyl)-4-oxobut-2-en-2-yl)amino)-1-methyl-1H-pyrazole-5-carboxylate:*

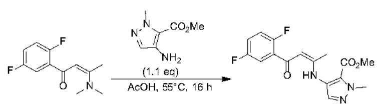


Intermediate 27 of Preparation Example 27 (5.30 g, 34.19 mmol, 1.0 equiv.), was dissolved in glacial acetic acid (55 mL). Then 4-amino-1-methyl-1H-pyrazole-5-carboxylate was added (dosage to be supplemented according to eq.alent ratio). The reaction progress was monitored by LCMS until the starting material was completely consumed. After the reaction was completed, the reaction mixture was spin-dried. The residue was separated and purified by silica gel column chromatography (200-300 mesh silica gel, eluent: petroleum ether (PE) : ethyl acetate (EA) = 0%~50% gradient elution) to obtain the title compound of this step as a yellow solid (9.00 g, yield: 86.4%).LCMS (ESI): $[M+H]^+$ = 336.1.

***Step 2: Preparation of Methyl 4-(6-fluoro-2-methyl-4-oxoquinolin-1(4H)-yl)-1-methyl-1H-pyrazole-5-carboxylate:***

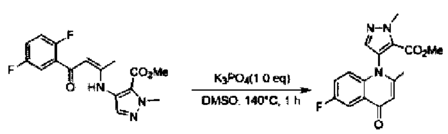


The intermediate obtained from step 1 (5.00 g, 14.91 mmol, 1.0 eq.) was dissolved in DMSO (20 mL). Potassium phosphate (3.16 g, 14.91 mmol, 1.0 eq.) was added, and the mixture was reacted at 140°C for 1 hour. The reaction was confirmed complete by LCMS. After cooling down, the reaction mixture was poured into water (50 mL), extracted with ethyl acetate (3×50 mL), washed with saturated sodium chloride solution (50 mL), and dried over anhydrous sodium sulfate. The organic phase was spin-dried, and the residue was separated and purified by silica gel column chromatography (200-300 mesh silica gel, eluent: PE:EA = 10%~80% gradient elution) to obtain the title compound as a pale yellow oil (2.20 g, yield: 46.8%).LCMS (ESI): $[M+H]^+$ = 316.1.

***Step 3: Preparation of Methyl 4-(3-bromo-2-(bromomethyl)-6-fluoro-4-oxoquinolin-1(4H)-yl)-1-methyl-1H-pyrazole-5-carboxylate:***

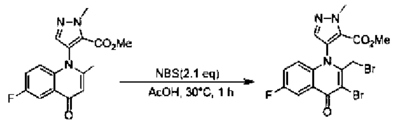


The intermediate obtained in step 2 (1.00 g, 3.17 mmol, 1.0 equiv.) was dissolved in anhydrous glacial acetic acid (10.0 mL). N-bromosuccinimide (NBS) (1.18 g, 6.66 mmol, 2.1 eq.) was added, and the mixture was reacted at 30°C for 1 hour. The reaction was confirmed complete by LCMS. After cooling down, the reaction mixture was poured into water (20 mL), extracted with ethyl acetate (3 × 20 mL), washed with saturated sodium chloride solution (20 mL), and dried over anhydrous sodium sulfate. The organic phase was spin-dried, and the residue was separated and purified by silica gel column chromatography (200-300 mesh silica gel, eluent: PE : EA = 10%~50% gradient elution) to obtain the title compound as a pale yellow solid (1.00 g, yield: 66.7%).LCMS (ESI): $[M+H]^+$ = 474.2.

***Step 4: Preparation of Methyl (S)-4-(3-bromo-2-((4-ethyl-4-hydroxy-3,8-dioxo-3,4-dihydro-1H-pyrano[3,4-c]pyridin-7(8H)-yl)methyl)-6-fluoro-4-oxoquinolin-1(4H)-yl)-1-methyl-1H-pyrazole-5-carboxylate:***

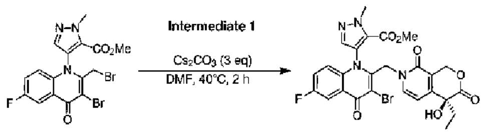


The intermediate obtained in step 3 (300 mg, 0.63 mmol, 1.0 eq.) and Intermediate 1 (145.9 mg, 0.70 mmol, 1.1 eq.) were dissolved in N,N-dimethylformamide (DMF) (3 mL). Potassium carbonate (261.2 mg, 1.89 mmol, 3.0 eq.) was added, and the mixture was reacted at 40°C for 2 hours. The reaction was confirmed by LCMS. After the reaction solution was cooled to room temperature, it was poured into water (10 mL), extracted with ethyl acetate (3 × 10 mL), and washed with saturated sodium chloride solution (10 mL). The residue purified by silica gel column chromatography (200-300 mesh silica gel, eluent: DCM: MeOH= 0%~3% gradient elution) to obtain the title compound as a pale yellow solid (200 mg, yield: 52.8%).LCMS (ESI): $[M+H]^+$ = 602.1.

***Step 5: Preparation of Methyl (S)-4-(8-fluoro-4-hydroxy-4-methyl-3,6,14-trioxo-3,4-dihydro-1H-pyrano[3,4:6,7] indolizino[2,1 -b]quinolin-11(6H,12H,14H)-yl)-1-methyl-1H-pyrazole-5-carboxylate:***

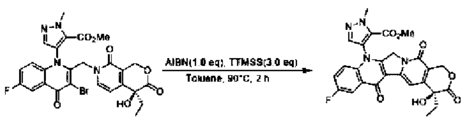


Under nitrogen protection, the product obtained in Step 4 (200 mg, 0.33 mmol, 1.0 equiv.) was dissolved in dry toluene (2 mL). AIBN (54.61 mg, 0.33 mmol, 1.0 eq.) and tris(trimethylsilyl)silane (TTMSS) (248.09 mg, 1.00 mmol, 3.0 eq.) were added. The reaction system was bubbled with $N_2$ for 1 minute, then stirred at 90°C for 2 hours. The reaction was confirmed complete by LCMS. The reaction mixture was spin-dried, dissolved in DMSO (3 mL), and 1 drop of 1 M hydrochloric acid (HCl) was added. The mixture was purified by preparative HPLC (C18 column, mobile phase: 0.1% aqueous HCl solution and acetonitrile) and lyophilized to obtain the title compound as an off-white solid (20 mg, yield: 11.6%). LCMS (ESI): $[M+H]^+$ = 521.3.$^1$H NMR (400 MHz, DMSO-$d_6$) $\delta$: 8.02-7.99 (m, 2H), 7.64-7.59 (m, 1H), 7.35-7.31 (m, 2H), 6.40 (s, 1H), 5.32 (s, 2H), 5.10-4.91 (m, 2H), 4.25 (s, 3H), 3.49 (s, 3H), 1.85-1.79 (m, 2H), 0.87 (t, J = 7.6 Hz, 3H).

**Example 56: Preparation of Methyl (S)-4-(4-ethyl-8-fluoro-4-hydroxy-3,6,14-trioxo-3,4-dihydro-1H-pyrano [3,4:6,7]indolizino[2,1 -b]quinolin-11(6H,12H,14H)-yl)-1-methyl-1H-pyrazole-3-carboxylate:**

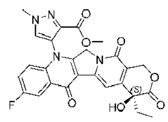

Using Intermediate 27 and methyl 1-methyl-4-amino-1H-pyrazole-3-carboxylate as the starting materials, the title compound was synthesized by following steps 1-5 of Example 55. The product was obtained as a pale yellow solid. LCMS (ESI): $[M+H]^+$ = 521.3, $^1H$ NMR (400 MHz, DMSO-$d_6$) δ: 8.39 (s, 1H), 7.96 (dd, J = 8.8, 3.0 Hz, 1H), 7.60-7.55 (m, 1H), 7.28-7.24 (m, 2H), 6.37 (s, 1H), 5.28 (s, 2H), 4.99-4.85 (m, 2H), 4.07 (s, 3H), 3.52 (s, 3H), 1.83-1.73 (m, 2H), 0.83 (t, J = 7.3 Hz, 3H).

**Example 57: Preparation of (S)-4-ethyl-8-fluoro-4-hydroxy-11-(1-methyl-1H-pyrazol-4-yl)-1H-pyrano[3',4':6,7] indolizino[2,1-b]quinoline-3,6,14(4H,11H,12H) -trione:**

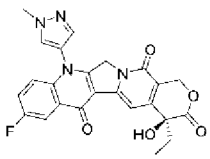

Using Intermediate 27 and the commercially available reagent 1-methyl-1H-pyrazol-4-amine as the starting materials, the synthesis was carried out by following steps 1-5 of Example 55, and the reaction was confirmed complete by LCMS.The reaction mixture was spin-dried, and the resulting solid was dissolved in DMSO (3 mL). 1 drop of 1 M HCl was added, and the mixture was purified by preparative HPLC (C18 column, mobile phase: 0.1% aqueous HCl solution and acetonitrile) and lyophilized to obtain the title compound as a yellow solid (2.9 mg, yield: 8.18%). LCMS (ESI): $[M+H]^+$ = 463.3. $^1H$ NMR (400 MHz, DMSO-$d_6$+$D_2O$) δ: 8.22 (s, 1H), 7.96-7.84 (m, 2H), 7.65-7.60 (m, 1H), 7.37-7.27 (m, 2H), 5.33-5.29 (m, 2H), 4.95-4.84 (m, 2H), 3.99-3.82 (m, 3H), 1.84-1.76 (m, 2H), 0.84 (t, J = 7.0 Hz, 3H).

**Example 58: Preparation of (S)-4-ethyl-8-fluoro-4-hydroxy-11-(3-(hydroxymethyl)-1-methyl-1H-pyrazol-4-yl)-1H-pyrano[3',4':6,7]indolizino[2,1-b]quinoline-3,6,14(4H,11H,12H)-trione:**

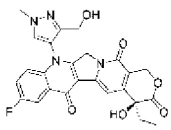

Using Intermediate 27 and 4-amino-1-methyl-1H-pyrazole-3-MeOH as the starting materials, the title compound was synthesized by following Steps 1-5 of Example 55. The product was obtained as a white solid.LCMS (ESI): $[M+H]^+$ = 493.2. $^1H$ NMR (400 MHz, DMSO-$d_6$) δ: 8.13 (d, J = 8.2 Hz, 1H), 7.98-7.94 (m, 1H), 7.62-7.57 (m, 1H), 7.29-7.26 (m, 2H), 5.33-5.22 (m, 2H), 4.93-4.88 (m, 2H), 4.40-4.28 (m, 1H), 4.12-4.10 (m, 1H), 3.94 (s, 3H), 1.83-1.74 (m, 2H), 0.83 (t, J = 7.3 Hz, 3H).

Example 59: Preparation of (S)-4-ethyl-8-fluoro-4-hydroxy-11-(5-(hydroxymethyl)-1-methyl-1H-pyrazol-4-yl)-1H-pyrano[3,4:6,7]indolizino[2,1 -b]quinoline-3,6,14(4H,11H,12H)-trione:

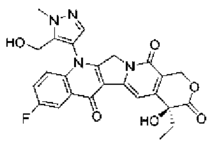

Using Intermediate 27 and 4-amino-1-methyl-1H-pyrazole-5-MeOH as the starting materials, the title compound

was synthesized by following Steps 1-5 of Example 55. The product was obtained as pale yellow solid. LCMS (ESI): $[M+H]^+$ = 493.3. $^1H$ NMR (400 MHz, DMSO-$d_6$) δ: 8.00 (dd, J = 8.8, 3.0 Hz, 1H), 7.76 (s, 1H), 7.67-7.62 (m, 1H), 7.31-7.28 (m, 2H), 5.36 (t, J = 5.5 Hz, 1H), 5.32 (s, 2H), 4.98-4.87 (m, 2H), 4.46 (dd, J = 13.9, 5.6 Hz, 1H), 4.31 (dd, J = 13.9, 5.4 Hz, 1H), 3.99 (s, 3H), 1.87-1.77 (m, 2H), 0.86 (t, J = 7.1 Hz, 3H).

**Example 60: Preparation of (S)-4-ethyl-8-fluoro-4-hydroxy-11-(pyridin-3-yl)-1H-pyrano[3',4':6,7]indolizino[2,1-b]quinoline-3,6,14(4H,11H,12H)-trione:**

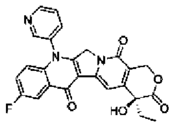

Using Intermediate 27 and the commercially available reagent pyridin-3-amine as the starting materials, the synthesis was carried out by following Steps 1-5 of Example 55. The title compound was obtained as a white solid.LCMS (ESI): $[M+H]^+$ = 460.2. $^1H$ NMR (400 MHz, DMSO-$d_6$) δ: 8.93-8.88 (m, 2H), 8.19 (dd, J = 14.4, 8.4 Hz, 1H), 8.01 (dd, J = 8.8, 3.0 Hz, 1H), 7.82 (t, J = 5.9 Hz, 1H), 7.61-7.56 (m, 1H), 7.29 (s, 1H), 7.12-6.99 (m, 1H), 5.28 (s, 2H), 4.92-4.77 (m, 2H), 1.83-1.72 (m, 2H), 0.82 (t, J = 7.1 Hz, 3H).

Example 61: Preparation of (S)-4-ethyl-8-fluoro-4-hydroxy-11-(2-(hydroxymethyl)-5-methoxyphenyl)-1,12-dihydro-14H-pyrano[3,4:6,7] indolizino[2,1-b]quinoline-3,6,14(4H,11H)-trione:

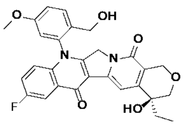

Using Intermediate 27 and (2-amino-4-methoxyphenyl)MeOH as the starting materials, the title compound was synthesized by following Steps 1-5 of Example 55. LCMS (ESI): $[M+H]^+$ = 519.3

**Example 62: Preparation of (S)-11-(1-cyclopropyl-3-(hydroxymethyl)-1H-pyrazol-4-yl)-4-ethyl-8-fluoro-4-hydroxy-1,12-dihydro-14H-pyrano[3,4:6,7] indolizino[2,1-b]quinoline-3,6,14(4H,11H)-trione:**

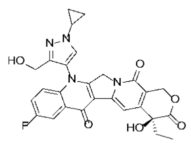

***Step 1: Preparation of methyl 1-cyclopropyl-4-nitro-1H-pyrazole-3-carboxylate:***

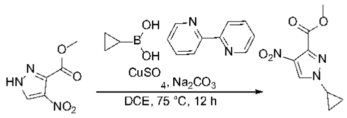


Methyl 4-nitro-1H-pyrazole-3-carboxylate (25.0 g, 146.1 mmol, 1.0 eq.) was added to 1,2-dichloroethane.

Cyclopropylboronic acid (25.1 g, 292.21 mmol, 2.0 eq.), 2,2'-bipyridine (22.8 g, 146.1 mmol, 1.0 eq.), and sodium carbonate (30.9 g, 292.2 mmol, 2.0 eq.) were added sequentially to the reaction system. The reaction mixture was heated to 75 °C and stirred for 16 hours, and the reaction completion was monitored by LCMS. After the reaction, the mixture was

filtered, and the filter cake was washed with DCM (100 mL × 3). The combined filtrate and washing solutions were concentrated under reduced pressure to dryness to obtain the title compound as a yellow solid (10.0 g, yield: 34.72%). LCMS (ESI): $[M + H]^+$ = 212.

***Step 2: Preparation of Methyl 4-amino-1-cyclopropyl-1H-pyrazole-3-carboxylate:***

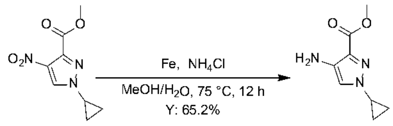


The intermediate obtained from step 1 (10.0 g, 47.36 mmol, 1.0 eq.) was dissolved in a mixed solvent of MeOH (100 mL) and water (10 mL). Iron powder (13.2 g, 236.8 mmol, 5.0 eq.) and ammonium chloride (25.3 g, 473.6 mmol, 10.0 eq.) were added to the solution successively. The reaction mixture was heated to 75 °C and stirred After the reaction was completed, the mixture was filtered, and the filter cake was washed with MeOH (200 mL × 3). The combined filtrate and washing solutions were concentrated to obtain a crude product. The crude product was purified by flash silica gel chromatography (eluent gradient: PE:EA = 10:1 to 5:1, v/v) to afford the title compound as a light red oil (5.6 g, yield: 65.2%). LCMS (ESI): $[M+H]^+$ = 182.

***Step 3: Preparation of Methyl (Z)-1-cyclopropyl-4-((4-(2,5-difluorophenyl)-4-oxobut-2-en-2-yl)amino)-1H-pyrazole-3-carboxylate:***

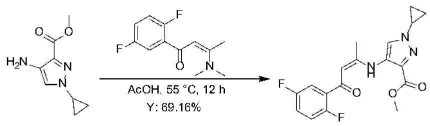


The intermediate obtained from step 2 (5.8 g, 32.01 mmol, 1.0 eq.) was added to acetic acid (100 mL). Then the intermediate from Preparation Example 27, (Z)-1-(2,5-difluorophenyl)-3-(dimethylamino)but-2-en-1-one (6.02 g, 26.64 mmol, 0.8 eq.), was added to the solution. The reaction mixture was heated to 55 °C and stirred for 12 hours, After the reaction was completed, the mixture was poured into ice water (500 mL). The resulting mixture was extracted with ethyl acetate (300 mL × 3). The combined organic phases were dried over anhydrous sodium sulfate, filtered, and concentrated under reduced pressure to obtain a crude product. The crude product was separated and purified by flash silica gel chromatography (eluent gradient: petroleum ether:ethyl acetate = 10:1 to 5:1, v/v) to afford the title compound as a yellow solid (8.0 g, yield: 69.16%). LCMS (ESI): $[M + H]^+$ = 362.0.

***Step 4: Preparation of Methyl 1-cyclopropyl-4-(6-fluoro-2-methyl-4-oxoquinolin-1(4H)-yl)-1H-pyrazole-3-carboxylate:***

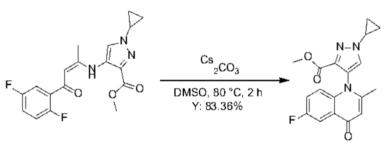


The intermediate obtained from step 3 (8.0 g, 22.14 mmol, 1.0 eq.) was dissolved in dimethyl sulfoxide (DMSO, 50 mL). Cesium carbonate ($Cs_2CO_3$, 14.4 g, 44.28 mmol, 2.0 eq.) was added to the solution, and the reaction mixture was heated to 80 °C and stirred for 2 hours. The reaction solution was cooled to room temperature, then poured into saturated ammonium chloride aqueous solution (250 mL). The resulting mixture was extracted with ethyl acetate (400 mL × 3). The combined organic phases were dried over anhydrous sodium sulfate, filtered, and concentrated under reduced pressure to obtain a crude product. The crude product was separated and purified by flash silica gel chromatography (eluent: petroleum ether:ethyl acetate = 5:1, v/v) to afford the title compound as a yellow solid (6.3 g, yield: 83.36%). LCMS (ESI):

$[M + H]^+$ = 342.0

***Step 5: Preparation of 1-(1-cyclopropyl-3-(hydroxymethyl)-1H-pyrazol-4-yl)-6-fluoro-2-methylquinolin-4(1H)-one:***

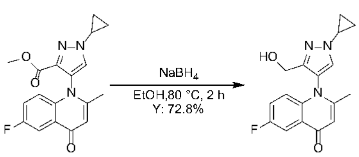


The intermediate obtained from step 4 (5.6 g, 13.58 mmol, 1.0 eq.) was dissolved in ethanol (100 mL). Sodium borohydride (1.5 g, 40.73 mmol, 3.0 eq.) was added to the solution at room temperature. The reaction mixture was heated to 80 °C and stirred for 2 hours, The reaction solution was cooled to 20 °C, then quenched by adding water (100 mL). The resulting mixture was extracted with ethyl acetate (200 mL × 3). The combined organic phases were dried over anhydrous sodium sulfate, filtered, and concentrated to obtain a crude product. The crude product was purified by flash silica gel chromatography (eluent gradient: petroleum ether:ethyl acetate = 10:1 to 3:1, v/v) to afford the title compound as a yellow solid (3.8 g, yield: 72.8%).LCMS (ESI): $[M + H]^+$ = 314.

The intermediate obtained from step 5 was subjected to two cycles of bromination, alkylation and cyclization by following the procedures of steps 2-5 in Example 8. The resulting solid was concentrated, then further purified by preparative high-performance liquid chromatography (Prep-HPLC, stationary phase: C18, mobile phase: 0.1% aqueous hydrochloric acid solution and acetonitrile) to afford the title compound. LCMS (ESI): $[M + H]^+$ = 519, $^1$H NMR (400 MHz, DMSO-$d_6$+$D_2O$): δ 8.25 (s, 1H), 7.98 (d, J = 8.8 Hz, 1H), 7.64-7.59 (m, 1H), 7.29-7.25 (m, 2H), 5.35-5.34 (m, 2H), 5.03-4.88 (m, 2H), 4.36 (d, J = 12.9 Hz, 1H), 4.15 (d, J = 13.2 Hz, 1H), 3.88-3.83 (m, 1H), 1.83-1.75 (m, 2H), 1.18-1.12 (m, 2H), 1.06-1.02 (m, 2H), 0.85 (t, J = 7.3 Hz, 3H).

**Example 63: Preparation of (4S)-11-(1-cyclopropyl-3-(1-hydroxyethyl)-1H-pyrazol-4-yl)-4-ethyl-8-fluoro-4-hydroxy-1,12-dihydro-14H-pyrano[3,4:6,7] indolizino[2,1-b]quinoline-3,6,14(4H,11H)-trione:**

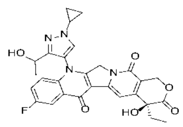

***Step 1: Preparation of 1-cyclopropyl-4-(6-fluoro-2-methyl-4-oxoquinolin-1(4H)-yl)-1H-pyrazole-3-carbaldehyde:***

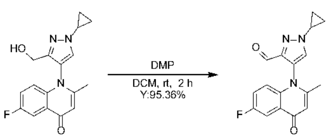


The intermediate obtained from step 5 of Example 62 (200 mg, 0.64 mmol, 1.0 eq.) was dissolved in DCM (5 mL). Dess-Martin periodinane (325.74 mg, 0.77 mmol, 1.2 eq.) was added to the solution, and the mixture was stirred at room temperature for 2 hours. The reaction mixture was filtered, and the filtrate was concentrated under reduced pressure. The crude product was purified by preparative thin-layer chromatography (TLC plate, developing solvent: DCM:MeOH = 20:1, v/v) to afford the title compound as a white solid (190 mg, yield: 95.36%). LCMS (ESI): $[M + H]^+$ = 312.3

***Step 2: Preparation of 1-(1-cyclopropyl-3-(1-hydroxyethyl)-1H-pyrazol-4-yl)-6-fluoro-2-methylquinolin-4(1H)-one:***

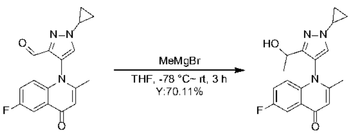


The intermediate obtained from Step 1 (190 mg, 0.61 mmol, 1.0 eq.) was added to tetrahydrofuran (10 mL). The reaction flask was purged with nitrogen for three times, then cooled down to -78 °C. Methylmagnesium bromide (1.02 mL, 3.05 mmol, 5.0 eq.) was added dropwise to the solution. The mixture was allowed to warm up to room temperature naturally and stirred for 3 hours, with the reaction completion confirmed by LCMS monitoring. MeOH (2 mL) was added to the reaction mixture, and the resulting solution was stirred for 5 min. Then 4 M dioxane hydrochloride solution (1 mL) was added dropwise. The mixture was filtered, and the filtrate was concentrated. The crude product was purified by preparative thin-layer chromatography (TLC plate, developing solvent: DCM:MeOH = 20:1, v/v) to afford the title compound as a white solid (140 mg, yield: 70.11%). LCMS (ESI): $[M + H]^+$ = 328.3. The intermediate obtained from Step 2 was subjected to two cycles of bromination, alkylation and cyclization by following the procedures of Steps 2-5 in Example 8. The resulting solid was concentrated, then further purified by preparative high-performance liquid chromatography (Prep-HPLC, stationary phase: C18, mobile phase: 0.1% aqueous HCl solution and acetonitrile) to afford the title compound as a yellow solid (3 mg, yield: 8.05%).LCMS (ESI): $[M + H]^+$ = 533.3. $^1$H NMR (400 MHz, DMSO-$d_6$): δ 8.21 (dd, J = 13.6, 6.5 Hz, 1H), 7.97 (dt, J = 8.8, 2.6 Hz, 1H), 7.65-7.59 (m, 1H), 7.31-7.23 (m, 2H), 5.34-5.20 (m, 2H), 5.06-4.89 (m, 2H), 4.76-4.71 (m, 0.5H), 4.41-4.34 (m, 0.5H), 3.89-3.80 (m, 1H), 2.53 (s, 1H), 1.87-1.74 (m, 2H), 1.36-1.30 (m, 2H), 1.21-1.09 (m, 2H), 1.07-1.00 (m, 3H), 0.85 (t, J = 7.3 Hz, 3H).

**Example 64: Preparation of (S)-11-(1-cyclopropyl-1H-pyrazol-4-yl)-4-ethyl-8-fluoro-4-hydroxy-1H-pyrano [3,4:6,7]indolizino[2,1 -b]quinoline-3,6,14(4H,11H,12H)-trione:**

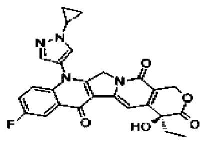

Using the intermediate from Preparation Example 27, (Z)-1-(2,5-difluorophenyl)-3-(dimethylamino)but-2-en-1-one, and the commercial reagent 1-cyclopropyl-1H-pyrazol-4-amine as the starting materials, the title compound was synthesized following the preparation method of Example 57. The product was obtained as a yellow solid. LCMS (ESI): $[M+H]^+$=489.4 , $^1$H NMR (400 MHz, DMSO-$d_6$): δ 8.33 (s, 1H), 7.92 (d, J=6.6 Hz, 1H), 7.84 (s, 1H), 7.62-7.57 (m, 1H), 7.32 (q, J=4.5 Hz, 1H), 7.25 (s, 1H), 6.40 (s, 1H), 5.30 (s, 2H), 4.95-4.82 (m, 2H), 3.92-3.88 (m, 1H), 1.78 (q, J=7.4 Hz, 2H), 1.18 (t, J=1.5 Hz, 2H), 1.06 (q, J=6.4 Hz, 2H), 0.83 (t, J=7.3 Hz, 3H).

**Example 65: Preparation of (S)-4-ethyl-8-fluoro-4-hydroxy-11-(pyridin-2-ylmethyl)-1,12-dihydro-14H-pyrano [3,4:6,7]indolizino[2,1 -b]quinoline-3,6,14 (4H,11H)-trione:**

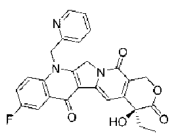

Using the intermediate from Preparation Example 27, **(Z)-1-(2,5-difluorophenyl)-3-(dimethylamino)but-2-en-1-one,** and **2-(aminomethyl)pyridine** as the starting materials, the title compound was synthesized following steps 1-5 of Example 55. LCMS (ESI): $[M+H]^+$=474.2 , $^1$H NMR (400 MHz, DMSO-$d_6$): δ 8.45 (d, J=3.8 Hz, 1H), 7.99-7.94 (m, 2H), 7.86 (d, J=7.1 Hz, 1H), 7.67 (d, J=7.7 Hz, 2H), 7.35-7.31 (m, 2H), 5.86 (s, 2H), 5.46 (s, 2H), 5.34 (d, J=3.0 Hz, 2H), 1.83 (t,

J=5.9 Hz, 2H), 0.88 (t, J=7.0 Hz, 3H).

**Example 66: Preparation of (S)-4-ethyl-8-fluoro-4-hydroxy-11-phenyl-1H-pyrano[3,4:6,7]indolizino[2,1 - b]quinoline-3,6,14(4H,11H,12H)-trione:**

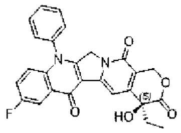

***Step 1: Preparation of 1-(5-fluoro-2-(phenylamino)phenyl)ethenone:***

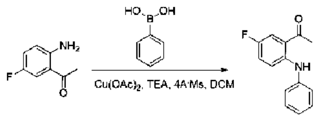


3-Fluoro-6-aminoacetophenone (5.00 g, 32.7 mmol) was dissolved in DCM (50 mL). Anhydrous copper acetate (5.93 g, 32.7 mmol, 1.0 eq.), phenylboronic acid (7.96 g, 65.3 mmol, 2.0 eq.), triethylamine (6.61 g, 65.3 mmol, 2.0 eq.) and 4Å molecular sieves (5 g) were added sequentially. After the addition, the mixture was stirred overnight at room temperature under an oxygen atmosphere. LCMS indicated that the reaction conversion was approximately 50%. The reaction solution was filtered, and the filtrate was concentrated under reduced pressure to obtain a crude product. The crude product was purified by silica gel column chromatography (eluent gradient: petroleum ether:ethyl acetate = 1:0 to 50:3, v/v) to afford the title compound as a yellow solid (3.3 g, yield: 44.1%). LCMS (ESI): $[M+H]^+$=230.3, $^1H$ NMR (400 MHz, DMSO-$d_6$): δ 10.09 (s, 1H), 7.77 (dd, J=10.0,3.0 Hz, 1H), 7.39-7.30 (m, 3H), 7.27-7.19 (m, 3H), 7.12-7.06 (m, 1H), 2.62 (s, 3H).

Using the intermediate 1-(5-fluoro-2-(phenylamino)phenyl)ethanone obtained from step 1 as the starting material, the synthetic operations of steps 2-7 in Example 31 were followed. Subsequently, the Boc group was removed under acidic conditions to afford the title compound as a yellow solid. LCMS (ESI): $[M+H]^+$=459.3, $^1H$ NMR (400 MHz, DMSO-$d_6$): δ 8.04 (dd, J=8.8,3.0 Hz, 1H), 7.85-7.76 (m, 3H), 7.76-7.66 (m, 2H), 7.66-7.60 (m, 1H), 7.33 (s, 1H), 7.05 (dd, J=9.3,4.3 Hz, 1H), 6.41 (s, 1H), 5.31 (s, 2H), 4.90-4.74 (m, 2H), 1.88-7.76 (m, 2H), 0.87 (t, J=7.3 Hz, 3H).

**Example 67: Preparation of (S)-5-(4-aminophenyl)-12-ethyl-12-hydroxy-9,12-dihydro-8H-[1,3]dioxolo[4,5-g]pyrano[3,4:6,7]indolizino[2,1 -b]quinoline-8,11,14 (SH,6H)-trione:**

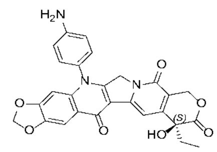

***Step 1: Synthesis of tert-Butyl (4-((6-acetylbenzo[d][1,3]dioxol-5-yl)amino)phenyl)carbamate:***

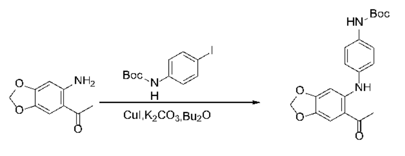


6-Amino-3,4-methylenedioxyacetophenone (5 g, 27.91 mmol, 1.0 eq.) was dissolved in dibutyl ether (70 mL).

Then tert-butyl (4-iodophenyl)carbamate (13.36 g, 41.86 mmol, 1.5 eq.), copper(I) iodide (1.06 g, 5.58 mmol, 0.2 eq.) and potassium carbonate (7.71 g, 55.81 mmol, 2.0 eq.) were added sequentially. Under nitrogen protection, the reaction mixture was heated to 180 °C and stirred for 8 hours. LCMS confirmed the reaction was complete. The reaction solution was filtered, and the filtrate was concentrated under reduced pressure to obtain a crude product. The crude product was purified by silica gel column chromatography (eluent gradient: petroleum ether:ethyl acetate = 1:0 to 5:1, v/v) to afford the title compound as a yellow solid (5 g, yield: 48.37%). LCMS (ESI): $[M+H]^+$=371.1.

Using the intermediate tert-butyl (4-((6-acetylbenzo[d][1,3]dioxol-5-yl)amino)phenyl)carbamate obtained from step 1 as the starting material, the synthetic procedures of Steps 2-7 in Example 31 were followed to afford the title compound as a white solid. LCMS (ESI): $[M+H]^+$=500.1. $^1$H NMR (400 MHz, DMSO-$d_6$): δ 7.61 (s, 1H), 7.32-7.15 (m, 3H), 6.80 (dd, J=5.6,3.3 Hz, 2H), 6.41 (s, 1H), 6.19 (s, 2H), 5.38-5.20 (m, 2H), 4.82-4.65 (m, 2H), 1.87-1.72 (m, 2H), 0.85 (t, J=7.3 Hz, 3H).

**Example 68: Preparation of (S)-11-(4-aminophenyl)-4-ethyl-8-fluoro-4-hydroxy-9-methyl-1,12-dihydro-14H-pyrano[3,4:6,7]indolizino[2,1 -b]quinoline-3,6,14 (4H,11H)-trione:**

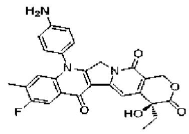

Using the intermediate from Preparation Example 29, (Z)-1-(4-methyl-2,5-difluorophenyl)-3-(dimethylamino) but-2-en-1-one, and tert-butyl (4-aminophenyl)carbamate as the starting materials, the title compound was synthesized following steps 1-5 of Example 55. The Boc group was then removed under acidic conditions to afford the title compound as a yellow solid. LCMS (ESI): $[M-1]^+$=488.0. $^1$H NMR (400 MHz, DMSO-$d_6$): δ 7.95 (d, J=9.6 Hz, 1H), 7.30 (s, 1H), 7.28-7.20 (m, 2H), 7.02 (d, J=6.2 Hz, 1H), 6.85-6.79 (m, 2H), 6.40 (s, 1H), 5.77 (s, 2H), 5.32 (s, 2H), 4.80 (s, 2H), 2.33 (s, 3H), 1.87-1.75 (m, 2H), 0.88 (t, J=7.3 Hz, 3H).

**Example 69: Preparation of (S)-9-amino-4-ethyl-8-fluoro-4-hydroxy-11-cyclopentyl-1,12-dihydro-14H-pyrano [3,4:6,7]indolizino[2,1 -b]quinoline-3,6,14(4H,11H)-trione:**

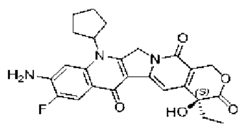

Using 7-bromo-1-cyclopentyl-6-fluoro-2-methylquinolin-4(1H)-one (Intermediate 6) obtained from Preparation Example 6 as the starting material, the title compound was synthesized by following the operations of Steps 1-6 in Example 8, and was obtained as a yellow solid. LCMS (ESI): $[M+H]^+$=466.3, $^1$H NMR (400 MHz, DMSO-$d_6$ + $D_2O$): δ 7.80 (d, J=11.6 Hz, 1H), 7.25 (s, 1H), 6.90 (d, J=7.2 Hz, 1H), 6.38 (s, 2H), 6.29 (s, 1H), 5.43 (s, 2H), 5.32 (dd, J=15.6,24.8 Hz, 2H), 4.90-4.78 (m, 1H), 2.30-2.08 (m, 6H), 1.86-1.70 (m, 4H), 0.85 (t, J=7.2 Hz, 3H).

**Example 70: Preparation of (S)-9-amino-4-ethyl-8-fluoro-4-hydroxy-11-(4,4-dimethylcyclohexyl)-1,12-dihydro-14H-pyrano[3,4:6,7]indolizino[2,1 -b] quinoline-3,6,14(4H,11H)-trione:**

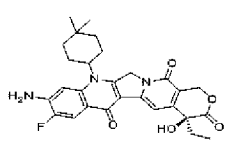

Using 7-bromo-1-(4,4-dimethylcyclohexyl)-6-fluoro-2-methylquinolin-4(1H)-one (Intermediate 14) obtained from Preparation Example 14 as the starting material, the title compound was synthesized by following the operations

of steps 1-6 in Example 8, and was obtained as a pale-colored solid (2.27 mg, yield: 16.08%). LCMS (ESI): $[M+H]^+$=508.2. $^1H$ NMR (400 MHz, DMSO-$d_6$): δ 7.79 (d, J=11.5 Hz, 1H), 7.26 (s, 1H), 7.21-7.16 (m, 1H), 6.42 (s, 2H), 6.30 (s, 1H), 5.46-5.27 (m, 5H), 1.90-1.73 (m, 6H), 1.63-1.53 (m, 4H), 1.16 (s, 3H), 1.01 (s, 3H), 0.85 (t, J=9.4,5.1Hz, 3H).

**Example 71: Preparation of (S)-11-(3-aminophenyl)-4-ethyl-8-fluoro-4-hydroxy-1H-pyrano[3,4:6,7]indolizino [2,1 -b]quinoline-3,6,14(4H,11H,12H)-trione:**

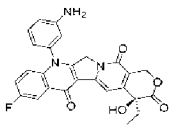

***Step 1: Preparation of 1-(5-fluoro-2-((3-nitrophenyl)amino)phenyl)ethenone:***

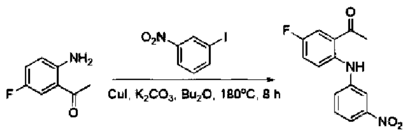


1-(2-Amino-5-fluorophenyl)ethanone (2.0 g, 13.1 mmol, 1.0 eq.) was dissolved in n-butyl ether (20 mL). Then potassium carbonate (3.63 g, 26.3 mmol, 2.0 eq.), 1-iodo-3-nitrobenzene (4.88 g, 19.6 mmol, 1.5 eq.) and copper(I) iodide (500 mg, 1.31 mmol, 0.1 eq.) were added sequentially. After the addition, the mixture was heated to 180 °C and stirred for 8 hours under nitrogen protection. LCMS monitoring confirmed the reaction was complete. The reaction solution was cooled to room temperature, filtered, and the filtrate was concentrated under reduced pressure to obtain a crude product. The crude product was purified by silica gel column chromatography (eluent gradient: DCM:MeOH = 1:0 to 20:1, v/v) to afford the title compound as a yellow solid (2.0 g, yield: 55.7%). LCMS (ESI): $[M+H]^+$=275.1. $^1H$ NMR (400 MHz, $CDCl_3$): δ 10.44 (s, 1H), 8.11 (dd, J=2.8,1.6 Hz, 1H), 7.93-7.86 (m, 1H), 7.57 (dd, J=9.4,3.0 Hz, 1H), 7.53-7.47 (m, 2H), 7.36 (dd, J=9.2,4.6 Hz, 1H), 7.25-7.16 (m, 1H), 2.67 (s, 3H).

Using the intermediate 1-(5-fluoro-2-((3-nitrophenyl)amino)phenyl)ethanone obtained from step 1 as the starting material, the title compound was synthesized by following the procedures of steps 2-7 in Example 31. LCMS (ESI): $[M+H]^+$-474.1. $^1H$ NMR (400 MHz, DMSO-$d_6$): δ 8.02 (dd, J=8.8,3.1 Hz, 1H), 7.68-7.61 (m, 1H), 7.40-7.34 (m, 1H), 7.31 (s, 1H), 7.23-7.16 (m, 1H), 6.90-6.67 (m, 4H), 6.40 (s, 1H), 5.69 (s, 1H), 5.35-5.26 (m, 2H), 4.89-4.78 (m, 2H), 1.87-7.73 (m, 2H), 0.86 (t, J=6.8 Hz, 3H).

**Example 72: Preparation of (S)-11-(4-aminophenyl)-4-ethyl-8-fluoro-4-hydroxy-1H-pyrano[3,4:6,7]indolizino [2,1 -b]quinoline-3,6,14(4H,11H,12H)-trione:**

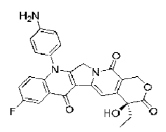

***Step 1: Preparation of 1-(5-fluoro-2-((4-nitrophenyl)amino)phenyl)ethenone:***

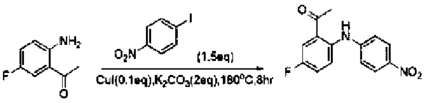

1-(2-Amino-5-fluorophenyl)ethanone (4.0 g, 26.1 mmol, 1.0 eq.) was dissolved in n-butyl ether (40 mL). Then potassium carbonate (7.26 g, 52.5 mmol, 2.0 eq.), 1-iodo-4-nitrobenzene (9.76 g, 39.2 mmol, 1.5 eq.) and copper(I) iodide (1.0 g, 5.22 mmol, 0.1 eq.) were added sequentially. After the addition, the mixture was heated to 180 °C and stirred for 8 hours under nitrogen protection. The reaction solution was cooled to room temperature, filtered, and the filtrate was concentrated under reduced pressure to obtain a crude product. The crude product was purified by silica gel column chromatography (eluent gradient: DCM:MeOH = 1:0 to 20:1, v/v) to afford the title compound as a yellow solid (7.2 g, yield: 100%). LCMS (ESI): $[M+H]^+$=275.1. $^1H$ NMR (400 MHz, $CDCl_3$): δ 10.48 (s, 1H), 8.28-8.16 (d, 2H), 7.59 (dd, J=9.2,3.0 Hz, 1H), 7.53 (dd, J=9.2,4.7 Hz, 1H), 7.30-7.22 (m, 3H), 2.68 (s, 3H). Using the intermediate 1-(5-fluoro-2-((4-nitrophenyl) amino)phenyl)ethanone obtained from Step 1 as the starting material, the title compound was synthesized by following the procedures of steps 2-7 in Example 31. LCMS (ESI): $[M+H]^+$=474.1. $^1H$ NMR (400 MHz, DMSO-$d_6$): δ 8.01 (dd, J=8.9,3.1 Hz, 1H), 7.66-7.60 (m, 1H), 7.31 (s, 1H), 7.28-7.12 (m, 4H), 6.84-6.75 (m, 2H), 6.39 (s, 1H), 5.76 (s, 1H), 5.34-5.26 (m, 2H), 4.83 (s, 2H), 1.88-1.76 (m, 2H), 0.88 (t, J=7.3 Hz, 3H).

**Example 73: Preparation of (S)-4-ethyl-8-fluoro-4-hydroxy-11-(3-methoxyphenyl)-1,12-dihydro-14H-pyrano [3,4:6,7]indolizino[2,1 -b]quinoline-3,6,14(4H,11H)-trione:**

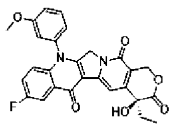

Similarly, using Intermediate 27 from Preparation Example 27, namely (Z)-1-(2,5-difluorophenyl)-3-(dimethylamino)but-2-en-1-one, and the commercial reagent 3-methoxyaniline as the starting materials, the title compound was synthesized by following Steps 1-5 of Example 55, and was obtained as an off-white solid. LCMS (ESI): $[M+H]^+$=489.2.

**Example 74: Preparation of (S)-4-ethyl-8-fluoro-4-hydroxy-11-(4-methoxyphenyl) -1,12-dihydro-14H-pyrano [3,4:6,7]indolizino[2,1 -b]quinoline-3,6,14(4H,11H)-trione:**

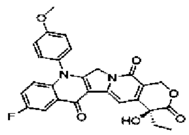

Using Intermediate 27 from Preparation Example 27, (Z)-1-(2,5-difluorophenyl)-3-(dimethylamino)but-2-en-1-one, and the commercial reagent 4-methoxyaniline as the starting materials, the title compound was synthesized by following Steps 1-5 of Example 55, and was obtained as an off-white solid. LCMS (ESI): $[M+H]^+$=489.2.

**Example 75: Preparation of (S)-11-(3-amino-1-methyl-1H-pyrazol-4-yl)-4-ethyl-8-fluoro-4-hydroxy-1H-pyrano [3,4:6,7]indolizino[2,1 -b]quinoline-3,6,14(4H,11H, 12H)-trione:**

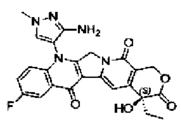

Using Intermediate 27 from Preparation Example 27, (Z)-1-(2,5-difluorophenyl)-3-(dimethylamino)but-2-en-1-one, and the commercial reagent 1-methyl-3-nitro-1H-pyrazol-4-amine as the starting materials, the title compound was synthesized by following Steps 1-5 of Example 55. In the final cyclization step, the nitro group was directly reduced to an amino group, affording the title compound as an off-white solid.

LCMS (ESI): $[M+H]^+$=478.3. $^1H$ NMR (400 MHz, DMSO-$d_6$): δ 7.98 (dd, J=8.8,2.7 Hz, 1H), 7.88 (s, 1H), 7.69-7.64 (m, 1H), 7.38 (q, J=4.6 Hz, 1H), 7.28 (d, J=4.9 Hz, 1H), 5.37-5.27 (m, 2H), 5.02-4.95 (m, 1H), 4.82-4.77 (m, 1H), 3.76 (s, 3H), 1.87-1.75 (m, 2H), 0.86 (t, J=7.3 Hz, 3H).

**Example 76: Preparation of (S)-4-ethyl-8-fluoro-4-hydroxy-11-(1-methyl-3-(methylamino)-1H-pyrazol-4-yl)-1H-pyrano[3,4:6,7]indolizino[2,1 -b]quinoline-3,6,14(4H,11H,12H)-trione:**

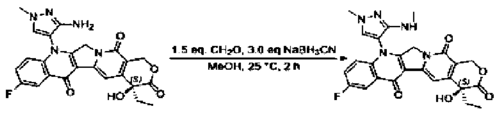


The compound from Example 75 (86 mg, 0.18 mmol, 1.0 eq.) was dissolved in MeOH (2 mL). Formaldehyde (8.07 mg, 0.27 mmol, 1.5 eq.) was added, and the mixture was stirred at room temperature for 30 minutes. Then sodium cyanoborohydride (33.22 mg, 0.54 mmol, 3.0 eq.) was added, and the reaction was stirred at room temperature for 2 hours. The reaction progress was monitored by LCMS. After the reaction was completed, 1 M hydrochloric acid (1.0 mL) was added to the system. The mixture was then subjected to preparative high-performance liquid chromatography (Prep-HPLC) for purification, affording the title compound as a reddish-brown solid. LCMS (ESI): $[M+H]^+$=492.3.

Example 77: Preparation of (4S,12R)-4-ethyl-8-fluoro-4-hydroxy-11,12-dimethyl-1,12-dihydro-14H-pyrano[3,4:6,7]indolizino[2,1-b]quinoline-3,6,14(4H,11H)-trione (Isomer 1 or Isomer 2):

Example 78: Preparation of (4S,12S)-4-ethyl-8-fluoro-4-hydroxy-11,12-dimethyl-1,12-dihydro-14H-pyrano[3,4:6,7]indolizino[2,1-b]quinoline-3,6,14(4H,11H)-trione (Isomer 2 or Isomer 1):

***Step 1: Preparation of 6-fluoro-1-methyl-4-oxo-1,4-dihydroquinoline-2-carbaldehyde:***

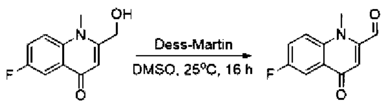


The intermediate 6-fluoro-2-(hydroxymethyl)-1-methylquinolin-4(1H)-one (9 g, 43.44 mmol, 1.0 eq.) from Step 1 of Example 6 was dissolved in dimethyl sulfoxide (DMSO, 100 mL). Under an ice bath, Dess-Martin periodinane (27.63 g, 65.15 mmol, 1.5 eq.) was added slowly. The mixture was stirred at room temperature for 16 hours, and the reaction completion was monitored by LCMS. The reaction mixture was filtered, and the filtrate was concentrated to afford the title compound as a yellow solid (5.4 g, yield: 60.6%). LCMS (ESI): $[M+H_2O+H]^+$=224.19. $^1H$ NMR (400 MHz, DMSO-$d_6$): $\delta$ 9.93 (s, 1H), 7.99 (dd, J=9.4,4.4 Hz, 1H), 7.91-7.64 (m, 2H), 6.70 (s, 1H), 4.00 (s, 3H).

***Step 2: Preparation of 6-fluoro-2-(2-hydroxyethyl)-1-methylquinolin-4(1H)-one:***

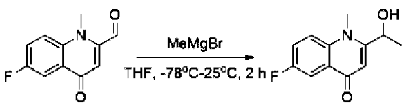


6-Fluoro-1-methyl-4-oxo-1,4-dihydroquinoline-2-carbaldehyde (1000 mg, 4.87 mmol, 1.0 equiv.) was dissolved in tetrahydrofuran (THF, 15 mL). The reaction system was purged with nitrogen three times to displace air, then cooled to -78 °C, and a 1 mol/L methylmagnesium bromide-THF solution (5.85 mL, 5.85 mmol, 1.2 eq.) was slowly added dropwise into the reaction mixture via a syringe. After the completion of dropwise addition, the reaction mixture was slowly warmed to room temperature and stirred for 2 hours. The reaction completion was monitored by LCMS. Upon reaction termination, saturated aqueous ammonium chloride solution (15 mL) was added to quench the reaction, followed by extraction with ethyl acetate (EA) (10 mL *3). The combined organic phases were dried over anhydrous sodium sulfate, filtered, and the filtrate was concentrated under reduced pressure to obtain a crude product. The crude product was separated and purified by silica gel column chromatography (eluent: DCM:MeOH = 20:1, v/v) to afford the title compound as a yellow solid (700 mg, yield: 65%). LCMS (ESI): $[M+H]^+$ = 222.19. $^1H$ NMR (400 MHz, DMSO-$d_6$) $\delta$: 7.89 (dd, J = 9.4, 4.4 Hz, 1H), 7.80 (dd, J = 9.2, 3.2 Hz, 1H), 7.64 (ddd, J = 9.2, 8.0, 3.2 Hz, 1H), 6.31 (s, 1H), 5.75 (d, J = 5.2 Hz, 1H), 5.08-4.93 (m, 1H), 3.81 (s, 3H), 1.42 (d, J = 6.4 Hz, 3H).

*Step 3: Preparation of (4S)-4-ethyl-7-(1-(6-fluoro-1-methyl-4-oxo-1,4-dihydroquinolin-2-yl)ethyl)-4-hydroxy-1,7-dihydro-3H-pyrano[3,4-c]pyridine-3,8(4H)-dione:*

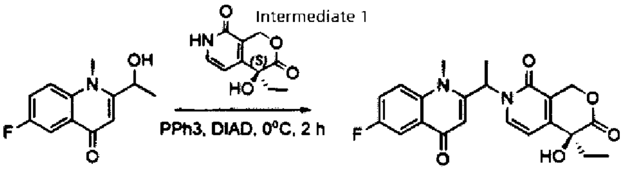


6-Fluoro-2-(2-hydroxyethyl)-1-methylquinolin-4(1H)-one (500 mg, 2.26 mmol, 1.0 equiv.) and Intermediate 1 (472.81 mg, 2.26 mmol, 1.0 equiv.) were dissolved in tetrahydrofuran (THF, 10 mL). The reaction system was purged with nitrogen to displace air, then triphenylphosphine ($PPh_3$, 1778.4 mg, 6.78 mmol, 3.0 eq.) was added. The mixture was cooled to 0 °C in an ice-water bath, and diisopropyl azodicarboxylate (DIAD, 685.52 mg, 3.39 mmol, 1.5 eq.) was slowly added dropwise via a syringe. The reaction was stirred at 0 °C for 2 hours, and the reaction progress was monitored by LCMS. After the reaction was complete, the reaction mixture was concentrated under reduced pressure to dryness. The residue was purified by silica gel column chromatography (eluent: DCM:MeOH = 20:1, v/v) to afford the title compound as a yellow solid (600 mg, yield: 64.37%). LCMS (ESI): $[M+H]^+$ = 413.07.

$^1H$ NMR (400 MHz, DMSO-$d_6$) $\delta$: 7.90 (td, J = 10.0, 4.4 Hz, 1H), 7.81 (dt, J = 8.8, 3.2 Hz, 1H), 7.73-7.64 (m, 2H), 6.52 (d, J = 7.2 Hz, 1H), 6.36-6.29 (m, 2H), 6.15 (d, J = 4.8 Hz, 1H), 5.31 (d, J = 2.4 Hz, 2H), 3.69 (d, J = 5.6 Hz, 3H), 1.78 (dt, J = 10.8, 7.2 Hz, 2H), 1.67 (t, J = 7.2 Hz, 3H), 0.80 (dt, J = 12.8, 7.2 Hz, 3H).

***Step 4: Preparation of (4S)-4-ethyl-7-(1-(6-fluoro-3-iodo-1-methyl-4-oxo-1,4-dihydroquinolin-2-yl)ethyl)-4-hydroxy-1,7-dihydro-3H-pyrano[3,4-c]pyridine-3,8(4H)-dione:***

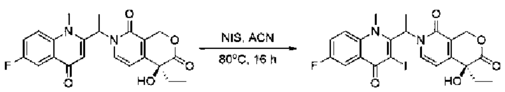


The intermediate obtained from Step 3 (300 mg, 0.73 mmol, 1.0 equiv.) and N-iodosuccinimide (NIS, 327.32 mg, 1.45 mmol, 2.0 eq.) were dissolved in acetonitrile (ACN, 10 mL). The mixture was heated to 80 °C and stirred for 16 hours. The reaction completion was monitored by LCMS. After the reaction was finished, the mixture was concentrated. The residue was separated and purified by silica gel column chromatography (eluent: DCM:MeOH = 20:1, v/v) to afford the title compound as a yellow solid (170 mg, yield: 43.3%). LCMS (ESI): $[M+H]^+$ = 538.86. $^1H$ NMR (400 MHz, DMSO-$d_6$) $\delta$: 8.11 (d, J = 7.2 Hz, 1H), 7.95 (ddd, J = 17.2, 9.6, 4.4 Hz, 1H), 7.87-7.77 (m, 1H), 7.67 (tt, J = 7.6, 3.6 Hz, 1H), 6.59 (dd, J = 7.2, 3.6 Hz, 1H), 6.39 (s, 1H), 6.05 (dt, J = 25.2, 7.2 Hz, 1H), 5.25-5.08 (m, 2H), 3.79 (d, J = 28.8 Hz, 3H), 1.95 (d, J = 7.2 Hz, 3H), 1.87-1.68 (m, 2H), 0.82 (td, J = 7.2, 3.6 Hz, 3H).

***Step 5: Preparation of (4S,12R)-4-ethyl-8-fluoro-4-hydroxy-11,12-dimethyl-1,12-dihydro-14H-pyrano[3,4:6,7]indolizino[2,1-b]quinoline-3,6,14(4H,11H)-trione (Isomer 1 or Isomer 2) and (4S,12S)-4-ethyl-8-fluoro-4-hydroxy-11,12-dimethyl-1,12-dihydro-14H-pyrano[3,4:6,7]indolizino[2,1-b]quinoline-3,6,14(4H,11H)-trione (Isomer 2 or Isomer 1):***

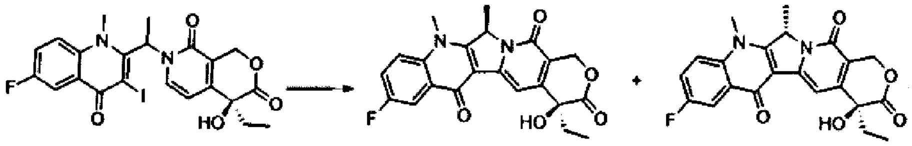

Isomer 1 or isomer 2 Isomer 2 or isomer 1

The intermediate obtained from step 4 (150 mg, 0.28 mmol, 1.0 equiv.) and benzoyl peroxide (BPO, 33.75 mg, 0.14 mmol, 0.5 eq.alent) were dissolved in toluene (40 mL). The reaction system was purged with nitrogen to displace air, then heated to 100 °C. A solution of tris(trimethylsilyl)silane ($(TMS)_3SiH$, 277.16 mg, 1.11 mmol, 4.0 eq.) in toluene (5 mL)

was slowly added dropwise to the reaction mixture, and the mixture was stirred at 100 °C for 16 hours. The reaction completion was monitored by LCMS. After the reaction was finished, the mixture was concentrated under reduced pressure to obtain a crude product. The crude product was dissolved in dimethyl sulfoxide (DMSO, 3 mL), and subjected to preparative high-performance liquid chromatography (Prep-HPLC) for separation and purification, affording Isomer 1 or Isomer 2 (10 mg) and Isomer 2 or Isomer 1 (18 mg), both as white solids.

**Characterization Data of Isomer 1 or Isomer 2:** LCMS (ESI): $[M+H]^+$ = 411.03, $t_R$ = 2.461 min; $^1H$ NMR (400 MHz, DMSO-$d_6$) δ: 8.04 (d, J = 9.6 Hz, 1H), 7.97 (d, J = 11.6 Hz, 1H), 7.85-7.76 (m, 1H), 7.27 (d, J = 7.6 Hz, 1H), 6.33 (d, J = 7.2 Hz, 1H), 6.00 (p, J = 7.2 Hz, 1H), 5.40-5.24 (m, 2H), 4.00 (s, 3H), 1.88-1.78 (m, 2H), 1.75 (d, J = 6.4 Hz, 3H), 0.85 (t, J = 7.2 Hz, 4H).

**Characterization Data of Isomer 2 or Isomer 1:** LCMS (ESI): $[M+H]^+$ = 411.04, $t_R$ = 2.667 min; $^1H$ NMR (400 MHz, DMSO-$d_6$) δ: 8.03 (dd, J = 9.2, 4.0 Hz, 1H), 7.97 (dd, J = 8.8, 3.2 Hz, 1H), 7.79 (td, J = 9.2, 8.8, 3.2 Hz, 1H), 7.27 (d, J = 7.6 Hz, 1H), 6.33 (d, J = 7.2 Hz, 1H), 6.00 (dd, J = 10.4, 6.4 Hz, 1H), 5.39 - 5.25 (m, 2H), 4.00 (s, 3H), 1.82 (dq, J = 8.0, 5.2, 4.0 Hz, 2H), 1.75 (dd, J = 6.4, 1.6 Hz, 3H), 0.86 (q, J = 7.2 Hz, 4H).

Example 79: Preparation of (3S,8S)-8,15-diethyl-4-fluoro-3,8-dihydroxy-1,2,3,11,14,15-hexahydro-6H,12H-cyclopenta [h]pyrano[3,4:6,7]indazino[2,1 -b]quinoline-6,9,12(8H)-trione (Isomer 1 or Isomer 2) :

Example 80: Preparation of (3R,8S)-8,15-diethyl-4-fluoro-3,8-dihydroxy-1,2,3,11,14,15-hexahydro-6H,12H-cyclopenta [h]pyrano[3,4:6,7]indazino[2,1 -b]quinoline-6,9,12(8H)-trione (Isomer 2 or Isomer 1):

*Step 1: Preparation of 4-Bromo-7-fluoro-2,3-dihydro-1H-inden-1-ol:*

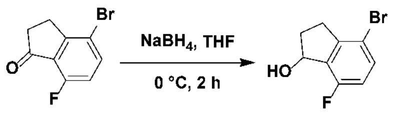


4-Bromo-7-fluoroindanone (10.0 g, 43.66 mmol, 1.0 eq) was dissolved in tetrahydrofuran (THF, 160 mL). The mixture was cooled to 0 °C, and sodium borohydride ($NaBH_4$, 2.48 g, 65.49 mmol, 1.5 eq) was added portionwise under stirring. The reaction was stirred at 0 °C for 2 hours, and the reaction completion was confirmed by LCMS analysis. The reaction mixture was slowly poured into ice water (300 mL) to quench the reaction, then extracted with ethyl acetate (EA, 200 mL × 2). The combined organic layers were washed with water (50 mL) and saturated brine (50 mL) sequentially, dried over anhydrous sodium sulfate, filtered, and the filtrate was concentrated under reduced pressure to obtain a crude product. The crude product was purified by silica gel column chromatography (eluent: PE:THF = 5:1, v/v) to afford the title compound as a pale yellow solid (8.6 g, yield: 85.25%). $^1H$ NMR (400 MHz, DMSO-$d_6$) δ: 7.50-7.47 (m, 1H), 7.02-6.95 (m, 1H), 5.49-5.32 (m, 2H), 2.98-2.91 (m, 1H), 2.76-2.61 (m, 1H), 2.31-2.28 (m, 1H), 1.91-1.90 (m, 1H).

***Step 2: Preparation of 1-(Benzyloxy)-4-bromo-7-fluoro-2,3-dihydro-1H-indene:***

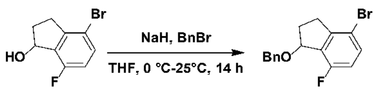


The intermediate obtained from Step 1 (9.6 g, 41.55 mmol, 1.0 equiv.) was dissolved in tetrahydrofuran (THF, 100 mL). The mixture was cooled to 0 °C, and sodium hydride (NaH, 1.43 g, 62.32 mmol, 1.5 eq.) was added portionwise under stirring. The reaction was stirred at 0 °C for 0.5 hours, then benzyl bromide (14.21 g, 83.1 mmol, 2.0 eq.) was added dropwise at 0 °C. The ice bath was removed, and the reaction mixture was naturally warmed to 25 °C, followed by continuous stirring for 14 hours. The reaction completion was confirmed by LCMS. The reaction mixture was slowly poured into water (100 mL) to quench the reaction, then extracted with ethyl acetate (EA, 200 mL × 2). The combined organic phases were washed with water (50 mL) and saturated sodium chloride solution (50 mL) sequentially, dried over anhydrous sodium sulfate, filtered, and the filtrate was concentrated, and purified by silica gel column chromatography (eluent: PE:THF = 5:1, v/v) to afford the title compound as a pale yellow oil (11.1 g, yield: 82.46%). $^1H$ NMR (400 MHz, DMSO-$d_6$) δ: 7.93-7.90 (m, 1H), 7.33-7.32 (m, 5H), 7.13-7.08 (m, 1H), 5.28-5.26 (m, 1H), 4.60-4.54 (m, 2H), 3.06-2.98 (m, 1H), 2.83-2.78 (m, 1H), 2.31-2.13 (m, 2H).

*Step 3: Preparation of 1-(Benzyloxy)-N-ethyl-7-fluoro-2,3-dihydro-1H-indole-4-amine:*

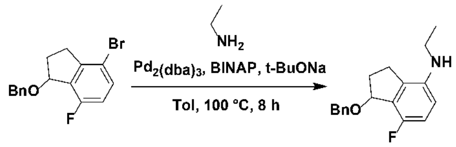


The intermediate obtained from step 2 (5.1 g, 15.88 mmol, 1.0 eq) was dissolved in toluene (100 mL). Then ethylamine (1.43 g, 31.76 mmol, 2.0 eq), sodium tert-butoxide (t-BuONa, 3.05 g, 31.76 mmol, 2.0 eq), 1,1 - binaphthyl-2,2 -bis(diphenylphosphine) (BINAP, 0.99 g, 1.59 mmol, 0.1 eq), and tris(dibenzylideneacetone)dipalladium(0) ($Pd_2(dba)_3$, 0.73 g, 0.79 mmol, 0.05 eq) were added sequentially under stirring. After the addition was complete, the reaction mixture was stirred at 100 °C for 16 hours. The reaction completion was confirmed by LCMS analysis. The mixture was cooled to room temperature, slowly poured into ice water (50 mL) to quench the reaction, and extracted with ethyl acetate (EA, 30 mL × 3). The combined organic layers were washed with saturated brine (30 mL), dried over anhydrous sodium sulfate, filtered, and the filtrate was concentrated, then purified by silica gel column chromatography (eluent: PE:THF = 3:1, v/v) to afford the title compound as a yellow solid (3.5 g, yield: 77.24%). LCMS (ESI): $[M+H]^+$ = 286.2, $t_R$ = 1.207 min.

*Step 4: Preparation of 1-(Benzyloxy)-5-bromo-N-ethyl-7-fluoro-2,3-dihydro-1H-indole-4-amine:*

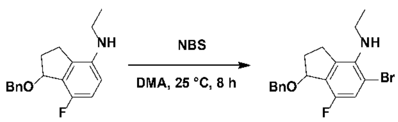


The intermediate obtained from step 3 (3.4 g, 11.91 mmol, 1.0 equiv.) was dissolved in N,N-dimethylacetamide (DMAC, 40 mL). Then N-bromosuccinimide (NBS, 2.33 g, 13.1 mmol, 1.1 eq.) was added to the reaction mixture under stirring. After the addition was complete, the reaction mixture was heated to 25 °C and stirred for 8 hours. The mixture was slowly poured into ice water (200 mL) to quench the reaction, then extracted with ethyl acetate (EA, 50 mL × 3). The combined organic layers were washed with saturated sodium chloride solution (30 mL), dried over anhydrous sodium sulfate, filtered, and the filtrate was concentrated under reduced pressure to obtain a crude product. then purified by silica gel column chromatography (eluent: PE:THF = 4:1, v/v) to afford the title compound as a yellow solid (3.1 g, 8.51 mmol, yield: 71.46%). LCMS (ESI): $[M+H]^+$ = 364.1, $t_R$ = 1.583 min.

*Step 5: Preparation of 1-[1-(Benzyloxy)-4-(ethylamino)-7-fluoro-2,3-dihydro-1H-indene-5-yl]ethan-1-one:*

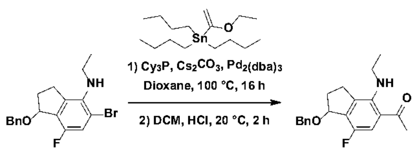


The intermediate obtained from step 4 (3.8 g, 10.43 mmol, 1.0 equiv.) was dissolved in 1,4-dioxane (80 mL). Subsequently, tricyclohexylphosphine ($PCy_3$, 0.58 g, 2.09 mmol, 0.2 eq.alent), tributyl(1-ethoxyvinyl)stannane (4.14 g, 11.47 mmol, 1.1 eq.alent), tris(dibenzylideneacetone)dipalladium(0) ($Pd_2(dba)_3$, 0.96 g, 1.04 mmol, 0.1 eq.alent), and cesium carbonate ($Cs_2CO_3$, 6.8 g, 20.86 mmol, 2.0 eq.alent) were added sequentially under stirring. After the addition was complete, the reaction mixture was stirred at 100 °C for 16 hours. The reaction completion was confirmed by LCMS. The mixture was cooled to room temperature, slowly poured into ice water (200 mL) to quench the reaction, and extracted with ethyl acetate (EA, 50 mL × 3). The combined organic phases were washed with saturated aqueous sodium chloride solution (30 mL), dried over anhydrous sodium sulfate, filtered, and the filtrate was concentrated under reduced pressure to obtain a crude product. The crude product was dissolved in DCM (DCM, 200 mL), and 1 N hydrochloric acid (1 N HCl, 20.86 mL) was added, followed by stirring for 2 hours. The mixture was poured into ice water (50 mL), extracted with ethyl

acetate (EA, 30 mL × 3), and the combined organic phases were washed with saturated aqueous sodium chloride solution (30 mL), dried over anhydrous sodium sulfate, filtered. The filtrate was concentrated to obtain a crude product, which was separated and purified by silica gel column chromatography (eluent: PE:THF = 3:1, v/v) to afford the title compound as a yellow solid (2.2 g, yield: 64.43%). LCMS (ESI): $[M+H]^+$ = 328.2, $t_R$ = 1.463 min.

***Step 6: Preparation of Ethyl {[5-acetyl-1-(benzyloxy)-7-fluoro-2,3-dihydro-1H-indol-4-yl](ethyl)carbamoyl}acetate:***

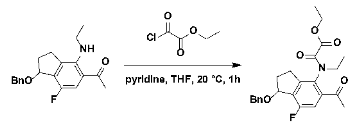


The intermediate obtained from step 5 (3.4 g, 10.39 mmol, 1.0 eq.) and pyridine (4.11 g, 51.95 mmol, 5.0 eq.) were dissolved in DCM (40 mL). The mixture was cooled to 0 °C, and ethyl oxalyl chloride (2.84 g, 20.78 mmol, 2.0 eq.) was added dropwise under stirring. The cooling bath was removed, and the reaction was stirred at 20 °C for 1 hour. The reaction completion was confirmed by LCMS analysis. The reaction mixture was slowly poured into ice water (50 mL), then extracted with DCM (30 mL × 3). The combined organic layers were washed with saturated sodium chloride solution (20 mL × 2) sequentially, dried over anhydrous sodium sulfate, filtered, and the filtrate was concentrated to obtain a crude product. The crude product was purified by silica gel column chromatography (eluent: PE:THF = 3:1, v/v) to afford the title compound as a yellow solid (3.5 g, yield: 78.8%). LCMS (ESI): $[M+H]^+$ = 428.2, $t_R$ = 1.453 min.

***Step 7: Preparation of Ethyl 7-(Benzyloxy)-1-ethyl-6-fluoro-4-oxo-1H,4H,7H,8H,9H-pentacyclo[h]quinoline-2-carboxylate:***

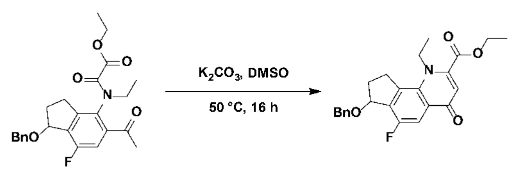


The intermediate obtained from step 6 (3.5 g, 8.19 mmol, 1.0 equiv.) was dissolved in dimethyl sulfoxide (DMSO, 40 mL). Then potassium carbonate ($K_2CO_3$, 2.26 g, 16.38 mmol, 2.0 eq.) was added under stirring, and the reaction mixture was stirred at 50 °C for 16 hours. The reaction completion was confirmed by LCMS analysis. The mixture was cooled to room temperature, slowly poured into ice water (200 mL), and extracted with ethyl acetate (EA, 100 mL × 3). The combined organic layers were washed with water (50 mL) and saturated brine (50 mL) sequentially, dried over anhydrous sodium sulfate, filtered, and the filtrate was concentrated under reduced pressure to afford the title compound as a yellow solid (1.8 g, yield: 53.68%). LCMS (ESI): $[M+H]^+$ = 410.3, $t_R$ = 1.133 min.

*Step 8: Preparation of 7-(Benzyloxy)-1-ethyl-6-fluoro-2-(hydroxymethyl)-1H,4H,7H,8H,9H-pentacyclo[h]quinolin-4-one:*

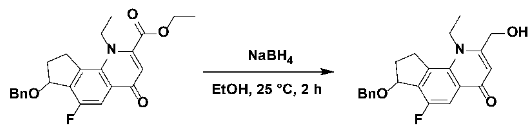


The intermediate obtained from step 7 (2.85 g, 6.96 mmol, 1.0 equiv.) was dissolved in ethanol (EtOH, 30 mL). The mixture was cooled to 0 °C, and sodium borohydride ($NaBH_4$, 394.98 mg, 10.44 mmol, 1.5 eq.) was added portionwise under stirring. After the addition was complete, the reaction mixture was warmed to 25 °C and stirred for 2 hours. The reaction completion was confirmed by LCMS analysis. The mixture was slowly poured into ice water (100 mL), then

extracted with ethyl acetate (EA, 100 mL × 3). The combined organic phases were washed with water (50 mL) and saturated brine (50 mL) sequentially, dried over anhydrous sodium sulfate, filtered, and the filtrate was concentrated under reduced pressure to afford the title compound as a yellow oil (1.9 g, yield: 74.3%). LCMS (ESI): $[M+H]^+$ = 368.2, $t_R$ = 1.26 min.

***Step 9: Preparation of 7-(Benzyloxy)-1-ethyl-6-fluoro-2-(hydroxymethyl)-3-iodo-1H,4H,7H,8H,9H-cyclopenta[h]quinolin-4-one:***

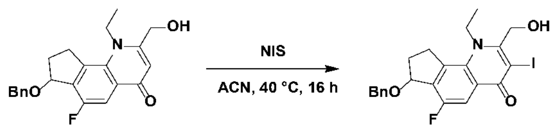


The intermediate obtained from step 8 (1.9 g, 5.17 mmol, 1.0 equiv.) was dissolved in acetonitrile (20 mL), and N-iodosuccinimide (1.28 g, 5.69 mmol, 1.1 eq.) was added. The reaction mixture was stirred at 40 °C for 16 hours. LCMS analysis indicated completion of the reaction. The reaction mixture was cooled to room temperature, filtered, and the filter cake was washed with methyl tert-butyl ether (50 mL × 2). The filter cake was collected and dried under vacuum to afford the title compound (2.0 g, yield 78.4%, yellow oil). LCMS (ESI): $[M+H]^+$ = 494.1, $t_R$ = 1.363 min.

*Step 10: Preparation of (4S)-7-((7-(Benzyloxy)-1-ethyl-6-fluoro-3-iodo-4-oxo-4,7,8,9-tetrahydro-1H-cyclopenta[h]quinolin-2-yl)methyl)-4-ethyl-4-hydroxy-1,7-dihydro-3H-pyrano[3,4-c]pyridine-3,8(4H)-dione:*

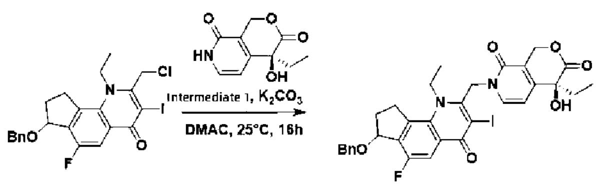


The intermediate obtained from Step 9 (1.5 g, 2.93 mmol, 1.0 eq) and Intermediate 1 (613.8 mg, 2.93 mmol, 1.0 eq) were dissolved in acetonitrile (ACN, 50 mL). Potassium carbonate ($K_2CO_3$, 1.25 g, 8.79 mmol, 3.0 eq) was added, and the reaction mixture was heated to 35 °C and stirred for 16 hours. The reaction mixture was filtered, and the filtrate was concentrated under reduced pressure to obtain a crude product. The crude product was purified by silica gel column chromatography (eluent: PE:EtOAc = 1:0 to 1:1, v/v) to afford the title compound as a yellow solid (1.5 g, yield: 74.79%). LCMS (ESI): $[M+H]^+$ = 683, $t_R$ = 1.707 min.

*Step 11: Preparation of (4S)-7-((7-Bromo-1-ethyl-6-fluoro-3-iodo-4-oxo-4,7,8,9-tetrahydro-1H-cyclopenta[h]quinolin-2-yl)methyl)-4-ethyl-4-hydroxy-1,7-dihydro-3H-pyrano[3,4-c]pyridine-3,8(4H)-dione:*

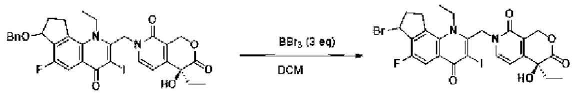


The intermediate obtained from step 10 (1.5 g, 2.19 mmol, 1.0 eq.) was dissolved in DCM (30 mL). Under nitrogen protection at -65 °C, a 1 mol/L solution of boron tribromide ($BBr_3$) in DCM (6.57 mL, 6.57 mmol, 3.0 eq.) was added dropwise, and the reaction was continued to stir at this temperature for 2 hours. The reaction mixture was poured into ice-cold saturated sodium bicarbonate solution (100 mL), and extracted with DCM (DCM, 30 mL × 3). The combined organic layers were dried over anhydrous sodium sulfate, filtered, and concentrated under reduced pressure to obtain a crude product. The crude product was purified by silica gel column chromatography (eluent: DCM:MeOH = 1:0 to 10:1, v/v) to afford the title compound as a yellow solid (500 mg, yield: 38.41%). LCMS (ESI): $[M+H]^+$ = 655, $t_R$ = 1.582 min.

***Step 12: Preparation of (4S)-4-ethyl-7-((1-ethyl-6-fluoro-7-hydroxy-3-iodo-4-oxo-4,7,8,9-tetrahydro-1H-cyclopenta[h]quinolin-2-yl)methyl)-4-hydroxy-1,7-dihydro-3H-pyrano[3,4-c]pyridine-3,8(4H)-dione:***

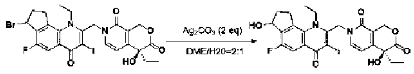


The intermediate obtained from step 11 (340 mg, 0.50 mmol, 1.0 eq.) was dissolved in dimethoxyethane (DME, 4 mL) and water (1.6 mL). Then silver carbonate ($Ag_2CO_3$, 151 mg, 0.54 mmol, 1.5 eq.) was added. The reaction was stirred at room temperature under nitrogen protection for 16 hours. The reaction mixture was filtered, and the filtrate was concentrated under reduced pressure to afford the title compound as a yellow solid (100 mg, crude). LCMS (ESI): $[M+H]^+$ = 595.

*Step 13: Preparation of (4S)-4-ethyl-7-((1-ethyl-6-fluoro-3-iodo-4-oxo-7-((tetrahydro-2H-pyran-2-yl)oxy)-4,7,8,9-tetrahydro-1H-cyclopenta[h]quinolin-2-yl)methyl)-4-hydroxy-1,7-dihydro-3H-pyrano[3,4-c]pyridine-3,8(4H)-dione*.

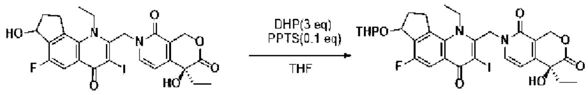


The intermediate obtained from step 12 (150 mg, 0.03 mmol, 1.0 eq.) was dissolved in tetrahydrofuran (THF, 5 mL). Then 3,4-dihydro-2H-pyran (DHP, 84.9 mg, 0.975 mmol, 3.0 eq.) and 3-(4-phenyl-2-pyridyl)-5-phenyl-1,2,4-thiadiazine disulfonate sodium salt (6.38 mg, 0.075 mmol, 0.1 eq.) were added. Under nitrogen protection, the reaction mixture was heated to 50 °C and stirred for 16 hours. The reaction mixture was concentrated under reduced pressure to obtain a crude product, which was purified by silica gel column chromatography (eluent: DCM:MeOH = 20:1, v/v) to afford the title compound as a yellow solid (80 mg, yield: 46.78%). LCMS (ESI): $[M-1]^-$ = 679, $t_R$ = 1.721 min.

*Step 14: Preparation of (3S,8S)-8,15-diethyl-4-fluoro-3,8-dihydroxy-1,2,3,11,14,15-hexahydro-6H,12H-cyclopenta[h]pyrano[3,4:6,7]indazino[2,1-b]quinoline-6,9,12(8H)-trione (Isomer 1 or Isomer 2) and (3R,8S)-8,15-diethyl-4-fluoro-3,8-dihydroxy-1,2,3,11,14,15-hexahydro-6H,12H-cyclopenta[h]pyrano[3,4:6,7] indazino[2,1-b]quinoline-6,9,12(8H)-trione (Isomer 2 or Isomer 1):*

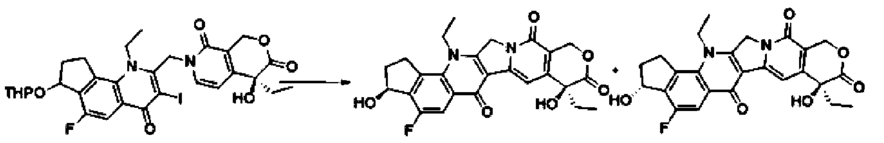

Isomer 1 or Isomer 2 Isomer 2 or Isomer 1

The intermediate obtained from Step 13 (80 mg, 0.24 mmol, 1.0 equiv.) was dissolved in toluene (20 mL). Then azodiisobutyronitrile (AIBN, 29.01 mg, 0.12 mmol, 0.5 eq.alent) and tris(trimethylsilyl)silane ($(TMS)_3SiH$, 87.96 mg, 0.36 mmol, 1.5 eq.) were added sequentially. The reaction system was purged with nitrogen three times to remove air, and then heated to 100 °C under nitrogen protection, followed by stirring for 2 hours. The reaction solution was concentrated under reduced pressure to obtain a crude product, which was subjected to reversed-phase preparative high-performance liquid chromatography (Reversed-Phase Prep-HPLC) for separation and purification to afford the two target isomers as red solids.

**Characterization Data of Isomer 1:** Yield: 20.0% (4.6 mg, red solid). LCMS (ESI): $[M-1]^-$ = 467, $t_R$ = 1.363 min; $^1H$ NMR (400 MHz, DMSO-$d_6$) δ: 7.94 (d, J = 8.8 Hz, 1H), 7.28 (s, 1H), 6.33 (s, 1H), 5.51-5.40 (m, 2H), 5.40-5.24 (m, 3H), 4.59-4.39 (m, 2H), 3.75-3.63 (m, 1H), 2.43-2.34 (m, 1H), 2.12-2.02 (m, 1H), 2.04-1.92 (m, 1H), 1.88-1.75 (m, 2H), 1.41 (t, J = 7.0 Hz, 3H), 0.87 (t, J = 6.0 Hz, 3H).

**Characterization Data of Isomer 2:** Yield: 22.6% (5.2 mg, red solid). LCMS(ESI)$[M-1]^+$ =467, $t_R$=1.390 min; $^1H$ NMR (400 MHz, DMSO-$d_6$) δ 7.94 (d, *J* = 8.8 Hz, 1H), 7.28 (s, 1H), 6.33 (s, 1H), 5.51 - 5.40 (m, 2H), 5.40 - 5.24 (m, 3H), 4.59 - 4.39 (m, 2H), 3.75 - 3.63 (m, 1H), 2.43 - 2.34 (m, 1H), 2.12 - 2.02 (m, 1H), 2.04 - 1.92 (m, 1H), 1.88 - 1.75 (m, 2H), 1.41 (t, *J* =

7.0 Hz, 3H), 0.87 (t, *J* = 6.0 Hz, 3H).

Example 81: Preparation of (S)-9-chloro-4-ethyl-8-fluoro-4-hydroxy-11-(((R)-1-isopropylpyrrolidin-2-yl)methyl)-1,12-dihydro-14H-pyrano[3,4:6,7]indolizino [2,1-b]quinoline-3,6,14(4H,11H)-trione:

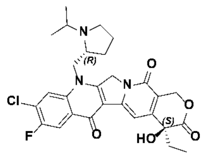

The intermediate (R)-tert-butyl 2-((7-chloro-6-fluoro-2-methyl-4-oxo-1,4-dihydroquinolin-1-yl)methyl)pyrrolidine-1-carboxylate obtained from Example 40 was subjected to dibromination and alkylation reactions with reference to steps 3 and 4 of Example 55, respectively, followed by a cyclization reaction with reference to step 5 of Example 55. Thus, the intermediate (R)-tert-butyl 2-((S)-9-chloro-4-ethyl-8-fluoro-4-hydroxy-3,6,14-trioxo-3,4,6,11,12,14-hexahydro-1H-pyrano[3',4':6,7]indolizino [2,1-b]quinolin-11-yl)methyl)pyrrolidine-1-carboxylate was obtained, and further converted to the intermediate (S)-9-chloro-4-ethyl-8-fluoro-4-hydroxy-11-(((R)-pyrrolidin-2-yl)methyl)-1,12-dihydro-14H-pyrano[3',4':6,7]indolizino[2,1-b]quinoline -3,6,14(4H,11H)-trione.

The intermediate (S)-9-chloro-4-ethyl-8-fluoro-4-hydroxy-11-(((R)-pyrrolidin-2-yl)methyl)-1,12-dihydro-14H-pyrano[3',4':6,7]indolizino[2,1-b]quinoline-3,6,14 (4H,11H)-trione (50 mg, 0.1 mmol, 1.0 eq.) was dissolved in MeOH (MeOH, 10 mL). Then acetone (58 mg, 1.0 mmol, 10.0 eq.) and acetic acid (HAc, 18 mg, 0.3 mmol, 3.0 eq.) were added, and the mixture was stirred at 25 °C for 30 minutes. Subsequently, sodium cyanoborohydride ($NaBH_3CN$, 18 mg, 0.3 mmol, 3.0 eq.) was added at 25 °C, and the reaction was stirred for another 1 hour. LCMS monitoring confirmed the reaction was completed. 0.05 mL of 1 M hydrochloric acid (1 M HCl) was directly added to the reaction mixture, and the product was purified by Prep-HPLC (C18 column, mobile phase: 0.1% hydrochloric acid **in** water-acetonitrile) to afford the title compound as a yellow solid (2.9 mg, yield: 5.3%).

LCMS:(ESI)$[M+H]^+$=542.3, $t_R$=1.366 min; $^1$H-NMR(400 MHz, DMSO-$d_6$+$D_2O$) δ 8.32 (d, *J* = 5.5 Hz, 1H), 8.14 (d, *J* = 9.1 Hz, 1H), 7.25 (s, 1H), 5.39-5.34 (m, 2H), 5.31-5.03 (m, 1H), 4.84-4.74 (m, 1H), 4.56-4.51 (m, 1H), 4.21-4.16(m, 1H), 3.84-3.78 (m, 1H), 3.45-3.37 (m, 1H), 1.94-1.88 (m, 1H), 1.83-1.72 (m, 5H), 1.48 (d, *J* = 6.9 Hz, 2H), 1.31 (d, *J* = 7.1 Hz, 3H), 1.24-1.15 (m, 3H), 0.81 (t, *J* = 7.3 Hz, 3H).

Example 82: Preparation of (S)-9-chloro-4-ethyl-8-fluoro-4-hydroxy-11-(((S)-1-isopropylpyrrolidin-2-yl)methyl)-1,12-dihydro-14H-pyrano[3,4:6,7]indolizino [2,1-b]quinoline-3,6,14(4H,11H)-trione:

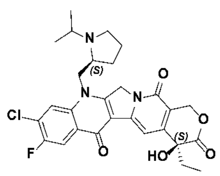

Intermediate 28, namely (Z)-1-(4-chloro-2,5-difluorophenyl)-3-(dimethylamino)but-2-en-1-one, and the commercial reagent (S)-tert-butyl 2-(aminomethyl)pyrrolidine-1-carboxylate were used as the starting materials. The title compound was synthesized with reference to the operation procedure of Example 81, and obtained as a yellow solid. LCMS (ESI): $[M+H]^+$ = 542.3, $t_R$ = 1.349 min. $^1$H NMR (400 MHz, DMSO-$d_6$/$D_2O$) δ: 8.32 (s, 1H), 8.15 (d, J = 9.1 Hz, 1H), 7.27 (s, 1H), 5.38-5.28 (m, 2H), 5.06-5.03 (m, 1H), 4.78-4.73 (m, 1H), 4.25-4.18 (m, 1H), 3.91-3.79 (m, 1H), 3.42-3.41 (m, 1H), 3.33-3.32 (m, 1H), 1.93-1.91 (m, 1H), 1.85-1.77 (m, 5H), 1.53-1.51 (m, 1H), 1.41-1.16 (m, 7H), 0.84 (t, J = 7.3 Hz, 3H).

**Example 83: Preparation of 3-((4S)-4-ethyl-8-fluoro-4-hydroxy-3,6,14-trioxo-4,4a,14,14a-tetrahydro-1H-pyrano [3,4:6,7]indazino[2,1 -b]quinolin-11(3H,6H, 12H)-yl)piperidine-1-carboxylic acid tert-butyl ester:**

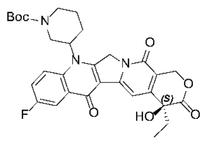

*Step 1: Preparation of (Z)-3-((4-(2,5-difluorophenyl)-4-oxobut-2-en-2-yl)amino)piperidine-1-carboxylic acid tert-butyl ester:*

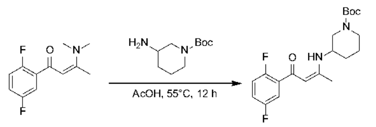


(Z)-1-(2,5-Difluorophenyl)-3-(dimethylamino)but-2-en-1-one (Intermediate 27, from Preparation Example 27, 9.00 g, 39.96 mmol, 1.0 eq.) was dissolved **in** ice-cold acetic acid (100 mL), followed by the addition of tert-butyl 3-aminopiperidine-1-carboxylate (8.00 g, 39.96 mmol, 1.0 eq.). The reaction mixture was stirred at 55 °C for 12 hours, and the completion of the reaction was confirmed by LCMS analysis. After the reaction was completed, the mixture was cooled and poured into water, then extracted with ethyl acetate (100 mL × 3). The combined organic layers were washed with saturated sodium chloride solution (200 mL × 2), dried over anhydrous sodium sulfate, and concentrated under reduced pressure. The crude product was purified by flash chromatography on 200-300 mesh silica gel (eluent: EA:PE = 0% to 50%, v/v) to afford the title compound as a brown oil (12.0 g, yield: 78.94%). LCMS(ESI) $[M+H]^+$ = 302.2.

*Step 2: Preparation of tert-butyl 3-(6-fluoro-2-methyl-4-oxo-1,4-dihydroquinolin-1-yl)-3-methylpiperidine-1-carboxylate:*

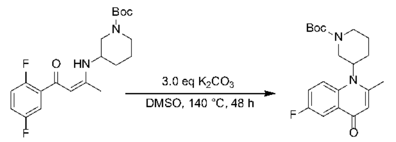


The intermediate obtained from step 1 (5.00 g, 13.14 mmol, 1.0 eq.) was dissolved in dimethyl sulfoxide (DMSO, 50 mL). Potassium carbonate (5.45 g, 39.42 mmol, 3.0 eq.) was added sequentially, and the reaction mixture was stirred at 140 °C for 48 hours. LCMS detection indicated the reaction was complete. The reaction solution was cooled and poured into water, extracted with ethyl acetate (100 mL × 3), washed with saturated sodium chloride solution (200 mL × 2), dried over anhydrous sodium sulfate, and the organic phase was concentrated under reduced pressure. The crude product was separated and purified by flash chromatography (200-300 mesh silica gel, eluent: EA:PE = 0%~100%, v/v) to obtain the title compound as a light yellow solid (3.00 g, yield: 63.25%). LCMS (ESI): $[M+H]^+$ = 361.2.

Using the intermediate obtained in Step 2 (tert-butyl 3-(6-fluoro-2-methyl-4-oxo-1,4-dihydroquinolin-1-yl)-3-methylpiperidine-1-carboxylate) as the raw material, the title compound was obtained as a light yellow solid via bromination, alkylation, and double cyclization reactions with reference to steps 2-5 of Example 8. LCMS (ESI): $[M+H]^+$ = 566.4; $^1H$ NMR (400 MHz, DMSO-$d_6$) δ: 8.37 (d, J = 5.8 Hz, 1H), 8.00 (dd, J = 8.4, 3.2 Hz, 1H), 7.63 (s, 1H), 7.29 (s, 1H), 6.38 (s, 1H), 5.76-5.59 (m, 1H), 5.40-5.24 (m, 3H), 4.31 (d, J = 9.1 Hz, 2H), 3.98-3.83 (m, 2H), 3.21-2.89 (m, 1H), 2.67-2.61 (m, 1H), 2.18-2.08 (m, 1H), 1.86-1.75 (m, 4H), 1.45 (s, 9H), 0.86 (t, J = 7.3 Hz, 3H).

Example 84: Preparation of tert-butyl (S)-[(4-ethyl-8-fluoro-4-hydroxy-11-methyl-3,6,14-trioxo-3,4,6,11,12,14-hexahydro-1H-pyrano[3,4:6,7]indolizino [2,1-b]quinolin-10-yl)methyl](methyl)carbamate:

*Step 1: Preparation of methyl 6-fluoro-1-methyl-4-oxo-8-vinyl-1,4-dihydroquinoline-2-carboxylate:*

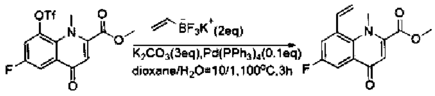


Methyl 6-fluoro-1-methyl-4-oxo-8-(((trifluoromethyl)sulfonyl)oxy)-1,4-dihydroquinoline-2-carboxylate (3.0 g, 7.83 mmol, 1.0 eq.) was dissolved in 1,4-dioxane (60 mL) and water (10 mL). Then potassium trifluoro(vinyl)borate (2.1 g, 15.7 mmol, 2.0 eq.), anhydrous potassium carbonate (3.20 g, 23.5 mmol, 3.0 eq.) and tetrakis(triphenylphosphine) palladium(0) (905 mg, 0.780 mmol, 0.1 eq.) were added sequentially. The reaction system was purged with nitrogen, then heated to 100 °C and stirred for 3 hours. LCMS monitoring indicated the reaction was complete. The mixture was filtered, and the filtrate was concentrated under reduced pressure to obtain a crude product. The crude product was separated and purified by silica gel column chromatography (eluent: PE:EA = 1:0 to 2:1, v/v) to afford the title compound as a light yellow solid (1.94 g, yield: 95%). LCMS (ESI): $[M+H]^+$ = 262.0.

*Step 2: Preparation of methyl 6-fluoro-8-formyl-1-methyl-4-oxo-1,4-dihydroquinoline-2-carboxylate:*

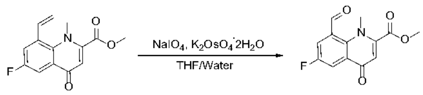


The intermediate obtained from step 1 (2.20 g, 8.42 mmol, 1.0 eq.) was dissolved in tetrahydrofuran (THF, 15 mL) and water (15 mL). Under an ice bath, sodium periodate (5.40 g, 25.3 mmol, 3.0 eq.) was added, followed by potassium osmate dihydrate (189 mg, 0.420 mmol, 0.05 eq.). The reaction was stirred at 25 °C for 1 hour. LCMS monitoring indicated the reaction was complete. The reaction solution was poured into water (50 mL), extracted with ethyl acetate (EA, 30 mL × 3), and the combined organic phases were dried over anhydrous sodium sulfate, filtered, and the filtrate was concentrated to obtain a crude product. The crude product was separated and purified by silica gel column chromatography (eluent: PE:EA = 1:0 to 5:1, v/v) to afford the title compound as a light yellow solid (1.75 g, yield: 78.9%). LCMS (ESI): $[M+H]^+$ = 264.2.

***Step 3: Preparation of methyl 6-fluoro-1-methyl-8-((methylamino)methyl)-4-oxo-1,4-dihydroquinoline-2-carboxylate:***

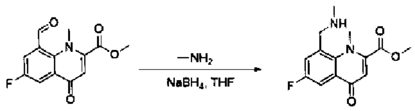


The intermediate obtained from step 2 (1.75 g, 6.84 mmol, 1.0 eq.) was dissolved in tetrahydrofuran (THF, 40 mL). A 2 mol/L methylamine in tetrahydrofuran solution (425 mg, 13.7 mmol, 2.0 eq.) was added, followed by a drop of acetic acid (HAc). The reaction solution was stirred at 25 °C for 0.5 hours. Sodium cyanoborohydride ($NaBH_3CN$, 862 mg, 13.7 mmol, 2.0 eq.) was added, and the mixture was stirred at 25 °C for 16 hours. LCMS monitoring indicated the reaction was incomplete, so sodium borohydride ($NaBH_4$, 517 mg, 13.7 mmol, 2.0 eq.) was added. After LCMS monitoring confirmed the reaction was complete, saturated aqueous ammonium chloride solution (5 mL) was added to the reaction solution to quench the reaction. The mixture was concentrated under reduced pressure to obtain a crude product, which was dissolved in a mixed solvent of DCM and MeOH (MeOH/DCM = 1/10, 55 mL), stirred for 30 minutes, and filtered. The filtrate was concentrated under reduced pressure to obtain the title compound as a crude light yellow solid (1.2 g). LCMS (ESI): $[M+H]^+$ = 279.2.

*Step 4: Preparation of methyl 6-fluoro-1-methyl-8-(((tert-butoxycarbonyl)(methyl)amino)methyl)-4-oxo-1,4-dihydroquinoline-2-carboxylate:*

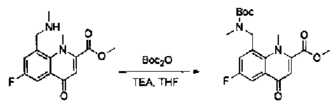


The crude intermediate obtained from step 3 (1.2 g, 4.32 mmol, 1.0 eq.) was dissolved in tetrahydrofuran (THF, 15 mL). Triethylamine (932 mg, 8.63 mmol, 2.0 eq.) and di-tert-butyl dicarbonate (1.31 g, 6.48 mmol, 1.5 eq.) were added sequentially, and the reaction was stirred at 25 °C for 16 hours. The reaction solution was concentrated under reduced pressure, and the residue was separated and purified by silica gel column chromatography (eluent: DCM:MeOH = 1:0 to 25:1, v/v) to afford the title compound as a pale yellow solid (820 mg, two-step yield: 31.8%). LCMS (ESI): $[M+H]^+$ = 379.1; $^1H$ NMR (400 MHz, DMSO-$d_6$) δ: 8.18 (s, 1H), 7.69 (d, J = 2.7 Hz, 1H), 7.49 (d, J = 5.5 Hz, 1H), 5.02 (s, 2H), 3.05 (s, 3H), 1.57 (s, 9H), 1.45 (s, 3H), 1.30 (s, 3H).

***Step 5: Preparation of tert-butyl ((6-fluoro-2-(hydroxymethyl)-1-methyl-4-oxo-1,4-dihydroquinolin-8-yl)methyl) (methyl)carbamate:***

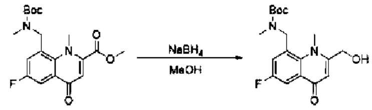


The intermediate obtained from step 4 (820 mg, 2.17 mmol, 1.0 eq.) was dissolved in MeOH (MeOH, 15 mL). Under an ice bath, sodium borohydride ($NaBH_4$, 163.96 mg, 4.33 mmol, 2.0 eq.) was added slowly, and the reaction solution was continuously stirred at 0 °C for 2 hours. The reaction was quenched with saturated aqueous sodium chloride solution (3 mL), and the mixture was concentrated under reduced pressure to obtain a crude product. The crude product was separated and purified by silica gel column chromatography (eluent: DCM:MeOH = 1:0 to 25:1, v/v) to afford the title compound as a white solid (560 mg, yield: 73.8%). LCMS (ESI): $[M+H]^+$ = 351.1; $^1H$ NMR (400 MHz, DMSO-$d_6$) δ: 7.68 (dd, J = 8.3, 3.2 Hz, 1H), 7.17 (s, 1H), 6.26 (s, 1H), 5.76 (t, J = 5.7 Hz, 1H), 4.80 (s, 2H), 4.57 (d, J = 5.8 Hz, 2H), 3.69 (s, 3H), 2.80 (d, J = 32.0 Hz, 3H), 1.60 - 1.01 (m, 9H).

*Step 6: Preparation of tert-butyl ((3-bromo-6-fluoro-2-(hydroxymethyl)-1-methyl-4-oxo-1,4-dihydroquinolin-8-yl)methyl) (methyl)carbamate:*

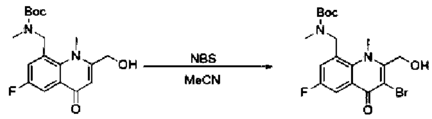


The intermediate obtained from step 5 (200 mg, 0.4 mmol, 1.0 eq.) was dissolved in acetonitrile (5 mL). N-Bromosuccinimide (NBS, 85.9 mg, 0.48 mmol, 1.2 eq.) was added, and the reaction was stirred at room temperature for 2 hours. The crude product was separated and purified by silica gel column chromatography (eluent: DCM:MeOH = 1:0 to 25:1, v/v) to afford the title compound as a yellow solid (170 mg, yield: 69.25%). LCMS (ESI): $[M+H]^+$ = 429.3; $^1H$ NMR (400 MHz, DMSO-$d_6$) δ: 7.73 (dd, J = 8.4, 3.1 Hz, 1H), 7.24 (s, 1H), 5.90 (s, 1H), 4.94 (d, J = 4.6 Hz, 2H), 4.79 (s, 2H), 3.88 (s, 3H), 2.80 (d, J = 29.6 Hz, 3H), 1.45 - 1.13 (m, 9H).

*Step 7: Preparation of tert-butyl ((3-bromo-2-(chloromethyl)-6-fluoro-1-methyl-4-oxo-1,4-dihydroquinolin-8-yl)methyl) (methyl)carbamate:*

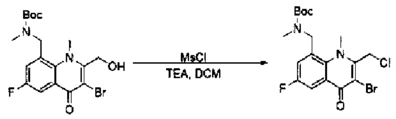

The intermediate obtained from step 6 (140 mg, 0.330 mmol, 1.0 eq.) was dissolved **in** anhydrous DCM (5 mL). Under ice bath conditions, triethylamine (99.0 mg, 0.980 mmol, 3.0 eq.) and methanesulfonyl chloride (74.7 mg, 0.650 mmol, 2.0 eq.) were added sequentially, and stirring was continued under ice bath for 30 minutes. LCMS detection indicated the reaction was complete. The reaction solution was poured into ice water (20 mL), extracted with DCM (15 mL × 2), and the combined organic phases were dried over anhydrous sodium sulfate, filtered, and the filtrate was concentrated to obtain the title compound as a crude brown oil (140 mg). LCMS (ESI): $[M+H]^+$ = 447.0.

*Step 8: Preparation of tert-butyl (S)-[(3-bromo-6-fluoro-2-((4-ethyl-4-hydroxy-3,8-dioxo-4,8-dihydro-1H-pyrano[3,4-c] pyridin-7(3H)-yl)methyl)-1-methyl-4-oxo-1,4-dihydroquinolin-8-yl)methyl](methyl)carbamate:*

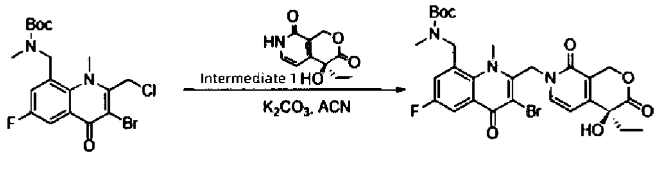


The intermediate obtained from step 7(140 mg, 0.31 mmol, 1.0 eq.) and Intermediate 1 (52.3 mg, 0.25 mmol, 0.8 eq.) were dissolved in acetonitrile (5 mL). Potassium carbonate (130 mg, 0.940 mmol, 3.0 eq.) was added, and the mixture was heated to 35 °C and stirred for 18 hours. The crude product was purified by silica gel column chromatography (eluent: DCM:MeOH = 1:0 to 20:1, v/v) to afford the title compound as a white solid (92.0 mg, two-step yield: 45.0%). LCMS (ESI): $[M+H]^+$ = 620.2; $^1H$ NMR (400 MHz, DMSO-$d_6$) δ: 7.72 (dd, J = 8.2, 3.3 Hz, 2H), 7.24 (s, 1H), 6.46 (d, J = 7.2 Hz, 1H), 6.38 (s, 1H), 5.54 (s, 2H), 5.26 (s, 2H), 4.65 - 4.50 (m, 2H), 3.65 (s, 3H), 2.74 (d, J = 10.0 Hz, 3H), 1.77 (dd, J = 14.2, 7.2 Hz, 2H), 1.24 (s, 9H), 0.80 (t, J = 7.3 Hz, 3H).

*Step 9: Preparation of tert-butyl (S)-[(4-ethyl-8-jluoro-4-hydroxy-11-methyl-3,6,14-trioxo-3,4,6,11,12,14-hexahydro-1H-pyrano[3,4:6,7]indolizino[2,1 -b]quinolin-10-yl)methyl](methyl)carbamate:*

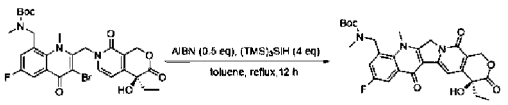


The intermediate obtained from step 8 (140 mg, 0.230 mmol, 1.0 eq.) and azobisisobutyronitrile (AIBN, 18.5 mg, 0.110 mmol, 0.5 eq.) were added sequentially to a dry three-necked flask. The flask was evacuated with an oil pump and protected with argon, then anhydrous toluene (30 mL) and tris(trimethylsilyl)silane ($(TMS)_3SiH$, 224 mg, 0.920 mmol, 4.0 eq.) were added to the reaction mixture. The mixture was heated to 100 °C and stirred for 12 hours. The reaction solution was concentrated under reduced pressure to obtain a crude product, which was separated and purified by silica gel column chromatography (eluent: DCM:MeOH = 1:0 to 15:1, v/v) to afford a crude title compound. Preparative HPLC purification gave the title compound as a white solid (12 mg, yield: 9.86%). LCMS (ESI) $[M+1]^+$=540.3; $^1H$ NMR (400 MHz, DMSO-$d_6$) δ 7.95 (dd, $J$ = 8.1, 3.2 Hz, 1H), 7.30 (d, $J$ = 8.9 Hz, 1H), 7.26 (s, 1H), 6.37 (s, 1H), 5.45 - 5.29 (m, 4H), 5.01 (s, 2H), 4.05 (s, 3H), 2.85 (s, 3H), 1.88 - 1.78 (m, 2H), 1.38 (s, 9H), 0.87 (t, $J$ = 7.3 Hz, 3H).

Example 85: Preparation of (S)-4-ethyl-8-fluoro-4-hydroxy-11-methyl-10-((methylamino)methyl)-1,12-dihydro-14H-pyrano[3,4:6,7]indolizino[2,1 -b] quinoline-3,6,14(4H,11H)-trione:

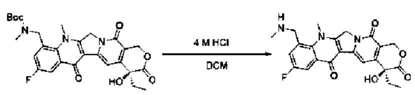


The compound obtained from Example 84 (12 mg, 0.02 mmol, 1.0 eq.) was dissolved in anhydrous DCM (0.5 mL). Under ice bath conditions, a 4 mol/L hydrogen chloride in dioxane solution (0.5 mL) was added slowly. The ice bath was removed, and the reaction was continued at room temperature for 30 minutes, with solids precipitated. LCMS detection indicated the reaction was complete. The reaction solution was allowed to stand for 15 minutes, the solvent was

removed, and the remaining solids were lyophilized to obtain the title compound hydrochloride as a yellow solid (6.5 mg, yield: 50.0%). LCMS (ESI): $[M+H]^+$ = 440.1; $^1H$ NMR (400 MHz, DMSO-$d_6$) δ: 9.22 (s, 1H), 8.10 (dd, J = 8.1, 3.2 Hz, 1H), 8.00 (dd, J = 9.0, 3.2 Hz, 1H), 7.26 (s, 1H), 6.39 (s, 1H), 5.43 (s, 2H), 5.39 - 5.32 (m, 2H), 4.79 (t, J = 5.6 Hz, 2H), 4.08 (s, 3H), 2.66 (q, J = 5.0, 4.5 Hz, 3H), 1.88 - 1.76 (m, 2H), 0.87 (t, J = 7.3 Hz, 3H).

Example 86: Preparation of (S)-10-((dimethylamino)methyl)-4-ethyl-8-fluoro-4-hydroxy-11-methyl-1,12-dihydro-14H-pyrano[3,4:6,7]indolizino[2,1 -b]quinoline -3,6,14(4H,11H)-trione:

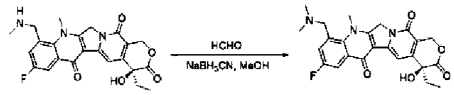


The compound obtained from Example 84 (45.0 mg, 0.103 mmol, 1.0 eq.) was dissolved in MeOH (MeOH, 3 mL). A 37% (mass fraction) aqueous formaldehyde solution (24.9 mg, 0.308 mmol, 3.0 eq.) was added. After the addition, the reaction was stirred at room temperature for 30 minutes. Under ice bath conditions, sodium cyanoborohydride ($NaBH_3CN$, 38.7 mg, 0.630 mmol, 6.1 eq.) was added slowly, and the stirring reaction was continued under ice bath conditions for 30 minutes. LCMS detection indicated the reaction was complete. The reaction was quenched with a 4 mol/L hydrogen chloride in dioxane solution (0.2 mL), and the reaction solution was concentrated under reduced pressure to obtain a crude product. The crude product was separated and purified by reversed-phase preparative HPLC to afford the title compound hydrochloride as a pale pink solid (6.9 mg, yield: 14.8%). LCMS (ESI): $[M+H]^+$ = 454.2; $^1H$ NMR (400 MHz, DMSO-$d_6$) δ: 10.41 (s, 1H), 8.11 (dd, J = 18.1, 8.4 Hz, 2H), 7.25 (s, 1H), 6.39 (s, 1H), 5.46 - 5.40 (m, 2H), 5.39 - 5.31 (m, 2H), 5.00 - 4.87 (m, 2H), 4.08 (s, 3H), 2.65 (s, 6H), 1.92 - 1.77 (m, 2H), 0.87 (t, J = 7.3 Hz, 3H).

Example 87: Preparation of tert-butyl (S)-[(4-ethyl-8-fluoro-4-hydroxy-11-methyl-3,6,14-trioxo-3,4,6,11,12,14-hexahydro-1H-pyrano[3,4:6,7]indolizino [2,1-b]quinolin-10-yl)methyl](cyclopropyl)carbamate

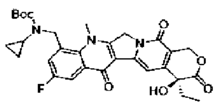

Using the intermediate obtained from step 2 of Example 84 and cyclopropylamine as starting materials, the title compound was synthesized with reference to the operation procedures of steps 3~9 of Example 84, and obtained as an off-white solid. LCMS (ESI): $[M+H]^+$ = 566.4; $^1H$ NMR (400 MHz, DMSO-$d_6$) δ: 7.93 (dd, J = 7.9, 2.8 Hz, 1H), 7.38 (s, 1H), 7.25 (d, J = 3.5 Hz, 1H), 7.12 (s, 1H), 6.38 (s, 1H), 5.48 - 5.23 (m, 3H), 4.96 (s, 1H), 4.06 (s, 2H), 3.33 (s, 3H), 2.10 - 1.94 (m, 1H), 1.90 - 1.75 (m, 2H), 1.56 - 1.13 (m, 11H), 0.88 (t, J = 7.0 Hz, 3H), 0.71 - 0.57 (m, 2H).

Example 88: Preparation of (S)-10-((cyclopropylamino)methyl)-4-ethyl-8-fluoro-4-hydroxy-11-methyl-1,12-dihydro-14H-pyrano[3,4:6,7]indolizino[2,1 -b] quinoline-3,6,14(4H,11H)-trione:

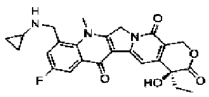

Using the compound obtained from Example 87 as the starting material, the title compound hydrochloride was synthesized with reference to the operation procedures of Example 85, and obtained as a white solid. LCMS (ESI): $[M+H]^+$ = 466.3; $^1H$ NMR (400 MHz, DMSO-$d_6$) δ: 9.81 (s, 2H), 8.14 (dd, J = 9.0, 3.1 Hz, 1H), 8.08 (dd, J = 8.1, 3.1 Hz, 1H), 7.25 (s, 1H), 5.43 (s, 2H), 5.39 - 5.31 (m, 2H), 4.89 (s, 2H), 4.12 (s, 3H), 2.81 - 2.69 (m, 1H), 1.92 - 1.75 (m, 2H), 0.99 - 0.92 (m, 2H), 0.87 (t, J = 7.3 Hz, 3H), 0.79 - 0.69 (m, 2H).

Example 89: Preparation of (S)-10-((cyclopropyl(methyl)amino)methyl)-4-ethyl-8-fluoro-4-hydroxy-11-methyl-1,12-dihydro-14H-pyrano[3,4:6,7]indolizino[2,1 -b]quinoline-3,6,14(4H,11H)-trione:

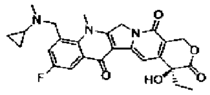

Using the compound obtained from Example 88 and formaldehyde as the starting materials, the title compound hydrochloride was synthesized with reference to the operation procedures of Example 86, and obtained as an off-white solid. LCMS (ESI): $[M+H]^+$ = 480.0; $^1H$ NMR (400 MHz, DMSO-$d_6$) δ: 10.35 (s, 1H), 8.26 - 7.80 (m, 2H), 7.25 (s, 1H), 5.48 - 5.28 (m, 4H), 5.08 (d, J = 46.8 Hz, 2H), 4.14 (s, 3H), 2.79 (s, 3H), 1.88 - 1.77 (m, 2H), 0.87 (t, J = 7.3 Hz, 4H), 0.63 - 0.55 (m, 1H), 0.43 - 0.07 (m, 3H).

Example 90: Preparation of (S)-10-((cyclopropyl(isobutyl)amino)methyl)-4-ethyl-8-fluoro-4-hydroxy-11-methyl-1,12-dihydro-14H-pyrano[3,4:6,7]indolizino[2,1 -b]quinoline-3,6,14(4H,11H)-trione:

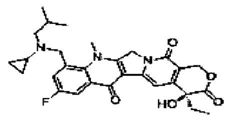

Using the compound obtained from Example 88 and isobutyraldehyde as the starting materials, the title compound hydrochloride was synthesized with reference to the operation procedures of Example 89, and obtained as a white solid. LCMS (ESI): $[M+H]^+$ = 522.0; $^1H$ NMR (400 MHz, DMSO-$d_6$) δ: 9.83 (s, 1H), 8.24 - 7.53 (m, 2H), 7.12 (s, 1H), 6.55 (s, 1H), 5.46 - 5.08 (m, 4H), 4.95 (d, J = 67.8 Hz, 1H), 4.05 (d, J = 40.5 Hz, 3H), 3.01 - 2.84 (m, 1H), 2.19 (d, J = 12.6 Hz, 1H), 1.93 - 1.81 (m, 1H), 1.79 - 1.62 (m, 2H), 1.40 - 1.30 (m, 1H), 1.22 - 0.99 (m, 3H), 0.96 - 0.58 (m, 8H), 0.32 - -0.07 (m, 3H).

Example 91: Preparation of (S)-10-((cyclopropyl(3,3-difluorocyclobutyl) amino)methyl)-4-ethyl-8-fluoro-4-hydroxy-11-methyl-1,12-dihydro-14H-pyrano [3,4:6,7]indolizino[2,1 -b]quinoline-3,6,14(4H,11H)-trione:

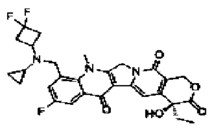

Using the compound obtained from Example 88 and 3,3-difluorocyclobutanone as the starting materials, the title compound hydrochloride was synthesized with reference to the operation procedures of Example 89, and obtained as a white solid. LCMS (ESI): $[M+H]^+$ = 556.3; $^1H$ NMR (400 MHz, DMSO-$d_6$) δ: 8.02 (dd, J = 8.1, 3.1 Hz, 1H), 7.90 (d, J = 8.9 Hz, 1H), 7.26 (s, 1H), 6.66 (s, 1H), 5.49 - 5.21 (m, 4H), 4.34 - 4.10 (m, 5H), 2.65 (d, J = 33.5 Hz, 2H), 2.09 - 1.96 (m, 1H), 1.91 - 1.76 (m, 2H), 1.55 - 1.41 (m, 1H), 0.96 - 0.62 (m, 3H), 0.32 (d, J = 6.1 Hz, 2H), 0.18 - 0.06 (m, 2H).

Example 92: Preparation of (S)-10-((cyclopropyl(4-hydroxycyclohexyl)amino) methyl)-4-ethyl-8-fluoro-4-hydroxy-11-methyl-1,12-dihydro-14H-pyrano[3,4: 6,7]indolizino[2,1-b]quinoline-3,6,14(4H,11H)-trione:

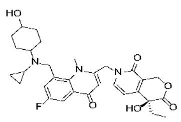

The compound obtained from Example 88 (30 mg, 0.06 mmol, 1.0 eq.) was dissolved in MeOH (MeOH, 3 mL). Acetic acid (HAc, 11.61 mg, 0.19 mmol, 3.0 eq.) and 4-hydroxycyclohexan-1-one (14.71 mg, 0.13 mmol, 2.0 eq.) were added sequentially, and the reaction was continued at room temperature for 2 hours. Under ice bath conditions, sodium cyanoborohydride ($NaBH_3CN$, 11.96 mg, 0.19 mmol, 3.0 eq.) was added slowly, and the reaction was continued at room temperature for 1 hour. LCMS detection indicated the reaction was complete. The reaction was quenched with 1 mol/L dilute hydrochloric acid (0.5 mL), water was added, and the mixture was lyophilized to obtain a crude product. The crude product was purified by preparative HPLC to afford the title compound as a pale orange solid (2.8 mg, yield: 8.28%). LCMS (ESI): $[M+H]^+$ = 564.4; $^1H$ NMR (400 MHz, DMSO-$d_6$) δ: 8.13 (s, 1H), 8.06 - 7.90 (m, 1H), 7.26 (s, 1H), 5.81 - 5.04 (m, 4H), 4.88 (s, 2H), 4.11 (s, 3H), 2.15 - 1.68 (m, 10H), 1.58 - 1.40 (m, 1H), 1.38 - 1.20 (m, 2H), 0.90 - 0.74 (m, 4H), 0.63 - 0.08 (m, 3H).

Example 93: Preparation of (S)-11-(((R)-4,4-difluoropyrrolidin-2-yl)methyl)-4-ethyl-8-fluoro-4-hydroxy-1,12-dihydro-14H-pyrano[3,4:6,7]indolizino[2,1 -b]quinoline-3,6,14(4H,11H)-trione:

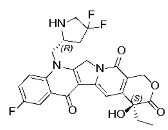

Intermediate (Z)-1-(2,5-difluorophenyl)-3-(dimethylamino)but-2-en-1-one (Intermediate 27) was prepared with reference to Example 27. Using this intermediate and the commercial reagent tert-butyl (S)-2-(aminomethyl)-4,4-difluoropyrrolidine-1-carboxylate as starting materials, the title compound was synthesized with reference to the synthetic methods of Steps 1~5 of Example 55, and finally the Boc protecting group was removed under acidic conditions to obtain the title compound. LCMS (ESI): $[M+H]^+$ = 502.2.

Example 94: Preparation of (S)-11-(((R)-4,4-difluoro-1-methylpyrrolidin-2-yl)methyl)-4-ethyl-8-fluoro-4-hydroxy-1,12-dihydro-14H-pyrano[3,4:6,7] indolizino[2,1-b]quinoline-3,6,14(4H,11H)-trione:

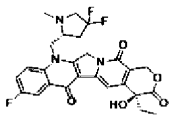

The compound obtained from Example 93 (10 mg, crude) was dissolved **in** N,N-dimethylformamide (DMF, 2 mL), and iodomethane (2 drops) was added. After the addition, the reaction solution was stirred at room temperature for 24 hours. LCMS monitoring indicated the reaction was complete. The reaction solution was separated and purified by reversed-phase preparative HPLC to afford the title compound as a white solid (1.2 mg, yield: 23.2%). LCMS (ESI): $[M+H]^+$ = 516.0.

Example 95: Preparation of tert-butyl (S)-cyclopropyl(2-(4-ethyl-8-fluoro-4-hydroxy-3,6,14-trioxo-4,6,12,14-tetrahydro-1H-pyrano[3,4:6,7]indolizino[2,1 -b]quinolin-11(3H)-yl)ethyl)carbamate:

*Step 1: Preparation of tert-butyl (2-(3-bromo-6-fluoro-2-methyl-4-oxoquinolin-1(4H)-yl)ethyl)(cyclopropyl)carbamate:*

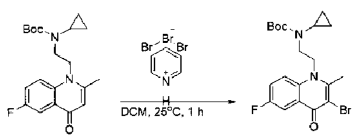


Intermediate 35 obtained from Preparation 35 (9.60 g, 26.6 mmol, 1.0 eq.) was dissolved in anhydrous DCM (100 mL). Tribromopyridine (10.2 g, 26.64 mmol, 1.0 eq.) was added, and the mixture was stirred at room temperature for 1 hour. The reaction solution was poured into ice-cold saturated aqueous sodium bicarbonate solution (100 mL), extracted with

DCM (50 mL × 2), and the combined organic phases were washed with saturated aqueous sodium chloride solution (100 mL), dried over anhydrous sodium sulfate, and filtered. The filtrate was concentrated to obtain a crude product, which was separated and purified by silica gel column chromatography (eluent: DCM:MeOH = 1:0 to 50:1, v/v) to afford the title compound as a pale yellow solid (11.23 g, yield: 96.0%). LCMS (ESI) $[M+1]^+$ =439.0/441.0;$^1$H NMR (400 MHz, $CDCl_3$) δ 8.07 (dd, *J* = 8.7, 3.1 Hz, 1H), 7.79 (s, 1H), 7.41 (t, *J* = 6.9 Hz, 1H), 4.43 (t, *J* = 7.0 Hz, 2H), 3.71 - 3.50 (m, 2H), 2.85 (s, 3H), 2.58 - 2.31 (m, 1H), 1.51 (s, 9H), 0.79 - 0.62 (m, 2H), 0.56 - 0.35 (m, 2H).

*Step 2: Preparation of tert-butyl (2-(3-bromo-2-(bromomethyl)-6-fluoro-4-oxoquinolin-1(4H)-yl)ethyl)(cyclopropyl)carbamate:*

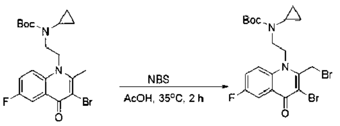


The intermediate from step1 (7.34 g, 16.68 mmol, 1.0 eq.) was dissolved in glacial acetic acid (80 mL). N-Bromosuccinimide (NBS, 3.27 g, 18.35 mmol, 1.1 eq.) was added, and the reaction solution was heated to 30 °C and stirred for 2 hours. LCMS detection indicated the reaction was complete. The reaction solution was poured into ice-cold saturated aqueous sodium bicarbonate solution (150 mL), extracted with DCM (DCM, 200 mL × 2), and the combined organic phases were washed with saturated aqueous sodium chloride solution (300 mL), dried over anhydrous sodium sulfate, and filtered. The filtrate was concentrated under reduced pressure to obtain a crude product, which was separated and purified by silica gel column chromatography (eluent: DCM:MeOH = 1:0 to 50:1, v/v) to afford the title compound as a pale yellow solid (4.5 g, yield: 51.9%). LCMS (ESI): $[M+H]^+$ = 518.8; $^1$H NMR (400 MHz, $CDCl_3$) δ: 8.07 (dd, J = 8.5, 2.9 Hz, 1H), 7.82 (s, 1H), 7.48 (t, J = 6.8 Hz, 1H), 5.19 - 4.71 (m, 2H), 4.53 (t, J = 7.6 Hz, 2H), 3.65 (s, 2H), 2.62 - 2.42 (m, 1H), 1.52 (s, 9H), 0.88 - 0.72 (m, 2H), 0.60 - 0.44 (m, 2H).

*Step 3: Preparation of tert-butyl (S)-(2-(3-bromo-2-((4-ethyl-4-hydroxy-3,8-dioxo-4,8-dihydro-1H-pyrano[3,4-c]pyridin-7(3H)-yl)methyl)-6-fluoro-4-oxoquinolin-1(4H)-yl)ethyl)(cyclopropyl)carbamate.*

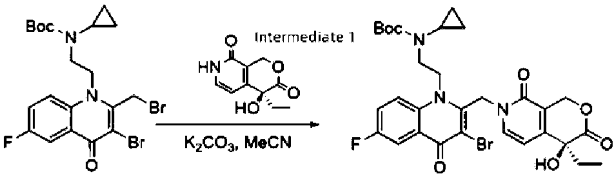


The intermediate obtained from Step 2 (2.00 g, 3.86 mmol, 1.0 eq.) was dissolved in acetonitrile (25 mL). Then Intermediate 1 (807 mg, 3.86 mmol, 1.0 eq.) and potassium carbonate (1.60 g, 11.6 mmol, 3.0 eq.) were added sequentially. The reaction system was protected with nitrogen, heated to 30 °C and stirred for 16 hours. The crude product was separated and purified by silica gel column chromatography (eluent: PE:EtOAc = 1:0 to 100:1, v/v) to afford the title compound as a white solid (1.40 g, yield: 56.1%). LCMS (ESI) $[M+1]^+$ =646.2; $^1$H NMR (400 MHz, $CDCl_3$) δ 8.11 (dd, *J* = 8.5, 3.0 Hz, 1H), 8.02 (s, 1H), 7.54 - 7.44 (m, 1H), 7.35 (d, *J* = 7.0 Hz, 1H), 6.55 (d, *J* = 7.2 Hz, 1H), 5.98 - 5.52 (m, 3H), 5.19 (d, *J* = 16.3 Hz, 1H), 4.59 - 4.20 (m, 2H), 3.64 - 3.24 (m, 2H), 2.59 - 2.44 (m, 1H), 1.89 - 1.74 (m, 2H), 1.46 (s, 9H), 0.96 (t, *J* = 7.4 Hz, 3H), 0.82 - 0.70 (m, 2H), 0.58 - 0.43 (m, 2H).

*Step 4: Preparation of tert-butyl (S)-cyclopropyl(2-(4-ethyl-8-fluoro-4-hydroxy-3,6,14-trioxo-4,6,12,14-tetrahydro-1H-pyrano[3,4:6,7]indolizino[2,1 -b]quinolin-11(3H)-yl)ethyl)carbamate:*

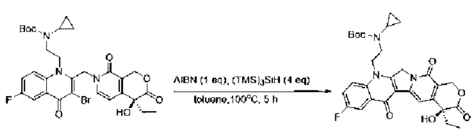

The intermediate obtained from Step 3 (800 mg, 1.24 mmol, 1.0 eq.) was dissolved in toluene (80 mL). Azobisisobutyronitrile (AIBN, 203 mg, 1.24 mmol, 1.0 eq.) and tris(trimethylsilyl)silane ($(TMS)_3SiH$, 1.23 g, 4.95 mmol, 4.0 eq.) were added sequentially. The reaction system was protected with nitrogen, heated to 100 °C and stirred for 5 hours. The crude product was separated and purified by silica gel column chromatography (eluent: DCM:MeOH = 1:0 to 20:1, v/v) to afford the title compound as an orange solid (197 mg, yield: 28.0%). LCMS (ESI): $[M+H]^+$ = 566.3; $^1H$ NMR (400 MHz, $CDCl_3$) δ: 7.88 (s, 1H), 7.68 (dd, J = 8.3, 2.6 Hz, 1H), 7.50 - 7.43 (m, 1H), 7.35 (s, 1H), 5.59 (d, J = 15.9 Hz, 1H), 5.26 (d, J = 19.9 Hz, 1H), 5.16 - 5.04 (m, 2H), 4.45 - 4.31 (m, 2H), 3.78 (t, J = 7.4 Hz, 2H), 2.57 - 2.49 (m, 1H), 1.90 - 1.77 (m, 2H), 1.51 (s, 9H), 0.98 (t, J = 7.4 Hz, 3H), 0.85 - 0.76 (m, 2H), 0.61 - 0.55 (m, 2H).

Example 96: Preparation of (S)-11-(2-(cyclopropylamino)ethyl)-4-ethyl-8-fluoro-4-hydroxy-1,12-dihydro-14H-pyrano [3,4:6,7]indolizino[2,1 -b]quinoline-3,6,14 (4H,11H)-trione:

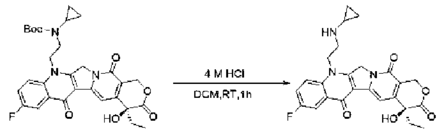


The compound obtained from Example 95 (200 mg, 0.354 mmol) was dissolved **in** DCM (2 mL). Under ice bath conditions, a 4 mol/L hydrogen chloride **in** dioxane solution (4 mL) was added slowly. The ice bath was removed, and the stirring reaction was continued at room temperature for 1 hour. LCMS detection indicated the reaction was complete, and the title compound was purified by preparative HPLC to obtain an orange solid (119 mg, yield: 72%). LCMS (ESI) $[M+1]^+$ =466.3; $^1H$ NMR (400 MHz, DMSO-$d_6$) δ 9.38 (s, 2H), 8.24 (dd, *J* = 9.4, 4.1 Hz, 1H), 8.02 (dd, *J* = 8.8, 3.1 Hz, 1H), 7.86 - 7.77 (m, 1H), 7.30 (s, 1H), 6.38 (s, 1H), 5.50 (s, 2H), 5.42 - 5.27 (m, 2H), 4.75 - 4.63 (m, 2H), 3.56 - 3.45 (m, 2H), 2.85 - 2.75 (m, 1H), 1.89 - 1.77 (m, 2H), 1.00 - 0.92 (m, 2H), 0.86 (t, *J* = 7.3 Hz, 3H), 0.80 - 0.73 (m, 2H).

Example 97: Preparation of (S)-11-(2-(cyclopropyl(methyl)amino)ethyl)-4-ethyl-8-fluoro-4-hydroxy-1,12-dihydro-14H-pyrano[3,4:6,7]indolizino[2,1 -b]quinoline-3,6,14(4H,11H)-trione:

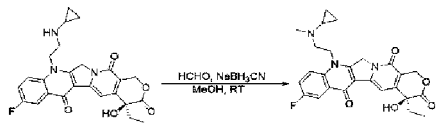


The compound obtained from Example 96 (110 mg, 0.24 mmol, 1.0 eq.) was dissolved in anhydrous MeOH (5 mL). Under ice bath conditions, a 37% (mass fraction) aqueous formaldehyde solution (39 mg, 0.47 mmol, 2.0 eq.) was added, and the reaction was continued under ice bath for 0.5 hours. Sodium cyanoborohydride ($NaBH_3CN$, 38 mg, 0.59 mmol, 2.5 eq.) was added slowly, the ice bath was removed, and the stirring reaction was continued at room temperature for 1 hour. LCMS detection indicated the reaction was complete. A 1 mol/L aqueous hydrochloric acid solution was added to the reaction solution to adjust the pH to 4~5, stirred for 20 minutes, and a solid precipitated. The mixture was filtered, the filter cake was washed with MeOH, and lyophilized to obtain the title compound as a pink solid (25 mg, yield: 21.7%). LCMS (ESI): $[M+H]^+$ = 480.4; $^1H$ NMR (400 MHz, DMSO-$d_6$) δ: 10.85 (s, 1H), 8.35 - 8.17 (m, 1H), 8.02 (dd, J = 8.8, 3.1 Hz, 1H), 7.87 - 7.73 (m, 1H), 7.30 (s, 1H), 6.38 (s, 1H), 5.53 (s, 2H), 5.44 - 5.24 (m, 2H), 4.99 - 4.69 (m, 2H), 3.87 - 3.57 (m, 2H), 3.12 - 2.82 (m, 4H), 1.92 - 1.75 (m, 2H), 1.31 - 0.92 (m, 4H), 0.87 (t, J = 7.3 Hz, 3H).

Example 99: Preparation of (S)-11-(2-(cyclopropyl(propyl)amino)ethyl)-4-ethyl-8-fluoro-4-hydroxy-1,12-dihydro-14H-pyrano[3,4:6,7]indolizino[2,1 -b]quinoline-3,6,14(4H,11H)-trione:

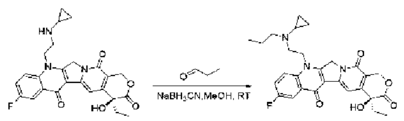

The compound obtained from Example 96 (80 mg, 0.17 mmol, 1.0 eq.) was dissolved in MeOH (3 mL). Propionaldehyde (20.0 mg, 0.34 mmol, 2.0 eq.) and glacial acetic acid (HAc, 31.0 mg, 0.54 mmol, 3.0 eq.) were added sequentially, and the mixture was stirred at room temperature for 1 hour. Under ice bath conditions, sodium cyanoborohydride ($NaBH_3CN$, 31.9 mg, 0.52 mmol, 3.0 eq.) was added slowly, and the stirring reaction was continued at room temperature for 1 hour. LCMS detection indicated the reaction was complete. A 1 mol/L dilute hydrochloric acid (0.5 mL) was added to quench the reaction, and a solid precipitated. The mixture was filtered, the filter cake was rinsed with MeOH, and lyophilized to obtain the title compound as a pale pink solid (6.44 mg, yield: 7.38%). LCMS (ESI): $[M+H]^+$ = 508.5; $^1H$ NMR (400 MHz, DMSO-$d_6$) δ: 8.01 (dd, J = 8.9, 3.0 Hz, 2H), 7.86 - 7.73 (m, 1H), 7.30 (s, 1H), 6.37 (s, 1H), 5.44 (s, 2H), 5.39 - 5.29 (m, 2H), 4.52 (s, 2H), 3.04 (s, 2H), 2.62 (s, 2H), 1.99 - 1.90 (m, 1H), 1.89 - 1.78 (m, 2H), 1.55 - 1.44 (m, 2H), 0.96 - 0.75 (m, 6H), 0.46 - 0.34 (m, 2H), 0.16 - 0.02 (m, 2H).

Example 100: Preparation of (S)-11-(2-(cyclopropyl(isobutyl)amino)ethyl)-4-ethyl-8-fluoro-4-hydroxy-1,12-dihydro-14H-pyrano[3,4:6,7]indolizino[2,1-b]quinoline-3,6,14(4H,11H)-trione:

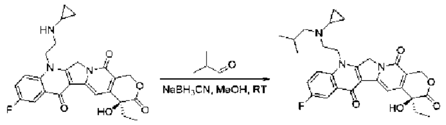


The compound obtained from Example 96 (80 mg, 0.17 mmol, 1.0 eq.) was dissolved **in** MeOH (3 mL), followed by the sequential addition of isobutyraldehyde (24.8 mg, 0.34 mmol, 2.0 eq.) and glacial acetic acid (HAc, 31.0 mg, 0.52 mmol, 3.0 eq.). After the addition, the reaction was stirred at room temperature for 1 hour, then sodium cyanoborohydride ($NaBH_3CN$, 31.9 mg, 0.52 mmol, 3.0 eq.) was added slowly under ice-bath conditions. The reaction was continued to stir at room temperature for 1 hour. LCMS analysis confirmed the reaction was complete, and the reaction was quenched by adding a 1 mol/L dilute hydrochloric acid (0.5 mL). A solid precipitated, which was filtered, and the filter cake was washed with MeOH. The title compound was obtained as a pale pink solid after lyophilization (5.38 mg, yield: 6.00%). LCMS (ESI): $[M+H]^+$ = 522.0.

Example 101: Preparation of (S)-11-(2-(cyclopropyl(3,3-difluorocyclobutyl) amino)ethyl)-4-ethyl-8-fluoro-4-hydroxy-1,12-dihydro-14H-pyrano[3,4:6,7] indolizino[2,1-b]quinoline-3,6,14(4H,11H)-trione:

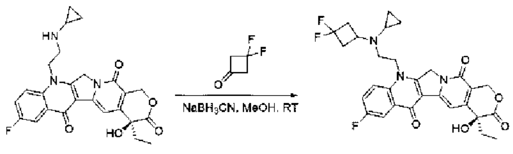


he compound obtained from Example 96 (80 mg, 0.17 mmol, 1.0 eq.) was dissolved in MeOH (3 mL). 3,3-Difluorocyclobutanone (36.5 mg, 0.34 mmol, 2.0 eq.) and glacial acetic acid (HAc, 31.0 mg, 0.54 mmol, 3.0 eq.) were added sequentially, and the mixture was stirred at room temperature for 1 hour. Sodium cyanoborohydride ($NaBH_3CN$, 31.9 mg, 0.52 mmol, 3.0 eq.) was added slowly under ice bath conditions, and the stirring reaction was continued at room temperature for 1 hour. LCMS detection indicated the reaction was incomplete; additional glacial acetic acid and 3,3-difluorocyclobutanone were added, and the reaction was monitored every 1 hour until completion. A 1 mol/L dilute hydrochloric acid (0.5 mL) was added to quench the reaction, and water was added to the mixture, which was then lyophilized to obtain a crude product. The crude product was separated and purified by preparative HPLC to afford the title compound as a pale orange solid (22.00 mg, yield: 23.04%). LCMS (ESI): $[M+H]^+$ = 556.1; $^1H$ NMR (400 MHz, DMSO-$d_6$) δ: 8.30 - 8.18 (m, 1H), 7.97 (dd, J = 8.7, 2.9 Hz, 1H), 7.85 - 7.71 (m, 1H), 7.27 (s, 1H), 5.53 (s, 2H), 5.43 - 5.27 (m, 2H), 4.94 - 4.74 (m, 2H), 4.13 - 3.94 (m, 2H), 3.27 - 3.06 (m, 3H), 3.08 - 2.79 (m, 3H), 1.93 - 1.72 (m, 2H), 1.19 - 1.01 (m, 2H), 1.00 - 0.51 (m, 5H).

Example 102: Preparation of tert-butyl (S)-(2-(4-ethyl-8-fluoro-4-hydroxy-3,6,14-trioxo-4,6,12,14-tetrahydro-1H-pyrano [3,4:6,7]indolizino[2,1 -b]quinolin-11(3H)-yl)ethyl)(methyl)carbamate:

*Step 1: Preparation of tert-butyl (2-(3-bromo-6-fluoro-2-methyl-4-oxoquinolin-1(4H)-yl)ethyl)(methyl)carbamate:*

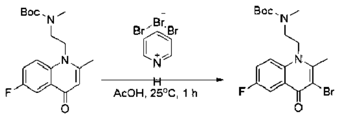


The Intermediate 36 (4.8 g, 14.4 mmol, 1.0 eq.) was dissolved **in** acetic acid (50 mL). Tribromopyridine (4.62 g, 14.4 mmol, 1.0 eq.) was added slowly. After the addition, the mixture was heated to 25 °C and stirred for 1 hour. TLC monitoring indicated the reaction was complete. The reaction solution was poured into ice-cold saturated aqueous sodium bicarbonate solution (100 mL), stirred for 10 minutes, and the pH was adjusted to weakly alkaline. The mixture was extracted with DCM (DCM, 40 mL × 3), the combined organic phases were dried over anhydrous sodium sulfate, filtered, and the filtrate was concentrated. The residue was separated and purified by silica gel column chromatography (eluent: DCM:MeOH = 1:0 to 30:1, v/v) to afford the title compound as a pale brown solid (2.23 g, yield: 37.6%). LCMS (ESI): $[M+H]^+$ = 413.2.

*Step 2: Preparation of tert-butyl (2-(3-bromo-2-(bromomethyl)-6-fluoro-4-oxoquinolin-1(4H)-yl)ethyl)(methyl)carbamate:*

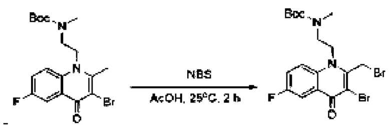


The intermediate obtained from step 1 (2.0 g, 4.85 mmol, 1.0 eq.) was dissolved **in** acetic acid (20 mL). N-Bromosuccinimide (NBS, 1.04 g, 5.83 mmol, 1.2 eq.) was added. After the addition, the mixture was warmed to 25 °C and stirred for 2 hours. TLC detection indicated the reaction was complete. The reaction solution was poured into ice-cold saturated aqueous sodium bicarbonate solution (60 mL), extracted with DCM (DCM, 30 mL × 3), the combined organic phases were dried over anhydrous sodium sulfate, filtered, and the filtrate was concentrated, and purified by silica gel column chromatography (eluent: DCM:MeOH = 1:0 to 30:1**,** v/v) to afford the title compound as a pale brown solid (1.70 g, yield: 71.4%). LCMS (ESI) $[M+1+2]^+$ =493.1; $^1$H NMR (400 MHz, DMSO-$d_6$) δ 7.92 - 7.84 (m, 2H), 7.81 - 7.72 (m, 1H), 5.13 (s, 2H), 3.68 - 3.51 (m, 2H), 2.88 (s, 3H), 2.86 - 2.78 (m, 2H), 1.25 (d, *J* = 64.4 Hz, 9H).

*Step 3: Preparation of tert-butyl (S)-(2-(3-bromo-2-((4-ethyl-4-hydroxy-3,8-dioxo-4,8-dihydro-1H-pyrano[3,4-c]pyridin-7(3H)-yl)methyl)-6-fluoro-4-oxoquinolin-1(4H)-yl)ethyl)(methyl)carbamate:*

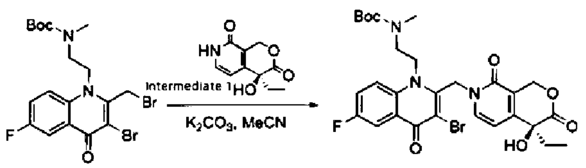


The intermediate obtained from step 2 (500 mg, 1.02 mmol, 1.0 eq.) and Intermediate 1 (214 mg, 1.02 mmol, 1.0 eq.) were dissolved in acetonitrile (10 mL). Potassium carbonate (422 mg, 3.06 mmol, 3.0 eq.) was added, and after the addition, the reaction was heated to 35 °C and stirred overnight. The mixture was separated and purified by silica gel column chromatography (eluent: DCM:MeOH = 1:0 to 20:1, v/v) to afford the title compound as a pale brown solid (260 mg, yield: 41.2%). LCMS (ESI): $[M+H]^+$ = 619.9; $^1$H NMR (400 MHz, DMSO-$d_6$) δ: 8.17 - 8.02 (m, 1H), 7.97 - 7.87 (m, 1H), 7.76 (s, 1H), 7.59 (s, 1H), 6.47 - 6.41 (m, 1H), 6.35 (s, 1H), 5.61 - 5.43 (m, 2H), 5.30 (s, 2H), 4.72 - 4.55 (m, 2H), 2.91 - 2.82 (m, 2H), 2.57 (d, J = 4.5 Hz, 3H), 1.82 - 1.71 (m, 2H), 1.15 (s, 9H), 0.80 (t, J = 9.3 Hz, 3H).

*Step 4: Preparation of tert-butyl (S)-(2-(4-ethyl-8-fluoro-4-hydroxy-3,6,14-trioxo-4,6,12,14-tetrahydro-1H-pyrano [3,4:6,7]indolizino[2,1-b]quinolin-11(3H)-yl)ethyl)(methyl)carbamate:*

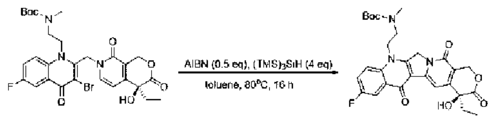


The intermediate obtained from step 3 (260 mg, 0.420 mmol, 1.0 eq.) and azobisisobutyronitrile (AIBN, 34.4 mg, 0.210 mmol, 0.5 eq.) were added to a dry three-necked flask. The flask was evacuated with an oil pump and protected with argon, then anhydrous toluene (30 mL) was added, followed by tris(trimethylsilyl)silane ($(TMS)_3SiH$, 418 mg, 1.68 mmol, 4.0 eq.). After the addition, the mixture was warmed to 80 °C and stirred for 18 hours. The reaction solution was concentrated under reduced pressure to obtain a crude product, which was separated and purified by silica gel column chromatography (eluent: DCM:MeOH = 1:0 to 20:1, v/v) to afford the title compound as yellow solid (110 mg, yield: 46.8%). LCMS (ESI) $[M+1]^+$ =540.4; $^1H$ NMR (400 MHz, DMSO-$d_6$) δ 8.16 - 7.96 (m, 2H), 7.86 - 7.75 (m, 1H), 7.30 (s, 1H), 6.38 (s, 1H), 5.44 - 5.28 (m, 4H), 4.68 - 4.52 (m, 2H), 3.75 - 3.55 (m, 2H), 2.85 (d, *J* = 14.2 Hz, 3H), 1.91 - 1.74 (m, 2H), 1.12 (d, *J* = 77.5 Hz, 9H), 0.86 (t, *J* = 7.2 Hz, 3H).

Example 103: Preparation of (S)-4-ethyl-8-fluoro-4-hydroxy-11-(2-(methylamino)ethyl)-1,12-dihydro-14H-pyrano [3,4:6,7]indolizino[2,1 -b] quinoline-3,6,14(4H,11H)-trione:

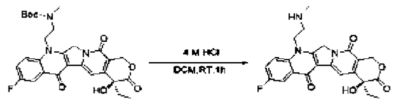


The compound obtained from Example 102 (110 mg, 0.229 mmol, 1.0 eq.) was dissolved in anhydrous DCM ( 2 mL). Under ice bath conditions, a 4 mol/L hydrogen chloride in dioxane solution (2 mL) was added slowly. After the addition, the reaction was stirred at room temperature for 60 minutes. LCMS detection indicated the reaction was complete. The reaction solution was concentrated to afford the title compound as a pale brown solid (23.4 mg, yield: 26.0%). LCMS (ESI): $[M+H]^+$ = 440.0.

Example 104: Preparation of (S)-11-(2-(dimethylamino)ethyl)-4-ethyl-8-fluoro-4-hydroxy-1,12-dihydro-14H-pyrano [3,4:6,7]indolizino[2,1 -b]quinoline-3,6,14 (4H,11H)-trione:

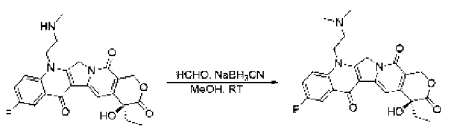


The compound obtained from Example 103 (110 mg, 0.251 mmol, 1.0 eq.) was dissolved in MeOH (MeOH, 5 mL). A 37% (mass fraction) aqueous formaldehyde solution (60.9 mg, 0.752 mmol, 3.0 eq.) was added, and the mixture was stirred at room temperature for 15 minutes. Under ice bath conditions, sodium cyanoborohydride ($NaBH_3CN$, 38.7 mg, 0.630 mmol, 2.5 eq.) was added slowly, and the stirring reaction was continued under ice bath conditions for 30 minutes. LCMS detection indicated the reaction was complete. The reaction was quenched by adding a 4 mol/L hydrogen chloride in dioxane solution (0.2 mL), and the reaction solution was concentrated to obtain a crude product. The crude product was separated and purified by reversed-phase preparative HPLC to afford the title compound as a pale yellow solid (8.20 mg, yield: 7.26%). LCMS (ESI): $[M+H]^+$ = 454.3; $^1H$ NMR (400 MHz, DMSO-$d_6$) δ: 10.72 (s, 1H), 8.23 (dd, J = 9.4, 4.1 Hz, 1H), 8.03 (dd, J = 8.8, 3.1 Hz, 1H), 7.89 - 7.79 (m, 1H), 7.31 (s, 1H), 6.40 (s, 1H), 5.55 (s, 2H), 5.45 - 5.31 (m, 2H), 4.90 - 4.75 (m, 2H), 3.63 - 3.52 (m, 2H), 2.93 (s, 6H), 1.90 - 1.77 (m, 2H), 0.87 (t, J = 7.3 Hz, 3H).

**Examples 105-144**

Using the intermediate (Z)-1-(2,5-difluorophenyl)-3-(dimethylamino)but-2-en-1-one prepared in Preparation Example 27 (Intermediate 27) and the commercially available reagent (2-aminoethyl)carbamate tert-butyl ester as starting materials, the compound of Example 105 was synthesized following the same method as in Examples 102 and 103. Then, according to the synthetic steps in Example 104, the compound of Example 105 was subjected to one or two reductive amination reactions with various aldehyde or ketone starting materials to synthesize the series of compounds of Examples 106-144. The compound structures and specific characterization data (LCMS and 1H NMR) are as follows:

| Examples | structure | $^1$H NMR | LCMS (ESI) $[M+H]^+$ |
|---|---|---|---|
| 105 | 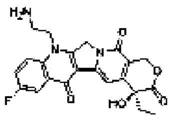 | (400 MHz, DMSO-$d_6$) δ 8.17 (brs, 4H), 8.02 (dd, *J* = 8.9, 3.2 Hz, 1H), 7.86-7.81 (m, 1H), 7.29 (s, 1H), 6.38 (s, 1H), 5.46 (s, 2H), 5.41-5.36 (m, 2H), 4.70-4.57 (m, 2H), 3.30-3.24 (m, 2H),1.89-1.76 (m, 2H), 0.86 (t, *J* = 7.3 Hz, 3H)。 | 426.3 |
| 106 | 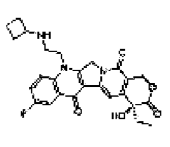 | (400 MHz, DMSO-$d_6$) δ 9.28 (s, 2H), 8.18 (dd, *J* = 9.5, 4.0 Hz, 1H), 8.00 (dd, *J* = 8.8, 3.0 Hz, 1H), 7.83-7.78 (m, 1H), 7.28 (s, 1H), 6.36 (s, 1H), 5.49 (s, 2H), 5.39-5.34 (m, 2H), 4.68-4.63 (m, 2H), 3.77-3.66 (m, 2H), 3.26 (q, *J* = 6.4 Hz, 2H), 2.26-2.12 (m, 4H), 1.88-1.71 (m, 3H), 0.85 (t, *J* = 7.3 Hz, 3H)。 | 480.2 |
| 107 | 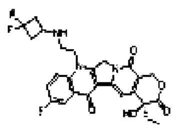 | (400 MHz, DMSO-$d_6$) δ 9.23 (s, 2H), 8.11 - 8.00 (m, 2H), 7.89 - 7.82 (m, 1H), 7.31 (s, 1H), 5.56 - 5.29 (m, 4H), 4.74 - 4.54 (m, 2H), 3.87 - 3.72 (m, 1H), 3.06 - 2.90 (m, 4H), 1.89 - 1.75 (m, 2H), 0.86 (t, *J* = 7.3 Hz, 3H)。 | 516.3 |
| 108 | 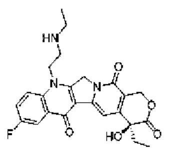 | (400 MHz, DMSO-$d_6$) δ 8.59 (s, 2H), 8.15 - 8.01 (m, 2H), 7.93 - 7.81 (m, 1H), 7.33 (s, 1H), 6.43 (s, 1H), 5.51 (s, 2H), 5.46 - 5.30 (m, 2H), 4.75 - 4.58 (m, 2H), 3.60 - 3.49 (m, 2H), 3.13 - 2.96 (m, 2H), 1.91 - 1.77 (m, 2H), 1.21 (t, *J* = 7.2 Hz, 3H), 0.88 (t, *J* = 7.2 Hz, 3H) | 454.3 |
| 109 | 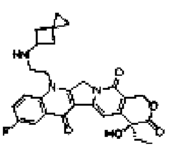 | (400 MHz, DMSO-$d_6$) δ 9.30 (s, 2H), 8.16 (dd, *J* = 9.3, 4.1 Hz, 1H), 8.03 (dd, *J* = 8.8, 3.3 Hz, 1H), 7.85-7.80 (m, 1H), 7.30 (s, 1H), 6.37 (s, 1H), 5.51 (s, 2H), 5.37 (m, 2H), 4.75-4.63 (m, 2H), 4.00-3.91 (m, 1H), 3.41 (t, *J* = 6.9 Hz, 2H), 2.59-2.54 (m, 2H), 2.25-2.15 (m, 2H), 1.88-1.78 (m, 2H), 0.86 (t, *J* = 7.3 Hz, 3H), 0.49 (m, 4H)。 | 506.2 |
| 110 | 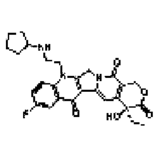 | (400 MHz, DMSO-$d_6$) δ 9.06 (s, 2H), 8.23 (dd, *J* = 9.3, 4.1 Hz, 1H), 8.03 (dd, *J* = 8.8, 3.0 Hz, 1H), 7.87-7.82 (m, 1H), 7.31 (s, 1H), 6.39 (s, 1H), 5.51 (s, 2H), 5.43-5.31 (m, 2H), 4.74-4.65 (m, 2H), 3.61-3.53 (m, 1H), 3.48-3.39 (m, 2H), 1.99-1.89 (m, 2H), 1.87-1.80 (m, 2H), 1.79-1.73 (m, 4H), 1.57 (s, 2H), 0.87 (t, *J* = 7.3 Hz, 3H)。 | 494.3 |
| 111 | 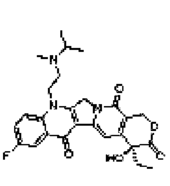 | (400 MHz, DMSO-$d_6$) δ 10.97 (s, 1H), 8.35 (dd, *J* = 9.5, 4.1 Hz, 1H), 8.08 - 7.93 (m, 1H), 7.86 - 7.77 (m, 1H), 7.30 (d, *J* = 2.3 Hz, 1H), 5.67 - 5.49 (m, 2H), 5.43 - 5.31 (m, 2H), 5.03 - 4.78 (m, 2H), 3.74 - 3.67 (m, 2H), 3.48 - 3.41 (m, 1H), 2.86 (t, *J* = 4.6 Hz, 3H), 1.90 - 1.76 (m, 2H), 1.32 (d, *J* = 6.5 Hz, 3H), 1.25 (d, *J* = 6.4 Hz, 3H), 0.87 (t, *J* = 7.2 Hz, 3H)。 | 482.0 |
| 112 | 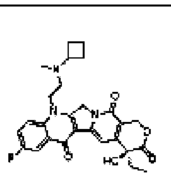 | (400 MHz, DMSO-$d_6$) δ 11.07 (s, 1H), 8.24 (dd, *J* = 9.3, 3.9 Hz, 1H), 8.02 (dd, *J* = 8.8, 3.1 Hz, 1H), 7.87 - 7.79 (m, 1H), 7.30 (s, 1H), 6.39 (s, 1H), 5.57 - 5.45 (m, 2H), 5.42 - 5.30 (m, 2H), 4.97 - 4.84 (m, 1H), 4.79 - 4.67 (m, 1H), 3.82 - 3.70 (m, 1H), 3.50 - 3.43 (m, 1H), 2.85 (d, *J* = 3.3 Hz, 3H), 2.42 - 2.10 (m, 5H), 1.90 - 1.62 (m, 4H), 0.86 (t, *J* = 7.3 Hz, 3H)。 | 494.3 |

(continued)

| Examples | structure | $^1$H NMR | LCMS (ESI) [M+H]$^+$ |
|---|---|---|---|
| 113 | 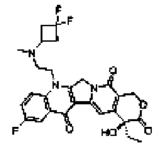 | (400 MHz, DMSO-$d_6$) δ 11.91 (s, 1H), 8.21 (s, 1H), 8.01 (dd, *J* = 8.8, 3.1 Hz, 1H), 7.86 - 7.79 (m, 1H), 7.30 (s, 1H), 6.38 (s, 1H), 5.52 (s, 2H), 5.43 - 5.30 (m, 2H), 4.74 (s, 3H), 3.82 (s, 2H), 3.00 (s, 7H), 1.90 - 1.76 (m, 2H), 0.86 (t, *J* = 7.3 Hz, 3H)。 | 529.8 |
| 114 | 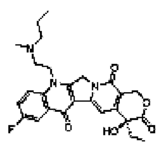 | (400 MHz, DMSO-$d_6$) δ 10.69 (s, 1H), 8.27 (s, 1H), 8.03 (dd, *J* = 8.8, 3.1 Hz, 1H), 7.90 - 7.79 (m, 1H), 7.31 (s, 1H), 6.40 (s, 1H), 5.55 (s, 2H), 5.38 (d, *J* = 3.4 Hz, 2H), 4.88 (s, 2H), 3.62 - 3.54 (m, 2H), 3.24 - 3.05 (m, 2H), 2.95 (s, 3H), 1.89 - 1.79 (m, 2H), 1.78 - 1.68 (m, 2H), 0.95 (t, *J* = 6.9 Hz, 3H), 0.88 (t, *J* = 7.3 Hz, 3H)。 | 481.9 |
| 115 | 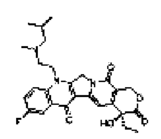 | (400 MHz, DMSO-$d_6$) δ 10.40 (s, 1H), 8.34 (d, *J* = 6.2 Hz, 1H), 8.06 - 7.98 (m, 1H), 7.87 - 7.80 (m, 1H), 7.31 (s, 1H), 6.42 (s, 1H), 5.55 (s, 2H), 5.43 - 5.30 (m, 2H), 5.01 - 4.82 (m, 2H), 3.64 - 3.54 (m, 2H), 3.20 - 3.01 (m, 2H), 2.98 - 2.91 (m, 3H), 2.18 - 2.09 (m, 1H), 1.89 - 1.77 (m, 2H), 1.08 - 0.97 (m, 6H), 0.87 (t, *J* = 7.3 Hz, 3H)。 | 496.4 |
| 116 | 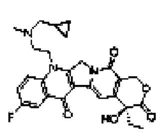 | (400 MHz, DMSO-$d_6$) δ 10.63 (s, 1H), 8.25 (dd, *J* = 9.4, 3.9 Hz, 1H), 8.01 (dd, *J* = 8.8, 3.1 Hz, 1H), 7.87 - 7.79 (m, 1H), 7.30 (s, 1H), 6.39 (s, 1H), 5.53 (s, 2H), 5.41 - 5.32 (m, 2H), 4.86 (s, 2H), 3.60 (s, 2H), 3.15 (s, 2H), 3.00 (s, 3H), 1.91 - 1.76 (m, 2H), 1.16 (d, *J* = 6.6 Hz, 1H), 0.90 - 0.80 (m, 3H), 0.75 - 0.61 (m, 2H), 0.45 (d, *J* = 3.8 Hz, 2H)。 | 494.4 |
| 117 | 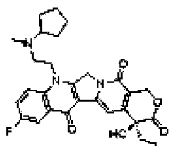 | (400 MHz, DMSO-$d_6$) δ 11.22 (s, 1H), 8.29 (s, 1H), 8.07 - 7.95 (m, 1H), 7.86 - 7.77 (m, 1H), 7.29 (s, 1H), 6.45 - 6.32 (m, 1H), 5.62 - 5.44 (m, 2H), 5.43 - 5.28 (m, 2H), 5.01 - 4.79 (m, 2H), 3.75 - 3.55 (m, 2H), 3.49 (s, 1H), 2.94 (s, 3H), 2.09 - 1.92 (m, 3H), 1.88 - 1.77 (m, 3H), 1.75 - 1.68 (m, 2H), 1.59 - 1.48 (m, 2H), 0.93 - 0.74 (m, 3H)。 | 508.4 |
| 118 | 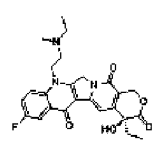 | (400 MHz, DMSO-$d_6$) δ 10.51 (s, 1H), 8.23 (dd, *J* = 9.5, 3.7 Hz, 1H), 8.03 (dd, *J* = 8.8, 2.9 Hz, 1H), 7.88 - 7.81 (m, 1H), 7.32 (s, 1H), 6.41 (s, 1H), 5.55 (s, 2H), 5.40 - 5.30 (m, 2H), 4.95 - 4.74 (m, 2H), 3.64 - 3.49 (m, 2H), 3.24 - 3.14 (m, 2H), 2.94 (t, *J* = 3.7 Hz, 3H), 1.90 - 1.78 (m, 2H), 1.28 (t, *J* = 6.4 Hz, 3H), 0.87 (t, *J* = 7.2 Hz, 3H)。 | 483.3 |
| 119 | 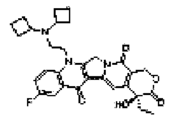 | (400 MHz, DMSO-$d_6$) δ 10.71-10.96 (brs, 1H), 8.28-8.18 (m, 1H), 7.99-7.95 (m, 1H), 7.81-7.76 (m, 1H), 7.26 (s, 1H), 6.36 (s, 1H), 5.49 (s, 2H), 5.37-5.28 (m, 2H), 4.92-4.82 (m, 2H), 3.51-3.37(m, 6H), 1.84-1.71 (m, 2H), 1.25-1.19 (m, 6H), 0.82 (t, *J* = 7.3 Hz, 3H)。 | 534.2 |
| 120 | 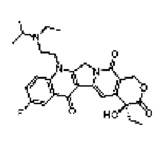 | (400 MHz, DMSO-$d_6$) δ 11.02 (s, 1H), 8.38-8.37 (m, 1H), 7.98-7.97 (m, 1H), 7.81-7.80 (m, 1H), 7.27 (s, 1H), 6.38 (s, 1H), 5.59-5.54 (m, 2H), 5.51-5.49(m, 2H), 5.14-4.88 (m, 2H), 3.81-3.78 (m, 2H), 3.54-3.46 (m, 2H), 2.57-2.50 (m, 1H), 1.83-1.80 (m, 2H), 1.37-1.27 (m, 9H), 0.88-0.80 (m, 3H)。 | 496.4 |
| 121 | 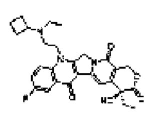 | (400 MHz, DMSO-$d_6$) δ 10.95 (br, 1H), 8.22-8.20 (m, 1H), 8.0-7.99 (m, 1H), 7.85-7.77 (m, 1H), 7.29 (s, 1H), 6.37 (s, 1H), 5.49 (s, 2H), 5.36-5.31 (m, 2H), 4.88-4.74 (m, 1H), 4.65-4.57 (m, 1H), 3.91-3.89 (m, 1H), 3.74-3.65 (m, 1H), 3.51-3.35 (m, 2H), 3.23 (d, *J* = 7.1 Hz, 2H), 2.67-2.60 (m, 1H), 2.20-2.35 (m, 4H), 1.85-1.65 (m, 4H), 1.16-1.32 (m,3H), 0.83 (t, *J* = 7.3 Hz, 3H)。 | 508.3 |

(continued)

| Examples | structure | $^1$H NMR | LCMS (ESI) [M+H]$^+$ |
|---|---|---|---|
| 122 | 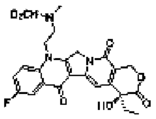 | $^1$H NMR (400 MHz, DMSO-$d_6$) δ 10.72 (s, 1H), 8.23 (dd, $J$ = 9.4, 4.1 Hz, 1H), 8.03 (dd, $J$ = 8.8, 3.1 Hz, 1H), 7.89 - 7.79 (m, 1H), 7.31 (s, 1H), 6.40 (s, 1H), 5.55 (s, 2H), 5.45 - 5.31 (m, 2H), 4.90 - 4.75 (m, 2H), 3.63 - 3.52 (m, 2H), 2.93 (s, 6H), 1.90 - 1.77 (m, 2H), 0.87 (t, $J$ = 7.3 Hz, 3H)。 | 456.1 |
| 123 | 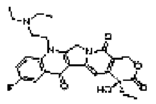 | (400 MHz, DMSO-$d_6$) δ 10.71-10.96 (brs, 1H), 8.28-8.18 (m, 1H), 7.99-7.95 (m, 1H), 7.81-7.76 (m, 1H), 7.26 (s, 1H), 6.36 (s, 1H), 5.49 (s, 2H), 5.37-5.28 (m, 2H), 4.92-4.82 (m, 2H), 3.51-3.37(m, 6H), 1.84-1.71 (m, 2H), 1.25-1.19 (m, 6H), 0.82 (t, $J$ = 7.3 Hz, 3H)。 | 482.3 |
| 124 | 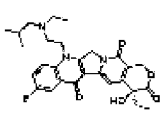 | (400 MHz, DMSO-$d_6$) δ 10.23-10.27 (brs, 1H), 8.30 (td, J = 5.8, 3.5 Hz, 1H), 7.97 (dd, J = 8.8, 3.3 Hz, 1H), 7.82-7.77 (m, 1H), 7.26 (s, 1H), 6.36 (s, 1H), 5.48 (s, 2H), 5.37-5.28 (m, 2H), 4.97-4.82 (m, 2H), 3.55-3.50 (m, 2H), 3.46-3.31 (m, 2H), 3.13-3.03 (m, 2H), 2.10-2.03 (m, 1H), 1.84-1.71 (m, 2H), 1.30-1.16 (m, 3H), 1.04-0.95 (m, 6H), 0.82 (t, $J$ = 7.3 Hz, 3H)。 | 510.3 |
| 125 | 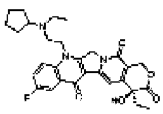 | (400 MHz, DMSO-$d_6$) δ 8.22-8.19 (m, 1H), 7.98 (dd, $J$ = 8.8, 3.0 Hz, 1H), 7.83-7.78 (m, 1H), 7.27 (s, 1H), 6.35 (s, 1H), 5.52-5.45 (m, 2H), 5.40-5.28 (m, 2H), 4.94-4.80 (m, 2H), 3.78-3.69 (m, 1H), 3.64-3.57 (m, 1H), 3.44-3.37 (m, 3H), 2.07-1.92 (m, 2H), 1.80-1.68 (m, 6H), 1.57-1.43 (m, 2H), 1.32 (td, $J$ = 7.1, 2.7 Hz, 3H), 0.82 (t, $J$ = 7.3 Hz, 3H)。 | 522.3 |
| 126 | 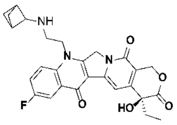 | N/A | 492.2 |
| 127 | 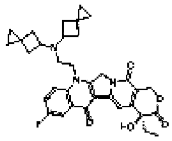 | (400 MHz, DMSO-$d_6$) δ 12.37 (s, 1H), 8.29 (d, $J$ = 6.0 Hz, 1H), 7.96 (dd, $J$ = 8.8, 3.0 Hz, 1H), 7.83-7.78 (m, 1H), 7.26 (s, 1H), 6.32 (s, 1H), 5.51 (s, 2H), 5.42-5.28 (m, 2H), 4.92-4.77 (m, 2H), 4.30-4.13 (m, 2H), 3.47 (s, 2H), 2.99-2.83 (m, 4H), 2.35-2.10 (m, 4H), 1.90-1.77 (m, 2H), 0.86 (t, $J$ = 7.3 Hz, 3H), 0.55 (d, $J$ = 6.3 Hz, 4H), 0.48 (d, $J$ = 6.3 Hz, 4H)。 | 586.5 |
| 128 | 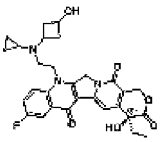 | (400 MHz, DMSO-$d_6$) δ 11.01 (s, 1H), 8.26 (s, 1H), 8.04 (dd, $J$ = 8.8, 3.1 Hz, 1H), 7.87 - 7.78 (m, 1H), 7.32 (s, 1H), 6.37 (s, 1H), 5.53 (s, 2H), 5.44 - 5.29 (m, 2H), 5.00 - 4.70 (m, 2H), 3.97 - 3.80 (m, 1H), 3.75 - 3.48 (m, 3H), 3.16 - 2.91 (m, 2H), 2.78 - 2.61 (m, 1H), 2.34 - 2.05 (m, 2H), 1.92 - 1.77 (m, 2H), 1.30 - 1.10 (m, 2H), 1.08 - 0.93 (m, 2H), 0.88 (t, $J$ = 7.3 Hz, 3H)。 | 536.3 |
| 129 | 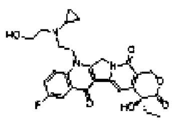 | $^1$H NMR (400 MHz, DMSO-$d_6$) δ 8.23 - 8.09 (m, 1H), 8.04 (dd, $J$= 8.8, 2.8 Hz, 1H), 7.87 - 7.78 (m, 1H), 7.32 (s, 1H), 6.38 (s, 1H), 5.50 (s, 2H), 5.42 - 5.29 (m, 2H), 5.01 - 4.37 (m, 4H), 4.05 - 3.63 (m, 4H), 2.92 - 2.69 (m, 1H), 1.92 - 1.74 (m, 2H), 1.28 - 0.46 (m, 7H) | 510.0 |
| 130 | 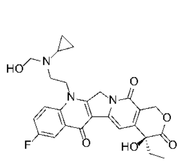 | $^1$H NMR (400 MHz, DMSO-$d_6$) δ 9.49 - 9.08 (m, 2H), 8.19 (dd, $J$ = 9.3, 3.8 Hz, 1H), 8.01 (dd, $J$ = 8.8, 3.1 Hz, 1H), 7.85 - 7.76 (m, 1H), 7.29 (s, 1H), 6.33 (s, 1H), 6.17 (s, 1H), 5.40 - 5.26 (m, 2H), 5.18 - 5.06 (m, 1H), 5.03 - 4.92 (m, 1H), 4.80 - 4.70 (m, 1H), 4.58 - 4.51 (m, 1H), 4.28 - 4.14 (m, 1H), 3.60 - 3.49 (m, 1H), 2.79 - 2.71 (m, 1H), 2.04 - 1.91 (m, 1H), 1.86 - 1.77 (m, 2H), 1.51 - 1.41 (m, 1H), 0.98 - 0.82 (m, 5H), 0.79 - 0.70 (m, 2H)。 | 496.0 |

(continued)

| Examples | structure | $^1$H NMR | LCMS (ESI) $[M+H]^+$ |
|---|---|---|---|
| 131 | 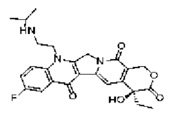 | (400 MHz, DMSO-$d_6$) δ 9.07 (s, 2H), 8.26 (dd, J = 9.4, 4.2 Hz, 1H), 8.04 (dd, J = 8.8, 2.9 Hz, 1H), 7.89 - 7.81 (m, 1H), 7.31 (s, 1H), 6.42 (s, 1H), 5.58 - 5.23 (m, 4H), 4.76 - 4.63 (m, 1H), 3.47 - 3.40 (m, 2H), 1.89 - 1.79 (m, 2H), 1.34 - 1.22 (m, 8H), 0.88 (t, J = 7.2 Hz, 3H)。 | 468.2 |
| 132 | 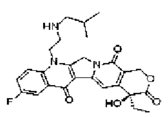 | (400 MHz, DMSO-$d_6$) δ 8.67 (s, 2H), 8.16 (d, *J* = 9.1 Hz, 1H), 8.03 (d, *J* = 7.6 Hz, 1H), 7.88 (d, *J* = 8.6 Hz, 1H), 7.30 (s, 1H), 6.40 (s, 1H), 5.50 - 5.29 (m, 4H), 4.67 - 4.60 (m, 1H), 3.41 - 3.37 (m, 2H), 2.84 - 2.76 (m, 2H), 2.02 - 1.92 (m, 2H), 1.87 - 1.77 (m, 2H), 0.99 (d, *J* = 6.3 Hz, 3H), 0.87 (d, *J* = 6.7 Hz, 3H)。 | 482.4 |
| 133 | 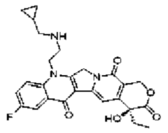 | (400 MHz, DMSO-$d_6$) δ 8.59 (s, 2H), 8.15 - 8.01 (m, 2H), 7.93 - 7.81 (m, 1H), 7.33 (s, 1H), 6.43 (s, 1H), 5.51 (s, 2H), 5.46 - 5.30 (m, 2H), 4.75 - 4.58 (m, 2H), 3.60 - 3.49 (m, 2H), 3.13 - 2.96 (m, 2H), 1.91 - 1.77 (m, 2H), 1.21 (t, *J* = 7.2 Hz, 3H), 0.88 (t, *J* = 7.2 Hz, 3H)。 | 494.1 |
| 134 | 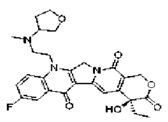 | (400 MHz, DMSO-$d_6$) δ 11.54 (s, 1H), 8.35 - 8.21 (m, 1H), 8.01 (dd, J = 8.7, 3.0 Hz, 1H), 7.86 - 7.75 (m, 1H), 7.30 (s, 1H), 6.38 (s, 1H), 5.55 (s, 2H), 5.43 - 5.28 (m, 2H), 4.99 - 4.77 (m, 2H), 4.28 - 4.07 (m, 2H), 4.01 - 3.88 (m, 1H), 3.77 - 3.52 (m, 4H), 2.92 (s, 3H), 2.32 - 2.16 (m, 2H), 1.91 - 1.72 (m, 2H), 0.86 (t, J = 7.3 Hz, 3H)。 | 510.2 |
| 135 | 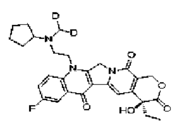 | (400 MHz, DMSO-$d_6$) δ 10.89 (s, 1H), 8.25 (dd, *J* = 9.4, 3.9 Hz, 1H), 8.01 (dd, *J* = 8.7, 3.0 Hz, 1H), 7.88 - 7.76 (m, 1H), 7.30 (s, 1H), 6.37 (s, 1H), 5.59 - 5.20 (m, 4H), 5.00 - 4.71 (m, 2H), 3.77 - 3.45 (m, 3H), 2.92 (s, 1H), 2.13 - 1.94 (m, 2H), 1.86 - 1.61 (m, 6H), 1.63 - 1.46 (m, 2H), 0.86 (t, *J* = 7.3 Hz, 3H)。 | 510.4 |
| 136 | 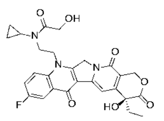 | (400 MHz, DMSO-$d_6$)δ 8.17 (dd, J = 9.4, 4.1 Hz, 1H), 8.02 (dd, J = 8.8, 3.1 Hz, 1H), 7.86 - 7.78 (m, 1H), 7.31 (s, 1H), 5.47 - 5.28 (m, 4H), 4.53 - 4.45 (m, 2H), 4.25 (s, 2H), 3.78 - 3.70 (m, 2H), 2.75 - 2.67 (m, 1H), 1.88 - 1.79 (m, 2H), 0.87 (t, J = 7.3 Hz, 3H), 0.82 - 0.77 (m, 2H), 0.72 - 0.66 (m, 2H)。(HCl salt) | 524.1 |
| 137 | 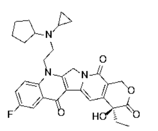 | (400 MHz, DMSO-$d_6$) δ 10.35 (s, 1H), 8.17 (s, 1H), 8.02 (dd, *J* = 8.8, 3.0 Hz, 1H), 7.85 (dd, *J* = 11.6, 5.1 Hz, 1H), 7.30 (s, 1H), 6.39 (s, 1H), 5.61 - 5.25 (m, 4H), 4.94 - 4.86 (m, 2H), 3.94 - 3.56 (m, 2H), 3.52 - 3.48 (m, 1H), 3.18 - 3.12 (m, 1H), 2.21 - 1.94 (m, 3H), 1.92 - 1.76 (m, 3H), 1.75 - 1.69 (m, 2H), 1.58 - 1.54 (m, 2H), 1.12 - 1.10 (m, 2H), 1.08 - 1.04 (m, 2H), 0.86 (t, *J* = 7.3 Hz, 3H)。 | 534.4 |
| 138 | 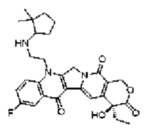 | (400 MHz, DMSO-$d_6$) δ 8.76 (s, 2H), 8.37 - 8.26 (m, 1H), 8.02 (dd, J = 8.8, 3.1 Hz, 1H), 7.93 - 7.81 (m, 1H), 7.30 (s, 1H), 5.51 - 5.44 (m, 2H), 5.41 - 5.25 (m, 2H), 4.94 - 4.77 (m, 1H), 4.73 - 4.56 (m, 1H), 3.43 - 3.33 (m, 1H), 3.23 - 3.11 (m, 1H), 2.08 - 1.97 (m, 1H), 1.90 - 1.66 (m, 4H), 1.62 - 1.45 (m, 4H), 1.19 (s, 3H), 1.09 (s, 3H), 0.92 - 0.80 (m, 3H)。 | 522.3 |
| 139 | 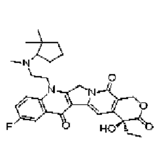 | (400 MHz, DMSO-$d_6$) δ 10.27 (s, 1H), 8.38 - 8.18 (m, 1H), 8.01 (dd, *J* = 8.7, 3.1 Hz, 1H), 7.84 (t, *J* = 8.4 Hz, 1H), 7.30 (s, 1H), 6.40 (s, 1H), 5.55 - 5.29 (m, 4H), 5.04 - 4.74 (m, 2H), 3.76 - 3.57 (m, 1H), 3.18 - 3.00 (m, 2H), 2.92 (t, *J* = 4.8 Hz, 2H), 2.23 - 2.08 (m, 1H), 2.04 - 1.91 (m, 1H), 1.85 - 1.72 (m, 2H), 1.59 - 1.43 (m, 2H), 1.30 - 1.07 (m, 9H), 0.86 (t, *J* = 7.2 Hz, 3H)。 | 536.4 |

(continued)

| Examples | structure | $^1$H NMR | LCMS (ESI) $[M+H]^+$ |
|---|---|---|---|
| 140 | 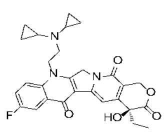 | (400 MHz, DMSO-$d_6$) δ 8.12 - 7.99 (m, 2H), 7.88 - 7.79 (m, 1H), 7.30 (s, 1H), 6.39 (s, 1H), 5.48 (s, 2H), 5.38 - 5.32 (m, 2H), 4.82 - 4.55 (m, 2H), 3.29 - 3.10 (m, 2H), 2.05 - 1.94 (m, 1H), 1.87 - 1.78 (m, 2H), 1.50 - 1.41 (m, 1H), 0.87 (t, *J* = 7.3 Hz, 3H), 0.76 - 0.34 (m, 8H)。 | |
| 141 | 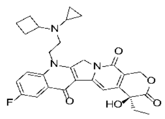 | (400 MHz, DMSO-$d_6$) δ 8.05 - 7.97 (m, 2H), 7.86 - 7.77 (m, 1H), 7.28 (s, 1H), 6.36 (s, 1H), 5.47 - 5.28 (m, 4H), 4.48 (t, *J* = 6.5 Hz, 2H), 3.47 - 3.39 (m, 1H), 2.97 (t, *J* = 6.6 Hz, 2H), 2.00 - 1.88 (m, 5H), 1.87 - 1.78 (m, 2H), 1.63 - 1.48 (m, 2H), 0.86 (t, *J* = 10.3, 4.3 Hz, 3H), 0.44 - 0.35 (m, 2H), 0.15 - 0.04 (m, 2H)。 | 520.5 |
| 142 | 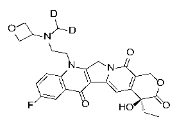 | (400 MHz, DMSO-$d_6$) δ 8.09 (d, *J* = 5.3 Hz, 1H), 8.03 (dd, *J* = 8.8, 3.1 Hz, 1H), 7.87 - 7.78 (m, 1H), 7.32 (s, 1H), 6.39 (s, 1H), 5.53 - 5.48 (m, 2H), 5.40 - 5.35 (m, 2H), 4.62 - 4.52 (m, 2H), 4.48 - 4.38 (m, 2H), 4.19 - 4.04 (m, 2H), 3.62 (s, 1H), 2.79 - 2.64 (m, 2H), 2.31 - 2.16 (m, 1H), 1.91 - 1.78 (m, 2H), 0.89 (t, *J* = 7.3 Hz, 3H)。 | |
| 143 | 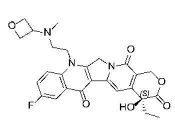 | (400 MHz, DMSO-$d_6$) δ 8.06 (dd, *J* = 9.5, 4.2 Hz, 1H), 8.01 (dd, *J* = 8.9, 3.1 Hz, 1H), 7.82 - 7.76 (m, 1H), 7.30 (s, 1H), 6.34 (s, 1H), 5.50 - 5.46 (m, 2H), 5.38 - 5.34 (m, 2H), 4.58 - 4.52 (m, 2H), 4.44 - 4.37 (m, 2H), 4.13 - 4.07 (m, 2H), 3.63 - 3.57 (m, 1H), 2.74 - 2.66 (m, 2H), 2.24 (s, 3H), 1.90 - 1.79 (m, 2H), 0.91 - 0.84 (m, 3H)。 | |
| 144 | 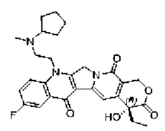 | (400 MHz, DMSO-$d_6$) δ 11.22 (s, 1H), 8.30 (d, *J* = 8.3 Hz, 1H), 8.03 - 7.96 (m, 1H), 7.84 - 7.77 (m, 1H), 7.29 (s, 1H), 6.38 (s, 1H), 5.60 - 5.44 (m, 2H), 5.42 - 5.29 (m, 2H), 4.99 - 4.80 (m, 2H), 3.74 - 3.57 (m, 2H), 3.53 - 3.43 (m, 1H), 2.94 (s, 3H), 2.08 - 1.94 (m, 3H), 1.88 - 1.77 (m, 3H), 1.73 - 1.64 (m, 2H), 1.59 - 1.49 (m, 2H), 0.86 (t, *J* = 9.1, 5.5 Hz, 3H)。 | |

Example 145: Preparation of (S)-11-((R)-1-(cyclopropylamino)-4-methylpentan-2-yl)-4-ethyl-8-fluoro-4-hydroxy-1,12-dihydro-14H-pyrano[3,4:6,7]indolizino [2,1-b]quinoline-3,6,14(4H,11H)-trione:

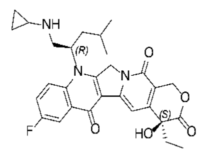

Intermediate 27 (prepared in Example 27), namely (2,5-difluorophenyl)-3-(dimethylamino)but-2-en-1-one, and commercially available D-leucinol were used as the starting materials. The title compound (hydrochloride salt) was synthesized according to the relevant operation procedures described in Example 96, and obtained as a light orange solid. LCMS (ESI): $[M+H]^+$ = 522.0; $^1$H NMR (400 MHz, DMSO-$d_6$) δ: 9.26 (br s, 1H), 9.02 (br s, 1H), 8.20-8.12 (m, 1H), 8.10-8.04 (m, 1H), 7.74-7.67 (m, 1H), 7.33 (s, 1H), 6.44 (s, 1H), 5.92-5.83 (m, 1H), 5.42-5.29 (m, 2H), 5.23-5.13 (m, 1H), 4.73-4.63 (m, 1H), 4.15-4.03 (m, 1H), 3.69-3.57 (m, 1H), 2.79-2.66 (m, 1H), 2.39-2.26 (m, 1H), 2.13-2.00 (m, 1H), 1.89-1.69 (m, 3H), 1.07-0.98 (m, 2H), 0.94-0.81 (m, 9H), 0.75-0.68 (m, 2H).

Example 146: Preparation of (S)-11-((R)-1-(cyclopropyl(methyl)amino)-4-methylpentan-2-yl)-4-ethyl-8-fluoro-4-hydroxy-1,12-dihydro-14H-pyrano[3,4: 6,7]indolizino[2,1-b]quinoline-3,6,14(4H,11H)-trione:

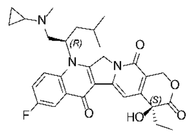

The compound prepared in Example 145 (30 mg, 0.06 mmol, 1.0 eq.) was dissolved in MeOH (5 mL). A 37% (mass fraction) aqueous formaldehyde solution (14.00 mg, 0.17 mmol, 3.0 eq.) and acetic acid (10.36 mg, 0.17 mmol, 3.0 eq.) were added sequentially. The reaction mixture was stirred at room temperature for 1 hour. Under ice-bath conditions, sodium cyanoborohydride ($NaBH_3CN$, 10.67 mg, 0.17 mmol, 3.0 eq.) was added slowly, and the reaction was continued at room temperature for 0.5 hours. LCMS detection indicated that the reaction was complete. The reaction was quenched by adding 1 mol/L dilute hydrochloric acid (0.5 mL), and water was added to the reaction solution, followed by lyophilization to obtain a crude product. The crude product was separated and purified by reversed-phase preparative HPLC to afford the title compound as pale yellow solid (9.20 mg, yield: 28.0%). LCMS (ESI) $[M+1]^+$ =536.4; $^1H$ NMR (400 MHz, DMSO-$d_6$) δ 8.15 - 8.03 (m, 2H), 7.73 (s, 1H), 7.33 (s, 1H), 5.43 - 5.27 (m, 4H), 4.94 - 4.74 (m, 1H), 3.06 - 2.92 (m, 2H), 2.70 - 2.57 (m, 1H), 2.30 - 2.16 (m, 2H), 2.08 - 1.99 (m, 1H), 1.90 - 1.79 (m, 2H), 1.74 - 1.66 (m, 1H), 1.59 - 1.45 (m, 2H), 0.99 (s, 3H), 0.92 - 0.72 (m, 10H).

Example 147: Preparation of (4S)-11-((R)-1-(cyclopropylamino)butan-2-yl)-4-ethyl-8-fluoro-4-hydroxy-1,12-dihydro-14H-pyrano[3,4:6,7]indolizino[2,1 -b]quinoline-3,6,14(4H,11H)-trione:

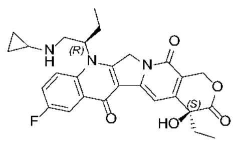

Intermediate 27 (prepared in Example 27) and commercially available (R)-(-)-2-amino-1-butanol were used as the starting materials. The title compound (hydrochloride salt) was synthesized according to the relevant operation procedures described in Example 96, and obtained as an orange-yellow solid. LCMS (ESI): $[M+H]^+$ = 494.3; $^1H$ NMR (400 MHz, DMSO-$d_6$) δ: 9.15 (d, J = 46.0 Hz, 1H), 8.13 (dd, J = 9.5, 4.0 Hz, 1H), 8.07 (dd, J = 8.8, 3.2 Hz, 1H), 7.72-7.66 (m, 1H), 7.33 (s, 1H), 6.38 (s, 1H), 5.81 (d, J = 20.2 Hz, 1H), 5.44-5.28 (m, 3H), 4.66-4.55 (m, 1H), 4.08-3.96 (m, 1H), 3.81-3.71 (m, 1H), 2.77-2.66 (m, 1H), 2.41-2.27 (m, 2H), 1.90-1.76 (m, 2H), 1.03-0.68 (m, 10H).

Example 148: Preparation of (S)-11-((S)-1-(cyclopropylamino)-3-methylbutan-2-yl)-4-ethyl-8-fluoro-4-hydroxy-1,12-dihydro-14H-pyrano[3,4:6,7]indolizino[2,1 -b]quinoline-3,6,14(4H,11H)-trione

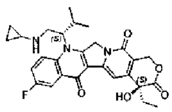

Intermediate 27 (prepared in Example 27) and commercially available L-valinol were used as the starting materials. The title compound (hydrochloride salt) was synthesized according to the relevant operation procedures described in Example 96, and obtained as an orange-yellow solid. LCMS(ESI)$[M+1]^+$ =508.2, $t_R$ = 1.303 min。$^1H$ NMR (400 MHz, DMSO-$d_6$) δ 9.03 (d, *J* = 41.1 Hz, 2H), 8.15 (dd, *J* = 9.5, 4.1 Hz, 1H), 8.08 (dd, *J* = 8.8, 3.2 Hz, 1H), 7.75 - 7.60 (m, 1H), 7.34 (s, 1H), 6.39 (s, 1H), 5.76 (d, *J* = 20.3 Hz, 1H), 5.43 - 5.26 (m, 3H), 4.28 (t, *J* = 9.9 Hz, 1H), 4.16 - 4.03 (m, 1H), 3.73 - 3.61 (m, 1H), 2.91 - 2.71 (m, 2H), 1.94 - 1.76 (m, 2H), 1.28 (d, *J* = 6.3 Hz, 3H), 0.98 - 0.83 (m, 4H), 0.82 - 0.59 (m, 6H).

Example 149: Preparation of (S)-11-((S)-1-(cyclopropylamino)-4-methylpentan-2-yl)-4-ethyl-8-fluoro-4-hydroxy-1,12-dihydro-14H-pyrano[3,4:6,7]indolizino[2,1 -b]quinoline-3,6,14(4H,11H)-trione:

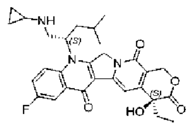

Intermediate 27 (prepared in Example 27) and commercially available L-leucanol were used as the starting materials. The title compound (hydrochloride salt) was synthesized according to the relevant operation procedures described in Example 96, and obtained as a white solid. LCMS (ESI): $[M+H]^+$ = 522.0; $^1H$ NMR (400 MHz, DMSO-$d_6$) δ: 9.14 (s, 1H), 8.92 (s, 1H), 8.15 (dd, J = 9.6, 4.0 Hz, 1H), 8.07 (dd, J = 8.8, 3.2 Hz, 1H), 7.74-7.66 (m, 1H), 7.34 (s, 1H), 6.46-6.29 (m, 1H), 5.84 (d, J = 20.0 Hz, 1H), 5.44-5.28 (m, 2H), 5.19 (d, J = 19.9 Hz, 1H), 4.77 4.61 (m, 1H), 4.17-4.01 (m, 1H), 3.70-3.56 (m, 1H), 2.77-2.66 (m, 1H), 2.37-2.27 (m, 1H), 2.14-1.99 (m, 1H), 1.88-1.79 (m, 2H), 1.02 (d, J = 6.6 Hz, 3H), 0.95-0.78 (m, 9H), 0.77-0.65 (m, 2H).

Example 150: Preparation of (S)-11-((1-((3,3-difluorocyclobutyl)amino) cyclopropyl)methyl)-4-ethyl-8-fluoro-4-hydroxy-1,12-dihydro-14H-pyrano[3,4: 6,7]indolizino[2,1-b]quinoline-3,6,14(4H,11H,12H)-trione:

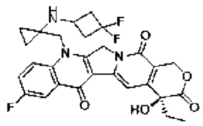

Intermediate 27 (prepared in Example 27) and commercially available tert-butyl (1-(aminomethyl)cyclopropyl) carbamate were used as the starting materials. According to the relevant operation procedures described in Example 105, the intermediate (S)-11-(((1-aminocyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione was synthesized, and obtained as a yellow solid. LCMS (ESI): $[M+H]^+$ = 451.9.

Using the above-prepared intermediate (S)-11-(((1-aminocyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione as the starting material, the title compound (hydrochloride salt) was synthesized according to the relevant operation procedures described in Example 107, and obtained as an off-white solid. LCMS (ESI): $[M+H]^+$ = 542.2; $^1H$ NMR (400 MHz, DMSO-$d_6$/$D_2O$) δ: 8.23-8.20 (m, 1H), 7.99 (dd, J = 8.8, 3.0 Hz, 1H), 7.81-7.76 (m, 1H), 7.29 (s, 1H), 5.42-5.31 (m, 2H), 4.83 (br s, 2H), 3.78-3.70 (m, 4H), 2.98 (br s, 1H), 2.68-2.33 (m, 2H), 1.87-1.77 (m, 2H), 0.98-0.85 (m, 5H), 0.60-0.53 (m, 2H).

Example 151: Preparation of (S)-11-((1-(cyclopentylamino)cyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione:

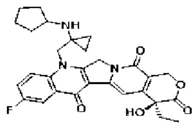

Similarly, using the intermediate (S)-11-(((1-aminocyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione (prepared as described above) as the starting material, the title compound (hydrochloride salt) was synthesized according to the relevant operation procedures described in Example 150, and obtained as an off-white solid. LCMS (ESI): $[M+H]^+$ = 520.2; $^1H$ NMR (400 MHz, DMSO-$d_6$) δ: 9.99 (br s, 2H), 8.32 (dd, J = 9.3, 4.1 Hz, 1H), 7.98 (dd, J = 8.7, 3.2 Hz, 1H), 7.84-7.79 (m, 1H), 7.29 (s, 1H), 5.40-5.31 (m, 3H), 5.20-5.00 (m, 2H), 3.96-3.93 (m, 1H), 2.27-2.15 (m, 2H), 1.87-1.76 (m, 7H), 1.70-1.59 (m, 2H), 1.20-1.13 (m, 2H), 0.87 (t, J = 7.3 Hz, 3H), 0.57-0.52 (m, 2H).

Example 152: Preparation of (S)-11-((1-((cyclopropylmethyl) amino)cyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione:

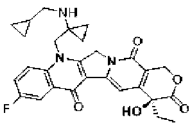

Similarly, using the intermediate (S)-11-(((1-aminocyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione (prepared as described above) as the starting material, the title compound (hydrochloride salt) was synthesized according to the relevant operation procedures described in Example 150, and obtained as an off-white solid. LCMS (ESI) $[M+H]^+$= 505.7. $^1$H NMR (400 MHz, DMSO-$d_6$) δ 9.99 (brs., 2H), 8.34 (dd, J = 9.6, 4.1 Hz, 1H), 7.97 (dd, J = 8.7, 3.2 Hz, 1H), 7.83-7.78 (m, 1H), 7.28 (s, 1H), 5.40-5.30 (m, 2H), 5.15-5.07 (m, 1H), 3.28-3.24 (m, 2H), 3.24-3.13 (m, 3H), 1.87-1.77 (m, 2H), 1.24-1.19 (m, 2H), 1.00 (d, *J* = 6.3 Hz, 4H), 0.87 (t, *J* = 7.3 Hz, 3H), 0.70-0.65 (m, 1H), 0.52-0.47 (m, 2H).

Example 153: Preparation of (S)-11-((1-(cyclopentyl(methyl) amino)cyclopropyl)methyl)-4-ethyl-8-fluoro-4-hydroxy-1,12-dihydro-14H-pyrano[3,4:6,7]indolizino[2,1 -b]quinoline-3,6,14(4H,11H,12H)-trione:

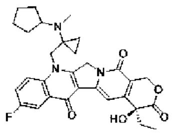

Similarly, using the compound prepared in Example 151 as the starting material, methylation reaction was carried out according to the relevant operation procedures described in Example 146 to synthesize the title compound (hydrochloride salt), which was obtained as a yellow solid. LCMS (ESI): $[M+H]^+$ = 534.2; $^1$H NMR (400 MHz, DMSO-$d_6$) δ: 11.17 (br s, 1H), 8.01-7.93 (m, 2H), 7.85-7.79 (m, 1H), 7.29 (s, 1H), 5.30-5.38 (m, 2H), 4.01-3.88 (m, 1H), 3.60-3.54 (m, 1H), 3.28-3.22 (m, 4H), 3.17-3.13 (m, 1H), 2.10-1.94 (m, 2H), 1.87-1.76 (m, 6H), 1.67-1.53 (m, 2H), 1.25-1.10 (m, 1H), 1.00 (d, J = 6.3 Hz, 4H), 0.87 (t, J = 7.4 Hz, 3H).

Example 154: Preparation of (S)-4-((4-ethyl-8-fluoro-10,11-dimethyl-3,6,14-trioxo-3,4,6,11,12,14-hexahydro-1H-pyrano[3,4:6,7]indolizino[2,1 -b]quinolin-4-yl)oxy)-4-formylpropanoic acid:

*Step 1: Preparation of (S)-4-((4-ethyl-7-(((6-fluoro-3-iodo-1,8-dimethyl-4-oxo-1,4-dihydroquinolin-2-yl)methyl)-3,8-dioxo-3,4,7,8-tetrahydro-1H-pyrano[3,4-c]pyridin-4-yl)oxy)-4-formylpropanoic acid:*

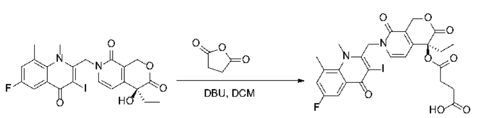


The intermediate (S)-4-ethyl-8-fluoro-4-hydroxy-10,11-dimethyl-1,12-dihydro-14H-pyrano[3,4:6,7]indolizino [2,1 - b]quinoline-3,6,14(4H,11H)-trione (120 mg, 0.22 mmol, 1.0 eq.) prepared in Step 8 of Example 2 was dissolved in anhydrous DCM (DCM, 12 mL). Cyclobutanecarboxylic anhydride (66.1 mg, 0.66 mmol, 3.0 eq.) and 1,8-diazabicyclo [5.4.0]undec-7-ene (DBU, 100 mg, 0.66 mmol, 3.0 eq.) were added sequentially. After the addition, the reaction was continued at room temperature for 30 minutes. The reaction solution was concentrated under reduced pressure to obtain a crude product, which was separated and purified by silica gel column chromatography (eluent: DCM:MeOH = 1:0 to 9:1, v/v) to afford the title compound as an off-white solid (140 mg, yield: 99.5%). LCMS(ESI)$[M+1]^+$=639.1, $t_R$ =1.450 min.

*Step 2: Preparation of (S)-4-((4-ethyl-8-fluoro-10,11-dimethyl-3,6,14-trioxo-3,4,6,11,12,14-hexahydro-1H-pyrano [3,4:6,7]indolizino[2,1 -b]quinolin-4-yl)oxy)-4-formylpropanoic acid:*

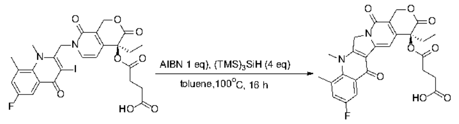


The intermediate prepared in step 1 (130 mg, 0.2 mmol, 1.0 eq.) was dissolved in toluene (13 mL) under nitrogen protection. Then, 2,2 -azobis(isobutyronitrile) (AIBN, 32.8 mg, 0.19 mmol, 0.95 eq.) and tris(trimethylsilyl)silane (199 mg, 0.80 mmol, 4.0 eq.) were added sequentially. After the addition, the reaction mixture was heated to 100 °C and stirred for 16 hours. LCMS detection indicated that the reaction was complete. The reaction solution was concentrated under reduced pressure to obtain a crude product, which was separated and purified by silica gel column chromatography (eluent: DCM:MeOH = 1:0 to 10:1, v/v) to afford a crude product (24 mg). The crude product was then triturated with MeOH, and the filter cake was lyophilized to obtain the title compound (10 mg, yield: 9.79%, off-white solid). LCMS (ESI): $[M+H]^+$ = 511.1, t = 1.412 min. $^1$H NMR (400 MHz, DMSO-$d_6$) δ: 12.24 (s, 1H), 7.87 (dd, J = 8.4, 3.2 Hz, 1H), 7.63 (dd, J = 9.1, 3.1 Hz, 1H), 6.87 (s, 1H), 5.51-5.28 (m, 4H), 4.09 (s, 3H), 2.87 (s, 3H), 2.76-2.64 (m, 2H), 2.49-2.42 (m, 2H), 2.15-2.01 (m, 2H), 0.90 (t, J = 7.4 Hz, 3H).

Example 155: Preparation of (S)-5-((4-ethyl-8-fluoro-10,11-dimethyl-3,6,14-trioxo-3,4,6,11,12,14-hexahydro-1H-pyrano[3,4:6,7]indolizino[2,1 -b]quinolin-4-yl)oxy)-5-formylpentanoic acid:

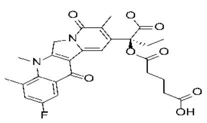

Similarly, the title compound was obtained according to the synthetic method described in Example 154 (7 mg, yield: 7.84%, off-white solid). LCMS(ESI)$[M+1]^+$ =525.3, $t_R$ =1.493 min. $^1$H NMR (400 MHz, DMSO-$d_6$) δ 7.88 (dd, *J* = 8.4, 3.2 Hz, 1H), 7.64 (dd, *J* = 9.0, 3.2 Hz, 1H), 6.86 (s, 1H), 5.49 - 5.35 (m, 4H), 4.10 (s, 3H), 2.88 (s, 3H), 2.61 - 2.53 (m, 2H), 2.33 (t, *J* = 7.4 Hz, 2H), 2.18 - 2.01 (m, 2H), 1.86 - 1.71 (m, 2H), 0.93 (t, *J* = 7.4 Hz, 3H).

Example 156: Preparation of (S)-4-ethyl-8-fluoro-10,11-dimethyl-3,6,14-trioxo-3,4,6,11,12,14-hexahydro-1H-pyrano [3,4:6,7]indolizino[2,1 -b]quinolin-4-yl 2-hydroxyacetate:

*Step 1: Preparation of (S)-7-(((3-bromo-6-fluoro-1,8-dimethyl-4-oxo-1,4-dihydroquinolin-2-yl)methyl)-4-ethyl-3,8-dioxo-3,4,7,8-tetrahydro-1H-pyrano[3,4-c]pyridin-4-yl 2-(benzyloxy)acetate:*

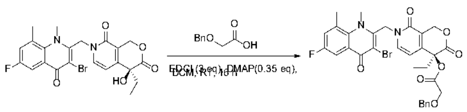


The intermediate (S)-4-ethyl-8-fluoro-4-hydroxy-10,11-dimethyl-1,12-dihydro-14H-pyrano[3,4:6,7]indolizino [2,1 - b]quinoline-3,6,14(4H,11H)-trione (500 mg, 1.05 mmol, 1.0 eq.) prepared in Step 8 of Example 2 was dissolved in DCM (5 mL). Then, 2-(benzyloxy)acetic acid (405 mg, 2.11 mmol, 2.0 eq.), N,N-diisopropylcarbodiimide (DIC, 461 mg, 3.15 mmol, 3.0 eq.) and 4-dimethylaminopyridine (DMAP, 53 mg, 0.37 mmol, 0.35 eq.) were added sequentially. The reaction mixture was stirred at room temperature for 16 hours. LCMS monitoring indicated that the reaction was complete. The reaction solution was poured into ice water (30 mL) and extracted with DCM (20 mL × 3). The organic phases were combined, dried over anhydrous sodium sulfate, filtered, and concentrated under reduced pressure to obtain a crude product. The crude product was separated and purified by silica gel column chromatography (eluent: DCM:MeOH = 1:0 to 20:1, v/v) to afford the title compound (600 mg, yield: 92.1%, white solid). LCMS (ESI): $[M+H]^+$ = 641.1, $t_R$ = 1.821 min.

Step 2: Preparation of (S)-4-ethyl-8-fluoro-10,11-dimethyl-3,6,14-trioxo-3,4,6,11,12,14-hexahydro-1H-pyrano[3,4:6,7] indolizino[2,1 -b]quinolin-4-yl 2-(benzyloxy)acetate:

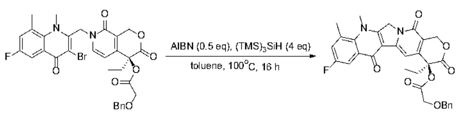


The intermediate prepared in step 1 (600 mg, 0.94 mmol, 1.0 eq.) was dissolved in toluene (60 mL). Under nitrogen protection, AIBN (146 mg, 0.94 mmol, 1.0 eq.) and tris(trimethylsilyl)silane (932 mg, 3.67 mmol, 4.0 eq.) were added sequentially. The reaction mixture was heated to 100 °C and stirred for 16 hours. The reaction solution was concentrated to obtain a crude product, which was separated and purified by silica gel column chromatography (eluent: DCM:MeOH = 1:0 to 10:1, v/v) to afford the title compound (150 mg, yield: 28.58%, off-white solid). LCMS (ESI): $[M+H]^+$ = 559.3.

*Step 3: Preparation of (S)-4-ethyl-8-fluoro-10,11-dimethyl-3,6,14-trioxo-3,4,6,11,12,14-hexahydro-1H-pyrano[3,4:6,7] indolizino[2,1 -b]quinolin-4-yl 2-hydroxyacetate:*

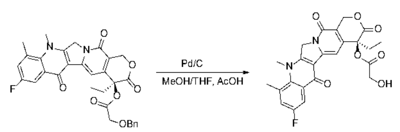


The intermediate prepared in step 2 (150 mg, 0.27 mmol, 1.0 eq.) was dissolved in a mixed solvent of MeOH (20 mL) and tetrahydrofuran (THF, 20 mL). Two drops of acetic acid and 10% palladium on carbon (Pd/C, 15 mg) were added, and the air in the reaction system was replaced with hydrogen. The reaction was stirred at room temperature for 16 hours under a hydrogen balloon. The reaction solution was filtered to remove Pd/C, and the filtrate was concentrated under reduced pressure to obtain a crude product. The crude product was separated and purified by reversed-phase preparative HPLC to afford the title compound (4.1 mg, yield: 3.26%, white solid). LCMS (ESI) $[M+1]^+$ -469.2, $t_R$ =1.287 min。$^1$H NMR (400 MHz, DMSO-$d_6$) δ 7.87 (dd, *J* = 8.5, 3.3 Hz, 1H), 7.64 (dd, *J* = 9.2, 3.0 Hz, 1H), 6.86 (s, 1H), 5.48 - 5.35 (m, 4H), 4.19 (d, *J* = 1.4 Hz, 2H), 4.09 (s, 3H), 2.87 (s, 3H), 2.13 - 2.01 (m, 3H), 0.91 (t, *J* = 7.4 Hz, 3H).

Example 157: Preparation of (S)-4-ethyl-8-fluoro-10,11-dimethyl-3,6,14-trioxo-3,4,6,11,12,14-hexahydro-1H-pyrano [3,4:6,7]indolizino[2,1 -b]quinolin-4-yl 2-aminoacetate:

*Step 1: Preparation of (S)-7-(((3-bromo-6-fluoro-1,8-dimethyl-4-oxo-1,4-dihydroquinolin-2-yl)methyl)-4-ethyl-3,8-dioxo-3,4,7,8-tetrahydro-1H-pyrano[3,4-c]pyridin-4-yl (tert-butoxycarbonyl)glycinate:*

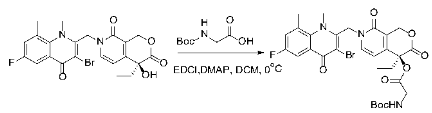


The intermediate (S)-4-ethyl-8-fluoro-4-hydroxy-10,11-dimethyl-1,12-dihydro-14H-pyrano[3,4:6,7]indolizino [2,1 - b]quinoline-3,6,14(4H,11H)-trione (1 g, 2.04 mmol, 1.0 eq.) prepared in Step 8 of Example 2 was dissolved in DCM (DCM, 10 mL). Then, (tert-butoxycarbonyl)glycine (710 mg, 4.08 mmol, 2.0 eq.), 1-(3-dimethylaminopropyl)-3-ethylcarbodiimide hydrochloride (EDC·HCl, 1.18 g, 6.12 mmol, 3.0 eq.) and 4-dimethylaminopyridine (DMAP, 90 mg, 0.71 mmol, 0.35 eq.) were added sequentially. After the addition, the reaction was continued to stir under ice bath for 2 hours. LCMS monitoring indicated that the reaction was complete. The reaction solution was poured into ice water (50 mL) and extracted with DCM (30 mL × 3). The organic phases were combined, dried over anhydrous sodium sulfate, filtered, and concentrated under reduced pressure to obtain a crude product. The crude product was separated and purified by silica gel column chromatography (eluent: DCM:MeOH = 1:0 to 20:1, v/v) to afford the title compound (750 mg, yield: 56.8%, white solid). LCMS (ESI): $[M+H]^+$ = 648.2, $t_R$ = 1.732 min.

*Step 2: Preparation of (S)-4-ethyl-8-fluoro-10,11-dimethyl-3,6,14-trioxo-3,4,6,11,12,14-hexahydro-1H-pyrano[3,4:6,7] indolizino[2,1 -b]quinolin-4-yl (tert-butoxycarbonyl)glycinate:*

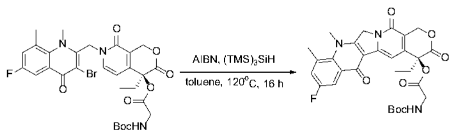


The intermediate prepared in step 1 (200 mg, 0.31 mmol, 1.0 eq.) was dissolved in toluene (20 mL). Under nitrogen protection, 2,2 -azobis(isobutyronitrile) (AIBN, 48.76 mg, 0.31 mmol, 1.0 eq.) and tris(trimethylsilyl)silane (295.33 mg, 1.19 mmol, 3.8 eq.) were added sequentially. The reaction mixture was heated to 100 °C and stirred for 16 hours. The reaction solution was concentrated under reduced pressure to obtain a crude product, which was separated and purified by silica gel column chromatography (eluent: DCM:MeOH = 1:0 to 10:1, v/v) to afford the title compound (60 mg, yield: 34.2%, off-white solid). LCMS (ESI): $[M+H]^+$ = 568.3.

*Step 3: Preparation of (S)-4-ethyl-8-fluoro-10,11-dimethyl-3,6,14-trioxo-3,4,6,11,12,14-hexahydro-1H-pyrano[3,4:6,7] indolizino[2,1 -b]quinolin-4-yl 2-aminoacetate:*

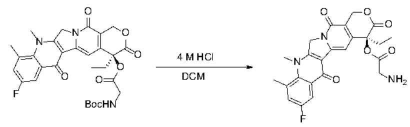


The intermediate prepared in step 2 (60 mg, 0.1 mmol, 1.0 eq.) was dissolved in DCM (1 mL). Under ice bath conditions, a 4 mol/L hydrochloric acid 1,4-dioxane solution (2 mL) was added slowly. The ice bath was removed, and the reaction was continued to stir at room temperature for 2 hours. LCMS detection indicated that the reaction was complete. The reaction solution was concentrated under reduced pressure to obtain a crude product, which was separated and purified by reversed-phase preparative HPLC to afford the title compound (20 mg, yield: 40.4%, off-white solid). LCMS(ESI)$[M+1]^+$=468.2。 $^1$H NMR (400 MHz, DMSO-$d_6$) δ 8.40 (s, 3H), 7.86 (dd, *J* = 8.3, 3.2 Hz, 1H), 7.65 (dd, *J* = 9.2, 3.0 Hz, 1H), 6.94 (s, 1H), 5.46 (d, *J* = 10.2 Hz, 4H), 4.11 (d, *J* = 8.7 Hz, 5H), 2.87 (d, *J* = 3.6 Hz, 3H), 2.16 (dq, *J* = 15.3, 7.2 Hz, 2H), 0.93 (t, *J* = 7.4 Hz, 3H).

Example 158: Preparation of (S)-4-((4-ethyl-8-fluoro-10-methoxy-11-methyl-3,6,14-trioxo-3,4,6,11,12,14-hexahydro-1H-pyrano[3,4:6,7]indolizino[2,1 -b]quinolin-4-yl)oxy)-4-formylbutanoic acid:

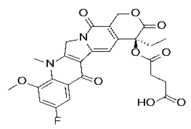

The title compound was obtained according to the method described in Example 154, and obtained as an off-white solid. LCMS (ESI):$[M+H]^+$=527.0, $t_R$=1.361 min; $^1$H NMR (400 MHz, DMSO-$d_6$) δ: 12.26 (s, 1H), 7.58 (dd, J = 8.6, 2.9 Hz, 1H), 7.45 (dd, J = 10.2, 2.9 Hz, 1H), 6.89 (s, 1H), 5.49-5.28 (m, 4H), 4.11 (s, 3H), 4.01 (s, 3H), 2.77-2.62 (m, 2H), 2.50-2.43 (m, 2H), 2.17-2.02 (m, 2H), 0.91 (t, J = 7.4 Hz, 3H).

General Synthetic Procedures for Examples 159-176:

Examples 159-172 were synthesized by two different synthetic routes based on different intermediates, and the specific synthetic principles are as follows:

Route 1 (for Examples 159-167): Using (Z)-1-(4-methyl-2,5-difluorophenyl)-3-(dimethylamino)but-2-en-1-one (Intermediate 29) and commercially available tert-butyl (2-aminoethyl)carbamate as the starting materials, the intermediate (S)-11-(2-aminoethyl)-4-ethyl-8-fluoro-4-hydroxy-9-methyl-1,12-dihydro-14H-pyrano[3',4': 6,7]indolizino[2,1-b] quinoline-3,6,14(4H,11H)-trione (prepared by referring to the synthetic method of Intermediate 29) was first obtained. Then, according to the relevant synthetic steps described in Example 104, the above intermediate was subjected to one or two reductive amination reactions with various aldehyde or ketone raw materials, or alkylation reactions with

various halogen-containing compounds, to synthesize a series of target compounds of Examples 159-167.

Route 2 (for Examples 168-172): Using (Z)-1-(2,5-difluoro-3-methylphenyl)-3-(dimethylamino)but-2-en-1-one (Intermediate 26) and commercially available tert-butyl (2-aminoethyl)carbamate as the starting materials, the intermediate (S)-11-(2-aminoethyl)-4-ethyl-8-fluoro-4-hydroxy-10-methyl-1,12-dihydro-14H-pyrano[3',4': 6,7]indolizino[2,1-b]quinoline-3,6,14(4H,11H)-trione was synthesized according to the same synthetic method as described in Examples 102 and 103. Then, according to the relevant synthetic steps described in Example 104, the above intermediate was subjected to reductive amination reactions with various aldehyde or ketone raw materials, or alkylation reactions with various halogen-containing compounds, to synthesize the target compounds of Examples 168-172.

Using intermediate (Z)-1-(4-chloro-2,5-difluorophenyl)-3-(dimethylamino)but-2-en-1-one (Intermediate 28) from Example 28 and the commercial reagent tert-butyl (2-aminoethyl)carbamate as starting materials, the intermediate (S)-11-(2-aminoethyl)-4-ethyl-8-fluoro-4-hydroxy-9-chloro-1,12-dihydro-14H-pyrano[3',4':6,7]indolizino[2,1-b]quinoline-3,6,14(4H,11H)-trione was synthesized according to the same methods as in Examples 102 and 103. Then, following the synthesis steps of Example 104, this intermediate was subjected to one or two reductive amination reactions with various aldehyde or ketone raw materials, or alkylation reactions with various halogen-containing compounds, to synthesize the compounds of Examples 173-176.

The compound structures and specific characterization data (LCMS and $^1$H NMR) are as follows:

| Examples | Structure | $^1$H NMR | LCMS (ESI) $[M+H]^+$ |
|---|---|---|---|
| 159 | 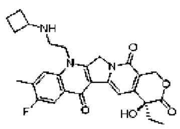 | (400 MHz, DMSO-$d_6$) δ 7.15-7.07 (m, 1H), 6.97-6.90 (m, 1H), 6.57 (s, 1H), 4.68-4.46 (m, 4H), 3.15-3.06 (m, 1H), 2.71-2.65 (m, 2H), 1.73-1.66 (m, 3H), 1.59-1.50 (m, 3H), 1.19-1.03 (m, 4H), 0.87-0.84 (m, 1H), 0.51-0.44 (m, 2H), 0.14-0.10 (m, 3H) | 494.3 |
| 160 | 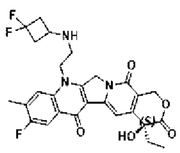 | (400 MHz, DMSO-$d_6$) δ 9.40 (s, 2H), 8.05 (d, *J* = 5.8 Hz, 1H), 7.98 (d, *J* = 9.6 Hz, 1H), 7.31 (s, 1H), 6.41 (s, 1H), 5.52 (s, 2H), 5.39 (d, *J* = 4.5 Hz, 2H), 4.65 (s, 2H), 3.81 (s, 1H), 3.47-3.43 (m, 3H), 3.02 (s, 6H), 1.88 - 1.81 (m, 2H), 0.88 (s, 3H)。 | 530.1 |
| 161 | 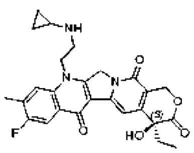 | (400 MHz, DMSO-$d_6$) δ 9.33 (s, 1H), 8.20-8.19 (m, 1H), 7.96-7.93 (m, 1H), 7.29-7.27 (m, 1H), 6.37 (s, 1H), 5.48-5.41 (m, 2H), 5.36-5.32 (m, 2H), 4.70-4.59 (m, 2H), 3.50-3.49 (m, 2H), 2.79-2.78 (m, 1H), 2.51 (s, 3H), 1.84-1.79 (m, 2H), 0.95-0.91(m, 2H), 0.88-0.84 (m, 3H), 0.78-0.75 (m, 2H)。 | 480.3 |
| 162 | 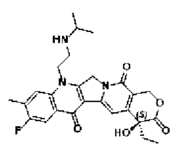 | (400 MHz, DMSO-$d_6$) δ 9.04-8.85 (br, 2H), 8.15 (d, J = 5.6 Hz, 1H), 7.91 (d, J = 9.6 Hz, 1H), 7.26 (s, 1H), 5.44 (s, 2H), 5.38-5.28 (m, 2H), 4.66-4.55 (m, 2H), 3.41-3.31 (m, 3H), 2.48 (s, 3H), 1.83-1.72 (m, 2H), 1.25-1.20 (m, 6H), 0.82 (t, J = 7.2 Hz, 3H) | 482.2 |
| 163 | 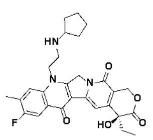 | N/A | 508.2 |
| 164 | 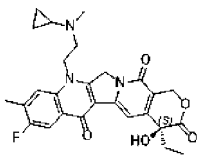 | (400 MHz, DMSO- $d_6$) δ 10.97 (s, 1H), 8.23 (s, 1H), 7.96-7.92 (m, 1H), 7.30 (s, 1H), 6.44-6.27 (m, 1H), 5.53-5.51 (m, 2H), 5.37-5.32 (m, 2H), 4.89-4.83 (m, 2H), 3.73-3.64 (m, 2H), 3.10-3.09 (m, 1H), 2.99-2.92 (m, 3H), 2.53-2.45 (m, 3H), 1.85-1.82 (m, 2H), 1.27-1.24 (m, 1H), 0.95-0.88 (m, 3H), 0.88-0.85 (m, 3H)。 | 494.2 |

(continued)

| Examples | Structure | $^1$H NMR | LCMS (ESI) $[M+H]^+$ |
|---|---|---|---|
| 165 | 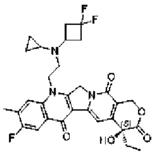 | 1H-NMR (400 MHz, DMSO-$d_6$) δ 7.95-7.93 (m, 2H), 7.28 (s, 1H), 5.47-5.39 (m, 2H), 5.36-5.31 (m, 2H), 4.65-4.57 (m, 2H), 3.70-3.63 (m, 1H), 3.27-3.24 (m, 2H), 2.87-2.84 (m, 2H), 2.67-2.64 (m, 1H), 2.51-2.47 (m, 3H), 2.44-2.33 (m, 1H), 1.83-1.81 (m, 2H), 1.26-1.24 (m, 1H), 0.88-0.87 (m, 3H), 0.73-0.42 (m, 4H)。 | 570.3 |
| 166 | 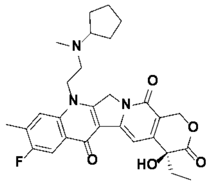 | (400 MHz, DMSO-$d_6$) δ 10.76 (s, 1H), 8.15 (d, $J$ = 5.3 Hz, 1H), 7.95 (d, $J$ = 9.5 Hz, 1H), 7.30 (s, 1H), 6.41 (d, $J$ = 9.5 Hz, 1H), 5.55 - 5.53 (m, 1H), 5.50 - 5.25 (m, 3H), 4.81 (d, $J$ = 65.1 Hz, 2H), 3.74 - 3.48 (m, 3H), 2.96 (s, 3H), 2.03-1.96 (m, 4H), 1.91-1.72 (m, 6H), 1.58 (d, $J$ = 8.3 Hz, 3H), 0.86 (d, $J$ = 2.1 Hz, 3H)。 | 522.2 |
| 167 | 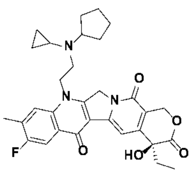 | (400 MHz, DMSO-$d_6$) δ 11.29 (s, 1H), 8.28 (s, 1H), 7.93 (d, $J$ = 9.5 Hz, 1H), 7.29 (s, 1H), 6.39 (s, 1H), 5.66 - 5.44 (m, 2H), 5.41 - 5.30 (m, 2H), 5.04 - 4.80 (m, 2H), 3.96 - 3.73 (m, 2H), 3.59 - 3.45 (m, 1H), 3.16 - 3.06 (m, 1H), 2.19 - 1.77 (m, 8H), 1.74 - 1.64 (m, 3H), 1.57 (s, 3H), 1.39 - 1.24 (m, 2H), 1.10 - 0.99 (m, 2H), 0.86 (t, $J$ = 7.3 Hz, 3H)。 | 548.3 |
| 168 | 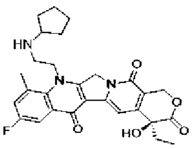 | (400 MHz, DMSO-$d_6$) δ 9.08 (d, $J$ = 32.7 Hz, 2H), 7.92 (dd, $J$ = 8.3, 3.3 Hz, 1H), 7.69 (dd, $J$ = 9.0, 3.2 Hz, 1H), 7.27 (s, 1H), 6.39 (s, 1H), 5.54 - 5.24 (m, 4H), 4.92 - 4.63 (m, 2H), 3.49 - 3.41 (m, 1H), 3.24 - 3.08 (m, 2H), 2.89 (s, 3H), 1.97 - 1.76 (m, 4H), 1.73 - 1.57 (m, 4H), 1.55 - 1.42 (m, 2H), 0.87 (t, $J$ = 7.3 Hz, 3H)。 | 508.3 |
| 169 | 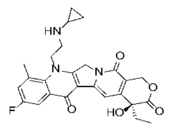 | N/A | 480.3 |
| 170 | 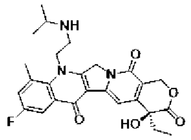 | (400 MHz, DMSO-$d_6$) δ 8.76 (s, 2H), 7.93 (dd, $J$ = 8.3, 3.3 Hz, 1H), 7.70 (dd, $J$ = 9.1, 3.2 Hz, 1H), 7.28 (s, 1H), 6.41 (s, 1H), 5.52 - 5.25 (m, 4H), 4.89 - 4.62 (m, 2H), 3.30 - 3.25 (m, 1H), 3.23 - 3.14 (m, 2H), 2.88 (s, 3H), 1.90 - 1.75 (m, 2H), 1.19 (dd, $J$ = 6.5, 3.4 Hz, 6H), 0.87 (t, $J$ = 7.3 Hz, 3H)。 | 483.2 |
| 171 | 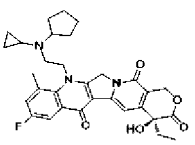 | (400 MHz, DMSO-$d_6$) δ 10.59 (s,1H),7.94 (dd $J$= 8.3, 3.2 Hz, 1H), 7.72 (d $J$= 6.4 Hz1H), 7.27(s,1H), 6.38(s,1H), 5.53-5.28(m,4H), 3.90-3.77 (m,1H),3.65-3.46(m 2H), 2.92 (s,3H), 2.04-1.91(m,2H), 1.83-1.75 (m,5H), 1.70-1.54(m,2H), 1.57-1.37(m,4H), 1.08-0.91(m,2H), 0.91-0.79(m,5H)。 | 548.1 |
| 172 | 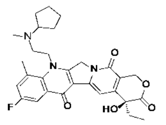 | (400 MHz, DMSO-$d_6$) δ 10.40 (s, 1H), 7.93 (dd, $J$ = 8.3, 3.0 Hz, 1H), 7.71 (dd, $J$ = 8.9, 3.1 Hz, 2H), 7.28 (s, 1H), 6.40 (s, 1H), 5.53 - 5.24 (m, 4H), 5.13 - 4.63 (m, 2H), 3.73 - 3.53 (m, 1H), 2.92 - 2.80 (m, 4H), 2.04 - 1.89 (m, 3H), 1.87 - 1.76 (m, 3H), 1.73 - 1.56 (m, 4H), 1.57 - 1.39 (m, 3H), 0.92 - 0.79 (m, 3H)。 | 522 |
| 173 | 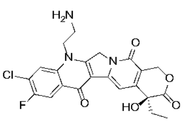 | (400 MHz, DMSO-$d_6$) δ 8.42 (d, $J$ = 5.8 Hz, 1H), 8.18 (d, $J$ = 9.1 Hz, 2H), 8.15 (s, 3H), 7.28 (s, 1H), 6.41 (s, 1H), 5.46 (s, 2H), 5.39 - 5.31 (m, 2H), 4.68 - 4.55 (m, 2H), 3.32 - 3.23 (m, 2H), 1.88 - 1.76 (m, 2H), 0.86 (t, $J$ = 7.3 Hz, 3H)。 | 460.1 |

(continued)

| Examples | Structure | $^1$H NMR | LCMS (ESI) $[M+H]^+$ |
|---|---|---|---|
| 174 | 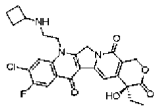 | (400 MHz, DMSO-$d_6$) δ 9.36 (s, 1H), 8.47-8.46 (m, 1H), 8.17-8.15 (m, 1H), 8.06 (s, 1H), 7.27 (s, 1H), 6.39 (s, 1H), 5.50-5.40 (m, 2H), 5.35-5.31 (m, 2H), 4.69-4.64 (m, 1H), 3.76-3.69 (m, 2H), 3.25-3.20 (m, 1H), 2.17-2.06 (m, 3H), 1.83-1.74 (m, 4H), 1.35-1.34 (m, 2H), 0.86-0.83 (m, 3H) | 514.2 |
| 175 | 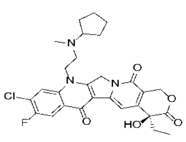 | (400 MHz, DMSO-$d_6$) δ 10.84 (s, 1H), 8.49 (d, *J* = 5.2 Hz, 1H), 8.17 (d, *J* = 9.0 Hz, 1H), 7.29 (s, 1H), 6.41 (s, 1H), 5.66 - 5.43 (m, 2H), 5.44 - 5.27 (m, 2H), 5.03 - 4.88 (m, 1H), 4.84 - 4.68 (m, 1H), 3.76 - 3.57 (m, 2H), 3.56 - 3.45 (m, 1H), 2.94 (s, 3H), 2.07 - 1.93 (m, 2H), 1.87 - 1.68 (m, 6H), 1.62 - 1.49 (m, 2H), 0.86 (t, *J* = 7.3 Hz, 3H)。 | 542.2 |
| 176 | 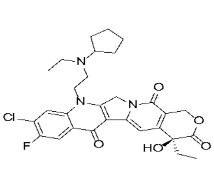 | (400 MHz, DMSO-$d_6$) δ 10.67 (s, 1H), 8.44 (dd, *J* = 5.8, 3.3 Hz, 1H), 8.18 (d, *J* = 9.1 Hz, 1H), 7.29 (s, 1H), 6.42 (s, 1H), 5.61 - 5.43 (m, 2H), 5.37 - 5.29 (m, 2H), 5.01 - 4.76 (m, 2H), 3.87 - 3.74 (m, 1H), 3.73 - 3.62 (m, 1H), 2.14 - 2.05 (m, 1H), 2.03 - 1.95 (m, 1H), 1.85 - 1.67 (m, 7H), 1.61 - 1.51 (m, 2H), 1.40 - 1.32 (m, 3H), 0.90 - 0.79 (m, 5H)。 | 556.3 |

**General Synthetic Procedures for Example 177-Example 181:**

Using commercial reagents 6-amino-3,4-methylenedioxyacetophenone and tert-butyl (2-aminoethyl)carbamate as the starting materials, the intermediate tert-butyl (S)-(2-(12-ethyl-12-hydroxy-8,11,14-tricarbonyl-6,11,12,14-tetrahydro-8H-[1,3]dioxolano[4,5-g]indolizino[3',4':6,7]indolizino[2,1-b]quinolin-5(9H)-yl)ethyl)carbamate was synthesized according to the method described in Steps 1-7 of Example 31. Then, the intermediate (S)-5-(2-aminoethyl)-12-ethyl-12-hydroxy-9,12-dihydro-8H-[1,3]dioxolano[4,5-g]pyrano[3',4':6,7]indolizino[2,1-b]quinoline-8,11,14(5H,6H)-trione was obtained by removing the Boc group under acidic conditions. Subsequently, according to the synthetic steps described in Example 104, the above intermediate was subjected to one or two reductive amination reactions with various aldehyde or ketone raw materials, or alkylation reactions with various halogen-containing compounds, to synthesize the compounds of Examples 177-180.

Using commercial reagents 2,5-difluoro-4-methoxyacetophenone and tert-butyl (2-aminoethyl)carbamate as the starting materials, the intermediate (S)-11-(2-aminoethyl)-4-ethyl-8-fluoro-4-hydroxy-9-methoxy-1,12-dihydro-14H-pyrano[3',4':6,7]indolizino[2,1-b]quinoline-3,6,14(4H,11H)-trione was synthesized according to the method described in Steps 1-7 of Example 31. Then, according to the synthetic steps described in Example 104, the intermediate (S)-11-(2-aminoethyl)-4-ethyl-8-fluoro-4-hydroxy-9-methoxy-1,12-dihydro-14H-pyrano[3',4':6,7]indolizino[2,1-b]quinoline-3,6,14(4H,11H)-trione was subjected to one or two reductive amination reactions with various aldehyde or ketone raw materials, or alkylation reactions with various halogen-containing compounds, to synthesize the compound of Example 181.

The compound structures and specific characterization data (LCMS and $^1$H NMR) are as follows:

| Examples | Structure | $^1$H NMR | LCMS (ESI) $[M+H]^+$ |
|---|---|---|---|
| 177 | 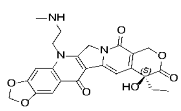 | (400 MHz, DMSO-d6) δ 8.85 (s, 2H), 7.67 (s, 1H), 7.66 (s, 1H), 7.29 (s, 1H), 6.27 (s, 2H), 5.42 (s, 2H), 5.40-5.30 (m, 2H), 4.63-4.54 (m, 2H), 3.41-3.33 (m, 2H), 2.62 (s, 3H), 1.87-1.77 (m, 2H), 0.86 (t, J = 7.2 Hz, 3H)。 | 466.2 |
| 178 | 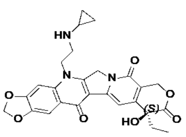 | (400 MHz, DMSO- *d6*) δ 9.29 (br, s, 2H), 7.69 (s, 1H), 7.63 (s, 1H), 7.26 (s, 1H), 6.24 (s, 2H), 5.40 (s, 2H), 5.37-5.27 (m, 2H), 4.58-4.51 (m, 2H), 3.48-3.44 (m, 2H), 2.79-2.72 (m, 1H), 1.83-1.73 (m, 2H), 0.94-0.88 (m, 2H), 0.83 (t, J = 7.2 Hz, 3H), 0.76-0.71 (m, 2H)。 | 492.5 |

(continued)

| Examples | Structure | $^1$H NMR | LCMS (ESI) $[M+H]^+$ |
|---|---|---|---|
| 179 | 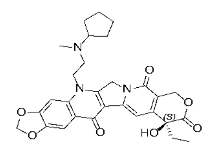 | (400 MHz, DMSO-d6) δ 11.11-10.71 (, s, 1H), 7.75 (d, J = 12.4 Hz, 1H), 7.65 (s, 1H), 7.29 (s, 1H), 6.27 (d, J = 5.2 Hz, 2H), 5.51-5.30 (m, 4H), 4.88-4.63 (m, 2H), 3.77-3.71 (m, 1H), 3.52-3.41 (m, 2H), 2.94 (d, J = 2.0 Hz, 3H), 2.06-1.49 (m, 10H), 0.86 (t, J = 7.2 Hz, 3H)。 | 534.3 |
| 180 | 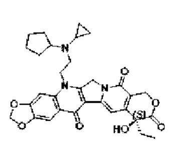 | (400 MHz, DMSO-*d6*) δ 10.72 (br, s, 1H), 7.69 (s, 1H), 7.63 (s, 1H), 7.27 (s, 1H), 6.35-6.25 (br, m, 1H), 6.24 (s, 2H), 5.51-5.36 (m, 2H), 5.32-5.25 (m, 2H), 4.87-4.73 (m, 2H), 3.94-3.70 (m, 2H), 3.55-3.48 (m, 1H), 3.15-3.08 (m, 1H), 2.12-1.48 (m, 10H), 1.23-0.98 (m, 4H), 0.83 (t, J = 7.2 Hz, 3H)。 | 560.5 |
| 181 | 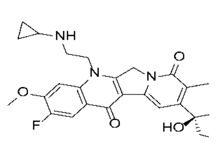 | (400 MHz, DMSO-*d6*) δ 9.40 (s, 2H), 8.01 (d, *J* = 11.3 Hz, 1H), 7.54 (d, *J* = 6.9 Hz, 1H), 7.28 (s, 1H), 6.39 (s, 1H), 5.49 (s, 2H), 5.41 - 5.32 (m, 2H), 4.79 - 4.64 (m, 2H), 4.16 (s, 3H), 3.57 - 3.49 (m, 2H), 1.88 - 1.77 (m, 2H), 1.66 - 1.61 (m, 1H), 0.99 - 0.92 (m, 2H), 0.90 - 0.86 (m, 3H), 0.79 - 0.75 (m, 2H)。 | 496.3 |

**Example 182: Preparation of (S)-11-(2-(5-azaspiro[2.3]hexan-5-yl)ethyl)-4-ethyl-8-fluoro-4-hydroxy-1,12-dihydro-14H-pyrano[3',4':6,7]indolizino[2,1-b]quinoline-3,6,14(4H,11H)-trione:**

***Step 1: Preparation of 3-bromo-2-(bromomethyl)-1-(2,2-dimethoxyethyl)-6-fluoroquinolin-4(1H)-one:***

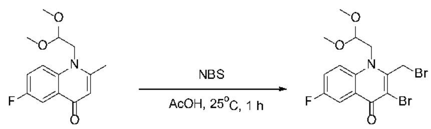


Intermediate 37 (30.7 g, 115.73 mmol, 1.0 eq.) from Preparation Example 37 was dissolved in acetic acid (310 mL). N-Bromosuccinimide (NBS, 51.5 g, 289.32 mmol, 2.5 eq.) was added portionwise at room temperature, and the color of the reaction mixture faded subsequently. The reaction was stirred at room temperature for 1 hour, and LCMS monitoring indicated the reaction was complete. The reaction mixture was poured into ice-cold saturated sodium bicarbonate solution, and the pH was adjusted to 8-9. The mixture was extracted with ethyl acetate (500 mL × 2), and the combined organic phases were washed with saturated sodium chloride solution (500 mL × 5), dried over anhydrous sodium sulfate, filtered and concentrated under reduced pressure to afford a crude product. The crude product was purified by silica gel column chromatography (eluent: DCM:MeOH = 1:0 to 100:3, v/v) to give the title compound as an orange solid (45.7 g, yield: 93.34%). LCMS (ESI): $[M+H]^+$ = 421.1.

***Step 2: Preparation of (S)-7-((3-bromo-1-(2,2-dimethoxyethyl)-6-fluoro-4-oxo-1,4-dihydroquinolin-2-yl) methyl)-4-ethyl-4-hydroxy-1, 7-dihydro-3H-pyrano[3,4-c]pyridine-3,8(4H)-dione:***

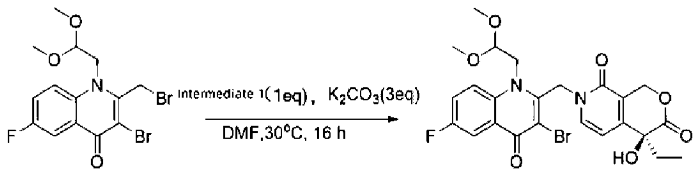


The intermediate prepared in step 1 (45 g, 106.37 mmol, 1.0 eq.) was dissolved in N,N-dimethylformamide (DMF, 450 mL). Then Intermediate 1 (22.25 g, 106.37 mmol, 1.0 eq.) and potassium carbonate (44.10 g, 319.1 mmol, 3.0 eq.)

were added sequentially. After the addition, the reaction mixture was heated to 30 °C and stirred for 16 hours under nitrogen protection. LCMS monitoring indicated the reaction was complete. The reaction mixture was filtered, and the filtrate was poured into ice-cold saturated aqueous ammonium chloride solution (300 mL). The mixture was extracted with ethyl acetate (300 mL × 2), and the combined organic phases were washed with saturated aqueous sodium chloride solution (300 mL × 4), dried over anhydrous sodium sulfate, filtered and concentrated to afford a crude product. The crude product was purified by silica gel column chromatography (eluent: DCM:MeOH = 1:0 to 100:2, v/v) to give the title compound as a white solid (42 g, yield: 71.61%). LCMS (ESI): $[M+H]^+$ = 551.0/553.2; HPLC: 96.58% (220 nm). $^1H$ NMR (400 MHz, DMSO-$d_6$) δ: 8.06 (dd, J = 9.7, 4.2 Hz, 1H), 7.90 (dd, J = 8.7, 3.2 Hz, 1H), 7.74 (ddd, J = 9.5, 7.7, 3.2 Hz, 1H), 7.54 (d, J = 7.2 Hz, 1H), 6.43 (d, J = 7.2 Hz, 1H), 6.32 (s, 1H), 5.60 (s, 2H), 5.28 (s, 2H), 4.66 (d, J = 3.9 Hz, 2H), 3.28 (s, 6H), 1.76 (p, J = 7.0 Hz, 2H), 0.79 (t, J = 7.3 Hz, 3H).

***Step 3: Preparation of (S)-11-(2,2-dimethoxyethyl)-4-ethyl-8-fluoro-4-hydroxy-1,12-dihydro-14H-pyrano [3',4':6,7]indolizino[2,1-b]quinoline-3,6,14(4H,11H)-trione:***

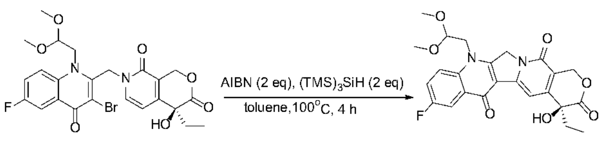


The intermediate prepared in step 2 (11 g, 19.95 mmol, 1.0 eq.) was dissolved in toluene (550 mL). Under nitrogen protection, the temperature was raised to an internal temperature of 100 °C, and the substrate was partially insoluble. A mixed solution consisting of 2,2'-azobis(isobutyronitrile) (AIBN, 6.552 g, 39.9 mmol, 2.0 eq.), tris(trimethylsilyl)silane (9.922 g, 39.9 mmol, 2.0 eq.) and toluene (220 mL) was then added dropwise over 2 hours. The reaction was continued at an internal temperature of 100 °C for another 2 hours, and LCMS monitoring indicated the reaction was complete. The reaction mixture was cooled to 5 °C in an ice-water bath, and quenched by the dropwise addition of a 10% aqueous ascorbic acid solution (3 mL). The mixture was filtered, and the filter cake was the crude product, which was purified by silica gel column chromatography (eluent: DCM:MeOH = 1:0 to 100:2, v/v) to afford the title compound as a pale yellow solid (3.8 g, yield: 40.49%). LCMS (ESI): $[M+H]^+$ = 471.3; $^1H$ NMR (400 MHz, DMSO-$d_6$) δ: 8.09 (dd, J = 9.5, 4.2 Hz, 1H), 7.96 (dd, J = 8.9, 3.1 Hz, 1H), 7.76 (ddd, J = 9.4, 7.7, 3.2 Hz, 1H), 7.28 (s, 1H), 6.35 (s, 1H), 5.46-5.27 (m, 4H), 4.84 (t, J = 5.1 Hz, 1H), 4.60 (t, J = 4.4 Hz, 2H), 3.35 (d, J = 1.3 Hz, 6H), 1.81 (qd, J = 6.9, 3.3 Hz, 2H), 0.86 (t, J = 7.3 Hz, 3H).

***Step 4: Preparation of (S)-2-(4-ethyl-8-fluoro-4-hydroxy-3,6,14-trioxo-4,6,12,14-tetrahydro-1H-pyrano[3',4':6,7] indolizino[2,1-b]quinolin-11(3H)-yl)acetaldehyde:***

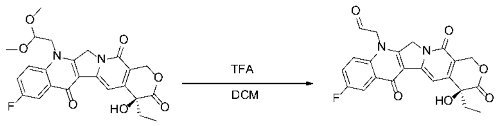


The intermediate prepared in Step 3 (200 mg, 0.43 mmol, 1.0 eq.) was dissolved in DCM (DCM, 1 mL), followed by the addition of trifluoroacetic acid (TFA, 5 mL). After the addition, the reaction mixture was heated to 50 °C and stirred for 16 hours. LCMS monitoring indicated the reaction was complete. The reaction mixture was concentrated under reduced pressure to afford the title compound as a yellow solid (150 mg, yield: 83.14%). LCMS (ESI): $[M+H]^+$ = 443.1 ($M+H_2O$).

***Step 5: Preparation of (S)-11-(2-(5-azaspiro[2.3]hexan-5-yl)ethyl)-4-ethyl-8-fluoro-4-hydroxy-1,12-dihydro-14H-pyrano[3',4':6,7]indolizino[2,1-b]quinoline-3,6,14 (4H,11H)-trione:***

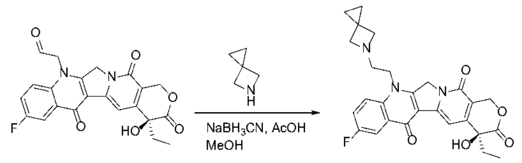


The intermediate prepared in step 4 (50 mg, 0.12 mmol, 1.0 eq.) was dissolved in DCM (DCM, 5 mL). Molecular sieves 4Å (50 mg), acetic acid (21 mg, 0.35 mmol, 3.0 eq.) and 5-azaspiro[2.3]hexane hydrochloride (28 mg, 0.24 mmol, 2.0 eq.) were added subsequently. After the addition, the reaction mixture was stirred at room temperature for 30 minutes, then sodium triacetoxyborohydride (62 mg, 0.29 mmol, 2.5 eq.) was added, and the mixture was stirred at room temperature for another 16 hours. Under an ice bath, sodium cyanoborohydride (7 mg, 0.12 mmol, 1.0 eq.) was added, and the reaction was stirred at room temperature for an additional 2 hours after the addition. LCMS monitoring indicated the reaction was complete. MeOH (2 mL) was added to the reaction mixture, followed by filtration. The filtrate was quenched with 0.1% formic acid aqueous solution (0.2 mL), and DCM was removed by rotary evaporation at low temperature. The crude product was directly purified by reversed-phase preparative HPLC to afford the title compound as a white solid (4 mg, yield: 6.78%). LCMS (ESI): $[M+H]^+ = 492.3$, $t_R = 1.131$ min; $^1H$ NMR (400 MHz, DMSO-$d_6$) δ: 11.21 (s, 1H), 8.24 (dd, J = 9.5, 4.1 Hz, 1H), 8.02 (dd, J = 8.8, 3.1 Hz, 1H), 7.91-7.74 (m, 1H), 7.30 (s, 1H), 6.40 (s, 1H), 5.56-5.41 (m, 2H), 5.40-5.27 (m, 2H), 4.69 (t, J = 7.0 Hz, 2H), 4.33-4.06 (m, 4H), 3.83-3.65 (m, 2H), 1.90-1.71 (m, 2H), 0.86 (t, J = 7.3 Hz, 3H), 0.83-0.56 (m, 4H).

### General Synthetic Procedures for Examples 183-194

Following the procedure described in step 5 of Example 182, the intermediate prepared in step 4 of Example 182 was subjected to reductive amination reactions with various amines to afford the target compounds of Examples 183-193.

Following the procedures described in steps 1-5 of Example 182 and using Intermediate 38 from Preparation Example 38 as the starting material, the intermediate (4S)-11-(2-(3,3-difluoro-8-azabicyclo[3.2.1]octan-8-yl)ethyl)-4-ethyl-8-fluoro-4-hydroxy-9-methyl-1,12-dihydro-14H-pyrano[3',4':6,7]indolizino[2,1-b]quinoline-3,6,14(4H,11H)-trione was first synthesized. This intermediate was then subjected to a reductive amination reaction with an amine to afford the target compound of Example 194.

The structures and detailed characterization data (LCMS and $^1H$ NMR) of the above compounds are as follows:

| Examples | Structure | $^1H$ NMR | LCMS (ESI) $[M+H]^+$ |
|---|---|---|---|
| 183 | 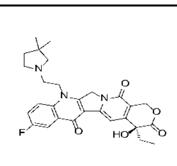 | (400 MHz, DMSO-$d_6$) δ 8.12 - 8.06 (m, 1H), 8.00 (dd, *J* = 8.9, 3.1 Hz, 1H), 7.83 - 7.74 (m, 1H), 7.29 (s, 1H), 6.34 (s, 1H), 5.54 - 5.30 (m, 4H), 4.66 - 4.43 (m, 2H), 3.42 - 3.33 (m, 2H), 3.15 - 2.94 (m, 2H), 2.54 - 2.51 (m, 2H), 1.87 - 1.77 (m, 2H), 1.65 - 1.45 (m, 2H), 1.01 (s, 6H), 0.86 (t, *J* = 7.3 Hz, 3H)。 | 508.3 |
| 184 | 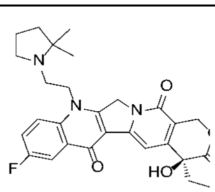 | (400 MHz, DMSO-$d_6$) δ 9.75 (s, 1H), 8.11 (dd, J = 9.4, 3.9 Hz, 1H), 8.03 (dd, J = 8.7, 3.0 Hz, 1H), 7.90 - 7.78 (m, 1H), 7.31 (s, 1H), 6.41 (s, 1H), 5.52 - 5.28 (m, 4H), 4.95 - 4.75 (m, 1H), 4.64 - 4.50 (m, 1H), 3.88 - 3.73 (m, 1H), 3.61 - 3.48 (m, 2H), 2.20 - 1.96 (m, 3H), 1.91 - 1.75 (m, 4H), 1.46 (d, J = 3.0 Hz, 3H), 1.18 (s, 3H), 0.86 (t, J = 7.3 Hz, 3H)。 | 508.3 |
| 185 | 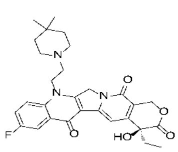 | (400 MHz, DMSO-$d_6$) δ 8.09 - 8.04 (m, 1H), 8.00 (dd, *J* = 8.9, 3.2 Hz, 1H), 7.82 - 7.74 (m, 1H), 7.30 (s, 1H), 6.35 (s, 1H), 5.52 (s, 2H), 5.36 (d, *J* = 5.8 Hz, 2H), 4.58 - 4.51 (m, 2H), 2.83 (s, 2H), 1.83 (dd, *J* = 7.4, 4.7 Hz, 2H), 1.30 - 1.25 (m, 4H), 0.97 (d, *J* = 9.8 Hz, 2H), 0.89 - 0.86 (m, 9H)。 | 522.2 |
| 186 | 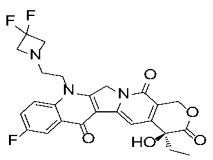 | (400 MHz, DMSO-$d_6$) δ 8.08 (dd, *J* = 9.4, 4.3 Hz, 1H), 7.99 (dd, *J* = 8.9, 3.2 Hz, 1H), 7.83 - 7.73 (m, 1H), 7.29 (s, 1H), 6.34 (s, 1H), 5.52 - 5.30 (m, 4H), 4.48 (t, *J* = 6.2 Hz, 2H), 3.65 (t, *J* = 11.7 Hz, 4H), 3.16 - 3.05 (m, 2H), 1.89 - 1.74 (m, 2H), 0.87 (t, *J* = 7.3 Hz, 3H)。 | 502.3 |

(continued)

| Examples | Structure | $^1$H NMR | LCMS (ESI) $[M+H]^+$ |
|---|---|---|---|
| 187 | 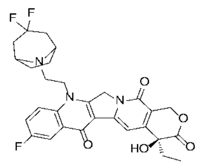 | (400 MHz, DMSO-$d_6$) δ 8.15 - 8.09 (m, 1H), 8.00 (dd, *J* = 8.9, 3.1 Hz, 1H), 7.82 - 7.75 (m, 1H), 7.29 (s, 1H), 6.37 (s, 1H), 5.61 - 5.46 (m, 2H), 5.41 - 5.32 (m, 2H), 4.62 - 4.47 (m, 2H), 3.02 - 2.75 (m, 2H), 2.06 - 1.73 (m, 10H), 1.69 - 1.60 (m, 2H), 0.86 (t, *J* = 8.9, 5.6 Hz, 3H)。 | 556.4 |
| 188 | 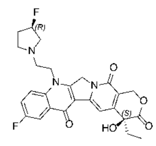 | (400 MHz, DMSO-$d_6$) δ 8.07 (dd, *J* = 9.5, 4.2 Hz, 1H), 8.00 (dd, J = 8.9, 3.1 Hz, 1H), 7.82 - 7.74 (m, 1H), 7.29 (s, 1H), 6.36 (s, 1H), 5.50 (s, 2H), 5.41 - 5.29 (m, 2H), 5.26 - 5.05 (m, 1H), 4.62 - 4.50 (m, 2H), 3.02 - 2.84 (m, 4H), 2.22 - 1.95 (m, 2H), 1.89 - 1.75 (m, 4H), 0.86 (t, *J* = 7.3 Hz, 3H)。 | 498.2 |
| 189 | 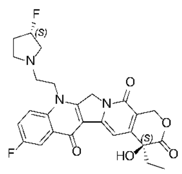 | (400 MHz, DMSO-$d_6$) δ 8.14 - 8.06 (m, 1H), 8.02 (dd, *J* = 8.8, 3.1 Hz, 1H), 7.84 - 7.76 (m, 1H), 7.31 (s, 1H), 6.39 (s, 1H), 5.57 - 5.45 (m, 2H), 5.41 - 5.28 (m, 3H), 4.69 - 4.46 (m, 2H), 3.15 - 2.77 (m, 4H), 2.28 - 2.00 (m, 2H), 1.92 - 1.72 (m, 4H), 0.88 (t, *J* = 7.3 Hz, 3H)o | 498.2 |
| 190 | 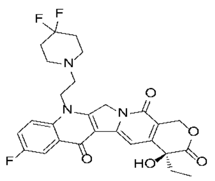 | (400 MHz, DMSO-$d_6$) δ 8.08 (dd, *J* = 9.5, 4.2 Hz, 1H), 8.01 (dd, *J* = 8.9, 3.1 Hz, 1H), 7.83 - 7.76 (m, 1H), 7.31 (s, 1H), 6.38 (s, 1H), 5.53 (s, 2H), 5.43 - 5.31 (m, 2H), 4.57 (t, *J* = 5.9 Hz, 2H), 2.88 (t, *J* = 5.9 Hz, 2H), 2.70 - 2.60 (m, 4H), 1.97 - 1.79 (m, 6H), 0.89 (t, *J* = 7.3 Hz, 3H)o | 530.2 |
| 191 | 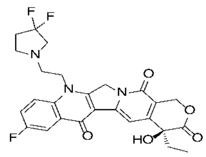 | (400 MHz, DMSO-$d_6$) δ 8.09 (dd, *J* = 9.3, 3.7 Hz, 1H), 8.01 (dd, *J* = 8.9, 3.1 Hz, 1H), 7.85 - 7.76 (m, 1H), 7.29 (s, 1H), 6.38 (s, 1H), 5.55 - 5.43 (m, 2H), 5.42 - 5.31 (m, 2H), 4.66 - 4.50 (m, 2H), 3.20 - 2.79 (m, 4H), 2.41 - 2.18 (m, 4H), 1.87 - 1.75 (m, 2H), 0.86 (t, *J* = 7.3 Hz, 3H) | N/A |
| 192 | 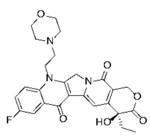 | N/A | 496.3 |
| 193 | 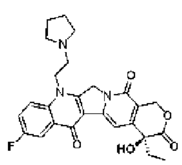 | (400 MHz, DMSO-$d_6$) δ 11.31 (s, 1H), 8.31 (dd, *J* = 9.3, 4.1 Hz, 1H), 8.03 (dd, *J* = 8.8, 3.0 Hz, 1H), 7.85-7.80 (m, 1H), 7.32 (s, 1H), 6.39 (s, 1H), 5.58 (s, 2H), 5.43-5.34 (m, 2H), 4.84 (t, *J*= 7.8 Hz, 2H), 3.73-3.59 (m, 4H), 3.24-3.11 (m, 2H), 2.17-2.03 (m, 2H), 1.99-1.90 (m, 2H), 1.88-1.78 (m, 2H), 0.89 (t, *J* = 7.3 Hz, 3H)o | 480.4 |
| 194 | 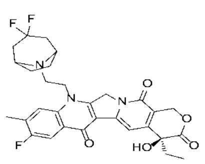 | (400 MHz, DMSO-$d_6$) δ 8.02 (d, *J* = 5.9 Hz, 1H), 7.92 (d, *J* = 9.6 Hz, 1H), 7.28 (s, 1H), 6.36 (s, 1H), 5.50 (s, 2H), 5.40 - 5.28 (m, 2H), 4.59 - 4.44 (m, 2H), 2.88 (t, *J* = 5.8 Hz, 2H), 2.46 (s, 3H), 1.96 - 1.73 (m, 10H), 1.70 - 1.60 (m, 2H), 0.86 (t, *J* = 7.3 Hz, 3H)。 | 470.3 |

**Example 195: Preparation of (S)-4-ethyl-8-fluoro-4-hydroxy-11-(4-(trifluoromethyl)phenyl)-1,12-dihydro-14H-pyrano[3',4':6,7]indolizino[2,1-b]quinoline-3,6,14(4H,11H)-trione:**

***Step 1: Preparation of 3-bromo-2-(bromomethyl)-6-fluoro-1-(4-(trifluoromethyl)phenyl)quinolin-4(1H)-one:***

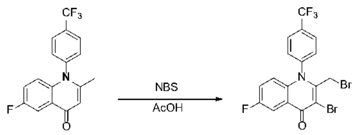


Intermediate 39 (870 mg, 2.71 mmol, 1.0 eq.) was dissolved in glacial acetic acid (8 mL), and N-bromosuccinimide (NBS, 1.2 g, 6.77 mmol, 2.5 eq.) was added. The reaction was stirred at room temperature for 16 hours, and LCMS monitoring indicated the reaction was complete. The reaction mixture was slowly poured into ice-cold saturated sodium bicarbonate solution (10 mL) under an ice bath, and the pH was adjusted to weakly basic. The mixture was extracted with ethyl acetate (15 mL × 3), and the combined organic phases were washed with saturated sodium chloride solution (10 mL × 3), dried over anhydrous sodium sulfate, filtered and concentrated under reduced pressure to afford a crude product. The crude product was purified by silica gel column chromatography (eluent: PE:EtOAc = 1:0 to 1:1, v/v) to give the title compound as a yellow solid (710 mg). LCMS (ESI): $[M+H]^+$ = 480.0, $t_R$ = 2.023 min.

***Step 2: Preparation of (S)-7-((3-bromo-6-fluoro-4-oxo-1-(4-(trifluoromethyl)phenyl)-1,4-dihydroquinolin-2-yl) methyl)-4-ethyl-4-hydroxy-1,7-dihydro-3H-pyrano[3,4-c]pyridine-3,8(4H)-dione:***

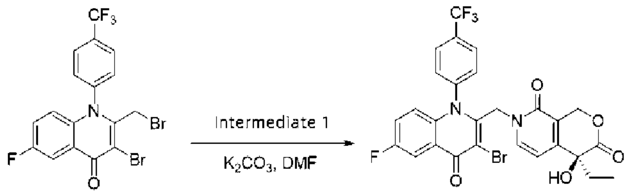


The intermediate prepared in step 1 (700 mg, 1.46 mmol, 1.0 eq.) was dissolved in N,N-dimethylformamide (DMF, 7 mL), and potassium carbonate (605.86 mg, 4.38 mmol, 3.0 eq.) and Intermediate 1 (305.68 mg, 1.46 mmol, 1.0 eq.) were added subsequently. The reaction mixture was stirred at room temperature for 16 hours, and LCMS monitoring indicated the reaction was complete. The mixture was filtered, and the filtrate was slowly poured into water (10 mL). The resulting mixture was extracted with ethyl acetate (10 mL × 3), and the combined organic phases were washed with saturated sodium chloride solution (10 mL × 3), dried over anhydrous sodium sulfate, filtered and concentrated under reduced pressure to afford a crude product. The crude product was purified by silica gel column chromatography (eluent: DCM:MeOH = 1:0 to 50:1, v/v) to give the title compound as a pale yellow solid (400 mg, yield: 45.11%). LCMS (ESI): $[M+H]^+$ = 609.1, $t_R$ = 1.728 min.

***Step 3: Preparation of (S)-4-ethyl-8-fluoro-4-hydroxy-11-(4-(trifluoromethyl) phenyl)-1,12-dihydro-14H-pyrano [3',4':6,7]indolizino[2,1-b]quinoline-3,6,14 (4H,11H)-trione:***

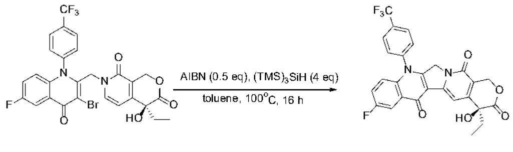


The intermediate prepared in step 2 (200 mg, 0.33 mmol, 1.0 eq.) was dissolved in toluene (10 mL). Under nitrogen protection, the reaction mixture was heated to 100 °C, followed by the dropwise addition of a mixed solution of 2,2'-azobis(isobutyronitrile) (AIBN, 108.15 mg, 0.66 mmol, 2.0 eq.) and tris(trimethylsilyl)silane (163.77 mg, 0.66 mmol, 2.0 eq.) in toluene (2 mL). After the addition, the reaction mixture was continuously stirred at 100 °C for 4 hours, and LCMS monitoring indicated the reaction was complete. The mixture was allowed to cool to room temperature and filtered, and the filter cake was directly purified by reversed-phase preparative HPLC to afford the title compound as a white solid (12 mg, yield: 6.91%). LCMS (ESI): $[M+H]^+$ = 526.9; $^1H$ NMR (400 MHz, DMSO-$d_6$) δ: 8.19 (d, J = 8.6 Hz, 2H), 8.09-7.92 (m, 3H), 7.65-7.57 (m, 1H), 7.33 (s, 1H), 7.09 (dd, J = 9.3, 4.3 Hz, 1H), 5.31 (s, 2H), 4.96-4.77 (m, 2H), 1.88-1.73 (m, 2H), 0.86 (t, J = 7.3 Hz, 3H).

**Example 196: Preparation of (S)-14-cyclopentyl-7-ethyl-3-fluoro-7-hydroxy-2-(2-hydroxyethyl)-10,14-dihydro-11H-pyrano[3',4':6,7]indolizino[2,1-b][1,8] naphthyridine-5,8,11(7H,13H)-trione:**

***Step 1: Preparation of (E)-2-chloro-6-(2-ethoxyvinyl)-5-fluoronicotinonitrile:***

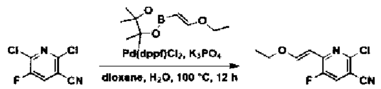


2,6-Dichloro-5-fluoronicotinonitrile (100 g, 523.59 mmol, 1.0 eq.) was dissolved in a mixed solvent of 1,4-dioxane (1000 mL) and water (20 mL). Under nitrogen protection, (E)-1-ethoxyvinyl-2-boronic acid pinacol ester (125 g, 628.31 mmol, 1.2 eq.), [1,1'-bis(diphenylphosphino)ferrocene]dichloropalladium DCM complex (19.21 g, 26.18 mmol, 0.05 eq.) and potassium phosphate (222.28 g, 1047.18 mmol, 2.0 eq.) were added sequentially. The reaction was stirred at 100 °C for 12 hours, and LCMS monitoring indicated the reaction was complete. The reaction mixture was cooled to room temperature, poured into water (300 mL) and extracted with ethyl acetate (500 mL × 2). The combined organic phases were washed with saturated aqueous sodium chloride solution (200 mL), dried over anhydrous sodium sulfate, filtered and concentrated to afford a crude product. Purification by silica gel column chromatography (eluent: PE:THF = 10:1, v/v) gave the title compound as a pale yellow solid (41.8 g, yield: 35.23%). LCMS (ESI): $[M+H]^+$ = 227.1, $t_R$ = 1.377 min.

***Step 2: Preparation of 2-chloro-5-fluoro-6-(2-oxoethyl)nicotinonitrile:***

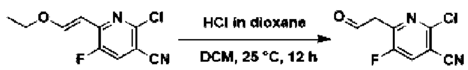


(E)-2-Chloro-6-(2-ethoxyvinyl)-5-fluoronicotinonitrile (30.0 g, 132.37 mmol, 1.0 eq.) was dissolved in DCM (150 mL), and 4 M hydrogen chloride in 1,4-dioxane (150 mL) was added. The reaction was stirred at 25 °C for 12 hours, and LCMS monitoring indicated the reaction was complete. The reaction mixture was quenched by adding water (200 mL), poured into saturated sodium bicarbonate solution (400 mL) and the pH was adjusted to 7-8. The mixture was extracted with ethyl acetate (500 mL × 2), and the combined organic phases were washed with saturated aqueous sodium chloride solution (200 mL), dried over anhydrous sodium sulfate, filtered and concentrated under reduced pressure to afford the title compound as a pale yellow solid (21.2 g, yield: 80.65%). LCMS (ESI): $[M+H]^+$ = 199.1, $t_R$ = 1.287 min.

***Step 3: Preparation of 2-chloro-5-fluoro-6-(2-hydroxyethyl)nicotinonitrile:***

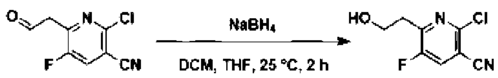


2-Chloro-5-fluoro-6-(2-oxoethyl)nicotinonitrile (21.2 g, 106.76 mmol, 1.0 eq.) was dissolved in a mixed solvent of tetrahydrofuran (THF, 100 mL) and DCM (100 mL), and the mixture was cooled to 0 °C. Sodium borohydride (8.08 g, 213.52 mmol, 2.0 eq.) was added portionwise. After the addition, the mixture was slowly warmed to 25 °C and stirred for 2 hours, and LCMS monitoring indicated the reaction was complete. The reaction mixture was poured into saturated aqueous ammonium chloride solution (100 mL) and extracted with ethyl acetate (200 mL × 2). The combined organic phases were washed with saturated aqueous sodium chloride solution (200 mL), dried over anhydrous sodium sulfate, filtered and concentrated under reduced pressure to afford a crude product. Purification by silica gel column chromatography (eluent: PE:THF = 8:1, v/v) gave the title compound as a pale yellow solid (15.1 g, 75.27 mmol, yield: 70.5%). LCMS (ESI): $[M+H]^+$= 201.1, $t_R$ = 1.003 min.

***Step 4: Preparation of 2-chloro-5-fluoro-6-(2-((tetrahydro-2H-pyran-2-yl)oxy)ethyl)nicotinonitrile:***

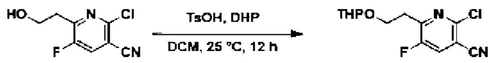


2-Chloro-5-fluoro-6-(2-hydroxyethyl)nicotinonitrile (32.3 g, 161 mmol, 1.0 eq.) was dissolved in DCM (300 mL) and cooled to 0 °C. p-Toluenesulfonic acid (2.76 g, 16.04 mmol, 0.1 eq.) and 3,4-dihydro-2H-pyran (26.9 g, 320.67 mmol, 2.0 eq.) were added sequentially. After the addition, the mixture was slowly warmed to 25 °C and stirred for 12 hours, and LCMS monitoring indicated the reaction was complete. The reaction mixture was poured into saturated aqueous sodium bicarbonate solution (100 mL) and extracted with ethyl acetate (200 mL × 2). The combined organic phases were washed with saturated aqueous sodium chloride solution (200 mL), dried over anhydrous sodium sulfate, filtered and concentrated to afford a crude product. Purification by silica gel column chromatography (eluent: PE:THF = 10:1, v/v) gave the title compound as a colorless oil (28.4 g, yield: 62.1%). $^1$H NMR (400 MHz, DMSO-$d_6$) δ: 8.55 (d, J = 8.4 Hz, 1H), 4.60-4.37 (m, 1H), 4.00-3.93 (m, 1H), 3.78-3.72 (m, 1H), 3.60-3.54 (m, 1H), 3.42-3.36 (m, 1H), 3.16-3.04 (m, 2H), 1.62-1.30 (m, 6H).

***Step 5: Preparation of 2-(cyclopentylamino)-5-fluoro-6-(2-((tetrahydro-2H-pyran-2-yl)oxy)ethyl)nicotinonitrile:***

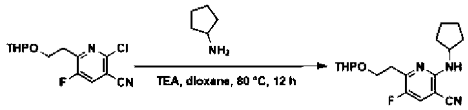


2-Chloro-5-fluoro-6-(2-((tetrahydro-2H-pyran-2-yl)oxy)ethyl)nicotinonitrile (10.0 g, 35.12 mmol, 1.0 eq.) was dissolved in 1,4-dioxane (100 mL). Cyclopentylamine (5.98 g, 70.24 mmol, 2.0 eq.) and triethylamine (7.10 g, 70.24 mmol, 2.0 eq.) were added sequentially. After the addition, the mixture was slowly warmed to 80 °C and stirred for 12 hours, and LCMS monitoring indicated the reaction was complete. The reaction mixture was poured into saturated aqueous sodium bicarbonate solution (100 mL) and extracted with ethyl acetate (200 mL × 2). The combined organic phases were washed with saturated aqueous sodium chloride solution (200 mL), dried over anhydrous sodium sulfate, filtered and concentrated to afford a crude product. Purification by silica gel column chromatography (eluent: PE:THF = 15:1, v/v) gave the title compound as a colorless oil (3.61 g, yield: 30.83%). LCMS (ESI): [M+H]$^+$=334.3, $t_R$ = 1.577 min.

***Step 6: Preparation of 1-(2-(cyclopentylamino)-5-fluoro-6-(2-((tetrahydro-2H-pyran-2-yl)oxy)ethyl)pyridin-3-yl) ethan-1-one:***

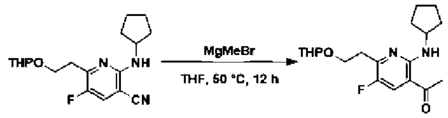


The intermediate prepared in step 5 (3.58 g, 10.74 mmol, 1.0 eq.) was dissolved in anhydrous tetrahydrofuran (THF, 30 mL) and cooled to 0 °C. Under nitrogen protection, 2 M methylmagnesium bromide in diethyl ether (25 mL, 5.0 eq.) was added dropwise. After the addition, the mixture was slowly warmed to 50 °C and stirred for 12 hours, and LCMS monitoring indicated the reaction was complete. The reaction mixture was cooled to room temperature, poured into saturated aqueous ammonium chloride solution (50 mL) and extracted with ethyl acetate (100 mL × 2). The combined organic phases were washed with saturated aqueous sodium chloride solution (100 mL), dried over anhydrous sodium sulfate, filtered and concentrated under reduced pressure to afford a crude product. Purification by silica gel column chromatography (eluent: PE:THF = 10:1, v/v) gave the title compound as a colorless oil (1.7 g, yield: 45.17%). LCMS (ESI): [M+H]$^+$ = 351.3, $t_R$= 2.491 min.

***Step 7: Preparation of ethyl 1-cyclopentyl-6-fluoro-7-(2-hydroxyethyl)-4-oxo-1,4-dihydro-1,8-naphthyridine-2-carboxylate:***

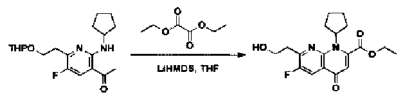


The intermediate prepared in step 6 (1.7 g, 4.85 mmol, 1.0 eq.) was dissolved in tetrahydrofuran (THF, 20 mL), and diethyl oxalate (1.77 g, 12.13 mmol, 2.5 eq.) was added. Under nitrogen protection at -65 °C, 1 M lithium bis(trimethylsilyl)amide in tetrahydrofuran (12.2 mL, 12.13 mmol, 2.5 eq.) was added dropwise. After the addition, the dry ice bath was retained, and the mixture was slowly warmed to room temperature and stirred overnight. LCMS monitoring indicated the reaction was complete. 0.1 M dilute hydrochloric acid (50 mL) was added to the reaction mixture and stirred for 2 hours, then the mixture was extracted with ethyl acetate (50 mL × 3). The combined organic phases were washed with saturated sodium chloride solution (200 mL), dried over anhydrous sodium sulfate, filtered and concentrated under reduced pressure to afford a crude product. Purification by silica gel column chromatography (eluent: PE:EtOAc = 1:0 to 10:1, v/v) gave the title compound as a yellow oil (1.5 g, yield: 88.76%). LCMS (ESI): $[M+H]^+$ = 349.1, $t_R$ = 1.600 min.

***Step 8: Preparation of ethyl 1-cyclopentyl-6-fluoro-4-oxo-7-(2-((tetrahydro-2H-pyran-2-yl)oxy)ethyl)-1,4-dihydro-1,8-naphthyridine-2-carboxylate:***

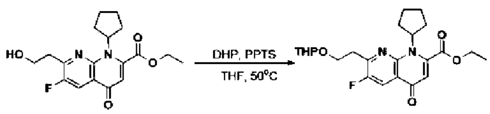


The intermediate prepared in step 7 (1.5 g, 4.31 mmol, 1.0 eq.) was dissolved in tetrahydrofuran (THF, 40 mL). 3,4-Dihydro-2H-pyran (1.01 g, 12.92 mmol, 3.0 eq.) and pyridinium p-toluenesulfonate (110 mg, 0.43 mmol, 0.1 eq.) were added sequentially. After the addition, the mixture was heated to 50 °C and stirred overnight, and LCMS monitoring indicated the reaction was complete. The reaction mixture was concentrated to afford a crude product. Purification by silica gel column chromatography (eluent: DCM:MeOH = 1:0 to 20:1, v/v) gave the title compound as a yellow oil (1.3 g, yield: 69.81%). LCMS (ESI): $[M+H]^+$ = 433.1, $t_R$ = 2.001 min.

***Step 9: Preparation of 1-cyclopentyl-6-fluoro-2-(hydroxymethyl)-7-(2-((tetrahydro-2H-pyran-2-yl)oxy)ethyl)-1,8-naphthyridin-4(1H)-one:***

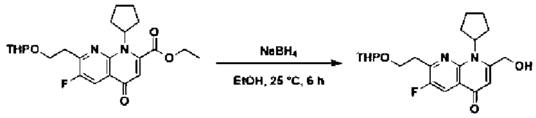


The intermediate prepared in step 8 (1.3 g, 3.01 mmol, 1.0 eq.) was dissolved in ethanol (20 mL), and sodium borohydride (570 mg, 15.3 mmol, 5.0 eq.) was added. After the addition, the mixture was stirred at room temperature for 6 hours, and LCMS monitoring indicated the reaction was complete. The reaction was quenched by adding saturated aqueous sodium chloride solution (2 mL), and the mixture was concentrated under reduced pressure to afford a crude product. Purification by silica gel column chromatography (eluent: DCM:MeOH = 1:0 to 20:1, v/v) gave the title compound as a yellow oil (500 mg, yield: 42.6%). LCMS (ESI): $[M+H]^+$ = 391.1, $t_R$ = 1.708 min.

***Step 10: Preparation of 3-bromo-1-cyclopentyl-6-fluoro-2-(hydroxymethyl)-7-(2-((tetrahydro-2H-pyran-2-yloxy) ethyl)-1,8-naphthyridin-4(1H)-one:***

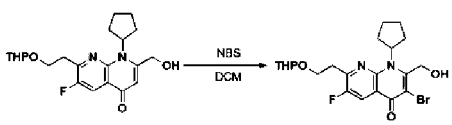

The intermediate prepared in step 9 (350 mg, 0.9 mmol, 1.0 eq.) was dissolved in DCM (10 mL), and N-bromosuccinimide (NBS, 240 mg, 1.34 mmol, 1.5 eq.) was added. After the addition, the mixture was stirred at room temperature for 30 minutes, and LCMS monitoring indicated the reaction was complete. The reaction mixture was poured into ice-cold saturated sodium bicarbonate solution (100 mL) and extracted with DCM (50 mL × 3). The combined organic phases were washed with saturated sodium chloride solution (200 mL), dried over anhydrous sodium sulfate, filtered and concentrated to afford a crude product. Purification by silica gel column chromatography (eluent: PE:EtOAc = 1:0 to 2:1, v/v) gave the title compound as a yellow oil (300 mg, yield: 71.37%). LCMS (ESI): $[M+H]^+$ = 469.0, $t_R$ = 1.815 min.

***Step 11: Preparation of 3-bromo-2-(chloromethyl)-1-cyclopentyl-6-fluoro-7-(2-((tetrahydro-2H-pyran-2-yloxy)ethyl)-1,8-naphthyridin-4(1H)-one:***

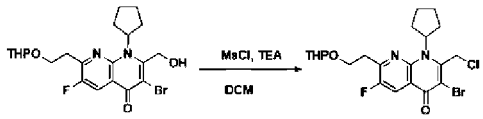


The intermediate prepared in step 10 (300 mg, 0.61 mmol, 1.0 eq.) was dissolved in anhydrous DCM (20 mL). Under an ice bath, triethylamine (323 mg, 3.2 mmol, 5.0 eq.) and methanesulfonyl chloride (220 mg, 1.92 mmol, 3.0 eq.) were added slowly. After the addition, the mixture was stirred under ice bath for 1 hour, and LCMS monitoring indicated the reaction was complete. The reaction mixture was poured into ice water (30 mL) and extracted with DCM (10 mL × 2). The combined organic phases were dried over anhydrous sodium sulfate, filtered and concentrated to afford the title compound as a yellow oil (250 mg, crude), which was used directly in the next step without further purification. LCMS (ESI): $[M+H]^+$ = 488.9, $t_R$ = 2.093 min.

***Step 12: Preparation of (S)-7-((3-bromo-1-cyclopentyl-6-fluoro-7-(2-hydroxyethyl)-4-oxo-1,4-dihydro-1,8-naphthyridin-2-yl)methyl)-4-ethyl-4-hydroxy-1,7-dihydro-3H-pyrano[3,4-c]pyridine-3,8(4H)-dione:***

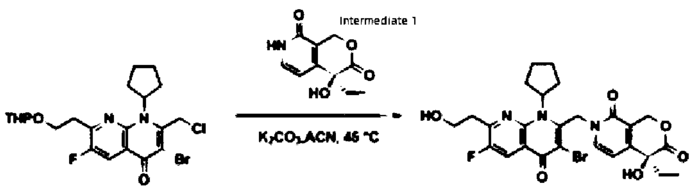


The intermediate prepared in step 11 (300 mg, 0.62 mmol, 1.0 eq.) was dissolved in anhydrous acetonitrile (10 mL). Potassium carbonate (255 mg, 1.85 mmol, 3.0 eq.) and Intermediate 1 (155 mg, 0.74 mmol, 1.2 eq.) were added sequentially. After the addition, the mixture was stirred at 45 °C for 16 hours, and LCMS monitoring indicated the reaction was complete. The reaction mixture was filtered, and the filtrate was concentrated to afford a crude product. Purification by silica gel column chromatography (eluent: DCM:MeOH = 1:0 to 20:1, v/v) gave the title compound as a yellow solid (150 mg, yield: 42.31%). LCMS (ESI): $[M+H]^+$ = 576.0, $t_R$ = 1.493 min.

***Step 13: Preparation of (S)-14-cyclopentyl-7-ethyl-3-fluoro-7-hydroxy-2-(2-hydroxyethyl)-10,14-dihydro-11H-pyranof3',4':6,7]indolizino[2,1-b][1,8]naphthyridine-5,8,11(7H,13H)-trione:***

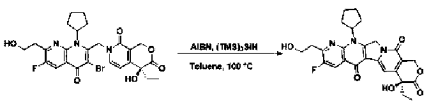


The intermediate prepared in Step 12 (120 mg, 0.21 mmol, 1.0 eq.) was dissolved in toluene (10 mL). Under nitrogen protection, the mixture was heated to 100 °C, followed by the dropwise addition of a mixed solution of 2,2'-azobis(isobutyronitrile) (AIBN, 68 mg, 0.42 mmol, 2.0 eq.) and tris(trimethylsilyl)silane (103 mg, 0.42 mmol, 2.0 eq.) in toluene (5 mL). After the addition, the mixture was stirred at 100 °C for another 6 hours, and LCMS monitoring indicated the

reaction was complete. The reaction mixture was cooled to room temperature and filtered, and the filter cake was purified by reversed-phase preparative HPLC to afford the title compound as a pale yellow solid (20 mg, yield: 19.22%). LCMS (ESI): $[M+H]^+$ = 496.3, t_R = 1.486 min; $^1$H NMR (400 MHz, DMSO-$d_6$) δ: 8.30 (d, J = 8.9 Hz, 1H), 7.27 (s, 1H), 6.36 (s, 1H), 5.54 (s, 2H), 5.42-5.29 (m, 2H), 4.90-4.78 (m, 2H), 3.91-3.79 (m, 2H), 3.15-3.08 (m, 2H), 2.24-2.02 (m, 5H), 1.88-1.65 (m, 5H), 0.86 (t, J = 7.3 Hz, 3H).

**Example 197: Preparation of (S)-4-ethyl-8-fluoro-4-hydroxy-11-((3S,4S)-4-hydroxypyrrolidin-3-yl)-1,12-dihydro-14H-pyrano[3',4':6,7]indolizino[2,1-b]quinoline-3,6,14(4H,11H)-trione:**

***Step 1: Preparation of tert-butyl (3S,4S)-3-(((E)-4-(2,5-difluorophenyl)-4-oxobut-2-en-2-yl)amino)-4-hydroxypyrrolidine-1-carboxylate:***

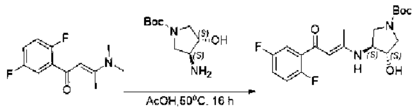


Intermediate (Z)-1-(2,5-difluorophenyl)-3-(dimethylamino)but-2-en-1-one (17 g, 75.5 mmol, 1.0 eq.) from Preparation Example 27 was dissolved in acetic acid (110 mL), and tert-butyl (3S,4S)-3-amino-4-hydroxypyrrolidine-1-carboxylate ((3S,4S)-N-Boc-3-amino-4-hydroxypyrrolidine, 16.8 g, 83.0 mmol, 1.1 eq.) was added. Under nitrogen protection, the reaction mixture was heated to 50 °C and stirred for 16 hours, and LCMS monitoring indicated the reaction was complete. After cooling to room temperature, the reaction mixture was poured into ice-cold aqueous sodium bicarbonate solution (300 mL) and stirred for 10 minutes, then extracted with DCM (100 mL × 3). The combined organic phases were dried over anhydrous sodium sulfate, filtered and concentrated under reduced pressure to afford a crude product. Purification by silica gel column chromatography (eluent: DCM:MeOH = 1:0 to 20:1, v/v) gave the title compound as a gray solid (17 g, yield: 58.9%). LCMS (ESI): $[M+H]^+$ = 383.2.

***Step 2: Preparation of tert-butyl (3S,4S)-3-(6-fluoro-2-methyl-4-oxoquinolin-1(4H)-yl)-4-hydroxypyrrolidine-1-carboxylate:***

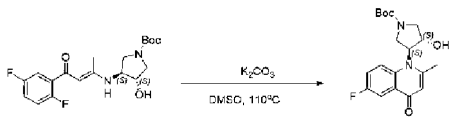


The intermediate prepared in step 1 (6 g, 15.69 mmol, 1.0 eq.) was dissolved in dimethyl sulfoxide (120 mL), and potassium phosphate (9.9 g, 47.07 mmol, 3.0 eq.) was added. Under nitrogen protection, the reaction mixture was heated to 110 °C and stirred for 8 hours, and LCMS monitoring indicated the reaction was complete. The reaction mixture was poured into ice water (300 mL) and extracted with ethyl acetate (100 mL × 3). The combined organic phases were washed with saturated sodium chloride solution (300 mL), dried over anhydrous sodium sulfate, filtered and concentrated to afford a crude product. Purification by silica gel column chromatography (eluent: DCM:MeOH = 1:0 to 10:1, v/v) gave the title compound as a yellow solid (3 g, yield: 61.6%). LCMS (ESI): $[M+H]^+$ = 363.3.

***Step 3: Preparation of tert-butyl (3S,4S)-3-(3-bromo-2-(bromomethyl)-6-fluoro-4-oxoquinolin-1(4H)-yl)-4-hydroxypyrrolidine-1-carboxylate:***

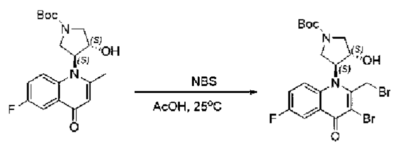


The intermediate prepared in step 2 (8 g, 22.08 mmol, 1.0 eq.) was dissolved in acetic acid (70 mL), and N-bromosuccinimide (NBS, 8.65 g, 48.58 mmol, 2.2 eq.) was added to the reaction mixture. Under nitrogen protection, the mixture was stirred at 25 °C for 16 hours. The reaction mixture was slowly poured into ice-cold saturated aqueous sodium bicarbonate solution (200 mL) and extracted with ethyl acetate (200 mL × 3). The combined organic phases were dried, concentrated, and mixed with 100~200 mesh silica gel for purification. Silica gel column chromatography (eluent: DCM:MeOH = 20:1, v/v) afforded the title compound as a yellow solid (10 g, yield: 87.06%). LCMS (ESI): $[M+H]^+$ = 521.1.

***Step 4: Preparation of tert-butyl (3S,4S)-3-(3-bromo-2-(bromomethyl)-6-fluoro-4-oxoquinolin-1(4H)-yl)-4-((tert-butyldimethylsilyl)oxy)pyrrolidine-1-carboxylate:***

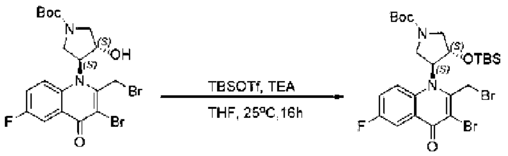


The intermediate prepared in step 3 (800 mg, 1.54 mmol, 1.0 eq.) was dissolved in tetrahydrofuran (20 mL). Under an ice bath, triethylamine (468 mg, 4.62 mmol, 3.0 eq.) and tert-butyldimethylsilyl trifluoromethanesulfonate (1.22 g, 4.62 mmol, 3.0 eq.) were added sequentially. After the addition, the mixture was allowed to warm to room temperature naturally and stirred for 16 hours, and TLC monitoring indicated the reaction was complete. The reaction mixture was poured into ice water (40 mL) and extracted with EA(50 mL × 3). The combined organic phases were dried over anhydrous sodium sulfate, filtered and concentrated , and purified by silica gel column chromatography (eluent: DCM:MeOH = 1:0 to 20:1, v/v) gave the title compound as a white solid (400 mg, yield: 40.9%). LCMS (ESI): $[M+H]^+$ = 635.2.

***Step 5: Preparation of tert-butyl (3S,4S)-3-(3-bromo-2-(((S)-4-ethyl-4-hydroxy-3,8-dioxo-4,8-dihydro-1H-pyrano [3,4-c]pyridin-7(3H)-yl)methyl)-6-fluoro-4-oxoquinolin-1(4H)-yl)-4-((tert-butyldimethylsilyl)oxy)pyrrolidine-1-carboxylate:***

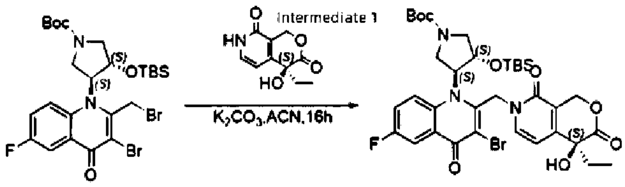


The intermediate prepared in step 4 (1 g, 1.58 mmol, 1.0 eq.) was dissolved in acetonitrile (10 mL), and Intermediate 1 (264 mg, 1.26 mmol, 0.8 eq.) and potassium carbonate (654 mg, 4.73 mmol, 3.0 eq.) were added sequentially. Under nitrogen protection, the reaction mixture was heated to 35 °C and stirred for 16 hours, and LCMS monitoring indicated the reaction was complete. The mixture was filtered, and the filtrate was concentrated under reduced pressure to afford a crude product. Purification by silica gel column chromatography (eluent: DCM:MeOH = 10:1, v/v) gave the title compound as a pale yellow solid (800 mg, yield: 66.4%). LCMS (ESI): $[M+H]^+$ = 764.3.

***Step 6: Preparation of tert-butyl (3S,4S)-3-((tert-butyldimethylsilyl)oxy)-4-((S)-4-ethyl-8-fluoro-4-hydroxy-3,6,14-trioxo-4,6,12,14-tetrahydro-1H-pyrano[3',4':6,7]indolizino[2,1-b]quinolin-11(3H)-yl)pyrrolidine-1-carboxylate:***

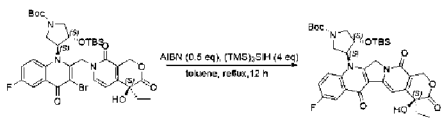


The intermediate prepared in step 5 (300 mg, 0.39 mmol, 1.0 eq.) was dissolved in toluene (50 mL), and 2,2'-azobis(isobutyronitrile) (64.0 mg, 0.39 mmol, 1.0 eq.) and tris(trimethylsilyl)silane (388 mg, 1.56 mmol, 4.0 eq.) were

added sequentially. The reaction system was purged with nitrogen three times, then heated to 100 °C under nitrogen protection and stirred for 12 hours, and LCMS monitoring indicated the reaction was complete. The reaction mixture was concentrated, and purified by silica gel column chromatography (eluent: DCM:MeOH = 1:0 to 20:1, v/v) gave the title compound as a yellow solid (110 mg, yield: 41.4%). LCMS (ESI): $[M+H]^+$ = 682.3.

***Step 7: Preparation of (S)-4-ethyl-8-fluoro-4-hydroxy-11-((3S,4S)-4-hydroxypyrrolidin-3-yl)-1,12-dihydro-14H-pyrano[3',4':6,7]indolizinof2,1-b]quinoline-3,6,14(4H,11H)-trione:***

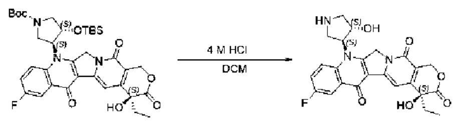


The intermediate prepared in step 6 (110 mg, 0.16 mmol, 1.0 eq.) was dissolved in DCM (5 mL). Under an ice bath, 4 M hydrogen chloride in 1,4-dioxane (5 mL) was added slowly. After the addition, the ice bath was removed, and the reaction mixture was stirred at room temperature for an additional 3 hours, and LCMS monitoring indicated the reaction was complete. The reaction mixture was concentrated, and triturated with acetonitrile (10 mL) and filtered. The filter cake was dissolved in N,N-dimethylformamide (DMF, 3 mL) and allowed to stand for 1 hour, resulting in the precipitation of a solid. The solid was filtered and lyophilized to give the title compound as a white solid (22 mg, yield: 29.0%). LCMS (ESI): $[M+H]^+$ = 468.0; $^1H$ NMR (400 MHz, DMSO-$d_6$) δ: 9.90 (s, 1H), 9.53 (s, 1H), 8.10 (dd, J = 8.8, 3.3 Hz, 2H), 7.75-7.66 (m, 1H), 7.31 (s, 1H), 6.42 (s, 1H), 6.21 (d, J = 4.1 Hz, 1H), 5.43-5.08 (m, 5H), 3.96-3.68 (m, 4H), 2.04-1.94 (m, 1H), 1.89-1.76 (m, 2H), 0.87 (t, J = 7.3 Hz, 3H).

**General Synthetic Procedure for Examples 198-206:**

Using the compound prepared in Step 7 of Example 197 as the starting material, the target compounds of Examples 198-206 were synthesized via one-pot reductive amination reactions with various aldehyde or ketone raw materials, or alkylation reactions with various halogenated compounds, following the identical synthetic procedure described in Example 104.

The structures and detailed characterization data (LCMS and $^1H$ NMR) of the above compounds are as follows:

| Examples | Structure | $^1H$ NMR | LCMS (ESI) $[M+H]^+$ |
|---|---|---|---|
| 198 | 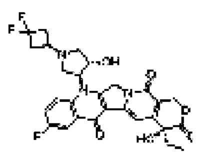 | (400 MHz, DMSO-$d_6$) δ 8.62 (s, 1H), 8.07 (dd, J = 8.8, 3.1 Hz, 1H), 7.68 (t, J = 6.6 Hz, 1H), 7.31 (s, 1H), 6.67 (s, 1H), 5.62 (d, J = 20.0 Hz, 1H), 5.43 - 5.15 (m, 4H), 5.08 - 4.90 (m, 1H), 3.80 - 3.59 (m, 2H), 3.08 - 2.71 (m, 6H), 2.05 - 1.95 (m, 1H), 1.90 - 1.78 (m, 2H), 0.88 (t, J = 7.3 Hz, 3H)$_o$ | 558.3 |
| 199 | 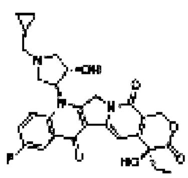 | (400 MHz, DMSO-$d_6$) δ 11.67 (s, 1H), 8.48 (s, 1H), 8.10 (d, J = 8.7 Hz, 1H), 7.71 (s, 1H), 7.32 (s, 1H), 6.54 - 6.24 (m, 2H), 5.58 (d, J = 19.4 Hz, 1H), 5.45 - 5.03 (m, 5H), 4.30 - 4.02 (m, 2H), 4.02 - 3.81 (m, 2H), 3.28 - 3.19 (m, 2H), 1.95 - 1.76 (m, 2H), 1.22 - 1.11 (m, 1H), 0.96 - 0.76 (m, 3H), 0.73 - 0.59 (m, 2H), 0.52 - 0.41 (m, 2H)$_o$ | 522.0 |
| 200 | 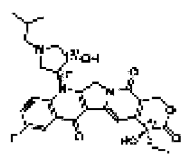 | (400 MHz, DMSO-$d_6$) δ 11.12 (s, 1H), 10.04 (s, 1H), 8.56 (s, 1H), 8.17 - 7.92 (m, 2H), 7.77 - 7.64 (m, 1H), 7.30 (s, 1H), 6.31 (s, 1H), 5.63 - 5.49 (m, 1H), 5.42 - 5.14 (m, 4H), 4.31 - 3.71 (m, 4H), 3.29 - 3.11 (m, 3H), 2.15 - 1.97 (m, 1H), 1.89 - 1.76 (m, 2H), 1.03 (s, 6H), 0.86 (t, J = 7.3 Hz, 3H)$_o$ | 524.3 |

(continued)

| Examples | Structure | $^1$H NMR | LCMS (ESI) $[M+H]^+$ |
|---|---|---|---|
| 201 | 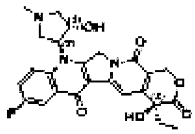 | (400 MHz, DMSO-$d_6$) δ 11.18 (s, 1H), 8.22 - 8.04 (m, 2H), 7.74 (d, J = 5.6 Hz, 1H), 7.31 (s, 1H), 6.41 (s, 1H), 6.30 (s, 1H), 5.41 - 5.12 (m, 5H), 3.14 - 2.89 (m, 4H), 2.53 (s, 3H), 1.88 - 1.80 (m, 2H), 1.29 - 1.22 (m, 1H), 0.87 (t, J = 7.3 Hz, 3H)。 | 482.0 |
| 202 | 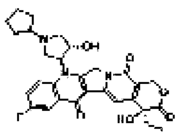 | (400 MHz, DMSO-$d_6$) δ 8.46 (s, 1H), 8.08 (dd, J = 8.7, 3.2 Hz, 1H), 7.99 - 7.81 (m, 1H), 7.67 (s, 1H), 7.31 (s, 1H), 5.44 - 5.28 (m, 3H), 5.14 (s, 1H), 4.18 (s, 2H), 3.98 - 3.66 (m, 3H), 3.27 (s, 1H), 2.17 - 1.93 (m, 3H), 1.94 - 1.69 (m, 6H), 1.67 - 1.54 (m, 2H), 0.91 - 0.81 (m, 3H) | 536.4 |
| 203 | 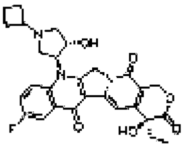 | (400 MHz, DMSO-$d_6$) δ 12.50 (s, 1H), 8.52 (s, 1H), 8.08 (dd, J = 8.6, 2.9 Hz, 1H), 7.65 (s, 1H), 7.31 (s, 1H), 6.33 (s, 2H), 5.59 (d, J = 19.7 Hz, 1H), 5.45 - 5.29 (m, 3H), 5.27 - 5.13 (m, 2H), 4.21 - 3.87 (m, 2H), 3.89 - 3.60 (m, 2H), 3.20 - 3.02 (m, 1H), 2.46 - 2.14 (m, 4H), 1.85 (dq, J = 13.2, 7.0 Hz, 4H), 0.93 - 0.80 (m, 3H)。 | 522.3 |
| 204 | 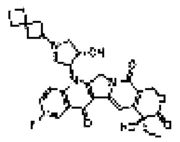 | (400 MHz, DMSO-$d_6$) δ 12.59 (s, 1H), 8.53 (d, 1H), 8.08 (dd, J = 8.7, 3.2 Hz, 1H), 7.69 - 7.55 (m, 1H), 7.31 (s, 1H), 6.54 - 6.08 (m, 2H), 5.68 - 5.48 (m, 1H), 5.43 - 5.27 (m, 3H), 5.27 - 5.09 (m, 2H), 4.15 - 3.99 (m, 1H), 3.93 - 3.62 (m, 3H), 3.20 - 2.97 (m, 1H), 2.47 - 2.23 (m, 4H), 2.11 - 2.03 (m, 2H), 2.03 - 1.95 (m, 2H), 1.90 - 1.76 (m, 4H), 0.92 - 0.79 (m, 3H)。 | 562.4 |
| 205 | 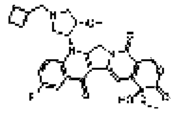 | $^1$H NMR (400 MHz, DMSO-$d_6$) δ 11.83 (s, 1H), 8.55 (s, 1H), 8.09 (dd, J = 8.7, 3.1 Hz, 1H), 7.78 - 7.57 (m, 1H), 7.32 (s, 1H), 6.30 (s, 1H), 5.63 - 5.51 (m, 1H), 5.44 - 5.28 (m, 3H), 5.27 - 5.13 (m, 2H), 4.21 - 3.98 (m, 2H), 3.88 - 3.57 (m, 2H), 3.26 - 3.14 (m, 2H), 2.89 - 2.69 (m, 1H), 2.20 - 2.03 (m, 2H), 1.99 - 1.71 (m, 6H), 0.95 - 0.83 (m, 3H)。 | 536.3 |
| 206 | 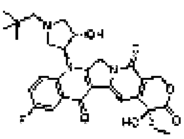 | (400 MHz, DMSO-$d_6$) δ 9.07 (d, J = 5.3 Hz, 1H), 8.04 (d, J = 6.0 Hz, 1H), 7.72 - 7.64 (m, 1H), 7.30 (s, 1H), 6.37 (s, 1H), 5.66 (d, J = 20.1 Hz, 2H), 5.39 - 5.27 (m, 3H), 5.19 (d, J = 20.3 Hz, 1H), 4.98 - 4.89 (m, 1H), 4.83 - 4.72 (m, 1H), 3.16 - 3.08 (m, 2H), 2.97 - 2.88 (m, 1H), 2.05 - 1.93 (m, 1H), 1.88 - 1.76 (m, 2H), 0.96 (s, 9H), 0.90 - 0.82 (m, 3H)。 | 538.4 |

**Example 207: Preparation of (S)-9-(aminomethyl)-4-ethyl-8-fluoro-4-hydroxy-11-methyl-1,12-dihydro-14H-pyrano[3',4':6,7]indolizino[2,1-b]quinoline-3,6,14 (4H,11H)-trione:**

***Step 1: Preparation of tert-butyl((6-fluoro-1,2-dimethyl-4-oxo-1,4-dihydroquinolin-7-yl)methyl)carbamate:***

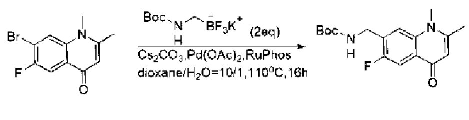


7-Bromo-6-fluoro-1,2-dimethylquinolin-4(1H)-one (7.80 g, 29.0 mmol, 1.0 eq.) was dissolved in a mixed solvent of 1,4-dioxane (100 mL) and water (10 mL). Potassium (tert-butoxycarbonylamino)methyltrifluoroborate (13.7 g, 58.0 mmol, 2.0 eq.), cesium carbonate (28.4 g, 87.0 mmol, 3.0 eq.), 2-dicyclohexylphosphino-2',6'-diisopropoxy-1,1'-biphenyl (1.35 g, 2.90 mmol, 0.1 eq.) and palladium acetate (657 mg, 2.90 mmol, 0.1 eq.) were added sequentially. Under nitrogen protection, the reaction mixture was heated to 110 °C and stirred for 16 hours. After cooling to room temperature, the mixture was filtered, and the filtrate was concentrated under reduced pressure to afford a crude product. Purification by silica gel column chromatography (eluent: DCM:MeOH = 1:0 to 20:1, v/v) gave the title compound as a brownish black solid

(2.5 g, yield: 26.9%). LCMS (ESI): $[M+H]^+$ = 321.3; $^1H$ NMR (400 MHz, DMSO-$d_6$) δ: 7.69 (d, J = 10.1 Hz, 1H), 7.64 (d, J = 6.0 Hz, 1H), 7.53-7.45 (m, 1H), 6.01 (s, 1H), 4.28 (d, J = 5.9 Hz, 2H), 3.66 (s, 3H), 2.43 (s, 3H), 1.37 (s, 9H).

***Step 2: Preparation of tert-butyl((3-bromo-2-(bromomethyl)-6-fluoro-1-methyl-4-oxo-1,4-dihydroquinolin-7-yl)methyl)carbamate:***

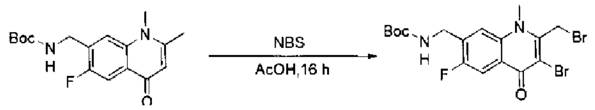


tert-butyl ((6-fluoro-1,2-dimethyl-4-oxo-1,4-dihydroquinolin-7-yl)methyl)carbamate (2.50 g, 7.80 mmol, 1.0 eq.) was dissolved in acetic acid (35 mL), and N-bromosuccinimide (NBS, 3.05 g, 17.16 mmol, 2.2 eq.) was added. The reaction was stirred at room temperature overnight, and LCMS monitoring indicated the reaction was complete. The reaction mixture was poured into ice-cold saturated sodium bicarbonate solution (50 mL) and extracted with ethyl acetate (30 mL × 3). The combined organic phases were dried over anhydrous sodium sulfate, filtered and concentrated under reduced pressure to afford a crude product. Purification by silica gel column chromatography (eluent: DCM:MeOH = 1:0 to 100:1, v/v) gave the title compound as a brown solid (2.50 g, yield: 67.03%). LCMS (ESI): $[M+H]^+$ = 478.8; $^1H$ NMR (400 MHz, DMSO-$d_6$) δ: 7.80-7.79 (m, 1H), 7.77 (s, 1H), 7.52-7.47 (m, 1H), 5.05 (s, 2H), 4.31 (d, J = 5.9 Hz, 2H), 3.91 (s, 3H), 1.36 (s, 9H).

***Step 3: Preparation of (S)-tert-butyl((3-bromo-2-((4-ethyl-4-hydroxy-3,8-dioxo-4,8-dihydro-1H-pyrano[3,4-c]pyridin-7(3H)-yl)methyl)-6-fluoro-1-methyl-4-oxo-1,4-dihydroquinolin-7-yl)methyl)carbamate:***

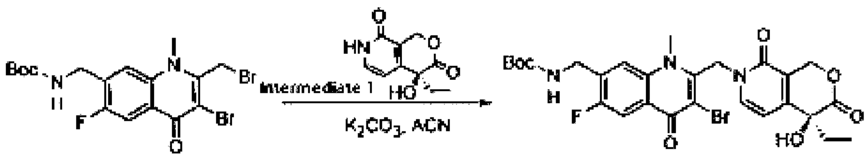


tert-butyl ((3-bromo-2-(bromomethyl)-6-fluoro-1-methyl-4-oxo-1,4-dihydroquinolin-7-yl)methyl)carbamate (1.5 g, 3.14 mmol, 1.0 eq.) was dissolved in acetonitrile (ACN, 20 mL). Intermediate 1 (656.89 mg, 3.14 mmol, 1.0 eq.) and anhydrous potassium carbonate (1.30 g, 9.42 mmol, 3.0 eq.) were added, and the reaction was stirred at room temperature for 2 hours. LCMS monitoring indicated the reaction was complete. The mixture was filtered, and the filtrate was poured into ice-cold saturated aqueous ammonium chloride solution (30 mL) and extracted with ethyl acetate (30 mL × 3). The combined organic phases were washed with saturated aqueous sodium chloride solution (30 mL × 3), dried over anhydrous sodium sulfate, filtered and concentrated under reduced pressure to afford a crude product. Purification by silica gel column chromatography (eluent: DCM:MeOH = 1:0 to 50:1, v/v) gave the title compound as a yellow solid (930 mg, yield: 48.36%). LCMS (ESI): $[M+H]^+$ = 608.2.

***Step 4: Preparation of (S)-tert-butyl((4-ethyl-8-fluoro-4-hydroxy-11-methyl-3,6,14-trioxo-3,4,6,11,12,14-hexahydro-1H-pyrano[3',4':6,7]indolizino[2,1-b]quinolin-9-yl)methyl)carbamate:***

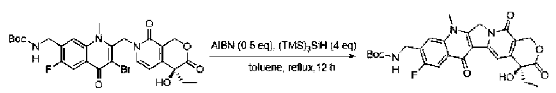


The intermediate prepared in step 3 (920 mg, 1.52 mmol, 1.0 eq.) and AIBN (249.11 mg, 1.52 mmol, 1.0 eq.) were added sequentially to a dry three-necked flask. The flask was evacuated under an oil pump and protected with argon, then anhydrous toluene (100 mL) was added. Tris(trimethylsilyl)silane (1.13 g, 4.56 mmol, 4.0 eq.) was added to the reaction mixture, and after the addition, the mixture was heated to 110 °C and stirred overnight. The reaction mixture was concentrated, and purified by silica gel column chromatography (eluent: DCM:MeOH=1:0 to50:1, v/v) to give the title

compound as a light brown solid (260 mg, yield: 32.61%). LCMS (ESI): $[M+H]^+$=526.0.

***Step 5: Preparation of (S)-9-(aminomethyl)-4-ethyl-8-fluoro-4-hydroxy-11-methyl-1,12-dihydro-14H-pyrano [3',4':6,7]indolizino[2,1-b]quinoline-3,6,14(4H,11H)-trione:***

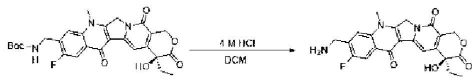


The intermediate prepared in step 4 (260 mg, 0.49 mmol, 1.0 eq.) was dissolved in DCM (DCM, 10 mL). Under an ice bath, 4 M hydrogen chloride in 1,4-dioxane (10 mL) was added slowly, and the reaction was stirred at room temperature for 1 hour. LCMS monitoring indicated the reaction was complete. The reaction mixture was concentrated to dryness under reduced pressure to afford the title compound as a pale yellow solid (210 mg, crude). LCMS (ESI): $[M+H]^+$ = 426.3.

General Synthetic Procedure for Examples 208-220:

Following the reductive amination procedures analogous to those described in Examples 104-181, the target compounds of Examples 208-220 were synthesized using the compound prepared in Example 207 as the starting material, via reductive amination reactions with various aldehyde or ketone raw materials, or alkylation reactions with various halogenated compounds.

The structures and detailed characterization data (LCMS and $^1H$ NMR) of the above compounds are as follows:

| Examples | structrure | $^1H$ NMR | LCMS (ESI) $[M+H]^+$ |
|---|---|---|---|
| 208 | 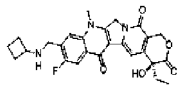 | $^1H$ NMR (400 MHz, DMSO-$d_6$) δ 9.71 (s, 2H), 8.45 (d, *J* = 5.8 Hz, 1H), 8.07 (d, *J* = 9.6 Hz, 1H), 7.29 (s, 1H), 6.37 (s, 1H), 5.50 - 5.31 (m, 4H), 4.30 (s, 2H), 3.99 (s, 3H), 3.86 - 3.77 (m, 1H), 2.30 - 2.18 (m, 4H), 1.89 - 1.78 (m, 4H), 0.88 (t, *J* = 7.3 Hz, 3H) | 480.0 |
| 209 | 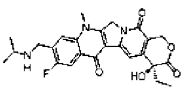 | (400 MHz, DMSO-$d_6$) δ 9.54 (s, 2H), 8.61 (d, *J* = 5.9 Hz, 1H), 8.06 (d, *J* = 9.7 Hz, 1H), 7.29 (s, 1H), 6.36 (s, 1H), 5.52 - 5.26 (m, 4H), 4.41 (t, *J* = 5.9 Hz, 2H), 4.01 (s, 3H), 3.51 - 3.42 (m, 1H), 1.90 - 1.78 (m, 2H), 1.39 (d, *J* = 6.5 Hz, 6H), 0.88 (t, *J* = 7.3 Hz, 3H). | 468.3 |
| 210 | 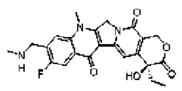 | (400 MHz, DMSO-$d_6$) δ 9.43 (s, 2H), 8.42 (d, *J* = 5.8 Hz, 1H), 8.06 (d, *J* = 9.7 Hz, 1H), 7.28 (s, 1H), 6.37 (s, 1H), 5.50 - 5.25 (m, 4H), 4.41 (s, 2H), 3.97 (s, 3H), 2.67 (s, 3H), 1.91 - 1.71 (m, 2H), 0.87 (t, *J* = 7.3 Hz, 3H). | 440.3 |
| 211 | 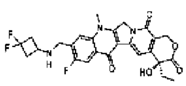 | (400 MHz, DMSO-$d_6$) δ 10.23 (s, 2H), 8.47 (d, *J* = 5.9 Hz, 1H), 8.08 (d, *J* = 9.7 Hz, 1H), 7.30 (s, 1H), 6.37 (s, 1H), 5.50 - 5.29 (m, 4H), 4.40 (s, 2H), 4.00 (s, 3H), 3.94 - 3.80 (m, 1H), 3.15 - 2.95 (m, 4H), 1.90 - 1.78 (m, 2H), 0.88 (t, *J* = 7.3 Hz, 1H). | 516.3 |
| 212 | 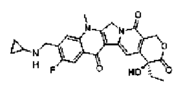 | (400 MHz, DMSO-$d_6$) δ 9.84 (s, 1H), 8.52 (d, *J* = 5.9 Hz, 1H), 8.06 (d, *J* = 9.6 Hz, 1H), 7.29 (s, 1H), 6.37 (s, 1H), 5.50 - 5.30 (m, 4H), 4.50 (s, 2H), 3.99 (s, 3H), 2.87 - 2.77 (m, 1H), 1.89 - 1.76 (m, 2H), 0.98 - 0.91 (m, 2H), 0.88 (t, *J* = 7.3 Hz, 3H), 0.83 - 0.72 (m, 2H). | 466.0 |
| 213 | 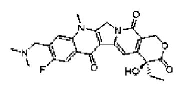 | (400 MHz, DMSO-$d_6$) δ 11.31 (s, 1H), 8.61 (d, *J* = 5.8 Hz, 1H), 8.07 (d, *J* = 9.6 Hz, 1H), 7.28 (s, 1H), 5.48 - 5.31 (m, 4H), 4.57 (d, *J* = 5.1 Hz, 2H), 4.00 (s, 3H), 2.81 (d, *J* = 2.7 Hz, 6H), 1.87 - 1.78 (m, 2H), 0.87 (t, *J* = 7.3 Hz, 3H). | 454.3 |

(continued)

| Examples | structrure | $^1$H NMR | LCMS (ESI) $[M+H]^+$ |
|---|---|---|---|
| 214 | 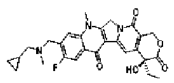 | (400 MHz, DMSO-$d_6$) δ 11.24 (s, 1H), 8.67 (d, *J* = 5.8 Hz, 1H), 8.06 (d, *J* = 9.6 Hz, 1H), 7.27 (s, 1H), 6.35 (s, 1H), 5.53 - 5.27 (m, 4H), 4.70 (dd, *J* = 13.4, 3.8 Hz, 1H), 4.50 (dd, *J* = 13.5, 6.4 Hz, 1H), 4.01 (s, 3H), 3.22 - 3.13 (m, 1H), 3.08 - 2.99 (m, 1H), 2.79 (d, *J* = 2.5 Hz, 3H), 1.87 - 1.77 (m, 2H), 1.28 - 1.20 (m, 1H), 0.87 (t, *J* = 7.3 Hz, 3H), 0.72 - 0.61 (m, 2H), 0.49 - 0.36 (m, 2H). | 494.3 |
| 215 | 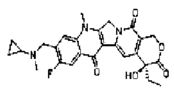 | (400 MHz, DMSO-$d_6$) δ 11.28 (s, 1H), 8.58 (s, 1H), 8.06 (d, *J* = 9.6 Hz, 1H), 7.29 (s, 1H), 6.32 (s, 1H), 5.53 - 5.30 (m, 4H), 4.70 (s, 2H), 4.01 (s, 3H), 3.07 - 2.94 (m, 1H), 2.89 (s, 3H), 1.91 - 1.75 (m, 2H), 1.15 - 1.03 (m, 1H), 0.88 (t, *J* = 7.3 Hz, 3H), 0.84 - 0.65 (m, 3H). | 480.0 |
| 216 | 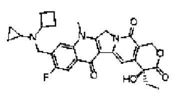 | (400 MHz, DMSO-$d_6$) δ 11.34 (s, 1H), 8.57 (s, 1H), 8.06 (d, *J* = 9.5 Hz, 1H), 7.29 (s, 1H), 5.77 (s, 1H), 5.67 - 5.25 (m, 4H), 4.64 - 4.47 (m, 2H), 4.14 - 4.07 (m, 1H), 4.02 (s, 3H), 3.52 - 3.45 (m, 1H), 2.77 - 2.58 (m, 2H), 2.25 - 2.09 (m, 2H), 1.89 - 1.79 (m, 2H), 1.76 - 1.68 (m, 2H), 1.26 - 1.15 (m, 1H), 0.88 (t, *J* = 7.3 Hz, 3H), 0.82 - 0.72 (m, 1H), 0.59 - 0.42 (m, 2H). | 520.3 |
| 217 | 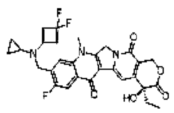 | (400 MHz, DMSO-$d_6$) δ 8.14 - 7.92 (m, 2H), 7.28 (s, 1H), 5.47 - 5.39 (m, 2H), 5.38 - 5.31 (m, 2H), 4.07 - 3.89 (m, 4H), 3.81 - 3.67 (m, 3H), 3.02 - 2.63 (m, 4H), 1.90 - 1.76 (m, 2H), 0.87 (t, *J* = 7.3 Hz, 3H), 0.66 - 0.26 (m, 4H). | 556.3 |
| 218 | 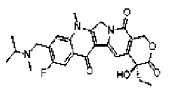 | (400 MHz, DMSO-$d_6$) δ 10.96 (s, 1H), 8.73 - 8.65 (m, 1H), 8.07 (d, *J* = 9.7 Hz, 1H), 7.28 (s, 1H), 5.47 - 5.30 (m, 4H), 4.65 (dd, *J* = 13.6, 4.3 Hz, 1H), 4.01 (s, 3H), 4.09 - 3.98 (m, 3H), 3.63 - 3.54 (m, 2H), δ 2.66 (d, *J* = 4.0 Hz, 3H), 1.87 - 1.77 (m, 2H), 1.38 (t, *J* = 6.6, 1.9 Hz, 6H), 0.87 (t, *J* = 7.3 Hz, 3H). | 482.0 |
| 219 | 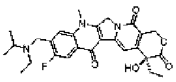 | (400 MHz, DMSO-$d_6$) δ 10.60 (s, 1H), 8.70 - 8.61 (m, 1H), 8.08 (d, *J* = 9.7 Hz, 1H), 7.28 (s, 1H), 6.35 (s, 1H), 5.50 - 5.28 (m, 4H), 4.67 - 4.55 (m, 1H), 4.50 - 4.36 (m, 1H), 4.02 (s, 3H), 3.69 - 3.56 (m, 1H), 3.26 - 3.07 (m, 2H), 1.87 - 1.74 (m, 2H), 1.45 - 1.24 (m, 9H), 0.87 (t, *J* = 7.3 Hz, 3H). | 496.0 |
| 220 | 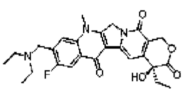 | (400 MHz, DMSO-$d_6$) δ 8.30 (d, *J* = 5.7 Hz, 1H), 8.12 (d, *J* = 9.7 Hz, 1H), 7.31 (s, 1H), 5.50 - 5.24 (m, 4H), 4.56 (s, 2H), 4.05 - 3.94 (m, 3H), 3.27 - 3.17 (m, 4H), 1.89 - 1.78 (m, 2H), 1.30 (t, *J* = 7.2 Hz, 6H), 0.92 - 0.83 (m, 3H). | 482.4 |

**Example 221: Preparation of tert-butyl(S)-((11-cyclopropyl-4-ethyl-8-fluoro-4-hydroxy-3,6,14-trioxo-3,4,6,11,12,14-hexahydro-1H-pyrano[3',4':6,7]indolizino[2,1-b]quinolin-9-yl)methyl)(isopropyl)carbamate:**

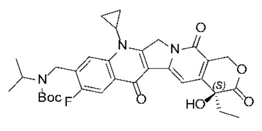

***Step 1: Preparation of 1-cyclopropyl-6-fluoro-2-methyl-7-vinylquinolin-4(1H)-one:***

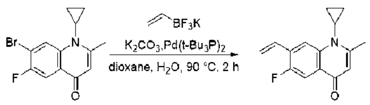


Intermediate 8 from Preparation Example 8 (6.00 g, 19.34 mmol, 1.0 eq.) was dissolved in a mixed solvent of 1,4-dioxane (60 mL) and water (6 mL). Potassium vinyltrifluoroborate (5.18 g, 38.69 mmol, 2.0 eq.), potassium carbonate (8.02 g, 58.03 mmol, 3.0 eq.) and bis(tri-tert-butylphosphine)palladium (989 mg, 1.93 mmol, 0.10 eq.) were added, and the reaction mixture was heated to 90 °C and stirred for 2 hours. LCMS monitoring indicated the reaction was complete. The reaction mixture was diluted with water and ethyl acetate, and the aqueous phase was extracted with ethyl acetate (100 mL×3). The combined organic phases were back-washed with saturated brine three times, dried over anhydrous sodium sulfate and concentrated to afford a crude product. Purification by silica gel column chromatography (eluent: EA:PE = 0 to 1:1, v/v) gave the title compound as a pale yellow solid (3.1 g, yield: 62.90%). LCMS (ESI):$[M+H]^+$=246.1.

***Step 2: Preparation of 1-cyclopropyl-6-fluoro-2-methyl-4-oxo-1,4-dihydroquinoline-7-carbaldehyde:***

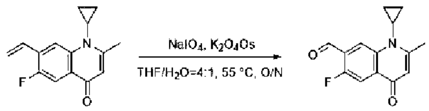


The compound from step 1 (3.10 g, 12.74 mmol, 1.0 eq.) was dissolved in a mixed solvent of tetrahydrofuran (30 mL) and water (6 mL). Potassium osmate dihydrate (571.6 mg, 1.27 mmol, 0.1 eq.) and sodium periodate (10.90 g, 50.97 mmol, 4.0 eq.) were added, and the reaction mixture was heated to 55 °C and stirred for 16 hours. LCMS monitoring indicated the reaction was complete. After cooling to room temperature, the reaction mixture was diluted with water and ethyl acetate, and the aqueous phase was extracted with ethyl acetate (50 mL × 3). The combined organic phases were back-washed with saturated brine three times, dried over anhydrous sodium sulfate and concentrated to afford a crude product. Purification by silica gel column chromatography (eluent: EtOAc:PE = 0 to 1:1, v/v) gave the title compound as a pale yellow solid (2.0 g, yield: 64.10%). LCMS (ESI): $[M+H]^+$ = 246.1.

***Step 3: Preparation of 1-cyclopropyl-6-fluoro-7-((isopropylamino)methyl)-2-methylquinolin-4(1H)-one:***

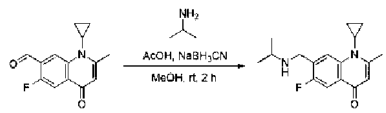


The compound from step 2 (2.00 g, 8.15 mmol, 1.0 eq.) was dissolved in MeOH (40 mL). Isopropylamine (963.49 mg, 16.30 mmol, 2.0 eq.) and glacial acetic acid (1.47 g, 24.45 mmol, 3.0 eq.) were added, and the mixture was stirred at room temperature for 0.5 hours. Sodium cyanoborohydride (1.51 g, 24.45 mmol, 3.0 eq.) was then added, and the reaction was stirred at room temperature for another 2 hours. LCMS monitoring indicated the reaction was complete. The reaction mixture was poured into aqueous sodium bicarbonate solution (100 mL), and the resulting mixture was extracted with ethyl acetate (50 mL × 3). The combined organic phases were back-washed with saturated brine, dried over anhydrous sodium sulfate and concentrated to afford a crude product. Purification by flash column chromatography (eluent: EtOAc:PE = 0 to 1:1, v/v) gave the title compound as a pale yellow solid (1.60 g, yield: 68.1%). LCMS (ESI): $[M+H]^+$ = 289.2.

***Step 4: Preparation of tert-butyl ((1-cyclopropyl-6-fluoro-2-methyl-4-oxo-1,4-dihydroquinolin-7-yl)methyl)(isopropyl)carbamate:***

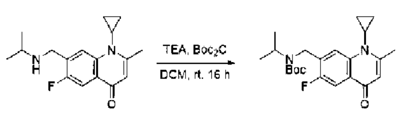

The compound from step 3 (1.60 g, 5.55 mmol, 1.0 eq.) was dissolved in DCM (DCM, 20 mL). Triethylamine (1.68 g, 16.65 mmol, 3.0 eq.) and di-tert-butyl dicarbonate (1.82 g, 8.32 mmol, 1.5 eq.) were added at room temperature, and the mixture was stirred at room temperature for 16 hours. LCMS monitoring indicated the reaction was complete. The organic phase was concentrated to afford a crude product, which was purified by flash column chromatography on silica gel (eluent: EA:PE = 0 to 1:4, v/v) to give the title compound as a yellow solid (1.6 g, yield: 74.42%). LCMS (ESI): $[M+H]^+$ = 389.3.

Using tert-butyl ((1-cyclopropyl-6-fluoro-2-methyl-4-oxo-1,4-dihydroquinolin-7-yl)methyl)(isopropyl)carbamate (the intermediate prepared in step 4) as the starting material, the title compound was obtained as a yellow solid via dibromination, alkylation with Intermediate 1 and radical cyclization reactions, following the synthetic procedures described in steps 2, 3 and 4 of Example 207. LCMS (ESI): $[M+H]^+$ = 594.3; $^1H$ NMR (400 MHz, DMSO-$d_6$) δ: 8.02 (s, 1H), 7.89 (d, J = 9.6 Hz, 1H), 7.24 (s, 1H), 6.32 (br, 1H), 5.40-5.30 (m, 4H), 4.49 (s, 2H), 3.56-3.41 (m, 2H), 1.79-1.76 (m, 2H), 1.46-1.10 (m, 19H), 0.81 (t, J = 7.2 Hz, 3H).

Example 222: Preparation of (S)-11-cyclopropyl-4-ethyl-8-fluoro-4-hydroxy-9-((isopropylamino)methyl)-1H-pyrano [3,4:6,7]indolizino[2,1 -b]quinoline-3,6,14(4H,11H,12H)-trione:

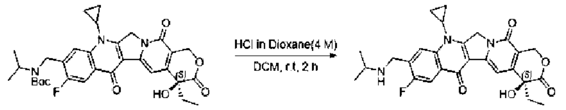


The compound from Example 221 (90 mg, 0.15 mmol, 1.0 eq.) was dissolved in DCM (4 mL), followed by the addition of 4 M hydrogen chloride in 1,4-dioxane (1 mL). The reaction mixture was stirred at room temperature for 2 hours, and LCMS monitoring indicated the reaction was complete. Concentration under reduced pressure afforded a crude product (80 mg), of which 20 mg was subjected to preparative purification. Purification by preparative HPLC (C18 column, 0.1% TFA in water, acetonitrile as eluent) gave the title compound as a yellow solid (10.58 mg, yield: 56.55%). LCMS (ESI): $[M+H]^+$ = 494.4; $^1H$ NMR (400 MHz, DMSO-$d_6$) δ: 9.30 (s, 2H), 8.63 (d, J = 6.0 Hz, 1H), 8.05 (d, J = 9.2 Hz, 1H), 7.29 (s, 1H), 6.38 (br, 1H), 5.50-5.33 (m, 4H), 4.43 (s, 2H), 3.63-3.59 (m, 1H), 3.49-3.44 (m, 1H), 1.86-1.79 (m, 2H), 1.50-1.33 (m, 10H), 0.85 (t, J = 7.2 Hz, 3H).

Example 223: Preparation of (S)-11-cyclopropyl-4-ethyl-8-fluoro-4-hydroxy-9-((isopropyl(methyl)amino)methyl)-1H-pyrano[3,4:6,7]indolizino[2,1 -b] quinoline-3,6,14(4H,11H,12H)-trione:

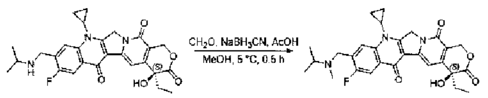


The compound from Example 222 (30 mg, 0.06 mmol, 1.0 eq.) was dissolved in MeOH (1 mL). Aqueous formaldehyde solution (9.59 mg, 0.12 mmol, 2.0 eq., 38% w/w) and glacial acetic acid (11 mg, 0.18 mmol, 3.0 eq.) were added, and the mixture was stirred in an ice-water bath for 0.5 hours. Sodium cyanoborohydride (11.3 mg, 0.18 mmol, 3.0 eq.) was then added, and the reaction was further stirred in an ice-water bath for another 0.5 hours. LCMS monitoring indicated the reaction was complete. One drop of 1 M hydrochloric acid was directly added to the reaction system, and the mixture was subjected to preparative HPLC purification (C18 column, 0.1% TFA in water, acetonitrile as eluent), affording the title compound as a yellow solid (11.5 mg, yield: 36.52%). LCMS (ESI): $[M+H]^+$ = 508.3; $^1H$ NMR (400 MHz, DMSO-$d_6$) δ: 8.59 (d, J = 6.0 Hz, 1H), 8.02 (d, J = 9.6 Hz, 1H), 7.25 (s, 1H), 5.46-5.27 (m, 4H), 4.67-4.37 (m, 2H), 3.68-3.55 (m, 2H), 2.67 (s, 3H), 1.81-1.74 (m, 2H), 1.48-1.28 (m, 10H), 0.81 (t, J = 7.2 Hz, 3H).

General Synthetic Procedure for Examples 224-239:

Examples 224 and 225 were synthesized following the synthetic procedures described in **Examples 222 and 223.** Examples 226, 227, 228, 229, 230 and 231 were synthesized using **7-bromo-1-cyclopentyl-6-fluoro-2-methylquinolin-4(1H)-one (Intermediate 6)** from Preparation Example 6 as the starting material, following the synthetic procedures described in **Examples 221 and 222.**

Examples 232-239 were synthesized using **(Z)-1-(4-bromo-2,5-difluorophenyl)-3-(dimethylamino)but-2-**

**en-1-one (Intermediate 4)** from Preparation Example 4 and various commercially available amines as the starting materials, following the synthetic procedures described in **Examples 96 and 97.**

The structures and specific characterization data (LCMS and $^1$H NMR) of the above compounds are as follows:

| Examples | Struchure | $^1$H NMR | LCMS (ESI) $[M+H]^+$ |
|---|---|---|---|
| 224 | 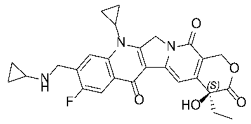 | (400 MHz, MeOD-$d_4$) δ 8.57-8.51 (m, 1H), 8.11-7.84 (m, 1H), 7.51-7.35 (m, 1H), 5.53-5.48 (m, 3H), 5.32-5.24 (m, 1H), 4.64 (s, 2H), 3.70-3.62 (m, 1H), 2.92-2.83 (m, 1H), 1.93-1.87 (m, 2H), 1.64-1.50 (m, 2H), 1.39 (s, 2H), 1.00-0.93 (m, 7H). | 492.2 |
| 225 | 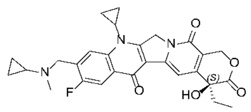 | (400 MHz, DMSO-$d_6$) δ 8.43 (d, *J* = 6.0 Hz, 1H), 7.99 (d, *J* = 9.6 Hz, 1H), 7.26 (s, 1H), 5.43 (d, *J* = 9.3 Hz, 2H), 5.31 (dd, *J* = 32.6, 15.8 Hz, 2H), 4.59 (s, 2H), 3.63-3.57 (m, 1H), 2.86 (s, 3H), 1.84-1.74 (m, 2H), 1.50 (d, *J* = 7.1 Hz, 1H), 1.43 (d, *J* = 5.8 Hz, 2H), 1.25 (s, 2H), 0.82 (t, *J* = 7.4 Hz, 3H), 0.75 (d, *J* = 7.1 Hz, 2H), 0.69 (s, 2H). | 506.3 |
| 226 | 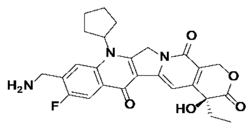 | (400 MHz, DMSO-$d_6$) δ 8.76-8.58 (m, br, 3H), 8.09 (d, *J* = 9.6 Hz, 1H), 7.88 (d, J = 5.6 Hz, 1H), 7.30 (s, 1H), 5.54 (s, 2H), 5.40-5.30 (m, 2H), 5.07-4.94(m, 1H), 4.30 (d, J = 5.2 Hz, 2H), 2.43-2.30 (m, 2H), 2.29-2.18 (m, 2H), 2.15-2.04 (m, 2H), 1.86-1.73 (m, 4H), 0.85 (t, J = 7.2 Hz, 3H). | 480.2 |
| 227 | 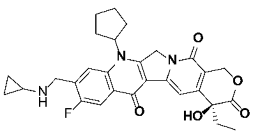 | (400 MHz, DMSO-$d_6$) 8.07 (d, *J* = 9.6 Hz, 1H), 8.02 (d, *J* = 5.8 Hz, 1H), 7.28 (s, 1H), 5.58-5.48 (m, 2H), 5.38-5.29 (m, 2H), 5.07-4.98 (m, 1H), 4.47 (s, 2H), 2.75 (s, 1H), 2.39 (t, *J* = 7.1 Hz, 2H), 2.22 (s, 2H), 2.11 (s, 2H), 1.87-1.69 (m, 4H), 0.94-0.92 (m, 2H), 0.84 (t, *J* = 7.3 Hz, 3H), 0.75 (q, *J* = 6.7 Hz, 2H). | 520.4 |
| 228 | 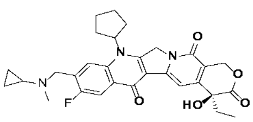 | (400 MHz, DMSO-$d_6$) δ 8.07 (d, *J* = 9.9 Hz, 1H), 7.84 (d, *J* = 5.5 Hz, 1H), 7.28 (s, 1H), 5.47 (brs, 2H), 5.33 (q, *J* = 15.6 Hz, 2H), 4.95 (t, *J* = 9.3 Hz, 1H), 4.50-4.33 (m, 2H), 2.80-2.65 (m, 3H), 2.63-2.54 (m,1H), 2.34-2.16 (m, 4H), 2.12-2.01 (m, 2H), 1.84-1.74 (m, 4H), 0.82 (t, *J* = 7.1 Hz, 3H), 0.74-0.65 (m, 2H), 0.64-0.53 (m, 2H). | 534.3 |
| 229 | 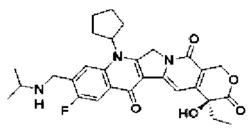 | (400 MHz, DMSO-$d_6$) δ 9.35-9.16 (m, br, 2H), 8.13 (d, *J* = 9.6 Hz, 1H), 7.96 (d, J = 6.0 Hz, 1H), 7.30 (s, 1H), 6.37 (br, s, 1H), 5.55 (s, 2H), 5.40-5.31 (m, 2H), 5.07-5.01 (m, 1H), 4.46-4.36 (m, 2H), 350-3.46 (m, 1H), 2.40-2.19-2.18 (m, 4H), 2.15-2.04 (m, 2H), 1.86-1.74 (m, 4H), 0.85 (t, J = 7.2 Hz, 3H). | 522.3 |
| 230 | 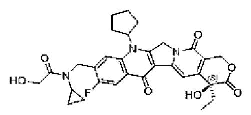 | (400 MHz, DMSO-$d_6$) δ 7.94 (d, *J* = 10.4 Hz, 1H), 7.71 (d, *J* = 6.0 Hz, 1H), 7.46 (s, 1H), 5.54-5.41 (m, 2H), 5.28 (d, *J* = 15.7 Hz, 1H), 5.00-4.92 (m, 2H), 4.85 (s, 2H), 4.52 (s, 2H), 2.80-2.74 (m, 1H), 2.42 (d, *J* = 8.0 Hz, 2H), 2.36-2.30 (m, 2H), 2.22-2.16 (m, 2H), 1.97-1.91 (m, 4H), 0.97-0.89 (m, 5H), 0.82 (s, 2H). | 578.3 |
| 231 | 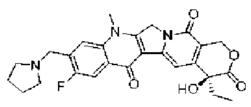 | (400 MHz, DMSO-$d_6$) δ 8.31 (d, *J* = 5.7 Hz, 1H), 8.10 (d, *J* = 9.6 Hz, 1H), 7.30 (s, 1H), 5.47 - 5.21 (m, 4H), 4.63 (s, 2H), 3.95 (s, 3H), 3.54 - 3.46 (m, 2H), 3.26 - 3.18 (m, 2H), 2.15 - 2.06 (m, 2H), 1.99 - 1.87 (m, 2H), 1.86 - 1.78 (m, 2H), 0.87 (t, *J* = 7.3 Hz, 3H). | 480.3 |

(continued)

| Examples | Struchure | $^1$H NMR | LCMS (ESI) $[M+H]^+$ |
|---|---|---|---|
| 232 | 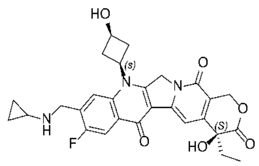 | (400 MHz, DMSO-$d_6$+$D_2O$) δ 8.22 (d, *J* = 6.0 Hz, 1H), 8.07 (d, *J* = 9.3 Hz, 1H), 7.33 (s, 1H), 5.46 (t, *J* = 4.4 Hz, 2H), 5.33 (dd, *J* = 24.2, 15.7 Hz, 2H), 4.68-4.59 (m, 1H), 4.52 (q, *J* = 7.0 Hz, 2H), 4.06-3.99 (m, 1H), 3.26-3.17 (m, 2H), 2.82-2.75 (m, 1H), 2.58-2.54 (m, 2H), 1.85-1.75 (m, 2H), 1.52 (d, *J* = 7.1 Hz, 1H), 1.28-1.21 (m, 1H), 0.86-0.79 (m, 7H). | 522.3 |
| 233 | 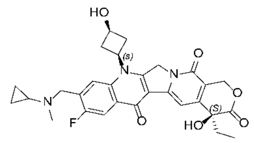 | (400 MHz, DMSO-$d_6$+$D_2O$) δ 8.19 (d, *J* = 5.8 Hz, 1H), 8.07 (d, *J* = 9.3 Hz, 1H), 7.33 (s, 1H), 5.49-5.42 (m, 2H), 5.32 (q, *J* = 15.6 Hz, 2H), 4.69-4.61 (m, 3H), 4.06-3.99 (m, 1H), 3.25-3.15 (m, 2H), 2.98-2.92 (m, 4H), 2.43 (t, *J* = 9.2 Hz, 2H), 1.86-1.73 (m, 2H), 0.85-0.68 (m, 7H). | 536.4 |
| 234 | 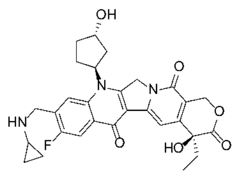 | (400 MHz, DMSO-$d_6$) δ 8.08 (d, J = 9.6 Hz, 1H), 7.75 (d, J = 5.5 Hz, 1H), 7.25 (s, 1H), 5.59-5.42 (m, 2H), 5.30 (dd, J = 23.5, 15.8 Hz, 2H), 5.17-5.08 (m, 1H), 4.47 (s, 3H), 2.77-2.69 (m, 1H), 2.63-2.53 (m, 1H), 2.42-2.26 (m, 3H), 2.17-2.08 (m, 1H), 1.82-1.70 (m, 3H), 0.82-0.72 (m, 7H). | 536.4 |
| 235 | 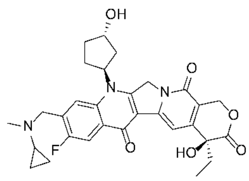 | (400 MHz, DMSO-$d_6$) δ 8.13 (d, J = 9.3 Hz, 1H), 7.84 (d, J = 5.5 Hz, 1H), 7.29 (s, 1H), 5.55 (dd, J = 48.2, 19.9 Hz, 2H), 5.40-5.29 (m, 2H), 5.22-5.13 (m, 1H), 4.69 (brs, 2H), 4.56-4.50 (m, 1H), 2.98-2.88 (m, 4H), 2.67-2.55 (m, 1H), 2.46-2.30 (m, 3H), 2.22-2.12 (m, 1H), 1.86-1.74 (m, 3H), 1.00-0.66 (m, 7H). | 550.4 |
| 236 | 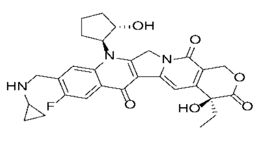 | δ 8.12 (d, *J* = 9.6 Hz, 1H), 7.84 (s, 1H), 7.29 (s, 1H), 5.64 (d, *J* = 20.1 Hz, 1H), 5.38-5.26 (m, 3H), 4.89 (q, *J* = 7.5 Hz, 1H), 4.70-4.63 (m, 1H), 4.52 (s, 2H), 2.81-2.73 (m, 1H), 2.41-2.29 (m, 2H), 2.25-2.03 (m, 2H), 1.92-1.63 (m, 4H), 0.86 (s, 2H), 0.84-0.81 (m, 3H), 0.80-0.75 (m, 2H)。 | 536.3 |
| 237 | 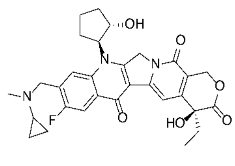 | (400 MHz, DMSO-$d_6$) 10.93-11.10 (m, 1H), 8.13 (d, *J* = 8.5 Hz, 1H), 7.99-7.82 (m, 1H), 7.29 (s, 1H), 6.55-6.14 (m, 1H), 5.67 (d, *J* = 20.1 Hz, 1H), 5.44-5.19 (m, 3H), 5.02-4.82 (m, 1H), 4.80-4.59 (m, 3H), 3.06-2.81 (m, 3H), 2.42-2.26 (m, 2H), 2.26-2.04 (m, 2H), 1.96-1.87 (m, 1H), 1.85-1.76 (m, 2H), 1.75-1.62 (m, 2H), 1.10-0.90 (m, 1H), 0.86 (d, *J* = 7.1 Hz, 3H), 0.81-0.59 (m, 3H). | 550.4 |
| 238 | 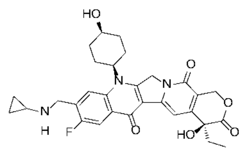 | (400 MHz, DMSO-$d_6$) δ 9.80 (s, 2H), 8.73 - 8.46 (m, 1H), 8.10 (d, *J* = 9.6 Hz, 1H), 7.30 (s, 1H), 6.38 (s, 1H), 5.66 - 5.46 (m, 2H), 5.44 - 5.28 (m, 2H), 4.91 (s, 1H), 4.55 - 4.36 (m, 3H), 4.08 - 3.91 (m, 1H), 2.97 - 2.72 (m, 3H), 2.00 - 1.76 (m, 7H), 1.01 - 0.83 (m, 6H), 0.86 - 0.72 (m, 2H). | 550.4 |
| 239 | 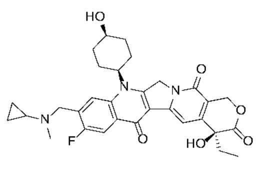 | (400 MHz, DMSO-$d_6$) δ 10.67 (s, 1H), 8.63 (d, J = 76.9 Hz, 1H), 8.18 - 8.02 (m, 1H), 7.30 (s, 1H), 6.37 (s, 1H), 5.66 - 5.49 (m, 2H), 5.41 - 5.29 (m, 2H), 4.73 - 4.41 (m, 3H), 4.04 - 3.97 (m, 1H), 3.00 - 2.75 (m, 7H), 1.95 - 1.77 (m, 8H), 1.50 - 1.41 (m, 1H), 1.10 - 0.97 (m, 1H), 0.91 - 0.82 (m, 5H), 0.81 - 0.66 (m, 2H). | 564.3 |

Example 240: Preparation of (S)-9-((cyclopropylamino)methyl)-4-ethyl-8-fluoro-4-hydroxy-11-(4-(trifluoromethyl)phenyl)-1,12-dihydro-14H-pyrano[3,4:6,7] indolizino[2,1-b]quinoline-3,6,14(4H,11H)-trione:

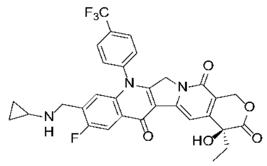

Using **Intermediate 23 from Preparation Example 23** as the starting material, the title compound was synthesized following the synthetic procedures described in **Steps 1-5 of Example 207,** as a pale yellow solid. LCMS (ESI): $[M+H]^+$ = 596.2.

Example 241: Preparation of (S)-9-((cyclopropylamino)methyl)-11-(2,2-difluorobenzo[d][1,3]dioxol-5-yl)-4-ethyl-8-fluoro-4-hydroxy-1,12-dihydro-14H-pyrano[3,4:6,7]indolizino[2,1 -b]quinoline-3,6,14(4H,11H)-trione:

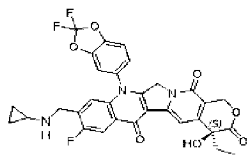

The title compound was synthesized following the synthetic procedures described in Example 240, as a pale yellow solid. LCMS (ESI): $[M+H]^+$ = 608.2.

General Synthetic Procedure for Examples 242-247:

Using **(2,5-difluorophenyl)-3-(dimethylamino)but-2-en-1-one (Intermediate 27)** from Preparation Example 27 and the commercial reagent **tert-butyl ((IR,3R)-3-aminocyclopentyl)carbamate** as the starting materials, the compound of Example 242 was synthesized following the synthetic procedures described in **Steps 1-6 of Example 102.**

Using the compound of Example 242 as the starting material, the compound of Example 243 was synthesized following the synthetic procedure described in **Example 103.**

Using the compound of Example 243 as the starting material, **methylation** afforded the compound of Example 244, **acetylation** afforded the compound of Example 245, and **reductive amination with cyclobutanone** afforded the compound of Example 246, respectively.

Using **Intermediate 27** from Preparation Example 27 and the commercial reagent **tert-butyl ((1R,3S)-3-aminocyclopentyl)carbamate** as the starting materials, the compound of Example 247 was synthesized following the synthetic procedure described in **Example 243.**

The structures and specific characterization data (LCMS and $^1$H NMR) of the above compounds are as follows:

| Examples | Structure | $^1$H NMR | LCMS (ESI) $[M+H]^+$ |
|---|---|---|---|
| 242 | 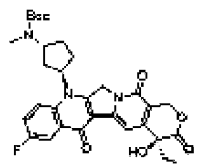 | (400 MHz, DMSO-$d_6$) δ 8.01 (dd, $J$ = 8.7, 3.2 Hz, 1H), 7.86 (dd, $J$ = 9.8, 4.0 Hz, 1H), 7.73-7.67 (m, 1H), 7.26 (s, 1H), 6.30 (brs., 1H), 5.50 (s, 2H), 5.36-5.27 (m, 2H), 5.17-5.10 (m, 1H), 5.09-4.90(m, 1H), 2.79 (s, 3H), 2.48-2.12 (m, 2H), 2.05-1.73 (m, 4H), 1.40 (s, 9H), 1.25 (s, 2H), 0.82 (t, $J$ = 7.3 Hz, 3H) | 580.4 |

(continued)

| Examples | Structure | $^1$H NMR | LCMS (ESI) [M+H]$^+$ |
|---|---|---|---|
| 243 | 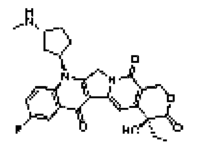 | (400 MHz, DMSO-$d_6$) δ 9.03 (brs., 2H), 8.05 (dd, J = 8.9, 3.2 Hz, 1H), 7.92 (dd, J = 9.3, 3.8 Hz, 1H), 7.70-7.65 (m, 1H), 7.31 (s, 1H), 6.37 (brs., 1H), 5.58-5.46 (m, 2H), 5.36-5.18 (m, 2H), 5.22-5.13 (m, 1H), 4.10-4.00 (m, 1H), 2.73-2.58 (m, 5H), 2.45-2.40 (m, 3H), 2.02-1.90 (m, 1H), 1.87-1.77 (m, 2H), 0.86 (t, J = 7.3 Hz, 3H) | 480.5 |
| 244 | 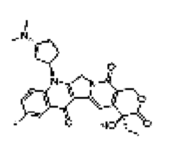 | (400 MHz, DMSO-$d_6$) δ 10.79 (brs., 1H), 8.05 (dd, J = 8.8, 3.6 Hz, 1H), 7.86 (dd, J = 9.6, 3.6 Hz, 1H), 7.70-7.65 (m, 1H), 7.30 (s, 1H), 6.37 (s, 1H), 5.62-5.50(m, 2H), 5.36-5.31 (m,2H), 5.18-5.08 (m, 1H), 4.33-4.23 (m, 1H), 2.82 (s, 6H), 2.67-2.60 (m, 2H), 2.46-2.32 (m, 3H), 2.08-1.98 (m, 1H), 1.88-1.77 (m, 2H), 0.86 (t, J = 7.3 Hz, 3H) | 494.3 |
| 245 | 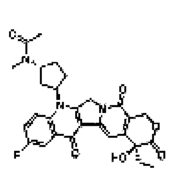 | (400 MHz, DMSO-$d_6$) δ 8.03 (dd, *J* = 8.8, 3.3 Hz, 1H), 7.99-7.77 (m, 1H), 7.80-7.69 (m, 1H), 7.30 (s, 1H), 6.34 (s, 1H), 5.62-5.45 (m, 2H), 5.39-5.36 (m, 2H), 5.25-4.94 (m, 1H), 3.17 (s, 1H), 2.98 (s, 2H), 2.82 (s, 1H), 2.45-2.24 (m, 3H), 2.18 (s, 1H), 2.09 (d, *J*= 11.8 Hz, 1H), 2.03 (s, 2H), 1.99-1.88 (m, 1H), 1.86-1.77 (m, 2H), 1.19-1.17 (m, 1H), 0.86 (t, *J* = 7.3 Hz, 3H)$_o$ | 522.3 |
| 246 | 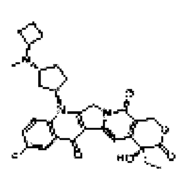 | (400 MHz, DMSO-$d_6$+$D_2O$) δ 8.07-8.02 (m, 1H), 7.92-7.84 (m, 1H), 7.71-7.66 (m, 1H), 7.31-7.09 (m, 1H), 5.63-5.49 (m, 1H), 5.41-5.29 (m, 2H), 5.15-5.04 (m, 1H), 4.35 (s, 1H), 3.86-3.72 (m, 1H), 2.76-2.70 (m, 5H), 2.43-2.25 (m, 7H), 2.07-1.96 (m, 1H), 1.88-1.67 (m, 4H), 1.23 (s, 1H), 0.86 (t, *J* = 7.1 Hz, 3H) | 534.4 |
| 247 | 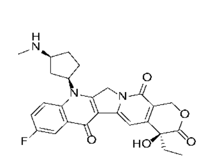 | (400 MHz, DMSO-$d_6$) δ 8.03 (dd, J = 8.7, 3.2 Hz, 1H), 7.92 (dd, J = 9.5, 4.0 Hz, 1H), 7.71-7.67 (m, 1H), 7.29 (s, 1H), 5.50-5.40 (m, 2H), 5.38-5.25 (m, 2H), 4.96-5.10 (1H), 3.66-3.58 (m, 1H), 2.66 (s, 1H), 2.61 (t, J = 6.6 Hz, 3H), 2.56 (d, J = 10.2 Hz, 1H), 2.44-2.36 (m, 2H), 2.32-2.24 (m, 1H), 2.19-2.12 (m, 1H), 1.84-1.74 (m, 2H), 0.82 (t, J = 7.3 Hz, 3H)$_o$ | 480.3 |

**Example 248: Preparation of (R)-3-((S)-4-ethyl-8-fluoro-4-hydroxy-3,6,14-trioxo-4,6,12,14-tetrahydro-1H-pyrano [3,4:6,7]indolizino[2,1 -b]quinolin-11(3H)-yl)pyrrolidine-1-carboxylate:**

***Step 1: Preparation of tert-butyl (R)-3-((2-cyano-4-fluorophenyl)amino)pyrrolidine-1-carboxylate:***

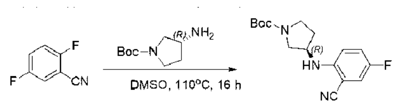


2,5-Difluorobenzonitrile (10.0 g, 71.9 mmol, 1.0 eq.) and tert-butyl (R)-3-aminopyrrolidine-1-carboxylate (26.8 g, 143.8 mmol, 2.0 eq.) were dissolved in dimethyl sulfoxide (DMSO, 80 mL) sequentially. Under nitrogen protection, the reaction mixture was heated to 110 °C and stirred for 16 hours, and LCMS monitoring indicated the reaction was complete. The reaction mixture was poured into ice water (150 mL) and extracted with ethyl acetate (50 mL × 2). The combined organic phases were washed with saturated sodium chloride solution (100 mL), dried over anhydrous sodium sulfate, filtered and concentrated to afford a crude product. Purification by silica gel column chromatography (eluent: PE:EtOAc = 1:0 to 10:1, v/v) gave the title compound as a white solid (12 g, yield: 54.8%). LCMS (ESI): [M-Boc]$^+$ = 206.1; $^1$H NMR (400 MHz, DMSO-$d_6$) δ: 7.48 (dd, J = 8.5, 3.1 Hz, 1H), 7.40-7.31 (m, 1H), 6.91 (dd, J = 9.7, 4.3 Hz, 1H), 6.02 (d, J = 6.5 Hz, 1H), 4.17-4.02 (m, 2H), 3.65-3.51 (m, 2H), 3.20-3.12 (m, 1H), 2.17-2.06 (m, 1H), 1.95-1.82 (m, 1H), 1.40 (s, 9H).

***Step 2: Preparation of tert-butyl(R)-3-((2-acetyl-4-fluorophenyl)amino)pyrrolidine-1-carboxylate:***

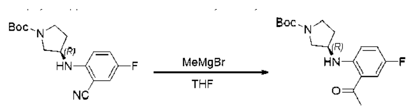


The intermediate prepared in step 1 (11 g, 36.0 mmol, 1.0 eq.) was dissolved in anhydrous tetrahydrofuran (THF, 200 mL). Under nitrogen protection and an ice bath, 3 M methylmagnesium bromide in tetrahydrofuran (24 mL, 72.0 mmol, 2.0 eq.) was added slowly. After the addition, the mixture was stirred at room temperature for an additional 2 hours, and LCMS monitoring indicated the reaction was complete. The reaction mixture was poured into ice-cold saturated aqueous ammonium chloride solution (300 mL) and extracted with ethyl acetate (50 mL × 3). The combined organic phases were dried over anhydrous sodium sulfate, filtered and concentrated to afford a crude product. Purification by silica gel column chromatography (eluent: PE:EA= 1:0 to 5:1, v/v) gave the title compound as a light brown solid (6.0 g, yield: 51.6%). LCMS (ESI): $[M+H]^+$ = 323.1; $^1H$ NMR (400 MHz, DMSO-$d_6$) δ: 8.72 (d, J = 6.6 Hz, 1H), 7.67 (dd, J = 10.2, 3.0 Hz, 1H), 7.40-7.21 (m, 1H), 6.88 (dd, J = 9.4, 4.5 Hz, 1H), 4.29-4.11 (m, 1H), 3.67-3.53 (m, 1H), 3.17-3.04 (m, 1H), 2.53-2.49 (m, 2H), 2.28-2.12 (m, 1H), 1.89-1.74 (m, 1H), 1.40 (s, 9H).

***Step 3: Preparation of ethyl(R)-1-(1-(tert-butoxycarbonyl)pyrrolidin-3-yl)-6-fluoro-4-oxo-1,4-dihydroquinoline-2-carboxylate:***

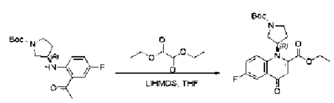


The intermediate prepared in step 2 (3.0 g, 9.31 mmol, 1.0 eq.) and diethyl oxalate (3.40 g, 23.3 mmol, 2.5 eq.) were dissolved in anhydrous tetrahydrofuran (THF, 50 mL) sequentially. Under nitrogen protection at -65 °C, 1 M lithium bis(trimethylsilyl)amide in tetrahydrofuran (32.6 mL, 32.6 mmol, 3.5 eq.) was added dropwise slowly. After the addition, the dry ice bath was retained, and the mixture was allowed to warm to room temperature slowly and stirred overnight. The reaction mixture was poured into ice-cold saturated aqueous ammonium chloride solution (100 mL) and extracted with ethyl acetate (40 mL × 3). The combined organic phases were dried over anhydrous sodium sulfate, filtered and concentrated under reduced pressure to afford a crude product. Purification by silica gel column chromatography (eluent: DCM:MeOH = 1:0 to 30:1, v/v) gave the title compound as a light brown solid (2.0 g, yield: 53.1%). LCMS (ESI): $[M+H]^+$ = 405.3; $^1H$ NMR (400 MHz, DMSO-$d_6$) δ: 7.88-7.83 (m, 1H), 7.76-7.67 (m, 2H), 6.38 (s, 1H), 5.31-5.18 (m, 1H), 4.23-4.08 (m, 2H), 3.81-3.68 (m, 2H), 3.47 (s, 4H), 2.41-2.29 (m, 2H), 1.42 (s, 9H), 1.36 (t, J = 7.1 Hz, 3H).

**Step 4: Preparation of tert-butyl(R)-3-(6-fluoro-2-(hydroxymethyl)-4-oxoquinolin-1(4H)-yl)pyrrolidine-1-carboxylate**

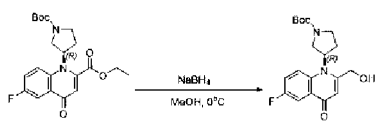


The intermediate prepared in step 3 (1.90 g, 4.70 mmol, 1.0 eq.) was dissolved in MeOH (40 mL). Under an ice bath, sodium borohydride (444 mg, 11.7 mmol, 2.5 eq.) was added slowly, and the mixture was stirred for an additional 15 minutes under the ice bath after the addition. TLC monitoring indicated the reaction was complete. The reaction was quenched by the addition of saturated sodium chloride solution (3 mL), and the reaction mixture was concentrated to afford a crude product. Purification by silica gel column chromatography (eluent: DCM:MeOH = 1:0 to 20:1, v/v) gave the title compound as an off-white solid (900 mg, yield: 52.8%). $^1H$ NMR (400 MHz, DMSO-$d_6$) δ: 7.95-7.80 (m, 1H), 7.74-7.52 (m, 2H), 6.26 (s, 1H), 5.86 (t, J = 5.7 Hz, 1H), 5.58-5.46 (m, 1H), 4.76-4.59 (m, 2H), 3.91-3.63 (m, 4H), 1.44 (s, 9H).

***Step 5: Preparation of tert-butyl(R)-3-(6-fluoro-2-(hydroxymethyl)-3-iodo-4-oxoquinolin-1(4H)-yl)pyrrolidine-1-carboxylate:***

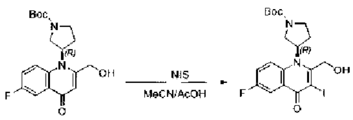


The intermediate prepared in Step 4 (900 mg, 2.48 mmol, 1.0 eq.) was dissolved in a mixture of acetonitrile (20 mL) and acetic acid (0.1 mL). N-Iodosuccinimide (669 mg, 2.98 mmol, 1.2 eq.) was added, and the mixture was stirred at room temperature for 16 hours after the addition. LCMS monitoring indicated the reaction was complete. The reaction mixture was concentrated under reduced pressure to afford a crude product. Purification by silica gel column chromatography (eluent: DCM:MeOH = 1:0 to 20:1, v/v) gave the title compound as a light brown solid (1.0 g, yield: 82.5%). LCMS (ESI): $[M+H]^+$ = 489.2; $^1H$ NMR (400 MHz, DMSO-$d_6$) δ: 7.90 (dd, J = 8.8, 2.9 Hz, 1H), 7.73-7.61 (m, 2H), 6.08 (s, 1H), 5.89-5.78 (m, 1H), 5.23 (s, 2H), 3.91-3.67 (m, 4H), 2.44-2.31 (m, 2H), 1.43 (s, 9H).

***Step 6: Preparation of tert-butyl(R)-3-(6-fluoro-2-(chloromethyl)-3-iodo-4-oxoquinolin-1(4H)-yl)pyrrolidine-1-carboxylate:***

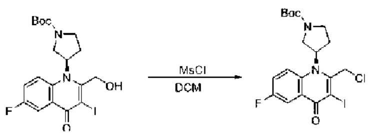


The intermediate prepared in Step 5 (800 mg, 1.64 mmol, 1.0 eq.) was dissolved in anhydrous DCM (20 mL). Under an ice bath, triethylamine (531 mg, 4.92 mmol, 3.0 eq.) and methanesulfonyl chloride (472 mg, 3.28 mmol, 2.0 eq.) were added sequentially. The mixture was stirred for an additional 1 hour under the ice bath, and TLC monitoring indicated the complete consumption of the starting material. The reaction mixture was poured into ice water (30 mL) and extracted with DCM (15 mL × 2). The combined organic phases were dried over anhydrous sodium sulfate, filtered and concentrated under reduced pressure to afford the title compound as a light brown oil (500 mg, crude, yield not calculated). LCMS (ESI): $[M+H]^+$ = 507.1.

***Step 7: Preparation of tert-butyl(R)-3-(2-(((S)-4-ethyl-4-hydroxy-3,8-dioxo-4,8-dihydro-1H-pyrano[3,4-c]pyridin-7(3H)-yl)methyl)-6-fluoro-3-iodo-4-oxoquinolin-1(4H)-yl)pyrrolidine-1-carboxylate:***

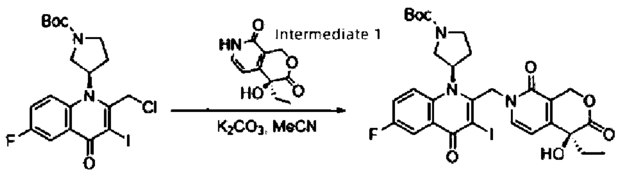


The intermediate prepared in Step 6 (500 mg, 0.988 mmol, 1.0 eq.) and Intermediate 1 (165 mg, 0.791 mmol, 0.8 eq.) were dissolved in acetonitrile (10 mL) sequentially. Potassium carbonate (409 mg, 2.96 mmol, 3.0 eq.) was added, and the mixture was heated to 35 °C and stirred overnight after the addition. LCMS monitoring indicated the reaction was complete. The reaction mixture was filtered, and the filtrate was concentrated under reduced pressure to afford a crude product. Purification by silica gel column chromatography (eluent: DCM:MeOH = 1:0 to 20:1, v/v) gave the title compound as a light brown solid (300 mg, yield: 44.7%). LCMS (ESI): $[M+H]^+$ = 680.

***Step 8: Preparation of tert-butyl (R)-3-((S)-4-ethyl-8-fluoro-4-hydroxy-3,6,14-trioxo-4,6,12,14-tetrahydro-1H-pyrano[3',4:6,7]indolizino[2,1-b]quinolin-1](3H)-yl)pyrrolidine-l-carboxylate:***

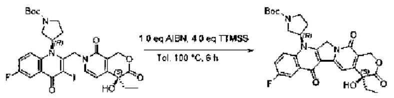


The intermediate prepared in Step 7 (6.6 g, 9.71 mmol, 1.0 eq.) was dissolved in ultra-dry toluene (440 mL). 2,2'-Azobis(isobutyronitrile) (1.595 g, 9.71 mmol, 1.0 eq.) and tris(trimethylsilyl)silane (9.66 g, 38.84 mmol, 4.0 eq.) were added, and the reaction system was purged with a stream of nitrogen for 10 minutes. The reaction mixture was heated to 100 °C and stirred for 6 hours, and LCMS monitoring indicated the reaction was complete. After cooling to room temperature, a white solid precipitated out. The reaction mixture was concentrated under reduced pressure, and then toluene (60 mL) was added and the mixture was stirred for half an hour. The mixture was filtered, the solid was collected and concentrated under reduced pressure to give the title compound as a yellow solid (2.9 g, yield: 54.2%). LCMS (ESI): $[M+H]^+$ = 552.2.

**Example 249: Preparation of (S)-4-ethyl-8-fluoro-4-hydroxy-11-((R)-pyrrolidin-3-yl)-1,12-dihydro-14H-pyrano[3',4':6,7]indolizino[2,1-b]quinoline-3,6,14(4H, 11H)-trione:**

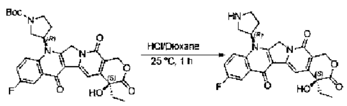


The compound from Example 248 (2.9 g, 5.26 mmol, 1.0 eq.) was dissolved in DCM (40 mL), followed by the addition of 4 M hydrogen chloride in 1,4-dioxane (2.0 mL). The reaction mixture was stirred at room temperature for 1 hour, and LCMS monitoring indicated the reaction was complete. The mixture was filtered, and the solid was collected and dried in vacuo to give the title compound as a yellow solid (2.0 g, yield: 84%). LCMS (ESI): $[M+H]^+$ = 452.2.

**Example 250: Preparation of (S)-4-ethyl-8-fluoro-4-hydroxy-11-((R)-1-methylpyrrolidin-3-yl)-1,12-dihydro-14H-pyrano[3',4':6,7]indolizino[2,1-b] quinoline-3,6,14(4H,11H)-trione:**

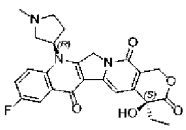

The compound from Example 249 (500 mg, 1.11 mmol, 1.0 eq.) was dissolved in MeOH (20 mL). Paraformaldehyde (66 mg, 2.22 mmol, 2.0 eq.) and acetic acid (199 mg, 3.33 mmol, 3.0 eq.) were added, and the mixture was stirred at 25 °C for 30 minutes. Sodium cyanoborohydride (205 mg, 3.33 mmol, 3.0 eq.) was then added, and the reaction was stirred at 25 °C for 1 hour. LCMS monitoring indicated the reaction was complete. 4 mL of 1 M hydrochloric acid was added to the system, and the mixture was subjected to preparative purification. Purification by preparative HPLC (C18 column, 0.1% aqueous HCl, acetonitrile as eluent) gave the title compound as a yellow solid (203.54 mg, yield: 39.39%). LCMS (ESI): $[M+H]^+$ = 466.3; $^1H$ NMR (400 MHz, DMSO-$d_6$) δ: 11.67 (brs, 1H), 8.31-8.29 (m, 1H), 8.07-8.05 (m, 1H), 7.73-7.63 (m, 1H), 7.31 (s, 1H), 6.40 (s, 1H), 5.62-5.33 (m, 4H), 4.18-4.13 (m, 1H), 3.89-3.60 (m, 2H), 3.26-3.36 (m, 1H), 3.06 (s, 1H), 2.95 (d, J = 3.8 Hz, 3H), 2.81-2.66 (m, 2H), 1.86-1.79 (m, 2H), 0.86 (t, J = 7.3 Hz, 3H).

**Example 251: Preparation of (S)-4-ethyl-8-fluoro-4-hydroxy-11-((R)-1-ethylpyrrolidin-3-yl)-1,12-dihydro-14H-pyrano[3',4':6,7]indolizino[2,1-b] quinoline-3,6,14(4H,11H)-trione:**

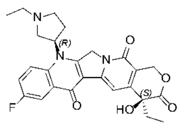

The compound from Example 249 (100 mg, 0.22 mmol, 1.0 eq.) was dissolved in MeOH (5 mL). Acetaldehyde (19 mg, 0.44 mmol, 2.0 eq.) and acetic acid (39 mg, 0.66 mmol, 3.0 eq.) were added, and the mixture was stirred at 25 °C for 30 minutes. Sodium cyanoborohydride (40 mg, 0.66 mmol, 3.0 eq.) was then added, and the reaction was stirred at 25 °C for 1 hour. LCMS monitoring indicated the reaction was complete. One drop of 1 M HCl was added to the system, and the mixture was purified by preparative HPLC (C18 column, 0.1% aqueous HCl, acetonitrile as eluent) and lyophilized to give the title compound as a yellow solid (8.87 mg, yield: 8.41%). LCMS (ESI): $[M+H]^+$ = 480.3; $^1H$ NMR (400 MHz, DMSO-$d_6$) δ: 11.47 (s, 1H), 8.39-8.35 (m, 1H), 8.08-8.05 (m, 1H), 7.64-7.63 (m, 1H), 7.31 (s, 1H), 6.39 (s, 1H), 5.62-5.36 (m, 4H), 4.13-4.09 (m, 1H), 3.94-3.90 (m, 1H), 3.76-3.66 (m, 1H), 3.29-3.20 (m, 2H), 2.67-2.65 (m, 3H), 2.33 (s, 1H), 1.83-1.81 (m, 2H), 1.33-1.31 (m, 3H), 0.86 (t, J = 7.3 Hz, 3H).

**Example 252: Preparation of (S)-4-ethyl-8-fluoro-4-hydroxy-11-((R)-1-(2-hydroxyethyl)pyrrolidin-3-yl)-1,12-dihydro-14H-pyrano[3',4':6,7]indolizino[2,1-b]quinoline-3,6,14(4H,11H)-trione:**

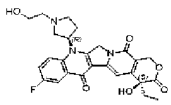

The compound from Example 249 (30 mg, 0.07 mmol, 1.0 eq.) was dissolved in MeOH (5 mL). tert-Butyldimethylsilyloxyacetaldehyde (23 mg, 0.14 mmol, 2.0 eq.) and acetic acid (12 mg, 0.2 mmol, 3.0 eq.) were added, and the mixture was stirred at 25 °C for 30 minutes. Sodium cyanoborohydride (13 mg, 0.2 mmol, 3.0 eq.) was then added, and the reaction was stirred at 25 °C for 1 hour. LCMS monitoring indicated the reaction was complete. One drop of 1 M HCl was added to the system, and the mixture was purified by preparative HPLC (C18 column, 0.1% aqueous HCl, acetonitrile as eluent) and lyophilized to give the title compound as a yellow solid (5.6 mg, yield: 13.82%). LCMS (ESI): $[M+H]^+$ = 496.3; $^1H$ NMR (400 MHz, DMSO-$d_6$) δ: 11.22 (s, 1H), 8.33 (s, 1H), 8.08-8.06 (m, 1H), 7.72-7.67 (m, 1H), 7.31 (s, 1H), 5.62-5.32 (m, 4H), 4.11 (s, 1H), 3.92-3.74 (m, 4H), 3.40-3.19 (m, 1H), 2.84-2.75 (m, 1H), 2.68-2.66 (m, 2H), 2.35-2.35 (m, 2H), 1.88-1.80 (m, 2H), 0.85 (t, J = 7.3 Hz, 3H).

**Example 253: Preparation of (S)-4-ethyl-8-fluoro-4-hydroxy-11-((R)-1-cyclobutylpyrrolidin-3-yl)-1,12-dihydro-14H-pyrano[3',4':6,7]indolizino[2,1-b]quinoline-3,6,14(4H,11H)-trione**

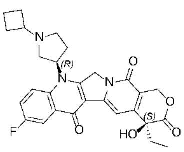

The compound from Example 249 (100 mg, 0.22 mmol, 1.0 eq.) was dissolved in MeOH (5 mL). Cyclobutanone (16 mg, 0.44 mmol, 2.0 eq.) and acetic acid (40 mg, 0.66 mmol, 3.0 eq.) were added, and the mixture was stirred at 25 °C for 30 minutes. Sodium cyanoborohydride (41 mg, 0.66 mmol, 3.0 eq.) was then added, and the reaction was stirred at 25 °C for 1 hour. LCMS monitoring indicated the reaction was complete. One drop of 1 M HCl was added to the system, and the mixture was purified by preparative HPLC (C18 column, 0.1% aqueous HCl, acetonitrile as eluent) and lyophilized to give the title compound as a yellow solid (19.19 mg, yield: 17.14%). LCMS (ESI): $[M+H]^+$ = 506.3; $^1H$ NMR (400 MHz, DMSO-$d_6$) δ: 12.40 (s, 1H), 8.49-8.46 (m, 1H), 8.06-8.05 (m, 1H), 7.65-7.63 (m, 1H), 7.30 (s, 1H), 5.60-5.50 (m, 2H), 5.45-5.32 (m, 2H), 4.06-4.00 (m, 1H), 3.95-3.77 (m, 3H), 3.16-3.14 (m, 1H), 2.70-2.67 (m, 1H), 2.65-2.62 (m, 2H), 2.46-2.37 (m, 1H), 2.27-2.19 (m, 3H), 1.84-1.81 (m, 4H), 0.86 (t, J = 7.3 Hz, 3H).

**Example 254: Preparation of (S)-4-ethyl-8-fluoro-4-hydroxy-11-((R)-1-(3,3-dimethylcyclobutyl)pyrrolidin-3-yl)-1,12-dihydro-14H-pyrano[3',4':6,7]indolizino[2,1-b]quinoline-3,6,14(4H,11H)-trione:**

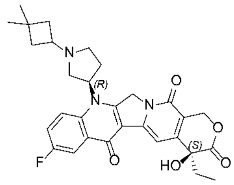

The compound from Example 249 (100 mg, 0.22 mmol, 1.0 eq.) was dissolved in MeOH (5 mL). 3,3-Dimethylcyclobutanone (43 mg, 0.44 mmol, 2.0 eq.) and acetic acid (40 mg, 0.66 mmol, 3.0 eq.) were added, and the mixture was stirred at 25 °C for 30 minutes. Sodium cyanoborohydride (41 mg, 0.66 mmol, 3.0 eq.) was then added, and the reaction was stirred at 25 °C for 1 hour. LCMS monitoring indicated the reaction was complete. One drop of 1 M HCl was added, and the mixture was purified by preparative HPLC (C18 column, 0.1% aqueous HCl, acetonitrile as eluent) and lyophilized to give the title compound as a yellow solid (24.01 mg, yield: 20.44%). LCMS (ESI): $[M+H]^+$ = 534.5; $^1H$ NMR (400 MHz, DMSO-$d_6$) δ: 12.03 (s, 1H), 8.35-8.32 (m, 1H), 8.06-8.04 (m, 1H), 7.68-7.63 (m, 1H), 7.30 (s, 1H), 6.40 (s, 1H), 5.61-5.50 (m, 2H), 5.37-5.36 (m, 2H), 4.05-3.99 (m, 1H), 3.97-3.93 (m, 1H), 3.84-3.82 (m, 1H), 3.61-3.36 (m, 1H), 3.18-3.17 (m, 1H), 2.67-2.50 (m, 2H), 2.33-2.22 (m, 3H), 2.29-2.03 (m, 2H), 1.84-1.81 (m, 2H), 1.18-1.16 (m, 6H), 0.85 (t, J = 7.3 Hz, 3H).

**Example 255: Preparation of (S)-4-ethyl-8-fluoro-4-hydroxy-11-((R)-1-(3,3-difluorocyclobutyl)pyrrolidin-3-yl)-1,12-dihydro-14H-pyrano[3',4':6,7]indolizino[2,1-b]quinoline-3,6,14(4H,11H)-trione:**

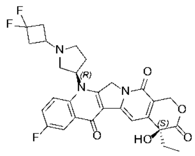

The compound from Example 249 (100 mg, 0.22 mmol, 1.0 eq.) was dissolved in MeOH (5 mL). 3,3-Difluorocyclobutanone (47 mg, 0.44 mmol, 2.0 eq.) and acetic acid (40 mg, 0.66 mmol, 3.0 eq.) were added, and the mixture was stirred at 25 °C for 30 minutes. Sodium cyanoborohydride (41 mg, 0.66 mmol, 3.0 eq.) was then added, and the reaction was stirred at 25 °C for 1 hour. LCMS monitoring indicated the reaction was complete. One drop of 1 M HCl was added to the system, and the mixture was purified by preparative HPLC (C18 column, 0.1% aqueous HCl, acetonitrile as eluent) and lyophilized to give the title compound as a yellow solid (30.89 mg, yield: 25.75%). LCMS (ESI): $[M+H]^+$ = 542.3; $^1H$ NMR (400 MHz, DMSO-$d_6$) δ: 12.96 (s, 1H), 8.42-8.38 (m, 1H), 8.01-7.98 (m, 1H), 7.67-7.61 (m, 1H), 7.25 (s, 1H), 5.57-5.28 (m, 4H), 4.25-3.62 (m, 4H), 3.26-2.99 (m, 5H), 2.65-2.63 (m, 1H), 2.51-2.50 (m, 1H), 2.31-2.29 (m, 1H), 1.80-1.77 (m, 2H), 0.82 (t, J = 7.0 Hz, 3H).

**Example 256: Preparation of (S)-4-ethyl-8-fluoro-4-hydroxy-11-((R)-1-methyl-D2-pyrrolidin-3-yl)-1,12-dihydro-14H-pyrano[3',4':6,7]indolizino[2,1-b]quinoline-3,6,14(4H,11H)-trione:**

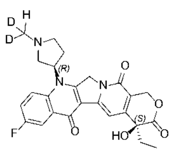

The compound from Example 249 (110 mg, 0.24 mmol, 1.0 eq.) was dissolved in MeOH (5 mL). Formaldehyde-d2 (16 mg, 0.48 mmol, 2.0 eq.) and acetic acid (44 mg, 0.72 mmol, 3.0 eq.) were added, and the mixture was stirred at 25 °C for 30 minutes. Sodium cyanoborohydride (46 mg, 0.48 mmol, 3.0 eq.) was then added, and the reaction was stirred at 25 °C for 1 hour. LCMS monitoring indicated the reaction was complete. One drop of 1 M HCl was added to the system, and the

mixture was purified by preparative HPLC (C18 column, 0.1% aqueous HCl, acetonitrile as eluent) and lyophilized to give the title compound as a yellow solid (25.91 mg, yield: 22.7%). LCMS (ESI): $[M+H]^+$ = 468.3; $^1$H NMR (400 MHz, DMSO-$d_6$) δ: 11.52 (s, 1H), 8.27-8.24 (m, 1H), 8.08-8.05 (m, 1H), 7.67-7.64 (m, 1H), 7.29 (s, 1H), 6.55 (s, 1H), 5.62-5.32 (m, 4H), 4.15-4.10 (m, 1H), 3.88-3.86 (m, 1H), 3.29-3.19 (m, 1H), 2.92-2.91 (m, 1H), 2.80-2.61 (m, 3H), 2.59-2.54 (m, 1H), 1.82-1.77 (m, 2H), 0.87-0.80 (m, 3H).

**Example 257: Preparation of (S)-4-ethyl-8-fluoro-4-hydroxy-11-((R)-1-cyclopentylpyrrolidin-3-yl)-1,12-dihydro-14H-pyrano[3',4':6,7]indolizino[2,1-b] quinoline-3,6,14(4H,11H)-trione:**

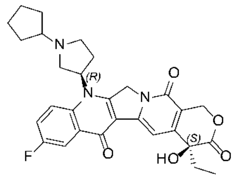

The compound from Example 249 (100 mg, 0.22 mmol, 1.0 eq.) was dissolved in MeOH (5 mL). Cyclopentanone (37 mg, 0.44 mmol, 2.0 eq.) and acetic acid (40 mg, 0.66 mmol, 3.0 eq.) were added, and the mixture was stirred at 25 °C for 30 minutes. Sodium cyanoborohydride (41 mg, 0.66 mmol, 3.0 eq.) was then added, and the reaction was stirred at 25 °C for 1 hour. LCMS monitoring indicated the reaction was complete. One drop of 1 M HCl was added to the system, and the mixture was purified by preparative HPLC (C18 column, 0.1% aqueous HCl, acetonitrile as eluent) and lyophilized to give the title compound as a yellow solid (67.85 mg, yield: 59.32%). LCMS (ESI): $[M+H]^+$ = 520.2; $^1$H NMR (400 MHz, DMSO-$d_6$) δ: 11.62 (s, 1H), 8.40-8.37 (m, 1H), 8.05-8.02 (m, 1H), 7.67-7.62 (m, 1H), 7.30 (s, 1H), 6.38 (s, 1H), 5.64-5.31 (m, 4H), 4.13 (m, J = 13.2 Hz, 1H), 3.90-3.70 (m, 3H), 2.82-2.61 (m, 2H), 2.55-2.53 (m, 3H), 2.32-2.31 (m, 1H), 2.10-1.77 (m, 6H), 1.59-1.57 (m, 2H), 0.85 (t, J = 7.3 Hz, 3H).

**Example 258: Preparation of (S)-4-ethyl-8-fluoro-4-hydroxy-11-((R)-1-n-propylpyrrolidin-3-yl)-1,12-dihydro-14H-pyrano[3',4':6,7]indolizino[2,1-b] quinoline-3,6,14(4H,11H)-trione:**

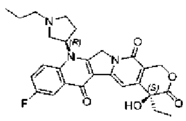

The compound from Example 249 (100 mg, 0.22 mmol, 1.0 eq.) was dissolved in MeOH (5 mL). Propanal (38.6 mg, 0.66 mmol, 3.0 eq.) and acetic acid (39.63 mg, 0.66 mmol, 3.0 eq.) were added, and the mixture was stirred at 25 °C for 20 minutes. Sodium cyanoborohydride (27 mg, 0.44 mmol, 2.0 eq.) was then added, and the reaction was stirred at 25 °C for 3 hours. LCMS monitoring indicated the reaction was complete. One drop of 1 M hydrochloric acid was added, and the reaction mixture was concentrated to 3 mL. Purification by preparative HPLC (C18 column, 0.01% aqueous HCl, acetonitrile as eluent) and lyophilization gave the title compound as a yellow solid (16 mg, yield: 14.74%). LCMS (ESI): $[M+H]^+$ = 494.2; $^1$H NMR (400 MHz, DMSO-$d_6$) δ: 8.51-8.47 (m, 1H), 8.08-7.95 (m, 1H), 7.62-7.59 (m, 1H), 7.30 (s, 1H), 5.62-5.49 (m, 2H), 5.44-5.32 (m, 2H), 4.13-4.09 (m, 1H), 3.89-3.69 (m, 3H), 3.36-3.18 (m, 3H), 2.85-2.63 (m, 2H), 1.88-1.70 (m, 4H), 1.00-0.95 (m, 3H), 0.92-0.86 (m, 3H).

**Example 259: Preparation of (S)-11-((R)-1-(cyclopropylmethyl)pyrrolidin-3-yl)-4-ethyl-8-fluoro-4-hydroxy-1H-pyrano[3',4':6,7]indolizino[2,1-b]quinoline-3,6,14(4H,11H,12H)-trione:**

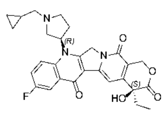

The compound from Example 249 (100 mg, 0.22 mmol, 1.0 eq.) was dissolved in MeOH (MeOH, 5 mL).

Cyclopropanecarbaldehyde (30 mg, 0.44 mmol, 2.0 eq.) and acetic acid (40 mg, 0.66 mmol, 3.0 eq.) were added, and the mixture was stirred at 25 °C for 30 minutes. Sodium cyanoborohydride (40 mg, 0.66 mmol, 3.0 eq.) was then added, and the reaction was stirred at 25 °C for 1 hour. LCMS monitoring indicated the reaction was complete. 1 mL of 1 M HCl was directly added to the system, and the mixture was subjected to preparative purification. Purification by preparative HPLC (C18 column, 0.1% aqueous HCl, acetonitrile as eluent) gave the title compound as a yellow solid (47 mg, yield: 42%). LCMS (ESI): $[M+H]^+$ = 506.2; $^1H$ NMR (400 MHz, DMSO-$d_6$) δ: 11.54 (s, 1H), 8.42-8.39 (m, 1H), 8.06-8.05 (m, 1H), 7.68-7.63 (m, 1H), 7.30 (s, 1H), 6.37 (s, 1H), 5.62-5.31 (m, 4H), 4.14-4.11 (m, 1H), 3.94 (d, J = 5.8 Hz, 1H), 3.78 (d, J = 9.3 Hz, 1H), 3.47-3.44 (m, 2H), 3.19-3.15 (m, 1H), 2.85-2.66 (m, 2H), 2.33-2.31 (m, 1H), 1.83-1.79 (m, 2H), 1.23-1.18 (m, 1H), 0.86-0.83 (m, 3H), 0.66-0.63 (m, 2H), 0.46-0.45 (m, 2H).

**Example 260: Preparation of (S)-4-ethyl-8-fluoro-4-hydroxy-11-((R)-1-isobutylpyrrolidin-3-yl)-1H-pyrano [3',4':6,7]indolizino[2,1-b]quinoline-3,6,14 (4H,11H,12H)-trione:**

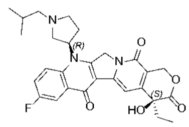

The compound from Example 249 (100 mg, 0.22 mmol, 1.0 eq.) was dissolved **in** MeOH (15 mL). 2-Methylpropanal (31 mg, 0.44 mmol, 2.0 eq.), acetic acid (39 mg, 0.66 mmol, 3.0 eq.) and sodium cyanoborohydride (27 mg, 0.44 mmol, 2.0 eq.) were added, and the reaction was stirred at 25 °C for 3 hours. LCMS monitoring indicated the reaction was complete. Purification by preparative HPLC gave the title compound as a yellow solid (18 mg, yield: 16.12%). LCMS (ESI): $[M+H]^+$ = 508.2; $^1H$ NMR (400 MHz, DMSO-$d_6$) δ: 8.59-8.56 (m, 1H), 8.08-7.94 (m, 1H), 7.74-7.61 (m, 1H), 7.30 (s, 1H), 5.64-5.45 (m, 2H), 5.40-5.32 (m, 2H), 4.19-3.74 (m, 3H), 3.34-3.15 (m, 5H), 2.95-2.86 (m, 1H), 2.75-2.61 (m, 1H), 2.11-2.04 (m, 1H), 1.87-1.75 (m, 2H), 1.05-0.98 (m, 6H), 0.85 (t, J = 7.3 Hz, 3H).

Example 261: Preparation of (S)-4-ethyl-8-fluoro-4-hydroxy-11-((R)-1-(pentan-3-yl)pyrrolidin-3-yl)-1H-pyrano[3,4:6,7] indolizino[2,1 -b]quinoline-3,6,14 (4H,11H,12H)-trione:

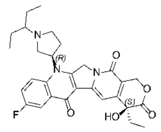

The compound from Example 249 (50 mg, 0.11 mmol, 1.0 eq.), glacial acetic acid (0.1 mL) and pentan-3-one (38.76 mg, 0.45 mmol, 5.0 eq.) were dissolved in anhydrous MeOH (5 mL). The reaction mixture was incubated at 30 °C for 0.5 h, followed by the addition of sodium cyanoborohydride (16.69 mg, 0.27 mmol, 3.0 eq.). The mixture was further incubated at 30 °C for 1 h, and LCMS monitoring indicated the reaction was complete. Two drops of 1 M HCl were directly added to the system, and the mixture was subjected to preparative purification. Purification by preparative HPLC (C18 column, 0.1% aqueous HCl, acetonitrile as eluent) afforded the title compound as a white solid (10.94 mg, yield: 23.31%). LCMS (ESI): $[M+H]^+$ = 522.3; $^1H$ NMR (400 MHz, DMSO-$d_6$+$D_2O$) δ: 8.38 (s, 1H), 8.00-7.98 (m, 1H), 7.64-7.61 (m, 1H), 7.25 (s, 1H), 5.42-5.26 (m, 4H), 4.15-4.05 (m, 1H), 3.85-3.82 (m, 2H), 3.39-3.38 (m, 1H), 2.91-2.84 (m, 1H), 2.79-2.68 (m, 1H), 2.65-2.55 (m, 1H), 1.79-1.76 (m, 6H), 0.94-0.92 (m, 6H), 0.81 (t, J = 7.3 Hz, 3H).

Example 262: Preparation of (S)-4-ethyl-8-fluoro-4-hydroxy-11-((R)-1-(2,2,2-trifluoroethyl)pyrrolidin-3-yl)-1,12-dihydro-14H-pyrano[3,4:6,7]indolizino[2,1 -b]quinoline-3,6,14(4H,11H)-trione:

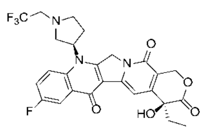

The compound from Example 249 (80.0 mg, 0.180 mmol, 1.0 eq.) was dissolved **in** DMF (2 mL). Under an ice bath, 2,2,2-trifluoroethyl trifluoromethanesulfonate (82.0 mg, 0.350 mmol, 2.0 eq.) and N,N-diisopropylethylamine (45.8 mg, 0.350 mmol, 2.0 eq.) were added slowly, and the mixture was stirred at room temperature for an additional 16 h. The reaction was quenched by the dropwise addition of 1 M dilute hydrochloric acid (0.1 mL), and the mixture was poured into ice water (20 mL) and extracted with ethyl acetate (10 mL × 3). The combined organic phases were washed with saturated sodium chloride solution (30 mL), dried over anhydrous sodium sulfate, filtered and concentrated, and purified by normal-phase silica gel column chromatography (eluent: DCM:MeOH = 1:0 to 50:1, v/v) gave a crude product, which was further purified by reverse-phase preparative HPLC to afford the title compound as an off-white solid (2.1 mg, yield: 2.19%). LCMS (ESI): $[M+H]^+$ = 534.3; $^1H$ NMR (400 MHz, DMSO-$d_6$) δ: 8.07-7.97 (m, 1H), 7.73-7.61 (m, 1H), 7.33-7.25 (m, 1H), 7.03 (s, 1H), 6.66 (s, 1H), 5.38-5.28 (m, 4H), 3.62-3.53 (m, 1H), 2.04-1.93 (m, 4H), 1.86-1.75 (m, 2H), 1.50-1.40 (m, 2H), 0.86 (t, J = 6.3 Hz, 3H).

Example 263: Preparation of (S)-4-ethyl-8-fluoro-4-hydroxy-11-((R)-1-isopropylpyrrolidin-3-yl)-1H-pyrano[3,4:6,7]indolizino[2,1 -b]quinoline-3,6,14 (4H,11H,12H)-trione:

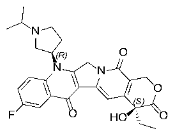

The compound from Example 249 (100 mg, 0.22 mmol, 1.0 eq.) was dissolved in MeOH (5 mL). Acetone (25 mg, 0.44 mmol, 2.0 eq.) and acetic acid (39 mg, 0.66 mmol, 3.0 eq.) were added, and the mixture was stirred at 25 °C for 30 min, followed by the addition of sodium cyanoborohydride (40 mg, 0.66 mmol, 3.0 eq.). The reaction was stirred at 25 °C for 1 h, and LCMS monitoring indicated the reaction was complete. 0.5 mL of 1 M hydrochloric acid was added to the system, and the mixture was purified by preparative HPLC (C18 column, 0.01% aqueous HCl, acetonitrile as eluent) to afford the title compound as a brownish yellow solid (7.13 mg, yield: 6.57%). LCMS (ESI): $[M+H]^+$ = 494; $^1H$ NMR (400 MHz, DMSO-$d_6$) δ: 11.91-11.85 (brs, 1H), 8.51-8.47 (m, 1H), 8.02-8.01 (m, 1H), 7.59-7.57 (m, 1H), 7.26 (s, 1H), 5.58-5.33 (m, 4H), 4.01-3.77 (m, 1H), 3.77-3.71 (m, 2H), 3.28-3.26 (m, 1H), 2.78-2.68 (m, 1H), 2.42-2.43 (m, 2H), 1.78-1.71 (m, 2H), 1.71 (s, 1H), 1.36-1.33 (m, 6H), 0.82 (t, J = 7.3 Hz, 3H).

Example 264: Preparation of (S)-4-ethyl-8-fluoro-4-hydroxy-11-((S)-1-methylpyrrolidin-3-yl)-1,12-dihydro-14H-pyrano [3,4:6,7]indolizino[2,1 -b] quinoline-3,6,14(4H,11H)-trione:

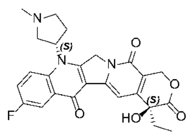

Similarly, using commercially available 2,5-difluorobenzonitrile and tert-butyl (S)-3-aminopyrrolidine-1-carboxylate as the starting materials, the title compound was synthesized according to the synthetic procedures of Example 249 and Example 250, as a yellow solid. LCMS (ESI): $[M+H]^+$ = 466.2; $^1H$ NMR (400 MHz, DMSO-$d_6$) δ: 8.01-7.92 (m, 2H), 7.67-7.65 (m, 1H), 7.24 (s, 1H), 5.54-5.20 (m, 4H), 4.22-3.81 (m, 3H), 3.61-3.58 (m, 1H), 3.33-3.15 (m, 1H), 2.96-2.92 (m, 3H), 2.70-2.62 (m, 2H), 1.85-1.73 (m, 2H), 0.80 (t, J = 7.3 Hz, 3H).

Example 265: Preparation of (S)-4-ethyl-8-fluoro-4-hydroxy-11-((R)-1-(2-hydroxyacetyl)pyrrolidin-3-yl)-1,12-dihydro-14H-pyrano[3,4:6,7]indolizino[2,1 -b]quinoline-3,6,14(4H,11H)-trione:

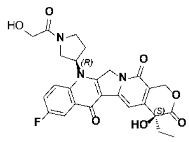

The compound from Example 249 (70.0 mg, 0.160 mmol, 1.0 eq.) was dissolved in DMF (2 mL). Then, glycolic acid (17.7 mg, 0.230 mmol, 1.5 eq.), O-(7-azabenzotriazol-1-yl)-N,N,N,N -tetramethyluronium hexafluorophosphate (HATU, 6.32 mg, 0.02 mmol, 1.5 eq.) and N,N-diisopropylethylamine (60.1 mg, 0.470 mmol, 3.0 eq.) were added sequentially. After the addition, the reaction mixture was stirred at room temperature for 16 h, and LCMS monitoring indicated the reaction was complete. The mixture was poured into ice water (15 mL) and extracted with ethyl acetate (10 mL × 3). The combined organic phases were dried over anhydrous sodium sulfate, filtered and concentrated, and purified by reverse-phase preparative HPLC to afford the title compound as an off-white solid (1.2 mg, yield: 1.47%). LCMS (ESI): $[M+H]^+$ = 510.3; $^1H$ NMR (400 MHz, DMSO-$d_6$) δ: 8.09-8.01 (m, 1H), 7.75-7.71 (m, 1H), 7.31 (s, 1H), 7.26-7.18 (m, 1H), 6.66 (s, 1H), 6.40-6.36 (m, 1H), 5.59-5.54 (m, 2H), 5.40-5.30 (m, 3H), 4.82-4.73 (m, 2H), 4.16-3.91 (m, 6H), 2.07-1.95 (m, 2H), 0.86 (t, J = 6.5 Hz, 3H).

Example 266: Preparation of (S)-11-((R)-1-acetylpyrrolidin-3-yl)-4-ethyl-8-fluoro-4-hydroxy-1H-pyrano[3,4:6,7]indolizino[2,1 -b]quinoline-3,6,14(4H,11H,12H)-trione:

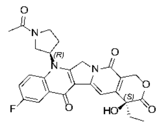

The compound from Example 249 (50 mg, 0.11 mmol, 1.0 eq.) was dissolved in a mixture of N,N-dimethylformamide and acetic anhydride (10:1, v/v, 0.5 mL). The reaction mixture was incubated at 30 °C for 1 h, and LCMS monitoring indicated the reaction was complete. The reaction mixture was directly purified by preparative HPLC (C18 column, 0.01% w/w aqueous HCl, acetonitrile as eluent) to afford the title compound as a white solid (7.06 mg, yield: 12.59%). LCMS (ESI): $[M+H]^+$ = 494.3; $^1H$ NMR (400 MHz, DMSO-$d_6$+$D_2O$) δ: 7.93-7.90 (m, 1H), 7.75-7.63 (m, 2H), 7.23 (d, J = 2.5 Hz, 1H), 5.44 (d, J = 3.6 Hz, 2H), 5.34-5.23 (m, 3H), 4.06-3.87 (m, 3H), 3.61-3.33 (m, 1H), 2.63-2.58 (m, 2H), 2.01-1.95 (m, 3H), 1.81-1.75 (m, 2H), 0.82 (td, J = 7.3, 2.8 Hz, 3H).

Example 267: Preparation of (S)-11-((R)-1-(4,4-difluorocyclohexyl)pyrrolidin-3-yl)-4-ethyl-8-fluoro-4-hydroxy-1H-pyrano[3,4:6,7]indolizino[2,1 -b]quinoline-3,6,14(4H,11H,12H)-trione:

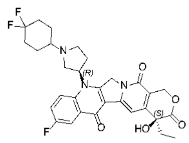

The compound from Example 249 (50 mg, 0.11 mmol, 1.0 eq.) was dissolved in MeOH (15 mL). 4,4-Difluorocyclohexanone (44.56 mg, 0.33 mmol, 3.0 eq.) and acetic acid (19.82 mg, 0.33 mmol, 3.0 eq.) were added, and the mixture was stirred for 0.5 h, followed by the addition of sodium cyanoborohydride (10.2 mg, 0.17 mmol, 1.5 eq.). The reaction was stirred at room temperature for 3 h, and LCMS monitoring indicated the reaction was complete. The reaction mixture was concentrated, dissolved in DMSO, and one drop of 1 M HCl was added. Purification by preparative HPLC (C18 column, 0.01% aqueous HCl, ACN as eluent) and lyophilization afforded the title compound as a yellow solid (9.2 mg, yield: 14.68%). LCMS (ESI): $[M+H]^+$ = 570.4; $^1H$ NMR (400 MHz, DMSO-$d_6$) δ: 8.68-8.65 (m, 1H), 8.03-7.88 (m, 1H), 7.70-7.54 (m, 1H), 7.28 (s, 1H), 5.63-5.44 (m, 2H), 5.36-5.31 (m, 2H), 4.09-3.76 (m, 3H), 3.43-3.28 (m, 3H), 2.87-2.67 (m, 2H), 2.26-2.07 (m, 4H), 2.00-1.75 (m, 6H), 0.90-0.82 (m, 3H).

Example 268: Preparation of (S)-11-((R)-1-cyclohexylpyrrolidin-3-yl)-4-ethyl-8-fluoro-4-hydroxy-1H-pyrano[3,4:6,7]indolizino[2,1 -b]quinoline-3,6,14(4H,11H, 12H)-trione:

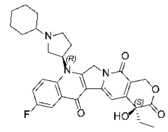

The compound from Example 249 (100 mg, 0.22 mmol, 1.0 eq.) was dissolved in MeOH (5 mL). Cyclohexanone (43 mg, 0.44 mmol, 2.0 eq.) and acetic acid (40 mg, 0.66 mmol, 3.0 eq.) were added, and the mixture was stirred at 25 °C for 30 min, followed by the addition of sodium cyanoborohydride (41 mg, 0.66 mmol, 3.0 eq.). The reaction was stirred at 25 °C for 1 h, and LCMS monitoring indicated the reaction was complete. 1 mL of 1 M HCl was directly added to the system, and the mixture was purified by preparative HPLC (C18 column, 0.1% aqueous HCl, acetonitrile as eluent) to afford the title compound as a yellow solid (39 mg, yield: 33%). LCMS (ESI): $[M+H]^+$ = 534.3; $^1H$ NMR (400 MHz, DMSO-$d_6$) δ: 11.77 (s, 1H), 8.51-8.41 (m, 1H), 8.08-8.04 (m, 1H), 7.65-7.60 (m, 1H), 7.27 (s, 1H), 6.38 (s, 1H), 5.68-5.47 (m, 2H), 5.45-5.32 (m, 2H), 4.06-3.99 (m, 1H), 3.92-3.76 (m, 2H), 3.19-3.15 (m, 1H), 2.83-2.72 (m, 1H), 2.67-2.64 (m, 2H), 2.33-2.31 (m, 1H), 2.20-2.19 (m, 1H), 2.04-2.01 (m, 1H), 1.87-1.75 (m, 4H), 1.66-1.44 (m, 3H), 1.28-1.13 (m, 3H), 0.88-0.84 (m, 3H).

Example 269: Preparation of (S)-11-((R)-1-(azetidin-3-yl)pyrrolidin-3-yl)-4-ethyl-8-fluoro-4-hydroxy-1H-pyrano[3,4:6,7] indolizino[2,1 -b]quinoline-3,6,14(4H, 11H,12H)-trione:

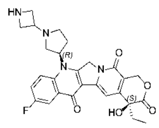

The compound from Example 249 (50.0 mg, 0.11 mmol, 1.0 eq.) was dissolved in MeOH (10 mL). tert-Butyl 3-oxoazetidine-1-carboxylate (56.88 mg, 0.33 mmol, 3.0 eq.) and acetic acid (19.95 mg, 0.33 mmol, 3.0 eq.) were added, and the mixture was stirred for 30 min, followed by the addition of sodium cyanoborohydride (13.7 mg, 0.22 mmol, 2.0 eq.). The reaction was stirred at room temperature for 3 **h,** and LCMS monitoring indicated the reaction was complete. The reaction mixture was directly concentrated under reduced pressure to afford the intermediate as a yellow solid (70 mg, crude, used directly in the next step). LCMS (ESI): $[M+H]^+$ = 607.1.

The above intermediate (70 mg, 0.12 mmol, 1.0 eq.) was dissolved in DCM (DCM, 1 mL), and 4 M hydrogen chloride in 1,4-dioxane (1 mL) was added. The reaction was stirred at r. t. for 2 **h,** and LCMS monitoring indicated the reaction was complete. The reaction mixture was concentrated, and purified by preparative HPLC (C18 column, 0.01% aqueous HCl, ACN as eluent) and lyophilization afforded the title compound as a yellow solid (20.1 mg, yield: 33.07%). LCMS (ESI): [M+H] =507.4; $^1H$ NMR (400 MHz, DMSO-$d_6$) δ: 9.75-9.57 (m, 1H), 8.71-8.63 (m, 1H), 7.98-7.95 (m, 1H), 7.81-7.72 (m, 1H), 7.25 (s, 1H), 5.52-5.29 (m, 4H), 4.39-4.03 (m, 5H), 3.37-3.15 (m, 3H), 3.10-2.88 (m, 1H), 2.73-2.59 (m, 2H), 1.86-1.76 (m, 3H), 0.87-0.83 (m, 3H).

Example 270: Preparation of (S)-4-ethyl-8-fluoro-4-hydroxy-11-((R)-1-(spiro[3.3]heptan-2-yl)pyrrolidin-3-yl)-1H-pyrano [3,4:6,7]indolizino[2,1 -b] quinoline-3,6,14(4H,11H,12H)-trione:

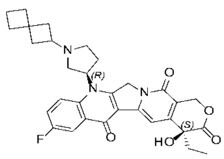

The compound from Example 249 (50 mg, 0.11 mmol, 1.0 eq.) was dissolved in MeOH (15 mL). Spiro[3.3] heptan-2-one (36.6 mg, 0.33 mmol, 3.0 eq.), acetic acid (19.95 mg, 0.33 mmol, 3.0 eq.) and sodium cyanoborohydride

(10.27 mg, 0.17 mmol, 1.5 eq.) were added successively. The reaction was stirred at room temperature for 2 h, and LCMS monitoring indicated the reaction was complete. The reaction mixture was concentrated under reduced pressure, dissolved in dimethyl sulfoxide (DMSO), and one drop of 1 M HCl was added. Purification by preparative HPLC (C18 column, 0.01% aqueous HCl, acetonitrile as eluent) and lyophilization afforded the title compound as a yellow solid (19.3 mg, yield: 32.16%). LCMS (ESI): $[M+H]^+$ = 546.3; $^1H$ NMR (400 MHz, DMSO-$d_6$) δ: 8.48-8.45 (m, 1H), 8.05-8.02 (m, 1H), 7.62-7.60 (m, 1H), 7.29 (s, 1H), 6.43 (s, 1H), 5.63-5.49 (m, 2H), 5.41-5.27 (m, 2H), 4.04-3.97 (m, 1H), 3.80-3.60 (m, 3H), 3.20-3.15 (m, 2H), 2.84-2.52 (m, 2H), 2.40-2.37 (m, 4H), 2.11-1.97 (m, 4H), 1.84-1.81 (m, 4H), 0.90-0.84 (m, 3H).

**Example 271: Preparation of (S)-4-ethyl-8-fluoro-4-hydroxy-11-((R)-1-(spiro[2.3]hexan-5-yl)pyrrolidin-3-yl)-1H-pyrano[3',4':6,7]indolizino[2,1-b]quinoline-3,6,14(4H,11H,12H)-trione:**

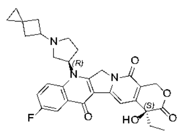

Compound 249 (50 mg, 0.11 mmol, 1.0 eq.) was dissolved in MeOH (15 mL), followed by the addition of spiro[2.3] hexan-5-one (31.94 mg, 0.33 mmol, 3.0 eq.), acetic acid (19.95 mg, 0.33 mmol, 3.0 eq.) and sodium cyanoborohydride (10.27 mg, 0.17 mmol, 1.5 eq.). The reaction mixture was stirred at room temperature for 2 **h,** and LCMS monitoring indicated the reaction was complete. The mixture was concentrated under reduced pressure, dissolved in dimethyl sulfoxide (DMSO), and one drop of 1 M HCl was added. Purification by preparative HPLC (C18 column, 0.01% aqueous HCl, acetonitrile as eluent) and lyophilization afforded the title compound as a yellow solid (20.17 mg, yield: 34.49%). LCMS (ESI): $[M+H]^+$ = 532.3; $^1H$ NMR (400 MHz, DMSO-$d_6$) δ: 8.61-8.54 (m, 1H), 8.02-7.93 (m, 1H), 7.73-7.57 (m, 1H), 7.27 (s, 1H), 6.36 (s, 1H), 5.63-5.46 (m, 2H), 5.40-5.31 (m, 2H), 4.18-4.06 (m, 2H), 3.85-3.67 (m, 2H), 3.28-3.18 (m, 2H), 2.90-2.53 (m, 4H), 2.33-2.17 (m, 2H), 1.87-1.75 (m, 2H), 0.87-0.82 (m, 3H), 0.53-0.50 (m, 4H).

**Example 272: Preparation of (S)-11-((R)-1-(3-(chloromethyl)-3-(hydroxymethyl)cyclobutyl)pyrrolidin-3-yl)-4-ethyl-8-fluoro-4-hydroxy-1H-pyrano[3',4':6,7]indolizino[2,1-b]quinoline-3,6,14(4H,11H,12H)-trione:**

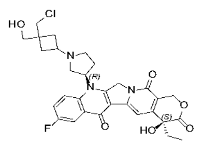

Compound 249 (50 mg, 0.11 mmol, 1.0 eq.) was dissolved in MeOH (15 mL), and 2-oxaspiro[3.3]heptan-6-one (37.26 mg, 0.33 mmol, 3.0 eq.) and acetic acid (19.95 mg, 0.33 mmol, 3.0 eq.) were added. After stirring for 0.5 h, sodium cyanoborohydride (10.27 mg, 0.17 mmol, 1.5 eq.) was added, and the mixture was stirred at room temperature for 2 h. LCMS monitoring indicated the reaction was complete. The mixture was concentrated under reduced pressure, dissolved in dimethyl sulfoxide (DMSO), and one drop of 1 M HCl was added. Purification by preparative HPLC (C18 column, 0.01% aqueous HCl, acetonitrile as eluent) and lyophilization afforded the title compound as a yellow solid (20.8 mg, yield: 34.53%). LCMS (ESI): $[M+H]^+$ = 584.5; $^1H$ NMR (400 MHz, DMSO-$d_6$) δ: 8.57-8.54 (m, 1H), 8.01-7.91 (m, 1H), 7.59-7.55 (m, 1H), 7.26 (s, 1H), 5.66-5.49 (m, 2H), 5.35-5.27 (m, 2H), 4.05-4.02 (m, 2H), 3.93-3.81 (m, 4H), 3.46-3.12 (m, 4H), 2.86-2.65 (m, 2H), 2.45-2.06 (m, 4H), 1.88-1.78 (m, 2H), 0.87-0.83 (m, 3H).

**Example 273: Preparation of (4S)-11-((3R)-1-(bicyclo[3.1.0]hexan-3-yl)pyrrolidin-3-yl)-4-ethyl-8-fluoro-4-hydroxy-1H-pyrano[3',4':6,7]indolizino[2,1-b]quinoline-3,6,14(4H,11H,12H)-trione:**

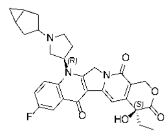

Compound 249 (50 mg, 0.11 mmol, 1.0 eq.) was dissolved in MeOH (15 mL), and bicyclo[3.1.0]hexan-3-one (31.72 mg, 0.33 mmol, 3.0 eq.) and acetic acid (19.82 mg, 0.33 mmol, 3.0 eq.) were added. The mixture was stirred at 25 °C for 30 min, then sodium cyanoborohydride (10.2 mg, 0.17 mmol, 1.5 eq.) was added, and the reaction was stirred at 25 °C for 2 h. LCMS monitoring indicated the reaction was complete. 1 mL of 1 M HCl was directly added to the system, and the mixture was purified by preparative HPLC (C18 column, 0.1% aqueous HCl, acetonitrile as eluent) to afford the title compound as a yellow solid (14.2 mg, yield: 24.28%). LCMS (ESI): $[M+H]^+$ = 532.3; $^1$H NMR (400 MHz, DMSO-$d_6$) δ: 12.40-12.12 (m, 1H), 8.55-8.52 (m, 1H), 8.07-8.01 (m, 1H), 7.72-7.56 (m, 1H), 7.29 (s, 1H), 6.40 (brs, 1H), 5.62-5.32 (m, 4H), 4.12-3.98 (m, 1H), 3.84-3.66 (m, 2H), 3.30-3.12 (m, 5H), 2.84-2.63 (m, 2H), 2.26-2.04 (m, 3H), 1.91-1.72 (m, 3H), 1.43-1.30 (m, 2H), 0.87 (t, J = 9.0 Hz, 3H).

**Example 274: Preparation of (4S)-11-((3R)-1-cyclopropylpyrrolidin-3-yl)-4-ethyl-8-fluoro-4-hydroxy-1H-pyrano [3',4':6,7]indolizino[2,1-b]quinoline-3,6,14(4H,11H,12H)-trione:**

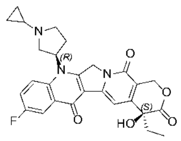

Compound 249 (50 mg, 0.11 mmol, 1.0 eq.) was dissolved in MeOH (15 mL), and bicyclo[3.1.0]hexan-3-one (31.72 mg, 0.33 mmol, 3.0 eq.) and acetic acid (19.82 mg, 0.33 mmol, 3.0 eq.) were added. The mixture was stirred at 25 °C for 30 min, then sodium cyanoborohydride (10.2 mg, 0.17 mmol, 1.5 eq.) was added, and the reaction was stirred at 25 °C for 2 h. LCMS monitoring indicated the reaction was complete. 1 mL of 1 M HCl was directly added to the system, and the mixture was purified by preparative HPLC (C18 column, 0.1% aqueous HCl, acetonitrile as eluent) to afford the title compound as a yellow solid (13 mg, yield: 24%). LCMS (ESI): $[M+H]^+$ = 492.3.

**Example 275: Preparation of (S)-4-ethyl-8-fluoro-4-hydroxy-11-((R)-1-(2-hydroxy-2-methylpropyl)pyrrolidin-3-yl)-1H-pyrano[3',4':6,7]indolizino[2,1-b]quinoline-3,6,14(4H,11H,12H)-trione:**

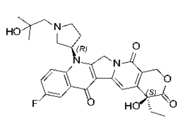

Acetone (1.0 g, 17.22 mmol, 1.0 eq.) was dissolved in MeOH (50 mL), and tosylmethyl isocyanide (TOSMIC) (3.7 g, 18.94 mmol, 1.1 eq.) and potassium carbonate (4.76 g, 34.44 mmol, 2.0 eq.) were added. The mixture was stirred at 25 °C for 5 h, then 2 N HCl (10 mL) was added, and the reaction was further stirred at 25 °C for 1 h. The reaction mixture was poured into saturated aqueous ammonium chloride solution (50 mL) and extracted with ethyl acetate (50 mL × 3). The combined organic phases were dried and concentrated under reduced pressure at low temperature to afford the title aldehyde as a crude product (1 g), which was used directly in the next step.

Compound 249 (50 mg, 0.11 mmol, 1.0 eq.) was dissolved in MeOH (3 mL), and the above crude aldehyde (100 mg) and acetic acid (19.95 mg, 0.33 mmol, 3.0 eq.) were added. The mixture was stirred at 25 °C for 30 min, then sodium cyanoborohydride (20.54 mg, 0.33 mmol, 3.0 eq.) was added, and the reaction was stirred at 25 °C for 1 h. LCMS monitoring indicated the reaction was complete. Two drops of 1 M HCl were directly added to the system, and the mixture was subjected to preparative purification. Purification by Prep-HPLC (C18 column, 0.1% aqueous HCl, acetonitrile as eluent) afforded the title compound as a yellow solid (3.1 mg, yield: 5.3%). LCMS (ESI): $[M+H]^+$ = 524.4; $^1$H NMR (400 MHz, DMSO-$d_6$) δ: 8.02-8.00 (m, 1H), 7.87-7.61 (m, 2H), 7.20-7.31 (m, 1H), 5.51-5.18 (m, 4H), 4.12-4.01 (m, 1H), 3.95-3.64 (m, 3H), 3.42-3.39 (m, 2H), 3.32-3.13 (m, 2H), 2.80-2.68 (m, 1H), 1.82-1.61 (m, 6H), 1.50-1.45 (m, 2H), 0.80 (t, J = 7.1 Hz, 3H).

**Example 276: Preparation of (S)-4-ethyl-8-fluoro-4-hydroxy-11-((R)-1-(3-hydroxycyclobutyl)pyrrolidin-3-yl)-1H-pyrano[3',4':6,7]indolizino[2,1-b]quinoline-3,6,14(4H,11H,12H)-trione:**

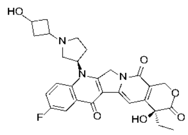

Compound 249 (50 mg, 0.11 mmol, 1.0 eq.) was dissolved in MeOH (10 mL), and 3-hydroxycyclobutan-1-one (19.07 mg, 0.22 mmol, 2.0 eq.), acetic acid (13.3 mg, 0.22 mmol, 2.0 eq.) and sodium cyanoborohydride (10.27 mg, 0.17 mmol, 1.5 eq.) were added sequentially. The reaction mixture was stirred at room temperature for 2 h, and LCMS monitoring indicated the reaction was complete. The mixture was concentrated under reduced pressure, dissolved in dimethyl sulfoxide (DMSO), and one drop of 1 M HCl was added. Purification by Prep-HPLC (C18 column, 0.01% aqueous HCl, acetonitrile as eluent) and lyophilization afforded the title compound as a yellow solid (6.9 mg, yield: 12.03%). LCMS (ESI): $[M+H]^+$ = 522.2; $^1$H NMR (400 MHz, DMSO-$d_6$) δ: 8.06-7.97 (m, 1H), 7.83-7.76 (m, 1H), 7.46-7.42 (m, 1H), 7.26 (s, 1H), 5.56-5.47 (m, 2H), 5.33-5.22 (m, 2H), 4.54-4.49 (m, 1H), 4.00-3.84 (m, 1H), 3.75-3.69 (m, 1H), 2.29-2.21 (m, 1H), 1.99-1.91 (m, 1H), 1.83-1.75 (m, 1H), 1.50 (d, *J* = 7.1 Hz, 2H), 1.33 (t, J = 7.0 Hz, 4H), 1.19-1.14 (s, 3H), 0.86-0.76 (m, 3H).

**Preparation of Example 277 - Example 283:**

Using (Z)-3-(dimethylamino)-1-(2,4,5-trifluorophenyl)but-2-en-1-one (Intermediate 30) obtained from Preparation Example 30 and commercially available tert-butyl (R)-3-aminopyrrolidine-1-carboxylate as the starting materials, the intermediate (S)-4-ethyl-8,9-difluoro-4-hydroxy-11-((R)-pyrrolidin-3-yl)-1H-pyrano[3',4':6,7]indolizino [2,1-b]quinoline-3,6,14(4H,11H,12H)-trione was synthesized according to the method of Example 95. Using this intermediate as the starting material, the target compounds (Example 277, 278, 279) were synthesized according to the method of Example 250.

Using (Z)-1-(4-methyl-2,5-difluorophenyl)-3-(dimethylamino)but-2-en-1-one (Intermediate 29) obtained from Preparation Example 29 and commercially available tert-butyl (R)-3-aminopyrrolidine-1-carboxylate as the starting materials, the intermediate (S)-4-ethyl-8-fluoro-4-hydroxy-9-methyl-11-((R)-pyrrolidin-3-yl)-1H-pyrano[3',4':6,7]indolizino[2,1-b]quinoline-3,6,14(4H,11H,12H)-trione was synthesized according to the method of Example 95. Using this intermediate as the starting material, the target compounds (Example 280, 281) were synthesized according to the method of Example 250.

Using (Z)-1-(4-methyl-2,5-difluorophenyl)-3-(dimethylamino)but-2-en-1-one (Intermediate 29) obtained from Preparation Example 29 and commercially available tert-butyl (R)-3-aminopyrrolidine-1-carboxylate as the starting materials, the intermediate (S)-4-ethyl-8-fluoro-4-hydroxy-9-methyl-11-((R)-pyrrolidin-3-yl)-1H-pyrano[3',4':6,7]indolizino[2,1-b]quinoline-3,6,14(4H,11H,12H)-trione was synthesized according to the method of Example 95. Using this intermediate as the starting material, the target compounds (Example 282, 283) were synthesized according to the method of Example 250.

The compound structures and specific characterization data (LCMS and $^1$H NMR) are as follows:

| Examples | Struture | $^1$H NMR |
|---|---|---|
| 277 | 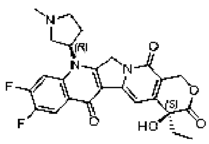 | (400 MHz, DMSO-$d_6$) δ 8.31-8.16 (m, 2H), 7.28 (s, 1H), 5.62-5.32 (m, 4H), 4.22-4.10 (m, 1H), 3.92-3.74 (m, 1H), 3.63-3.41 (m, 1H), 3.08-2.95 (m, 2H), 2.73-2.60 (m, 1H), 2.05-1.98 (m, 1H), 1.91-1.80 (m, 2H), 1.28-1.23 (m, 3H), 0.86-0.83 (m, 3H) |
| 278 | 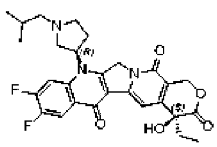 | (400 MHz, DMSO-$d_6$) δ 9.04-8.51 (m, 1H), 8.28-8.16 (m, 1H), 7.04 (s, 1H), 6.31 (s, 1H), 5.58-5.45 (m, 2H), 5.36-5.27 (m, 2H), 4.52-4.05 (m, 1H), 4.13-3.68 (m, 2H), 3.19-3.06 (m, 1H), 2.90-2.75 (m, 1H), 2.63-2.58 (m, 1H), 2.29-2.17 (m, 1H), 2.07-1.93 (m, 1H), 1.83-1.72 (m, 2H), 1.50 (d, *J* = 6.9 Hz, 1H), 1.23-1.19 (m, 2H), 1.07-0.96 (m, 5H), 0.81 (t, *J* = 7.3 Hz, 3H) |

(continued)

| Examples | Struture | $^1$H NMR |
|---|---|---|
| 279 | 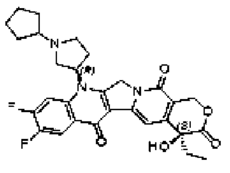 | (400 MHz, DMSO-$d_6$) δ 9.11-8.42 (m, 1H), 8.24-8.19 (m, 1H), 7.25 (s, 1H), 6.33 (s, 1H), 5.58-5.44 (m, 2H), 5.36-5.20 (m, 2H), 4.06-3.99 (m, 1H), 3.93-3.68 (m, 2H), 3.23 (s, 2H), 2.89-2.57 (m, 2H), 2.07-1.91 (m, 2H), 1.83-1.71 (m, 5H), 1.56-1.49 (m, 2H), 1.25-1.19 (m, 2H), 0.81 (t, J = 7.3 Hz, 3H) |
| 280 | 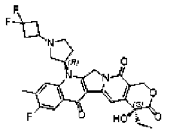 | (400 MHz, DMSO-$d_6$) δ 13.41 (s, 1H), 8.13-7.68 (m, 2H), 7.27 (s, 1H), 5.57-5.44 (m, 2H), 5.34-5.29 (m, 3H), 4.15-3.64 (m, 3H), 2.92-2.64 (m, 9H), 2.36-2.14 (m, 2H), 1.87-1.76 (m, 2H), 0.85 (t, J = 7.3 Hz, 3H) |
| 281 | 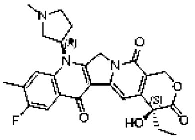 | (400 MHz, DMSO-$d_6$)δ 12.07 (s, 1H), 7.99-7.89 (m, 2H), 7.30 (s, 1H), 6.35 (s, 1H), 5.60-5.48 (m, 2H), 5.43-5.32 (m, 2H), 4.16-4.07 (m, 1H), 3.85-3.76 (m, 1H), 3.63-3.49 (m, 1H), 3.06-2.85 (m, 4H), 2.64-2.54 (m, 4H), 2.42-2.26 (m, 2H), 1.88-1.76 (m, 2H), 0.86 (t, J = 7.3 Hz, 3H) |
| 282 | 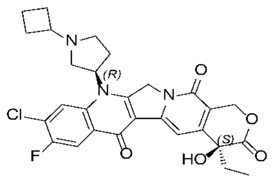 | (400 MHz, DMSO-$d_6$) δ 11.95 (s, 1H), 8.30 (d, *J* = 5.4 Hz, 1H), 8.22 (d, *J* = 9.0 Hz, 1H), 7.30 (s, 1H), 6.41 (s, 1H), 5.66 - 5.47 (m, 3H), 5.37 (s, 2H), 4.21 - 3.94 (m, 2H), 3.89 - 3.74 (m, 1H), 3.71 - 3.52 (m, 1H), 3.23 - 3.14 (m, 1H), 2.85 - 2.72 (m, 1H), 2.69 - 2.60 (m, 1H), 2.39 - 2.27 (m, 3H), 2.24 - 2.19 (m, 1H), 1.88 - 1.78 (m, 4H), 0.85 (t, *J* = 7.3 Hz, 3H)。 |
| 283 | 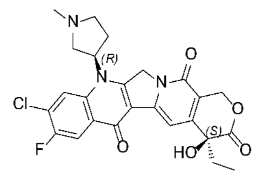 | (400 MHz, DMSO-$d_6$) δ 11.65 (s, 1H), 8.26 (s, 1H), 8.21-8.20 (m, 1H), 7.32-7.28 (m, 1H), 6.41 (s, 1H), 5.57-5.46 (m, 2H), 5.40-5.31 (m, 2H), 4.11-4.07 (m, 1H), 3.83-3.81 (m, 1H), 3.66-3.51 (m, 2H), 3.19-3.17 (m, 1H), 2.99-2.95 (m, 3H), 2.82 -2.79(m, 1H), 2.53-2.49(m, 1H), 1.83-1.78 (m, 2H), 0.86-0.79 (m, 3H)。 |

Example 284: Preparation of (S)-4-ethyl-8-fluoro-4-hydroxy-11-((S)-5-methyl-5-azaspiro[2.4]heptan-7-yl)-1H-pyrano [3,4:6,7]indolizino[2,1-b]quinoline-3,6,14 (4H,11H,12H)-trione:

*Step 1: Preparation of benzyl (S)-7-((tert-butoxycarbonyl)amino)-5-azaspiro[2.4]heptane-5-carboxylate:*

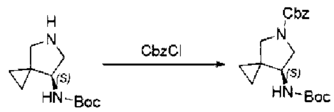


tert-Butyl (S)-(5-azaspiro[2.4]heptan-7-yl)carbamate (4.9 g, 23.1 mmol, 1.0 eq.) was dissolved **in** DCM (50 mL). Under an ice bath, triethylamine (7.01 g, 69.2 mmol, 3.0 eq.) and benzyl chloroformate (5.91 g, 34.6 mmol, 1.5 eq.) were added, and the mixture was slowly warmed to room temperature and stirred for 16 h. LCMS monitoring indicated the reaction was complete. The mixture was poured into ice-cold aqueous sodium bicarbonate solution (300 mL) and extracted with DCM (100 mL × 3). The combined organic phases were dried over anhydrous sodium sulfate, filtered and concentrated, and purified by silica gel column chromatography (eluent: PE:EA= 1:0 to 2:1, v/v) to afford the title compound as a white solid (7.9 g, yield: 98.8%). LCMS (ESI): $[M-56]^+$ = 291.0; $^1$H NMR (400 MHz, $CDCl_3$) δ: 7.40-7.27 (m, 5H), 5.20-5.01 (m, 2H), 3.80-3.63 (m, 2H), 3.57 (dd, J = 11.5, 6.9 Hz, 2H), 3.20 (dd, J = 15.7, 10.8 Hz, 1H), 1.43 (s, 9H), 0.89-0.69 (m, 2H), 0.68-0.50 (m, 2H).

*Step 2: Preparation of benzyl (S)-7-amino-5-azaspiro[2.4]heptane-5-carboxylate:*

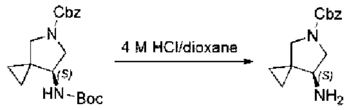


The intermediate obtained from step 1 (7.90 g, 22.8 mmol, 1.0 eq.) was dissolved in DCM (35 mL). Under an ice bath, 4 M hydrogen chloride in 1,4-dioxane (35 mL) was added slowly, the ice bath was then removed, and the mixture was stirred at room temperature for an additional 16 h. LCMS monitoring indicated the reaction was complete. The reaction mixture was concentrated under reduced pressure to afford the title compound as a yellow oily liquid (6.1 g, crude). LCMS (ESI): $[M+H]^+$ = 247.1.

*Step 3: Preparation of benzyl (SE)-7-((4-(2,5-difluorophenyl)-4-oxobut-2-en-2-yl)amino)-5-azaspiro[2.4]heptane-5-carboxylate:*

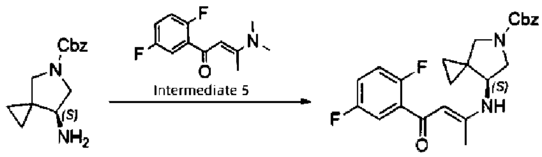


The intermediate obtained from step 2 (6.10 g, 24.8 mmol, 1.0 eq.) and Intermediate 5 (5.58 g, 24.8 mmol, 1.0 eq.) were dissolved **in** acetic acid (50 mL). Under a nitrogen atmosphere, the reaction mixture was heated to 50 °C and stirred for 16 h. LCMS monitoring indicated the reaction was complete. The mixture was poured into ice-cold saturated aqueous sodium bicarbonate solution (300 mL) and the pH was adjusted to 7~8, then extracted with EA (200 mL × 3). The combined organic phases were washed with saturated aqueous sodium chloride solution (300 mL), dried over anhydrous sodium sulfate, filtered and concentrated, and purified by silica gel column chromatography (eluent: PE:EA = 1:0 to 1:1, v/v) to afford the title compound as a yellow solid (2 g, yield: 18.9%). LCMS (ESI): $[M+H]^+$ = 427.0; $^1H$ NMR (400 MHz, $CDCl_3$) δ: 11.54 (dd, J = 30.1, 8.7 Hz, 1H), 7.54-7.45 (m, 1H), 7.44-7.27 (m, 5H), 7.02 (m, 2H), 5.65 (t, J = 2.5 Hz, 1H), 5.22-5.04 (m, 2H), 4.03-3.78 (m, 2H), 3.73-3.51 (m, 2H), 3.43 (m, 1H), 2.04 (d, J = 3.7 Hz, 3H), 0.95 (m, 1H), 0.82-0.59 (m, 3H).

*Step 4: Preparation of benzyl (S)-7-(6-fluoro-2-methyl-4-oxoquinolin-1(4H)-yl)-5-azaspiro[2.4]heptane-5-carboxylate:*

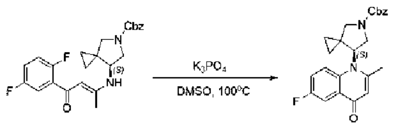


The intermediate obtained from step 3 (2 g, 4.69 mmol, 1.0 eq.) was dissolved **in** dimethyl sulfoxide (30 mL). Potassium phosphate (2.99 g, 14.1 mmol, 3.0 eq.) was added portionwise at room temperature, and after the addition, the mixture was heated to 100 °C and stirred for 16 h under a nitrogen atmosphere. LCMS monitoring indicated the reaction was complete. The mixture was poured into ice water (100 mL) and extracted with ethyl acetate (50 mL × 3). The combined organic phases were washed with saturated aqueous sodium chloride solution (100 mL), dried over anhydrous sodium sulfate, filtered and concentrated, and purified by silica gel column chromatography (eluent: DCM:MeOH = 1:0 to 10:1, v/v) to afford the title compound as a dark gray solid (1.5 g, yield: 78.7%). LCMS (ESI): $[M+H]^+$ = 407.2.

*Step 5: Preparation of benzyl (S)-7-(3-bromo-2-(bromomethyl)-6-fluoro-4-oxoquinolin-1(4H)-yl)-5-azaspiro[2.4]heptane-5-carboxylate:*

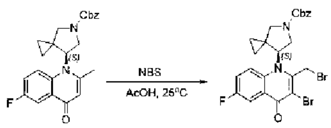

The intermediate obtained from step 4 (1.45 g, 3.57 mmol, 1.0 eq.) was dissolved in acetic acid (30 mL). NBS (1.4 g, 7.85 mmol, 2.2 eq.) was added portionwise, and after the addition, the mixture was stirred for an additional 2 h at room temperature under a nitrogen atmosphere. LCMS monitoring indicated the reaction was complete. The mixture was poured into ice-cold saturated aqueous sodium bicarbonate solution (100 mL) and extracted with ethyl acetate (50 mL × 3). The combined organic phases were washed with saturated aqueous sodium chloride solution (200 mL), dried over anhydrous sodium sulfate, filtered and concentrated under reduced pressure to afford a crude product, which was purified by silica gel column chromatography (eluent: DCM:MeOH = 1:0 to 20:1, v/v) to afford the title compound as a yellow solid (1.54 g, yield: 76.5%). LCMS (ESI): $[M+H]^+$ = 562.8.

*Step 6: Preparation of benzyl (S)-7-(3-bromo-2-(((S)-4-ethyl-4-hydroxy-3,8-dioxo-3,4-dihydro-1H-pyrano[3,4-c]pyridin-7(8H)-yl)methyl)-6-fluoro-4-oxoquinolin-1(4H)-yl)-5-azaspiro[2.4]heptane-5-carboxylate:*

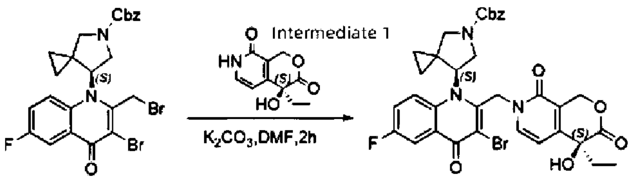


The intermediate obtained from step 5 (1.44 g, 2.55 mmol, 1.0 eq.) was dissolved **in** anhydrous DMF (20 mL). Under an ice bath, potassium carbonate (1.06 g, 7.66 mmol, 3.0 eq.) and Intermediate 1 (0.530 g, 2.55 mmol, 1.0 eq.) were added sequentially, and the mixture was stirred at room temperature for 2 h. LCMS monitoring indicated the reaction was complete. The mixture was poured into ice water (50 mL) and extracted with EA (20 mL × 3). The combined organic phases were washed with saturated brine (50 mL), dried over anhydrous sodium sulfate, filtered and concentrated, and purified by silica gel column chromatography (eluent: DCM:MeOH = 1:0 to 20:1, v/v) to afford the title compound as a yellow solid (890 mg, yield: 50.4%). LCMS (ESI): $[M+H]^+$ = 691.8.

*Step 7: Preparation of benzyl (S)-7-((S)-4-ethyl-8-fluoro-4-hydroxy-3,6,14-trioxo-3,4-dihydro-1H-pyrano[3,4:6,7]indolizino[2,1 -b]quinolin-11(6H,12H,14H)-yl)-5-azaspiro[2.4]heptane-5-carboxylate:*

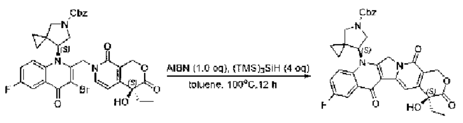


The intermediate obtained from step 6 (690 mg, 1 mmol, 1.0 eq.) was dissolved in toluene (69 mL). Then, AIBN (164 mg, 1 mmol, 1.0 eq.) and tris(trimethylsilyl)silane (991 mg, 3.99 mmol, 4.0 eq.) were added sequentially. After the addition, the mixture was heated to 100 °C and stirred for 12 h under a nitrogen atmosphere. LCMS monitoring indicated the reaction was complete. The reaction mixture was concentrated under reduced pressure to afford a crude product, which was purified by silica gel column chromatography (eluent: DCM:MeOH = 1:0 to 10:1, v/v) to afford the title compound as a brown solid (250 mg, yield: 40.9%). LCMS (ESI): $[M+H]^+$ = 612.2. $^1H$ NMR (400 MHz, DMSO-$d_6$) δ: 8.03 (dd, J = 8.6, 3.3 Hz, 1H), 7.87-7.80 (m, 1H), 7.77-7.68 (m, 1H), 7.51-7.31 (m, 5H), 7.28 (s, 1H), 6.38 (s, 1H), 5.53-5.29 (m, 4H), 5.26-5.10 (m, 3H), 4.34-4.22 (m, 1H), 4.00-3.82 (m, 2H), 3.78-3.64 (m, 1H), 1.86-1.75 (m, 2H), 1.15-1.04 (m, 1H), 0.95-0.82 (m, 4H), 0.82-0.73 (m, 1H), 0.26-0.18 (m, 1H).

*Step 8: Preparation of (S)-4-ethyl-8-fluoro-4-hydroxy-11-((S)-5-azaspiro[2.4]heptan-7-yl)-1,12-dihydro-14H-pyrano[3,4:6,7]indolizino[2,1-b]quinoline-3,6,14(4H,11H)-trione:*

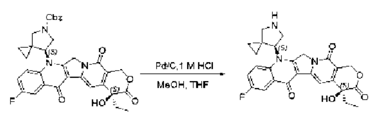

The intermediate obtained from step 7 (250 mg, 0.41 mmol, 1.0 eq.) was dissolved in a mixed solvent of tetrahydrofuran (10 mL) and MeOH (10 mL). Palladium on carbon (30 mg) and 1 M dilute hydrochloric acid (2 mL) were added, and the mixture was stirred for 5 h under a hydrogen atmosphere. LCMS monitoring indicated the reaction was complete. The reaction mixture was filtered through Celite to obtain the filtrate, which was concentrated under reduced pressure to afford the title compound as a pale yellow solid (119 mg as hydrochloride, yield: 60.8%). LCMS (ESI): $[M+H]^+$ = 478.2; $^1H$ NMR (400 MHz, DMSO-$d_6$) $\delta$: 10.01-9.65 (br, 2H), 8.08-7.94 (m, 2H), 7.77-7.66 (m, 1H), 7.30 (s, 1H), 6.39 (s, 1H), 5.57-5.22 (m, 5H), 4.06-3.95 (m, 1H), 3.93-3.80 (m, 1H), 3.79-3.71 (m, 1H), 3.58-3.48 (m, 1H), 1.89-1.75 (m, 1H), 1.21-1.10 (m, 1H), 1.04-0.90 (m, 2H), 0.86 (t, J = 7.3 Hz, 3H), 0.43-0.34 (m, 1H).

*Step 9: Preparation of (S)-4-ethyl-8-fluoro-4-hydroxy-11-((S)-5-methyl-5-azaspiro[2.4]heptan- 7-yl)-1H-pyrano[3,4:6,7] indolizino[2,1-b]quinoline-3,6,14(4H,11H,12H)-trione:*

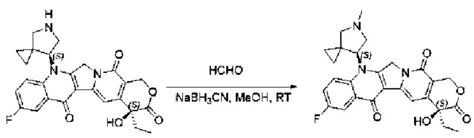


The intermediate obtained from step 8 (30 mg, 0.06 mmol, 1.0 eq.) was dissolved **in** MeOH (5 mL). Under an ice bath, acetic acid (11.3 mg, 0.19 mmol, 3.0 eq.) and 30% w/w aqueous formaldehyde solution (8 mg, 0.13 mmol, 2.0 eq.) were added sequentially. The mixture was continuously stirred for 30 min under an ice bath, then sodium cyanoborohydride (11.65 mg, 0.19 mmol, 3.0 eq.) was added, and the stirring was continued for an additional 20 min under an ice bath. LCMS monitoring indicated the reaction was complete. The reaction was quenched by the slow dropwise addition of 1 M dilute hydrochloric acid (0.5 mL) under an ice bath, and the mixture was lyophilized after the addition of water to afford a crude product. Purification by preparative HPLC afforded the title compound as an off-white solid (4.1 mg as hydrochloride, yield: 13.6%). LCMS (ESI): $[M+H]^+$ = 492.2; $^1H$ NMR (400 MHz, DMSO-$d_6$) $\delta$: 11.01 (s, 1H), 8.11-7.86 (m, 2H), 7.77-7.62 (m, 1H), 7.30 (s, 1H), 6.39 (s, 1H), 5.50-5.19 (m, 5H), 4.33-4.05 (m, 2H), 3.92-3.80 (m, 1H), 3.04 (s, 3H), 2.06-1.94 (m, 1H), 1.91-1.77 (m, 2H), 1.54-1.43 (m, 1H), 1.10-0.93 (m, 2H), 0.86 (t, J = 7.2 Hz, 3H), 0.52-0.37 (m, 1H).

Example 285: Preparation of (S)-4-ethyl-8-fluoro-4-hydroxy-11-((R)-5-methyl-5-azaspiro[2.4]heptan-7-yl)-1H-pyrano [3,4:6,7]indolizino[2,1 -b]quinoline-3,6,14(4H,11H,12H)-trione:

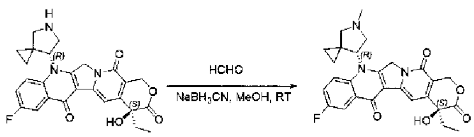


Using commercially available tert-butyl (R)-(5-azaspiro[2.4]heptan-7-yl)carbamate as the starting material, the title compound was synthesized following the synthetic procedures of Steps 1~9 in Example 284, affording the hydrochloride salt as an orange solid. LCMS (ESI): $[M+H]^+$ = 492.3; $^1H$ NMR (400 MHz, DMSO-$d_6$) $\delta$: 10.03 (s, 1H), 8.09-8.00 (m, 1H), 7.79-7.66 (m, 1H), 7.49-7.42 (m, 1H), 7.30 (s, 1H), 6.40 (s, 1H), 5.54-5.22 (m, 4H), 4.28-4.05 (m, 2H), 3.93-3.73 (m, 2H), 3.58 (m, 1H), 3.03 (s, 3H), 1.92-1.75 (m, 2H), 1.33-1.11 (m, 2H), 1.01-0.78 (m, 4H), 0.49-0.44 (m, 1H).

Example 286: Preparation of 4-ethyl-8-fluoro-4-hydroxy-11-(4,5,6,7-tetrahydropyrazolo[1,5-a]pyrazin-3-yl)-1H-pyrano [3,4:6,7]indolizino[2,1 -b] quinoline-3,6,14(4H,11H,12H)-trione:

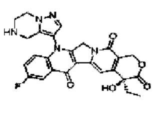

Using commercially available tert-butyl 3-bromo-6,7-dihydropyrazolo[1,5-a]pyrazine-5(4H)-carboxylate as the starting material, the title compound was synthesized following the synthetic procedures of Example 249, affording a

yellow solid. LCMS (ESI): $[M+H]^+$=504.3; $^1H$ NMR (400 MHz, DMSO-$d_6$) δ: 9.99-9.53 (br, 2H), 8.04-7.94 (m, 2H), 7.69-7.64 (m, 1H), 7.38-7.36 (m, 1H), 7.29 (s, 1H), 6.43 (s, 1H), 5.33-5.32 (m, 2H), 4.96-4.94 (m, 2H), 4.60-4.35 (m, 2H), 4.08-4.04 (m, 1H), 3.76-3.73 (m, 1H), 1.81-1.76 (m, 2H), 1.54-1.52 (m, 1H), 1.24-1.23 (m, 1H), 0.91-0.85 (m, 3H).

Example 287: Preparation of 4-ethyl-8-fluoro-4-hydroxy-11-(5-methyl-4,5,6,7-tetrahydropyrazolo[1,5-a]pyrazin-3-yl)-1H-pyrano[3,4:6,7]indolizino[2,1 -b] quinoline-3,6,14(4H,11H,12H)-trione:

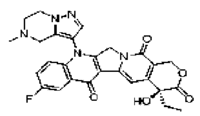

Using the compound from Example 286 as the starting material, the title compound was synthesized following the synthetic procedures of Example 250, affording a yellow solid. LCMS (ESI): $[M+H]^+$ = 518.4; $^1H$ NMR (400 MHz, DMSO-$d_6$) δ: 8.92 (s, 1H), 8.04-7.98 (m, 3H), 7.69-7.64 (m, 1H), 7.30 (s, 1H), 6.44 (s, 1H), 5.33-5.23 (m, 2H), 4.96-4.94 (m, 1H), 4.59-4.56 (m, 2H), 3.77-3.73 (m, 1H), 3.43-3.42 (m, 1H), 2.92-2.85 (m, 3H), 1.85-1.76 (m, 2H), 1.54-1.53 (m, 1H), 1.37-1.24 (m, 2H), 0.88-0.85 (m, 3H).

Example 288: Preparation of tert-butyl (S)-(3-(4-ethyl-8-fluoro-4-hydroxy-3,6,14-trioxo-4,6,12,14-tetrahydro-1H-pyrano [3,4:6,7]indolizino[2,1 -b]quinolin-11(3H) -yl)propyl)(methyl)carbamate:

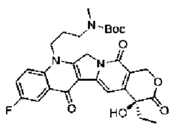

Using (2,5-difluorophenyl)-3-(dimethylamino)but-2-en-1-one (Intermediate 27) and commercially available tert-butyl (3-aminopropyl)(methyl)carbamate as the starting materials, the title compound was synthesized following the procedures of steps 1~7 **in** Example 102, affording a pale pink solid. LCMS (ESI): $[M+H]^+$ = 554.4; $^1H$ NMR (400 MHz, DMSO-$d_6$) δ: 8.63 (s, 1H), 8.17-7.97 (m, 2H), 7.85-7.76 (m, 1H), 7.30 (d, J = 6.5 Hz, 1H), 6.38 (s, 1H), 5.45-5.29 (m, 4H), 4.48 (d, J = 9.4 Hz, 1H), 4.38 (d, J = 5.1 Hz, 1H), 3.17-3.08 (m, 2H), 2.59-2.55 (m, 3H), 2.19-2.10 (m, 1H), 2.09-1.97 (m, 1H), 1.88-1.78 (m, 2H), 1.45-1.21 (m, 9H), 0.93-0.81 (m, 3H).

Example 289: Preparation of (S)-4-ethyl-8-fluoro-4-hydroxy-11-(3-(methylamino)propyl)-1,12-dihydro-14H-pyrano [3,4:6,7]indolizino[2,1 -b] quinoline-3,6,14(4H,11H)-trione:

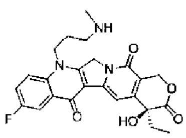

The compound from Example 288 (7 mg) was dissolved in anhydrous DCM (3 mL). Under an ice bath, 4 M hydrogen chloride in 1,4-dioxane (3 mL) was added slowly, and the mixture was stirred at room temperature for 30 min. LCMS monitoring indicated the reaction was complete. The reaction mixture was concentrated to afford the title compound as a pale pink solid (5 mg). LCMS (ESI): $[M+H]^+$ = 454.2; $^1H$ NMR (400 MHz, DMSO-$d_6$) δ: 8.91 (s, 2H), 8.21-8.15 (m, 1H), 8.04-7.97 (m, 1H), 7.84-7.75 (m, 1H), 7.30 (s, 1H), 6.39 (s, 1H), 5.44-5.29 (m, 4H), 4.51 (s, 2H), 3.19-3.06 (m, 2H), 2.58-2.53 (m, 3H), 2.19-2.15 (m, 2H), 1.87-1.78 (m, 2H), 0.92-0.82 (m, 3H).

Example 290: Preparation of (S)-11-(3-(dimethylamino)propyl)-4-ethyl-8-fluoro-4-hydroxy-1,12-dihydro-14H-pyrano [3,4:6,7]indolizino[2,1 -b]quinoline-3,6,14 (4H,11H)-trione:

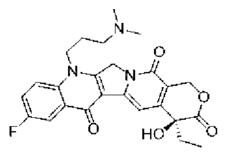

The compound from Example 289 (35 mg, 0.08 mmol, 1.0 eq.) was dissolved in MeOH (8 mL), and 37% w/w aqueous formaldehyde solution (6.95 mg, 0.23 mmol, 3.0 eq.) was added. The mixture was stirred at room temperature for 30 min, then sodium cyanoborohydride (9.54 mg, 0.15 mmol, 2.0 eq.) was added slowly under an ice bath, and stirring was continued for an additional 1 h under an ice bath. LCMS monitoring indicated the reaction was complete. The reaction was quenched by the dropwise addition of 4 M hydrogen chloride **in** 1,4-dioxane (0.1 mL), and the mixture was concentrated to afford a crude product. Purification by reversed-phase preparative HPLC afforded the title compound as a pale pink solid (9 mg, yield: 24.1%). LCMS (ESI): $[M+H]^+$ = 468.2; $^1H$ NMR (400 MHz, DMSO-$d_6$) $\delta$: 10.28 (s, 1H), 8.18 (dd, J = 9.5, 4.2 Hz, 1H), 8.02 (dd, J = 8.9, 3.1 Hz, 1H), 7.85-7.75 (m, 1H), 7.31 (s, 1H), 6.39 (s, 1H), 5.52-5.25 (m, 4H), 4.58-4.42 (m, 2H), 3.33-3.22 (m, 2H), 2.76 (d, J = 4.7 Hz, 6H), 2.30-2.12 (m, 2H), 1.90-1.78 (m, 2H), 0.87 (t, J = 7.3 Hz, 3H).

Example 291: Preparation of (S)-11-cyclopentyl-4-ethyl-8-fluoro-4-hydroxy-9-(hydroxymethyl)-1,12-dihydro-14H-pyrano[3,4:6,7]indolizino[2,1 -b]quinoline-3,6,14(4H,11H)-trione:

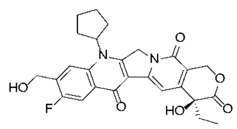

*Step 1: Preparation of 1-cyclopentyl-6-fluoro-7-(hydroxymethyl)-2-methylquinolin-4(1H)-one:*

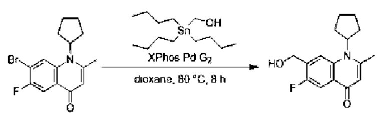


Intermediate 6 (2.3 g, 7.09 mmol, 1.0 eq.) was dissolved in dry 1,4-dioxane (46 mL). Tribromopyridine (4.55 g, 14.18 mmol, 2.0 eq.) was added at room temperature, and the mixture was stirred at 80 °C for 8 h under a nitrogen atmosphere. LCMS monitoring indicated the reaction was complete. The reaction mixture was poured into water (100 mL) and extracted with ethyl acetate (60 mL × 3). The combined organic phases were dried and concentrated under reduced pressure, and the residue was purified by silica gel column chromatography (100-200 mesh silica gel, eluent: DCM:MeOH = 20:1, v/v) to afford the title compound as a yellow solid (1.3 g, yield: 66.6%). LCMS (ESI): $[M+H]^+$ = 276.1, $t_R$ = 1.007 min.

Using the above intermediate 1-cyclopentyl-6-fluoro-7-(hydroxymethyl)-2-methylquinolin-4(1H)-one as the starting material, the title compound was prepared via dibromination, alkylation and cyclization following the synthetic protocol of Example 45 and the procedures of steps 2-5 in Example 8, affording a yellow solid. LCMS (ESI): $[M+H]^+$ = 481.3, $t_R$ = 1.558 min; $^1H$ NMR (400 MHz, DMSO-$d_6$) $\delta$: 7.96 (s, 1H), 7.94 (s, 1H), 7.28 (s, 1H), 5.51 (s, 2H), 5.37-5.29 (m, 2H), 5.05-4.96 (m, 1H), 4.76 (s, 2H), 2.36-2.18 (m, 4H), 2.17-2.05 (m, 2H), 1.88-1.74 (m, 4H), 0.85 (t, J = 7.3 Hz, 3H).

Example 292: Preparation of (4S)-11-cyclopentyl-4-ethyl-8-fluoro-4-hydroxy-9-(2-hydroxypropyl)-1,12-dihydro-14H-pyrano[3,4:6,7]indolizino[2,1 -b]quinoline -3,6,14(4H,11H)-trione:

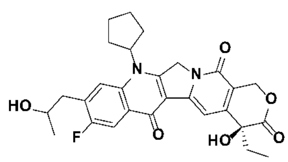

*Step 1: Preparation of 1-cyclopentyl-6-fluoro-2-methyl-7-(2-oxopropyl)quinolin-4(1H)-one:*

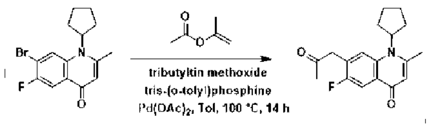


Intermediate 6 (3.0 g, 9.25 mmol, 1.0 eq.) was dissolved **in** toluene (10 mL). Isopropenyl acetate (1.85 g, 18.5 mmol, 2.0 eq.), tributylmethoxytin (5.94 g, 18.5 mmol, 2.0 eq.), tris(o-tolyl)phosphine (281.54 mg, 0.93 mmol, 0.1 eq.) and palladium acetate (103.8 mg, 0.46 mmol, 0.05 eq.) were added sequentially, and the mixture was stirred at 100 °C for 14 h. LCMS monitoring was performed for the reaction progress. The reaction mixture was cooled to room temperature, poured into ice water (50 mL) and extracted with ethyl acetate (50 mL × 3). The combined organic phases were washed with water (50 mL × 2) and saturated brine (50 mL × 2), then dried over anhydrous sodium sulfate, filtered and concentrated under reduced pressure. The residue was purified by column chromatography (200-300 mesh silica gel, eluent: THF:petroleum ether = 1:1, v/v) to afford the title compound as a yellow solid (920 mg, yield: 33.0%). LCMS (ESI): $[M+H]^+ = 302.2$, $t_R = 0.99$ min.

*Step 2: Preparation of 1-cyclopentyl-6-fluoro-7-(2-hydroxypropyl)-2-methylquinolin-4(1H)-one:*

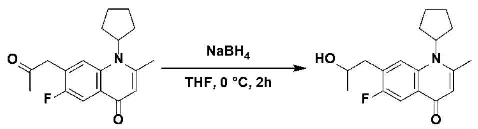


The intermediate obtained from Step 1 (940 mg, 3.12 mmol, 1.0 eq.) was dissolved **in** tetrahydrofuran (20 mL) at room temperature. Sodium borohydride (177.0 mg, 4.68 mmol, 2.0 eq.) was added portionwise at 0 °C, and the mixture was stirred at 0 °C for 2 h with LCMS monitoring for the reaction progress. Upon completion, the reaction mixture was poured into ice water (10 mL) at 0~10 °C and extracted with ethyl acetate (20 mL × 2). The combined organic phases were washed with water (10 mL) and saturated brine (10 mL), dried over anhydrous sodium sulfate, filtered and concentrated under reduced pressure. The residue was purified by column chromatography (200-300 mesh silica gel, eluent: THF:petroleum ether = 1:1, v/v) to afford the title compound as a yellow solid (550 mg, yield: 58.1%). LCMS (ESI): $[M+H]^+ = 304.2$, $t_R = 0.953$ min.

Using the above intermediate 1-cyclopentyl-6-fluoro-7-(2-hydroxypropyl)-2-methylquinolin-4(1H)-one as the starting material, the title compound was prepared via dibromination, alkylation and cyclization following the synthetic protocol of Example 45 and the procedures of Steps 2-5 in Example 8, affording a yellow solid. LCMS (ESI): $[M+H]^+ = 509.4$, $t_R = 1.638$ min; $^1H$ NMR (400 MHz, DMSO-$d_6$ + $D_2O$) δ: 7.60 (dd, J = 2.4, 9.6 Hz, 1H), 7.92 (d, J = 5.2 Hz, 1H), 7.26 (s, 1H), 5.40 (s, 2H), 5.29 (dd, J = 15.6, 34.8 Hz, 2H), 4.94-4.82 (m, 1H), 4.44 (q, J = 7.2 Hz, 0.3H), 3.73 (q, J = 7.2 Hz, 1H), 2.90-2.76 (m, 2H), 2.34-2.16 (m, 2H), 2.12-1.98 (m, 2H), 1.86-1.70 (m, 2H), 1.51 (d, J = 7.2 Hz, 1H), 1.12 (d, J = 4.4 Hz, 3H), 0.82 (t, J = 7.2 Hz, 3H).

Example 293: Preparation of (S)-11-cyclopentyl-4-ethyl-8-fluoro-4-hydroxy-9-((S)-1-hydroxyethyl)-1,12-dihydro-14H-pyrano[3,4:6,7]indolizino[2,1 -b]quinoline-3,6,14(4H,11H)-trione (Isomer 1 or Isomer 2)

Example 294: Preparation of (S)-11-cyclopentyl-4-ethyl-8-fluoro-4-hydroxy-9-((R)-1-hydroxyethyl)-1,12-dihydro-14H-pyrano[3,4:6,7]indolizino[2,1 -b]quinoline-3,6,14(4H,11H)-trione (Isomer 2 or Isomer 1)

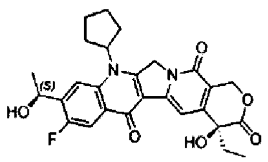

Isomer 1 or Isomer 2

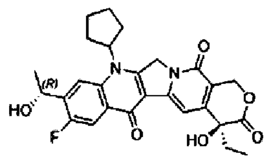

Isomer 2 or Isomer 1

*Step 1: Preparation of 1-cyclopentyl-6-fluoro-2-methyl-7-vinylquinolin-4(1H)-one:*

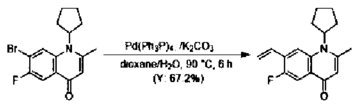


Intermediate 6 (8.0 g, 24.68 mmol, 1.0 eq.) was added to a mixture of 1,4-dioxane (50 mL) and water (5 mL), followed by vinyl boronate (7.6 g, 49.35 mmol, 2.0 eq.), tetrakis(triphenylphosphine)palladium (1.43 g, 1.23 mmol, 0.05 eq.) and potassium carbonate (10.23 g, 74.03 mmol, 3.0 eq.). The mixture was stirred at 90 °C for 6 h under a nitrogen atmosphere, and LCMS monitoring indicated the reaction was complete. The reaction mixture was poured into water (300 mL) and extracted with ethyl acetate (200 mL × 3). The combined organic phases were dried and concentrated, and purified by flash column chromatography (silica gel, eluent: petroleum ether/ethyl acetate = 5:1 to 3:1, v/v) to afford the title compound as a yellow solid (4.5 g, yield: 67.2%). LCMS (ESI): $[M+H]^+$ = 272.2, $t_R$ = 1.227 min.

*Step 2: Preparation of 1-cyclopentyl-6-fluoro-2-methyl-4-oxo-1,4-dihydro quinoline-7-carbaldehyde:*

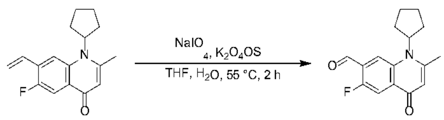


The compound obtained from step 1 (3.5 g, 12.9 mmol, 1.0 eq.) was dissolved in a mixture of tetrahydrofuran (30 mL) and water (10 mL), followed by potassium osmate (0.58 g, 1.29 mmol, 0.1 eq.) and sodium periodate (8.28 g, 38.7 mmol, 3.0 eq.). The mixture was stirred at 55 °C for 2 h, and LCMS monitoring indicated the reaction was complete. Saturated aqueous ammonium chloride solution (200 mL) was added, and the mixture was extracted with ethyl acetate (100 mL × 3). The combined organic phases were dried and concentrated under reduced pressure. The crude product was purified by flash column chromatography (silica gel, eluent: petroleum ether/ethyl acetate = 0:1, v/v) to afford the title compound as a yellow solid (3.0 g, yield: 85.09%). LCMS (ESI): $[M+H]^+$ = 274.1, $t_R$ = 1.150 min.

*Step 3: Preparation of 1-cyclopentyl-6-fluoro-7-(1-hydroxyethyl)-2-methyl quinolin-4(1H)-one:*

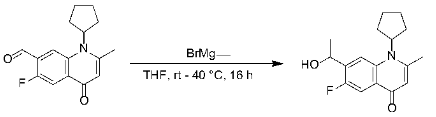


The intermediate obtained from step 2 (2.0 g, 7.32 mmol, 1.0 eq.) was dissolved in tetrahydrofuran (20 mL). Methylmagnesium bromide (29.27 mL, 1 mol/L in THF, 4.0 eq.) was added at -78 °C, and the mixture was stirred at 40 °C for 16 h. LCMS monitoring indicated the reaction was complete. Saturated aqueous ammonium chloride solution (200 mL) was added, and the mixture was extracted with ethyl acetate (100 mL × 3). The combined organic phases were dried and concentrated under reduced pressure. The crude product was purified by flash column chromatography (silica gel, eluent: petroleum ether/ethyl acetate = 5:1 to 3:1, v/v) to afford the title compound as a yellow solid (1.3 g, yield: 61.38%). LCMS (ESI): $[M+H]^+$ = 290.2, $t_R$ = 1.057 min.

Using the above intermediate 1-cyclopentyl-6-fluoro-7-(1-hydroxyethyl)-2-methylquinolin-4(1H)-one as the starting material, the title compounds were prepared via dibromination, alkylation and cyclization following the synthetic protocol of Example 45 and the procedures of Steps 2-5 in Example 8, both affording yellow solids.

**Isomer 1 or Isomer 2:** LCMS (ESI): $[M+H]^+$ = 495.2, $t_R$ = 1.964 min; $^1H$ NMR (400 MHz, DMSO-$d_6$) δ: 9.15 (s, 1H), 8.14 (s, 1H), 8.00 (d, J = 6.0 Hz, 1H), 7.86 (d, J = 10.4 Hz, 1H), 7.26 (s, 1H), 5.47 (s, 2H), 5.33 (d, J = 6.3 Hz, 2H), 5.13-5.05 (m, 1H), 5.03-4.96 (m, 1H), 2.36-2.10 (m, 5H), 1.82-1.75 (m, 4H), 1.55 (d, J = 7.1 Hz, 1H), 1.42 (d, J = 6.3 Hz, 3H), 0.85 (t, J = 7.3 Hz, 3H).

**Isomer 2 or Isomer 1:** LCMS (ESI): $[M+H]^+$ = 495.2, $t_R$ = 1.644 min; $^1H$ NMR (400 MHz, DMSO-$d_6$) δ: 8.98 (s, 1H), 8.01 (d, J = 5.8 Hz, 1H), 7.94 (d, J = 10.4 Hz, 1H), 7.28 (s, 1H), 6.37 (s, 1H), 5.51 (s, 2H), 5.39-5.30 (m, 2H), 5.12 (q, J = 6.3

Hz, 1H), 5.06-4.97 (m, 1H), 2.32-2.07 (m, 5H), 1.87-1.73 (m, 4H), 1.54 (d, J = 6.9 Hz, 1H), 1.45-1.41 (m, 3H), 0.86 (t, J = 7.3 Hz, 3H).

Example 295: Preparation of (4S)-4-ethyl-8-fluoro-11-(3-fluoro-5-methoxyphenyl) -4-hydroxy-9-(1-hydroxyethyl)-1,12-dihydro-14H-pyrano[3,4:6,7]indolizino[2,1 -b]quinoline-3,6,14(4H,11H)-trione:

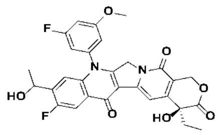

Similarly, using (Z)-1-(4-bromo-2,5-difluorophenyl)-3-(dimethylamino)but-2-en-1-one (Intermediate from Preparation Example 4) and 3-fluoro-5-methoxyaniline as the starting materials, 7-(1-ethoxyvinyl)-6-fluoro-1-(3-fluoro-5-methoxyphenyl)-2-methylquinolin-4(1H)-one was obtained following the synthetic method of Preparation Example 6. Using this intermediate, the racemate of the title compound was synthesized following the synthetic methods of Example 293 and Example 294, affording a white solid. LCMS (ESI): $[M+H]^+$ = 551.1, t_R = 0.914 min; $^1$H NMR (400 MHz, DMSO-$d_6$) δ: 7.94 (d, J = 10.0 Hz, 1H), 7.35-7.18 (m, 5H), 6.41 (s, 1H), 5.61-5.53 (m, 1H), 5.34-5.26 (m, 2H), 5.05-4.95 (m, 1H), 4.92-4.83 (m, 1H), 3.90-3.84 (m, 3H), 1.87-1.75 (m, 2H), 1.34-1.28 (m, 3H), 0.86 (t, J = 7.2 Hz, 3H).

Example 296: Preparation of (S)-11-(1-cyclopropyl-1H-pyrazol-4-yl)-4-ethyl-8-fluoro-4-hydroxy-9-(2-hydroxyethyl)-1,12-dihydro-14H-pyrano[3,4:6,7] indolizino[2,1-b]quinoline-3,6,14(4H,11H)-trione:

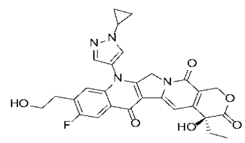

Similarly, using (Z)-1-(4-bromo-2,5-difluorophenyl)-3-(dimethylamino)but-2-en-1-one (Intermediate from Preparation Example 4) and 4-amino-1-cyclopropylpyrazole as the starting materials, 7-bromo-1-(1-cyclopropyl-1H-pyrazol-4-yl)-6-fluoro-2-methylquinolin-4(1H)-one was obtained following the synthetic method of Preparation Example 6, affording a light brown solid. LCMS (ESI): $[M+H]^+$=362.0, $t_R$=1.200 min.

7-Bromo-1-(1-cyclopropyl-1H-pyrazol-4-yl)-6-fluoro-2-methylquinolin-4(1H)-one (7.5 g, 20.71 mmol, 1.0 eq.) and 2-[(1E)-2-ethoxyvinyl]-4,4,5,5-tetramethyl-1,3,2-dioxaborolane (6.1 g, 31.06 mmol, 1.5 eq.) were dissolved in a mixture of 1,4-dioxane (60 mL) and water (20 mL). Potassium carbonate (8.5 g, 62.12 mmol, 3.0 eq.) and [1,1 -bis(diphenylphosphino)ferrocene]dichloropalladium(II) DCM complex (1.5 g, 2.07 mmol, 0.1 eq.) were added, and the mixture was heated to 90 °C for 3 h under a nitrogen atmosphere. LCMS monitoring indicated the reaction was complete. After cooling to room temperature, the reaction mixture was diluted with water (50 mL) and extracted with ethyl acetate (20mL×3). The combined organic phases were dried and concentrated under reduced pressure, and the residue was purified by silica gel column chromatography (100-200 mesh silica gel, eluent: petroleum ether:ethyl acetate = 3:1 to 0:1, v/v) to afford (E)-1-(1-cyclopropyl-1H-pyrazol-4-yl)-7-(2-ethoxyvinyl)-6-fluoro-2-methylquinolin-4(1H)-one as a yellow solid (6.4 g, yield: 87.45%). LCMS (ESI): $[M+H]^+$ = 354.2, $t_R$ =1.230 min. (E)-1-(1-cyclopropyl-1H-pyrazol-4-yl)-7-(2-ethoxyvinyl)-6-fluoro-2-methylquinolin-4(1H)-one (6400 mg, 18.11 mmol, 1.0 eq.) was dissolved in DCM (60 mL), and hydrogen chloride in 1,4-dioxane (6.6 mL) was added dropwise.

The mixture was stirred at room temperature for 5 h, and LCMS monitoring indicated the reaction was complete. The reaction mixture was concentrated under reduced pressure, and the residue was purified by silica gel column chromatography (100-200 mesh silica gel, eluent: DCM:MeOH = 10:1, v/v) to afford 2-(1-(1-cyclopropyl-1H-pyrazol-4-yl)-6-fluoro-2-methyl-4-oxo-1,4-dihydroquinolin-7-yl)acetaldehyde as a yellow solid (1100 mg, yield: 18.67%). LCMS (ESI): $[M+H]^+$ = 326.2, $t_R$ = 1.030 min.

2-(1-(1-cyclopropyl-1H-pyrazol-4-yl)-6-fluoro-2-methyl-4-oxo-1,4-dihydroquinolin-7-yl)acetaldehyde (1100 mg, 3.38 mmol, 1.0 eq.) was dissolved in ethanol (20 mL). Sodium borohydride (383.72 mg, 10.14 mmol, 3.0 eq.) was added under an ice bath, and the mixture was stirred for 10 min under an ice bath. LCMS monitoring indicated the reaction was complete. The reaction was quenched by the addition of water, and the mixture was extracted with ethyl acetate. The combined organic phases were dried and concentrated under reduced pressure, and the residue was purified by silica gel

column chromatography (100-200 mesh silica gel, eluent: DCM:MeOH = 10:1, v/v) to afford 1-(1-cyclopropyl-1H-pyrazol-4-yl)-6-fluoro-7-(2-hydroxyethyl)-2-methylquinolin-4(1H)-one as a yellow solid (1106.8 mg). LCMS (ESI): $[M+H]^+$=328.2, $t_R$=1.023 min.

Using 1-(1-cyclopropyl-1H-pyrazol-4-yl)-6-fluoro-7-(2-hydroxyethyl)-2-methyl quinolin-4(1H)-one as the starting material, the title compound was prepared via dibromination, alkylation and cyclization following the synthetic protocol of Example 45 and the procedures of steps 2-5 in Example 8, affording a yellow solid. LCMS (ESI): $[M+H]^+$ = 533.3, t_R = 1.302 min.

Preparation of Compounds in Examples 297-346:

Similarly, the compounds in Examples 297-346 were synthesized from commercially available reagents following the synthetic methods of Examples 291-296. The chemical structures and detailed characterization data (LCMS and $^1$H NMR) of the compounds are shown below:

| Examples | Strucures | $^1$H NMR | LCMS (ESI) $[M+H]^+$ |
|---|---|---|---|
| 297 | 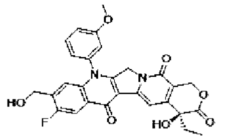 | (400 MHz, DMSO-$d_6$) δ 7.92 (dd, *J* = 10.0, 2.0 Hz, 1H), 7.73 - 7.66 (m, 1H), 7.38 - 7.22 (m, 4H), 7.22 - 7.17 (m, 1H), 6.37 (d, *J* = 4.3 Hz, 1H), 5.50 (t, *J* = 5.6 Hz, 1H), 5.30 (s, 2H), 4.93 - 4.70 (m, 2H), 4.62 (d, *J* = 5.4 Hz, 2H), 3.86 (d, *J* = 6.8 Hz, 3H), 1.87 - 1.76 (m, 2H), 0.85 (t, *J* = 7.3 Hz, 3H)。 | 519.1, $t_R$=1.31min ; |
| 298 | 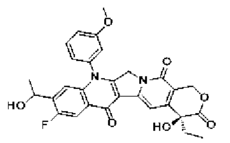 | (400 MHz, DMSO-$d_6$) δ 7.95 (d, *J* = 10.3 Hz, 1H), 7.75 - 7.66 (m, 1H), 7.41 - 7.18 (m, 5H), 6.41 (d, *J* = 1.4 Hz, 1H), 5.56 - 5.51 (m, 1H), 5.31 (s, 2H), 5.04 - 4.95 (m, 1H), 4.93 - 4.70 (m, 2H), 3.90 - 3.83 (m, 3H), 1.86 - 1.75 (m, 2H), 1.30 (dd, *J* = 6.3, 2.2 Hz, 3H), 0.86 (t, *J* = 7.3 Hz, 3H)。 | 533.2, $t_R$=0.766min |
| 299 | 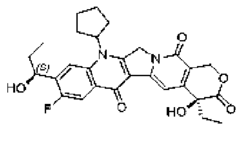 Isomer 1 or Isomer 2 | (400 MHz, DMSO-$d_6$ +$D_2O$) δ 7.95 (d, *J* = 5.2 Hz, 1H), 7.88 (d, *J* = 10.4 Hz, 1H), 7.31 (s, 1H), 5.56 (s, 2H), 5.29 (dd, *J* = 15.6, 31.2 Hz, 2H), 5.06-4.88 (m, 2H), 2.38-2.06 (m, 6H), 1.94-1.70 (m, 5H), 1.70-1.56 (m, 1H), 1.0-0.78 (m, 6H)。 | 509.4, $t_R$=1.698min; |
| 300 | 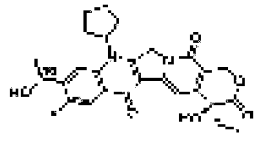 Isomer 2 or Isomer 1 | (400 MHz, DMSO-$d_6$ +$D_2O$) δ 7.95 (d, *J* = 5.6 Hz, 1H), 7.93 (d, *J* = 10.4 Hz, 1H), 7.28 (s, 1H), 5.52 (s, 2H), 5.29 (dd, *J* = 16.0, 28.4 Hz, 2H), 5.02-4.92 (m, 2H), 3.80-3.72 (m, 1H), 2.36-2.04 (m, 6H), 1.86-1.76 (m, 5H), 1.66-1.58 (m, 1H), 0.90-0.80 (m, 6H)。 | 509.4, $t_R$=1.755min; |
| 301 | 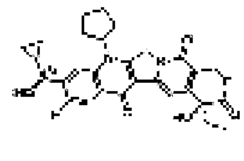 Isomer 1 or Isomer 2 | (400 MHz, DMSO-$d_6$ +$D_2O$) δ 7.98 (d, *J* = 6.0 Hz, 1H), 7.93 (d, *J* = 10.0 Hz, 1H), 7.29 (s, 1H), 5.48 (s, 2H), 5.29 (dd, *J* = 15.6, 29.6 Hz, 2H), 5.02-4.90 (m, 1H), 4.65 (d, *J* = 6.4 Hz, 1H), 2.38-2.04 (m, 6H), 1.86-1.76 (m, 4H), 1.16-1.06 (m, 1H), 0.88-0.80 (m, 3H), 0.48-0.36 (m, 4H)。 | 521.4, $t_R$=1.693min |
| 302 | 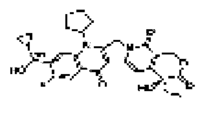 Isomer 2 or Isomer 1 | (400 MHz, DMSO-$d_6$ +$D_2O$) δ 8.00-7.92 (m, 2H), 7.29 (s, 1H), 5.50 (s, 2H), 5.34 (dd, *J* = 16.0, 26.0 Hz, 2H), 5.04-4.96 (m, 1H), 4.65 (d, *J* = 6.0 Hz, 1H), 2.36-2.04 (m, 6H), 1.88-1.74 (m, 4H), 1.14-1.06 (m, 1H), 0.88-0.80 (m, 3H), 0.50-0.36 (m, 4H)。 | 521.4, $t_R$=1.741mi n |

(continued)

| Examples | Strucures | $^1$H NMR | LCMS (ESI) $[M+H]^+$ |
|---|---|---|---|
| 303 | 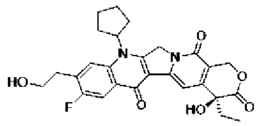 | (400 MHz, DMSO-$d_6$) δ 7.97 (d, *J* = 9.9 Hz, 1H), 7.76 (d, *J* = 5.8 Hz, 1H), 7.29 (s, 1H), 6.35 (s, 1H), 5.52 (s, 2H), 5.41 - 5.28 (m, 2H), 5.05 - 4.95 (m, 1H), 4.94 - 4.81 (m, 1H), 3.74 (t, *J* = 6.0 Hz, 2H), 2.97 (t, *J* = 6.0 Hz, 2H), 2.35 - 2.21 (m, 4H), 2.15 - 2.01 (m, 2H), 1.87 - 1.75 (m, 4H), 0.86 (t, *J* = 7.2 Hz, 3H)。 | 495.3, $t_R$=1.405 min |
| 304 | 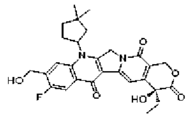 | (400 MHz, DMSO-$d_6$) δ 8.01 (d, *J* = 5.7 Hz, 1H), 7.96 (d, *J* = 10.1 Hz, 1H), 7.30 (s, 1H), 5.73 - 5.63 (m, 1H), 5.58 - 5.46 (m, 1H), 5.43 - 5.30 (m, 2H), 5.17 - 5.05 (m, 1H), 4.84 - 4.72 (m, 2H), 2.64 - 2.53 (m, 1H), 2.35 - 2.18 (m, 3H), 2.12 - 2.02 (m, 1H), 1.89 - 1.78 (m, 2H), 1.74 - 1.65 (m, 1H), 1.25 (s, 3H), 1.15 (s, 3H), 0.89 - 0.83 (m, 3H)。 | 509.3, $t_R$=1.536 min |
| 305 | 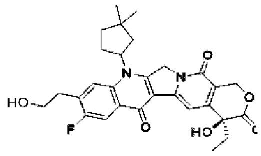 | (400 MHz, DMSO-$d_6$) δ 7.98 (d, *J* = 9.8 Hz, 1H), 7.74 (d, *J* = 5.3 Hz, 1H), 7.30 (s, 1H), 6.36 (s, 1H), 5.71 - 5.63 (m, 1H), 5.57 - 5.47 (m, 1H), 5.37 - 5.29 (m, 2H), 5.14 - 5.04 (m, 1H), 4.97 - 4.84 (m, 1H), 3.80 - 3.70 (m, 2H), 2.99 - 2.93 (m, 2H), 2.40 - 2.29 (m, 1H), 2.29 - 2.20 (m, 1H), 2.16 - 1.96 (m, 3H), 1.88 - 1.77 (m, 2H), 1.75 - 1.68 (m, 1H), 1.25 (s, 3H), 1.15 (s, 3H), 0.86 (t, *J* = 7.1 Hz, 3H)。 | 523.3, $t_R$=1.44 min |
| 306 | 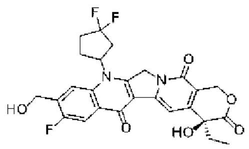 | (400 MHz, DMSO-$d_6$+$D_2O$) δ 7.70 (d, *J* = 10.0 Hz, 1H), 7.25 (d, *J* = 5.2 Hz, 1H), 7.29 (s, 1H), 5.54 (s, 2H), 5.38 (dd, *J* = 16.0 Hz, *J* = 29.2 Hz, 2H), 5.22 (t, *J* = 4.8 Hz, 1H), 4.73 (s, 2H), 4.86 (dd, *J* = 7.2 Hz, *J* = 13.6 Hz, 1H), 3.02-2.84 (m, 2H), 1.86-1.76 (m, 2H), 1.08 (d, *J* = 6.8 Hz, 2H), 0.84 (d, *J* = 7.2 Hz, 3H)。 | 517.3, $t_R$=1.559min |
| 307 | 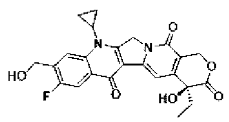 | (400 MHz, DMSO-$d_6$) δ 8.37-8.27 (m, 1H), 7.90-7.80 (m, 1H), 7.25 (s, 1H), 6.32 (s, 1H), 5.76-5.66 (m, 1H), 5.42 (s, 1H), 5.35-5.27 (m, 2H), 4.79-4.67 (m, 2H), 3.66-3.54 (m, 1H), 1.83-1.73 (m, 2H), 1.44-1.39 (m, 1H), 1.24-1.16 (m, 4H), 0.82 (t, *J* = 7.1 Hz, 3H)。 | 453.3, $t_R$=1.371min |
| 308 | 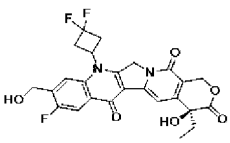 | (400 MHz, DMSO-$d_6$) δ 7.95-7.91 (m, 2H), 7.31 (s, 1H), 6.34 (s, 1H), 5.64 (t, *J* = 5.6 Hz, 1H), 5.47 (s, 2H), 5.38-5.28 (m, 2H), 5.24-5.15 (m, 1H), 4.74 (d, *J* = 4.9 Hz, 2H), 3.62-3.42 (m, 4H), 1.88-1.74 (m, 2H), 0.84 (t, *J* = 7.4 Hz, 3H)。 | 503.3, $t_R$=1.483min |
| 309 | 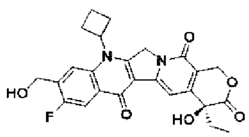 | (400 MHz, DMSO-$d_6$ + $D_2O$) δ 8.10 (d, *J* = 5.6 Hz, 1H), 7.91 (d, *J* = 10.0 Hz, 1H), 7.32 (s, 1H), 5.43 (s, 2H), 5.32 (dd, *J* = 27.2, 16.0 Hz, 2H), 5.22-5.10 (m, 1H), 4.75 (s, 2H), 2.82-2.68 (m, 4H), 1.98-1.86 (m, 2H), 1.84-1.74 (m, 2H), 0.84 (t, *J* = 7.2 Hz, 3H)。 | 467.3, $t_R$= 1.468min |
| 310 | 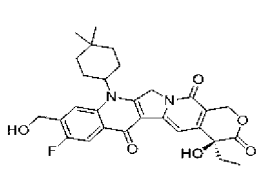 | (400 MHz, DMSO-$d_6$) δ 8.37 (d, *J* = 5.9 Hz, 1H), 7.97 - 7.91 (m, 1H), 7.31 (s, 1H), 6.34 (s, 1H), 5.80 (d, *J* = 5.1 Hz, 1H), 5.53 (s, 1H), 5.34 (dd, *J* = 14.4, 5.8 Hz, 3H), 4.78 (d, *J* = 4.9 Hz, 2H), 4.74 (d, *J* = 5.1 Hz, 1H), 2.03 - 1.90 (m, 4H), 1.87 - 1.77 (m, 4H), 1.55 (d, *J* = 11.8 Hz, 4H), 1.47 (d, *J* = 6.8 Hz, 2H), 1.16 (s, 3H), 1.02 (s, 3H), 0.89 - 0.78 (m, 3H)。 | 523.1, $t_R$=1.542min |

(continued)

| Examples | Strucures | $^1$H NMR | LCMS (ESI) $[M+H]^+$ |
|---|---|---|---|
| 311 | 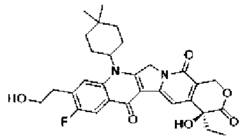 | (400 MHz, DMSO-*d6*) δ 8.14-8.12 (m, 1H), 7.96-7.94 (m, 1H), 7.30 (s, 1H), 6.34 (s, 1H), 5.60-5.57 (m, 2H), 5.40-5.35 (m, 2H), 4.92 (t, J = 4.8 Hz, 1H), 4.27-4.18 (m, 1H), 3.82-3.71 (m, 2H), 3.03-2.95 (m, 2H), 2.67-2.60 (m, 2H), 1.97-1.77 (m, 4H), 1.68-1.54 (m, 4H), 1.13 (d, J = 19.2 Hz, 3H), 1.02 (s, 3H), 0.86 (t, J = 7.3 Hz, 3H)。 | 537.5, $t_R$=1.792min |
| 312 | 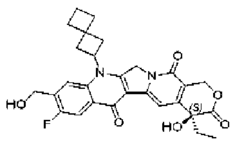 | (400 MHz, DMSO-$d_6$) δ 8.05 (d, *J* = 5.7 Hz, 1H), 7.92 (d, *J* = 9.9 Hz, 1H), 7.31 (s, 1H), 5.44 - 5.30 (m, 4H), 5.03 (p, *J* = 8.8 Hz, 1H), 4.76 (s, 2H), 2.88 (t, *J* = 9.4 Hz, 2H), 2.76 (t, *J* = 10.4 Hz, 2H), 2.24 (t, *J* = 7.3 Hz, 2H), 2.09 (t, *J* = 7.3 Hz, 2H), 1.90 (t, 2H), 1.85 - 1.76 (m, 2H), 0.85 (t, *J* = 7.2 Hz, 3H)。 | 507.1, $t_R$=1.496min |
| 313 | 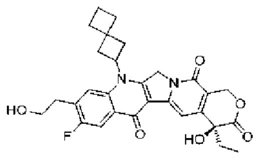 | (400 MHz,DMSO-$d_6$) δ 8.14-8.12 (m, 1H), 7.96-7.94 (m, 1H), 7.30 (s, 1H), 6.34 (s, 1H), 5.60-5.57 (m, 2H), 5.40-5.35 (m, 2H), 4.92 (t, J = 4.8 Hz, 1H), 4.27-4.18 (m, 1H), 3.82-3.71 (m, 2H), 3.03-2.95 (m, 2H), 2.67-2.60 (m, 2H), 1.97-1.77 (m, 4H), 1.68-1.54 (m, 4H), 1.13 (d, J = 19.2 Hz, 3H), 1.02 (s, 3H), 0.86 (t, J = 7.3 Hz, 3H)。 | 521.3, $t_R$=1.529min |
| 314 | 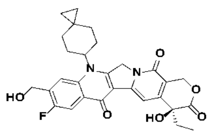 | N/A | 521.2, $t_R$ =1.712min |
| 315 | 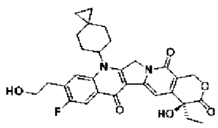 | (400 MHz, DMSO-$d_6$) δ 8.22 (d, *J* = 5.2 Hz, 1H), 7.96 (d, *J* = 9.6 Hz, 1H), 7.30 (s, 1H), 6.33 (s, 1H), 5.61 (s, 2H), 5.41-5.29 (m, 2H),4.89 (s, 1H), 4.49-4.30 (m, 1H), 3.88-3.69 (m, 2H), 3.05-2.92 (m, 2H), 2.72-2.60 (m, 2H), 2.22-2.02 (m, 4H), 1.88-1.76 (m, 2H), 1.10-0.98 (m, 2H), 0.85 (t, *J* = 7.2 Hz, 3H), 0.45 (s, 4H)。 | 535.2, $t_R$ =1.741min |
| 316 | 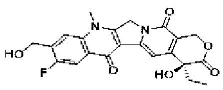 | (400 MHz, DMSO-$d_6$) δ 7.99 (d, *J* = 5.7 Hz, 1H), 7.92 (d, *J* = 10.0 Hz, 1H), 7.27 (s, 1H), 6.36 (s, 1H), 5.67 (t, *J* = 5.6 Hz, 1H), 5.40 (s, 2H), 5.35 (d, *J* = 4.5 Hz, 2H), 4.76 (d, *J* = 5.5 Hz, 2H), 3.93 (s, 3H), 1.82 (m, *J* = 10.9, 6.8 Hz, 2H), 0.87 (t, *J* = 7.3 Hz, 3H)。 | 427.1, $t_R$=0.356min |
| 317 | 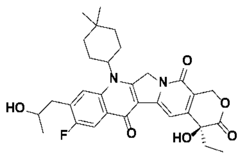 | (400 MHz, DMSO-$d_6$+$D_2O$) δ 8.15-8.05 (m, 1H), 7.34 (d, J = 9.6 Hz, 1H), 7.30 (s, 1H), 6.33 (s, 1H), 5.22 (s, 2H), 5.32 (dd, J = 5.6, 21.2 Hz, 2H), 4.89 (s, 1H), 4.28-4.15 (m, 1H), 4.02-3.92 (m, 1H), 2.92-2.80 (m, 2H), 2.70-2.50 (m, 2H), 2.00-1.48 (m, 8H), 1.15-1.05 (m, 6H), 1.01 (s, 3H), 0.85 (t, J = 7.2 Hz, 3H) | 551.4, $t_R$=1.876min |
| 318 | 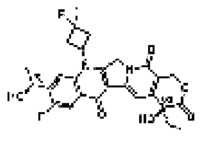 Isomer 1 or Isomer 2 | (400 MHz, DMSO-$d_6$+$D_2O$) δ 7.94 (s, 1H), 7.91 (s, 1H), 7.31 (s, 1H), 5.45 (s, 2H), 5.40-5.25 (m, 2H), 5.20-5.13 (m, 1H), 5.12-5.03 (m, 1H), 4.30-4.73 (m, 1H), 1.86-1.75 (m, 2H), 1.52 (d, *J* = 6.8 Hz, 2H), 1.42 (d, *J* = 6.0 Hz, 3H), 1.34 (d, *J* = 7.2 Hz, 2H), 0.90-0.81 (m, 3H)。 | 517.3, $t_R$ =1.549 min |

(continued)

| Examples | Strucures | $^1$H NMR | LCMS (ESI) $[M+H]^+$ |
|---|---|---|---|
| 319 | 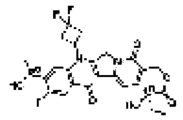 Isomer 2 or Isomer 1 | (400 MHz, DMSO-$d_6$+$D_2O$) δ 7.96 (s, 1H), 7.93 (s, 1H), 7.32 (s, 1H), 5.46 (s, 2H), 5.39-5.35 (m, 2H), 5.32-5.28 (m, 1H), 5.11-5.10 (m, 1H), 4.55-4.52 (m, 1H), 1.82-1.80 (m, 2H), 1.52 (d, *J* = 6.4 Hz, 2H), 1.42 (d, *J* = 6.4 Hz, 3H), 0.86-0.83 (m, 3H)。 | 517.4, $t_R$ =1.579 min |
| 320 | 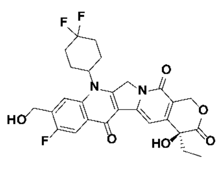 | (400 MHz, DMSO-$d_6$+$D_2O$) δ 8.15 (d, *J* = 6.0 Hz, 1H), 7.95 (d, *J* = 10.0 Hz, 1H), 7.30 (s, 1H), 5.54 (s, 2H), 5.42 (dd, *J* = 15.6 Hz, *J* = 28.4 Hz, 2H), 4.73 (s, 1H), 4.61-4.51 (s, 1H), 2.82-2.70 (m, 2H), 2.29-2.04 (m, 2H), 1.86-1.76 (m, 2H), 1.52 (d, *J* = 6.8 Hz, 2H), 0.84 (d, *J* = 6.8 Hz, 3H)。 | 531.3, $t_R$=1.592min |
| 321 | 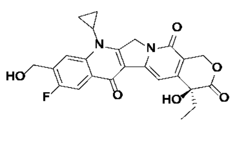 | (400 MHz, DMSO-$d_6$) δ 8.37-8.27 (m, 1H), 7.90-7.80 (m, 1H), 7.25 (s, 1H), 6.32 (s, 1H), 5.76-5.66 (m, 1H), 5.42 (s, 1H), 5.35-5.27 (m, 2H), 4.79-4.67 (m, 2H), 3.66-3.54 (m, 1H), 1.83-1.73 (m, 2H), 1.44-1.39 (m, 1H), 1.24-1.16 (m, 4H), 0.82 (t, *J* = 7.1 Hz, 3H)。 | 453.3, $t_R$=1.371min |
| 322 | 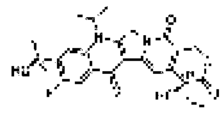 Isomer 1 or Isomer 2 | (400 MHz, DMSO-$d_6$) δ 8.20 (d, J = 5.9 Hz, 1H), 7.95 (d, J = 10.3 Hz, 1H), 7.29 (s, 1H), 5.56 - 5.39 (m, 2H), 5.39 - 5.27 (m, 2H), 5.18 - 5.05 (m, 1H), 4.91 - 4.77 (m, 1H), 1.89 - 1.80 (m, 2H), 1.76 (t, J = 7.4 Hz, 6H), 1.43 (d, J = 6.4 Hz, 3H), 0.85 (t, J = 7.3 Hz, 3H)。 | 469.3, $t_R$=1.301min |
| 323 | 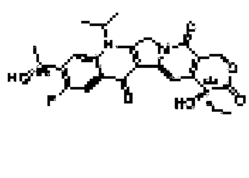 Isomer 2 or Isomer 1 | (400 MHz, DMSO-$d_6$) δ 8.21 (d, J = 5.9 Hz, 1H), 7.95 (d, J = 10.3 Hz, 1H), 7.29 (s, 1H), 6.37 (s, 1H), 5.82 (s, 1H), 5.47 (s, 2H), 5.36 (m, J = 5.1 Hz, 2H), 5.17 - 5.08 (m, 1H), 4.92 - 4.81 (m, 1H), 1.85 - 1.79 (m, 2H), 1.78 - 1.73 (m, 6H), 1.44 (d, J = 6.4 Hz, 2H), 0.86 (t, J = 7.3 Hz, 3H)。 | 469.3, $t_R$=1.353min |
| 324 | 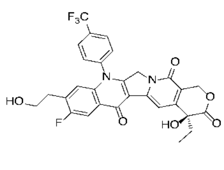 | (400 MHz, DMSO-$d_6$) δ 7.98 (d, *J* = 7.1 Hz, 1H), 7.33 (s, 1H), 7.30 - 7.08 (m, 5H), 6.43 (s, 1H), 5.37 - 5.29 (m, 2H), 4.96 - 4.87 (m, 1H), 4.77 - 4.70 (m, 1H), 3.91 - 3.86 (m, 2H), 3.64 - 3.54 (m, 1H), 2.85 (t, *J* = 6.2 Hz, 2H), 1.89 - 1.79 (m, 1H), 1.71 - 1.61 (m, 1H), 0.89 (t, *J* = 7.2 Hz, 3H)。 | 571.1, $t_R$=0.808min |
| 325 | 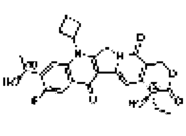 Isomer 1 or Isomer 2 | (400 MHz, DMSO-$d_6$+ $D_2O$) δ 8.14 (d, *J* = 5.6 Hz, 1H), 7.92 (d, *J* = 12.8 Hz, 1H), 7.31 (s, 1H), 5.45-5.37 (m, 1H), 5.36-5.23 (m, 2H), 5.20-5.13 (m, 1H), 5.10-5.06 (m, 1H), 2.84-2.62 (m, 4H), 1.98-1.86 (m, 2H), 1.85-1.68 (m, 2H), 1.42 (d, *J* = 6.4 Hz, 3H), 0.84 (t, *J* = 7.2 Hz, 3H)。 | 481.3 $t_R$=1.521min |
| 326 | 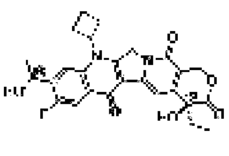 Isomer 2 or Isomer 1 | (400 MHz, DMSO-$d_6$+ $D_2O$) δ 8.16 (d, *J* = 12.8 Hz, 1H), 7.92 (d, *J* = 12.8 Hz, 1H), 7.32 (s, 1H), 5.52-5.41 (m, 2H), 5.39-5.27 (m, 2H), 5.23-5.16 (m, 2H), 5.12 (dd, *J* = 6.0, 12.4 Hz, 1H), 2.88-2.66 (m, 4H), 2.00-1.88 (m, 2H), 1.86-1.74 (m, 2H), 1.43 (d, *J* = 6.4 Hz, 3H), 0.85 (t, *J* = 7.2 Hz, 3H). | 481.3 $t_R$=1.561min |

(continued)

| Examples | Strucures | $^1$H NMR | LCMS (ESI) $[M+H]^+$ |
|---|---|---|---|
| 327 | 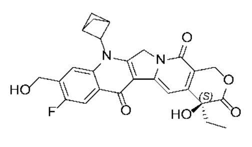 | (400 MHz, DMSO-$d_6$) δ 8.41 (d, $J$ = 5.8 Hz, 1H), 7.88 (d, $J$ = 9.9 Hz, 1H), 7.33 (s, 1H), 6.34 (s, 1H), 5.83 (t, $J$ = 5.9 Hz, 1H), 5.64 - 5.47 (m, 2H), 5.40 - 5.23 (m, 2H), 4.77 (d, $J$ = 5.6 Hz, 2H), 2.91 - 2.73 (m, 7H), 1.90 - 1.73 (m, 2H), 0.86 (t, $J$ = 7.3 Hz, 3H)。 | 479.0, $t_R$=1.407 min |
| 328 | 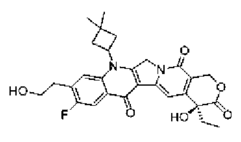 | (400 MHz, DMSO-$d_6$) δ 7.84 (d, $J$ = 20.8 Hz, 1H), 7.75 - 7.63 (m, 1H), 7.21 (d, $J$ = 9.7 Hz, 1H), 6.65 (s, 1H), 6.33 (s, 1H), 5.40 - 5.14 (m, 4H), 4.84 (s, 1H), 3.70 (s, 2H), 3.00 - 2.91 (m, 2H), 2.00 (q, $J$ = 6.4, 5.6 Hz, 4H), 1.46 (s, 2H), 0.85 (td, $J$ = 14.9, 12.5, 9.1 Hz, 9H) | 509.3, $t_R$=1.407min |
| 329 | 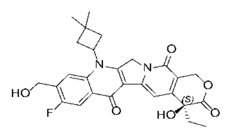 | (400 MHz, DMSO-$d_6$) δ 8.10 (d, $J$ = 5.7 Hz, 1H), 7.92 (d, $J$ = 9.9 Hz, 1H), 7.31 (s, 1H), 6.35 (s, 1H), 5.74 (s, 1H), 5.45 (s, 2H), 5.38 - 5.27 (m, 2H), 5.22 - 5.10 (m, 1H), 4.77 (s, 2H), 2.69 - 2.57 (m, 4H), 1.87 - 1.76 (m, 2H), 1.34 (s, 3H), 1.24 (s, 3H), 0.85 (t, $J$ = 7.3 Hz, 3H)。 | 495.3, $t_R$=1.442min |
| 330 | 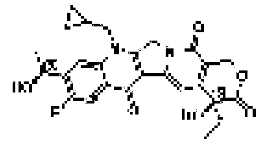 Isomer 1 or Isomer 2 | (400 MHz, DMSO-$d_6$+$D_2O$) δ 8.08 (d, $J$ = 5.5 Hz, 1H), 7.93 (d, $J$ = 10.4 Hz, 1H), 7.28 (s, 1H), 5.43-5.28 (m, 4H), 5.12 (q, $J$ = 6.5 Hz, 1H), 4.56-4.26 (m, 2H), 1.86-1.75 (m, 2H), 1.44-1.33 (m, 4H), 0.85 (t, $J$ = 7.1 Hz, 3H), 0.63-0.53 (m, 4H)。 | 481.3, $t_R$=1.541 |
| 331 | 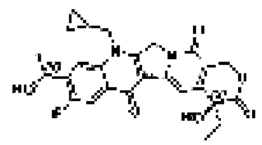 Isomer 2 or Isomer 1 | (400 MHz, DMSO-$d_6$+$D_2O$) δ 8.08 (d, $J$ = 5.2 Hz, 1H), 7.94 (d, $J$ = 10.2 Hz, 1H), 7.29 (s, 1H), 5.43-5.26 (m, 4H), 5.12 (q, $J$ = 6.1 Hz, 1H), 4.55-4.29 (m, 2H), 1.86-1.75 (m, 2H), 1.44-1.34 (m, 4H), 0.86 (t, $J$ = 7.3 Hz, 3H), 0.58 (d, $J$ = 6.6 Hz, 4H)。 | 481.3, $t_R$=1.577 |
| 332 | 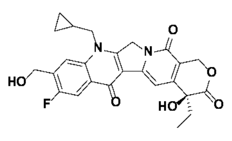 | (400 MHz, DMSO-$d_6$+$D_2O$) δ 8.08 (d, $J$ = 5.5 Hz, 1H), 7.94 (d, $J$ = 9.9 Hz, 1H), 7.29 (s, 1H), 5.42-5.28 (m, 4H), 4.75 (s, 2H), 4.40-4.30 (m, 2H), 1.88-1.75 (m, 2H), 1.43-1.35 (m, 1H), 0.85 (t, $J$ = 7.1 Hz, 3H), 0.57 (d, $J$ = 7.4 Hz, 4H)。 | 467.3, $t_R$=1.471 |
| 333 | 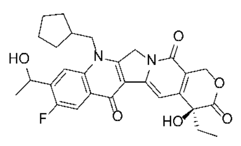 | (400 MHz, DMSO-$d_6$) δ 8.05 (d, $J$ = 5.7 Hz, 1H), 7.96 - 7.91 (m, 1H), 7.29 (s, 1H), 5.42 - 5.33 (m, 4H), 5.12 (q, $J$ = 6.4 Hz, 1H), 4.49 - 4.31 (m, 2H), 1.87 - 1.78 (m, 2H), 1.75 - 1.65 (m, 4H), 1.56 - 1.48 (m, 2H), 1.51 - 1.40 (m, 5H), 0.89 - 0.82 (m, 3H)。 | 509.3, $t_R$ =1.46 min |
| 334 | 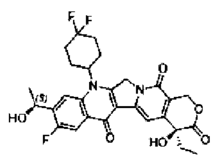 Isomer 1 or Isomer 2 | (400 MHz, DMSO-$d_6$ +$D_2O$) δ 8.20 (d, $J$ = 6.0 Hz, 1H), 7.94 (d, $J$ = 10.4 Hz, 1H), 7.29 (s, 1H), 5.54 (s, 2H), 5.31 (dd, $J$ = 15.6, 24.0 Hz, 2H), 5.08 (q, $J$ = 6.0 Hz, 1H), 4.64-4.52 (m, 1H), 3.73 (q, $J$ = 7.2 Hz, 1H), 2.30-2.16 (m, 5H), 1.84-1.76 (m, 2H), 1.51 (d, $J$ = 7.2 Hz, 1H), 1.40 (d, $J$ = 6.4 Hz, 3H), 1.35 (d, $J$ = 7.2 Hz, 3H), 0.85 (t, $J$ = 7.2 Hz, 3H)。 | 545.4 $t_R$=1.677 min |

(continued)

| Examples | Strucures | $^1$H NMR | LCMS (ESI) $[M+H]^+$ |
|---|---|---|---|
| 335 | 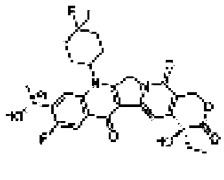 Isomer 2 or Isomer 1 | (400 MHz, DMSO-$d_6$ +$D_2O$): δ 8.21 (d, *J* = 7.6 Hz, 1H), 7.94 (d, *J* = 14.0 Hz, 1H), 7.29 (s, 1H), 5.55 (s, 2H), 5.36 (dd, *J* = 15.9, 20.4 Hz, 2H), 5.09 (q, *J* = 6.9 Hz, 1H), 4.64-4.52 (m, 1H), 3.80-3.70 (m, 1H), 2.30-2.14 (m, 5H), 1.86-1.74 (m, 2H), 1.50 (d, *J* = 5.2 Hz, 2H), 1.42-1.34 (m, 4H), 1.23 (s, 1H), 0.85 (t, *J* = 9.6 Hz, 3H)。 | 545.4 $t_R$=1.695min |
| 336 | 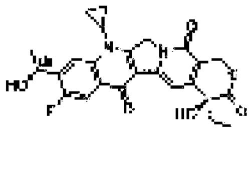 Isomer 1 or Isomer 2 | (400 MHz, DMSO-*d*$_6$)6 8.35 (d, *J* = 6.0 Hz, 1H), 7.87-7.81 (m, 1H), 7.25 (s, 1H), 5.42 (s, 2H), 5.32-5.27 (m, 2H), 5.11-5.05 (m, 2H), 4.48-4.42 (m, 1H), 3.62-3.56 (m, 1H), 1.81-1.74 (m, 2H), 1.42-1.36 (m, 3H), 1.20-1.10 (m, 4H), 0.81 (t, *J* = 7.3 Hz, 3H)。 | 467.4; $t_R$=1.460min |
| 337 | 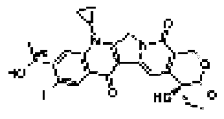 Isomer 2 or Isomer 1 | (400 MHz, DMSO-*d*$_6$) δ 8.36 (d, *J* = 6.0 Hz, 1H), 7.86 (d, *J* = 10.2 Hz, 1H), 7.25 (s, 1H), 6.31 (s, 1H), 5.69 (d, *J* = 4.7 Hz, 1H), 5.42 (s, 1H), 5.36-5.27 (m, 2H), 5.13-5.07 (m, 1H), 3.64-3.56 (m, 1H), 1.83-1.72 (m, 2H), 1.40 (d, *J* = 6.3 Hz, 3H), 1.28-1.15 (m, 4H), 0.82 (t, *J* = 7.3 Hz, 3H)。 | 467.4; $t_R$=1.497min |
| 338 | 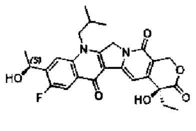 Isomer 1 or Isomer 2 | (400 MHz, DMSO-*d*$_6$) δ 7.99 (d, *J* = 5.8 Hz, 1H), 7.94 (d, *J* = 10.3 Hz, 1H), 7.29 (s, 1H), 6.37 (s, 2H), 5.72 (d, *J* = 4.8 Hz, 1H), 5.42 - 5.29 (m, 4H), 5.18 - 5.04 (m, 1H), 4.43 - 4.07 (m, 2H), 1.85 - 1.77 (m, 2H), 1.43 (d, *J* = 6.4 Hz, 3H), 1.05 - 0.95 (m, 6H), 0.90 - 0.83 (m, 3H)。 | 483.3, $t_R$=1.381min |
| 339 | 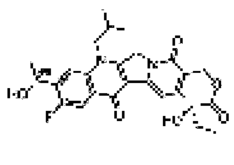 Isomer 2 or Isomer 1 | (400 MHz, DMSO-*d*$_6$) $^1$H NMR (400 MHz, DMSO-*d*$_6$) δ 7.99 (d, *J* = 5.8 Hz, 1H), 7.94 (d, *J* = 10.3 Hz, 1H), 7.29 (s, 1H), 6.37 (s, 2H), 5.72 (d, *J* = 4.8 Hz, 1H), 5.42 - 5.29 (m, 4H), 5.18 - 5.04 (m, 1H), 4.43 - 4.07 (m, 2H), 1.85 - 1.77 (m, 2H), 1.43 (d, *J* = 6.4 Hz, 3H), 1.05 - 0.95 (m, 6H), 0.90 - 0.83 (m, 3H)。 | 483.3, $t_R$=1.422min |
| 340 | 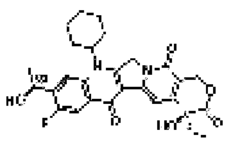 Isomer 1 or Isomer 2 | (400 MHz, DMSO-*d*$_6$) δ 8.36 (d, J = 5.9 Hz, 1H), 7.94 (d, J = 10.3 Hz, 1H), 7.30 (s, 1H), 5.64 - 5.47 (m, 2H), 5.41-5.26 (m, 2H), 5.15 - 5.04 (m, 1H), 4.37 - 4.25 (m, 1H), 2.48 - 2.40 (m, 1H), 2.21 - 2.07 (m, 2H), 2.02 - 1.76 (m, 6H), 1.69 - 1.56 (m, 2H), 1.44 (d, J = 6.4 Hz, 3H), 1.37 - 1.25 (m, 1H), 0.86 (t, J = 7.3 Hz, 3H)。 | 509.2, $t_R$=0.840min |
| 341 | 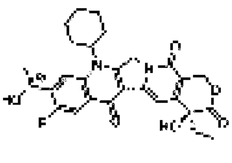 Isomer 2 or Isomer 1 | (400 MHz, DMSO-*d*$_6$) δ 8.36 (d, J = 5.9 Hz, 1H), 7.94 (d, J = 10.3 Hz, 1H), 7.30 (s, 1H), 5.62 - 5.48 (m, 2H), 5.42 - 5.29 (m, 2H), 5.14 - 5.07 (m, 1H), 4.36 - 4.24 (m, 1H), 2.48 - 2.41 (m, 1H), 2.17 - 2.08 (m, 2H), 2.01 - 1.74 (m, 6H), 1.69 - 1.55 (m, 2H), 1.44 (d, J = 6.4 Hz, 3H), 1.37 - 1.26 (m, 1H), 0.86 (t, J = 7.3 Hz, 3H)。 | 509.2, $t_R$=0.862min |
| 342 | 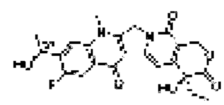 Isomer 1 or Isomer 2 | (400 MHz, DMSO-*d*$_6$) δ 8.00 (d, *J* = 5.8 Hz, 1H), 7.93 (d, *J* = 10.4 Hz, 1H), 7.27 (s, 1H), 6.37 (s, 1H), 5.76 - 5.69 (m, 1H), 5.46 - 5.30 (m, 4H), 5.17 - 5.10 (m, 1H), 3.93 (s, 3H), 1.89 - 1.77 (m, 2H), 1.45 (d, *J* = 6.4 Hz, 3H), 0.86 (t, *J* = 7.3 Hz, 3H)。 | 441.3, $t_R$=1.131min |

(continued)

| Examples | Strucures | $^1$H NMR | LCMS (ESI) [M+H]$^+$ |
|---|---|---|---|
| 343 | 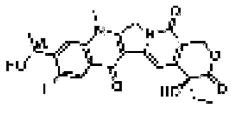 Isomer 2 or Isomer 1 | (400 MHz, DMSO-$d_6$) δ 8.00 (d, *J* = 5.9 Hz, 1H), 7.93 (d, *J* = 10.3 Hz, 1H), 7.27 (s, 1H), 6.37 (s, 1H), 5.75 - 5.67 (m, 1H), 5.44 - 5.29 (m, 4H), 5.13 (t, *J* = 5.7 Hz, 1H), 3.93 (s, 3H), 1.87 - 1.77 (m, 2H), 1.45 (d, *J* = 6.4 Hz, 3H), 0.86 (t, *J* = 7.3 Hz, 3H)。 | 441.2, $t_R$=0.449min |
| 344 | 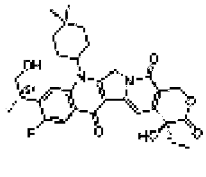 Isomer 1 or Isomer 2 | (400 MHz, DMSO-$d_6$) δ 8.06(d, *J* = 5.6 Hz, 1H), 7.95(d, *J* = 10.4 Hz, 1H), 7.30 (s, 1H), 6.33(s, 1H), 5.53(s, 2H), 5.41-5.29(m, 2H),4.95 (s, 1H), 4.29-4.17 (m, 1H), 3.71-3.59(m, 2H), 2.64-2.59 (m, 2H), 2.04-1.89 (m, 2H), 1.86-1.73 (m, 2H), 1.71-1.61 (m, 2H), 1.59-1.49 (m, 2H), 1.32 (d, *J* = 7.2 Hz, 3H), 1.15 (s, 3H), 1.02 (s, 3H), 0.86 (t, *J* = 7.2 Hz, 3H)。 | 551.5, $t_R$=1.833min |
| 345 | 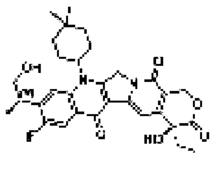 Isomer 2 or Isomer 1 | (400 MHz, DMSO-$d_6$) δ 8.82 (bar, 1H), 8.07 (d, *J* = 5.6 Hz, 1H), 8.00 (bar, 1H), 7.95 (d, *J* = 10.4 Hz, 1H), 7.30 (s, 1H), 6.45 (bar, 1H), 5.53 (s, 2H), 5.41-5.29 (m, 2H), 5.00-4.92 (m, 1H), 4.29-4.17 (m, 1H), 3.71-3.59 (m, 2H), 2.64-2.59 (m, 2H), 2.04-1.89 (m, 2H), 1.86-1.73 (m, 2H), 1.71-1.61 (m, 2H), 1.59-1.49 (m, 2H), 1.32 (d, *J* = 7.2 Hz, 3H), 1.15 (s, 3H), 1.02 (s, 3H), 0.86 (t, *J* = 7.2 Hz, 3H) | 551.4, $t_R$=1.882min |
| 346 | 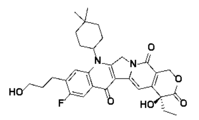 | (400MHz,DMSO-$d_6$ + $D_2O$) δ 8.05-7.92 (m, 2H), 7.38-7.28 (m, 1H), 5.52 (s, 2H), 5.36 (dd, *J*=16.0, 23.2 Hz,2H), 4.30-4.15 (m, 1H), 3.44 (t, *J*=6.0 Hz, 2H), 2.89 (t, *J*=7.2 Hz, 2H), 2.68-2.53 (m, 2H), 1.96-1.88 (m, 2H), 1.88-1.78 (m, 4H), 1.70-1.52 (m, 4H), 1.13 (s, 3H), 1.03 (s, 3H), 0.85 (t, *J* = 7.2 Hz, 3H)。 | 551.4, $t_R$=1.823 min |

Example 347: Preparation of (S)-11-cyclopentyl-4-ethyl-8-fluoro-4-hydroxy-9-((S)-2,2,2-trifluoro-1-hydroxyethyl)-1,12-dihydro-14H-pyrano[3,4:6,7]indolizino[2,1 -b]quinoline-3,6,14(4H,11H)-trione (Isomer 1 or Isomer 2):

Example 348: Preparation of (S)-11-cyclopentyl-4-ethyl-8-fluoro-4-hydroxy-9-((S)-2,2,2-trifluoro-1-hydroxyethyl)-1,12-dihydro-14H-pyrano[3,4:6,7]indolizino[2,1 -b]quinoline-3,6,14(4H,11H)-trione (Isomer 2 or Isomer 1):

*Step 1: Preparation of 1-cyclopentyl-6-fluoro-2-methyl-7-(2,2,2-trifluoro-1-hydroxyethyl)quinolin-4(1H)-one.*

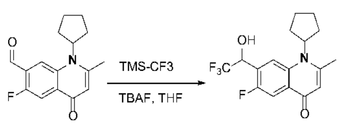


1-Cyclopentyl-6-fluoro-2-methyl-4-oxo-1,4-dihydroquinoline-7-carbaldehyde (0.8 g, 2.93 mmol, 1.0 eq.), the intermediate obtained from Step 2 of Example 293, was dissolved in tetrahydrofuran (8 mL). Tetra-n-butylammonium fluoride (917.8 mg, 3.52 mmol, 1.2 eq.) and (trifluoromethyl)trimethylsilane (624.2 mg, 4.4 mmol, 1.5 eq.) were added, and the mixture was stirred at room temperature for 2 h. LCMS monitoring indicated the reaction was complete. The reaction mixture was poured into ice water (100 mL) and extracted with ethyl acetate (50 mL × 3). The combined organic phases were washed with saturated sodium chloride solution (200 mL), dried over anhydrous sodium sulfate and filtered. The filtrate was concentrated under reduced pressure to afford a crude product, which was purified by silica gel column chromatography (eluent: DCM:MeOH = 1:0 to 20:1, v/v) to yield the title compound as a yellow solid (140 mg, yield: 14%). LCMS (ESI): [M+H]$^+$ = 344.3, $t_R$ = 1.567 min.

*Step 2: Preparation of 3-bromo-2-(bromomethyl)-1-cyclopentyl-6-fluoro-7-(2,2,2-trifluoro-1-hydroxyethyl)quinolin-4(1H)-one.*

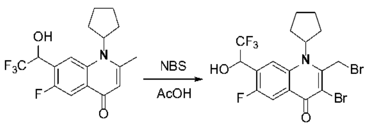


The intermediate obtained from step 1 (140 mg, 0.41 mmol, 1.0 eq.) was dissolved in acetic acid (3 mL). N-Bromosuccinimide (159.8 mg, 0.90 mmol, 2.2 eq.) was added at room temperature, and the mixture was stirred at room temperature for 16 h under a nitrogen atmosphere after the addition was complete. LCMS monitoring indicated the reaction was complete. The reaction mixture was poured into ice-cold saturated sodium bicarbonate solution (100 mL) and extracted with ethyl acetate (50 mL × 3). The combined organic phases were washed with saturated sodium chloride solution (200 mL), dried over anhydrous sodium sulfate and filtered. The filtrate was concentrated to afford a crude product, which was purified by silica gel column chromatography (eluent: DCM:MeOH = 1:0 to 20:1, v/v) to yield the title compound as a yellow solid (120 mg, yield: 59%). LCMS (ESI): $[M+H]^+$ = 501.9, $t_R$ = 1.689 min.

*Step 3: Preparation of (4S)-7-((3-bromo-1-cyclopentyl-6-fluoro-4-oxo-7-(2,2,2-trifluoro-1-hydroxyethyl)-1,4-dihydroquinolin-2-yl)methyl)-4-ethyl-4-hydroxy-1,7-dihydro-3H-pyrano[3,4-c]pyridine-3,8(4H)-dione:*

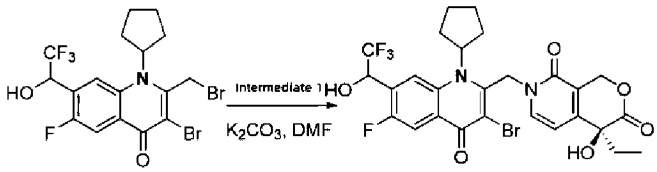


The intermediate obtained from step 2 (120 mg, 0.24 mmol, 1.0 eq.) was dissolved in anhydrous N,N-dimethylformamide (3 mL). Potassium carbonate (99.8 mg, 0.72 mmol, 3.0 eq.) and Intermediate 1 (50.3 mg, 0.95 mmol, 1.0 eq.) were added sequentially, and the mixture was stirred at room temperature for 2 h. LCMS monitoring indicated the reaction was complete. The reaction mixture was poured into ice-cold saturated ammonium chloride solution (50 mL) and extracted with ethyl acetate (20 mL × 3). The combined organic phases were washed with saturated brine (50 mL), dried over anhydrous sodium sulfate and filtered. The filtrate was concentrated under reduced pressure to afford a crude product, which was purified by silica gel column chromatography (eluent: DCM:MeOH = 1:0 to 20:1, v/v) to yield the title compound as a white solid (60 mg, yield: 39.6%). LCMS (ESI): $[M+H]^+$ = 629.2, $t_R$ = 1.517 min.

*Step 4: Preparation of (4S)-7-((3-bromo-1-cyclopentyl-6-fluoro-4-oxo-7-(2,2,2-trifluoro-1-((tetrahydro-2H-pyran-2-yl) oxy)ethyl)-1,4-dihydroquinolin-2-yl)methyl)-4-ethyl-4-hydroxy-1,7-dihydro-3H-pyrano[3,4-c]pyridine-3,8(4H)-dione:*

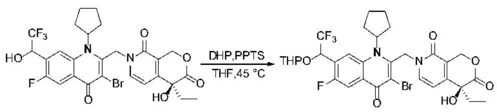


The intermediate obtained from step 3 (60 mg, 0.1 mmol, 1.0 eq.) was dissolved in anhydrous tetrahydrofuran (3 mL). 3,4-Dihydropyran (32.1 mg, 0.38 mmol, 4.0 eq.) and pyridinium p-toluenesulfonate (0.96 mg, 0.004 mmol, 0.04 eq.) were added sequentially, and the mixture was heated to 50 °C and stirred for 16 h under a nitrogen atmosphere. LCMS monitoring indicated the reaction was complete. The reaction mixture was concentrated under reduced pressure to afford a crude product, which was purified by silica gel column chromatography (eluent: DCM:MeOH = 1:0 to 50:1, v/v) to yield the target product as a pale yellow solid (30 mg, yield: 44%). LCMS (ESI): $[M+H]^+$ = 715.2, $t_R$ = 1.970 min.

*Step 5: Preparation of (S)-11-cyclopentyl-4-ethyl-8-fluoro-4-hydroxy-9-((S)-2,2,2-trifluoro-1-hydroxyethyl)-1,12-dihydro-14H-pyrano[3,4:6,7]indolizino[2,1-b]quinoline-3,6,14(4H,11H)-trione (Isomer 1 or Isomer 2) and (S)-11-cyclopentyl-4-ethyl-8-fluoro-4-hydroxy-9-((S)-2,2,2-trifluoro-1-hydroxyethyl)-1,1,2-dihydro-14H-pyrano[3,4:6,7]indolizino[2,1 -b] quinoline-3,6,14(4H,11H)-trione (Isomer 2 or Isomer 1):*

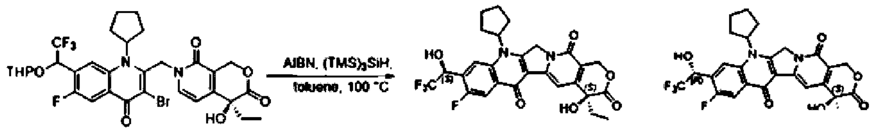


Isomer 1 or Isomer 2 Isomer 2 or Isomer 1

The intermediate obtained from Step 4 (30 mg, 0.04 mmol, 1.0 eq.) was dissolved in toluene (5 mL). 2,2 -Azobis(2-methylpropionitrile) (6.9 mg, 0.04 mmol, 1.0 eq.) and tris(trimethylsilyl)silane (41.8 mg, 0.17 mmol, 4.0 eq.) were added, and the mixture was heated to 100 °C and stirred for 16 h under a nitrogen atmosphere. LCMS monitoring indicated the reaction was complete. The reaction mixture was concentrated under reduced pressure, and the residue was purified by silica gel plate chromatography (eluent: DCM:MeOH = 10:1, v/v) to afford a crude product, which was further purified by reversed-phase preparative HPLC to yield the title compounds (Isomer 1 or Isomer 2: 0.9 mg; Isomer 2 or Isomer 1: 2.6 mg, total yield: 15.2%), both as brown solids.

**Isomer 1 or Isomer 2:** LCMS (ESI): $[M+H]^+$ = 549.2, t_R = 1.453 min; $^1$H NMR not determined.

**Isomer 2 or Isomer 1:** LCMS (ESI): $[M+H]^+$ = 549.2, t_R = 1.581 min; $^1$H NMR (400 MHz, DMSO-$d_6$) δ: 8.12-8.07 (m, 1H), 7.48 (d, J = 5.2 Hz, 1H), 7.29 (s, 1H), 6.37 (s, 1H), 5.67-5.60 (m, 1H), 5.56-5.49 (m, 1H), 5.41-5.23 (m, 3H), 5.12-5.01 (m, 1H), 2.16-2.05 (m, 4H), 2.02-1.95 (m, 2H), 1.90-1.77 (m, 5H), 0.86 (t, J = 6.1 Hz, 3H). Example 349: Preparation of (S)-9-cyclopropyl-11-((3S,4S)-1-(cyclopropylmethyl)-4-hydroxypyrrolidin-3-yl)-4-ethyl-8-fluoro-4-hydroxy-1,12-dihydro-14H-pyrano[3,4:6,7]indolizino[2,1 -b]quinoline-3,6,14(4H,11H)-trione

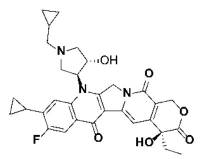

Similarly, using (Z)-1-(4-bromo-2,5-difluorophenyl)-3-(dimethylamino)but-2-en-1-one (Intermediate 4, the product of Preparation Example 4) and commercially available (3S,4S)-N-Boc-3-amino-4-hydroxypyrrolidine as the starting materials, the title compound was synthesized following the synthetic methods of Example 197 and Example 199, with the bromine group further converted to a cyclopropyl group via a coupling reaction, affording an off-white solid. LCMS (ESI): $[M+H]^+$ = 562.4, t_R = 1.562 min; $^1$H NMR (400 MHz, DMSO-$d_6$) δ: 8.46-7.79 (m, 2H), 7.30 (s, 1H), 6.31 (s, 1H), 5.70-5.52 (m, 1H), 5.41-5.08 (m, 4H), 4.89-4.69 (m, 1H), 4.24-3.67 (m, 2H), 3.05-2.88 (m, 1H), 2.83-2.68 (m, 1H), 2.47-2.35 (m, 1H), 2.30-2.18 (m, 1H), 1.88-1.74 (m, 2H), 1.24-0.91 (m, 6H), 0.86 (t, J = 7.3 Hz, 3H), 0.74-0.61 (m, 1H), 0.61-0.42 (m, 2H), 0.25-0.11 (m, 2H).

Preparation of Compounds in Examples 350-360

Similarly, the compounds in Examples 350-360 were synthesized from commercially available reagents following the synthetic methods of Example 8, Example 21 and Example 69. The chemical structures and detailed characterization data (LCMS and $^1$H NMR) of the compounds are shown below:

| Examples | Structure | $^1$H NMR | LCMS (ESI) $[M+H]^+$ |
|---|---|---|---|
| 350 | 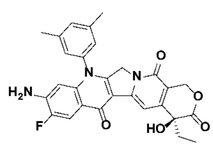 | (400 MHz, DMSO-$d_6$) δ 7.77 (d, *J* = 11.6 Hz, 1H), 7.36 (s, 1H), 7.26 (s, 2H), 7.23 (s, 1H), 6.34 - 6.17 (m, 3H), 5.37 - 5.19 (m, 2H), 4.78 - 4.61 (m, 2H), 2.41 (s, 6H), 1.85 - 1.73 (m, 2H), 0.85 (t, *J* = 7.3 Hz, 3H)。 | 502, $t_R$ =1.562 min |

(continued)

| Examples | Structure | $^1$H NMR | LCMS (ESI) $[M+H]^+$ |
|---|---|---|---|
| 351 | 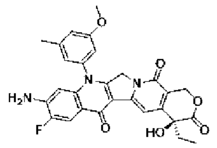 | (400 MHz, DMSO-$d_6$) δ 7.77 (d, J = 11.6 Hz, 1H), 7.26 (s, 1H), 7.11 (s, 1H), 7.08-6.99 (m, 2H), 6.28 (d, J = 7.6 Hz, 1H), 5.33-5.24 (m, 2H), 4.81-4.65 (m, 2H), 3.82 (d, J = 2.4 Hz, 3H), 2.42 (s, 3H), 1.85-1.75 (m, 2H), 0.85 (t, J = 7.2 Hz, 3H)。 | 518.3, $t_R$=1.694min , $t_R$=1.715 min |
| 352 | 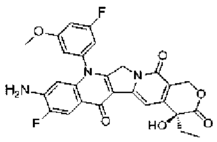 | (400 MHz, DMSO-$d_6$) δ 7.79 (d, *J* = 11.4 Hz, 1H), 7.28 (s, 1H), 7.23 - 7.16 (m, 3H), 6.37 (s, 1H), 6.30 (d, *J* = 7.8 Hz, 3H), 5.31 (s, 2H), 4.87 - 4.73 (m, 2H), 3.88 (d, *J* = 3.0 Hz, 3H), 1.87 - 1.76 (m, 2H), 0.91 - 0.80 (m, 3H)。 | 522, $t_R$=1.382 min |
| 353 | 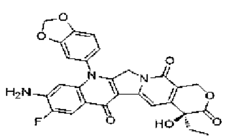 | (400 MHz, DMSO-$d_6$) δ 7.77 (d, *J* = 11.6 Hz, 1H), 7.29 - 7.21 (m, 3H), 7.17 - 7.08 (m, 1H), 6.35 (s, 1H), 6.30 (d, *J* = 7.5 Hz, 1H), 6.28 - 6.21 (m, 4H), 5.36 - 5.23 (m, 2H), 4.81 - 4.68 (m, 2H), 1.87 - 1.74 (m, 2H), 0.85 (t, *J* = 7.3 Hz, 3H)。 | 518.1, $t_R$=0.783min |
| 354 | 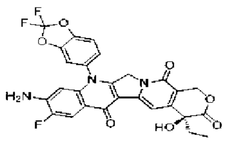 | (400 MHz, DMSO-$d_6$) δ 7.87 - 7.73 (m, 3H), 7.61 - 7.52 (m, 1H), 7.27 (s, 1H), 6.34 (s, 1H), 6.26 - 6.20 (m, 3H), 5.35 - 5.22 (m, 2H), 4.87 - 4.69 (m, 2H), 1.85 - 1.74 (m, 2H), 0.85 (t, *J* = 7.2 Hz, 3H)。 | 554, $t_R$ =1.595 min |
| 355 | 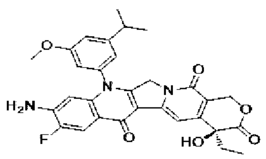 | (400 MHz, DMSO-$d_6$) δ 7.90 (s, 1H), 7.78 (d, *J* = 11.6 Hz, 1H), 7.26 (s, 1H), 7.17 - 7.01 (m, 2H), 6.35 (s, 1H), 6.31 - 6.24 (m, 2H), 5.33 - 5.22 (m, 2H), 4.78 - 4.65 (m, 1H), 3.83 (d, *J* = 2.8 Hz, 3H), 3.05 - 2.98 (m, 2H), 1.86 - 1.75 (m, 2H), 1.33 - 1.23 (m, 6H), 0.85 (t, *J* = 7.3 Hz, 3H)。 | 546, $t_R$=1.720min |
| 356 | 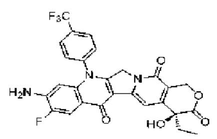 | (400 MHz, DMSO-$d_6$) δ 8.18 (d, *J* = 8.6 Hz, 2H), 7.99 - 7.87 (m, 2H), 7.79 (d, *J* = 11.6 Hz, 1H), 7.27 (s, 1H), 6.42 - 6.22 (m, 3H), 6.15 (d, *J* = 7.4 Hz, 1H), 5.37 - 5.21 (m, 2H), 4.82 - 4.65 (m, 2H), 1.87 - 1.72 (m, 2H), 0.85 (t, *J* = 7.3 Hz, 3H)。 | 542, $t_R$ =1.589 min |
| 357 | 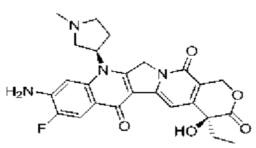 | (400 MHz, DMSO-$d_6$) δ 7.80 (d, *J* = 11.5 Hz, 1H), 7.27 (d, *J* = 7.1 Hz, 1H), 7.23 (s, 1H), 5.51-5.26 (m, 4H), 4.22-4.13 (m, 1H), 3.90-3.83 (m, 1H), 3.77-3.62 (m, 1H), 3.32-3.19 (m, 1H), 3.09-3.01 (m, 1H), 2.92 (s, 3H), 2.84-2.72 (m, 1H), 2.69-2.56 (m, 1H), 1.83-1.73 (m, 2H), 0.82 (t, *J* = 7.3 Hz, 3H). | 481.2, $t_R$= 1.130 min |
| 358 | 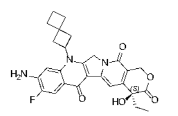 | N/A | 492.2, $t_R$= 1.530 min |
| 359 | 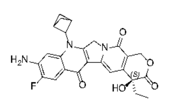 | N/A | 464.2, $t_R$= 1.613 min |
| 360 | 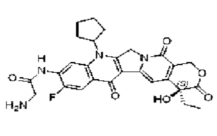 | (400 MHz, DMSO-$d_6$) δ 10.87 (s, 1H), 8.78 (d, *J* = 6.3 Hz, 1H), 8.31 (s, 3H), 8.13 (d, *J* = 11.1 Hz, 1H), 7.30 (s, 1H), 5.61 - 5.48 (m, 2H), 5.44 - 5.30 (m, 2H), 5.04 - 4.91 (m, 1H), 4.06 - 3.96 (m, 2H), 2.37 - 2.25 (m, 4H), 2.25 - 2.13 (m, 2H), 1.86 - 1.76 (m, 4H), 0.87 (t, *J* = 7.3 Hz, 3H)。 | 523.2, $t_R$= 0.972 min |

Example 361: Preparation of (S)-11-(bicyclo[1.1.1]pentan-2-yl)-4-ethyl-8-fluoro-4-hydroxy-1,12-dihydro-14H-pyrano [3,4:6,7]indolizino[2,1 -b]quinoline-3,6,14(4H,11H)-trione:

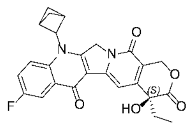

Using (2,5-difluorophenyl)-3-(dimethylamino)but-2-en-1-one (Intermediate 27 from Preparation Example 27) and commercially available bicyclo[1.1.1]pentan-2-amine as the starting materials, the title compound was synthesized following the procedures of Steps 1~7 in Example 102, affording a pale yellow solid. LCMS (ESI): $[M+H]^+$ = 449.15.

Example 362: Preparation of (S)-4-ethyl-4-hydroxy-8,11-dimethyl-1,12-dihydro-14H-pyrano[3,4:6,7]indolizino[2,1 -b] quinoline-3,6,14(4H,11H)-trione:

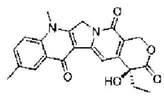

Using 4-methylaniline as the starting material, the title compound was synthesized following the procedures of Steps 1~8 in Example 2, affording a white solid. LCMS (ESI): $[M+H]^+$ = 393.3, = 1.343 min.

Example 363: Preparation of (S)-14-(4,4-dimethylcyclohexyl)-7-ethyl-3-fluoro-7-hydroxy-2-(2-hydroxyethyl)-10,14-dihydro-11H-pyrano[3,4:6,7]indolizino[2,1 -b][1,8]naphthyridine-5,8,11(7H,13H)-trione:

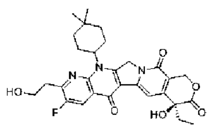

Similarly, following the synthetic method of Example 196, 2-chloro-5-fluoro-6-(2-((tetrahydro-2H-pyran-2-yl)oxy) ethyl)nicotinonitrile (the intermediate obtained from Step 4 of Example 196) was used as the starting material to undergo a substitution reaction with 4,4-dimethylcyclohexanamine, affording the intermediate 2-((4,4-dimethylcyclohexyl)amino)-5-fluoro-6-(2-((tetrahydro-2H-pyran-2-yl)oxy)ethyl) nicotinonitrile. The title compound was further synthesized from this intermediate following the procedures of steps 6~13 in Example 196, affording a pale yellow solid. LCMS (ESI): $[M+H]^+$ = 538.2.

Example 364: Preparation of (S)-7-ethyl-3-fluoro-7-hydroxy-2-(2-hydroxyethyl)-14-(spiro[3.3]heptan-2-yl)-10,14-dihydro-11H-pyrano[3,4:6,7]indolizino[2,1 -b][1,8]naphthyridine-5,8,11(7H,13H)-trione

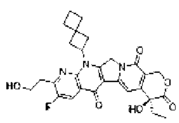

Similarly, following the synthetic method of Example 196, 2-chloro-5-fluoro-6-(2-((tetrahydro-2H-pyran-2-yl)oxy) ethyl)nicotinonitrile (the intermediate obtained from Step 4 of Example 196) was used as the starting material to undergo a substitution reaction with spiro[3.3]heptan-2-amine, affording the intermediate 2-((spiro[3.3]heptan-2-yl)amino)-5-fluoro-6-(2-((tetrahydro-2H-pyran-2-yl)oxy)ethyl) nicotinonitrile. The title compound was further synthesized from this intermediate following the procedures of Steps 6~13 in Example 196, affording a pale yellow solid. LCMS (ESI): $[M+H]^+$ =

522.2.

Preparation of Compounds in Examples 365-373:

Similarly, following the synthetic method of Example 83, tert-butyl (R)-3-(6-fluoro-2-methyl-4-oxoquinolin-1(4H)-yl)piperidine-1-carboxylate (Intermediate 47) was used as the starting material to synthesize tert-butyl (R)-3-((S)-4-ethyl-8-fluoro-4-hydroxy-3,6,14-trioxo-4,6,12,14-tetrahydro-1H-pyrano[3,4:6,7]indolizino[2,1 -b]quinolin-11(3H)-yl)piperidine-1-carboxylate. This intermediate was subjected to **Boc deprotection under acidic conditions** to afford (S)-4-ethyl-8-fluoro-4-hydroxy-11-((R)-piperidin-3-yl)-1,12-dihydro-14H-pyrano[3,4:6,7]indolizino[2,1 - b]quinoline-3,6,14(4H,11H)-trione (Example 365). The target compounds (Examples 366-370) were further synthesized from this intermediate following the method of Example 250.

Similarly, following the synthetic method of Example 83, (Z)-1-(2,5-difluorophenyl)-3-(dimethylamino)but-2-en-1-one (Intermediate 27 from Preparation Example 27) and commercially available tert-butyl (S)-3-aminopiperidine-1-carboxylate were used as the starting materials to synthesize tert-butyl (S)-3-((S)-4-ethyl-8-fluoro-4-hydroxy-3,6,14-trioxo-4,6,12,14-tetrahydro-1H-pyrano[3,4:6,7]indolizino[2,1 -b]quinolin-11(3H)-yl)piperidine-1-carboxylate. This intermediate was subjected to **Boc deprotection under acidic conditions** to afford (S)-4-ethyl-8-fluoro-4-hydroxy-11-((S)-piperidin-3-yl)-1,12-dihydro-14H-pyrano[3,4:6,7]indolizino[2,1 -b]quinoline-3,6,14(4H,11H)-trione. The target compounds (Examples 371, 372, 373) were further synthesized from this intermediate following the method of Example 250.

The chemical structures and detailed characterization data (LCMS and $^1$H NMR) of the specific compounds are shown below:

| **Examples** | Structure | $^1$H NMR | LCMS (ESI) [M+H]$^+$ |
|---|---|---|---|
| 365 | 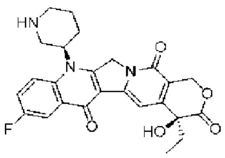 | (400 MHz, DMSO-$d_6$ +$D_2O$) δ 8.36 (dd, *J* = 9.5, 3.7 Hz, 1H), 8.02 (dd, *J* = 8.5, 3.0 Hz, 1H), 7.69-7.65 (m, 1H), 7.30 (s, 1H), 5.60-5.29 (m, 4H), 4.74-4.57 (m, 1H), 3.90 (t, *J* = 12.2 Hz, 1H), 3.82-3.71 (m, 1H), 3.40 (d, *J* = 12.4 Hz, 1H), 3.21 (t, *J* = 12.9 Hz, 1H), 2.77-2.67 (m, 1H), 2.37-2.19 (m, 1H), 2.15-1.76 (m, 4H), 0.84 (t, *J* = 7.1 Hz, 3H)。 | 466.2, $t_R$=1.174min |
| 366 | 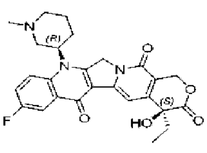 | (400 MHz, DMSO-$d_6$+$D_2O$) δ 8.31 (dd, *J* = 9.5 Hz, 4.0 Hz, 1H), 8.03 (dd, *J* = 8.8 Hz, 3.3 Hz, 1H), 7.71-7.66 (m, 1H), 7.30 (s, 1H), 5.65-5.30 (m, 4H), 4.69-4.62 (m, 1H), 3.98-4.12 (1H), 3.89-3.80 (m, 1H), 3.61-3.54 (m, 1H), 3.31-3.24 (m, 1H), 2.88 (s, 3H), 2.70-2.62 (m, 1H), 2.28-2.21 (m, 1H), 2.16-1.75 (m, 4H), 0.84 (t, *J* = 7.4 Hz, 3H)。 | 480.2, $t_R$=1.196min |
| 367 | 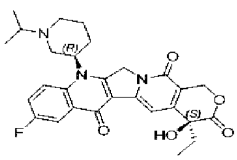 | (400 MHz, DMSO-$d_6$+$D_2O$) δ 8.41 (dd, *J* = 9.5 Hz, 4.0 Hz, 1H), 8.03 (dd, *J* = 8.8 Hz, 3.3 Hz, 1H), 7.69-7.64 (m, 1H), 7.31 (s, 1H), 5.61-5.30 (m, 4H), 4.79-4.68 (m, 1H), 3.92-3.82 (m, 2H), 3.63-3.51 (m, 1H), 3.40-3.31 (m, 1H), 2.77-2.65 (m, 1H), 2.33-2.25 (m, 1H), 2.18-1.96 (m, 2H), 1.88-1.72 (m, 3H), 1.31 (dd, *J* = 6.5, 2.6 Hz, 6H), 0.84 (t, *J* = 7.3 Hz, 3H)。 | 508.3, $t_R$=1.364min |
| 368 | 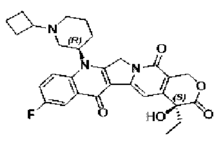 | (400 MHz, DMSO-$d_6$+$D_2O$) δ 8.28-8.23 (m, 1H), 7.99-7.96 (m, 1H), 7.68-7.62 (m, 1H), 7.27 (s, 1H), 5.58-5.43 (m, 2H), 5.39-5.27 (m, 2H), 4.64-4.54 (m, 1H), 3.92-3.78 (m, 2H), 3.51-3.44 (m, 1H), 3.08-3.00 (m, 1H), 2.73-2.64 (m, 1H), 2.35-1.71 (m, 12H), 0.83 (t, *J* = 7.2 Hz, 3H)。 | 520.4, $t_R$=1.237min |
| 369 | 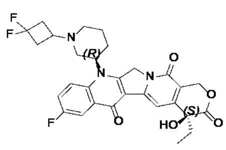 | (400 MHz, DMSO-$d_6$) δ 11.57 (br, 1H), 8.38-8.33 (m, 1H), 8.05-8.01 (m, 1H), 7.71-7.65 (m, 1H), 7.30 (s, 1H), 5.70-5.57 (m, 2H), 5.40-5.31 (m, 2H), 4.82-4.72 (m, 1H), 4.00-3.73 (m, 3H), 3.26-2.66 (m, 7H), 2.34-2.05 (m, 3H), 1.86-1.76 (m, 2H), 0.84 (t, *J* = 7.6 Hz, 3H)。 | 556.4, $t_R$=1.541min |

(continued)

| **Examples** | Structure | $^1$H NMR | LCMS (ESI) $[M+H]^+$ |
|---|---|---|---|
| 370 | 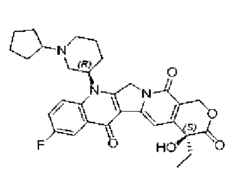 | (400 MHz, DMSO-$d_6$+ $D_2O$) δ 8.33 (dd, *J* = 9.6, 4.4 Hz, 1H), 7.98 (dd, *J* = 8.8, 3.2 Hz, 1H), 7.68-7.63 (m, 1H), 7.27 (s, 1H), 5.60-5.42 (m, 2H), 5.38-5.27 (m, 2H), 4.70-4.60 (m, 1H), 4.03-3.91 (m, 2H), 3.61-3.57 (m, 1H), 3.32-3.22 (m, 1H), 2.75-2.65 (m, 1H), 2.33-1.52 (m, 14H), 0.83 (t, *J* = 6.8 Hz, 3H)。 | 534.5, $t_R$=1.297min |
| 371 | 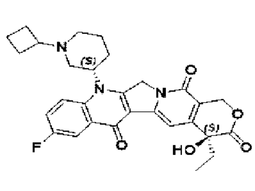 | (400 MHz, DMSO-$d_6$+ $D_2O$) δ 8.33 (dd, *J* = 9.6, 4.0 Hz, 1H), 8.03 (dd, *J* = 8.8, 3.2 Hz, 1H), 7.70-7.65 (m, 1H), 7.31 (s, 1H), 5.68-5.51 (m, 2H), 5.40-5.31 (m, 2H), 4.74-4.63 (m, 1H), 3.92-3.67 (m, 3H), 3.16-3.08 (m, 1H), 2.72-2.63 (m, 1H), 2.35-1.66 (m, 12H), 0.86 (t, *J* = 7.2 Hz, 3H)。 | 520.4, $t_R$=1.380min |
| 372 | 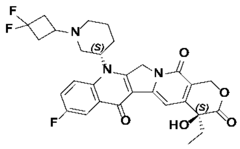 | (400 MHz, DMSO-$d_6$) δ 11.54 (br, 1H), 8.34-8.29 (m, 1H), 8.03-7.99 (m, 1H), 7.68-7.62 (m, 1H), 7.28 (s, 1H), 5.60 (s, 2H), 5.33 (s, 2H), 4.80-4.71 (m, 1H), 3.94-3.73 (m, 3H), 3.35-2.64 (m, 7H), 2.32-2.01 (m, 3H), 1.84-1.75 (m, 2H), 0.83 (t, J = 6.8 Hz, 3H)。 | 556.4, $t_R$=1.518min |
| 373 | 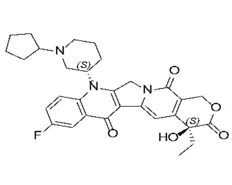 | (400 MHz, DMSO-$d_6$+$D_2O$) δ 8.33 (dd, *J* = 9.6, 4.4 Hz, 1H), 8.03 (dd, *J* = 8.8, 3.6 Hz, 1H), 7.70-7.65 (m, 1H), 7.31 (s, 1H), 5.66-5.44 (m, 2H), 5.40-5.30 (m, 2H), 4.69-4.60 (m, 1H), 4.05-3.92 (m, 2H), 3.64-3.58 (m, 1H), 3.31-3.20 (m, 1H), 2.75-2.65 (m, 1H), 2.24-1.51 (m, 14H), 0.85 (t, *J* = 7.2 Hz, 3H)。 | 534.4, $t_R$=1.408min |

Example 374: Preparation of (S)-11-((R)-1-cyclobutylpyrrolidin-3-yl)-4-ethyl-8-fluoro-4-hydroxy-9-(hydroxymethyl)-1,12-dihydro-14H-pyrano[3,4:6,7] indolizino[2,1-b]quinoline-3,6,14(4H,11H)-trione:

*Step 1: Preparation of tert-butyl (R,Z)-3-((4-(4-bromo-2,5-difluorophenyl)-4-oxobut-2-en-2-yl)amino)pyrrolidine-1-carboxylate:*

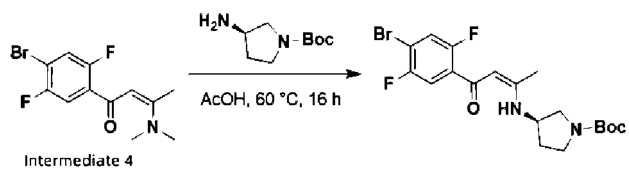


Intermediate 4

Intermediate 4 (21.0 g, 69.1 mmol, 1.0 eq.) was dissolved in acetic acid (220 mL), and tert-butyl (R)-3-aminopyrrolidine-1-carboxylate (12.9 g, 69.1 mmol, 1.0 eq.) was added. The mixture was stirred at 60 °C for 16 h. LCMS monitoring indicated the reaction was complete, then the reaction mixture was cooled to room temperature, diluted with water (800 mL) and extracted with ethyl acetate (500 mL × 3). The combined organic layers were washed with water (200 mL) and saturated aqueous sodium bicarbonate solution (800 mL) successively, dried over anhydrous sodium sulfate, filtered and concentrated, and purified by column chromatography (100-200 mesh silica gel, eluent: THF:petroleum ether = 1:3, v/v) to afford the title compound as a yellow solid (22.0 g, yield: 71.55%). LCMS (ESI): $[M+H]^+$ = 445.1.

*Step 2: Preparation of tert-butyl (R)-3-(6-fluoro-2-methyl-4-oxoquinolin-1(4H)-yl)pyrrolidine-1-carboxylate:*

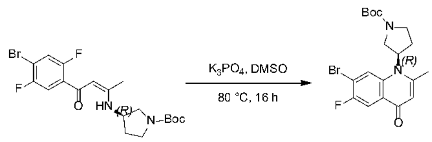


The intermediate obtained from step 1 (22.0 g, 49.4 mmol, 1.0 eq.) was dissolved in dimethyl sulfoxide (460 mL), and potassium phosphate (31.4 g, 148.2 mmol, 3.0 eq.) was added. The mixture was heated to 80 °C and stirred for 16 h. LCMS monitoring indicated the reaction was complete, then the reaction mixture was poured into ice water (1500 mL). The precipitated solid was collected by suction filtration, rinsed with water (200 mL × 3) and concentrated, and slurried with methyl tert-butyl ether (200 mL) to afford the title compound as a yellow solid (13.5 g, yield: 64.26%). LCMS (ESI): $[M+H]^+$ = 425.2, $t_R$ = 1.327 min.

*Step 3: Preparation of tert-butyl (R)-3-(6-fluoro-2-methyl-4-oxo-7-vinylquinolin-1(4H)-yl)pyrrolidine-1-carboxylate:*

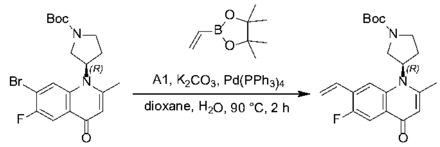


The intermediate obtained from step 2 (15.0 g, 35.27 mmol, 1.0 eq.) was dissolved in a mixture of 1,4-dioxane (150 mL) and water (15 mL). Vinyl boronate (9.05 g, 52.91 mmol, 1.5 eq.), potassium carbonate (14.62 g, 105.81 mmol, 3.0 eq.) and tetrakis(triphenylphosphine)palladium (2.03 g, 1.76 mmol, 0.05 eq.) were added. The system was purged with nitrogen three times, then the mixture was slowly warmed to 90 °C and stirred for 2 h. LCMS monitoring indicated the reaction was complete, then the reaction mixture was poured into water (400 mL) and extracted with DCM (150 mL × 3) and isopropanol (50 mL × 3). The combined organic phases were washed with saturated sodium chloride solution (200 mL), dried over anhydrous sodium sulfate, filtered and concentrated, and purified by flash column chromatography (100-200 mesh silica gel, eluent: DCM:MeOH = 1:0 to 30:1, v/v) afforded the title compound as a yellow solid (12.0 g, yield: 91.35%). LCMS (ESI): $[M+H]^+$ = 373.2, $t_R$ = 1.260 min.

*Step 4: Preparation of tert-butyl (R)-3-(6-fluoro-7-formyl-2-methyl-4-oxoquinolin-1(4H)-yl)pyrrolidine-1-carboxylate:*

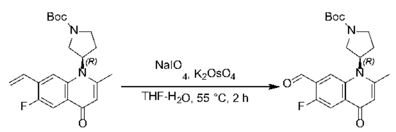


The intermediate obtained from step 3 (12.0 g, 32.31 mmol, 1.0 eq.) was dissolved in a mixture of THF (200 mL) and water (50 mL). Sodium periodate (20.73 g, 96.92 mmol, 3.0 eq.) and potassium osmate dihydrate (1.07 g, 3.23 mmol, 0.1 eq.) were added, then the mixture was heated to 55 °C and stirred for 2 h. LCMS monitoring indicated the reaction was complete, then the mixture was poured into water (400 mL) and extracted with EA (200 mL×2). The combined organic phases were washed with saturated sodium chloride solution (100 mL × 3), dried over anhydrous sodium sulfate, filtered and concentrated, and purified by flash column chromatography (100-200 mesh silica gel, eluent: DCM:MeOH = 1:0 to 40:1, v/v) afforded the title compound as a brown solid (10.0 g, 26.78 mmol, yield: 82.88%). LCMS (ESI): $[M+H]^+$ = 375.2, $t_R$ = 1.183 min.

*Step 5: Preparation of tert-butyl (R)-3-(6-fluoro-7-(hydroxymethyl)-2-methyl-4-oxoquinolin-1(4H)-yl)pyrrolidine-1-carboxylate:*

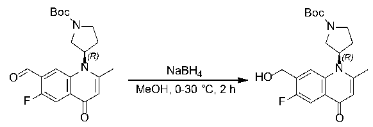


The intermediate obtained from step 4 (3.0 g, 8.01 mmol, 1.0 eq.) was dissolved in MeOH (50 mL). Sodium borohydride (0.61 g, 16.03 mmol, 2.0 eq.) was added portionwise under an ice bath, then the mixture was stirred at room temperature for 2 h after the addition. LCMS monitoring indicated the reaction was complete, and the reaction was quenched by the addition of saturated ammonium chloride solution (10 mL). The reaction mixture was poured into water (200 mL) and extracted with ethyl acetate (50 mL × 3). The combined organic phases were washed with saturated sodium chloride solution (100 mL), dried over anhydrous sodium sulfate, filtered and concentrated, and purified by flash column chromatography (100-200 mesh silica gel, eluent: DCM:MeOH = 1:0 to 30:1, v/v) afforded the title compound as a pale yellow solid (2.4 g, yield: 79.57%). LCMS (ESI): $[M+H]^+$ = 377.2, $t_R$ = 1.093 min.

*Step 6: Preparation of tert-butyl (R)-3-(3-bromo-2-(bromomethyl)-6-fluoro-7-(hydroxymethyl)-4-oxoquinolin-1(4H)-yl) pyrrolidine-1-carboxylate:*

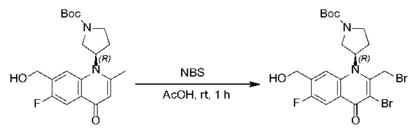


The intermediate obtained from step 5 (2.4 g, 6.38 mmol, 1.0 eq.) was dissolved in acetic acid (50 mL), and N-bromosuccinimide (2.38 g, 13.39 mmol, 2.1 eq.) was added. The mixture was stirred at room temperature for 1 h, and LCMS monitoring indicated the reaction was complete. The mixture was diluted with water (200 mL) and extracted with ethyl acetate (50 mL × 3). The combined organic phases were washed with saturated sodium bicarbonate solution (200 mL × 3), dried over anhydrous sodium sulfate and concentrated, and purified by flash column chromatography (100-200 mesh silica gel, eluent: DCM:MeOH = 1:0 to 35:1, v/v) afforded the title compound as a yellow solid (2.4 g, yield: 70.42%). LCMS (ESI): $[M+H]^+$ = 535.3, $t_R$ = 1.287 min.

*Step 7: Preparation of tert-butyl (R)-3-(3-bromo-2-(((S)-4-ethyl-4-hydroxy-3,8-dioxo-4,8-dihydro-1H-pyrano[3,4-c]pyridin-7(3H)-yl)methyl)-6-fluoro-7-(hydroxy methyl)-4-oxoquinolin-1(4H)-yl)pyrrolidine-1-carboxylate:*

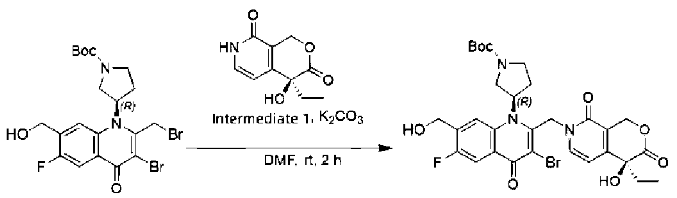


The intermediate obtained from step 6 (1.4 g, 2.62 mmol, 1.0 eq.) was dissolved in DMF (25 mL). Intermediate 1 (603 mg, 2.88 mmol, 1.1 eq.) and potassium carbonate (725 mg, 5.25 mmol, 2.0 eq.) were added, and the mixture was stirred at room temperature for 2 h. LCMS monitoring indicated the reaction was complete, then the pH was adjusted to 6~7. The mixture was diluted with water (100 mL) and extracted with ethyl acetate (20 mL × 3). The combined organic phases were washed with saturated sodium chloride solution (50 mL × 2) to remove residual N,N-dimethylformamide, dried over anhydrous sodium sulfate and concentrated, and purified by flash column chromatography (100-200 mesh silica gel, eluent: DCM:MeOH = 1:0 to 25:1, v/v) afforded the title compound as a white solid (820 mg, yield: 47.25%). LCMS (ESI): $[M+H]^+$ = 663.8.

*Step 8: Preparation of tert-butyl (R)-3-((S)-4-ethyl-8-fluoro-4-hydroxy-9-(hydroxymethyl)-3,6,14-trioxo-4,6,12,14-tetrahydro-1H-pyrano[3,4:6,7] indolizino[2,1-b]quinolin-11(3H)-yl)pyrrolidine-1-carboxylate:*

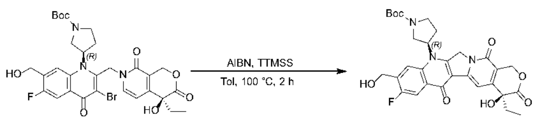


The intermediate obtained from step 7 (820 mg, 1.24 mmol, 1.0 eq.) was dissolved in toluene (20 mL). AIBN (203 mg, 1.24 mmol, 1.0 eq.) and tris(trimethylsilyl)silane (1.23 g, 4.95 mmol, 4.0 eq.) were added, and the mixture was stirred at 100 °C for 2 h. LCMS monitoring indicated the reaction was complete, then the reaction mixture was concentrated, and purified by flash column chromatography (100-200 mesh silica gel, eluent: DCM:MeOH = 1:0 to 25:1, v/v) afforded the title compound as a pale yellow solid (88 mg, yield: 12.2%). LCMS (ESI): $[M+H]^+$ = 582.0.

*Step 9: Preparation of (S)-4-ethyl-8-fluoro-4-hydroxy-9-(hydroxymethyl)-11-((R)-pyrrolidin-3-yl)-1,12-dihydro-14H-pyrano[3,4:6,7]indolizino[2,1 -b]quinoline-3,6,14(4H,11H)-trione*

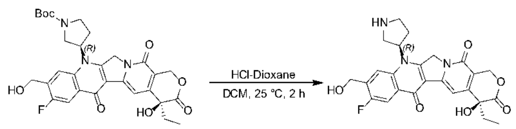


The intermediate obtained from step 8 (80 mg, 0.14 mmol, 1.0 eq.) was dissolved in DCM (1 mL), and 4 M hydrogen chloride in 1,4-dioxane (1 mL) was added. The mixture was stirred at 25 °C for 2 h, and LCMS monitoring indicated the reaction was complete. The reaction mixture was concentrated to afford the title compound as a pale yellow solid (65 mg, yield: 98.15%). LCMS (ESI): $[M+H]^+$ = 482.3, $t_R$ = 1.257 min.

*Step 10: Preparation of (S)-11-((R)-1-cyclobutylpyrrolidin-3-yl)-4-ethyl-8-fluoro-4-hydroxy-9-(hydroxymethyl)-1,12-dihydro-14H-pyrano[3,4:6,7]indolizino[2,1 -b]quinoline-3,6,14(4H,11H)-trione:*

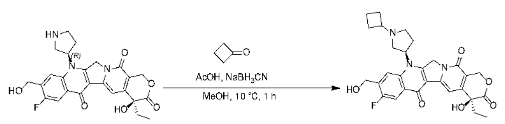


The intermediate obtained from step 9 (30 mg, 0.06 mmol, 1.0 eq.) was dissolved in MeOH (1 mL). Cyclobutanone (43.67 mg, 0.62 mmol, 10.0 eq.) and acetic acid (7.48 mg, 0.12 mmol, 2.0 eq.) were added, the system was purged with nitrogen twice, then the mixture was cooled to 10 °C and stirred for 0.5 h. Sodium cyanoborohydride (7.71 mg, 0.14 mmol, 2.0 eq.) was added, and stirring was continued for an additional 0.5 h. LCMS monitoring indicated the reaction was complete. Two drops of 1 M hydrochloric acid solution were added dropwise to the reaction mixture, and the product was purified by Prep-HPLC (C18 column, 0.01% aqueous HCl, acetonitrile as eluent) to afford the title compound as a yellow solid (10.6 mg, yield: 32.99%). LCMS (ESI): $[M+H]^+$ = 536.3, $t_R$= 1.234 min; $^1$H NMR (400 MHz, DMSO-$d_6$) δ: 7.94 (d, J = 9.9 Hz, 1H), 7.82-7.68 (m, 1H), 7.24 (s, 1H), 5.28 (dd, J = 29.8, 15.8 Hz, 2H), 4.75 (s, 2H), 4.11-3.79 (m, 4H), 3.68-3.50 (m, 2H), 2.83-2.61 (m, 2H), 2.49 (s, 2H), 2.30-2.12 (m, 4H), 1.85-1.69 (m, 4H), 0.79 (t, J = 7.3 Hz, 3H).

Examples 375-385:

Similarly, using the intermediate obtained from Step 9, **Example 375** was synthesized following the procedure of Step 10 in Example 374.

Similarly, using tert-butyl (R)-3-(6-fluoro-7-formyl-2-methyl-4-oxoquinolin-1(4H)-yl)pyrrolidine-1-carboxylate (the intermediate obtained from step 4 of Example 374) as the starting material, **Example 376** was synthesized following the synthetic methods of Example 301 and Example 374.

Similarly, using tert-butyl (R)-3-(6-fluoro-7-formyl-2-methyl-4-oxoquinolin-1(4H)-yl)pyrrolidine-1-carboxylate (the intermediate obtained from step 4 of Example 374) as the starting material, **Examples 377-382** were synthesized following the synthetic methods of Example 293 and Example 374.

Similarly, using Intermediate 4 from Preparation Example 4 and commercially available tert-butyl (R)-3-aminopiperidine-1-carboxylate as the starting materials, **Examples 383 and 384** were synthesized following the procedures of

steps 1~10 in Example 374.

Similarly, using Intermediate 4 from Preparation Example 4 and commercially available tert-butyl (2-aminoethyl) carbamate as the starting materials, **Example 385** was synthesized following the procedures of steps 1~10 in Example 374.

The chemical structures and detailed characterization data (LCMS and $^1$H NMR) of the compounds in Examples 375-385 are shown below:

| Examples | structure | $^1$H NMR | LCMS (ESI) $[M+H]^+$ |
|---|---|---|---|
| 375 | 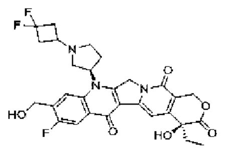 | (400 MHz, DMSO-$d_6$) δ 8.20 (s, 1H), 7.97 (d, J = 9.9 Hz, 1H), 7.28 (s, 1H), 5.53-5.29 (m, 4H), 4.83-4.75 (m, 2H), 3.96-3.63 (m, 4H), 3.40-3.19 (m, 1H), 3.15-2.91 (m, 4H), 2.79-2.65 (m, 2H), 1.86-1.76 (m, 2H), 0.84 (t, J = 7.3 Hz, 3H)。 | 572.4, $t_R$=1.65 3min |
| 376 | 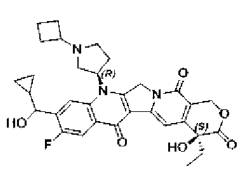 | (300 MHz, DMSO-$d_6$ +$D_2O$) δ 8.08-7.95 (m, 1H), 7.94-7.76 (m, 1H), 7.29 (s, 1H), 5.66-5.44 (m, 2H), 5.42-5.30 (m, 2H), 4.57-4.50 (m, 1H), 4.12-3.92 (m, 2H), 3.91-3.76 (m, 2H), 3.72-3.56 (m, 1H), 2.90-2.70 (m, 2H), 2.40-2.18 (m, 4H), 1.90-1.74 (m, 4H), 1.42-1.22 (m, 1H), 0.86 (t, *J* = 7.2 Hz, 3H), 0.58-0.38 (m, 2H)。 | 576.4, $t_R$=1.39 1min |
| 377 | 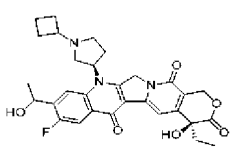 | (400 MHz, DMSO-$d_6$) δ 11.98 (brs., 0.4H), 11.81 (brs., 0.6H), 7.99 (d, J = 9.9 Hz, 1H), 7.95-7.79 (m, 1H), 7.30 (s, 1H), 6.37 (brs., 0.6H), 5.98 (brs., 0.4H), 5.71-5.52 (m, 2H), 5.41-5.15 (m, 4H), 4.16-3.84 (m, 3H), 3.70-3.53 (m, 1H), 3.28-3.13 (m, 1H), 2.85-2.65 (m, 2H), 2.42-2.13 (m, 4H), 1.92-1.75 (m, 4H), 1.54-1.46 (m, 3H), 0.99 (d, J = 6.3 Hz, 1H), 0.85 (t, J = 7.4 Hz, 3H)。 | 550.4, $t_R$=1.28 7min |
| 378 | 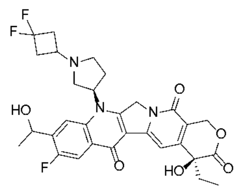 | (400 MHz, DMSO-$d_6$+$D_2O$) δ 8.76-8.51 (m, 1H), 7.94 (dd, *J* = 15.9, 5.8 Hz, 1H), 7.28 (s, 1H), 5.38-5.23 (m, 3H), 5.15-5.11 (m, 1H), 3.52-3.42 (m, 5H), 3.29-3.27 (m, 1H), 2.96-2.76 (m, 4H), 2.67-2.56 (m, 1H), 2.51-2.55 (m, 1H), 1.86-1.75 (m, 2H), 1.52-1.51 (m, 1H), 1.45 (t, *J* = 7.0 Hz, 3H), 0.86-0.79 (t, *J* = 7.0 Hz, 3H)。 | 586.3, $t_R$=1.73 8min |
| 379 | 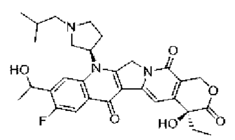 | (400 MHz, DMSO-$d_6$+$D_2O$) δ 7.99-7.95 (m, 1H), 7.88-7.72 (m, 1H), 7.29 (s, 1H), 5.48-5.22 (m, 3H), 5.22-5.12 (m, 1H), 4.27-3.98 (m, 1H), 3.73-3.71 (m, 2H), 3.63-3.61 (m, 3H), 3.27-3.15 (m, 2H), 2.87-2.70 (m, 2H), 2.03-1.95 (m, 1H), 1.88-1.74 (m, 2H), 1.53-1.37 (m, 3H), 1.08-0.95 (m, 6H), 0.89-0.77 (m, 3H)。 | 552.4, $t_R$=1.33 7min |
| 380 | 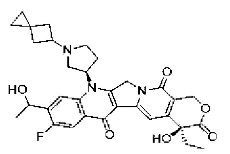 | (400 MHz, DMSO-$d_6$+$D_2O$)δ 8.00-7.95 (m, 1H), 7.82 (s, 1H), 7.30 (s, 1H), 5.40-5.25 (m, 3H), 5.19-5.15 (m, 1H), 4.55-4.48 (m, 1H), 4.28-4.22 (m, 1H), 4.05 (s, 1H), 3.82-3.64 (m, 1H), 3.39 -3.24(m, 2H), 2.79-2.67 (m, 2H), 2.54-2.53 (m, 1H), 2.37-2.27 (m, 3H), 1.81-1.80 (m, 2H), 1.51-1.45 (m, 4H), 0.86-0.79 (m, 3H), 0.53-0.44 (m, 4H)。 | 576.4, $t_R$=1.41 3min |
| 381 | 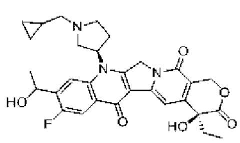 | (400 MHz, DMSO-$d_6$+$D_2O$) δ 8.04-8.0 (m, 1H), 7.87-7.78 (m, 1H), 7.33 (s, 1H), 5.52-5.29 (m, 2H), 5.29-5.17 (m, 2H), 4.53 (q, *J* = 7.0 Hz, 2H), 3.37-3.17 (m, 1H), 2.90-2.74 (m, 1H), 1.86-1.79 (m, 2H), 1.55-1.54 (m, 6H), 1.49-1.48 (m, 3H), 0.91-0.85 (m, 4H), 0.70-0.64 (m, 2H), 0.51-0.39 (m, 2H)。 | 550.4, $t_R$=1.28 3min |

(continued)

| Examples | structure | $^1$H NMR | LCMS (ESI) $[M+H]^+$ |
|---|---|---|---|
| 382 | 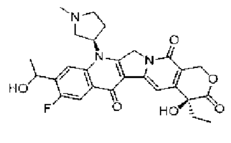 | (400 MHz, DMSO-$d_6$) δ 11.76-11.70(m, 1H), 7.99-7.86 (m, 2H), 7.29 (s, 1H), 5.68-5.47 (m, 2H), 5.40-5.31 (m, 2H), 5.27-5.16 (m, 1H), 4.13-3.88 (m, 2H), 3.82-3.59 (m, 4H), 3.06-2.98 (m, 3H), 2.92-2.66 (m, 2H), 1.87-1.76 (m, 2H), 1.56-1.36 (m, 4H), 0.85 (t, J = 7.3 Hz, 3H)。 | 510.2, $t_R$=0.98 0min |
| 383 | 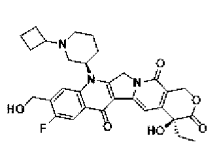 | (400 MHz, DMSO-$d_6$+$D_2O$) δ 8.06-8.05 (m, 1H), 8.00-7.96 (m, 1H), 7.31 (s, 1H), 5.64-5.41 (m, 2H), 5.37-5.30 (m, 2H), 4.77 (d, *J* = 6.9 Hz, 2H), 4.61-4.50 (m, 1H), 3.93-3.76 (m, 2H), 3.05-2.99 (m, 1H), 2.73-2.66 (m, 1H), 2.33-2.31 (m, 3H), 2.26-2.16 (m, 3H), 2.05-1.96 (m, 1H), 1.85-1.71 (m, 4H), 1.52 (d, *J* = 7.1 Hz, 1H), 0.84 (q, *J* = 6.9 Hz, 4H)。 | 550.3, $t_R$=1.23 7min |
| 384 | 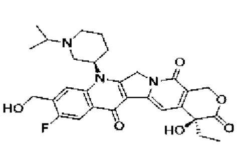 | (400 MHz, DMSO-$d_6$+$D_2O$) δ 8.06-8.04 (m, 1H), 8.00-7.95 (m, 1H), 7.31 (s, 1H), 5.39-5.29(m, 3H), 4.76-4.69 (m, 3H), 3.96-3.72 (m, 3H), 3.16-3.14 (m, 1H), 2.69-2.50 (m, 2H), 2.32-2.31 (m, 1H), 2.18-1.97 (m, 2H), 1.84-1.75 (m, 2H), 1.52-1.51 (m, 1H), 1.34-1.26 (m, 6H), 0.82 (t, *J* = 7.1 Hz, 3H)。 | 538.4, $t_R$=1.21 2min |
| 385 | 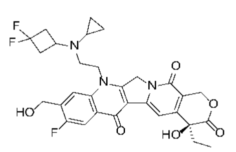 | (400 MHz, DMSO-$d_6$) δ 8.00 - 7.87 (m, 2H), 7.29 (s, 1H), 6.37 (s, 1H), 5.70 (t, *J* = 5.4 Hz, 1H), 5.49 - 5.30 (m, 4H), 4.82 - 4.72 (m, 2H), 4.58 - 4.52 (m, 1H), 3.07 - 3.01 (m, 1H), 2.80 - 2.67 (m, 2H), 2.06 - 1.98 (m, 3H), 1.86 - 1.77 (m, 2H), 1.50 - 1.41 (m, 2H), 0.91 - 0.81 (m, 4H), 0.57 - 0.49 (m, 2H), 0.39 - 0.30 (m, 2H)。 | 586.3, $t_R$=1.51 2min |

**Example 386: Preparation of (4S)-10-(2-cyclopropyl-1-hydroxyethyl)-4-ethyl-8-fluoro-4-hydroxy-11-methyl-1,12-dihydro-14H-pyrano[3',4':6,7]indolizino[2,1-b]quinoline-3,6,14(4H,11H)-trione:**

***Step 1: Preparation of ethyl 8-(2-cyclopropyl-1-hydroxyethyl)-6-fluoro-1-methyl-4-oxo-1,4-dihydroquinoline-2-carboxylate:***

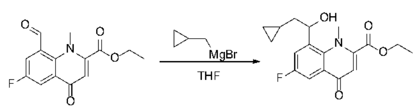


The intermediate obtained from step 2 of Example 84 (1.2 g, 4.33 mmol, 1.0 eq.) was dissolved in anhydrous THF (5 mL). Under a nitrogen atmosphere and an ice bath, 1 mol/L cyclopropylmethylmagnesium bromide (6.5 mL, 6.50 mmol, 1.5 eq.) was added dropwise slowly. After the addition, the mixture was warmed to room temperature naturally and stirred for 1 h. LCMS monitoring indicated the reaction was complete. The reaction mixture was poured into ice-cold saturated ammonium chloride solution (30 mL) and extracted with ethyl acetate (20 mL × 3). The combined organic phases were dried over anhydrous sodium sulfate, filtered, concentrated and purified by silica gel column chromatography (eluent: petroleum ether:ethyl acetate = 1:0 to 1:1, v/v) to yield the title compound as a pale yellow solid (320 mg, yield: 22.22%). LCMS (ESI): $[M+H]^+$ = 334.2, $t_R$ = 0.365 min.

***Step 2: Preparation of ethyl 8-(2-cyclopropyl-1-((tetrahydro-2H-pyran-2-yl)oxy)ethyl)-6-fluoro-1-methyl-4-oxo-1,4-dihydroquinoline-2-carboxylate:***

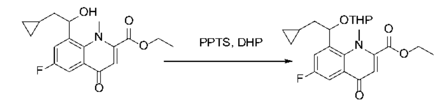


The intermediate obtained from step 1 (320 mg, 0.96 mmol, 1.0 eq.) was dissolved in anhydrous THF (5 mL). Then 3,4-dihydropyran (323 mg, 3.844 mmol, 4.0 eq.) and pyridinium p-toluenesulfonate (10 mg, 0.038 mmol, 0.04 eq.) were added sequentially. Under a nitrogen atmosphere, the mixture was heated to 50 °C and stirred for 16 h. LCMS monitoring indicated the reaction was complete. The reaction mixture was concentrated and purified by silica gel column chromatography (eluent: DCM:MeOH = 1:0 to 50:1, v/v) to yield the title compound as a pale yellow solid (360 mg, yield: 55.55%). LCMS (ESI): $[M+H]^+$ = 418.2, $t_R$ = 1.679 min.

***Step 3: Preparation of 8-(2-cyclopropyl-1-((tetrahydro-2H-pyran-2-yl)oxy)ethyl)-6-fluoro-2-(hydroxymethyl)-1-methylquinolin-4(1H)-one:***

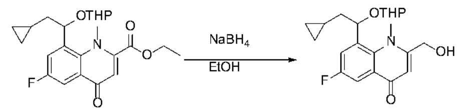


The intermediate obtained from step 2 (360 mg, 0.86 mmol, 1.0 eq.) was dissolved in anhydrous ethanol (10 mL). Under an ice bath, sodium borohydride (82 mg, 2.16 mmol, 2.5 eq.) was added slowly. After the addition, the mixture was stirred at room temperature for 1 h. LCMS monitoring indicated the reaction was complete. The reaction mixture was concentrated, and purified by silica gel column chromatography (eluent: DCM:MeOH = 1:0 to 20:1, v/v) to yield the title compound as a white solid (177 mg, yield: 54.80%). LCMS (ESI): $[M+H]^+$ = 376.3, $t_R$ = 1.698 min.

***Step 4: Preparation of 3-bromo-8-(2-cyclopropyl-1-((tetrahydro-2H-pyran-2-yl)oxy)ethyl)-6-fluoro-2-(hydroxymethyl)-1-methylquinolin-4(1H)-one:***

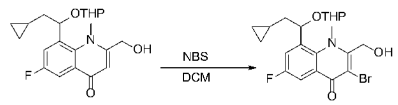


The intermediate obtained from step 3 (177 mg, 0.47 mmol, 1.0 eq.) was dissolved in DCM (5 mL). Under an ice bath, NBS (88.0 mg, 0.49 mmol, 1.05 eq.) was added slowly, and stirring was continued for 1 h. LCMS monitoring indicated the reaction was complete. The reaction mixture was poured into ice-cold saturated aqueous sodium bicarbonate solution (20 mL) and extracted with DCM (5 mL × 3). The combined organic phases were dried over anhydrous sodium sulfate, filtered, and the filtrate was used directly in the next step. LCMS (ESI): $[M+H]^+$ = 454.2/456.0, $t_R$ = 1.835 min.

***Step 5: Preparation of 3-bromo-2-(chloromethyl)-8-(2-cyclopropyl-1-((tetrahydro-2H-pyran-2-yl)oxy)ethyl)-6-fluoro-1-methylquinolin-4(1H)-one:***

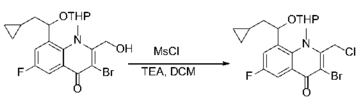


To the filtrate obtained from Step 4, N,N-diisopropylethylamine (124 mg, 0.96 mmol, 3.0 eq.) and methanesulfonyl chloride (73 mg, 0.64 mmol, 2.0 eq.) were added slowly sequentially under an ice bath. After the addition, stirring was continued for 1 h under the ice bath. LCMS monitoring indicated the reaction was complete. The reaction mixture was poured into ice water (15 mL) and extracted with DCM (10 mL × 2). The combined organic phases were dried over anhydrous sodium sulfate, filtered and concentrated under reduced pressure to afford a crude mixture of the OMs-substituted product and chlorinated product (155 mg), which was used directly in the next step. LCMS (ESI):

[M+H]+=532.0/534.0, $t_R$=1.532 min; [M+H]+=472.0/474.0, $t_R$=1.735 min.

***Step 6: Preparation of (4S)-7-((3-bromo-8-(2-cyclopropyl-1-((tetrahydro-2H-pyran-2-yl)oxy)ethyl)-6-fluoro-1-methyl-4-oxo-1,4-dihydroquinolin-2-yl)methyl)-4-ethyl-4-hydroxy-1,7-dihydro-3H-pyrano[3,4-c]pyridine-3,8(4H)-dione:***

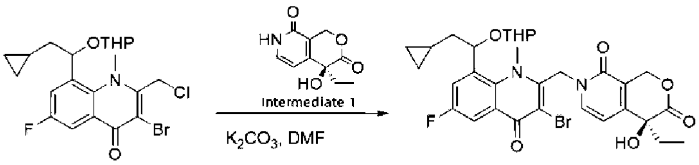


The crude mixture of the OMs-substituted product and chlorinated product obtained from Step 5 (155 mg, 0.32 mmol, 1.0 eq.) was dissolved in DMF (2 mL). Then Intermediate 1 (67 mg, 0.32 mmol, 1.0 eq.) and potassium carbonate (133 mg, 0.96 mmol, 3.0 eq.) were added sequentially. Under a nitrogen atmosphere, the mixture was heated to 30 °C and stirred for 16 h. LCMS monitoring indicated the reaction was complete. The reaction mixture was filtered, and the filtrate was poured into ice-cold saturated aqueous ammonium chloride solution (20 mL) and extracted with ethyl acetate (15 mL × 3). The combined organic phases were washed with saturated aqueous sodium chloride solution (20 mL), dried over anhydrous sodium sulfate, filtered and concentrated under, and purified by silica gel column chromatography (eluent: petroleum ether:ethyl acetate = 1:0 to 100:1, v/v) to yield the title compound as a white solid (140 mg, yield: 56.1%). LCMS (ESI): [M+H]+ = 645.3/647.3, $t_R$ = 1.833 min.

***Step 7: Preparation of (4S)-10-(2-cyclopropyl-1-hydroxyethyl)-4-ethyl-8-fluoro-4-hydroxy-11-methyl-1,12-dihydro-14H-pyrano[3',4':6,7]indolizino[2,1-b]quinoline -3,6,14(4H,11H)-trione:***

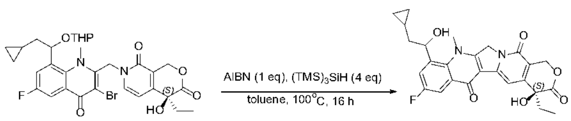


The intermediate obtained from step 6 (140 mg, 0.22 mmol, 1.0 eq.) was dissolved in toluene (14 mL). Then AIBN (36 mg, 0.22 mmol, 1.0 eq.) and tris(trimethylsilyl)silane (216 mg, 0.87 mmol, 4.0 eq.) were added sequentially. The system was purged with nitrogen three times, and under a nitrogen atmosphere, the mixture was heated to 100 °C and stirred for 16 h. LCMS monitoring indicated the reaction was complete. The reaction mixture was concentrated to afford a crude product, which was purified by silica gel column chromatography (eluent: DCM:MeOH = 1:0 to 20:1, v/v) to yield the title compound as a white solid (2.3 mg, yield: 2.21%). LCMS (ESI): [M+H]+ = 481.3, $t_R$=1.465 min; $^1$H NMR (400 MHz, DMSO-$d_6$) δ: 7.92 (m, 2H), 7.24 (s, 1H), 6.36 (s, 1H), 5.88-5.74 (m, 2H), 5.42-5.35 (m, 1H), 5.37-5.28 (m, 3H), 5.01-4.88 (m, 2H), 4.07 (s, 3H), 2.11-1.94 (m, 3H), 1.88-1.77 (m, 2H), 1.29-1.24 (m, 2H), 0.86-0.82 (m, 3H).

**Preparation of Compounds in Examples 387-389:**

Similarly, using methyl 6-fluoro-8-formyl-1-methyl-4-oxo-1,4-dihydroquinoline-2-carboxylate (the intermediate obtained from Step 2 of Example 84) as the starting material and reacting it with various Grignard reagents, the compounds in Examples 387-389 were synthesized following the synthetic method of Example 386.

The chemical structures and detailed characterization data (LCMS and $^1$H NMR) of the specific compounds are shown below:

| Examples | structure | $^1$H NMR | LCMS (ESI) [M+H]+ |
|---|---|---|---|
| 387 | 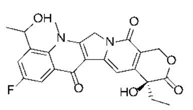 | (400 MHz, DMSO-$d_6$) δ 7.99 - 7.88 (m, 2H), 7.25 (s, 1H), 5.61 - 5.22 (m, 5H), 4.20 (s, 1H), 4.11 (s, 3H), 1.89 - 1.78 (m, 2H), 1.48 (d, $J$ = 6.3 Hz, 3H), 0.87 (t, $J$ = 6.4 Hz, 3H). | 441.3, $t_R$=1.143min |

(continued)

| Examples | structure | $^{1}$H NMR | LCMS (ESI) [M+H]$^+$ |
|---|---|---|---|
| 388 | 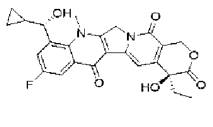 Isomer 1 or isomer 2. | (400 MHz, DMSO-$d_6$) δ 8.04 - 7.93 (m, 2H), 7.24 (s, 1H), 5.43 - 5.37 (m, 2H), 5.36 - 5.28 (m, 2H), 4.80 (d, J = 6.4 Hz, 1H), 4.18 (s, 3H), 1.89 - 1.77 (m, 2H), 1.39 - 1.30 (m, 1H), 0.87 (t, J = 7.3 Hz, 3H), 0.62 - 0.54 (m, 2H), 0.49 - 0.39 (m, 1H), 0.29 - 0.17 (m, 1H). | 467.3, $t_R$=1.421min |
| 389 | 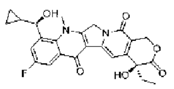 Isomer 2 or isomer 1. | (400 MHz, DMSO-$d_6$)δ 8.05 - 7.95 (m, 2H), 7.27 (s, 1H), 6.65 (s, 1H), 5.53 - 5.14 (m, 4H), 4.17 (s, 3H), 1.88 - 1.79 (m, 2H), 1.52 - 1.42 (m, 1H), 0.87 (t, J = 5.6 Hz, 3H), 0.60 - 0.51 (m, 2H), 0.49 - 0.36 (m, 1H), 0.29 - 0.17 (m, 1H). | 467.3, $t_R$=1.354min |

**Example 390: Preparation of (5S)-17-(2-aminoethyl)-14-(4,4-dimethylcyclohexyl)-5-ethyl-18-fluoro-5-hydroxy-7-oxa-11,14-diazapentacyclo[11.8.0.0$^{2,11}$.0$^{4,9}$.0$^{15,20}$]heneicosa-1(13),2,4(9),15(20),16,18-hexaene-6,10,21-trione:**

***Step 1: Preparation of tert-butyl N-{2-[1-(4,4-dimethylcyclohexyl)-6-fluoro-2-methyl-4-oxo-1,4-dihydroquinolin-7-yl]ethyl}carbamate:***

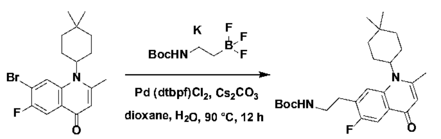


Under a $N_2$ atmosphere, Intermediate 14 (2.0 g, 5.46 mmol, 1.0 eq.) was dissolved in a mixture of 1,4-dioxane (20 mL) and water (2 mL). Potassium (2-((tert-butoxycarbonyl)amino)ethyl)trifluoroborate (9.77 g, 32.76 mmol, 6.0 eq.), potassium carbonate (5.34 g, 16.38 mmol, 3.0 eq.) and [1,1'-bis(diphenylphosphino)ferrocene]dichloropalladium(II) (0.36 g, 0.55 mmol, 0.1 eq.) were added sequentially. After the addition, the mixture was stirred at 90 °C for 12 h. LCMS monitoring indicated the reaction was complete, then the reaction mixture was cooled to room temperature, quenched by pouring into ice water (30 mL) and extracted with ethyl acetate (50 mL × 3). The combined organic phases were washed with saturated aqueous sodium chloride solution (20 mL × 2), dried over anhydrous sodium sulfate and concentrated, and purified by column chromatography (silica gel, eluent: petroleum ether:tetrahydrofuran=1:5, v/v) to afford the title compound as a yellow solid (1.1 g, 2.55 mmol, yield: 46.79%). LCMS (ESI): [M+H]$^+$ = 431.4, $t_R$= 1.363 min.

***Step 2: Preparation of tert-butyl N-{2-[3-bromo-2-(bromomethyl)-1-(4,4-dimethylcyclohexyl)-6-fluoro-4-oxo-1,4-dihydroquinolin-7-yllethyl}carbamate:***

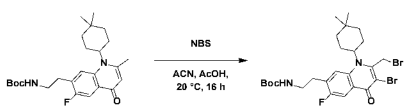


The intermediate obtained from step 1 (1.0 g, 2.32 mmol, 1.0 eq.) was dissolved in a mixed solvent of acetonitrile (10 mL) and acetic acid (3 mL), and NBS (1.34 g, 6.97 mmol, 3.0 eq.) was added. After the addition, the mixture was stirred at 20 °C for 16 h. LCMS monitoring indicated the reaction was complete, then the reaction mixture was quenched with saturated aqueous sodium chloride solution (50 mL) and extracted with ethyl acetate (50 mL × 3).

The combined organic phases were washed with saturated aqueous sodium chloride solution (50 mL × 2), dried over anhydrous sodium sulfate and concentrated, and purified by column chromatography (silica gel, eluent: petroleum ether:tetrahydrofuran = 1:3, v/v) to afford the title compound as a yellow solid (950 mg, yield: 69.52%). LCMS (ESI): [M+H]$^+$ = 587.3, $t_R$ = 1.537 min.

***Step 3: Preparation of tert-butyl N-{2-[3-bromo-1-(4,4-dimethylcyclohexyl)-2-{[(4S)-4-ethyl-4-hydroxy-3,8-dioxo-1H,3H,4H,7H,8H-pyrano[3,4-c]pyridin-7-yl]methyl}-6-fluoro-4-oxo-1,4-dihydroquinolin-7-yl]ethyl}carbamate:***

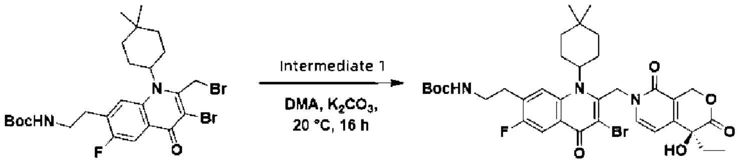


The intermediate obtained from step 2 (950 mg, 1.61 mmol, 1.0 eq.) was dissolved in DMF (10 mL). Intermediate 1 (405 mg, 1.94 mmol, 1.2 eq.) and potassium carbonate (670 mg, 4.84 mmol, 3.0 eq.) were added sequentially. After the addition, the mixture was stirred at 20 °C for 16 h. LCMS monitoring indicated the reaction was complete, then the reaction mixture was quenched by pouring into saturated aqueous ammonium chloride solution (50 mL) and extracted with ethyl acetate (50 mL × 3). The combined organic phases were washed with saturated aqueous sodium chloride solution (20 mL × 3), dried over anhydrous sodium sulfate and concentrated, and purified by column chromatography (silica gel, eluent: petroleum ether:tetrahydrofuran = 1:1, v/v) to afford the title compound as a yellow solid (830 mg, yield: 71.73%). LCMS (ESI): $[M+H]^+$ = 716.2, = 1.383 min; $^1H$ NMR (400 MHz, DMSO-$d_6$) δ: 7.90-7.80 (m, 2H), 7.31 (s, 1H), 5.68-5.55 (m, 2H), 5.42-5.31 (m, 2H), 4.30-4.20 (m, 1H), 3.20-3.10 (m, 4H), 2.00-1.50 (m, 10H), 1.16 (s, 3H), 1.04 (s, 3H), 0.87 (t, J = 7.2 Hz, 3H).

***Step 4: Preparation of (5S)-14-(4,4-dimethylcyclohexyl)-5-ethyl-18-fluoro-5-hydroxy-17-(3-hydroxypropyl)-7-oxa-11,14-diazapentacyclo[11.8.0.0$^2$,$^{11}$.0$^4$,$^9$.0$^{15}$,$^{20}$]heneicosa-1(13),2,4(9),15(20),16,18-hexaene-6,10,21-trione:***

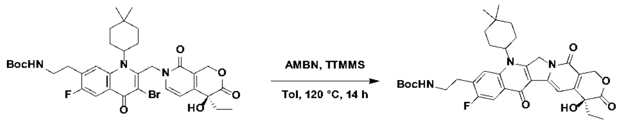


Under a nitrogen atmosphere, the intermediate obtained from Step 3 (200.0 mg, 0.28 mmol, 1.0 eq.) was dissolved in toluene (40 mL). Tris(trimethylsilyl)silane (208 mg, 0.84 mmol, 3.0 eq.) and 2,2'-azobis(2-methylpropionitrile) (AIBN) (54 mg, 0.28 mmol, 1.0 eq.) were added sequentially. After the addition, the mixture was stirred at 120 °C for 14 h. LCMS monitoring indicated the reaction was complete, then the reaction mixture was cooled to room temperature and the solid was filtered off. The crude product was slurried with methyl tert-butyl ether (2 mL) to afford the title compound as a brown solid (50 mg, crude). LCMS (ESI): $[M+H]^+$ = 636.4, $t_R$ = 1.390 min.

***Step 5: Preparation of (5S)-17-(2-aminoethyl)-14-(4,4-dimethylcyclohexyl)-5-ethyl-18-fluoro-5-hydroxy-7-oxa-11,14-diazapentacyclo[11.8.0.0$^2$,$^{11}$.0$^4$,$^9$.0$^{15}$,$^{20}$]heneicosa-1(13),2,4(9),15(20),16,18-hexaene-6,10,21-trione:***

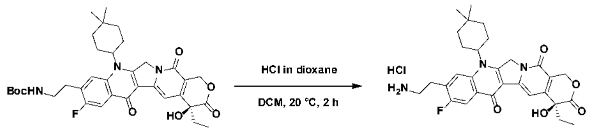


The intermediate obtained from step 4 (50 mg, 0.08 mmol, 1.0 eq.) was dissolved in DCM (2 mL), and 4 M HCl in 1,4-dioxane (2 mL) was added. The mixture was stirred at 20 °C for 2 h. LCMS monitoring indicated the reaction was complete, then the reaction mixture was concentrated, and purified by Prep-HPLC (0.05% HCl in water-ACN) to afford the title compound as a yellow solid (9.15 mg, 0.02 mmol, yield: 25.10%). LCMS (ESI): $[M+H]^+$ = 536.4, $t_R$=1.483min; $^1H$ NMR (400 MHz, DMSO-$d_6$) δ: 8.05-7.92 (m, 2H), 7.44 (d, J = 7.2 Hz, 1H), 6.92-6.85 (m, 1H), 6.49 (d, J = 7.2 Hz, 1H), 6.38 (s, 1H), 5.90-5.72 (m, 2H), 5.32 (dd, J = 16.0, 27.6 Hz, 2H), 4.25-4.14 (m, 1H), 3.27-3.20 (m, 2H), 2.93-2.82 (m, 2H), 1.82-1.70 (m, 2H), 1.42-1.30 (m, 4H), 1.30-1.15 (m, 13H), 1.03 (s, 3H), 0.91 (s, 3H), 0.77 (t, J = 7.2 Hz, 3H).

**Example 391: Preparation of (S)-4-ethyl-8-fluoro-4-hydroxy-10-(2-hydroxyethyl)-11-methyl-1,12-dihydro-14H-pyrano[3',4':6,7]indolizino[2,1-b]quinoline-3,6,14 (4H,11H)-trione:**

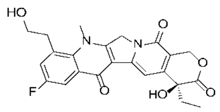

Intermediate 46 from Preparation Example 46 (4.6 g, 14.64 mmol, 1.0 eq.) was dissolved in 1,4-dioxane (50 mL). Water (15 mL), sodium carbonate (4.66 g, 43.92 mmol, 3.0 eq.), (E)-1-ethoxyvinyl-2-boronic acid pinacol ester (3.20 g, 16.1 mmol, 1.1 eq.) and tetrakis(triphenylphosphine)palladium (1.02 g, 0.88 mmol, 0.06 eq.) were added sequentially. The system was purged with nitrogen, and under a nitrogen atmosphere, the mixture was heated to 80 °C and stirred for 16 h. LCMS monitoring indicated the reaction was complete, then the reaction mixture was cooled to room temperature and filtered. The filtrate was concentrated and purified by silica gel column chromatography (eluent: DCM:MeOH = 1:0 to 30:1, v/v) to afford methyl (E)-8-(2-ethoxyvinyl)-6-fluoro-1-methyl-4-oxo-1,4-dihydroquinoline-2-carboxylate as a pale yellow solid (2.5 g, yield: 56.2%). LCMS (ESI): $[M+H]^+$ = 306.1, $t_R$ = 1.932 min.

Methyl (E)-8-(2-ethoxyvinyl)-6-fluoro-1-methyl-4-oxo-1,4-dihydroquinoline-2-carboxylate (2.5 g, 8.19 mmol, 1.0 eq.) was dissolved in DCM (30 mL). Trifluoroacetic acid (10 mL) was added under a nitrogen atmosphere, and the mixture was stirred at room temperature for 16 h. LCMS monitoring indicated the reaction was complete, then the reaction mixture was concentrated under reduced pressure to afford methyl 6-fluoro-1-methyl-4-oxo-8-(2-oxomethylidene)-1,4-dihydroquinoline-2-carboxylate as a pale yellow solid (2.5 g, crude). LCMS (ESI): $[M+H]^+$ = 278.1, $t_R$ = 1.001 min.

Crude methyl 6-fluoro-1-methyl-4-oxo-8-(2-oxomethylidene)-1,4-dihydroquinoline-2-carboxylate (700 mg, 2.52 mmol, 1.0 eq.) was dissolved in MeOH (15 mL), and acetic acid (455 mg, 7.57 mmol, 3.0 eq.) was added. Under an ice bath, sodium cyanoborohydride (313 mg, 5.05 mmol, 2.0 eq.) was added, and the mixture was warmed to room temperature naturally and stirred for 1 h. LCMS monitoring indicated the reaction was complete, then the reaction mixture was quenched with saturated ammonium chloride solution (0.5 mL) and concentrated to afford a crude product, which was purified by silica gel column chromatography (DCM as eluent) to afford methyl 6-fluoro-8-(2-hydroxyethyl)-1-methyl-4-oxo-1,4-dihydroquinoline-2-carboxylate as a pale yellow solid (600 mg, crude). LCMS (ESI): [M+H]*=280.1.

Methyl 6-fluoro-8-(2-hydroxyethyl)-1-methyl-4-oxo-1,4-dihydroquinoline-2-carboxylate (600 mg, 2.15 mmol, 1.0 eq.) was dissolved in anhydrous tetrahydrofuran (15 mL). 3,4-Dihydropyran (362 mg, 4.3 mmol, 2.0 eq.) and pyridinium p-toluenesulfonate (22 mg, 0.09 mmol, 0.04 eq.) were added, and the mixture was stirred at 50 °C for 16 h. LCMS monitoring indicated the reaction was complete, then the reaction mixture was concentrated, and purified by silica gel column chromatography (eluent: DCM:MeOH = 1:0 to 20: 1, v/v) to afford methyl 6-fluoro-1-methyl-4-oxo-8-(2-((tetrahydro-2H-pyran-2-yl)oxy)ethyl)-1,4-dihydroquinoline-2-carboxylate as a pale yellow oil (630 mg). LCMS (ESI): $[M+H]^+$ =364.1, $t_R$ = 1.527 min.

Methyl 6-fluoro-1-methyl-4-oxo-8-(2-((tetrahydro-2H-pyran-2-yl)oxy)ethyl)-1,4-dihydroquinoline-2-carboxylate (630 mg, 1.73 mmol, 1.0 eq.) was dissolved in MeOH (20 mL). Sodium borohydride (165 mg, 4.34 mmol, 2.5 eq.) was added portionwise under an ice bath, and after the addition, the mixture was warmed to room temperature naturally and stirred for 1 h. LCMS monitoring indicated the reaction was complete, then the reaction mixture was quenched with saturated brine (1 mL) and concentrated under, and purified by silica gel column chromatography (eluent: DCM:MeOH = 1:0 to 97:3, v/v) to afford 6-fluoro-2-(hydroxymethyl)-1-methyl-8-(2-((tetrahydro-2H-pyran-2-yl)oxy)ethyl)quinolin-4(1H)-one as a white solid (440 mg, yield: 75.60%). LCMS (ESI): $[M+H]^+$ = 336.2, $t_R$=1.112 min; $^1H$ NMR (400 MHz, DMSO-$d_6$) δ: 7.64 (dd, J = 8.5, 3.2 Hz, 1H), 7.59 (d, J = 9.4 Hz, 1H), 6.23 (s, 1H), 5.74 (t, J = 5.8 Hz, 1H), 4.58 (s, 1H), 4.56 (d, J = 3.7 Hz, 2H), 4.36 (t, J = 5.2 Hz, 1H), 3.87 (dt, J = 9.9, 6.7 Hz, 1H), 3.75 (s, 3H), 3.70-3.62 (m, 1H), 3.53 (td, J = 8.4, 4.2 Hz, 1H), 3.30 (t, J = 6.6 Hz, 2H), 1.49-1.32 (m, 6H). 6-Fluoro-2-(hydroxymethyl)-1-methyl-8-(2-((tetrahydro-2H-pyran-2-yl)oxy)ethyl) quinolin-4(1H)-one (440 mg, 1.32 mmol, 1.0 eq.) was dissolved in DCM (20 mL), and the reaction mixture was cooled to 0 °C. NBS (246 mg, 1.38 mmol, 1.05 eq.) was added portionwise, and the mixture was warmed to room temperature naturally and stirred for 1 h. LCMS monitoring indicated the reaction was complete, then the reaction mixture was poured into ice-cold saturated sodium bicarbonate solution (40 mL) and extracted with DCM (20 mL × 3). The combined organic phases were dried over anhydrous sodium sulfate, filtered and concentrated, and purified by silica gel column chromatography (eluent: DCM:MeOH = 1:0 to 50:1, v/v) to afford 3-bromo-6-fluoro-2-(hydroxymethyl)-1-methyl-8-(2-((tetrahydro-2H-pyran-2-yl)oxy)ethyl)quinolin-4(1H)-one as a pale yellow solid (520 mg, yield: 63.78%). LCMS (ESI): $[M+H]^+$ = 414.0/416.0, $t_R$ = 1.432 min.

3-Bromo-6-fluoro-2-(hydroxymethyl)-1-methyl-8-(2-((tetrahydro-2H-pyran-2-yl)oxy) ethyl)quinolin-4(1H)-one (520 mg, 1.26 mmol, 1.0 eq.) was dissolved in DCM (10 mL), and the reaction mixture was cooled to 0 °C. N,N-diisopropylethylamine (487 mg, 3.77 mmol, 3.0 eq.) and methanesulfonyl chloride (288 mg, 2.51 mmol, 2.0 eq.) were

added. After the addition, the mixture was warmed to room temperature naturally and stirred for 1 h. LCMS monitoring indicated the reaction was complete, then the reaction mixture was poured into ice water (30 mL) and extracted with DCM (20 mL × 3). The combined organic phases were dried over anhydrous sodium sulfate, filtered and concentrated to afford 3-bromo-2-(chloromethyl)-6-fluoro-1-methyl-8-(2-((tetrahydro-2H-pyran-2-yl)oxy)ethyl)quinolin-4(1H)-one as a white solid (600 mg, crude). LCMS (ESI): $[M+H]^+$ = 432.1, $t_R$ = 1.651 min. 3-Bromo-2-(chloromethyl)-6-fluoro-1-methyl-8-(2-((tetrahydro-2H-pyran-2-yl)oxy) ethyl)quinolin-4(1H)-one (600 mg, 1.39 mmol, 1.0 eq.) was dissolved in DMF (20 mL). Intermediate 1 (290 mg, 1.39 mmol, 1.0 eq.) and potassium carbonate (575 mg, 4.16 mmol, 3.0 eq.) were added sequentially. Under a nitrogen atmosphere, the mixture was heated to 30 °C and stirred for 16 h. LCMS monitoring indicated the reaction was complete, then the reaction mixture was filtered, and the filtrate was poured into ice-cold saturated aqueous ammonium chloride solution (40 mL) and extracted with ethyl acetate (30 mL × 3). The combined organic phases were washed with saturated aqueous sodium chloride solution (30 mL), dried over anhydrous sodium sulfate, filtered and concentrated under reduced pressure to afford a crude product, which was purified by silica gel column chromatography (eluent: DCM:MeOH = 1:0 to 50:1, v/v) to afford (4S)-7-((3-bromo-6-fluoro-1-methyl-4-oxo-8-(2-((tetrahydro-2H-pyran-2-yl)oxy)ethyl) -1,4-dihydroquinolin-2-yl)methyl)-4-ethyl-4-hydroxy-1,7-dihydro-3H-pyrano[3,4-c] pyridine-3,8(4H)-dione as a pale yellow solid (440 mg, yield: 52.41%). LCMS (ESI): $[M+H]^+$ = 604.9/607.1, $t_R$ = 1.527 min; $^1H$ NMR (400 MHz, DMSO-$d_6$) δ: 7.77-7.72 (m, 1H), 7.65 (ddd, J = 20.9, 8.8, 3.1 Hz, 2H), 6.48 (dd, J = 7.1, 1.3 Hz, 1H), 6.36 (s, 1H), 5.57 (s, 2H), 5.24 (s, 2H), 4.41 (ddd, J = 9.3, 4.2, 2.5 Hz, 1H), 3.66 (d, J = 3.3 Hz, 4H), 3.34-3.25 (m, 3H), 3.20-3.11 (m, 2H), 1.77 (ddd, J = 17.8, 11.1, 6.7 Hz, 2H), 1.58-1.24 (m, 6H), 0.81 (t, J = 7.4 Hz, 3H). (4S)-7-((3-bromo-6-fluoro-1-methyl-4-oxo-8-(2-((tetrahydro-2H-pyran-2-yl)oxy)ethyl) -1,4-dihydroquinolin-2-yl)methyl)-4-ethyl-4-hydroxy-1,7-dihydro-3H-pyrano[3,4-c]pyridine-3,8(4H)-dione (100 mg, 0.17 mmol, 1.0 eq.) was dissolved in toluene (15 mL). 2,2'-Azobis(2-methylpropionitrile) (AIBN) (28 mg, 0.17 mmol, 1.0 eq.) and tris(trimethylsilyl)silane (169 mg, 0.68 mmol, 4.0 eq.) were added sequentially. The system was purged with nitrogen three times, and under a nitrogen atmosphere, the mixture was heated to 100 °C and stirred for 16 h. LCMS monitoring indicated the reaction was complete, then the reaction mixture was concentrated under reduced pressure to afford a crude product, which was purified by silica gel column chromatography (eluent: DCM:MeOH = 1:0 to 10:1, v/v) and further purified by reversed-phase preparative HPLC to afford the title compound as an off-white solid (3 mg, yield: 4.01%). LCMS (ESI): $[M+H]^+$ = 441.2, $t_R$ = 0.445 min; $^1H$ NMR (400 MHz, DMSO-$d_6$) δ: 7.91 (dd, J = 8.3, 3.2 Hz, 1H), 7.71 (dd, J = 9.2, 3.2 Hz, 1H), 7.25 (s, 1H), 6.37 (s, 1H), 5.43-5.30 (m, 4H), 4.94 (t, J = 5.1 Hz, 1H), 4.10 (s, 3H), 3.80-3.74 (m, 2H), 3.38-3.35 (m, 2H), 1.86-1.78 (m, 2H), 0.87 (t, J = 7.3 Hz, 3H). Example 392: Preparation of (S)-10-((cyclopropylamino)methyl)-4-ethyl-8-fluoro-4-hydroxy-1,12-dihydro-3H,14H-chromeno[3,2-a]pyrano[3,4-f]indolizine-3,6,14(4H)-trione:

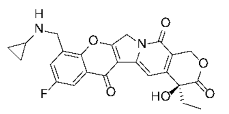

*Step 1: Preparation of 1-(3-bromo-5-fluoro-2-hydroxyphenyl)ethenone:*

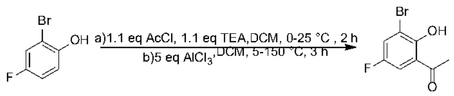


2-Bromo-4-fluorophenol (50.0 g, 261.78 mmol, 1.0 eq.) was dissolved in DCM (500 mL) and cooled to an internal temperature of 0 °C in an ice bath. Acetyl chloride (22.6 g, 287.96 mmol, 1.1 eq.) and triethylamine (29.2 g, 287.96 mmol, 1.1 eq.) were added, and the mixture was slowly warmed to room temperature and stirred for 2 h. TLC monitoring (eluent: petroleum ether:ethyl acetate = 10:1, v/v) indicated the reaction was complete. The mixture was washed sequentially with water (500 mL), saturated sodium bicarbonate solution (300 mL) and saturated sodium chloride solution (300 mL). The organic phase was collected, dried over anhydrous sodium sulfate, filtered and concentrated under reduced pressure to afford **2-bromo-4-fluorophenyl acetate** as a colorless oil (55.0 g, yield: 90.16%). $^1H$ NMR (400 MHz, DMSO-$d_6$) δ: 7.67 (dd, 1H), 7.34-7.26 (m, 2H), 2.26-2.32 (s, 3H).

2-Bromo-4-fluorophenyl acetate (11.0 g, 47.2 mmol, 1.0 eq.) was dissolved in DCM (100 mL) and cooled to an internal temperature of 0 °C in an ice bath. Aluminium trichloride (31.47 g, 236 mmol, 5.0 eq.) was added portionwise, and the mixture was heated to 150 °C and stirred for 3 h. TLC monitoring (eluent: petroleum ether:ethyl acetate = 10:1, v/v) indicated the reaction was complete. After cooling to room temperature, the reaction mixture solidified and was sonicated to dissolve in MeOH (200 mL), then the pH was adjusted to 6~7. The mixture was extracted with ethyl acetate (200 mL × 3), and the combined organic phases were washed with saturated sodium chloride solution (200 mL), dried over anhydrous

sodium sulfate, filtered and concentrated to afford the crude title compound as a colorless oil (10.6 g, yield: 96.37%). $^1H$ NMR (400 MHz, DMSO-$d_6$) δ: 12.52 (s, 1H), 7.94-7.87 (m, 2H), 2.68 (s, 3H).

*Step 2: Preparation of ethyl 4-(3-bromo-5-fluoro-2-hydroxyphenyl)-2,4-dioxobutanoate:*

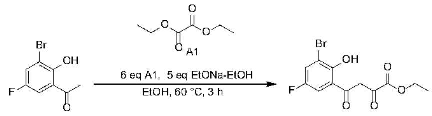


The intermediate obtained from step 1 (10.6 g, 45.49 mmol, 1.0 eq.) was dissolved in ethanol (100 mL), followed by the addition of diethyl oxalate (39.89 g, 272.93 mmol, 6.0 eq.) and 20% sodium ethoxide in ethanol (77.4 g, 227.44 mmol, 5.0 eq.). After the addition, the mixture was heated to 60 °C and stirred for 3 h. TLC monitoring (eluent: petroleum ether:ethyl acetate=5:1,v/v) indicated the reaction was complete, and ethanol was removed by concentration. The residual mixture was diluted with water (300 mL), filtered with suction, and the filter cake was washed with water (10 mL × 3) and dried to afford the crude title compound as a reddish brown solid (13.0 g, yield: 85.79%).

*Step 3: Preparation of ethyl 8-bromo-6-fluoro-4-oxo-4H-chromene-2-carboxylate:*

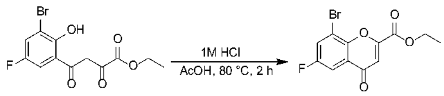


The intermediate obtained from step 2 (13.0 g, 39.03 mmol, 1.0 eq.) was dissolved in acetic acid (100 mL), and 1 M aqueous hydrochloric acid (100 mL) was added. The mixture was heated to 80 °C and stirred for 3 h, and LCMS monitoring indicated the reaction was complete. After cooling to room temperature, the mixture was diluted with water (150 mL) and extracted with ethyl acetate (100 mL × 3). The combined organic phases were washed with saturated sodium bicarbonate solution (300 mL × 3) and saturated sodium chloride solution (200 mL), then concentrated, and purified by flash column chromatography (silica gel, eluent: petroleum ether:ethyl acetate = 10: 1 to 5:1, v/v) to afford the title compound as a pale yellow solid (9.5 g, yield: 77.25%). LCMS (ESI): $[M+H]^+$ = 315.0, $t_R$ = 1.367 min.

*Step 4: Preparation of ethyl 6-fluoro-4-oxo-8-vinyl-4H-chromene-2-carboxylate:*

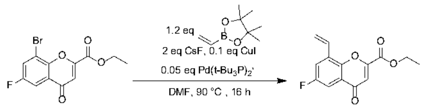


The intermediate obtained from step 4 (8.0 g, 24.91 mmol, 1.0 eq.) was dissolved in DMF (100 mL), followed by the addition of vinylboronic acid pinacol ester (4.6 g, 29.89 mmol, 1.2 eq.), cesium fluoride (7.57 g, 49.82 mmol, 2.0 eq.), copper(I) iodide (0.47 g, 2.49 mmol, 0.1 eq.) and palladium bis(tri-tert-butylphosphine) (0.64 g, 1.25 mmol, 0.05 eq.). The system was purged with nitrogen three times, then heated to 90 °C and stirred overnight. LCMS monitoring indicated the reaction was complete. The reaction mixture was poured into water (300 mL) and extracted with ethyl acetate (100 mL × 3). The combined organic phases were washed with saturated sodium chloride solution (100 mL × 3), dried over anhydrous sodium sulfate and concentrated, and purified by flash column chromatography (silica gel, eluent: petroleum ether:ethyl acetate = 10:1 to 4:1, v/v) to afford the title compound as a pale yellow solid (4.5 g, yield: 68.89%). LCMS (ESI): $[M+H]^+$ = 263.2, $t_R$ = 1.347 min.

*Step 5: Preparation of ethyl 6-fluoro-8-formyl-4-oxo-4H-chromene-2-carboxylate:*

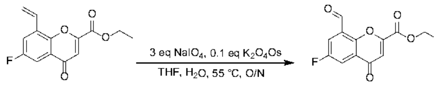


The intermediate obtained from step 5 (4.5 g, 17.03 mmol, 1.0 eq.) was dissolved in a mixture of tetrahydrofuran (80 mL) and water (20 mL), followed by the addition of sodium periodate (10.93 g, 51.09 mmol, 3.0 eq.) and potassium osmate (0.57 g, 1.7 mmol, 0.1 eq.). The mixture was heated to 55 °C and stirred overnight. LCMS monitoring indicated the reaction was complete, then the mixture was diluted with water (200 mL) and extracted with ethyl acetate (100 mL × 3). The combined organic phases were dried over anhydrous sodium sulfate and concentrated, and purified by flash column chromatography (silica gel, eluent: petroleum ether:ethyl acetate = 1:0 to 5:1, v/v) to afford the title compound as a white solid (3.0 g, yield: 66.17%). LCMS (ESI): $[M-H]^-$ = 265.1, $t_R$= 1.227 min.

*Step 6: Preparation of methyl 8-((cyclopropylamino)methyl)-6-fluoro-4-oxo-4H-chromene-2-carboxylate:*

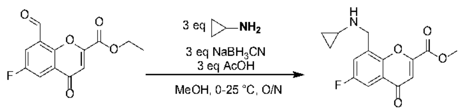


The intermediate obtained from step 5 (2.0 g, 7.57 mmol, 1.0 eq.) was dissolved in MeOH (30 mL) and cooled to an internal temperature of 0 °C in an ice bath. Cyclopropylamine (1.3 g, 22.71 mmol, 3.0 eq.) and acetic acid (1.36 g, 22.71 mmol, 3.0 eq.) were added, and the mixture was stirred for 30 min. Sodium cyanoborohydride (1.4 g, 22.71 mmol, 3.0 eq.) was then added portionwise, and the mixture was slowly warmed to room temperature and stirred overnight. LCMS monitoring indicated the reaction was complete, then the mixture was cooled in an ice bath and quenched by the addition of saturated ammonium chloride solution. The mixture was extracted with ethyl acetate (50 mL × 3), and the combined organic phases were dried over anhydrous sodium sulfate and concentrated to afford the crude title compound as a brown oil (2.1 g, yield: 90.86%). LCMS (ESI): $[M+H]^+$ = 292.2, $t_R$ = 0.997 min.

*Step 7: Preparation of tert-butyl (cyclopropyl((6-fluoro-4-oxo-2-(methoxycarbonyl)-4H-chromen-8-yl)methyl)carbamate):*

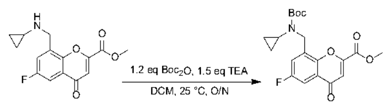


The intermediate obtained from step 6 (2.0 g, 6.55 mmol, 1.0 eq.) was dissolved in DCM (15 mL), followed by the addition of di-tert-butyl dicarbonate (1.72 g, 7.86 mmol, 1.2 eq.) and triethylamine (1.0 g, 9.88 mmol, 1.5 eq.). The mixture was stirred at room temperature overnight. LCMS monitoring indicated the reaction was complete, then the mixture was concentrated under reduced pressure. The crude product was purified by flash column chromatography (silica gel, eluent: MeOH:DCM = 0:1 to 1:25, v/v) to afford the title compound as a yellow solid (1.8 g, 4.6 mmol, yield: 66.98%). LCMS (ESI): $[M+H]^+$ = 392.3, $t_R$ = 1.400 min; $^1H$ NMR (400 MHz, DMSO-$d_6$) δ: 7.63 (dd, 1H), 7.37 (dd, 1H), 6.95 (s, 1H), 4.62 (s, 2H), 3.93 (s, 3H), 2.70-2.68 (m, 1H), 1.33 (s, 9H), 0.70-0.60 (m, 4H).

Using the above intermediate, the **target compound** was synthesized via subsequent transformations following the synthetic methods of Example 87 and Example 88, affording a pale yellow solid. LCMS (ESI): $[M+H]^+$ = 453.1, $t_R$ = 1.458 min.

Example 393: Preparation of (S)-11-(3-((cyclopropylamino)methyl)-1-methyl-1H-pyrazol-4-yl)-4-ethyl-8-fluoro-4-hydroxy-1,12-dihydro-14H-pyrano[3,4:6,7] indolizino [2,1-b]quinoline-3,6,14(4H,11H)-trione:

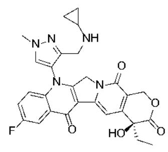

The primary hydroxyl group of the compound from Example 58 was oxidized to an aldehyde under the conditions of Dess-Martin periodinane. The resulting aldehyde was then subjected to a reductive amination reaction with cyclopropylamine and sodium cyanoborohydride to afford the title compound as a yellow solid. LCMS (ESI): $[M+H]^+$ = 532.3, $t_R$= 1.288 min; $^1H$ NMR (400 MHz, DMSO-$d_6$ + $D_2O$) δ: 8.32 (s, 1H), 8.02 (dd, J = 8.8, 3.0 Hz, 1H), 7.67-7.62 (m, 1H), 7.32 (s, 1H), 7.29-7.24 (m, 1H), 5.36-5.27 (m, 2H), 4.98 (s, 1H), 4.05-4.01 (m, 4H), 3.68-3.79 (m, 2H), 2.73-2.65 (m, 1H), 1.86-1.77 (m, 2H), 0.85 (t, J = 7.3 Hz, 3H), 0.81-0.73 (m, 2H), 0.72-0.61 (m, 2H).

Example 394: Preparation of (S)-4-ethyl-8-fluoro-4-hydroxy-11-((1S,2R)-2-(2-hydroxyethyl)cyclopentyl)-1H-pyrano [3,4:6,7]indolizino[2,1 -b]quinoline-3,6,14 (4H,11H,12H)-trione:

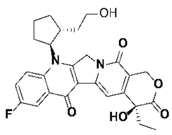

The primary hydroxyl group of the compound from Example 54 was oxidized to an aldehyde under the conditions of Dess-Martin periodinane. The resulting aldehyde was then subjected to a **sodium borohydride reduction reaction** to afford the title compound as a yellow solid. LCMS (ESI): $[M+H]^+$ = 495.1, $t_R$= 1.484 min; $^1H$ NMR (400 MHz, DMSO-$d_6$) δ: 8.04 (dd, J = 8.8, 3.2 Hz, 1H), 7.78-7.70 (m, 1H), 7.31 (s, 1H), 7.22-7.18 (m, 1H), 6.54 (s, 1H), 6.41-6.36 (m, 1H), 5.39-5.23 (m, 4H), 4.33-4.23 (m, 1H), 3.26-3.15 (m, 2H), 2.03-1.94 (m, 2H), 1.88-1.76 (m, 5H), 1.52-1.41 (m, 4H), 0.90-0.83 (m, 3H).

Example 395: Preparation of (S)-N-cyclopropyl-N-(2-(4-ethyl-8-fluoro-4-hydroxy-3,6,14-trioxo-4,6,12,14-tetrahydro-1H-pyrano[3,4:6,7]indolizino[2,1 -b] quinolin-11(3H)-yl)ethyl)methanesulfonamide:

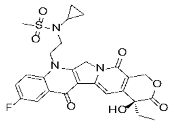

The compound from Example 96 (10 mg, 21.48 μmol, 1.0 eq.) was dissolved in DCM (0.1 mL). Under an ice bath, 0.1 mol/L triethylamine in DCM (0.43 mL, 42.97 μmol, 2.0 eq.) and 0.1 mol/L methanesulfonyl chloride in DCM (0.21 mL, 21.48 μmol, 1.0 eq.) were added sequentially. After the addition, the mixture was stirred for an additional 2 h under the ice bath. LCMS monitoring indicated the reaction was complete, then 1 mol/L dilute hydrochloric acid (1 mL) was added dropwise slowly to quench the reaction. Water was added, and the mixture was **lyophilized** to afford a crude product, which was purified by reversed-phase preparative HPLC to afford the title compound as a yellow solid (2 mg, yield: 17.1%). LCMS (ESI): $[M+H]^+$ = 544.3, $t_R$ = 1.492 min; $^1H$ NMR (400 MHz, DMSO-$d_6$) δ: 8.03 (dd, J = 8.9, 3.2 Hz, 2H), 7.88-7.81 (m, 1H), 7.31 (s, 1H), 6.36 (s, 1H), 5.47 (s, 2H), 5.42-5.29 (m, 2H), 4.67-4.55 (m, 2H), 3.68-3.60 (m, 2H), 3.06 (s, 3H), 2.65-2.57 (m, 1H), 1.88-1.78 (m, 2H), 0.88 (t, J = 7.3 Hz, 3H), 0.77-0.61 (m, 4H).

Example 396: Preparation of (S)-4-ethyl-8-fluoro-4-hydroxy-11-((3R,5S)-5-(2-hydroxypropan-2-yl)-1-methylpyrrolidin-3-yl)-1H-pyrano[3,4:6,7]indolizino [2,1-b]quinoline-3,6,14(4H,11H,12H)-trione:

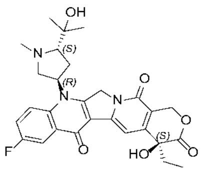

Using (Z)-1-(2,5-difluorophenyl)-3-(dimethylamino)but-2-en-1-one (Intermediate 27 from Preparation Example 27) and methyl (2S,4R)-1-(tert-butoxycarbonyl)-4-aminopyrrolidine-2-carboxylate as the starting materials, **methyl (2S,4R)-1-(tert-butoxycarbonyl)-4-(6-fluoro-2-methyl-4-oxoquinolin-1(4H)-yl)pyrrolidine-2-carboxylate** was synthesized following the procedures of Steps 1~2 in Example 45, affording a light brown solid (yield: 64.39%). LCMS (ESI): $[M+H]^+$ = 405.2, $t_R$= 1.213 min.

A solution of methyl (2S,4R)-1-(tert-butoxycarbonyl)-4-(6-fluoro-2-methyl-4-oxoquinolin-1(4H)-yl)pyrrolidine-2-carboxylate (1.90 g, 4.70 mmol, 1.0 eq.) in tetrahydrofuran (20 mL) was added dropwise to a 1 M solution of methylmagnesium bromide in tetrahydrofuran (18.79 mL, 18.79 mmol, 4.0 eq.) at 25 °C. The mixture was stirred at 25 °C for 1 h, and LCMS monitoring indicated the reaction was complete. The reaction mixture was poured into saturated aqueous ammonium chloride solution (100 mL) to quench the reaction, then extracted with ethyl acetate (100 mL $\times$ 2). The combined organic phases were dried over anhydrous sodium sulfate and concentrated, and purified by flash column chromatography (silica gel, eluent: MeOH:DCM = 0:1 to 10:90, v/v) to afford tert-butyl **(2S,4R)-4-(6-fluoro-2-methyl-4-oxoquinolin-1(4H)-yl)-2-(2-hydroxypropan-2-yl)pyrrolidine-1-carboxylate** as a light brown solid (1.14 g, yield: 59.97%). LCMS (ESI): $[M+H]^+$ = 405.3, $t_R$= 1.977 min.

Using the above intermediate, the title compound was synthesized via **dibromination, alkylation and cyclization** following the procedures of Steps 2~5 in Example 8, and purified by Prep-HPLC (C18 column, 0.1% aqueous HCl, acetonitrile as eluent), affording a pale yellow solid. LCMS (ESI): $[M+H]^+$ = 523.9, $t_R$= 1.357 min; $^1$H NMR (400 MHz, DMSO-$d_6$) δ: 10.32 (brs, 1H), 8.23 (dd, J = 9.5, 4.0 Hz, 1H), 8.04 (dd, J = 8.7, 3.2 Hz, 1H), 7.75-7.70 (m, 1H), 7.29 (s, 1H), 5.68 (d, J = 19.8 Hz, 1H), 5.42 (d, J = 19.8 Hz, 1H), 5.36-5.31 (m, 2H), 5.15-5.05 (m, 1H), 4.16-3.96 (m, 3H), 3.19 (s, 3H), 3.03 (dd, J = 23.5, 10.3 Hz, 1H), 2.74-2.64 (m, 1H), 1.87-1.77 (m, 2H), 1.39 (s, 3H), 1.34 (s, 3H), 0.86 (t, J = 7.3 Hz, 3H).

Example 397: Preparation of (S)-2-(11-(4,4-dimethylcyclohexyl)-4-ethyl-8-fluoro-4-hydroxy-3,6,14-trioxo-3,4,6,11,12,14-hexahydro-1H-pyrano[3,4:6,7]indolizino [2,1-b]quinolin-9-yl)ethyl 6-aminohexanoate:

*Step 1: Preparation of benzyl (6-chloro-6-oxohexyl)carbamate:*

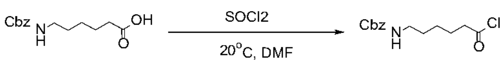


6-(((Benzyloxy)carbonyl)amino)hexanoic acid (300 mg, 1.13 mmol, 1.0 eq.) was dissolved in thionyl chloride (2 mL), and one drop of N,N-dimethylformamide was added. The mixture was stirred at room temperature for 4 h, and LCMS monitoring indicated the reaction was complete. The reaction mixture was concentrated under reduced pressure to afford the target product as a colorless oil (280 mg, crude). LCMS (ESI): $[M+H]^+$ = 333.0, t_R = 1.719 min.

*Step 2: Preparation of (S)-2-(11-(4,4-dimethylcyclohexyl)-4-ethyl-8-fluoro-4-hydroxy-3,6,14-trioxo-3,4,6,11,12,14-hexahydro-1H-pyrano[3,4:6,7]indolizino [2,1-b]quinolin-9-yl)ethyl 6-(((benzyloxy)carbonyl)amino)hexanoate:*

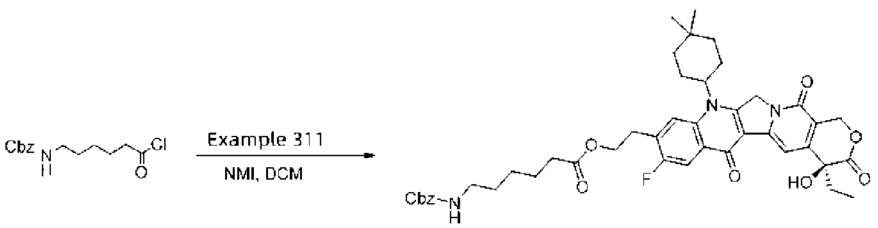


The intermediate obtained from Step 1 (40 mg, 0.07 mmol, 1.0 eq.) was dissolved in DCM (2 mL). Under an ice bath, the compound from Example 311 (105.76 mg, 0.37 mmol, 5.0 eq.) and N-methylimidazole (15.15 mg, 0.07 mmol, 1.0

eq.) were added sequentially. After the addition, the mixture was slowly warmed to room temperature and stirred for 16 h. LCMS monitoring indicated the reaction was complete, then the mixture was poured into water (30 mL) and extracted with DCM (20 mL × 3). The combined organic phases were washed with saturated brine (80 mL), dried over anhydrous sodium sulfate, filtered and concentrated, and purified by reversed-phase preparative HPLC (0.1% formic acid) to afford the title compound as a yellow solid (35 mg, yield: 63.79%). LCMS (ESI): $[M+H]^+$ = 784.0, $t_R$= 1.992 min.

*Step 3: Preparation of (S)-2-(11-(4,4-dimethylcyclohexyl)-4-ethyl-8-fluoro-4-hydroxy-3,6,14-trioxo-3,4,6,11,12,14-hexahydro-1H-pyrano[3,4:6,7]indolizino [2,1-b]quinolin-9-yl)ethyl 6-aminohexanoate:*

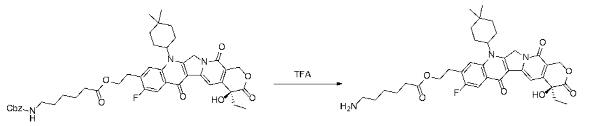


The intermediate obtained from step 2 (50 mg, 0.06 mmol, 1.0 eq.) was dissolved in trifluoroacetic acid (2 mL), and the mixture was stirred at room temperature for 16 h. LCMS monitoring indicated the reaction was complete, then the mixture was concentrated, and purified by reversed-phase preparative HPLC (0.1% formic acid) to afford the title compound as a reddish brown solid (10 mg, yield: 26.65%). LCMS (ESI): $[M+H]^+$ = 650.0, $t_R$= 1.435 min; $^1$H NMR (400 MHz, DMSO-$d_6$) δ: 8.00-7.92 (m, 2H), 7.29 (s, 1H), 6.35 (s, 1H), 5.63-5.45 (m, 2H), 5.40-5.28 (m, 2H), 4.45-4.34 (m, 3H), 3.22-3.12 (m, 4H), 2.72 (t, J = 7.6 Hz, 2H), 2.66-2.55 (m, 2H), 2.27-2.20 (m, 2H), 1.86-1.77 (m, 2H), 1.74-1.60 (m, 4H), 1.52-1.42 (m, 5H), 1.28-1.20 (m, 3H), 1.19-1.08 (m, 6H), 0.86 (t, J = 7.3 Hz, 3H).

Example 398: Preparation of (S)-4-ethyl-8-fluoro-4-hydroxy-11-(1-methyl piperidin-4-yl)-1,12-dihydro-14H-pyrano [3,4:6,7]indolizino[2,1 -b]quinoline-3,6,14(4H,11H)-trione:

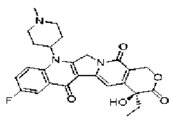

*Step 1: Preparation of tert-butyl 4-((2-acetyl-4-fluorophenyl)amino)piperidine-1-carboxylate:*

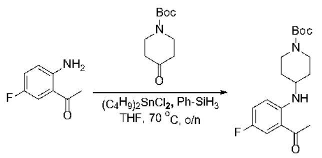


2-Amino-5-fluoroacetophenone (CAS: 2343-25-1) (5.0 g, 32.65 mmol, 1.0 eq.) was dissolved in anhydrous tetrahydrofuran (50 mL). tert-Butyl 4-oxopiperidine-1-carboxylate (CAS: 79099-07-3) (6.51 g, 32.65 mmol, 1.0 eq.) and dibutyltin dichloride (CAS: 683-18-1) (1.98 g, 6.53 mmol, 0.2 eq.) were added, and the mixture was stirred at 70 °C for 1 h. Phenylsilane (CAS: 694-53-1) (3.53 g, 32.65 mmol, 1.0 eq.) was added to the reaction system, and the mixture was stirred at 70 °C for 18 h. LCMS monitoring indicated the reaction was complete, then the reaction mixture was concentrated under reduced pressure. The crude product was purified by flash column chromatography (200-300 mesh silica gel, eluent: petroleum ether:ethyl acetate = 10:1 to 2:1, v/v) to afford the title compound as a yellow solid (11 g, yield: 85%). LCMS (ESI): $[M-55]^+$ = 281.1, $t_R$= 3.137 min.

*Step 2: Preparation of ethyl 1-(1-(tert-butoxycarbonyl)piperidin-4-yl)-6-fluoro-4-oxo-1,4-dihydroquinoline-2-carboxylate:*

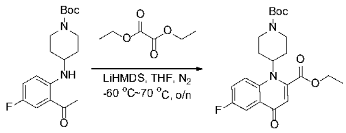


tert-Butyl 4-((2-acetyl-4-fluorophenyl)amino)piperidine-1-carboxylate (11 g, 32.7 mmol, 1.0 eq.) was dissolved in anhydrous tetrahydrofuran (80 mL). A 1 M solution of lithium bis(trimethylsilyl)amide in tetrahydrofuran (81 mL, LiHMDS/THF) was added at -60 °C, and the mixture was stirred at -60 °C for 30 min. Diethyl oxalate (11.95 g, 81.75 mmol, 2.5 eq.) was added to the reaction system, and the mixture was stirred at 70 °C overnight. LCMS monitoring indicated the reaction was complete, then the reaction mixture was concentrated under reduced pressure. The crude product was purified by flash column chromatography (200-300 mesh silica gel, eluent: DCM:MeOH = 40:1 to 20:1, v/v) to afford the title compound as a yellow solid (11 g, yield: 80%). LCMS (ESI): $[M+H]^+$ = 419.2, $t_R$ = 2.974 min.

Using ethyl 1-(1-(tert-butoxycarbonyl)piperidin-4-yl)-6-fluoro-4-oxo-1,4-dihydroquinoline-2-carboxylate (the intermediate obtained from step 2) as the starting material, the title compound was synthesized following the synthetic methods of Example 249 and Example 250, affording an off-white solid. LCMS (ESI): $[M+H]^+$ = 480.1, $t_R$= 0.346 min; $^1$H NMR (400 MHz, DMSO-$d_6$) δ: 8.70-8.59 (m, 1H), 8.05 (dd, J = 8.7, 3.2 Hz, 1H), 7.74-7.65 (m, 1H), 7.32 (s, 1H), 6.41 (s, 1H), 5.65-5.49 (m, 2H), 5.44-5.32 (m, 2H), 4.79-4.66 (m, 1H), 3.70-3.58 (m, 2H), 3.22-3.04 (m, 2H), 2.85 (s, 3H), 2.41-2.02 (m, 4H), 1.90-1.73 (m, 2H), 0.87 (t, J = 7.3 Hz, 3H).

Example 399: Preparation of (S)-11-(((R)-1-cyclobutylpyrrolidin-3-yl)methyl)-4-ethyl-8-fluoro-4-hydroxy-1H-pyrano [3,4:6,7]indolizino[2,1 -b]quinoline-3,6,14(4H,11H,12H)-trione:

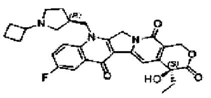

Using (Z)-1-(2,5-difluorophenyl)-3-(dimethylamino)but-2-en-1-one (Intermediate 27 from Preparation Example 27) and commercially available tert-butyl (R)-3-(aminomethyl)pyrrolidine-1-carboxylate as the starting materials, the title compound was synthesized following the procedures of Example 253, affording an off-white solid. LCMS (ESI): $[M+H]^+$ = 520.3; $^1$H NMR (400 MHz, DMSO-$d_6$) δ: 10.96 (d, J = 86.3 Hz, 1H), 8.29-8.26 (m, 1H), 8.01 (dd, J = 8.8, 3.0 Hz, 1H), 7.82-7.76 (m, 1H), 7.30 (s, 1H), 6.42-6.32 (m, 1H), 5.49-5.31 (m, 4H), 4.63-4.52 (m, 2H), 3.79-3.69 (m, 1H), 3.64-3.49 (m, 1H), 3.18-2.83 (m, 3H), 2.33-1.63 (m, 11H), 0.87 (t, J = 7.4 Hz, 3H).

Example 400: Preparation of (S)-11-(((R)-1-(3,3-difluorocyclobutyl)pyrrolidin-3-yl)methyl)-4-ethyl-8-fluoro-4-hydroxy-1H-pyrano[3,4:6,7]indolizino[2,1 -b] quinoline-3,6,14(4H,11H,12H)-trione:

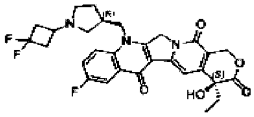

Similarly, using (Z)-1-(2,5-difluorophenyl)-3-(dimethylamino)but-2-en-1-one (Intermediate 27 from Preparation Example 27) and commercially available tert-butyl (R)-3-(aminomethyl)pyrrolidine-1-carboxylate as the starting materials, the title compound was synthesized following the procedures of Example 365, affording an off-white solid. LCMS (ESI): $[M+H]^+$ = 556.3; $^1$H NMR (400 MHz, DMSO-$d_6$) δ: 11.92-11.75 (m, 1H), 8.30-8.17 (m, 1H), 8.01-7.98 (m, 1H), 7.79 (t, J = 8.1 Hz, 1H), 7.30 (s, 1H), 6.43-6.32 (m, 1H), 5.50-5.30 (m, 4H), 4.70-4.48 (m, 2H), 3.88-3.77 (m, 1H), 3.67-3.58 (m, 2H), 3.21-2.89 (m, 7H), 2.20-1.75 (m, 4H), 0.86 (t, J = 7.3 Hz, 3H).

Preparation of Compounds in Examples 401-404

Similarly, the compounds in Examples 401-404 were synthesized from commercially available reagents following the synthetic methods of Example 399 and Example 400.

The chemical structures and detailed characterization data (LCMS and $^1$H NMR) of the compounds are shown below:

| Examples | structure | $^1H$ NMR | LCMS (ESI) $[M+H]^+$ |
|---|---|---|---|
| 401 | 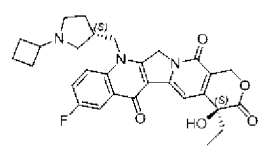 | (400 MHz, DMSO-$d_6$+$D_2O$) δ 8.25-8.15 (m, 1H), 8.02-7.97 (m, 1H), 7.81-7.75 (m, 1H), 7.30 (s, 1H), 5.42-5.23 (m, 3H), 4.73-4.46 (m, 2H), 3.80-3.68 (m, 1H), 3.62-3.45 (m, 2H), 3.40-3.30 (m, 2H), 3.18-3.06 (m, 1H), 2.95-2.82 (m, 1H), 2.24-2.02 (m, 5H), 1.99-1.63 (m, SH), 0.86 (t, *J* = 7.3 Hz, 3H)。 | 520.3, $t_R$=1.300 min |
| 402 | 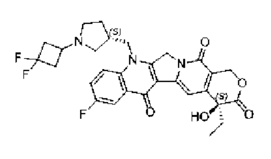 | (400 MHz, DMSO-$d_6$+$D_2O$) δ 8.20-8.14 (m, 1H), 7.99 (dd, *J* = 8.1, 1.5 Hz, 1H), 7.79-7.74 (m, 1H), 7.29 (s, 1H), 5.39-5.29 (m, 4H), 4.68-4.44 (m, 2H), 3.88-3.79 (m, 1H), 3.75-3.56 (m, 2H), 3.26-2.88 (m, 7H), 2.25-1.75 (m, 4H), 0.85 (t, *J* = 7.3 Hz, 3H)。 | 556.4, $t_R$=1.255 min |
| 403 | 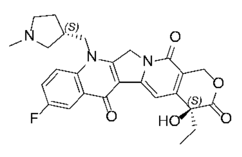 | (400 MHz, DMSO-$d_6$) δ 8.29-8.18 (m, 1H), 8.01 (dd, *J* = 8.8, 3.0 Hz, 1H), 7.82-7.76 (m, 1H), 7.30 (s, 1H), 6.37 (s, 1H), 5.53-5.31 (m, 4H), 4.66-4.49 (m, 2H), 3.55-3.70 (m, 1H), 3.21-2.89 (m, 2H), 2.81-2.74 (m, 2H), 2.54 (s, 3H), 2.07-1.90 (m, 1H), 1.87-1.72 (m, 3H), 0.87 (t, *J* = 7.3 Hz, 3H) | 480.3, $t_R$=1.226 min |
| 404 | 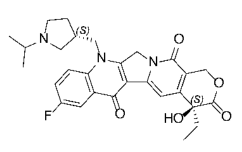 | (400 MHz, DMSO-$d_6$) δ 8.29-8.12 (m, 1H), 7.97 (td, *J* = 6.0, 2.9 Hz, 1H), 7.79-7.72 (m, 1H), 7.24-7.29 (s, 1H), 5.42-5.27 (m, 4H), 4.68-4.42 (m, 2H), 3.58-3.52 (m, 1H), 3.36-3.19 (m, 3H), 3.10-2.82 (m, 2H), 2.13-2.04 (m, 1H), 1.91-1.72 (m, 3H), 1.21 (t, *J* = 7.4 Hz, 6H), 0.83 (t, *J* = 7.1 Hz, 3H) | 508.3, $t_R$=1.265 min |

Example 405: Preparation of (S)-1-ethyl-12-fluoro-1-hydroxy-9,10-dimethyl-4,7,8,9-tetrahydro-2H,5H-benzo[b]pyrano[4,3:4,5]pyrido[2,1 -f][1,6] naphthyridine-2,5,14(1H)-trione:

*Step 1: Preparation of 6-fluoro-1,8-dimethyl-4-oxo-1,4-dihydroquinoline-2-carbaldehyde:*

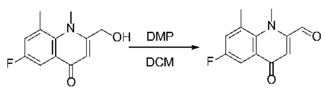


6-Fluoro-2-(hydroxymethyl)-1,8-dimethylquinolin-4(1H)-one (2 g, 9.05 mmol, 1.0 eq.), the intermediate obtained from Step 4 of Example 2, was dissolved in DCM (20 mL). Dess-Martin periodinane (5.76 g, 13.57 mmol, 1.5 eq.) was added under an ice bath. After the addition, the ice bath was removed, and the mixture was stirred at room temperature for 4 h. LCMS monitoring indicated the reaction was complete. The reaction mixture was filtered, and the filtrate was concentrated to afford the crude title compound as a dark gray solid (3 g). LCMS (ESI): $[M+H]^+$ = 220.2, $t_R$= 1.308 min.

*Step 2: Preparation of (E)-6-fluoro-2-(2-methoxyvinyl)-1,8-dimethylquinolin-4(1H)-one:*

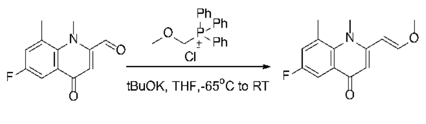


(Methoxymethyl)triphenylphosphonium chloride (7.05 g, 20.55 mmol, 1.5 eq.) was dissolved in tetrahydrofuran (30 mL). Under a nitrogen atmosphere at -65 °C, a 1 mol/L solution of potassium tert-butoxide in THF (27.4 mL, 27.4 mmol, 2.0 eq.) was added slowly, and stirring was continued for 30 min after the addition. A solution of the crude intermediate obtained from step 1 (3 g) in tetrahydrofuran (30 mL) was added dropwise to the reaction mixture, which was then stirred at -65 °C for an additional 2 h. LCMS monitoring indicated the reaction was complete. The reaction mixture was poured into ice water (300 mL) and extracted with ethyl acetate (50 mL × 3). The combined organic phases were washed with

saturated sodium chloride solution (200 mL), dried over anhydrous sodium sulfate, filtered and concentrated, and purified by silica gel column chromatography (eluent: DCM:MeOH = 1:0 to 20:1, v/v) afforded the title compound as a yellow solid (1.5 g, yield: 44.3%). LCMS (ESI): $[M+H]^+$ = 344.3, $t_R$= 1.567 min.

*Step 3: Preparation of 2-(6-fluoro-1,8-dimethyl-4-oxo-1,4-dihydroquinolin-2-yl)acetaldehyde:*

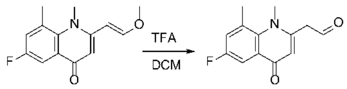


The intermediate obtained from step 2 (1.5 g, 6.07 mmol, 1.0 eq.) was dissolved in DCM (15 mL). Trifluoroacetic acid (1.04 g, 9.11 mmol, 1.5 eq.) was added under an ice bath, and the mixture was stirred at room temperature for 2 h. LCMS monitoring indicated the reaction was complete. The reaction mixture was concentrated under reduced pressure to afford the crude title compound as a dark gray solid (1.5 g). LCMS (ESI): $[M-H]^-$ = 232.0, $t_R$= 1.152 min.

*Step 4: Preparation of 6-fluoro-2-(2-hydroxyethyl)-1,8-dimethylquinolin-4(1H)-one:*

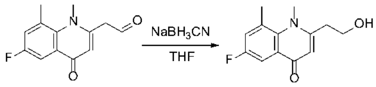


The intermediate obtained from Step 3 (1.5 g, 6.44 mmol, 1.0 eq.) was dissolved in tetrahydrofuran (15 mL). Sodium cyanoborohydride (811.1 mg, 12.88 mmol, 1.5 eq.) was added slowly under an ice bath. After the addition, the ice bath was removed, and the mixture was stirred at room temperature for an additional 2 h. LCMS monitoring indicated the reaction was complete. The reaction was quenched by the addition of ice water (1 mL), and the mixture was concentrated under reduced pressure to afford a crude product. Purification by silica gel column chromatography (eluent: DCM:MeOH = 1:0 to 20:1, v/v) afforded the title compound as a dark gray solid (1.2 g, yield: 79.3%). LCMS (ESI): $[M-H]^-$ = 236.2, $t_R$= 1.071 min.

*Step 5: Preparation of 3-bromo-6-fluoro-2-(2-hydroxyethyl)-1,8-dimethylquinolin-4(1H)-one:*

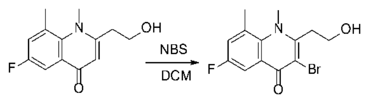


The intermediate obtained from Step 4 (1.2 g, 5.11 mmol, 1.0 eq.) was dissolved in DCM (15 mL). NBS (1.0 g, 5.62 mmol, 1.1 eq.) was added at room temperature, and the mixture was stirred at room temperature for an additional 2 h. LCMS monitoring indicated the reaction was complete. The mixture was concentrated under reduced pressure to afford a crude product. Purification by silica gel column chromatography (eluent: DCM:MeOH = 1:0 to 20:1, v/v) afforded the title compound as a yellow solid (1.2 g, yield: 75.1%). LCMS (ESI): $[M+H]^+$ = 314.1, $t_R$= 1.200 min.

*Step 6: Preparation of 3-bromo-2-(2-chloroethyl)-6-fluoro-1,8-dimethylquinolin-4(1H)-one:*

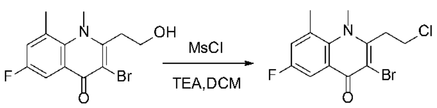


The intermediate obtained from step 5 (1.2 g, 3.83 mmol, 1.0 eq.) was dissolved in DCM (15 mL). Triethylamine (774.4 mg, 7.67 mmol, 2.0 eq.) and methanesulfonyl chloride (655.6 mg, 5.75 mmol, 1.5 eq.) were added under an ice bath. After the addition, the ice bath was removed, and the mixture was stirred at room temperature for an additional 2 h. LCMS

monitoring indicated the reaction was complete. The reaction mixture was poured into ice water (50 mL) and extracted with DCM (30 mL × 3). The combined organic phases were dried over anhydrous sodium sulfate, filtered and concentrated to afford the crude title compound as a yellow solid (1.1 g), which was **used directly in the next step.** LCMS (ESI): $[M+H]^+$ = 332.1, $t_R$= 1.394 min.

*Step 7: Preparation of (S)-7-(2-(3-bromo-6-fluoro-1,8-dimethyl-4-oxo-1,4-dihydroquinolin-2-yl)ethyl)-4-ethyl-4-hydroxy-1,7-dihydro-3H-pyrano[3,4-c]pyridine-3,8(4H)-dione:*

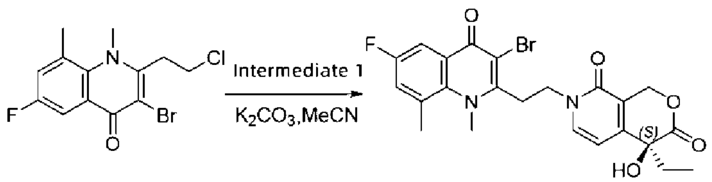


The intermediate obtained from step 6 (1.1 g, 3.31 mmol, 1.0 eq.) was dissolved in anhydrous ACN (15 mL). Potassium carbonate (1.37 g, 9.94 mmol, 3.0 eq.) and Intermediate 1 (692.5 mg, 3.31 mmol, 1.0 eq.) were added, and the mixture was heated to 50 °C and stirred for 16 h. LCMS monitoring indicated the reaction was complete. The reaction mixture was filtered, and the filtrate was concentrated, and purified by silica gel column chromatography (eluent: DCM:MeOH = 1:0 to 20:1, v/v) afforded the title compound as a white solid (410 mg, yield: 24.5%). LCMS (ESI): $[M+H]^+$ = 505.0, $t_R$= 1.302 min.

*Step 8: Preparation of (S)-1-ethyl-12-fluoro-1-hydroxy-9,10-dimethyl-4,7,8,9-tetrahydro-2H,5H-benzo[b]pyrano[4,3:4,5]pyrido[2,1 -f][1,6]naphthyridine-2,5,14(1H)-trione:*

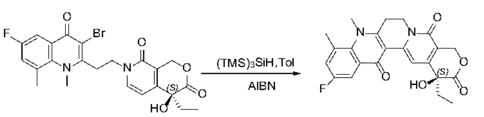


The intermediate obtained from step 7 (410 mg, 0.81 mmol, 1.0 eq.) was dissolved in toluene (50 mL). Under a nitrogen atmosphere, the mixture was heated to 100 °C, and a solution of AIBN (266.8 mg, 1.63 mmol, 2.0 eq.) and tris(trimethylsilyl)silane (403.5 mg, 1.63 mmol, 2.0 eq.) in toluene (5 mL) was added dropwise. After the addition, the mixture was stirred at 100 °C for an additional 16 h. LCMS monitoring indicated the reaction was complete. The reaction mixture was concentrated, and purified by silica gel column chromatography (eluent: DCM:MeOH = 1:0 to 10:1, v/v) and further purified by **reversed-phase preparative HPLC** to afford the title compound as a brown solid (23.36 mg, yield: 6.92%). LCMS (ESI): $[M+H]^+$ = 425.2, $t_R$= 1.320 min; $^1$H NMR (400 MHz, DMSO-$d_6$) $\delta$: 8.35 (s, 1H), 7.79 (dd, J = 8.7, 3.1 Hz, 1H), 7.55 (dd, J = 9.2, 2.9 Hz, 1H), 5.33-5.20 (m, 2H), 4.37-4.24 (m, 1H), 4.18-4.03 (m, 1H), 3.85 (s, 3H), 3.25-3.10 (m, 2H), 2.71 (s, 3H), 1.84-1.71 (m, 2H), 0.86 (t, J = 7.4 Hz, 3H).

Example 406: Preparation of 5-ethyl-9-fluoro-5-hydroxy-11,12-dimethyl-1,4,5,13-tetrahydro-3H,15H-oxepino[3,4:6,7]indolizino[2,1 -b]quinoline-3,7,15(12H)-trione:

*Step 1: Preparation of (4-iodo-2-methoxypyridin-3-yl)MeOH:*

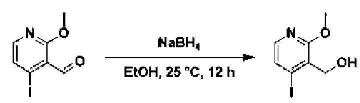


(4-Iodo-2-methoxypyridin-3-yl)carbaldehyde (24.0 g, 91.24 mmol, 1.0 eq.) was dissolved in ethanol (200 mL). Sodium borohydride (5.18 g, 136.87 mmol, 1.5 eq.) was added portionwise at 0 °C, and the mixture was stirred at 25 °C for 12 h after the addition. LCMS monitoring indicated the reaction was complete. The reaction mixture was diluted with ethyl

acetate (100 mL), quenched with saturated aqueous ammonium chloride solution (400 mL) and extracted with ethyl acetate (300 mL × 3). The combined organic phases were washed with saturated brine (100 mL × 3), dried over anhydrous sodium sulfate, filtered and concentrated under vacuum. The residue was purified by column chromatography (200-300 mesh silica gel, eluent: petroleum ether:ethyl acetate = 10:1 to 5:1, v/v) to afford the title compound as a yellow solid (22.0 g, yield: 90.9%). LCMS (ESI): $[M+H]^+$ = 266.0, $t_R$= 1.153 min.

*Step 2: Preparation of 3-(benzyloxymethyl)-4-iodo-2-methoxypyridine:*

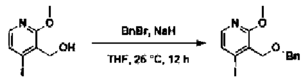


Sodium hydride (6.64 g, 166.01 mmol, 60% w/w, 2.0 eq.) was suspended in anhydrous tetrahydrofuran (200 mL). Under a nitrogen atmosphere, the intermediate obtained from Step 1 (22.0 g, 83 mmol, 1.0 eq.) was added at 0 °C, and the mixture was stirred at 0 °C for 0.5 h. Benzyl bromide (17.04 g, 99.6 mmol, 1.2 eq.) was then added dropwise, and the mixture was stirred at 25 °C for 12 h after the addition. LCMS monitoring indicated the reaction was complete. The reaction mixture was diluted with ethyl acetate (100 mL), quenched with saturated aqueous ammonium chloride solution (500 mL) and extracted with ethyl acetate (400 mL × 3). The combined organic phases were washed with saturated brine (200 mL × 3), dried over anhydrous sodium sulfate, filtered and concentrated under vacuum. The residue was purified by column chromatography (200-300 mesh silica gel, eluent: petroleum ether:ethyl acetate = 10:1, v/v) to afford the title compound as a yellow solid (19.1 g, yield: 64.5%). LCMS (ESI): $[M+H]^+$ = 356.0, $t_R$= 1.587 min.

*Step 3: Preparation of 1-(3-(benzyloxymethyl)-2-methoxypyridin-4-yl)propan-1-ol:*

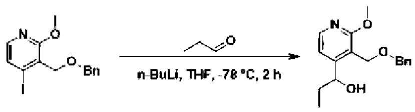


The intermediate obtained from step 2 (18.0 g, 50.68 mmol, 1.0 eq.) was dissolved in anhydrous tetrahydrofuran (120 mL). Under a nitrogen atmosphere, a 2.5 M solution of n-butyllithium in THF/hexane (40.5 mL, 2.0 eq.) was added dropwise at -78 °C, and the mixture was stirred at -78 °C for 0.5 h. A solution of propionaldehyde (8.8 g, 151.72 mmol, 3.0 eq.) in THF (10 mL) was then added dropwise, and the mixture was stirred at -78 °C for an additional 2 h. LCMS monitoring indicated the reaction was complete. The reaction mixture was diluted with ethyl acetate (60 mL), quenched with saturated aqueous ammonium chloride solution (200 mL) and extracted with ethyl acetate (200 mL × 3). The combined organic phases were washed with saturated brine (50 mL × 3), dried over anhydrous sodium sulfate, filtered and concentrated under vacuum. The residue was purified by column chromatography (200-300 mesh silica gel, eluent: petroleum ether:ethyl acetate = 8:1, v/v) to afford the title compound as a yellow solid (8.1 g, yield: 55.6%). LCMS (ESI): $[M+H]^+$ = 288.2, $t_R$= 1.330 min.

*Step 4: Preparation of 1-(3-(benzyloxymethyl)-2-methoxypyridin-4-yl)propan-1-one:*

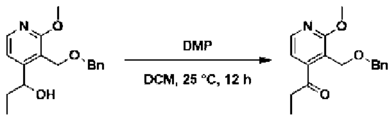


The intermediate obtained from step 3 (7.1 g, 24.7 mmol, 1.0 eq.) was dissolved in DCM (70 mL). Under a nitrogen atmosphere, Dess-Martin periodinane (20.96 g, 49.42 mmol, 2.0 eq.) was added, and the mixture was stirred at 25 °C for 12 h. LCMS monitoring indicated the reaction was complete. The reaction mixture was diluted with DCM (150 mL), quenched with saturated aqueous sodium sulfite solution (200 mL) and aqueous sodium bicarbonate solution (200 mL) sequentially, and extracted with DCM (200 mL × 3). The combined organic phases were washed with saturated brine (60 mL × 2), dried over anhydrous sodium sulfate, filtered and concentrated under vacuum. The residue was purified by

column chromatography (200-300 mesh silica gel, eluent: petroleum ether:ethyl acetate = 5:1 to 1:10, v/v) to afford the title compound as a yellow solid (5.3 g, yield: 75.2%). LCMS (ESI): $[M+H]^+$ = 286.2, $t_R$= 1.460 min.

*Step 5: Preparation of tert-butyl 3-(3-(benzyloxymethyl)-2-methoxypyridin-4-yl)-3-hydroxypentanoate:*

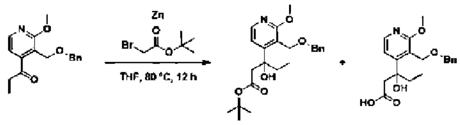


Zinc powder (3.94 g, 60.28 mmol, 4.0 eq.) was suspended in tetrahydrofuran (50 mL). Under a nitrogen atmosphere, iodine (0.76 g, 3.01 mmol, 0.2 eq.) and tert-butyl bromoacetate (11.76 g, 60.28 mmol, 4.0 eq.) were added sequentially at 25 °C, and the mixture was stirred at 80 °C for 10 h. A solution of the intermediate obtained from step 4 (4.3 g, 15.07 mmol, 1.0 eq.) in tetrahydrofuran (10 mL) was added to the system, and the mixture was stirred at 80 °C for an additional 2 h. LCMS monitoring indicated the reaction was complete. The solid was filtered off and washed with ethyl acetate (30 mL × 2). The filtrate was concentrated, and purified by **reversed-phase silica gel column chromatography (C18)** (eluent: water (0.5% formic acid):acetonitrile = 10:1 to 1:5, v/v) to afford a mixture of the product (tert-butyl ester) and the byproduct (carboxylic acid, the product of Step 6) as a yellow solid (6.3 g, crude) and pure byproduct (carboxylic acid, the product of Step 6) as a yellow solid (2.1 g). Product LCMS (ESI): $[M+H]^+$ = 402.3, $t_R$= 1.593 min; Byproduct LCMS (ESI): $[M+H]^+$ = 286.2, $t_R$= 1.460 min.

*Step 6: Preparation of 3-(3-(benzyloxymethyl)-2-methoxypyridin-4-yl)-3-hydroxypentanoic acid:*

The crude mixture of the product (tert-butyl ester) and the byproduct (carboxylic acid, the product of Step 6) (6.3 g) was dissolved in DCM (60 mL). Trifluoroacetic acid (50 mL) was added at 25 °C, and the mixture was stirred at 25 °C for 12 h. LCMS monitoring indicated the reaction was complete. The reaction mixture was concentrated, then water (80 mL) and saturated aqueous sodium bicarbonate solution (60 mL) were added. The mixture was extracted with EA (100 mL×3), and the combined organic phases were washed with saturated aqueous sodium chloride solution (50 mL×3), dried over anhydrous sodium sulfate and filtered, concentrated and purified by column chromatography (100-200 mesh silica gel, eluent: DCM:MeOH=8:1, v/v) to afford the title compound as a yellow solid (2.8 g). LCMS (ESI): $[M+H]^+$ = 346.1, $t_R$ = 0.763 min.

*Step 7: Preparation of 3-hydroxy-3-(3-(hydroxymethyl)-2-methoxypyridin-4-yl)pentanoic acid:*

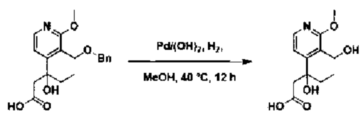


The intermediate obtained from step 6 (4.0 g, 11.58 mmol, 1.0 eq.) was dissolved in MeOH (100 mL). Palladium on carbon (10%) (1.0 g, 25% w/w) and palladium hydroxide (1.0 g, 25% w/w) were added, and the system was **purged with hydrogen three times.** Under a hydrogen atmosphere, the mixture was heated to 40 °C and stirred for 12 h. LCMS monitoring indicated the reaction was complete. The reaction mixture was cooled to room temperature and filtered, and the filter cake was washed with MeOH (20 mL). The filtrate was concentrated under vacuum to afford the crude title compound as a brown solid (1.6 g). LCMS (ESI): $[M+H]^+$ = 256.1, $t_R$= 0.980 min.

*Step 8: Preparation of 5-ethyl-5-hydroxy-9-methoxy-4,5-dihydrooxepino[3,4-c]pyridin-3(1H)-one:*

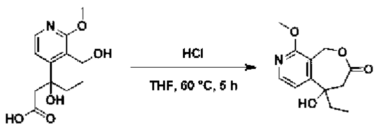

The intermediate obtained from step 7 (1.6 g, 6.27 mmol, 1.0 eq.) was dissolved in THF (100 mL). A 4 M solution of hydrogen chloride in 1,4-dioxane (3.2 mL, 12.53 mmol, 2.0 eq.) was added, and the mixture was stirred at 60 °C for 5 h. LCMS monitoring indicated the reaction was complete. The mixture was cooled to room temperature and concentrated, and purified by **preparative HPLC** (C18 column, 0.1% formic acid, 30-50% acetonitrile in water) to afford the title compound as a yellow solid (600 mg, yield: 40.3%). LCMS (ESI): $[M+H]^+$ = 238.2, $t_R$ = 1.053 min.

*Step 9: Preparation of 5-ethyl-5-hydroxy-1,4,5,8-tetrahydrooxepino[3,4-c]pyridine-3,9-dione:*

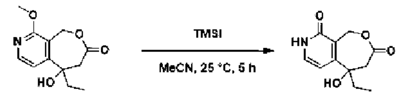


The intermediate obtained from step 8 (600 mg, 2.53 mmol, 1.0 eq.) was dissolved in ACN (20 mL). TMSI (759 mg, 3.79 mmol, 1.5 eq.) was added, and the mixture was stirred at 60 °C for 3 h. LCMS monitoring indicated the reaction was complete. The mixture was cooled to room temperature and filtered, and the filter cake was washed with ACN (3 mL). The filter cake was collected and dried to afford the title compound as a yellow solid (200 mg, yield: 35.6%). LCMS (ESI): $[M+H]^+$ =224.1, $t_R$ = 0.247 min.

*Step 10: Preparation of 5-Ethyl-8-{[6-fluoro-3-iodo-1,8-dimethyl-4-oxo-1,4-dihydroquinolin-2-yl]methyl}-5-hydroxy-1,4,5,8-tetrahydroheptano[3,4-c]pyridine*

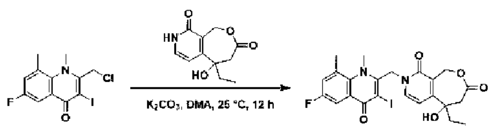


The intermediate from Step 6 of Example 2 was dissolved in DMF (6 mL), followed by addition of intermediate from step 9. The reaction was complete detected by LC-MS, and the mixture was quenched with ice-cold aqueous ammonium chloride (20 mL), extracted with EA (30 mL × 3), and the combined organic phases were washed with saturated brine (10 mL × 2), dried over anhydrous sodium sulfate and filtered. The filtrate was concentrated and purified by silica gel column chromatography (200-300 mesh, PE/THF = 1:1) to afford the title compound as a yellow solid (230.0 mg, 60.8% yield). LCMS(ESI) $[M+H]^+$= 553.1, $t_R$= 1.153 min.

*Step 11: Preparation of 5-Ethyl-9-fluoro-5-hydroxy-11,12-dimethyl-1,4,5,13-tetrahydro-3H,15H-oxaheptano[3,4:6,7]indolizino[2,1 -b]quinoline:*

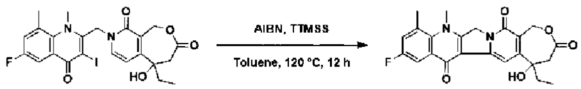


The intermediate obtained from step 10 (180.0 mg, 0.33 mmol, 1.0 eq.) was dissolved in toluene (20 mL). AIBN (53.5 mg, 0.33 mmol, 1.0 eq.) and tris(trimethylsilyl)silane (324.2 mg, 1.3 mmol, 4.0 eq.) were added, and the system was purged with nitrogen three times. The mixture was heated to 120 °C and stirred for 12 h. The reaction mixture was cooled to room temperature and filtered, and the filter cake was slurried with methyl tert-butyl ether (10 mL) to afford a crude product. Purification by preparative HPLC afforded the title compound as a white solid (3.3 mg, yield: 2.3%). LCMS (ESI): $[M+H]^+$ = 425.2, $t_R$= 1.518 min; $^1H$ NMR (400 MHz, DMSO-$d_6$) δ: 7.86 (dd, J = 8.4, 3.2 Hz, 1H), 7.61 (dd, J = 9.2, 2.8 Hz, 1H), 7.23 (s, 1H), 5.90 (s, 1H), 5.47-5.29 (m, 4H), 4.08 (s, 3H), 3.43 (d, J = 13.6 Hz, 1H), 3.02 (d, J = 14.0 Hz, 1H), 2.87 (s, 3H), 1.84-1.73 (m, 2H), 0.84 (t, J = 6.8 Hz, 3H).

**Example 407: Preparation of (S)-11-(benzo[d][1,3]dioxol-5-yl)-4-ethyl-8-fluoro-4-hydroxy-9-(2-hydroxyethyl)-1,12-dihydro-14H-pyrano[3,4:6,7]indolizino[2,1 -b]quinoline-3,6,14(4H,11H)-trione:**

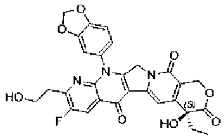

Similarly, with reference to Example 196, the intermediate 2-chloro-5-fluoro-6-(2-((tetrahydro-2H-pyran-2-yl)oxy)ethyl)nicotinonitrile obtained in step 4 of Example 196 was employed as the starting material. The title compound was synthesized via the formation of 2-(benzo[d][1,3]dioxol-5-ylamino)-5-fluoro-6-(2-((tetrahydro-2H-pyran-2-yl)oxy)ethyl)nicotinonitrile, followed by the procedures of steps 6 to 13, affording the title compound as a pale yellow solid. LCMS(ESI)$[M+1]^+$=548.2.

**Example 408: Preparation of (S)-8-methoxy-11-cyclopentyl-4-ethyl-4-hydroxy- 1,12-dihydro-14H-pyrano[3,4:6,7]indolizino[2,1 -b]quinoline-3,6,14(4H,11H)-trione:**

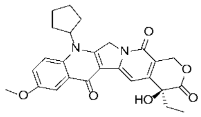

Similarly, following the synthetic procedure of steps 1-8 in Example 2, the title compound 408 was synthesized as a white solid using the compound of 4-methoxyaniline. LCMS(ESI)$[M+1]^+$-463.3, $t_R$= 1.271 min.

**Example 409: Preparation of (S)-8-chloro-11-cyclopentyl-4-ethyl-4-hydroxy- 1,12-dihydro-14H-pyrano[3,4:6,7]indolizino[2,1 -b]quinoline-3,6,14(4H,11H)-trione:**

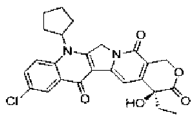

Similarly, following the synthetic procedure of steps 1-8 in Example 2, the title compound 409 was synthesized as a white solid using the compound of 4-chloroaniline. LCMS(ESI)$[M+1]^+$=467.2, $t_R$= 1.407 min.

**Example 410: Preparation of (S)-5-cyclopentyl-12-ethyl-12-hydroxy-9,12 -dihydro-8H-[1,3]dioxolo[4,5-g]pyrano[3,4:6,7]indolizino[2,1 -b]quinoline-8,11,14(5H,6H)-trione:**

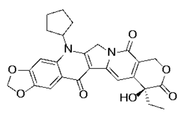

Similarly, following the synthetic procedure of steps 1-8 in Example 2, the title compound 410 was synthesized as a white solid using the compound of benzo[d][1,3]dioxol-5-amine as the starting material. LCMS(ESI)$[M+1]^+$=477.2, $t_R$= 1.539 min.

**Example 411: Preparation of (S)-8-amino-11-cyclopentyl-4-ethyl-4-hydroxy-1,12 -dihydro-14H-pyrano[3,4:6,7] indolizino[2,1 -b]quinoline-3,6,14(4H,11H)-trione:**

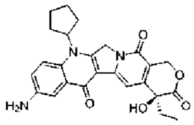

Similarly, following the synthetic procedure of step 2 in Example 20, the title compound 411 was synthesized as a white solid using the compound of Example 409 as the starting material, with the chlorine group converted to a BocNH group. LCMS(ESI)$[M+1]^+$=448.2, $t_R$= 1.039 min.

**Example 412: Preparation of ((S)-11-cyclopentyl-4-ethyl-4-hydroxy-(hydroxy methyl)-1,12-dihydro-14H-pyrano [3,4:6,7]indolizino[2,1 -b]quinoline-3,6,14 (4H,11H)-trione:**

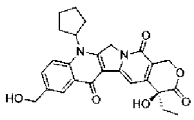

Similarly, following the synthetic procedure described in Example 221, the title compound 412 was synthesized as a white solid using the compound of Example 409 as the starting material, with the chlorine group converted to a hydroxymethyl group. LCMS(ESI)$[M+1]^+$-477.2, $t_R$= 1.539 min.

**Biological Evaluation**

The following test examples are provided to further illustrate and explain the present invention; however, they are not intended to limit the scope of the invention.

**Test Example 1. Determination of Topo I Enzyme-Inhibitory Activity by Agarose-Gel Electrophoresis**

**Principle:** Type-I topoisomerase (Topo I) relaxes positive and negative supercoils by introducing transient single-strand breaks and forming reversible cleavage complexes (Topo I-DNA covalent complexes). Gel electrophoresis can resolve DNA species of identical molecular weight but different topology: supercoiled plasmid migrates fastest, whereas the nicked, relaxed form moves more slowly. Quantifying the decrease in supercoiled DNA therefore provides a direct measure of Topo I activity.

**Experimental Procedure:** A 1× reaction buffer was prepared (20 mM Tris-HCl pH 7.5, 200 mM NaCl, 0.25 mM EDTA, 5 % (v/v) glycerol, 0.5 μg pNO1 plasmid substrate). Test compounds (5 % DMSO final) were added to the buffer, followed by Topo I enzyme (0.75 U per reaction). After gentle mixing, reactions were incubated at 37 °C for 30 min. Positive (enzyme + vehicle) and negative (no enzyme) controls were included in parallel. Reactions were terminated by adding STEB buffer and chloroform/iso-amyl alcohol (24:1, v/v), vortexed and centrifuged for 2 min. The aqueous (upper) phase was loaded onto a 1 % (w/v) agarose gel, electrophoresed for 1-2 h, stained with GelRed, and imaged under UV trans-illumination.

**Data Analysis:** Band intensities of supercoiled DNA were quantified with image-analysis software. Relaxation activity (%) was calculated as (Max-Sample)/ (Max-Min)*100%, where Max = intensity of the no-enzyme negative control, Min = intensity of the vehicle-only positive control, and Sample = intensity of the compound-treated lane. Dose-response data (compound concentration, X; relaxation activity %, Y) were fitted in Excel or GraphPad Prism 5.0 using the four-parameter logistic equation:

Y=Bottom + (Top-Bottom)/(1+(IC50/X)*HillSlope) to derive the $IC_{50}$ value for each compound.

**Conclusion:** Under the above conditions, the exemplified compounds of the present invention exhibited the Topo I inhibitory activities summarized in Table 1.

Table 1. Compound-mediated inhibition of topoisomerase I (Topo I) enzymatic activity

| | |
|---|---|
| Group A (200~500 nM) compounds | 19, 23, 60, 63, 57, 67, 68, 70, 71, 72, 73, 88, 89, 101, 106, 119, 117, 133, 137, 138, 139, 141, 159, 160, 161, 163, 165, 166, 164, 167, 168, 169, 170, 171, 172, 179, 182, 185, 186, 187, 193, 225, 227, 228, 291, 292, 293, 294, 355 |
| Group B(< 200 nM) compounds | 1, 2, 5, 6, 10, 24, 36, 37, 38, 39, 69, 96, 101, 140, 184, 190, 354, |

The data shown in the table indicate that this series of compounds possesses potent inhibitory activity against topoisomerase I (Topo I).

**Test Example 2. Determination of Compound-Mediated Inhibition of Cell Proliferation by CTG (CellTiter-Glo) Luminescence Assay**

**Principle:** The Promega CellTiter-Glo™ luciferase reagent was used. Luciferase consumes ATP to generate light; metabolically active cells continuously produce ATP through respiration and other processes. After adding an equal volume of CellTiter-Glo™ reagent to the culture medium, the resulting luminescence is directly proportional to the ATP content, which in turn correlates with the number of viable cells. Thus, the assay quantifies compound-induced suppression of cell proliferation.

**Experimental Procedure:** HCT116 cells (ATCC) were revived in complete medium (McCoy's 5a + 10 % FBS). After two passages, exponentially growing cells were harvested, counted, and resuspended to the appropriate density. A 100 μL aliquot of cell suspension (2 000 cells per well) was seeded into 96-well plates and incubated overnight (37 °C, 5 % $CO_2$, 100 % relative humidity). Test compounds were dissolved in DMSO to prepare 10 mM stock solutions and serially diluted 1:3 in DMSO to generate nine concentration steps. Each DMSO solution was further diluted 200-fold into medium; 25 μL of the resulting solutions were added to the cell plates (final DMSO concentration 0.25 %). After 72 h incubation (37 °C, 5 % $CO_2$), 50 μL of CellTiter-Glo reagent was added per well. Plates were shaken gently, incubated for 30 min at room temperature in the dark, and luminescence was read on an EnVision multimode reader. Percent inhibition was calculated as:

$$\text{Inhibition } \% = (1 - As / Ac) \times 100,$$

where As = signal from compound-treated wells and Ac = signal from DMSO-only control wells. Concentration-response data were fitted in Excel with the XLfit add-in using the four-parameter logistic equation:

$$Y = Bottom + (Top - Bottom) / [1 + (IC_{50} / X)\text{^}HillSlope],$$

to derive the $IC_{50}$ value for each compound.

Table 2. $IC_{50}$ of compounds

| HCT116, $IC_{50}$ < 20 nM |
|---|
| 1, 2, 5, 6, 10, 24, 36, 37, 38, 39, 69, 140, 184, 190, 354,<br>19, 23, 60, 63, 57, 67, 68, 70, 71, 72, 73, 88, 89, 101, 106, 119, 117, 133, 137, 138, 139, 141, 159, 160, 161, 163, 165, 166, 164, 167, 168, 169, 170, 171, 172, 182, 185, 186, 187, 215, 225, 227, 228, 250, 251, 253, 254, 255,, 256, 257, 258, 259, 260, 261, 263, 264, 267, 268, 270, 271, 273, 274, 277, 278, 279, 280, 281, 282, 283, 305, 310, 311, 312, 313314, 315, 317, 327, 344, 345, 350, 351, 352, 353, 355, 356, 358, 359, 366, 367, 368, 369, 370, 371, 372, 373, 399, 400, 401, 402, 403, 404, 347 |

**Test Example 3. Pharmacokinetic Study for Determining Compound Concentrations in Mice by LC-MS/MS**

**Principle:** LC-MS/MS is used to quantify the plasma concentrations of the test article at successive time points, generating an in-vivo pharmacokinetic profile.

**Procedure:** The test compound was dissolved in DMSO to prepare a 10 mg/mL stock solution. This stock was further diluted to 1 mg/mL with a vehicle consisting of 10 % DMSO + 10 % Solutol HS-15 + 80 % saline. Male CD-1 mice (JH Laboratory Animal Co., LTD) were employed; six animals were assigned per compound group. Doses of 10 mg $kg^{-1}$ (p.o.)

and 2 mg $kg^{-1}$ (i.v.) were administered. Serial blood samples (~50 μL whole blood, $K_2$EDTA anticoagulant) were collected at 0.083, 0.25, 0.5, 1, 2, 4, 8 and 24 h (three mice per time point in a crossover design). Blood was centrifuged at 6 000 g for 5 min at 4 °C; plasma was harvested and stored at -70 °C until analysis. Plasma drug concentrations were determined by LC-MS/MS.

Calibration Curve and QC Preparation: Working solutions were prepared by dilution with MeOH:$H_2O$ (1:1). For each calibration or QC level, 3 μL of working solution was spiked into 57 μL of blank mouse plasma.

**Sample Processing:** To 30 μL of plasma was added 200 μL of methanol containing the internal standard (propranolol, 40 ng $mL^{-1}$). After vortex-mixing for 1 min, the mixture was centrifuged at 12 000 rpm for 10 min. A 100 μL aliquot of the supernatant was transferred to a clean plate for injection.

**Pharmacokinetic Analysis:** Concentration-time data were analyzed with WinNonlin 8.2 using a non-compartmental approach to derive PK parameters including Cmax, Tmax, AUC and terminal elimination half-life ($t_1/_2$). AUC was calculated by the linear-up/log-down trapezoidal method.

Table 3. Pk parameters of compounds

| Cmpd | PK mouse IV | | | | | PK Mouse PO | | | | | |
|---|---|---|---|---|---|---|---|---|---|---|---|
| | **Dose** MPK | **$T_{1/2}$, h** | **$C_{max}$, ng/ mL** | **$AUC_{0-24}$, h*ng/mL** | **CL, ml/min/ kg** | **Dose , MPK** | **$T_{max}$, h** | **$C_{max}$, ng/ mL** | **$AUC_{0-24h}$, h*ng/mL** | **$T_{1/2}$, h** | **F%** |
| **DXd** | 1 | 0.155 | 455 | 154 | 108 | 10 | 0.33 | 6.99 | 4.32 | 0.39 | / |
| **SN38** | 1 | 0.566 | 278 | 103 | 163 | 10 | 0.25 | 1.87 | 0.23 | / | / |
| **2** | 2 | 1.16 | 604 | 323 | 110 | 20 | 0.42 | 956 | 911 | 2.25 | 28.5 |
| **250** | 2 | 0.298 | 1214 | 543 | 62.9 | 20 | 0.5 | 1120 | 1931 | 3.85 | 35.9 |
| **252** | 2 | 1.17 | 558 | 674 | 49.2 | 20 | 1.33 | 680 | 2816 | 5.75 | 43.1 |
| **97** | 1 | 0.482 | 256 | 114 | 146 | 10 | 0.92 | 131 | 395 | 1.26 | 34.5 |
| **257** | 1 | 1.24 | 635 | 352 | 47.4 | 10 | 0.83 | 528 | 1371 | 7.95 | 40.2 |
| **259** | 1 | 0.96 | 497 | 524 | 31.8 | 10 | 0.83 | 1233 | 2404 | 6.24 | 46.6 |
| **117** | 1 | 0.235 | 107 | 38.7 | 439 | N/A | N/A | N/A | N/A | N/A | N/A |

The results demonstrate that the compounds of the present invention exhibit favorable in vivo pharmacokinetic profiles and possess drug-development potential.

**Test Example 4. Pharmacodynamic Evaluation of Compound 2, Compound 250, and Compound 253 in a Subcutaneous Xenograft Model of Human Colon Cancer HCT-116 Cells in BALB/c Female Nude Mice**

Human colon cancer HCT116 cells were cultured in vitro as monolayers in McCoys 5a medium supplemented with 10% fetal bovine serum and 1% penicillin/streptomycin/amphotericin B at 37 °C in a 5% CO2 incubator. Cells were routinely passaged twice a week using trypsin-EDTA digestion. When cells reached 80%-90% confluence and the required cell number was achieved, cells were harvested. HCT116 cells (0.1 mL, $5\times10^6$ cells) were subcutaneously inoculated into the right dorsal region near the upper forelimb of each mouse (BALB/c nude mice, female, 6-8 weeks old). Treatment was initiated when the average tumor volume reached approximately 158 $mm^3$. Tumor volume was measured twice weekly. At the end of the experiment, the tumor growth inhibition rate (TGI) was calculated as: TGI (%) = [1 - (average tumor volume at the end of treatment in a particular group - average tumor volume at the start of treatment in that group) / (average tumor volume at the end of treatment in the vehicle control group - average tumor volume at the start of treatment in the vehicle control group)] × 100%.

Experimental Groups and Dosing Regimen:

1, Vehicle control group
2, Compound 2, 5 mg/kg, IV, BIW×3W
3, Compound 250, 5 mg/kg, IV, BIW×3W
4, Compound 253, 5 mg/kg, IV, BIW×3W

As shown in Figure 1, on day 21 post-treatment, the tumor volume in the vehicle control group was 1146 $mm^3$, while the Compound 2 group had a tumor volume of 335 $mm^3$, with a TGI of 81.9%; the Compound 250 group had a tumor volume of 326 $mm^3$, with a TGI of 83.1%; and the Compound 253 group had a tumor volume of 351 $mm^3$, with a TGI of 80.6%. All three compounds significantly inhibited tumor growth, and the animals tolerated the treatment well with no abnormal changes in body weight.

**Test Example 5: Pharmacodynamic Evaluation of Compound 117, Compound 250, and Compound 253 in the CR00090 Human Colon Cancer Model**

Human colon cancer CR00090 cells were thawed, counted, and mixed with Matrigel at a 1:1 ratio. Then, 0.1 mL ($5 \times 10^5$ cells) of CR00090 cells were subcutaneously inoculated into the right dorsal region near the upper forelimb of each mouse (NPSG mice, female, 6-8 weeks old). Treatment was initiated when the average tumor volume reached approximately 84 $mm^3$. Tumor volume was measured twice weekly. At the end of the experiment, the tumor growth inhibition rate (TGI) was calculated as: TGI (%) = [1 - (average tumor volume at the end of treatment in a particular group - average tumor volume at the start of treatment in that group) / (average tumor volume at the end of treatment in the vehicle control group - average tumor volume at the start of treatment in the vehicle control group)] $\times$ 100%.

Experimental Groups and Dosing Regimen:

1, Vehicle control group
2, Compound 117, 5 mg/kg, IV, BIW×4W
3, Compound 250, 5 mg/kg, IV, BIW×4W
4, Compound 253, 5 mg/kg, IV, BIW×4W

As shown in Figure 2, on day 28 post-treatment, the tumor volume in the vehicle control group was 1093 $mm^3$, while the Compound 117 group had a tumor volume of 262 $mm^3$, with a TGI of 82.4%; the Compound 250 group had a tumor volume of 286 $mm^3$, with a TGI of 80.0%; and the Compound 253 group had a tumor volume of 323 $mm^3$, with a TGI of 76.3%. All three compounds significantly inhibited tumor growth.

## Claims

1. A compound represented by formula (I) or (I)B below, and its stereoisomers, geometric isomers, tautomers, pharmaceutically acceptable salts, prodrugs, solvates, esters, polymorphs, N-oxides, metabolites or isotope-labeled analogs,

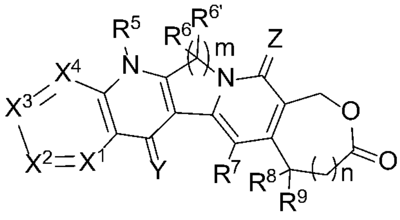

**(I)**

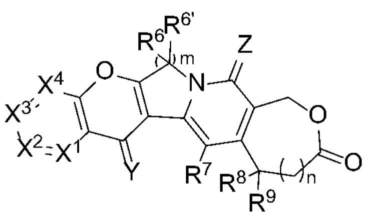

(I)B

wherein,

Y is selected from S, O, $-CR^{10a}R^{10b}$ or $-NR^{10a}$, wherein $R^{10a}$ and $R^{10b}$ are independently selected from hydrogen, hydroxy, amino, alkyl, alkenyl, alkynyl, cycloalkyl, heterocycloalkyl, aryl and heteroaryl;
Z is selected from S, O, $-CR^{11a}R^{11b}$ or $-NR^{11a}$, wherein $R^{11a}$ and $R^{11b}$ are independently selected from hydrogen, hydroxy, amino, alkyl, alkenyl, alkynyl, cycloalkyl, heterocycloalkyl, aryl and heteroaryl;
n is 0, 1,2 or 3; m is 0, 1, 2 or 3;
$X^1$ is selected from N or $C(R^1)$, where $R^1$ is selected from hydrogen, deuterium, halogen, hydroxy, mercapto, amino, cyano, nitro, optionally substituted alkyl, optionally substituted alkoxy, optionally substituted cycloalkyl,

optionally substituted heterocycloalkyl, $-OR^{1a}$, $-SR^{1a}$, $-NR^{1a}R^{1b}$, $-COR^{1a}$, $-SOR^{1a}$, $-SO_2R^{1a}$, $-COOR^{1a}$, $-CONR^{1a}R^{1b}$, $-NR^{1a}COR^{1b}$, $-OCOR^{1a}$, $-SONR^{1a}R^{1b}$, $-SO_2NR^{1a}R^{1b}$, $-NR^{1a}SOR^{1b}$, $-NR^{1a}SO_2R^{1b}$ and $-Si(R^{1a})(R^{1b})(R^{1c})$, and $R^{1a}$, $R^{1b}$ and $R^{1c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

$X^2$ is selected from N or $C(R^2)$, where $R^2$ is selected from hydrogen, deuterium, halogen, hydroxy, mercapto, amino, cyano, nitro, optionally substituted alkyl, optionally substituted alkoxy, optionally substituted cycloalkyl, optionally substituted heterocycloalkyl, $-OR^{2a}$, $-SR^{2a}$, $-NR^{2a}R^{2b}$, $-COR^{2a}$, $-SOR^{2a}$, $-SO_2R^{2a}$, $-COOR^{2a}$, $-CONR^{2a}R^{2b}$, $-NR^{2a}COR^{2b}$, $-OCOR^{2a}$, $-SONR^{2a}R^{2b}$, $-SO_2NR^{2a}R^{2b}$, $-NR^{2a}SOR^{2b}$, $-NR^{2a}SO_2R^{2b}$ and $-Si(R^{2a})(R^{2b})(R^{2c})$, and $R^{2a}$, $R^{2b}$ and $R^{2c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

Alternatively, R1 and R2 together with the atoms to which they are attached form an optionally substituted cyclic structure.

$X^3$ is selected from N or $C(R^3)$, wherein $R^3$ is selected from hydrogen, deuterium, halogen, hydroxy, mercapto, amino, cyano, nitro, optionally substituted alkyl, optionally substituted alkoxy, optionally substituted cycloalkyl, optionally substituted heterocycloalkyl, $-OR^{3a}$, $-SR^{3a}$, $-NR^{3a}R^{3b}$, $-COR^{3a}$, $-SOR^{3a}$, $-SO_2R^{3a}$, $-COOR^{3a}$, $-CONR^{3a}R^{3b}$, $-NR^{3a}COR^{3b}$, $-OCOR^{3a}$, $-SONR^{3a}R^{3b}$, $-SO_2NR^{3a}R^{3b}$, $-NR^{3a}SOR^{3b}$, $-NR^{3a}SO_2R^{3b}$ and $-Si(R^{3a})(R^{3b})(R^{3c})$ , and $R^{3a}$, $R^{3b}$ and $R^{3c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

or $R^2$ and $R^3$ together with the atoms to which they are attached form an optionally substituted cyclic structure;

$X^4$ is selected from N or $C(R^4)$, wherein $R^4$ is selected from hydrogen, deuterium, halogen, hydroxy, mercapto, amino, cyano, nitro, optionally substituted alkyl, optionally substituted alkoxy, optionally substituted cycloalkyl, optionally substituted heterocycloalkyl, $-OR^{4a}$, $-SR^{4a}$, $-NR^{4a}R^{4b}$, $-COR^{4a}$, $-SOR^{4a}$, $-SO_2R^{4a}$, $-COOR^{4a}$, $-CONR^{4a}R^{4b}$, $-NR^{4a}COR^{4b}$, $-OCOR^{4a}$, $-SONR^{4a}R^{4b}$, $-SO_2NR^{4a}R^{4b}$, $-NR^{4a}SOR^{4b}$, $-NR^{4a}SO_2R^{4b}$ and $-Si(R^{4a})(R^{4b})(R^{4c})$, and $R^{4a}$, $R^{4b}$ and $R^{4c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

or $R^3$ and $R^4$ together with the atoms to which they are attached form an optionally substituted cyclic structure;

$R^5$ is selected from hydrogen, deuterium, hydroxy, amino, optionally substituted alkyl, optionally substituted alkoxy, optionally substituted cycloalkyl, optionally substituted heterocycloalkyl, optionally substituted aryl, optionally substituted heteroaryl, $-OR^{5a}$, $-SR^{5a}$, $-NR^{5a}R^{5b}$, $-COR^{5a}$, $-SOR^{5a}$, $-SO_2R^{5a}$, $-COOR^{5a}$, $-CONR^{5a}R^{5b}$, $-NR^{5a}COR^{5b}$, $-OCOR^{5a}$, $-SONR^{5a}R^{5b}$, $-SO_2NR^{5a}R^{5b}$, $-NR^{5a}SOR^{5b}$, $-NR^{5a}SO_2R^{5b}$ and $-Si(R^{5a})(R^{5b})(R^{5c})$, and $R^{5a}$, $R^{5b}$ and $R^{5c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

$R^6$ and $R^{6'}$ are independently selected from hydrogen, deuterium, hydroxy, amino, optionally substituted alkyl, optionally substituted alkoxy, optionally substituted cycloalkyl, optionally substituted heterocycloalkyl, $-OR^{6a}$, $-SR^{6a}$, $-NR^{6a}R^{6b}$, $-COR^{6a}$, $-SOR^{6a}$, $-SO_2R^{6a}$, $-COOR^{6a}$, $-CONR^{6a}R^{6b}$, $-NR^{6b}COR^{6a}$, $-OCOR^{6a}$, $-SONR^{6a}R^{6b}$, $-SO_2NR^{6a}R^{6b}$, $-NR^{6a}SOR^{6b}$, $-NR^{6a}SO_2R^{6b}$ and $-Si(R^{6a})(R^{6b})(R^{6c})$, and $R^{6a}$, $R^{6b}$ and $R^{6c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

$R^7$ is selected from hydrogen, deuterium, halogen, hydroxy, mercapto, amino, cyano, nitro, optionally substituted alkyl, optionally substituted alkoxy, optionally substituted cycloalkyl, optionally substituted heterocycloalkyl, $-OR^{7a}$, $-SR^{7a}$, $-NR^{7a}R^{7b}$, $-COR^{7a}$, $-SOR^{7a}$, $-SO_2R^{7a}$, $-COOR^{7a}$, $-CONR^{7a}R^{7b}$, $-NR^{7a}COR^{7b}$, $-OCOR^{7a}$, $-SONR^{7a}R^{7b}$, $-SO_2NR^{7a}R^{7b}$, $-NR^{7a}SOR^{7b}$, $-NR^{7a}SO_2R^{7b}$ and $-Si(R^{7a})(R^{7b})(R^{7c})$, wherein $R^{7a}$, $R^{7b}$ and $R^{7c}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl;

$R^8$ is selected from hydrogen, deuterium, halogen, $-OR^{8a}$, $-OCOR^{8a}$, $-SR^{8a}$ ,$-OR^{8a}$-$SR^{8b}$ or $-NR^{8a}R^{8b}$, wherein $R^{8a}$ and $R^{8b}$ are independently selected from hydrogen, deuterium, optionally substituted alkyl and optionally substituted cycloalkyl;

$R^9$ is selected from $-CH_2R^{9a}$, $-CF_2R^{9a}$, $-CHFR^{9a}$, $-CD_2R^{9a}$, $-CDHR^{9a}$, $-CDFR^{9a}$, $-OR^{9a}$, $-SR^{9a}$ and $-NR^{9a}R^{9b}$, wherein $R^{9a}$ and $R^{9b}$ are independently selected from hydrogen, deuterium, optionally substituted alkyl, optionally substituted alkenyl, optionally substituted alkynyl and optionally substituted cycloalkyl.

2. The compound according to claim 1, wherein Y is O.

3. The compound according to any one of the preceding claims, wherein Z is S or O.

4. The compound according to any one of the preceding claims, wherein $R^1$ is selected from unsubstituted alkyl, alkoxy, cycloalkyl or heterocycloalkyl; and alkyl, alkoxy, cycloalkyl or heterocycloalkyl substituted with one or more substituents independently selected from deuterium, -Boc, -Cbz, halogen, hydroxy, mercapto, amino, cyano, nitro, $-(CH_2)_oOR^{1d}$, -(CH2)oSR1d, $-(CH_2)_oNR^{1d}R^{1e}$, $-(CH_2)_oCOR^{1d}$, $-(CH_2)_oSOR^{1d}$, $-(CH_2)_oSO_2R^{1d}$, $-(CH_2)_oCOOR^{1d}$, $-(CH_2)_oCONR^{1d}R^{1e}$, $-(CH_2)_oNR^{1d}COR^{1e}$, $-(CH_2)_oOCOR^{1d}$, $-(CH_2)_oSONR^{1d}R^{1e}$, $-(CH_2)_oSO_2NR^{1d}R^{1e}$, $-(CH_2)_oNR^{1d}SOR^{1e}$, $-(CH_2)_oNR^{1d}SO_2R^{1e}$, =O, $=N-OR^{1d}$, $=N-NR^{1d}R^{1e}$, $-(CH_2)_oSi(R^{1d})(R^{1e})(R^{1f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{1d}$, $R^{1e}$ and $R^{1f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

5. The compound according to any one of the preceding claims, wherein $R^{1a}$, $R^{1b}$ and R1c are independently selected from hydrogen; deuterium; -Boc; -Cbz; unsubstituted alkyl, alkenyl, alkynyl, cycloalkyl, heterocycloalkyl, aryl or heteroaryl; and alkyl, alkenyl, alkynyl, cycloalkyl, heterocycloalkyl, aryl or heteroaryl substituted with one or more substituents independently selected from deuterium, halogen, hydroxy, mercapto, amino, cyano, nitro, carboxyl, phenyl, optionally substituted alkyl, optionally substituted alkoxy, optionally substituted cycloalkyl and optionally substituted heterocycloalkyl.

6. The compound according to any one of the preceding claims, wherein $R^1$ is selected from hydrogen or halogen.

7. The compound according to any one of the preceding claims, wherein $R^2$ is selected from unsubstituted alkyl, alkoxy, cycloalkyl or heterocycloalkyl; and alkyl, alkoxy, cycloalkyl or heterocycloalkyl substituted with one or more substituents independently selected from the following: deuterium, -Boc, -Cbz, halogen, hydroxy, mercapto, amino, cyano, nitro, $-(CH_2)_oOR^{2d}$, $-(CH_2)_oSR^{2d}$, $-(CH_2)_oNR^{2d}R^{2e}$, $-(CH_2)_oCOR^{2d}$, $-(CH_2)_oSOR^{2d}$, $-(CH_2)_oSO_2R^{2d}$, $-(CH_2)_o$-$COOR^{2d}$, $-(CH_2)_oCONR^{2d}R^{2e}$, $-(CH_2)_oNR^{2d}COR^{2e}$, -(CH2)o$COR^{2d}$, $-(CH_2)_oSONR^{2d}R^{2e}$, $-(CH_2)_oSO_2NR^{2d}R^{2e}$, $-(CH_2)_oNR^{2d}SOR^{2e}$, $-(CH_2)_oNR^{2d}SO_2R^{2e}$, =O, $=N-OR^{2d}$, $=N-NR^{2d}R^{2e}$, $-(CH_2)_oSi(R^{2d})(R^{2e})(R^{2f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{2d}$, $R^{2e}$ and $R^{2f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

8. The compound according to any one of the preceding claims, wherein $R^{2a}$, $R^{2b}$ and $R^{2c}$ are independently selected from hydrogen; deuterium; -Boc; -Cbz; unsubstituted alkyl, alkenyl, alkynyl, cycloalkyl, heterocycloalkyl, aryl or heteroaryl; and alkyl, alkenyl, alkynyl, cycloalkyl, heterocycloalkyl, aryl or heteroaryl substituted with one or more substituents independently selected from deuterium, halogen, hydroxy, mercapto, amino, cyano, nitro, carboxyl, phenyl, optionally substituted alkyl, optionally substituted alkoxy, optionally substituted cycloalkyl and optionally substituted heterocycloalkyl.

9. The compound according to any one of the preceding claims, wherein $R^2$ is selected from hydrogen, amino, halogen, hydroxy, alkoxy, alkyl or substituted alkyl.

10. The compound according to any one of the preceding claims, wherein $R^3$ is selected from unsubstituted alkyl, alkoxy, cycloalkyl or heterocycloalkyl; and alkyl, alkoxy, cycloalkyl or heterocycloalkyl substituted with one or more substituents independently selected from the following: deuterium, -Boc, -Cbz, halogen, hydroxy, mercapto, amino, cyano, nitro, $-(CH_2)_oOR^{3d}$, $-(CH_2)_oSR^{3d}$, $-(CH_2)_oNR^{3d}R^{3e}$, $-(CH_2)_oCOR^{3d}$, $-(CH_2)_oSOR^{3d}$, $-(CH_2)^oSO_2 R^{3d}$, $-(CH_2)_o$-$COOR^{3d}$, $-(CH_2)_oCONR^{3d}R^{3e}$, $-(CH_2)_oNR^{3d}COR^{3e}$, $-(CH_2)_oOCOR^{3d}$, $-(CH_2)_oSONR^{3d}R^{3e}$, - (CH2)o$SO_2NR^{3d}R^{3e}$, $-(CH_2)_oNR^{3d}SOR^{3e}$, $-(CH_2)_oNR^{3d}SO_2R^{3e}$, =O, $=N-OR^{3d}$, $=N-NR^{3d}R^{3e}$, $-(CH_2)_oSi(R^{3d})(R^{3e})(R^{3f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{3d}$, $R^{3e}$ and $R^{3f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

**11.** The compound according to any one of the preceding claims, wherein $R^{3a}$, $R^{3b}$ and $R^{3c}$ are independently selected from hydrogen; deuterium; -Boc; -Cbz; unsubstituted alkyl, alkenyl, alkynyl, cycloalkyl, heterocycloalkyl, aryl or heteroaryl; and alkyl, alkenyl, alkynyl, cycloalkyl, heterocycloalkyl, aryl or heteroaryl substituted with one or more substituents independently selected from deuterium, halogen, hydroxy, mercapto, amino, cyano, nitro, carboxyl, phenyl, optionally substituted alkyl, optionally substituted alkoxy, optionally substituted cycloalkyl and optionally substituted heterocycloalkyl.

**12.** The compound according to any one of the preceding claims, wherein $R^3$ is selected from hydrogen; halogen; amino; alkyl; alkoxy; cycloalkyl; $-NR^{2a}COR^{2b}$, where $R^{2a}$ is selected from hydrogen, alkyl or cycloalkyl and $R^{26}$ is selected from alkyl or alkyl substituted with hydroxy or amino; alkyl substituted with halogen, hydroxy, cycloalkyl and/or heterocycloalkyl; alkyl substituted with $-(CH_2)_oNR^{3d}R^{3e}$, where o is independently 0, 1, 2 or 3, and $R^{3d}$ and $R^{3e}$ are independently selected from hydrogen, -Boc, alkyl, cycloalkyl, halogenated cycloalkyl and alkyl substituted with cycloalkyl; alkyl substituted with $-(CH_2)_oNR^{3d}COR^{3e}$, where o is 0 and $R^{3d}$ and $R^{3e}$ are independently selected from hydrogen, alkyl, cycloalkyl, and hydroxyalkyl; and alkyl substituted with $-(CH_2)_oOCOR^{3d}$, where o is 0 and $R^{3d}$ is selected from alkyl or alkyl substituted with amino.

**13.** The compound according to any one of the preceding claims, wherein R4 is selected from unsubstituted alkyl, alkoxy, cycloalkyl or heterocycloalkyl; and alkyl, alkoxy, cycloalkyl or heterocycloalkyl substituted with one or more substituents independently selected from the following: deuterium, -Boc, -Cbz, halogen, hydroxy, mercapto, amino, cyano, nitro, $-(CH_2)_oOR^{4d}$, $-(CH_2)_oSR^{4d}$, $-(CH_2)_oNR^{4d}R^{4e}$, $-(CH_2)_oCOR^{4d}$, $-(CH_2)_oSOR^{4d}$, $-(CH_2)_oSO_2R^{4d}$, $-(CH_2)_o$-$COOR_{4d}$, $-(CH_2)_oCONR^{4d}R^{4e}$, $-(CH_2)_oNR^{4d}COR^{4e}$, $-(CH_2)_oOCOR^{4d}$, $-(CH_2)_oSONR^{4d}R^{4e}$, $-(CH_2)_oSO_2NR^{4d}R^{4e}$, $-(CH_2)_oNR^{4d}SOR^{4e}$, $-(CH_2)_oNR^{4d}SO_2R^{4e}$, =O, $=N-OR^{4d}$, $=N-NR^{4d}R^{4e}$, $-(CH_2)_oSi(R^{4d})(R^{4e})(R^{4f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{4d}$, $R^{4e}$ and $R^{4f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

**14.** The compound according to any one of the preceding claims, wherein $R^{4a}$, $R^{4b}$ and $R^{4c}$ are independently selected from hydrogen; deuterium; -Boc; -Cbz; unsubstituted alkyl, alkenyl, alkynyl, cycloalkyl, heterocycloalkyl, aryl or heteroaryl; and alkyl, alkenyl, alkynyl, cycloalkyl, heterocycloalkyl, aryl or heteroaryl substituted with one or more substituents independently selected from deuterium, halogen, hydroxy, mercapto, amino, cyano, nitro, carboxyl, phenyl, optionally substituted alkyl, optionally substituted alkoxy, optionally substituted cycloalkyl and optionally substituted heterocycloalkyl.

**15.** The compound according to any one of the preceding claims, wherein $R^4$ is selected from hydrogen; halogen; hydroxy; mercapto; $-NR^{4a}R^{4b}$; alkyl; alkoxy; alkyl substituted with halogen, cycloalkyl, heterocycloalkyl, $-OR^{4d}$, $-NR^{4d}R^{4e}$, =O, $=N-OR^{4d}$ and/or $=N-NR^{4d}R^{4e}$; and alkoxy substituted with heterocycloalkyl, $-OR^{4d}$, $-COOR^{4d}$ and/or $-NR^{4d}R^{4e}$, where o is independently 0, 1, 2 or 3, and $R^{4a}$, $R^{4b}$, $R^{4d}$ and $R^{4e}$ are independently selected from hydrogen, -Boc, alkyl, cycloalkyl, cycloalkylalkyl, halogenated cycloalkyl, hydroxycycloalkyl.

**16.** The compound according to any one of the preceding claims, wherein $R^5$ is selected from unsubstituted alkyl, alkoxy, cycloalkyl, heterocycloalkyl, aryl or heteroaryl; and alkyl, alkoxy, cycloalkyl, heterocycloalkyl, aryl or heteroaryl substituted with one or more substituents independently selected from deuterium, -Boc, -Cbz, halogen, hydroxy, mercapto, amino, cyano, nitro, $-(CH_2)_oOR^{5d}$, $-(CH_2)_oSR^{5d}$, $-(CH_2)_oNR^{5d}R^{5e}$, $-(CH_2)_oCOR^{5d}$, $-(CH_2)_oSOR^{5d}$, $-(CH_2)_o$-$SO_2R^{5d}$, $-(CH_2)_oCOOR^{5d}$, -(CH2)oCONR5dR5e, $-(CH_2)_oNR^{5d}COR^{5e}$, $-(CH_2)_oOCOR^{5d}$, $-(CH_2)_oSONR^{5d}R^{5e}$, $-(CH_2)_oSO_2NR^{5d}R^{5e}$, $-(CH_2)_oNR^{5d}SOR^{5e}$, $-(CH_2)_oNR^{5d}SO_2R^{5e}$, =O, $=N-OR^{5d}$ $=N-NR^{5d}R^{5e}$, $-(CH_2)_oSi(R^{5d})(R^{5e})(R^{5f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{5d}$, $R^{5e}$ and $R^{5f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

**17.** The compound according to any one of the preceding claims, wherein $R^{5a}$, $R^{5b}$ and $R^{5c}$ are independently selected from hydrogen; deuterium; -Boc; -Cbz; unsubstituted alkyl, alkenyl, alkynyl, cycloalkyl, heterocycloalkyl, aryl or heteroaryl; and alkyl, alkenyl, alkynyl, cycloalkyl, heterocycloalkyl, aryl or heteroaryl substituted with one or more substituents independently selected from deuterium, halogen, hydroxy, mercapto, amino, cyano, nitro, carboxyl, phenyl, optionally substituted alkyl, optionally substituted alkoxy, optionally substituted cycloalkyl and optionally

substituted heterocycloalkyl.

**18.** The compound according to any one of the preceding claims, wherein $R^5$ is selected from hydrogen; $-COR^{5d}$, where $R^{5a}$ is a heterocycloalkyl; alkyl; cycloalkyl; heterocycloalkyl; aryl; heteroaryl; alkyl substituted with $-OR^{5d,}$ $-N^{R5}dR^{5e}$, $-COR^{5d}$, $-COOR^{5d}$, $-NR^{5d}COR^{5e}$, $-NR^{5d}SO_2R^{5e}$, $=N-OR^{5d}$, a heterocycloalkyl, a heteroaryl, and/or a heterocycloalkyl substituted with halogen, acyl, carbonyl, alkyl, cycloalkyl and/or halogenated cycloalkyl; unsubstituted or substituted by $-(CH_2)_oOR^{5d}$ and/or $-(CH2)_oNR^{5d}R^{5e}$ -alkylene - cycloalkyl; cycloalkyl substituted with halogen, alkyl, heterocycloalkyl, hydroxyalkyl, $-(CH_2)_oOR^{5d}$, $-(CH_2)_oNR^{5d}R^{5e}$ or $-(CH_2)_oNR^{5d}COR^{5e}$; a heterocycloalkyl substituted with hydroxy, -Boc, -Cbz, $-(CH2)_oCOR^{5d}$, alkyl, hydroxyalkyl, deuteroalkyl, haloalkyl, cycloalkylalkyl, heterocycloalkyl, cycloalkyl and/or cycloalkyl substituted with halogen, hydroxy, alkyl, hydroxyalkyl and/or haloalkyl; aryl substituted with halogen, $-OR^{5d}$, $-NR^{5d}R^{5e}$, alkyl, haloalkyl and/or hydroxyalkyl; heteroaryl substituted with alkyl, hydroxyalkyl, cycloalkyl, $-(CH_2)_oNR^{5d}R^{5e}$, $-(CH_2)_oCOOR^{5d}$ and/or $-(CH_2)_oSO_2R^{5d}$. where o is independently 0, 1, 2 or 3, and $R^{5d}$ and $R^{5e}$ are independently selected from hydrogen, -Boc, alkyl, cycloalkyl, heterocycloalkyl, and alkyl or cycloalkyl substituted with deuterium, halogen, hydroxy, alkyl, cycloalkyl and/or phenyl.

**19.** The compound according to any one of the preceding claims, wherein $R^6$ and $R^{6'}$ are independently selected from unsubstituted alkyl, alkoxy, cycloalkyl or heterocycloalkyl; and alkyl, alkoxy, cycloalkyl or heterocycloalkyl substituted with one or more substituents independently selected from the following: deuterium, -Boc, -Cbz, halogen, hydroxy, mercapto, amino, cyano, nitro, $-(CH_2)_oOR^{6d}$, $-(CH_2)_oSR^{6d}$, $-(CH_2)_oNR^{6d}R^{6e}$, $-(CH_2)_oCOR^{6d}$, $-(CH_2)_oSOR^{6d}$, $-(CH_2)_oSO_2R^{6d}$, $-(CH_2)_oCOOR^{6d}$, $-(CH_2)_oCONR^{6d}R^{6e}$, $-(CH_2)_oNR^{6d}COR^{6e}$, $-(CH_2)_oOCOR^{6d}$, $-(CH_2)_oSONR^{6d}R^{6e}$, $-(CH_2)_oSO_2NR^{6d}R^{6e}$, $-(CH_2)_oNR^{6d}SOR^{6e}$, $-(CH_2)_oNR^{6d}SO_2R^{6e}$, =O, $=N-OR^{6d}$ $=N-NR^{6d}R^{6e}$, $-(CH_2)_oSi(R^{6d})(R^{6e})(R^{6f})$, optionally substituted alkyl, optionally substituted cycloalkyl, optionally substituted heterocycloalkyl, optionally substituted aryl and optionally substituted heteroaryl, where o is independently 0, 1, 2 or 3, and $R^{6d}$, $R^{6e}$ and $R^{6f}$ are independently selected from hydrogen, deuterium, -Boc, -Cbz, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

**20.** The compound according to any one of the preceding claims, wherein $R^6$ and $R^{6'}$ are independently selected from hydrogen, alkyl and-$(CH_2)_pOR^{6a}$, $-(CH_2)_pSR^{6a}$, $-(CH_2)_pNR^{6a}R^{6b}$, $-(CH_2)_pCOR^{6a}$, where p is 0, 1, 2or 3.

**21.** The compound according to any one of the preceding claims, wherein $R^1$ and $R^2$, $R^2$ and $R^3$, or $R^3$ and $R^4$, together with the atoms to which they are attached, form an optionally substituted 5- to 12- membered cyclic structure.

**22.** The compound according to any one of the preceding claims, wherein $R^7$ is hydrogen.

**23.** The compound according to any one of the preceding claims, wherein $R^{8a}$ and $R^{8b}$ are independently selected from hydrogen, alkyl, and alkyl substituted with hydroxy, amino and/or carboxyl groups.

**24.** The compound according to any one of the preceding claims, wherein $R^{9a}$ and $R^{9b}$ are independently selected from alkyl.

**25.** The compound according to claim 1, wherein the compound is selected from :

| | |
|---|---|
| 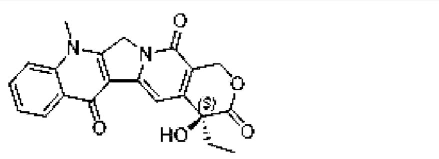 | 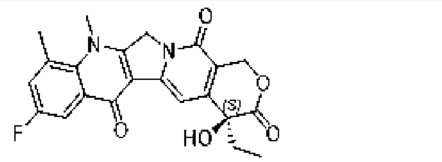 |
| 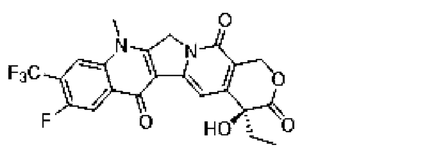 | 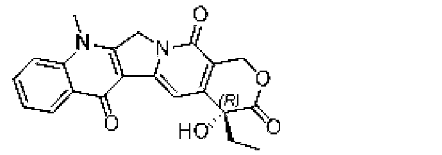 |

| | |
|---|---|
| 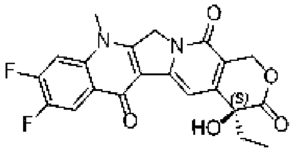 | 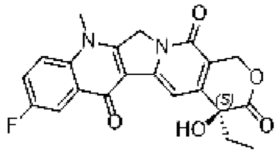 |
| 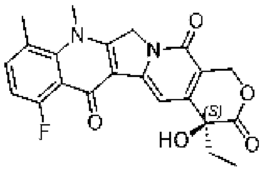 | 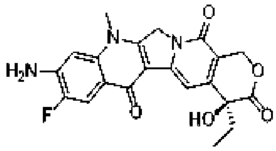 |
| 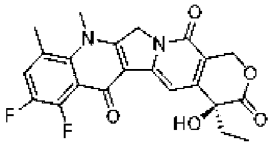 | 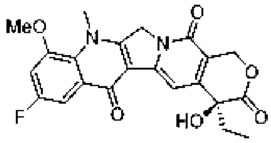 |
| 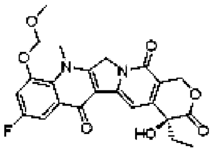 | 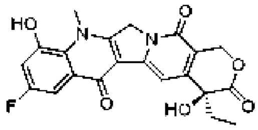 |
| 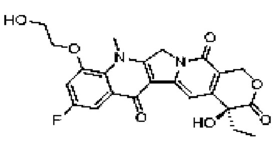 | 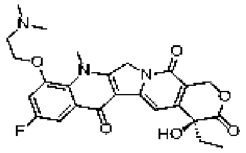 |
| 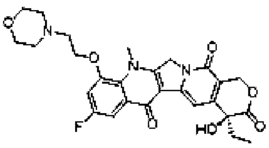 | 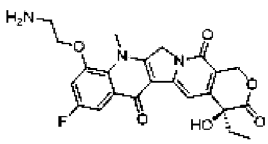 |
| 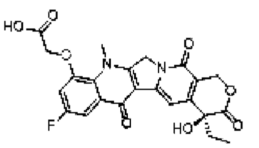 | 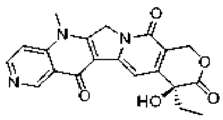 |
| 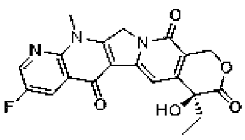 | 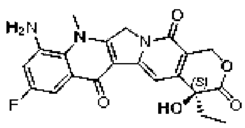 |
| 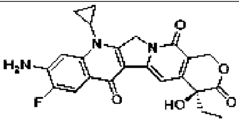 | 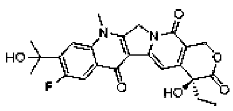 |
| 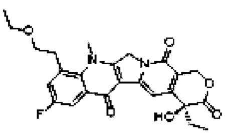 | 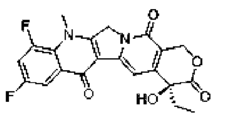 |
| 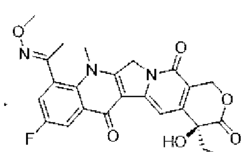 | 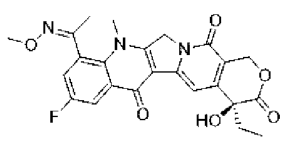 |

| | |
|---|---|
| 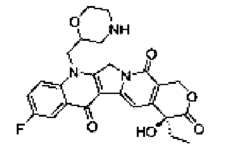 | 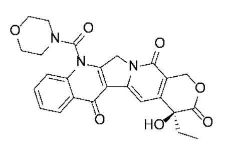 |
| 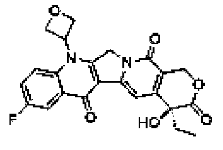 | 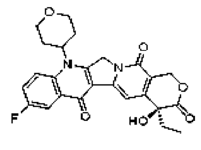 |
| 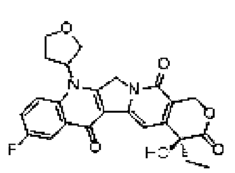 | 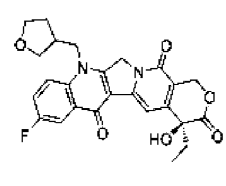 |
| 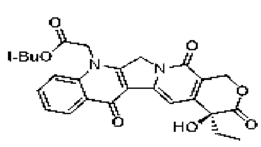 | 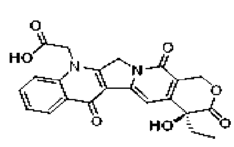 |
| 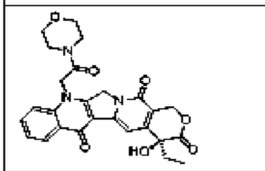 | 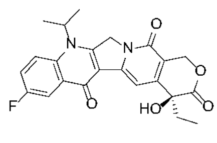 |
| 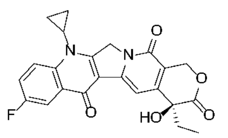 | 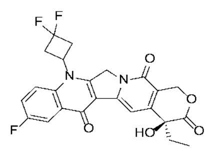 |
| 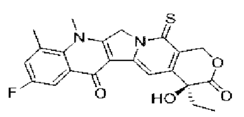 | 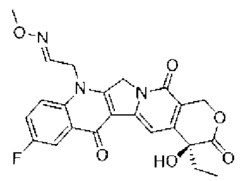 |
| 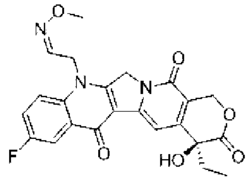 | 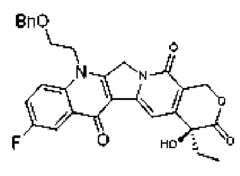 |
| 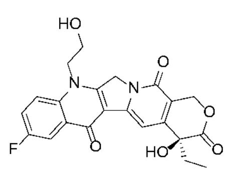 | 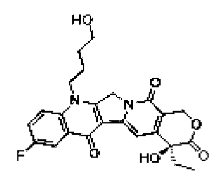 |
| 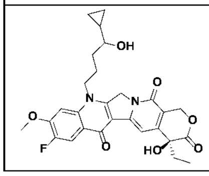 | 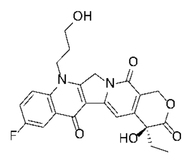 |

| | |
|---|---|
| 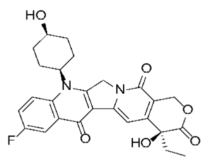 | 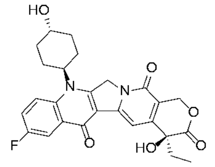 |
| 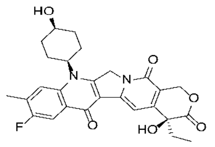 | 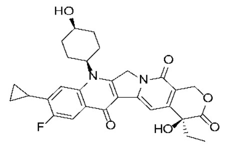 |
| 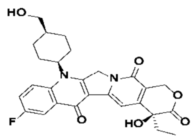 | 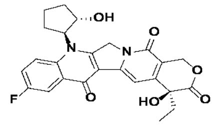 |
| 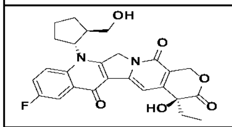 | 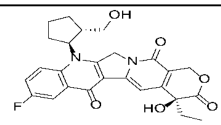 |
| 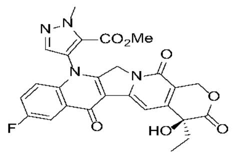 | 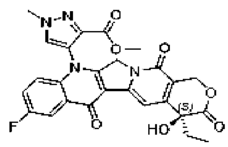 |
| 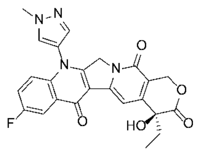 | 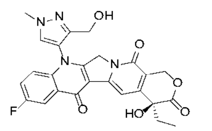 |
| 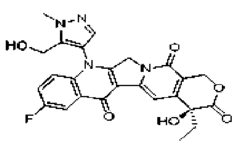 | 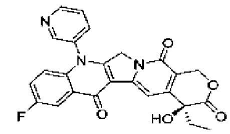 |
| 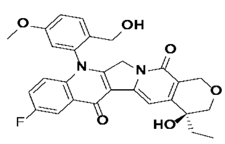 | 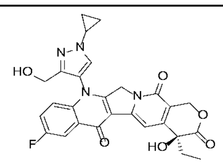 |
| 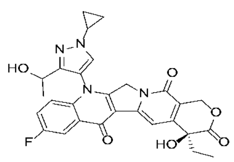 | 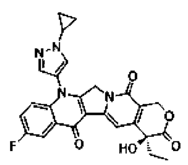 |
| 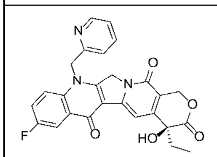 | 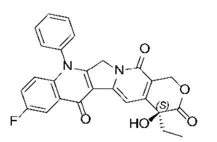 |
| 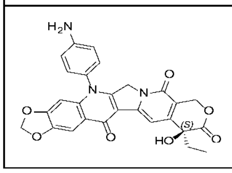 | 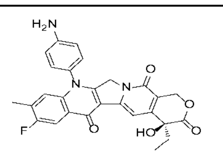 |

| | |
|---|---|
| 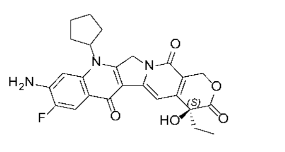 | 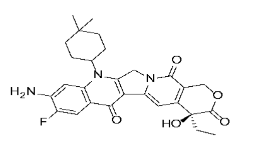 |
| 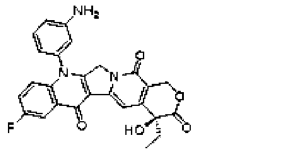 | 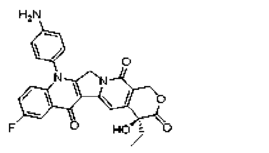 |
| 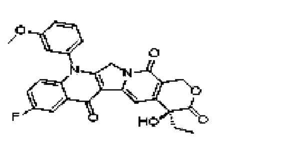 | 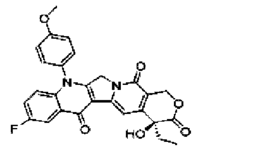 |
| 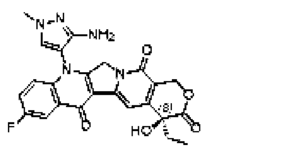 | 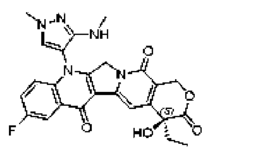 |
| 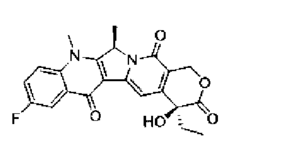 | 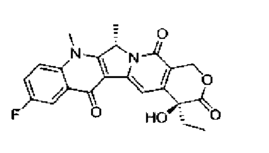 |
| 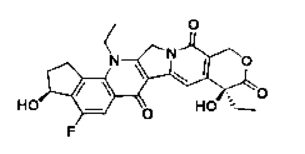 | 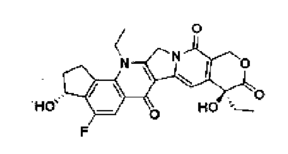 |
| 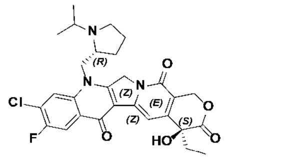 | 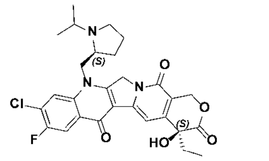 |
| 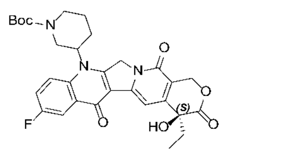 | 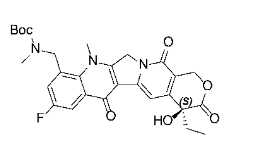 |
| 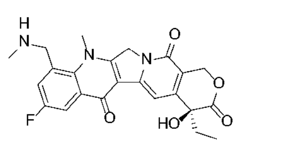 | 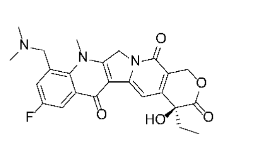 |
| 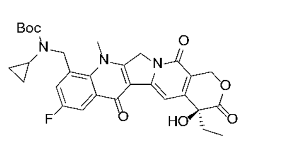 | 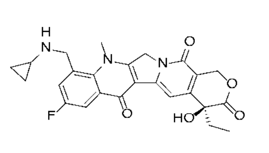 |
| 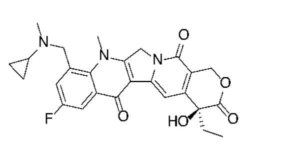 | 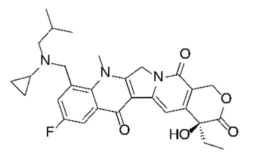 |

| | |
|---|---|
| 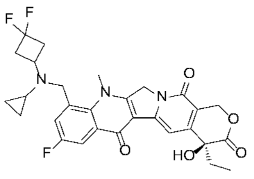 | 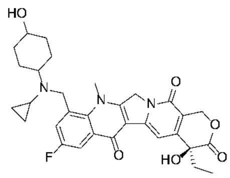 |
| 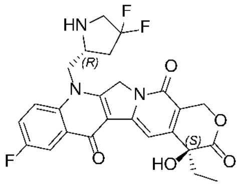 | 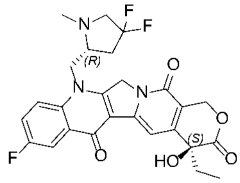 |
| 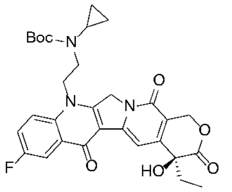 | 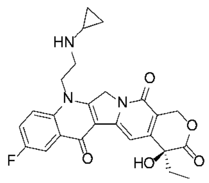 |
| 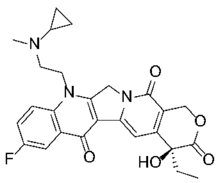 | 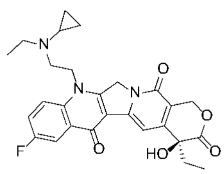 |
| 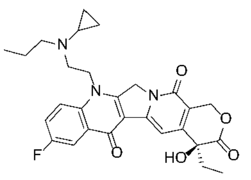 | 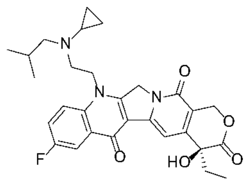 |
| 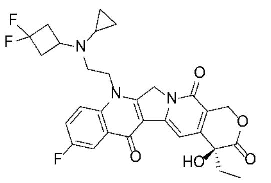 | 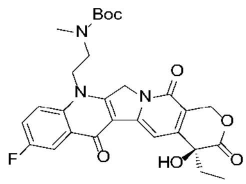 |
| 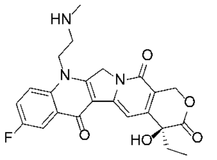 | 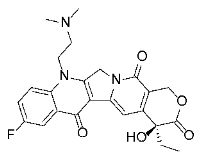 |
| 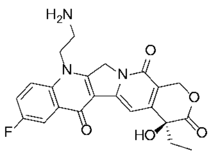 | 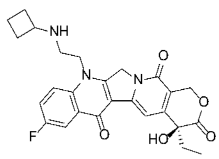 |
| 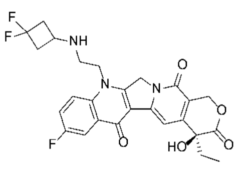 | 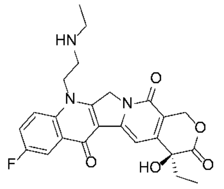 |

| | |
|---|---|
| 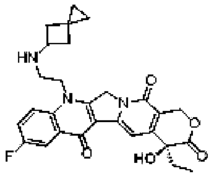 | 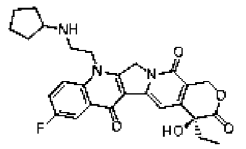 |
| 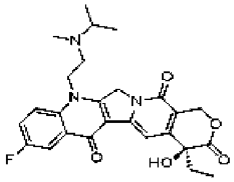 | 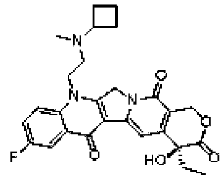 |
| 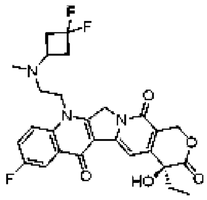 | 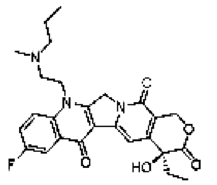 |
| 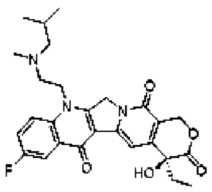 | 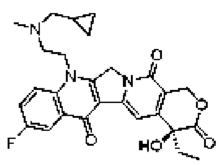 |
| 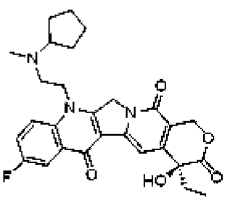 | 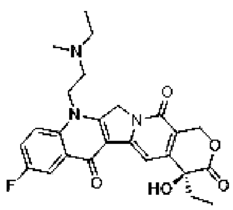 |
| 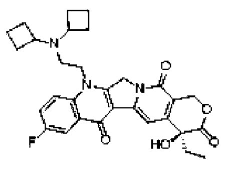 | 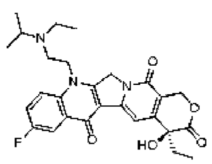 |
| 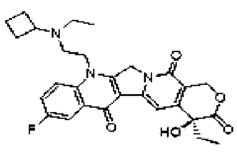 | 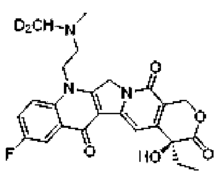 |
| 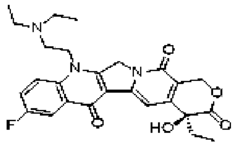 | 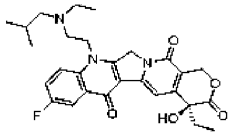 |

| | |
|---|---|
| 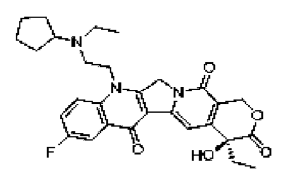 | 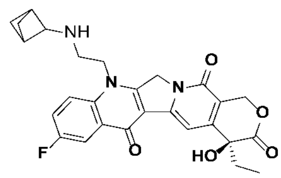 |
| 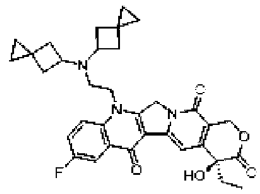 | 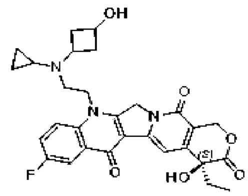 |
| 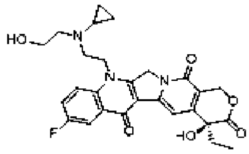 | 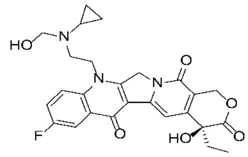 |
| 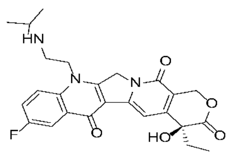 | 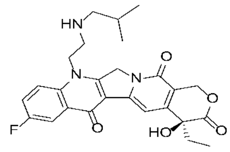 |
| 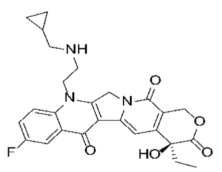 | 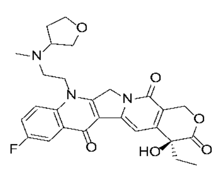 |
| 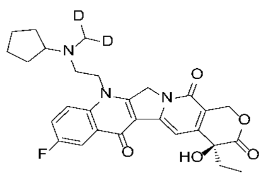 | 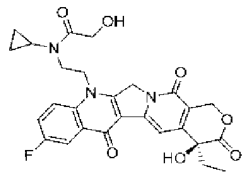 |
| 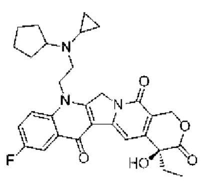 | 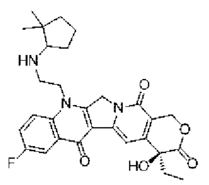 |
| 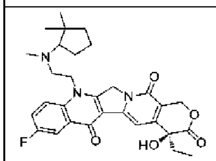 | 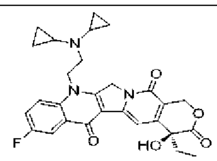 |
| 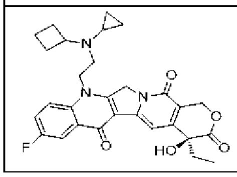 | 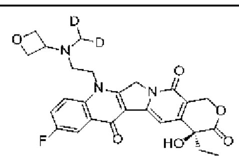 |

| | |
|---|---|
| 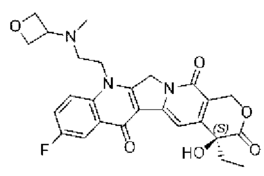 | 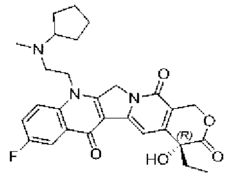 |
| 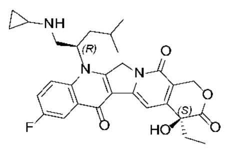 | 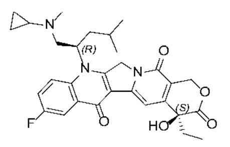 |
| 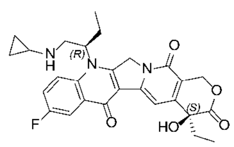 | 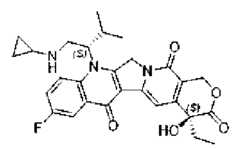 |
| 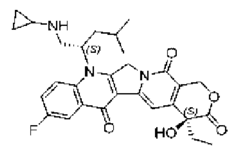 | 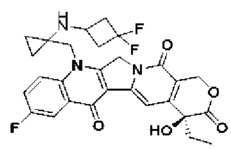 |
| 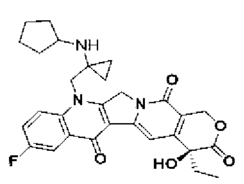 | 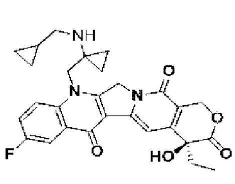 |
| 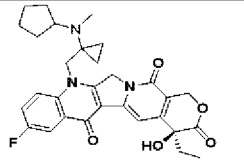 | 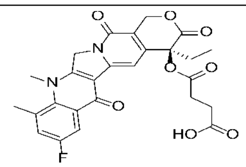 |
| 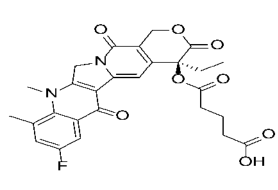 | 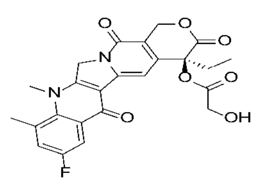 |
| 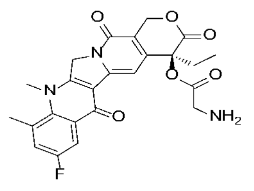 | 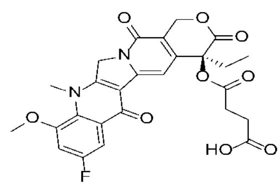 |
| 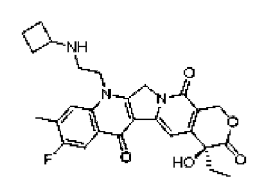 | 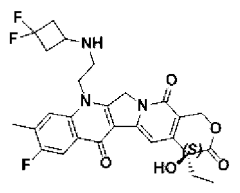 |

| | |
|---|---|
| 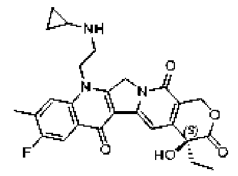 | 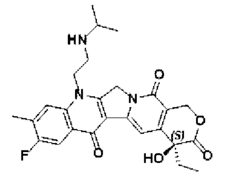 |
| 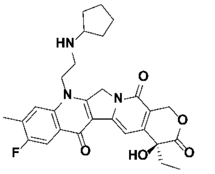 | 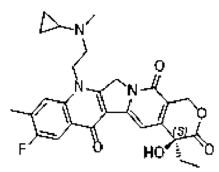 |
| 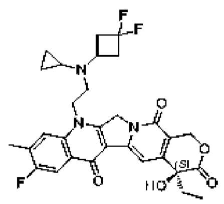 | 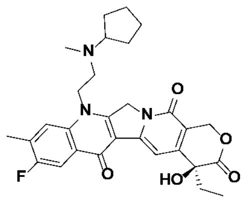 |
| 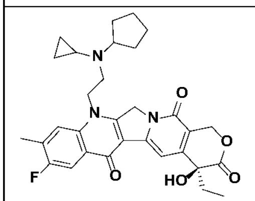 | 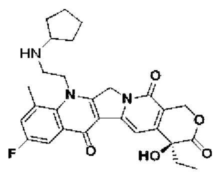 |
| 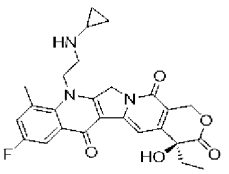 | 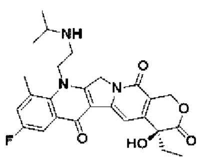 |
| 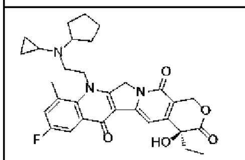 | 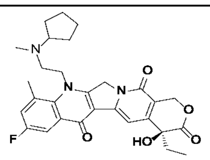 |
| 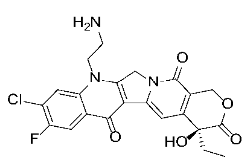 | 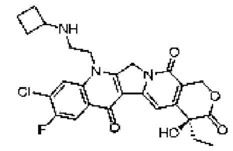 |
| 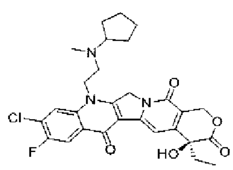 | 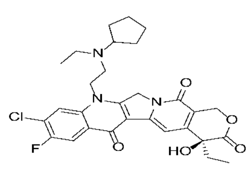 |
| 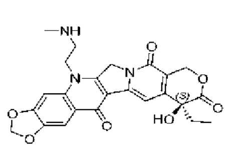 | 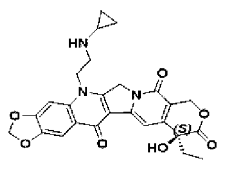 |

| | |
|---|---|
| 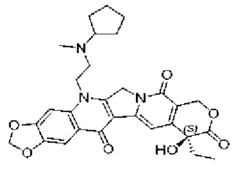 | 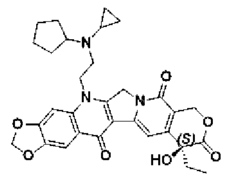 |
| 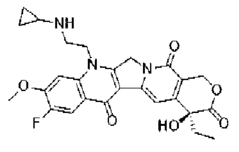 | 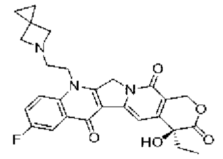 |
| 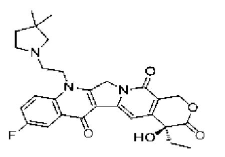 | 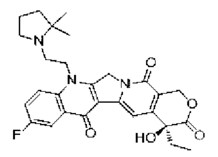 |
| 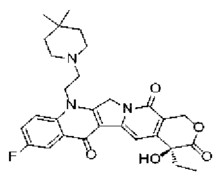 | 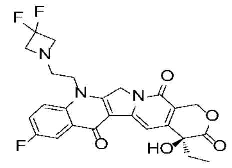 |
| 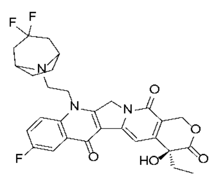 | 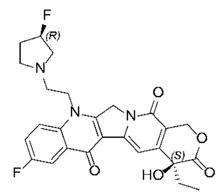 |
| 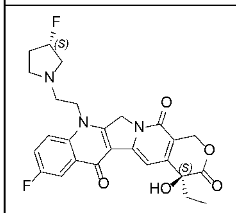 | 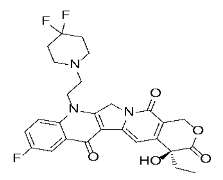 |
| 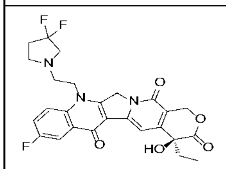 | 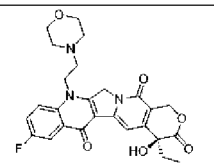 |
| 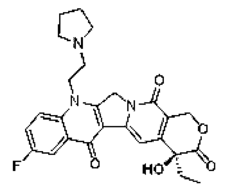 | 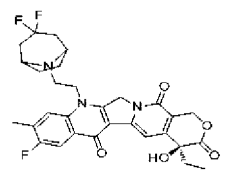 |
| 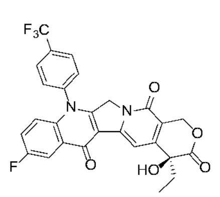 | 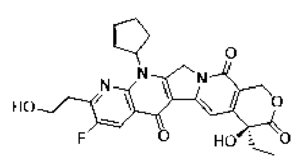 |

| | |
|---|---|
| 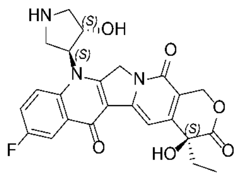 | 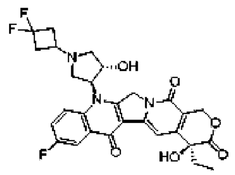 |
| 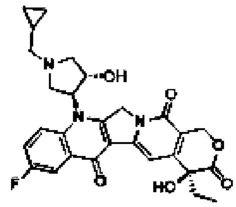 | 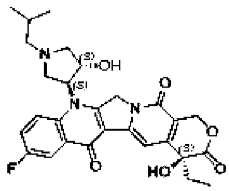 |
| 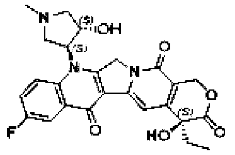 | 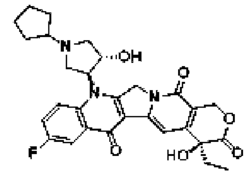 |
| 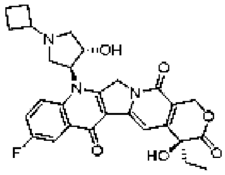 | 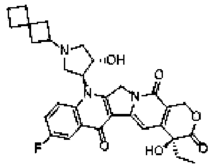 |
| 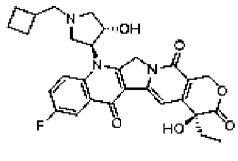 | 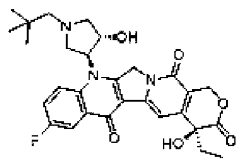 |
| 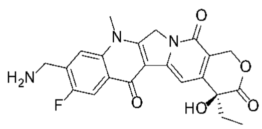 | 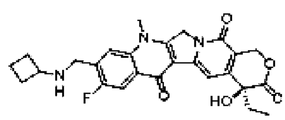 |
| 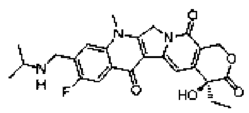 | 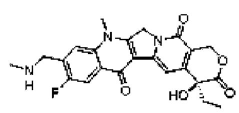 |
| 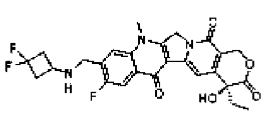 | 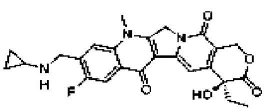 |
| 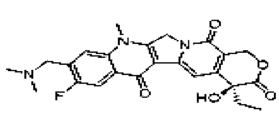 | 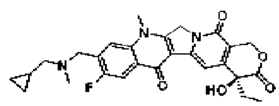 |

| | |
|---|---|
| 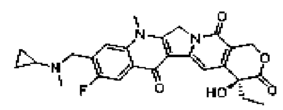 | 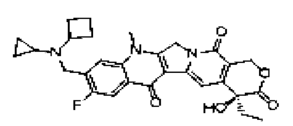 |
| 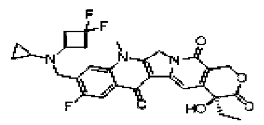 | 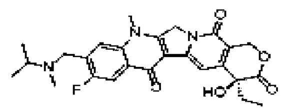 |
| 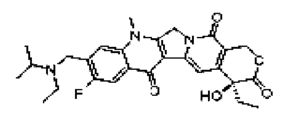 | 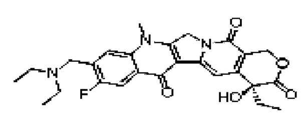 |
| 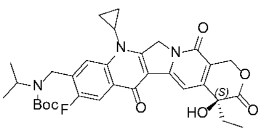 | 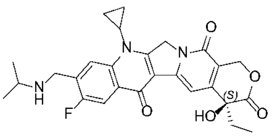 |
| 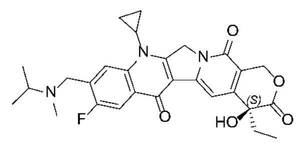 | 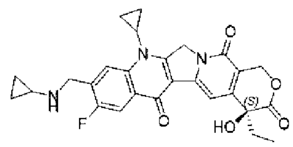 |
| 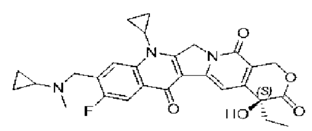 | 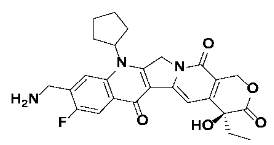 |
| 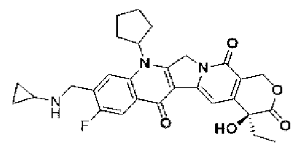 | 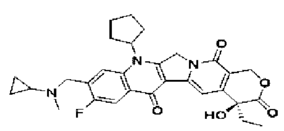 |
| 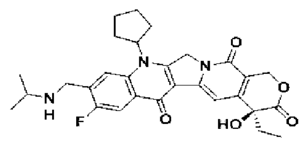 | 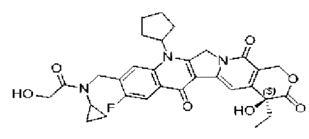 |
| 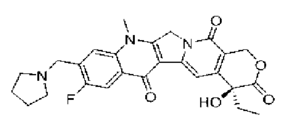 | 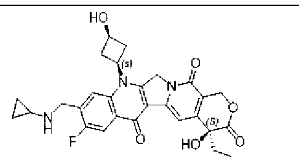 |
| 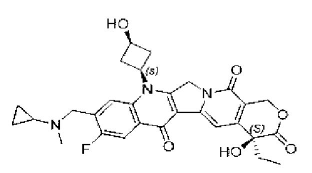 | 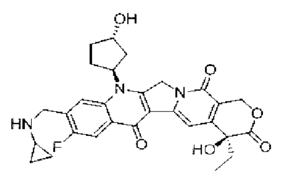 |
| 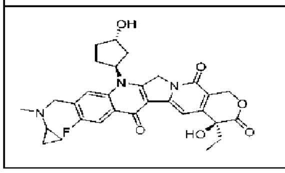 | 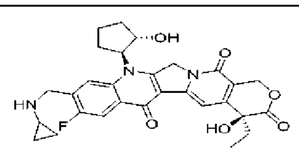 |

| | |
|---|---|
| 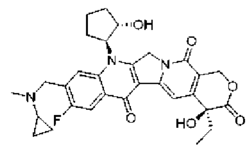 | 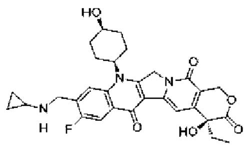 |
| 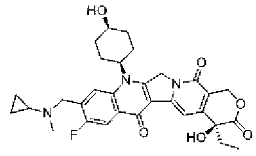 | 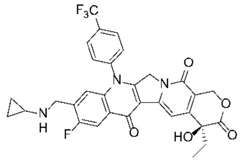 |
| 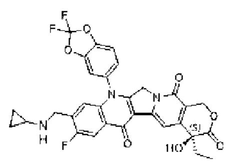 | 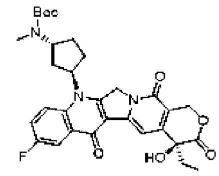 |
| 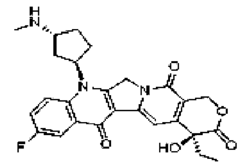 | 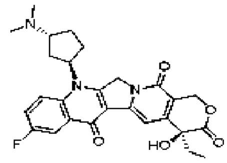 |
| 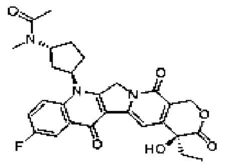 | 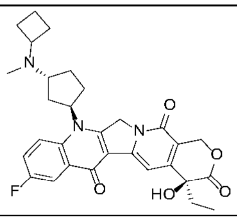 |
| 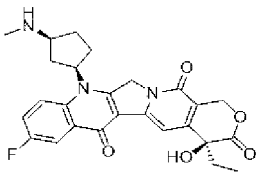 | 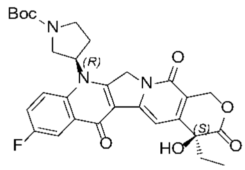 |
| 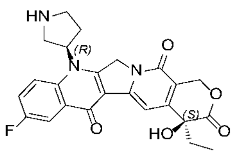 | 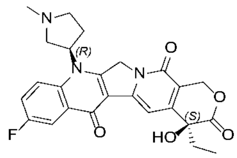 |
| 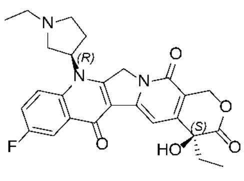 | 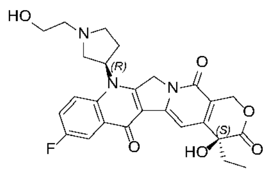 |
| 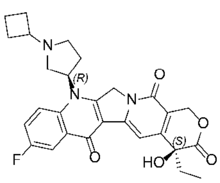 | 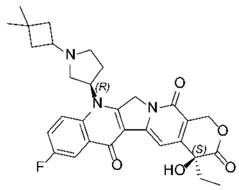 |

| | |
|---|---|
| 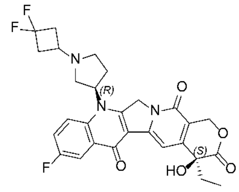 | 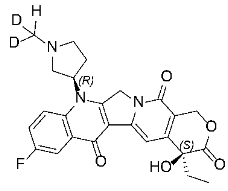 |
| 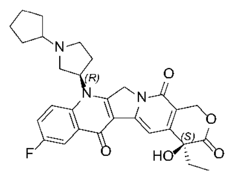 | 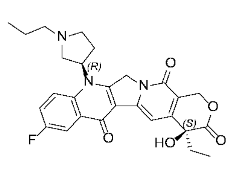 |
| 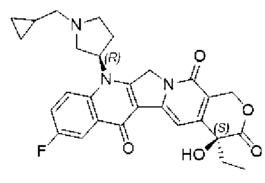 | 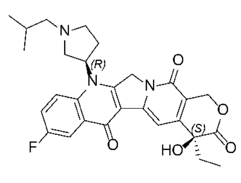 |
| 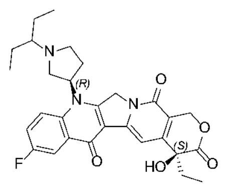 | 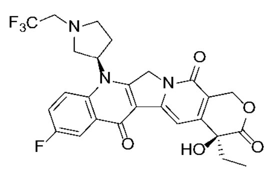 |
| 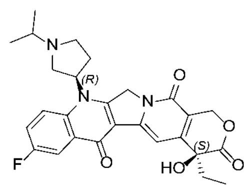 | 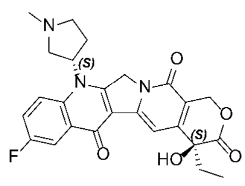 |
| 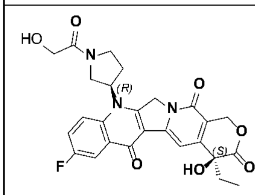 | 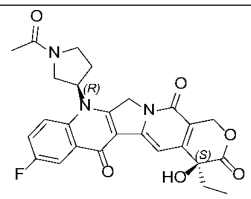 |
| 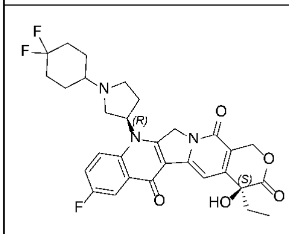 | 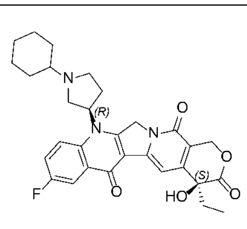 |
| 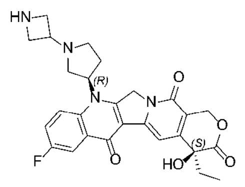 | 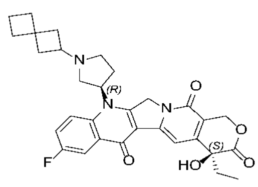 |
| 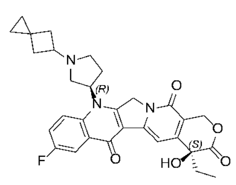 | 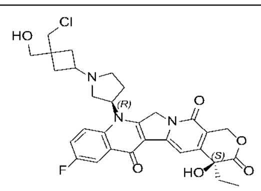 |

| | |
|---|---|
| 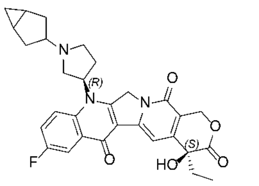 | 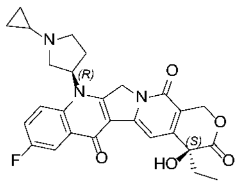 |
| 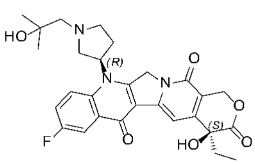 | 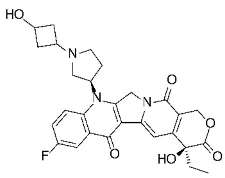 |
| 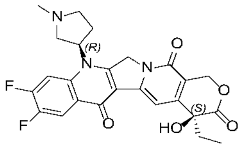 | 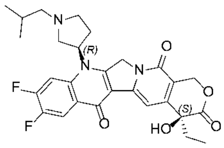 |
| 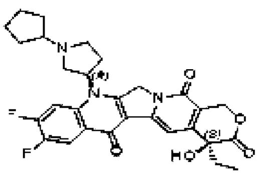 | 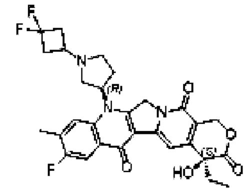 |
| 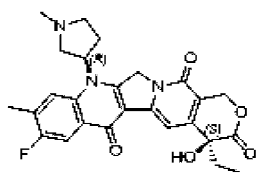 | 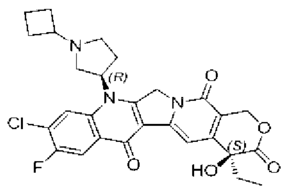 |
| 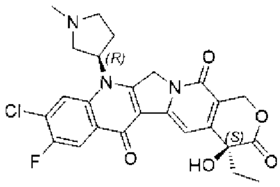 | 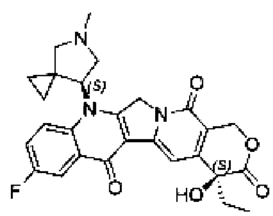 |
| 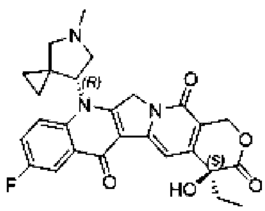 | 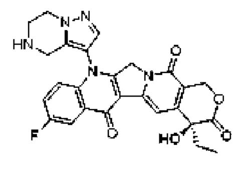 |
| 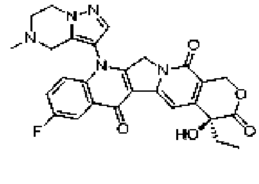 | 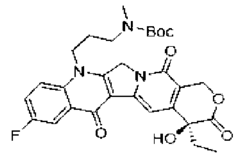 |

| | |
|---|---|
| 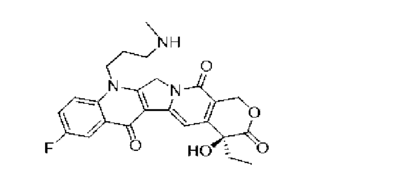 | 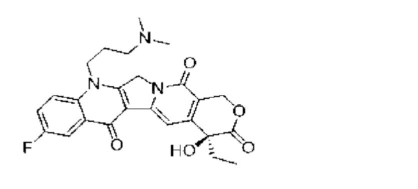 |
| 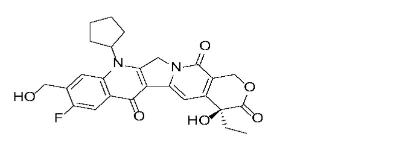 | 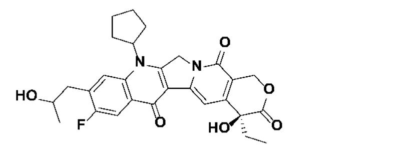 |
| 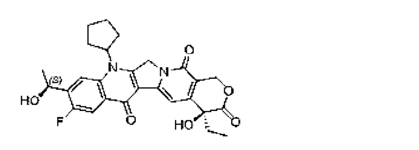 | 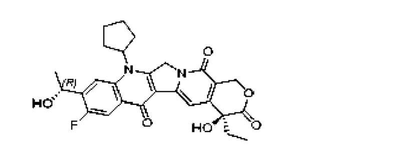 |
| 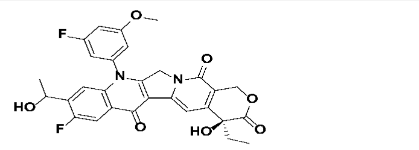 | 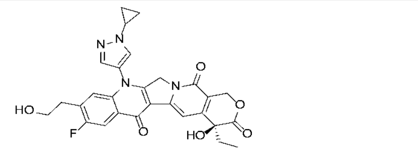 |
| 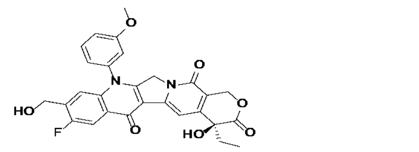 | 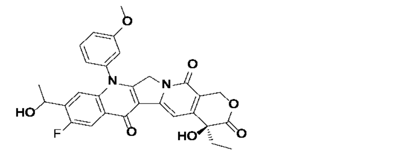 |
| 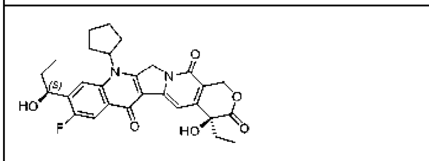 | 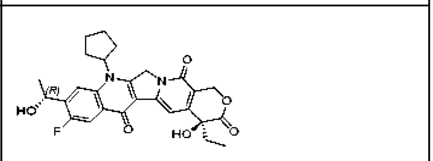 |
| 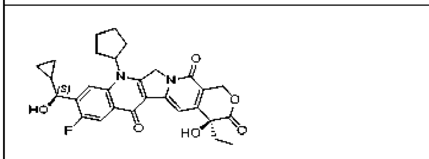 | 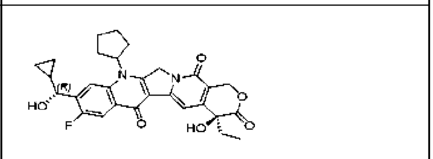 |
| 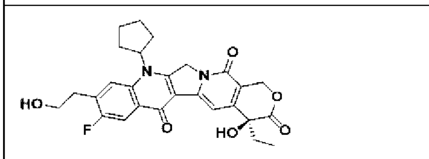 | 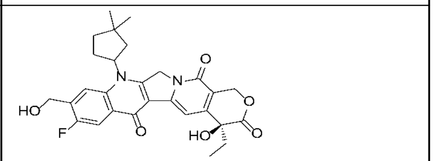 |
| 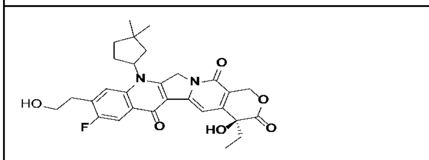 | 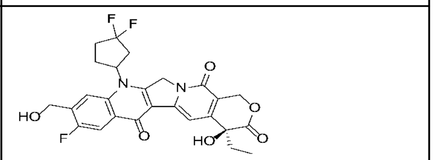 |
| 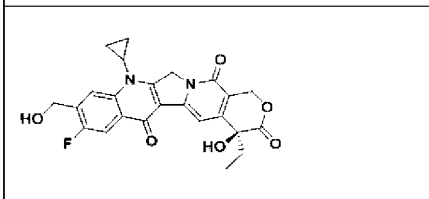 | 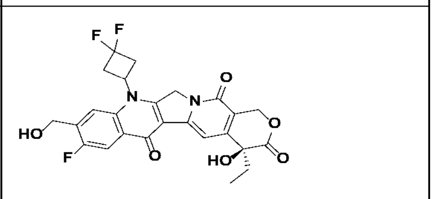 |
| 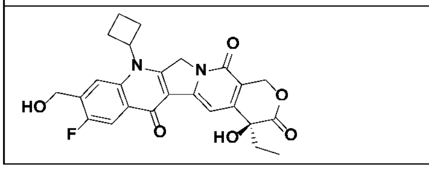 | 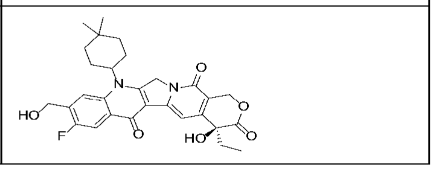 |

| | |
|---|---|
| 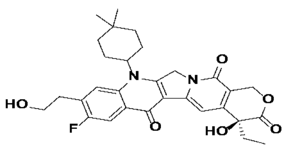 | 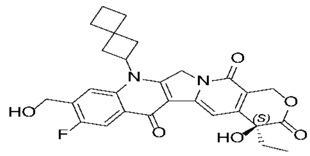 |
| 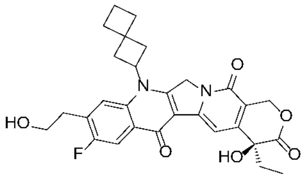 | 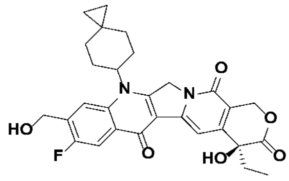 |
| 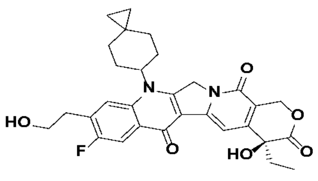 | 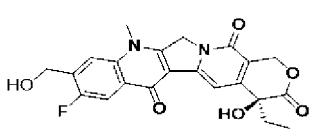 |
| 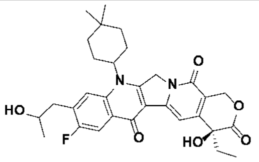 | 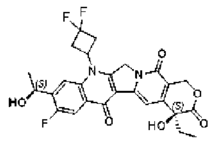 |
| 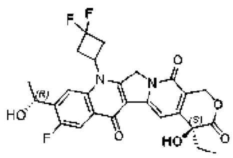 | 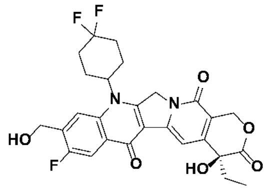 |
| 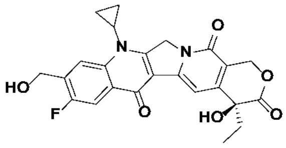 | 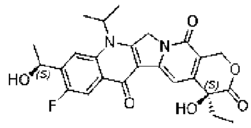 |
| 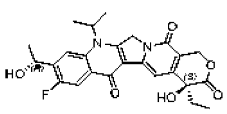 | 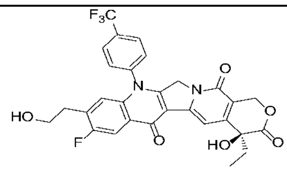 |
| 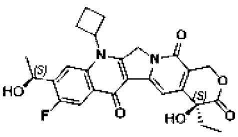 | 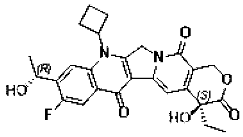 |
| 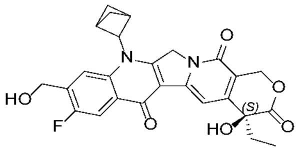 | 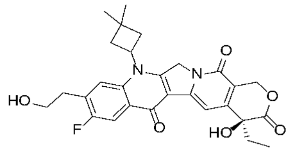 |
| 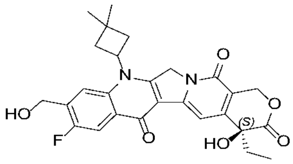 | 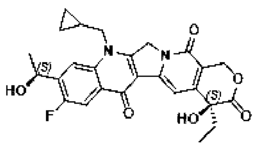 |

| | |
|---|---|
| 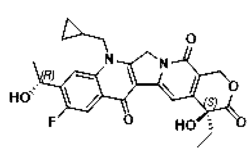 | 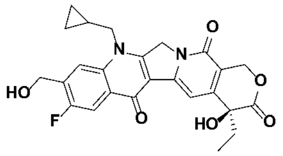 |
| 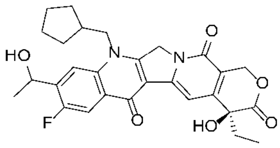 | 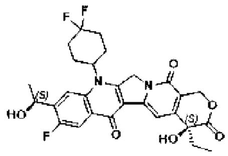 |
| 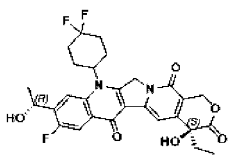 | 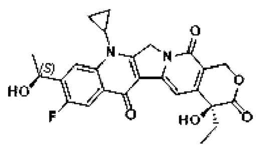 |
| 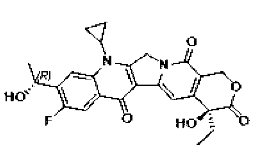 | 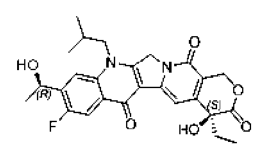 |
| 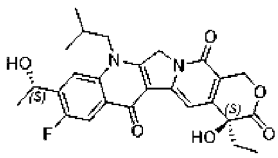 | 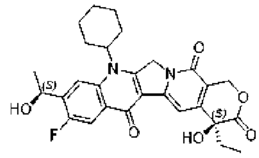 |
| 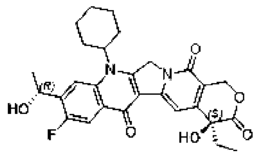 | 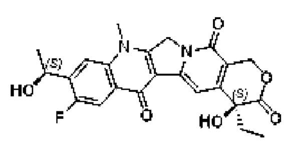 |
| 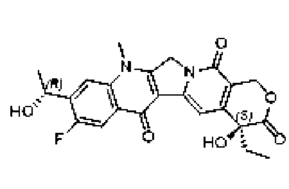 | 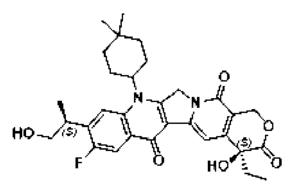 |
| 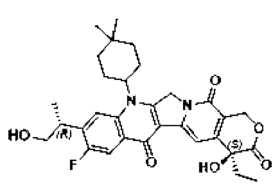 | 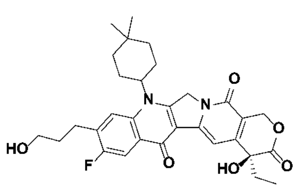 |
| 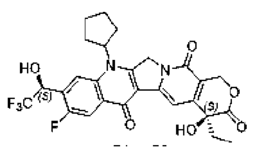 | 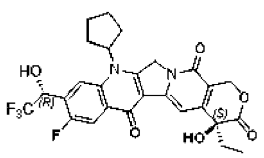 |
| 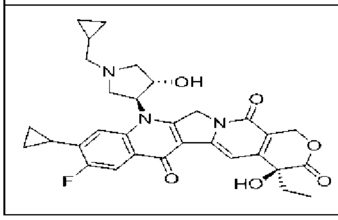 | 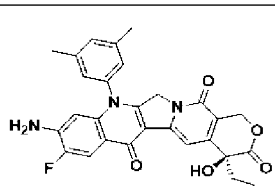 |

| | |
|---|---|
| 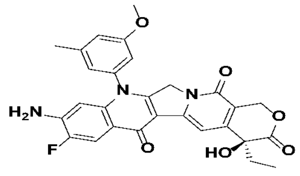 | 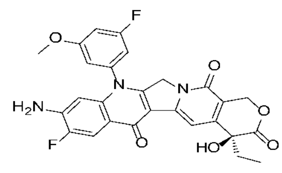 |
| 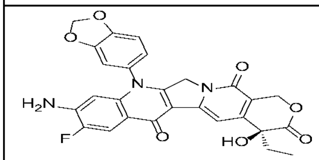 | 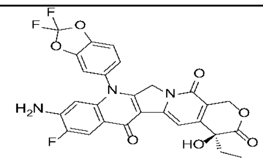 |
| 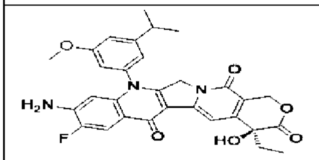 | 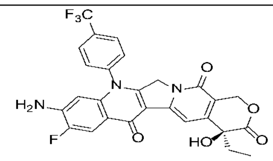 |
| 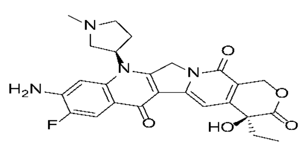 | 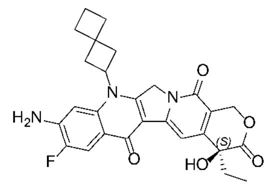 |
| 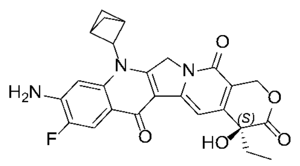 | 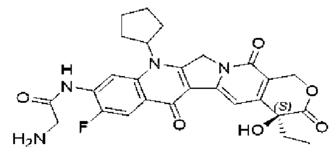 |
| 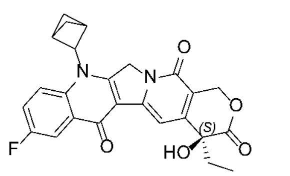 | 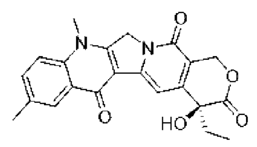 |
| 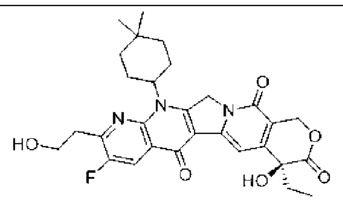 | 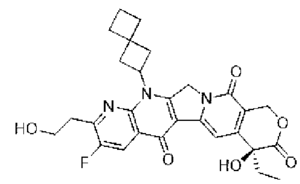 |
| 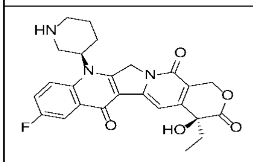 | 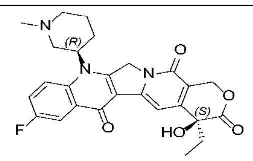 |
| 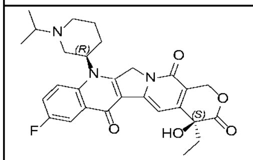 | 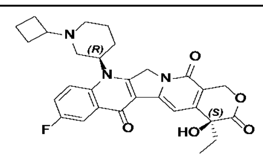 |
| 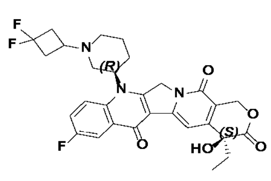 | 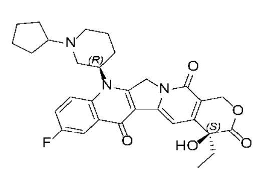 |

| | |
|---|---|
| 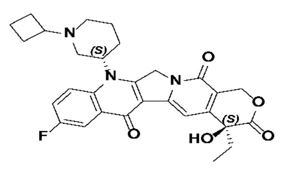 | 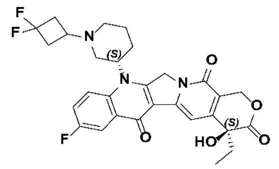 |
| 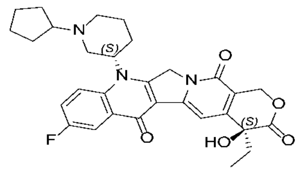 | 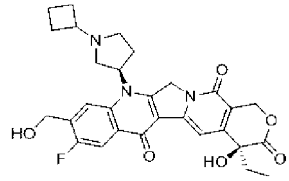 |
| 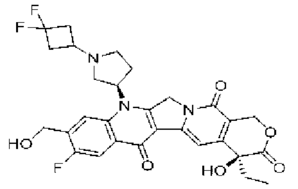 | 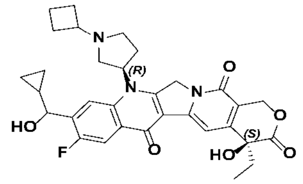 |
| 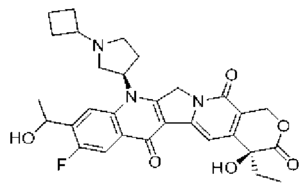 | 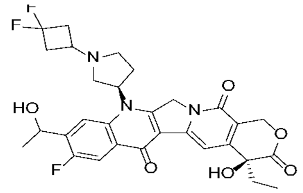 |
| 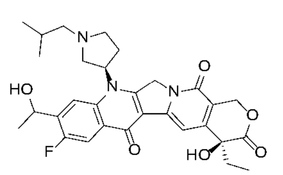 | 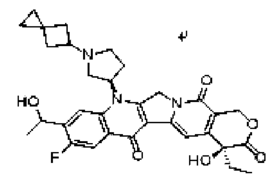 |
| 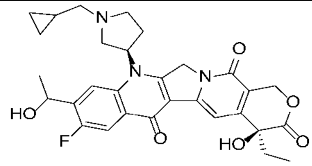 | 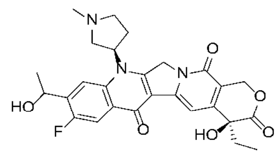 |
| 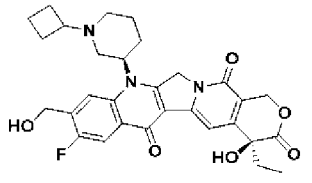 | 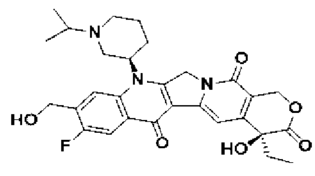 |
| 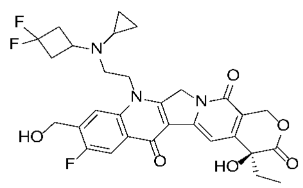 | 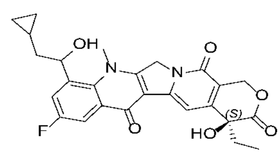 |
| 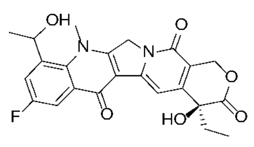 | 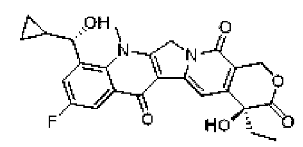 |

| | |
|---|---|
| 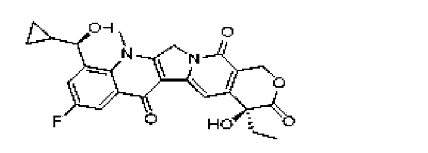 | 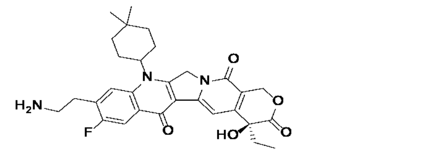 |
| 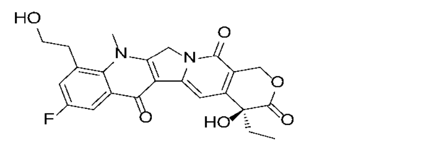 | 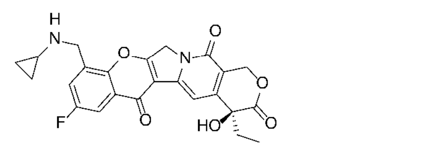 |
| 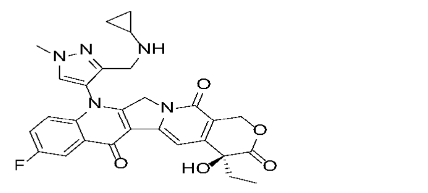 | 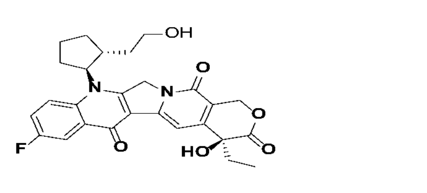 |
| 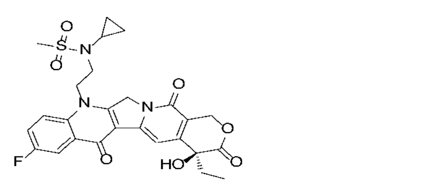 | 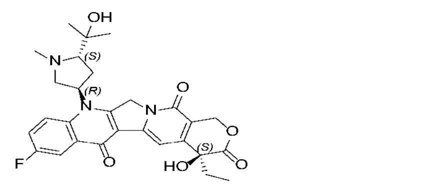 |
| 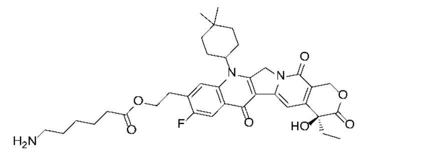 | 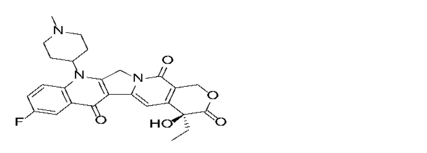 |
| 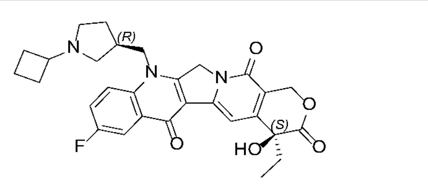 | 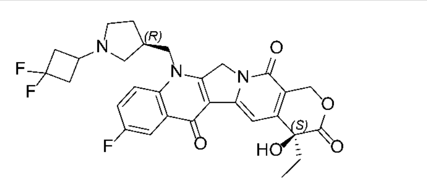 |
| 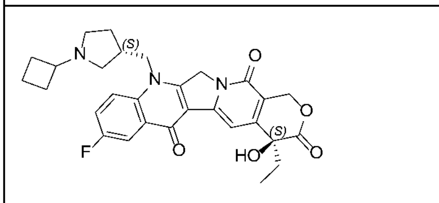 | 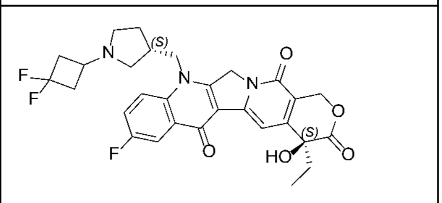 |
| 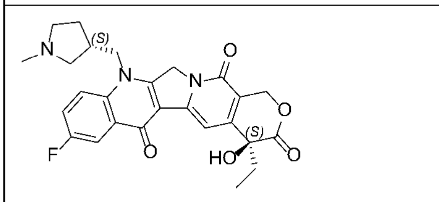 | 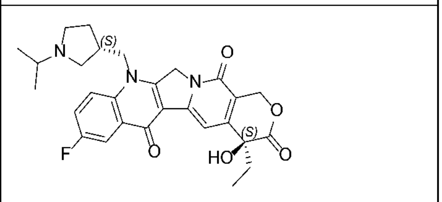 |
| 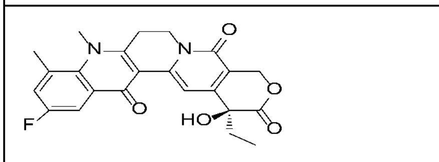 | 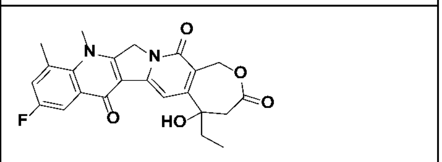 |
| 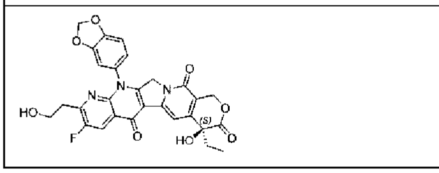 | 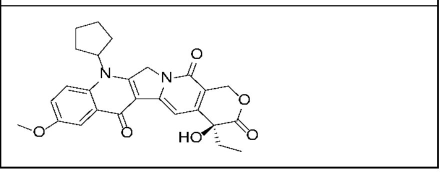 |

| | |
|---|---|
| 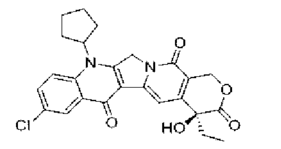 | 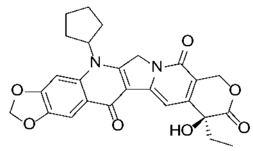 |
| 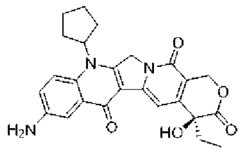 | 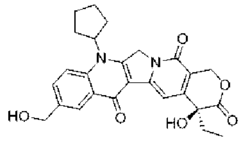 |

**26.** A pharmaceutical composition comprising the compound as described in any one of claims 1 - 25, and its stereoisomers, geometric isomers, tautomers, pharmaceutically acceptable salts, prodrugs, solvates, esters, polymorphs, N-oxides, metabolites or isotope-labeled analogs, together with pharmaceutically acceptable excipients.

**27.** The pharmaceutical composition according to claim 26, wherein the pharmaceutical composition further comprises additional medicaments for the prevention and/or treatment of cancer or tumors.

**28.** A compound according to any one of claims 1-25, or a pharmaceutical composition according to claim 26 or 27, for use in treating a disease mediated by topoisomerase I (Topo I).

**29.** The compound or pharmaceutical composition for use according to claim 28, wherein the topoisomerase I (Topo I)-mediated disease is cancer or a tumor-related disease.

**30.** The compound according to any one of claims 1-25, or the pharmaceutical composition according to claim 26 or 27, for use according to claim 28 or 29, wherein the topoisomerase I (Topo I)-mediated disease is cancer or a tumor-related disease..

**31.** The compound or pharmaceutical composition for use according to claim 30, wherein the topoisomerase I (Topo I)-mediated disease is skin cancer, bladder cancer, ovarian cancer, breast cancer, gastric cancer, pancreatic cancer, prostate cancer, lung cancer, bone cancer, brain cancer, neuroblastoma, rectal cancer, colon cancer, familial adenomatous polyposis cancer, hereditary non-polyposis colorectal cancer, esophageal cancer, lip cancer, laryngeal cancer, hypopharyngeal cancer, tongue cancer, salivary gland cancer, adenocarcinoma, medullary thyroid cancer, papillary thyroid cancer, kidney cancer, renal parenchymal cancer, cervical cancer, corpus uteri cancer, endometrial cancer, choriocarcinoma, testicular cancer, urinary cancer, melanoma, brain tumors (such as glioblastoma, astrocytoma, meningioma, medulloblastoma and peripheral neuroectodermal tumor), Hodgkin lymphoma, non-Hodgkin lymphoma, Burkitt lymphoma, leukemia, acute lymphoblastic leukemia (ALL), chronic lymphocytic leukemia (CLL), acute myeloid leukemia (AML), chronic myeloid leukemia (CML), adult T-cell leukemia/lymphoma, diffuse large B-cell lymphoma (DLBCL), hepatocellular carcinoma, gallbladder cancer, bronchial cancer, small-cell lung cancer, non-small-cell lung cancer, multiple myeloma, basal cell carcinoma, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing sarcoma, and plasmacytoma..

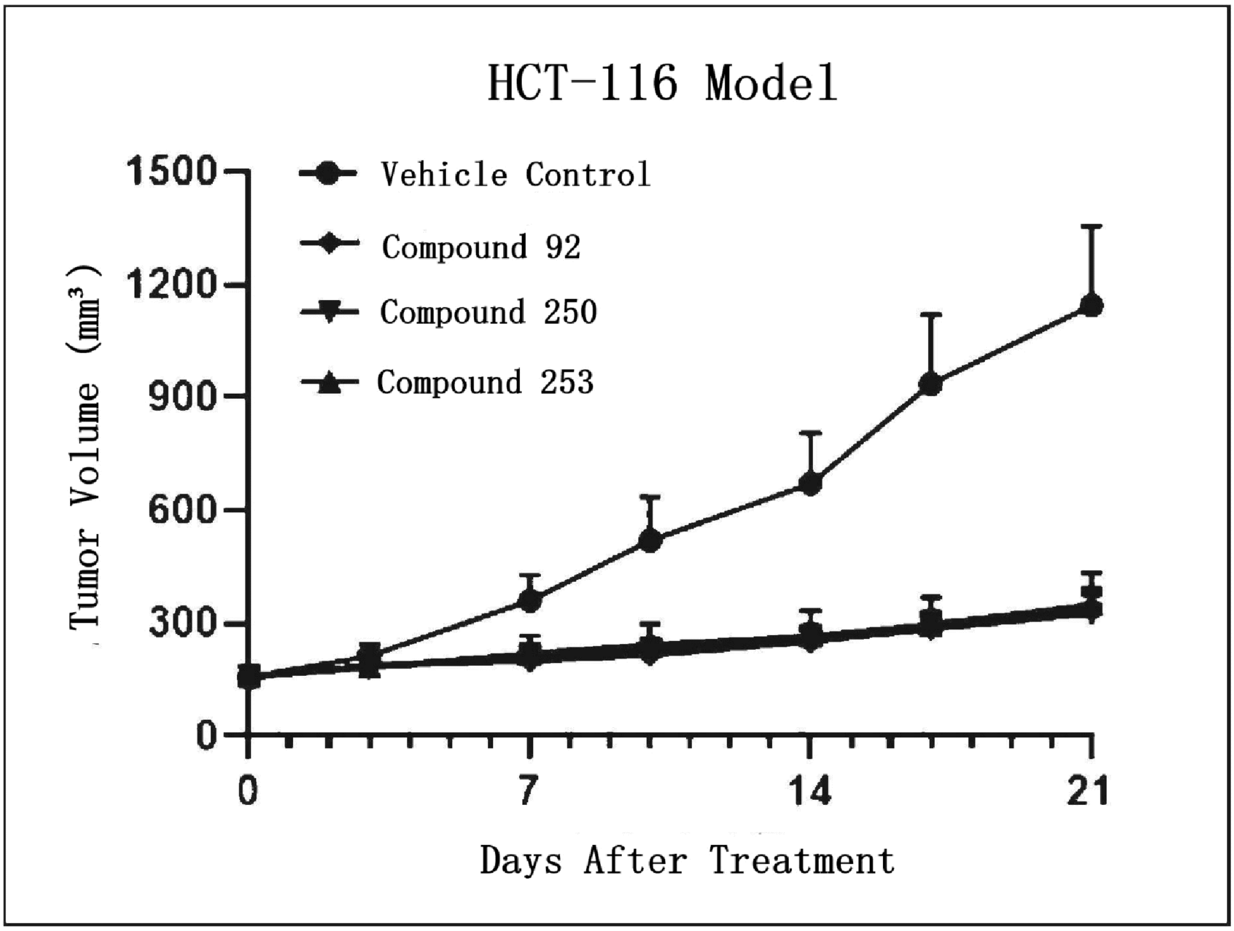
HCT-116 Model
Vehicle Control
Compound 92
Compound 250
Compound 253
Tumor Volume (mm³)
1500
1200
900
600
300
0
0
7
14
21
Days After Treatment

FIG. 1

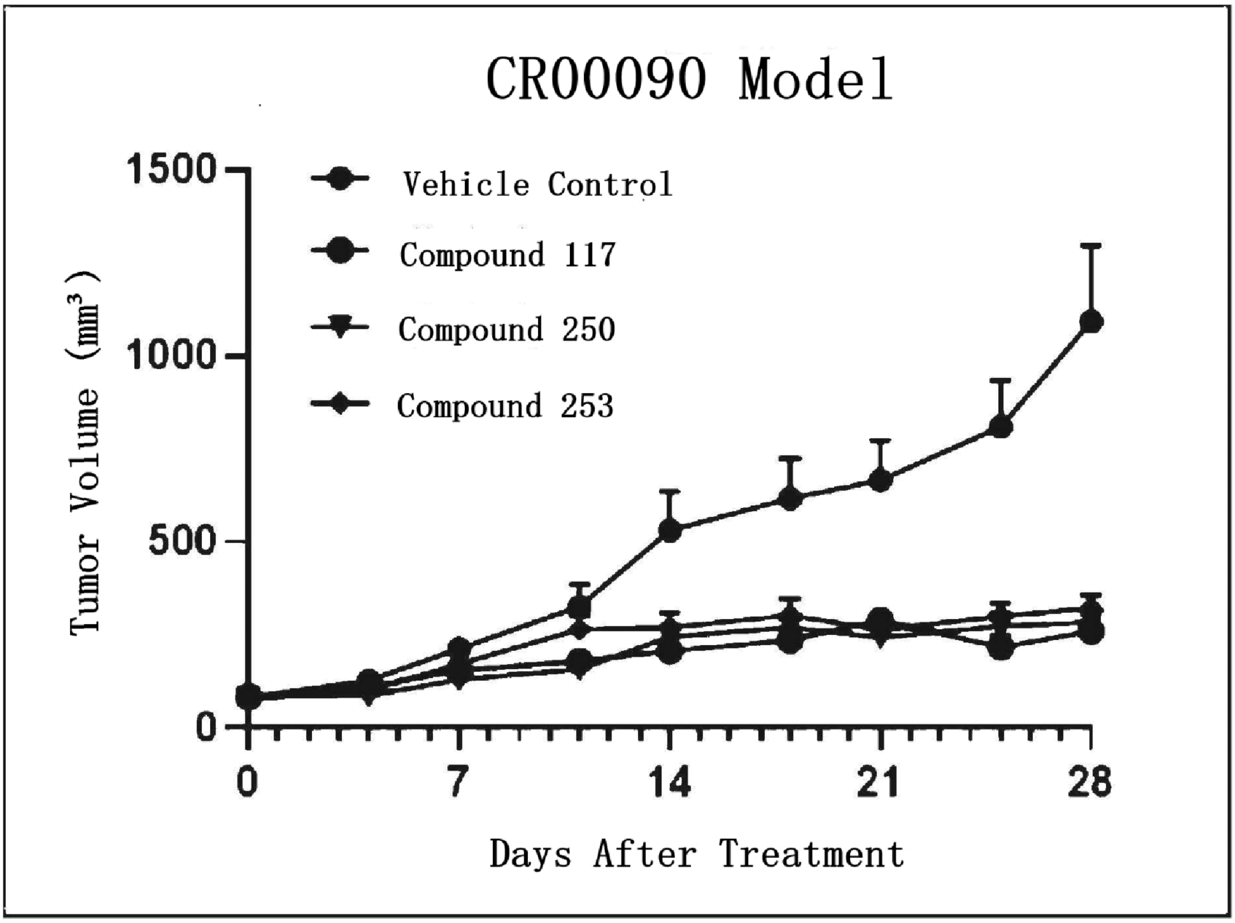
CR00090 Model
Vehicle Control
Compound 117
Compound 250
Compound 253
Tumor Volume (mm³)
1500
1000
500
0
0
7
14
21
28
Days After Treatment

FIG. 2

**INTERNATIONAL SEARCH REPORT**

International application No. **PCT/CN2024/123915**

**A. CLASSIFICATION OF SUBJECT MATTER**

C07D 491/22(2006.01)i; A61K 31/4745(2006.01)i; A61P 35/00(2006.01)i; A61P 31/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, WPABS, WPABSC, REGISTRY (STN), CAPLUS (STN): 上海翊维康医药有限责任公司, 拓扑异构酶, TOPO, 喜树碱, CPT, 癌症, 肿瘤, cancer, tumor, tumour, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 1557814 A (SECOND MILITARY MEDICAL UNIVERSITY OF PLA) 29 December 2004 (2004-12-29)<br>claims 1-10 | 1-31 |
| A | CN 101397302 A (SECOND MILITARY MEDICAL UNIVERSITY OF PLA) 01 April 2009 (2009-04-01)<br>claims 1-7 | 1-31 |
| A | CN 102010418 A (SECOND MILITARY MEDICAL UNIVERSITY OF PLA) 13 April 2011 (2011-04-13)<br>claims 1-5 | 1-31 |
| A | CN 1793145 A (SECOND MILITARY MEDICAL UNIVERSITY OF PLA) 28 June 2006 (2006-06-28)<br>claims 1-8 | 1-31 |
| A | CN 1192740 A (SOCIETE DE CONSEILS ET DE RECHERCHES D'APPLICATIONS SCIENTIFIQUES) 09 September 1998 (1998-09-09)<br>claims 1-31 | 1-31 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 January 2025** | **10 January 2025** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/123915**

**C. DOCUMENTS CONSIDERED TO BE RELEVANT**

| Category* | Citation of document, with indication, where appropriate, of the relevant passages | Relevant to claim No. |
|---|---|---|
| A | CN 101220037 A (SECOND MILITARY MEDICAL UNIVERSITY OF PLA) 16 July 2008 (2008-07-16)<br>claims 1-5 | 1-31 |
| A | US 5981542 A (SOCIETE DE CONSEILS DE RECHERCHES ETD'APPLICATIONS SCIENTIFIQUES) 09 November 1999 (1999-11-09)<br>claims 1-13 | 1-31 |

Form PCT/ISA/210 (second sheet) (July 2022)

**INTERNATIONAL SEARCH REPORT**

International application No.
**PCT/CN2024/123915**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **29-31**
because they relate to parts of the international application that do not comply with the prescribed requirements to such an extent that no meaningful international search can be carried out, specifically:

Claims 29-31 relate to a method for treating a disease, which falls within the cases set out in PCT Rule 39.1(iv). The present search report is provided on the basis of the use of a compound in the preparation of a drug for treating the disease.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/123915**

| Patent document cited in search report | Publication date (day/month/year) | Patent family member(s) | Publication date (day/month/year) |
|---|---|---|---|
| CN 1557814 A | 29 December 2004 | None | |
| CN 101397302 A | 01 April 2009 | None | |
| CN 102010418 A | 13 April 2011 | None | |
| CN 1793145 A | 28 June 2006 | None | |
| CN 1192740 A | 09 September 1998 | IL 128044 A0 | 30 November 1999 |
| | | CZ 415397 A3 | 12 August 1998 |
| | | CZ 296156 B6 | 11 January 2006 |
| | | DE 69623961 D1 | 31 October 2002 |
| | | DE 69623961 T2 | 08 May 2003 |
| | | DK 1251125 T3 | 18 April 2006 |
| | | CL 2004001168 A1 | 20 May 2005 |
| | | MX 9710228 A | 31 March 1998 |
| | | NZ 312715 A | 28 January 2000 |
| | | BR 9608639 A | 29 June 1999 |
| | | CA 2225528 A1 | 09 January 1997 |
| | | CA 2225528 C | 05 June 2007 |
| | | HK 1056726 A1 | 27 February 2004 |
| | | ATE 312105 T1 | 15 December 2005 |
| | | EP 0835258 A1 | 15 April 1998 |
| | | EP 0835258 B1 | 25 September 2002 |
| | | DE 69635560 D1 | 12 January 2006 |
| | | DE 69635560 T2 | 17 August 2006 |
| | | NO 975988 D0 | 19 December 1997 |
| | | NO 975988 L | 19 February 1998 |
| | | NO 316749 B1 | 26 April 2004 |
| | | AU 6460896 A | 22 January 1997 |
| | | AU 716377 B2 | 24 February 2000 |
| | | IL 122635 A0 | 16 August 1998 |
| | | IL 122635 A | 31 October 2001 |
| | | RU 2164515 C2 | 27 March 2001 |
| | | KR 19990028263 A | 15 April 1999 |
| | | KR 100440838 B1 | 17 December 2004 |
| | | PL 324339 A1 | 25 May 1998 |
| | | PL 185354 B1 | 30 April 2003 |
| | | WO 9700876 A1 | 09 January 1997 |
| | | DK 0835258 T3 | 03 February 2003 |
| | | TW 457234 B | 01 October 2001 |
| | | RO 117918 B1 | 30 September 2002 |
| | | JPH 11508249 A | 21 July 1999 |
| | | JP 3576171 B2 | 13 October 2004 |
| | | JP 2004123756 A | 22 April 2004 |
| | | PT 835258 E | 28 February 2003 |
| | | ES 2254600 T3 | 16 June 2006 |
| | | HK 1015783 A1 | 22 October 1999 |
| | | HK 1050686 A1 | 04 July 2003 |
| | | ES 2184882 T3 | 16 April 2003 |
| | | ATE 224900 T1 | 15 October 2002 |
| | | EP 1251125 A2 | 23 October 2002 |
| | | EP 1251125 A3 | 11 December 2002 |
| | | EP 1251125 B1 | 07 December 2005 |

Form PCT/ISA/210 (patent family annex) (July 2022)

**INTERNATIONAL SEARCH REPORT**
**Information on patent family members**

International application No.
**PCT/CN2024/123915**

| Patent document cited in search report | | | Publication date (day/month/year) | Patent family member(s) | | | Publication date (day/month/year) |
|---|---|---|---|---|---|---|---|
| CN | 101220037 | A | 16 July 2008 | None | | | |
| US | 5981542 | A | 09 November 1999 | ZA | 965318 | B | 24 January 1997 |
| | | | | GB | 9512670 | D0 | 23 August 1995 |
| | | | | ZA | 9605318 | B | 24 January 1997 |
| | | | | UA | 63891 | C2 | 16 February 2004 |
| | | | | US | 6313135 | B1 | 06 November 2001 |

Form PCT/ISA/210 (patent family annex) (July 2022)